# EUROPEAN PATENT APPLICATION

(11) **EP 4 053 277 A1**
(43) Date of publication of application: **07.09.2022**
(21) Application number: 21213766.5
(22) Date of filing: 26.12.2016
(51) Int. Cl.: C12N 15/113

(54) **COMPOSITIONS AND METHODS FOR THE TREATMENT OF HEMOGLOBINOPATHIES**

(30) Priority: 28.12.2015 US 201562271968 P; 08.06.2016 US 201662347484 P
(62) Divisional of application: 16826777.1
(71) Applicant: Novartis AG, 4056 Basel (CH); Intellia Therapeutics, Inc., Cambridge, MA 02139 (US)
(72) Inventor: BOITANO, Anthony Edward, San Diego, 92129 (US); COOKE, Michael, San Diego, 92103 (US); KLICKSTEIN, Lloyd B., Cambridge, 02139 (US); LESCARBEAU, Reynald, Cambridge, 02139 (US); MICKANIN, Craig Stephen, Cambridge, 02139 (US); MULUMBA, Kabungo, Cambridge, 02139 (US); POLICE, Seshidhar Reddy, Burlington, 01803 (US); SNEAD, Jennifer, San Diego, 92121 (US); STEVENSON, Susan C., Cambridge, 02139 (US); STEWART, Morag, Cambridge, 02139 (US); YANG, Yi, Cambridge, 02139 (US)
(74) Representative: Campbell, Lachlan Clive

(57) **Abstract**

The present invention is directed to genome editing systems, reagents and methods for the treatment of hemoglobinopathies.

## Description

### RELATED APPLICATIONS

This application claims priority to U.S. Provisional patent application number 62/271,968, filed December 28, 2015, and U.S. Provisional patent application number 62/347,484, filed June 8, 2016. The entire contents of these applications are incorporated herein by reference.

### BACKGROUND

CRISPRs (Clustered Regularly Interspaced Short Palindromic Repeats) evolved in bacteria as an adaptive immune system to defend against viral attack. Upon exposure to a virus, short segments of viral DNA are integrated into the CRISPR locus of the bacterial genome. RNA is transcribed from a portion of the CRISPR locus that includes the viral sequence. That RNA, which contains sequence complimentary to the viral genome, mediates targeting of a Cas9 protein to the sequence in the viral genome. The Cas9 protein cleaves and thereby silences the viral target.

Recently, the CRISPR/Cas system has been adapted for genome editing in eukaryotic cells. The introduction of site-specific single (SSBs) or double strand breaks (DSBs) allows for target sequence alteration through, for example, non-homologous end-joining (NHEJ) or homology-directed repair (HDR).

### SUMMARY OF THE INVENTION

Without being bound by theory, the invention is based in part on the discovery that CRISPR systems, e.g., Cas9 CRISPR systems, e.g., as described herein, can be used to modify cells (e.g., hematopoietic stem and progenitor cells (HSPCs)) to increase fetal hemoglobin (HbF) expression and/or decrease expression of beta globin (e.g., a beta globin gene having a disease-causing mutation), and that such cells may be used to treat hemoglobinopathies, e.g., sickle cell disease and beta thalassemia.

Thus, in an aspect, the invention provides CRISPR systems (e.g., Cas CRISPR systems, e.g., Cas9 CRISPR systems, e.g., *S. pyogenes* Cas9 CRISPR systems) comprising one or more, e.g., one, gRNA molecule as described herein. Any of the gRNA molecules described herein may be used in such systems, and in the methods and cells described herein.

In an aspect, the invention provides a gRNA molecule that includes a tracr and crRNA, wherein the crRNA includes a targeting domain that is complementary with a target sequence of the BCL11A gene, a BCL11a enhancer, or a HFPH region.

In another aspect, the invention provides a gRNA molecule that includes a targeting domain that is complementary with a target sequence of the BCL11A gene, e.g., is complementary with a target sequence within a BCL11a coding region (e.g., within a BCL11a exon, e.g., within BCL11a exon 2). In embodiments, the gRNA comprises a targeting domain that includes, e.g., consists of, any one of SEQ ID NO: 1 to SEQ ID NO: 85 or SEQ ID NO: 400 to SEQ ID NO: 1231.

In another aspect, the invention provides a gRNA molecule that includes a targeting domain that is complementary with a target sequence of a BCL11A enhancer.

In embodiments, the gRNA comprises a targeting domain that includes, e.g., consists of, any one of SEQ ID NO: 1232 to SEQ ID NO: 1499.

In embodiments, the gRNA to a target sequence of a BCL11a enhancer is to a target sequence within the +58 region of the BCL11a enhancer, and the targeting domain includes, e.g., consists of, any one of SEQ ID NO: 182 to SEQ ID NO: 277 or SEQ ID NO: 334 to SEQ ID NO: 341. In embodiments, the targeting domain includes, e.g., consists of, any one of SEQ ID NO: 341, SEQ ID NO: 246, SEQ ID NO: 248, SEQ ID NO: 247, SEQ ID NO: 245, SEQ ID NO: 249, SEQ ID NO: 244, SEQ ID NO: 199, SEQ ID NO: 251, SEQ ID NO: 250, SEQ ID NO: 334, SEQ ID NO: 185, SEQ ID NO: 186, SEQ ID NO: 336, or SEQ ID NO: 337. In embodiments, the targeting domain includes, e.g., consists of, SEQ ID NO: 248. In embodiments, the targeting domain includes, e.g., consists of, SEQ ID NO: 247. In embodiments, the targeting domain includes, e.g., consists of, SEQ ID NO: 245. In embodiments, the targeting domain includes, e.g., consists of, SEQ ID NO: 336. In embodiments, the targeting domain includes, e.g., consists of, SEQ ID NO: 337. In embodiments, the targeting domain includes, e.g., consists of, SEQ ID NO: 338. In embodiments, the targeting domain includes, e.g., consists of, SEQ ID NO: 335. In embodiments, the targeting domain includes, e.g., consists of, SEQ ID NO: 252. In an embodiment, the gRNA is a dgRNA that includes a crRNA that includes, e.g., consists of, e.g., from 5' to 3', SEQ ID NO: 248 - SEQ ID NO: 6607, and a tracr that includes, e.g., consists of, e.g., from 5' to 3', SEQ ID NO: 6660. In an embodiment, the gRNA is a dgRNA that includes a crRNA that includes, e.g., consists of, e.g., from 5' to 3', SEQ ID NO: 247 - SEQ ID NO: 6607, and a tracr that includes, e.g., consists of, e.g., from 5' to 3', SEQ ID NO: 6660. In an embodiment, the gRNA molecule is a sgRNA molecule and includes, e.g., consists of, e.g., from 5' to 3', SEQ ID NO: 338 - SEQ ID NO: 6604 - UUUU. In an embodiment, the gRNA molecule is a sgRNA molecule and includes, e.g., consists of, e.g., from 5' to 3', SEQ ID NO: 335 - SEQ ID NO: 6604 - UUUU. In an embodiment, the gRNA molecule is a sgRNA molecule and includes, e.g., consists of, e.g., from 5' to 3', SEQ ID NO: 336 - SEQ ID NO: 6604 - UUUU. In an embodiment, the gRNA molecule is a sgRNA molecule and includes, e.g., consists of, e.g., from 5' to 3', SEQ ID NO: 245 - SEQ ID NO: 6604 - UUUU. In an embodiment, the gRNA molecule is a sgRNA molecule and includes, e.g., consists of, e.g., from 5' to 3', SEQ ID NO: 337 - SEQ ID NO: 6604 - UUUU. In an embodiment, the gRNA molecule is a sgRNA molecule and includes, e.g., consists of, e.g., from 5' to 3', SEQ ID NO: 252 - SEQ ID NO: 6604 - UUUU.

In embodiments, the gRNA to a target sequence of a BCL11a enhancer is to a target sequence within the +62 region of the BCL11a enhancer, and the targeting domain includes, e.g., consists of, any one of SEQ ID NO: 278 to SEQ ID NO: 333. In embodiments, the targeting domain includes, e.g., consists of, any one of SEQ ID NO: 318, SEQ ID NO: 312, SEQ ID NO: 313, SEQ ID NO: 294, SEQ ID NO: 310, SEQ ID NO: 319, SEQ ID NO: 298, SEQ ID NO: 322, SEQ ID NO: 311, SEQ ID NO: 315, SEQ ID NO: 290, SEQ ID NO: 317, SEQ ID NO: 309, SEQ ID NO: 289, or SEQ ID NO: 281. In embodiments, the targeting domain includes, e.g., consists of, SEQ ID NO: 318.

In embodiments, the gRNA to a target sequence of a BCL11a enhancer is to a target sequence within the +55 region of the BCL11a enhancer, and the targeting domain includes, e.g., consists of, any one of SEQ ID NO: 1596 to SEQ ID NO: 1691. In embodiments, the targeting domain includes, e.g., consists of, any one of SEQ ID NO: 1683, SEQ ID NO: 1638, SEQ ID NO: 1647, SEQ ID NO: 1609, SEQ ID NO: 1621, SEQ ID NO: 1617, SEQ ID NO: 1654, SEQ ID NO: 1631, SEQ ID NO: 1620, SEQ ID NO: 1637, SEQ ID NO: 1612, SEQ ID NO: 1656, SEQ ID NO: 1619, SEQ ID NO: 1675, SEQ ID NO: 1645, SEQ ID NO: 1598, SEQ ID NO: 1599, SEQ ID NO: 1663, SEQ ID NO: 1677, or SEQ ID NO: 1626.

In another aspect, the invention provides a gRNA molecule that includes a targeting domain that is complementary with a target sequence of a hereditary persistence of fetal hemoglobin (HPFH) region. In an embodiment, the HPFH region is the French HPFH region. In embodiments, the targeting domain includes, e.g., consists of, any one of SEQ ID NO: 86 to SEQ ID NO: 181 or SEQ ID NO: 1500 to SEQ ID NO: 1595. In embodiments, the targeting domain includes, e.g., consists of, any one of SEQ ID NO: 100, SEQ ID NO: 165, SEQ ID NO: 113, SEQ ID NO: 99, SEQ ID NO: 112, SEQ ID NO: 98, SEQ ID NO: 1580, SEQ ID NO: 106, SEQ ID NO: 1503, SEQ ID NO: 1589, SEQ ID NO: 160, SEQ ID NO: 1537, SEQ ID NO: 159, SEQ ID NO: 101, SEQ ID NO: 162, SEQ ID NO: 104, SEQ ID NO: 138, SEQ ID NO: 1536, SEQ ID NO: 1539, SEQ ID NO: 1585. In embodiments, the targeting domain includes, e.g., consists of, SEQ ID NO: 100. In embodiments, the targeting domain includes, e.g., consists of, SEQ ID NO: 165. In embodiments, the targeting domain includes, e.g., consists of, SEQ ID NO: 113.

In any of the aforementioned embodiments, the gRNA molecule may further have regions and/or properties described herein. In an aspect, the gRNA molecule (e.g., of any of the aforementioned aspects or embodiments) includes a targeting domain that includes, e.g., consists of, 17, 18, 19, 20, 21, 22, 23, or 24 consecutive nucleic acids of any one of the recited targeting domain sequences. In embodiments, the 17, 18, 19, 20, 21, 22, 23, or 24 consecutive nucleic acids of any one of the recited targeting domain sequences are the 17, 18, 19, 20, 21, 22, 23, or 24 consecutive nucleic acids disposed at the 3' end of the recited targeting domain sequence. In embodiments, the 17, 18, 19, 20, 21, 22, 23, or 24 consecutive nucleic acids of any one of the recited targeting domain sequences are the 17, 18, 19, 20, 21, 22, 23, or 24 consecutive nucleic acids disposed at the 5' end of the recited targeting domain sequence. In embodiments, the 17, 18, 19, 20, 21, 22, 23, or 24 consecutive nucleic acids of any one of the recited targeting domain sequences do not include either the 5' or 3' nucleic acid of the recited targeting domain sequence. In any of the aforementioned aspects or embodiments, the targeting domain may consist of the recited targeting domain sequence.

In embodiments of the gRNA molecule, including in any of the aforementioned aspects and embodiments, the targeting domain includes 17, 18, 19, 20, 21 (if present in the reference sequence), 22 (if present in the reference sequence), 23 (if present in the reference sequence), 24 (if present in the reference sequence), or 25 (if present in the reference sequence) consecutive nucleic acids of any one of the recited targeting domain sequences. In other embodiments of the gRNA molecule, including in any of the aforementioned aspects and embodiments, the targeting domain consists of 17, 18, 19, 20, 21 (if present in the reference sequence), 22 (if present in the reference sequence), 23 (if present in the reference sequence), or 24 (if present in the reference sequence), or 25 (if present in the reference sequence) consecutive nucleic acids of any one of the recited targeting domain sequences. In embodiments of the gRNA molecule, including in any of the aforementioned aspects and embodiments, the 17, 18, 19, 20, 21 (if present in the reference sequence), 22 (if present in the reference sequence), 23 (if present in the reference sequence), or 24 (if present in the reference sequence), or 25 (if present in the reference sequence) consecutive nucleic acids of any one of the recited targeting domain sequences are the 17, 18, 19, 20, 21 (if present in the reference sequence), 22 (if present in the reference sequence), 23 (if present in the reference sequence), or 24 (if present in the reference sequence), or 25 (if present in the reference sequence) consecutive nucleic acids disposed at the 3' end of the recited targeting domain sequence. In other embodiments of the gRNA molecule, including in any of the aforementioned aspects and embodiments, the 17, 18, 19, 20, 21 (if present in the reference sequence), 22 (if present in the reference sequence), 23 (if present in the reference sequence), or 24 (if present in the reference sequence), or 25 (if present in the reference sequence) consecutive nucleic acids of any one of the recited targeting domain sequences are the 17, 18, 19, 20, 21 (if present in the reference sequence), 22 (if present in the reference sequence), 23 (if present in the reference sequence), or 24 (if present in the reference sequence), or 25 (if present in the reference sequence) consecutive nucleic acids disposed at the 5' end of the recited targeting domain sequence. In other embodiments of the gRNA molecule, including in any of the aforementioned aspects and embodiments, the 17, 18, 19, 20, 21 (if present in the reference sequence), 22 (if present in the reference sequence), 23 (if present in the reference sequence), or 24 (if present in the reference sequence), or 25 (if present in the reference sequence) consecutive nucleic acids of any one of the recited targeting domain sequences do not include either the 5' or 3' nucleic acid of the recited targeting domain sequence.

In an aspect, including in any of the aforementioned aspects or embodiments, the gRNA molecule includes a portion of the crRNA and a portion of the tracr that hybridize to form a flagpole, that includes SEQ ID NO: 6584 or 6585. In embodiments, the flagpole further includes a first flagpole extension, located 3' to the crRNA portion of the flagpole, wherein said first flagpole extension includes SEQ ID NO: 6586. In embodiments, the flagpole further includes a second flagpole extension located 3' to the crRNA portion of the flagpole and, if present, the first flagpole extension, wherein said second flagpole extension includes SEQ ID NO: 6587.

In an aspect, including in any of the aforementioned aspects or embodiments, the invention provides a gRNA molecule that includes a tracr that includes, e.g., consists of, SEQ ID NO: 6660 or SEQ ID NO: 6661. In embodiments, the crRNA portion of the flagpole includes SEQ ID NO: 6607 or SEQ ID NO: 6608.

In an aspect, including in any of the aforementioned aspects or embodiments, the invention provides a gRNA molecule that includes a tracr that includes SEQ ID NO: 6589 or 6590, and optionally, if a first flagpole extension is present, a first tracr extension, disposed 5' to SEQ ID NO: 6589 or 6590, said first tracr extension including SEQ ID NO: 6591.

In an aspect, including in any of the aforementioned aspects or embodiments, the invention provides a gRNA molecule wherein the targeting domain and the tracr are disposed on separate nucleic acid molecules (e.g., a dgRNA molecule).

In an aspect, including in any of the aforementioned aspects or embodiments, the invention provides a gRNA molecule wherein the targeting domain and the tracr are disposed on a single nucleic acid molecule (e.g., a sgRNA molecule), and wherein the tracr is disposed 3' to the targeting domain. In embodiments, the sgRNA molecule includes a loop, disposed 3' to the targeting domain and 5' to the tracr, e.g., a loop including, e.g., consisting of, SEQ ID NO: 6588.

In an aspect, including in any of the aforementioned aspects or embodiments, the invention provides a gRNA molecule including, from 5' to 3', [targeting domain]-:
(a) SEQ ID NO: 6601;
(b) SEQ ID NO: 6602;
(c) SEQ ID NO: 6603;
(d) SEQ ID NO: 6604; or
(e) any of (a) to (d), above, further including, at the 3' end, 1, 2, 3, 4, 5, 6 or 7 uracil (U) nucleotides, e.g., 4 uracil nucleotides. In embodiments, there are no intervening nucleotides between the targeting domain and the sequence of any of (a)-(e).

In another aspect, the invention provides CRISPR systems, e.g., Cas CRISPR systems, e.g., Cas9 CRISPR systems, e.g., *S*. *pyogenes* Cas9 CRISPR systems, the include one or more, e.g., one, gRNA molecule of any of the aforementioned aspects and embodiments. In an aspect, the invention provides a composition including a first gRNA molecule of any of the aforementioned aspects and embodiments, further including a Cas9 molecule. In embodiments, the Cas9 molecule is an active or inactive *s*. *pyogenes* Cas9. In embodiments, the first gRNA molecule and Cas9 molecule are present in a ribonuclear protein complex (RNP).

In another aspect, the invention provides compositions that include more than one gRNA, e.g., more than one gRNA molecule as described herein, e.g., more than one gRNA molecule of any of the aforementioned gRNA molecule aspects or embodiments. Thus, in a further aspect, the invention provides a composition of any of the aforementioned composition aspects and embodiments, further including a second gRNA molecule; a second gRNA molecule and a third gRNA molecule; or a second gRNA molecule, a third gRNA molecule, and a fourth gRNA molecule, wherein the second gRNA molecule, the third gRNA molecule (if present), and the fourth gRNA molecule (if present) are a gRNA molecule as described herein, e.g., a gRNA molecule of any of the aforementioned gRNA molecule aspects or embodiments. In an embodiment, each gRNA molecule of the composition is complementary to a different target sequence. In an embodiment, each gRNA molecule is complementary to target sequences within the same gene or region. In an aspect, the first gRNA molecule, the second gRNA molecule, the third gRNA molecule (if present), and the fourth gRNA molecule (if present) are complementary to target sequences not more than 20000 nucleotides, not more than 10000 nucleotides, not more than 6000, not more than 5000 nucleotides, not more than 4000, not more than 1000 nucleotides, not more than 500 nucleotides, not more than 400 nucleotides, not more than 300 nucleotides, not more than 200 nucleotides, not more than 100 nucleotides, not more than 90 nucleotides, not more than 80 nucleotides, not more than 70 nucleotides, not more than 60 nucleotides, not more than 50 nucleotides, not more than 40 nucleotides, not more than 30 nucleotides, not more than 20 nucleotides or not more than 10 nucleotides apart. In another aspect, each gRNA molecule of the composition is complementary to target sequence within a different gene or region.

Specific and preferred combinations of more than one gRNA molecule of the invention are described herein. In an aspect, the composition includes a first gRNA molecule and a second gRNA molecule, wherein the first gRNA molecule and second gRNA molecule are complementary to different target sequences, and are:
(a) independently selected from the gRNA molecules described herein (e.g., above) to the BCL11a gene;
(b) independently selected from the gRNA molecules described herein (e.g., above) to the +58 BCL11a enhancer;
(c) independently selected from the gRNA molecules described herein (e.g., above) to the +62 BCL11a enhancer;
(d) independently selected from the gRNA molecules described herein (e.g., above) to the +55 BCL11a enhancer; or
(e) independently selected from the gRNA molecules described herein (e.g., above) to a HPFH region.

In an aspect, the composition includes a first gRNA molecule and a second gRNA molecule, wherein the first gRNA molecule and second gRNA molecule are complementary to different target sequences, and:
(a) the first gRNA molecule is selected from the gRNA molecules described herein (e.g., above) to the BCL11a gene, and the second gRNA molecule is selected from the gRNA molecules described herein (e.g., above) to the +58 BCL11a enhancer;
(b) the first gRNA molecule is selected from the gRNA molecules described herein (e.g., above) to the BCL11a gene, and the second gRNA molecule is selected from the gRNA molecules described herein (e.g., above) to the +62 BCL11a enhancer;
(c) the first gRNA molecule is selected from the gRNA molecules described herein (e.g., above) to the BCL11a gene, and the second gRNA molecule is selected from the gRNA molecules described herein (e.g., above) to the +55 BCL11a enhancer;
(d) the first gRNA molecule is selected from the gRNA molecules described herein (e.g., above) to the BCL11a gene, and the second gRNA molecule is selected from the gRNA molecules described herein (e.g., above) to a HPFH region;
(e) the first gRNA molecule is selected from the gRNA molecules described herein (e.g., above) to the +58 BCL11a enhancer, and the second gRNA molecule is selected from the gRNA molecules described herein (e.g., above) to the +62 BCL11a enhancer;
(f) the first gRNA molecule is selected from the gRNA molecules described herein (e.g., above) to the +58 BCL11a enhancer, and the second gRNA molecule is selected from the gRNA molecules described herein (e.g., above) to the +55 BCL11a enhancer;
(g) the first gRNA molecule is selected from the gRNA molecules described herein (e.g., above) to the +58 BCL11a enhancer, and the second gRNA molecule is selected from the gRNA molecules described herein (e.g., above) to a HPFH region;
(h) the first gRNA molecule is selected from the gRNA molecules described herein (e.g., above) to the +62 BCL11a enhancer, and the second gRNA molecule is selected from the gRNA molecules described herein (e.g., above) to the +55 BCL11a enhancer;
(i) the first gRNA molecule is selected from the gRNA molecules described herein (e.g., above) to the +62 BCL11a enhancer, and the second gRNA molecule is selected from the gRNA molecules described herein (e.g., above) to a HPFH region;
(j) the first gRNA molecule is selected from the gRNA molecules described herein (e.g., above) to the +55 BCL11a enhancer, and the second gRNA molecule is selected from the gRNA molecules described herein (e.g., above) to a HPFH region;

In another aspect, the composition that includes a first gRNA molecule and a second gRNA molecule, includes:
(a) a first gRNA molecule that is selected from the gRNA molecules described herein (e.g., above) to a HPFH region, and a second gRNA molecule includes a targeting domain that is complementary to a target sequence of the beta globin gene; or
(b) a first gRNA molecule that is selected from the gRNA molecules described herein (e.g., above) to a BCL11a enhancer, e.g., a +58 BCL11a enhancer, a +55 BCL11a enhancer or a +62 BCL11a enhancer, and a second gRNA molecule includes a targeting domain that is complementary to a target sequence of the beta globin gene.

In any of the aforementioned composition aspects and embodiments, the composition may consist of, with respect to the gRNA components of the composition, a first gRNA molecule and a second gRNA molecule.

In any of the aforementioned composition aspects and embodiments, the compositions may be formulated in a medium suitable for electroporation.

In another aspect, the invention provides nucleic acids encoding the gRNA molecule(s) and/or Cas molecules of the CRISPR systems. Without being bound by theory, it is believed that delivering such nucleic acids to cells will lead to the expression of the CRISPR system within the cell. In an aspect, the invention provides a nucleic acid sequence that encodes one or more gRNA molecules of any of the previous gRNA aspects and embodiments. In embodiments, the nucleic acid includes a promoter operably linked to the sequence that encodes the one or more gRNA molecules. In embodiments, the promoter is a promoter recognized by an RNA polymerase II or RNA polymerase III. In embodiments, the promoter is a U6 promoter or an HI promoter.

In further aspects, the nucleic acid further comprises sequence encoding a Cas9 molecule. In embodiments, the nucleic acid includes a promoter operably linked to the sequence that encodes a Cas9 molecule. In embodiments, the promoter is an EF-1 promoter, a CMV IE gene promoter, an EF-1α promoter, an ubiquitin C promoter, or a phosphoglycerate kinase (PGK) promoter.

In an aspect the invention provides a vector that includes the nucleic acid of any of the previous nucleic acid aspects and embodiments. In embodiments, the vector is selected from the group consisting of a lentiviral vector, an adenoviral vector, an adeno-associated viral (AAV) vector, a herpes simplex virus (HSV) vector, a plasmid, a minicircle, a nanoplasmid, and an RNA vector.

In another aspect, the invention provides a composition including one or more gRNA molecules, e.g., as described herein, e.g., in any of the previous gRNA molecule aspects and embodiments, and nucleic acid encoding a Cas9 molecule.

In another aspect, the invention provides a composition including nucleic acid encoding one or more gRNA molecules (e.g., as described herein, e.g., of any of the previous gRNA molecule aspects and embodiments), and a Cas9 molecule.

In another aspect, the invention provides a composition, e.g., a composition of any of the aforementioned composition aspects and embodiments, further including a template nucleic acid. In another aspect, the invention provides a composition, e.g., a composition of any of the aforementioned composition aspects and embodiments, further including nucleic acid sequence encoding a template nucleic acid. In embodiments, the template nucleic acid includes a nucleotide that corresponds to a nucleotide of a target sequence of the gRNA molecule. In embodiments, the template nucleic acid includes nucleic acid encoding human beta globin, e.g., human beta globin including one or more of the mutations G16D, E22A and T87Q. In embodiments, the template nucleic acid includes nucleic acid encoding human gamma globin.

In another aspect, the invention provides cells that include (or at any time included) a gRNA molecule, CRISPR system, composition or nucleic acid (e.g., as described herein, e.g., as in any of the aforementioned aspects and embodiments). In such aspects, the invention provides cells that have been modified by such inclusion.

Thus, in an aspect, the invention provides a method of altering e.g., altering the structure, e.g., sequence, of a target sequence within nucleic acid of a cell, including contacting said cell with:
1) one or more gRNA molecules of any of the aforementioned gRNA molecule aspects and embodiments, and a Cas9 molecule (e.g., as described herein);
2) one or more gRNA molecules of any of the aforementioned gRNA molecule aspects and embodiments, and nucleic acid encoding a Cas9 molecule (e.g., as described herein);
3) nucleic acid encoding one or more gRNA molecules of any of the aforementioned gRNA molecule aspects and embodiments, and a Cas9 molecule (e.g., as described herein);
4) nucleic acid encoding one or more gRNA molecules of the aforementioned gRNA molecule aspects and embodiments, and nucleic acid encoding a Cas9 molecule (e.g., as described herein); or
5) any of 1) to 4), above, and a template nucleic acid (e.g., as described herein);
6) any of 1) to 4) above, and nucleic acid including sequence encoding a template nucleic acid (e.g., as described herein);
7) a composition as described herein (e.g., of any of the aforementioned composition aspects and embodiments); or
8) the vector of any of the aforementioned vector aspects and embodiments.

In embodiments, the gRNA molecule or nucleic acid encoding the gRNA molecule, and the Cas9 molecule or nucleic acid encoding the Cas9 molecule, are formulated in a single composition. In other embodiments, the gRNA molecule or nucleic acid encoding the gRNA molecule, and the Cas9 molecule or nucleic acid encoding the Cas9 molecule, are formulated in more than one composition. In embodiments, the more than one composition are delivered simultaneously or sequentially.

In embodiments, the cell is an animal cell. In embodiments, the cell the cell is a mammalian, primate, or human cell. In embodiments, the cell is a hempatopoietic stem or progenitor cell (HSPC) (e.g., a population of HSPCs). In embodiments, the cell is a CD34+ cell. In embodiments, the cell is a CD34+/CD38-/CD90+/CD45RA- cell. In embodiments, the cell is a CD34+/CD90+/CD49f+ cell. In embodiments, the cell is a CD34+/CD38-/CD90+/CD45RA-/CD49f+ cell. In embodiments, the method includes a population of cells that has been enriched for HSPCs, e.g., for CD34+ cells.

In embodiments, the cell (e.g. population of cells) has been isolated from bone marrow. In embodiments, the cell (e.g. population of cells) has been isolated from mobilized peripheral blood. In embodiments, the cell (e.g. population of cells) has been isolated from umbilical cord blood.

In embodiments, the cell (e.g. population of cells) is autologous with respect to a patient to be administered said cell (e.g. population of cells). In embodiments, the cell (e.g. population of cells) is allogeneic with respect to a patient to be administered said cell (e.g. population of cells).

In embodiments, of the methods described herein, the altering results in an increase of fetal hemoglobin expression in the cell. In embodiments, of the methods described herein, the altering results in a reduction of fetal hemoglobin expression in the cell.

In another aspect, the invention provides a cell, altered by the method of any of the aforementioned method aspects and embodiments. In another aspect, the invention provides a cell, including a first gRNA molecule (e.g., as described herein), or a composition (e.g., as described herein), or a nucleic acid encoding the first gRNA molecule (e.g., as described herein), of any of the previous aspects and embodiments. In embodiments, the cell is an animal cell. In embodiments, the cell the cell is a mammalian, primate, or human cell. In embodiments, the cell is a hematopoietic stem or progenitor cell (HSPC) (e.g., a population of HSPCs). In embodiments, the cell is a CD34+ cell. In embodiments, the cell is a CD34+/CD38-/CD90+/CD45RA- cell. In embodiments, the cell is a CD34+/CD90+/CD49f+ cell. In embodiments, the method includes a population of cells that has been enriched for HSPCs, e.g., for CD34+ cells.

In embodiments, the cell (e.g. population of cells) has been isolated from bone marrow. In embodiments, the cell (e.g. population of cells) has been isolated from mobilized peripheral blood. In embodiments, the cell (e.g. population of cells) has been isolated from umbilical cord blood.

In embodiments, the cell (e.g. population of cells) is autologous with respect to a patient to be administered said cell (e.g. population of cells). In embodiments, the cell (e.g. population of cells) is allogeneic with respect to a patient to be administered said cell (e.g. population of cells).

In embodiments, the cell includes, has included, or will include a second gRNA molecule, e.g., as described herein, e.g., of any of the previous gRNA molecule aspects and embodiments, or a nucleic acid encoding the second gRNA molecule, wherein the first gRNA molecule and second gRNA molecule include nonidentical targeting domains.

In embodiments, expression of fetal hemoglobin is increased in the cell, e.g., relative to a cell of the same cell type that has not been modified to include a gRNA molecule (e.g., as described herein). In embodiments, expression of beta globin is decreased in the cell, e.g., relative to a cell of the same cell type that has not been modified to include a gRNA molecule (e.g., as described herein). In embodiments, expression of fetal hemoglobin is increased and expression of beta globin is decreased in the cell, e.g., relative to a cell of the same cell type that has not been modified to include a gRNA molecule (e.g., as described herein).

In an aspect, the cells of the invention (or methods comprising a cell) have been contacted with a stem cell expander, e.g., as described herein, e.g., compound 1, compound 2, compound 3 or compound 4. In an aspect, the cells of the invention (or methods comprising a cell) have been contacted with a stem cell expander, e.g., as described herein, e.g., compound 1, compound 2, compound 3, compound 4 or a combination thereof (e.g., compound 1 and compound 4). In an embodiment, the stem cell expander is compound 4. In an embodiment, the stem cell expander is a combination of compound 1 and compound 4. In embodiments, the contacting is ex vivo.

In another aspect, the invention provides methods of treatment, e.g., methods of treatment of hemoglobinopathies. In an aspect, the invention provides a method of treating a hemoglobinopathy, that includes administering to a patient a cell (e.g., a population of cells) of any of the previous cell or method aspects and embodiments.

In another aspect, the invention provides a method of increasing fetal hemoglobin expression in a mammal, including administering to a patient a cell of any of the previous cell or method aspects and embodiments. In an embodiment, the hemoglobinopathy is beta-thalassemia or sickle cell disease.

In another aspect, the invention provides a guide RNA molecule, e.g., as described herein, for use as a medicament, e.g., for use in the treatment of a disease. In embodiments, the disease is a hemoglobinopathy, e.g., beta-thalassemia or sickle cell disease.

In another aspect, the invention provides a gRNA molecule, e.g., as described herein, e.g., as described in any of the aforementioned gRNA molecule aspects and embodiments, wherein when a CRISPR system including the gRNA is introduced into a cell (e.g., a CD34+ cell, e.g., an HSC), at least about 15% of the indels produced include (a) a frameshift mutation; or (b) a large deletion, relative to unmodified target DNA, as measured by NGS. In embodiments, at least about 25% of the indels produced include (a) a frameshift mutation; or (b) a large deletion, relative to unmodified target DNA, as measured by NGS. In embodiments, at least about 40%, at least about 50%, at least about 60%, at least about 70% at least about 80%, at least about 90%, at least about 95%, at least about 96%, at least about 97%, at least about 98%, or at least about 99% of the indels produced include (a) a frameshift mutation; or (b) a large deletion, relative to unmodified target DNA, as measured by NGS.

In another aspect, the invention provides methods for the ex vivo expansion and modification of HSPCs (e.g., CD34+ cells). In an aspect, the invention provides a method of modifying cells (e.g., a population of cells) including:
(a) providing a population of cells;
(b) expanding said cells ex vivo in the presence of a stem cell expander; and
(c) introducing into said cells a first gRNA molecule (e.g., as described herein, e.g., of any of the aforementioned gRNA molecule aspects and embodiments), a nucleic acid molecule encoding the first gRNA molecule, or a composition (e.g., as described herein, e.g., of any of the aforementioned composition aspects and embodiments);
such that hemoglobin, e.g., fetal hemoglobin, expression is increased in said cells relative to the same cell type which have not been subjected to step (c).

In embodiments, the cells are introduced into a subject in need thereof, e.g., a subject that has a hemoglobinopathy, e.g., sickle cell disease or beta thalassemia.

In embodiments, the cells are or comprise CD34+ cells. In embodiments, the cells are or comprise HSPCs. In embodiments, the cells are isolated from bone marrow, mobilized peripheral blood or umbilical cord blood. In a preferred embodiment, the cells are isolated from bone marrow. In other embodiments, the cells are isolated from mobilized peripheral blood. In aspects, the moblized peripheral blood is isolated from a subject who has been administered a G-CSF. In aspects, the moblized peripheral blood is isolated from a subject who has been administered a moblization agent other than G-CSF, for example, Plerixafor^{®} (AMD3100). In embodiments, the cells are an enriched population of cells.

In embodiments, the stem cell expander is compound 1, compound 2, compound 3, or compound 4, e.g., compound 4. In embodiments, the stem cell expander is compound 1, compound 2, compound 3, compound 4, or a combination thereof (e.g., a combination of compound 1 and compound 4). In embodiments, the cells are contacted with compound 4 at a concentration of from about 1 to about 200 micromolar (uM). In an embodiment, the concentration of compound 4 is about 75 micromolar (uM). In embodiments, the expanding said cells ex vivo in the presence of a stem cell expander occurs for a period of about 1-10 days, e.g., about 1-5 days, e.g., about 2-5 days, e.g., about 4 days.

In embodiments, the cells are autologous to a patient intended to be administered said cells. In embodiments, the cells are allogeneic to a patient intended to be administered said cells.

In embodiments, the expanding of step (b) is further in the presence of thrombopoietin (Tpo), Flt3 ligand (Flt-3L), human stem cell factor (SCF) and human interleukin-6 (IL-6). In embodiments, the thrombopoietin (Tpo), Flt3 ligand (Flt-3L), human stem cell factor (SCF) and human interleukin-6 (IL-6) are each at a concentration of about 50 ng/mL. In embodiments, the thrombopoietin (Tpo), Flt3 ligand (Flt-3L), human stem cell factor (SCF) and human interleukin-6 (IL-6) are each at a concentration of 50 ng/mL.

Additional aspects and embodiments are described below.

In an aspect, the invention provides a gRNA molecule that includes a tracr and crRNA, wherein the crRNA includes a targeting domain that is complementary with a target sequence of a BCL11A gene (e.g., a human BCL11a gene), a BCL11a enhancer (e.g., a human BCL11a enhancer), or a HFPH region (e.g., a human HPFH region).

In embodiments, the target sequence is of the BCL11A gene, and the targeting domain includes, e.g., consists of, any one of SEQ ID NO: 1 to SEQ ID NO: 85 or SEQ ID NO: 400 to SEQ ID NO: 1231. These embodiments are referred to herein as gRNA molecule embodiment 2.

In other embodiments, the target sequence is of a BCL11a enhancer, and the targeting domain includes, e.g., consists of, any one of SEQ ID NO: 1232 to SEQ ID NO: 1499. These embodiments are referred to herein as gRNA molecule embodiment 3. In preferred embodiments, the target sequence is of a BCL11a enhancer, and the targeting domain includes, e.g., consists of, any one of SEQ ID NO: 182 to SEQ ID NO: 277 or SEQ ID NO: 334 to SEQ ID NO: 341. These embodiments are referred to herein as gRNA molecule embodiment 4. In more preferred embodiments, the targeting domain includes, e.g., consists of, any one of SEQ ID NO: 341, SEQ ID NO: 246, SEQ ID NO: 248, SEQ ID NO: 247, SEQ ID NO: 245, SEQ ID NO: 249, SEQ ID NO: 244, SEQ ID NO: 199, SEQ ID NO: 251, SEQ ID NO: 250, SEQ ID NO: 334, SEQ ID NO: 185, SEQ ID NO: 186, SEQ ID NO: 336, or SEQ ID NO: 337. These embodiments are referred to herein as gRNA molecule embodiment 5. In still more preferred embodiments, the targeting domain includes, e.g., consists of, any one of SEQ ID NO: 247, SEQ ID NO: 248, SEQ ID NO: 335, SEQ ID NO: 336, SEQ ID NO: 337, or SEQ ID NO: 338 (referred to herein as gRNA molecule embodiment 6), for example, includes, e.g., consists of, any one of SEQ ID NO: 248 or SEQ ID NO: 338 (referred to herein as gRNA molecule embodiment 7). In embodiments, the targeting domain includes, e.g., consists of, SEQ ID NO: 248 (referred to herein as gRNA molecule embodiment 8). In embodiments, the targeting domain includes, e.g., consists of, SEQ ID NO: 338 (referred to herein as gRNA molecule embodiment 9).

In other embodiments, the target sequence is of a BCL11a enhancer, and the targeting domain includes, e.g., consists of, any one of SEQ ID NO: 278 to SEQ ID NO: 333 (referred to herein as gRNA molecule embodiment 10). In preferred embodiments, the targeting domain includes, e.g., consists of, any one of SEQ ID NO: 318, SEQ ID NO: 312, SEQ ID NO: 313, SEQ ID NO: 294, SEQ ID NO: 310, SEQ ID NO: 319, SEQ ID NO: 298, SEQ ID NO: 322, SEQ ID NO: 311, SEQ ID NO: 315, SEQ ID NO: 290, SEQ ID NO: 317, SEQ ID NO: 309, SEQ ID NO: 289, or SEQ ID NO: 281 (referred to herein as gRNA molecule embodiment 11). In one preferred embodiment, the targeting domain includes, e.g., consists of, SEQ ID NO: 318 (referred to herein as gRNA molecule embodiment 12).

In other embodiments, the target sequence is of a BCL11a enhancer, and the targeting domain includes, e.g., consists of, any one of SEQ ID NO: 1596 to SEQ ID NO: 1691 (referred to herein as gRNA molecule embodiment 13). In preferred embodiments, the targeting domain includes, e.g., consists of, any one of SEQ ID NO: 1683, SEQ ID NO: 1638, SEQ ID NO: 1647, SEQ ID NO: 1609, SEQ ID NO: 1621, SEQ ID NO: 1617, SEQ ID NO: 1654, SEQ ID NO: 1631, SEQ ID NO: 1620, SEQ ID NO: 1637, SEQ ID NO: 1612, SEQ ID NO: 1656, SEQ ID NO: 1619, SEQ ID NO: 1675, SEQ ID NO: 1645, SEQ ID NO: 1598, SEQ ID NO: 1599, SEQ ID NO: 1663, SEQ ID NO: 1677, or SEQ ID NO: 1626 (referred to herein as gRNA molecule embodiment 14).

In other embodiments, the target sequence is of a HFPH region (e.g., a French HPFH region), and the targeting domain includes, e.g., consists of, any one of SEQ ID NO: 86 to SEQ ID NO: 181, SEQ ID NO: 1500 to SEQ ID NO: 1595, or SEQ ID NO: 1692 to SEQ ID NO: 1761 (referred to herein as gRNA molecule embodiment 15). In preferred embodiments, the targeting domain includes, e.g., consists of, any one of SEQ ID NO: 100, SEQ ID NO: 165, SEQ ID NO: 113, SEQ ID NO: 99, SEQ ID NO: 112, SEQ ID NO: 98, SEQ ID NO: 1580, SEQ ID NO: 106, SEQ ID NO: 1503, SEQ ID NO: 1589, SEQ ID NO: 160, SEQ ID NO: 1537, SEQ ID NO: 159, SEQ ID NO: 101, SEQ ID NO: 162, SEQ ID NO: 104, SEQ ID NO: 138, SEQ ID NO: 1536, SEQ ID NO: 1539, SEQ ID NO: 1585 (referred to herein as gRNA molecule embodiment 16). In still more preferred embodiments, the targeting domain includes, e.g., consists of, any one of SEQ ID NO: 98, SEQ ID NO: 100, SEQ ID NO: 1505, SEQ ID NO: 1589, SEQ ID NO: 1700, or SEQ ID NO: 1750 (referred to herein as gRNA molecule embodiment 17). In other preferred embodiments, the targeting domain includes, e.g., consists of, any one of SEQ ID NO: 100, SEQ ID NO: 165, or SEQ ID NO: 113 (referred to herein as gRNA molecule embodiment 18).

In embodiments, the gRNA molecule includes a targeting domain that includes, e.g., consists of, 17, 18, 19, 20, 21, 22, 23, or 24, preferably 20, consecutive nucleic acids of any targeting domain sequence described herein, for example a targeting domain sequence of Table 1 or Table 2. In embodiments, the 17, 18, 19, 20, 21, 22, 23, or 24, preferably 20, consecutive nucleic acids of any one of targeting domain sequences described herein, for example a targeting domain sequence of Table 1 or Table 2, are the 17, 18, 19, 20, 21, 22, 23, or 24, preferably 20, consecutive nucleic acids disposed at the 3' end of the recited targeting domain sequence. In other embodiments, the 17, 18, 19, 20, 21, 22, 23, or 24, preferably 20, consecutive nucleic acids of any one of the targeting domain sequences described herein, for example a targeting domain sequence of Table 1 or Table 2, are the 17, 18, 19, 20, 21, 22, 23, or 24, preferably 20, consecutive nucleic acids disposed at the 5' end of the recited targeting domain sequence. In other embodiments, the 17, 18, 19, 20, 21, 22, 23, or 24, preferably 20, consecutive nucleic acids of any one of the targeting domain sequences described herein, for example a targeting domain sequence of Table 1 or Table 2, do not include either the 5' or 3' nucleic acid of the recited targeting domain sequence.

In embodiments, the gRNA molecule includes a targeting domain that includes, e.g., consists of, 17, 18, 19, or 20, preferably 20, consecutive nucleic acids of any targeting domain sequence described herein, for example a targeting domain sequence of Table 5, Table 6, Table 7, Table 8 or Table 9. In embodiments, the 17, 18, 19, or 20, preferably 20, consecutive nucleic acids of any one of targeting domain sequences described herein, for example a targeting domain sequence of Table 5, Table 6, Table 7, Table 8 or Table 9, are the 17, 18, 19, or 20, preferably 20, consecutive nucleic acids disposed at the 3' end of the recited targeting domain sequence. In other embodiments, the 17, 18, 19, or 20, preferably 20, consecutive nucleic acids of any one of the targeting domain sequences described herein, for example a targeting domain sequence of Table 5, Table 6, Table 7, Table 8 or Table 9, are the 17, 18, 19, or 20, preferably 20, consecutive nucleic acids disposed at the 5' end of the recited targeting domain sequence. In other embodiments, the 17, 18, 19, or 20, preferably 20, consecutive nucleic acids of any one of the targeting domain sequences described herein, for example a targeting domain sequence of Table 5, Table 6, Table 7, Table 8 or Table 9, do not include either the 5' or 3' nucleic acid of the recited targeting domain sequence.

The following aspects describe features of the gRNA molecule that may be combined with any of the aforementioned aspects and embodiments. In embodiments, the gRNA molecule, including the gRNA molecule of any of the aforementioned aspects and embodiments, include a portion of the crRNA and a portion of the tracr that hybridize to form a flagpole that includes SEQ ID NO: 6584 or 6585. In embodiments, the flagpole further includes a first flagpole extension, located 3' to the crRNA portion of the flagpole, wherein said first flagpole extension includes SEQ ID NO: 6586. In embodiments, the flagpole further includes (in addition to or in alternative to the first flagpole extension) a second flagpole extension located 3' to the crRNA portion of the flagpole and, if present, the first flagpole extension, wherein said second flagpole extension includes SEQ ID NO: 6587.

In embodiments, including in any of the aforementioned aspects and embodiments, the tracr includes SEQ ID NO: 6660 or SEQ ID NO: 6661. In embodiments, the tracr includes SEQ ID NO: 7812, optionally further including, at the 3' end, an additional 1, 2, 3, 4, 5, 6, or 7 uracil (U) nucleotides. In embodiments, the crRNA includes, from 5' to 3', [targeting domain]-: a) SEQ ID NO: 6584; b) SEQ ID NO: 6585; c) SEQ ID NO: 6605; d) SEQ ID NO: 6606; e) SEQ ID NO: 6607; f) SEQ ID NO: 6608; or g) SEQ ID NO: 7806.

In embodiments, including in any of the aforementioned aspects and embodiments, the tracr includes, from 5' to 3': a) SEQ ID NO: 6589; b) SEQ ID NO: 6590; c) SEQ ID NO: 6609; d) SEQ ID NO: 6610; e) SEQ ID NO: 6660; f) SEQ ID NO: 6661; g) SEQ ID NO: 7812; h) SEQ ID NO: 7807; i) SEQ ID NO: 7808; j) SEQ ID NO: 7809; k) any of a) to j), above, further including, at the 3' end, at least 1, 2, 3, 4, 5, 6 or 7 uracil (U) nucleotides, e.g., 1, 2, 3, 4, 5, 6, or 7 uracil (U) nucleotides; 1) any of a) to k), above, further including, at the 3' end, at least 1, 2, 3, 4, 5, 6 or 7 adenine (A) nucleotides, e.g., 1, 2, 3, 4, 5, 6, or 7 adenine (A) nucleotides; or m) any of a) to 1), above, further including, at the 5' end (e.g., at the 5' terminus), at least 1, 2, 3, 4, 5, 6 or 7 adenine (A) nucleotides, e.g., 1, 2, 3, 4, 5, 6, or 7 adenine (A) nucleotides.

In embodiments, the targeting domain and the tracr are disposed on separate nucleic acids molecules. In embodiments of such gRNA molecules, the nucleic acid molecule including the targeting domain includes SEQ ID NO: 6607, optionally disposed immediately 3' to the targeting domain, and the nucleic acid molecule including the tracr includes, e.g., consists of, SEQ ID NO: 6660.

In embodiments, the crRNA portion of the flagpole includes SEQ ID NO: 6607 or SEQ ID NO: 6608.

In embodiments, the tracr includes SEQ ID NO: 6589 or 6590, and optionally, if a first flagpole extension is present, a first tracr extension, disposed 5' to SEQ ID NO: 6589 or 6590, said first tracr extension including SEQ ID NO: 6591.

In embodiments, including in embodiments of any of the aforementioned aspects and embodiments, the targeting domain and the tracr are disposed on separate nucleic acid molecules. In other embodiments, including in embodiments of any of the aforementioned aspects and embodiments, the targeting domain and the tracr are disposed on a single nucleic acid molecule, for example, the tracr is disposed 3' to the targeting domain.

In embodiments, when the targeting domain and the tracr are disposed on a single nucleic acid molecule, the gRNA molecule further includes a loop, disposed 3' to the targeting domain and 5' to the tracr. In embodiments, the loop includes, e.g., consists of, SEQ ID NO: 6588.

In embodiments, when the targeting domain and the tracr are disposed on a single nucleic acid molecule, the gRNA molecule includes, from 5' to 3', [targeting domain]-: (a) SEQ ID NO: 6601; (b) SEQ ID NO: 6602; (c) SEQ ID NO: 6603; (d) SEQ ID NO: 6604; (e) SEQ ID NO: 7811; or (f) any of (a) to (e), above, further including, at the 3' end, 1, 2, 3, 4, 5, 6 or 7 uracil (U) nucleotides. In embodiments, the gRNA molecule includes, e.g., consists of, said targeting domain and SEQ ID NO: 7811, optionally disposed immediately 3' to said targeting domain.

In embodiments, including in any of the aforementioned aspects and embodiments, each of the nucleic acid residues of the gRNA molecule is an unmodified A, U, G or C nucleic acid residue and unmodified phosphate bonds between each residue of the nucleic acid molecule(s). In other embodiments, including in any of the aforementioned aspects and embodiments, one, or optionally more than one, of the nucleic acid molecules that make up the gRNA molecule includes: a) one or more, e.g., three, phosphorothioate modifications at the 3' end of said nucleic acid molecule or molecules; b) one or more, e.g., three, phosphorothioate modifications at the 5' end of said nucleic acid molecule or molecules; c) one or more, e.g., three, 2'-O-methyl modifications at the 3' end of said nucleic acid molecule or molecules; d) one or more, e.g., three, 2'-O-methyl modifications at the 5' end of said nucleic acid molecule or molecules; e) a 2' O-methyl modification at each of the 4^{th}-to-terminal, 3^{rd}-to-terminal, and 2^{nd}-to-terminal 3' residues of said nucleic acid molecule or molecules; f) a 2' O-methyl modification at each of the 4^{th}-to-terminal, 3^{rd}-to-terminal, and 2^{nd}-to-terminal 5' residues of said nucleic acid molecule or molecules; or f) any combination thereof.

In preferred embodiments, the invention provides a gRNA molecule (e.g., that is a sgRNA molecule) including, e.g., consisting of, the sequence: (a) SEQ ID NO: 342; (b) SEQ ID NO: 343; or (c) SEQ ID NO: 1762 (referred to in this summary of invention as gRNA molecule embodiment 41).

In other preferred embodiments, the invention provides gRNA molecule (e.g., that is a dual gRNA molecule) including, e.g., consisting of: (a) a crRNA including, e.g., consisting of, SEQ ID NO: 344, and a tracr including, e.g., consisting of, SEQ ID NO: 6660; (b) a crRNA including, e.g., consisting of, SEQ ID NO: 344, and a tracr including, e.g., consisting of, SEQ ID NO: 346; (c) a crRNA including, e.g., consisting of, SEQ ID NO: 345, and a tracr including, e.g., consisting of, SEQ ID NO: 6660; or (d) a crRNA including, e.g., consisting of, SEQ ID NO: 345, and a tracr including, e.g., consisting of, SEQ ID NO: 346 (referred to in this summary of invention as gRNA molecule embodiment 42).

In other preferred embodiments, the invention provides a gRNA molecule (e.g., that is a sgRNA molecule) including, e.g., consisting of, the sequence: (a) SEQ ID NO: 347; (b) SEQ ID NO: 348; or (c) SEQ ID NO: 1763 (referred to in this summary of invention as gRNA molecule embodiment 43).

In other preferred embodiments, the invention provides gRNA molecule (e.g., that is a dual gRNA molecule) including, e.g., consisting of: (a) a crRNA including, e.g., consisting of, SEQ ID NO: 349, and a tracr including, e.g., consisting of, SEQ ID NO: 6660; (b) a crRNA including, e.g., consisting of, SEQ ID NO: 349, and a tracr including, e.g., consisting of, SEQ ID NO: 346; (c) a crRNA including, e.g., consisting of, SEQ ID NO: 350, and a tracr including, e.g., consisting of, SEQ ID NO: 6660; or (d) a crRNA including, e.g., consisting of, SEQ ID NO: 350, and a tracr including, e.g., consisting of, SEQ ID NO: 346 (referred to in this summary of invention as gRNA molecule embodiment 44).

In other preferred embodiments, the invention provides a gRNA molecule (e.g., that is a sgRNA molecule) including, e.g., consisting of, the sequence: (a) SEQ ID NO: 351; (b) SEQ ID NO: 352; or (c) SEQ ID NO: 1764 (referred to in this summary of invention as gRNA molecule embodiment 45).

In other preferred embodiments, the invention provides gRNA molecule (e.g., that is a dual gRNA molecule) including, e.g., consisting of: (a) a crRNA including, e.g., consisting of, SEQ ID NO: 353, and a tracr including, e.g., consisting of, SEQ ID NO: 6660; (b) a crRNA including, e.g., consisting of, SEQ ID NO: 353, and a tracr including, e.g., consisting of, SEQ ID NO: 346; (c) a crRNA including, e.g., consisting of, SEQ ID NO: 354, and a tracr including, e.g., consisting of, SEQ ID NO: 6660; or (d) a crRNA including, e.g., consisting of, SEQ ID NO: 354, and a tracr including, e.g., consisting of, SEQ ID NO: 346 (referred to in this summary of invention as gRNA molecule embodiment 46).

In other preferred embodiments, the invention provides a gRNA molecule (e.g., that is a sgRNA molecule) including, e.g., consisting of, the sequence: (a) SEQ ID NO: 355; (b) SEQ ID NO: 356; or (c) SEQ ID NO: 1765 (referred to in this summary of invention as gRNA molecule embodiment 47).

In other preferred embodiments, the invention provides gRNA molecule (e.g., that is a dual gRNA molecule) including, e.g., consisting of: (a) a crRNA including, e.g., consisting of, SEQ ID NO: 357, and a tracr including, e.g., consisting of, SEQ ID NO: 6660; (b) a crRNA including, e.g., consisting of, SEQ ID NO: 357, and a tracr including, e.g., consisting of, SEQ ID NO: 346; (c) a crRNA including, e.g., consisting of, SEQ ID NO: 358, and a tracr including, e.g., consisting of, SEQ ID NO: 6660; or (d) a crRNA including, e.g., consisting of, SEQ ID NO: 358, and a tracr including, e.g., consisting of, SEQ ID NO: 346 (referred to in this summary of invention as gRNA molecule embodiment 48).

In other preferred embodiments, the invention provides a gRNA molecule (e.g., that is a sgRNA molecule) including, e.g., consisting of, the sequence: (a) SEQ ID NO: 359; (b) SEQ ID NO: 360; or (c) SEQ ID NO: 1766 (referred to in this summary of invention as gRNA molecule embodiment 49).

In other preferred embodiments, the invention provides gRNA molecule (e.g., that is a dual gRNA molecule) including, e.g., consisting of: (a) a crRNA including, e.g., consisting of, SEQ ID NO: 361, and a tracr including, e.g., consisting of, SEQ ID NO: 6660; (b) a crRNA including, e.g., consisting of, SEQ ID NO: 361, and a tracr including, e.g., consisting of, SEQ ID NO: 346; (c) a crRNA including, e.g., consisting of, SEQ ID NO: 362, and a tracr including, e.g., consisting of, SEQ ID NO: 6660; or (d) a crRNA including, e.g., consisting of, SEQ ID NO: 362, and a tracr including, e.g., consisting of, SEQ ID NO: 346 (referred to in this summary of invention as gRNA molecule embodiment 50).

In other preferred embodiments, the invention provides a gRNA molecule (e.g., that is a sgRNA molecule) including, e.g., consisting of, the sequence: (a) SEQ ID NO: 363; (b) SEQ ID NO: 364; or (c) SEQ ID NO: 1767 (referred to in this summary of invention as gRNA molecule embodiment 51).

In other preferred embodiments, the invention provides gRNA molecule (e.g., that is a dual gRNA molecule) including, e.g., consisting of: (a) a crRNA including, e.g., consisting of, SEQ ID NO: 365, and a tracr including, e.g., consisting of, SEQ ID NO: 6660; (b) a crRNA including, e.g., consisting of, SEQ ID NO: 365, and a tracr including, e.g., consisting of, SEQ ID NO: 346; (c) a crRNA including, e.g., consisting of, SEQ ID NO: 366, and a tracr including, e.g., consisting of, SEQ ID NO: 6660; or (d) a crRNA including, e.g., consisting of, SEQ ID NO: 366, and a tracr including, e.g., consisting of, SEQ ID NO: 346 (referred to in this summary of invention as gRNA molecule embodiment 52).

In other preferred embodiments, the invention provides a gRNA molecule (e.g., that is a sgRNA molecule) including, e.g., consisting of, the sequence: (a) SEQ ID NO: 367; (b) SEQ ID NO: 368; or (c) SEQ ID NO: 1768 (referred to in this summary of invention as gRNA molecule embodiment 53).

In other preferred embodiments, the invention provides gRNA molecule (e.g., that is a dual gRNA molecule) including, e.g., consisting of: (a) a crRNA including, e.g., consisting of, SEQ ID NO: 369, and a tracr including, e.g., consisting of, SEQ ID NO: 6660; (b) a crRNA including, e.g., consisting of, SEQ ID NO: 369, and a tracr including, e.g., consisting of, SEQ ID NO: 346; (c) a crRNA including, e.g., consisting of, SEQ ID NO: 370, and a tracr including, e.g., consisting of, SEQ ID NO: 6660; or (d) a crRNA including, e.g., consisting of, SEQ ID NO: 370, and a tracr including, e.g., consisting of, SEQ ID NO: 346 (referred to in this summary of invention as gRNA molecule embodiment 54).

In other preferred embodiments, the invention provides a gRNA molecule (e.g., that is a sgRNA molecule) including, e.g., consisting of, the sequence: (a) SEQ ID NO: 371; (b) SEQ ID NO: 372; or (c) SEQ ID NO: 1769 (referred to in this summary of invention as gRNA molecule embodiment 55).

In other preferred embodiments, the invention provides gRNA molecule (e.g., that is a dual gRNA molecule) including, e.g., consisting of: (a) a crRNA including, e.g., consisting of, SEQ ID NO: 373, and a tracr including, e.g., consisting of, SEQ ID NO: 6660; (b) a crRNA including, e.g., consisting of, SEQ ID NO: 373, and a tracr including, e.g., consisting of, SEQ ID NO: 346; (c) a crRNA including, e.g., consisting of, SEQ ID NO: 374, and a tracr including, e.g., consisting of, SEQ ID NO: 6660; or (d) a crRNA including, e.g., consisting of, SEQ ID NO: 374, and a tracr including, e.g., consisting of, SEQ ID NO: 346 (referred to in this summary of invention as gRNA molecule embodiment 56).

In other preferred embodiments, the invention provides a gRNA molecule (e.g., that is a sgRNA molecule) including, e.g., consisting of, the sequence: (a) SEQ ID NO: 375; (b) SEQ ID NO: 376; or (c) SEQ ID NO: 1770 (referred to in this summary of invention as gRNA molecule embodiment 57).

In other preferred embodiments, the invention provides gRNA molecule (e.g., that is a dual gRNA molecule) including, e.g., consisting of: (a) a crRNA including, e.g., consisting of, SEQ ID NO: 377, and a tracr including, e.g., consisting of, SEQ ID NO: 6660; (b) a crRNA including, e.g., consisting of, SEQ ID NO: 377, and a tracr including, e.g., consisting of, SEQ ID NO: 346; (c) a crRNA including, e.g., consisting of, SEQ ID NO: 378, and a tracr including, e.g., consisting of, SEQ ID NO: 6660; or (d) a crRNA including, e.g., consisting of, SEQ ID NO: 378, and a tracr including, e.g., consisting of, SEQ ID NO: 346 (referred to in this summary of invention as gRNA molecule embodiment 58).

In other preferred embodiments, the invention provides a gRNA molecule (e.g., that is a sgRNA molecule) including, e.g., consisting of, the sequence: (a) SEQ ID NO: 379; (b) SEQ ID NO: 380; or (c) SEQ ID NO: 1771 (referred to in this summary of invention as gRNA molecule embodiment 59).

In other preferred embodiments, the invention provides gRNA molecule (e.g., that is a dual gRNA molecule) including, e.g., consisting of: (a) a crRNA including, e.g., consisting of, SEQ ID NO: 381, and a tracr including, e.g., consisting of, SEQ ID NO: 6660; (b) a crRNA including, e.g., consisting of, SEQ ID NO: 381, and a tracr including, e.g., consisting of, SEQ ID NO: 346; (c) a crRNA including, e.g., consisting of, SEQ ID NO: 382, and a tracr including, e.g., consisting of, SEQ ID NO: 6660; or (d) a crRNA including, e.g., consisting of, SEQ ID NO: 382, and a tracr including, e.g., consisting of, SEQ ID NO: 346 (referred to in this summary of invention as gRNA molecule embodiment 60).

In other preferred embodiments, the invention provides a gRNA molecule (e.g., that is a sgRNA molecule) including, e.g., consisting of, the sequence: (a) SEQ ID NO: 383; (b) SEQ ID NO: 384; or (c) SEQ ID NO: 1772 (referred to in this summary of invention as gRNA molecule embodiment 61).

In other preferred embodiments, the invention provides gRNA molecule (e.g., that is a dual gRNA molecule) including, e.g., consisting of: (a) a crRNA including, e.g., consisting of, SEQ ID NO: 385, and a tracr including, e.g., consisting of, SEQ ID NO: 6660; (b) a crRNA including, e.g., consisting of, SEQ ID NO: 385, and a tracr including, e.g., consisting of, SEQ ID NO: 346; (c) a crRNA including, e.g., consisting of, SEQ ID NO: 386, and a tracr including, e.g., consisting of, SEQ ID NO: 6660; or (d) a crRNA including, e.g., consisting of, SEQ ID NO: 386, and a tracr including, e.g., consisting of, SEQ ID NO: 346 (referred to in this summary of invention as gRNA molecule embodiment 62).

In other preferred embodiments, the invention provides a gRNA molecule (e.g., that is a sgRNA molecule) including, e.g., consisting of, the sequence: (a) SEQ ID NO: 387; (b) SEQ ID NO: 388; or (c) SEQ ID NO: 1773 (referred to in this summary of invention as gRNA molecule embodiment 63).

In other preferred embodiments, the invention provides gRNA molecule (e.g., that is a dual gRNA molecule) including, e.g., consisting of: (a) a crRNA including, e.g., consisting of, SEQ ID NO: 389, and a tracr including, e.g., consisting of, SEQ ID NO: 6660; (b) a crRNA including, e.g., consisting of, SEQ ID NO: 389, and a tracr including, e.g., consisting of, SEQ ID NO: 346; (c) a crRNA including, e.g., consisting of, SEQ ID NO: 390, and a tracr including, e.g., consisting of, SEQ ID NO: 6660; or (d) a crRNA including, e.g., consisting of, SEQ ID NO: 390, and a tracr including, e.g., consisting of, SEQ ID NO: 346 (referred to in this summary of invention as gRNA molecule embodiment 64).

In other preferred embodiments, the invention provides a gRNA molecule (e.g., that is a sgRNA molecule) including, e.g., consisting of, the sequence: (a) SEQ ID NO: 391; (b) SEQ ID NO: 392; or (c) SEQ ID NO: 1774 (referred to in this summary of invention as gRNA molecule embodiment 65).

In other preferred embodiments, the invention provides gRNA molecule (e.g., that is a dual gRNA molecule) including, e.g., consisting of: (a) a crRNA including, e.g., consisting of, SEQ ID NO: 393, and a tracr including, e.g., consisting of, SEQ ID NO: 6660; (b) a crRNA including, e.g., consisting of, SEQ ID NO: 393, and a tracr including, e.g., consisting of, SEQ ID NO: 346; (c) a crRNA including, e.g., consisting of, SEQ ID NO: 394, and a tracr including, e.g., consisting of, SEQ ID NO: 6660; or (d) a crRNA including, e.g., consisting of, SEQ ID NO: 394, and a tracr including, e.g., consisting of, SEQ ID NO: 346 (referred to in this summary of invention as gRNA molecule embodiment 66).

In other preferred embodiments, the invention provides a gRNA molecule (e.g., that is a sgRNA molecule) including, e.g., consisting of, the sequence: (a) SEQ ID NO: 395; (b) SEQ ID NO: 396; or (c) SEQ ID NO: 1775 (referred to in this summary of invention as gRNA molecule embodiment 67).

In other preferred embodiments, the invention provides gRNA molecule (e.g., that is a dual gRNA molecule) including, e.g., consisting of: (a) a crRNA including, e.g., consisting of, SEQ ID NO: 397, and a tracr including, e.g., consisting of, SEQ ID NO: 6660; (b) a crRNA including, e.g., consisting of, SEQ ID NO: 397, and a tracr including, e.g., consisting of, SEQ ID NO: 346; (c) a crRNA including, e.g., consisting of, SEQ ID NO: 398, and a tracr including, e.g., consisting of, SEQ ID NO: 6660; or (d) a crRNA including, e.g., consisting of, SEQ ID NO: 398, and a tracr including, e.g., consisting of, SEQ ID NO: 346 (referred to in this summary of invention as gRNA molecule embodiment 68).

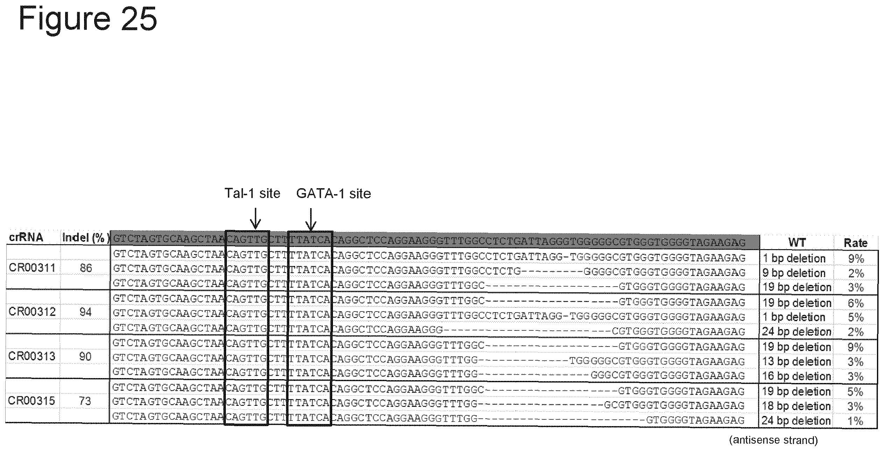

In embodiments, including in any of the aforementioned aspects and embodiments, the invention provides a gRNA molecule, wherein when a CRISPR system (e.g., an RNP as described herein) including the gRNA molecule is introduced into a cell, an indel is formed at or near the target sequence complementary to the targeting domain of the gRNA molecule. In embodiments, including in embodiments comprising a targeting domain complementary to a target sequence of a +58 Enhancer region, the indel does not include a nucleotide of a GATA-1 and/or TAL-1 binding site. In embodiments, the indel does not interfere with the binding of GATA-1 and/or TAL-1 to their binding sites. In embodiments, including in any of the aforementioned aspects and embodiments, the invention provides a gRNA molecule, wherein when a CRISPR system (e.g., an RNP as described herein) including the gRNA molecule is introduced into a population of cells, an indel is formed at or near the target sequence complementary to the targeting domain of the gRNA molecule in at least about 40%, e.g., at least about 50%, e.g., at least about 60%, e.g., at least about 70%, e.g., at least about 80%, e.g., at least about 90%, e.g., at least about 95%, e.g., at least about 96%, e.g., at least about 97%, e.g., at least about 98%, e.g., at least about 99%, of the cells of the population. In embodiments, including in any of the aforementioned aspects and embodiments, the invention provides a gRNA molecule, wherein when a CRISPR system (e.g., an RNP as described herein) including the gRNA molecule is introduced into a population of cells, an indel that does not include a nucleotide of a GATA-1 and/or TAL-1 binding site is formed at or near the target sequence complementary to the targeting domain of the gRNA molecule in at least about 20%, e.g., at least about 30%, e.g., at least about 35%, e.g., at least about 40%, e.g., at least about 45%, e.g., at least about 50%, e.g., at least about 55%, e.g., at least about 60%, e.g., at least about 65%, e.g., at least about 70%, e.g., at least about 75%, e.g., at least about 80%, e.g., at least about 85%, e.g., at least about 90%, e.g., at least about 95%, e.g., at least about 99%, of the cells of the population. In embodiments, including in any of the aforementioned aspects and embodiments, the indel is an indel listed in any of Figure 25, Table 15, Table 26, Table 27 or Table 37. In embodiments, in at least about 30%, e.g., least about 40%, e.g., at least about 50%, e.g., at least about 60%, e.g., at least about 70%, e.g., at least about 80%, e.g., at least about 90%, e.g., at least about 95%, e.g., at least about 96%, e.g., at least about 97%, e.g., at least about 98%, e.g., at least about 99%, of the cells of the population, the indel is an indel listed in any of Figure 25, Table 15, Table 26, Table 27 or Table 37. In embodiments, the three most frequently detected indels in said population of cells include the indels associated with any gRNA molecule listed in any of Figure 25, Table 15, Table 26, Table 27 or Table 37. In embodiments, the indel (or pattern of top indels) is as measured by next generation sequencing (NGS), e.g., as described in the art. In embodiments, including in any of the aforementioned aspects and embodiments, the invention provides a gRNA molecule, wherein when a CRISPR system (e.g., an RNP as described herein) including the gRNA molecule is introduced into a cell, no off-target indels are formed in said cell, e.g., as detectible by next generation sequencing and/or a nucleotide insertional assay, e.g., assayed in an HPCS cell, e.g., a CD34+ cell. In embodiments, including in any of the aforementioned aspects and embodiments, the invention provides a gRNA molecule, wherein when a CRISPR system (e.g., an RNP as described herein) including the gRNA molecule is introduced into a population of cells, no off-target indel is detected in more than about 5%, e.g., more than about 1%, e.g., more than about 0.1%, e.g., more than about 0.01%, of the cells of the population of cells, e.g., as detectible by next generation sequencing and/or a nucleotide insertional assay, e.g., assayed in a population of HPSC cells, e.g., a population of CD34+ cells.

In embodiments, including in any of the aforementioned aspects and embodiments, the invention provides a gRNA molecule, wherein when a CRISPR system (e.g., an RNP as described herein) including the gRNA molecule is introduced into a cell, expression of fetal hemoglobin is increased in said cell or its progeny, e.g., its erythroid progeny, e.g., its red blood cell progeny. In embodiments, expression of fetal hemoglobin is increased in said cell or its progeny, e.g., its erythroid progeny, e.g., its red blood cell progeny, by at least about 20%, e.g., at least about 30%, e.g., at least about 40%, e.g., at least about 50%, e.g., at least about 60%, e.g., at least about 70%, e.g., at least about 80%, e.g., at least about 90%, e.g., at least about 95%, e.g., at least about 96%, e.g., at least about 97%, e.g., at least about 98%, e.g., at least about 99%. In embodiments, said cell or its progeny, e.g., its erythroid progeny, e.g., its red blood cell progeny, produces at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin per cell. In embodiments, including in any of the aforementioned aspects and embodiments, the invention provides a gRNA molecule, wherein when a CRISPR system (e.g., an RNP as described herein) including the gRNA molecule is introduced into a cell, levels of fetal hemoglobin (e.g., gamma globin) mRNA are increased in said cell or its progeny, e.g., its erythroid progeny, e.g., its red blood cell progeny. In embodiments, including in any of the aforementioned aspects and embodiments, the invention provides a gRNA molecule, wherein when a CRISPR system (e.g., an RNP as described herein) including the gRNA molecule is introduced into a cell, expression of BCL11a mRNA is decreased in said cell or its progeny, e.g., its erythroid progeny, e.g., its red blood cell progeny. In embodiments, including in any of the aforementioned aspects and embodiments, the invention provides a gRNA molecule, wherein when a CRISPR system (e.g., an RNP as described herein) including the gRNA molecule is introduced into a cell, levels of BCL11a mRNA are decreased in said cell or its progeny, e.g., its erythroid progeny, e.g., its red blood cell progeny.

In any of the aforementioned aspects and embodiments that mention a cell, the cell is (or population of cells includes) a mammalian, primate, or human cell, e.g., is a human cell or population of human cells. In embodiments, the cell is (or population of cells includes) an HSPC, for example, is CD34+, for example, is CD34+CD90+. In embodiments, the cell (or population of cells) is autologous with respect to a patient to be administered said cell. In other embodiments, the cell (or population of cells) is allogeneic with respect to a patient to be administered said cell.

In another aspect, the invention provides a composition including: 1) one or more gRNA molecules (including a first gRNA molecule) described herein, for example, one or more gRNA molecules of any of the previous aspects and embodiments, and a Cas9 molecule, for example, as described herein; 2) one or more gRNA molecules (including a first gRNA molecule) described herein, for example, one or more gRNA molecules of any of the previous aspects and embodiments, and nucleic acid encoding a Cas9 molecule (described herein); 3) nucleic acid encoding one or more gRNA molecules (including a first gRNA molecule) described herein, for example, one or more gRNA molecules of any of the previous aspects and embodiments, and a Cas9 molecule (described herein); 4) nucleic acid encoding one or more gRNA molecules (including a first gRNA molecule) described herein, for example, one or more gRNA molecules of any of the previous aspects and embodiments, and nucleic acid encoding a Cas9 molecule (described herein); or 5) any of 1) to 4), above, and a template nucleic acid; or 6) any of 1) to 4) above, and nucleic acid including sequence encoding a template nucleic acid.

In preferred embodiments, the invention provides a composition including a first gRNA molecule (e.g., described herein, e.g., a first gRNA molecule of any of the previous gRNA molecule aspects and embodiments), further including a Cas9 molecule (described herein).

In embodiments, the Cas9 molecule is an active or inactive *s*. *pyogenes* Cas9. In embodiments, the Cas9 molecule includes SEQ ID NO: 6611. In embodiments, the Cas9 molecule includes, e.g., consists of: (a) SEQ ID NO: 7821; (b) SEQ ID NO: 7822; (c) SEQ ID NO: 7823; (d) SEQ ID NO: 7824; (e) SEQ ID NO: 7825; (f) SEQ ID NO: 7826; (g) SEQ ID NO: 7827; (h) SEQ ID NO: 7828; (i) SEQ ID NO: 7829; (j) SEQ ID NO: 7830; or (k) SEQ ID NO: 7831.

In preferred embodiments, the first gRNA molecule and Cas9 molecule are present in a ribonuclear protein complex (RNP).

In embodiments, the invention provides a composition, e.g., a composition of any of the previous aspects and embodiments, further including a second gRNA molecule; a second gRNA molecule and a third gRNA molecule; or a second gRNA molecule, optionally, a third gRNA molecule, and, optionally, a fourth gRNA molecule, wherein the second gRNA molecule, the optional third gRNA molecule, and the optional fourth gRNA molecule are a gRNA molecule of a gRNA molecule described herein, e.g., a gRNA molecule of any of the previous gRNA molecule aspects and embodiments, and wherein each gRNA molecule of the composition is complementary to a different target sequence.

In embodiments, two or more of the first gRNA molecule, the second gRNA molecule, the optional third gRNA molecule, and the optional fourth gRNA molecule are complementary to target sequences within the same gene or region. In embodiments, the first gRNA molecule, the second gRNA molecule, the optional third gRNA molecule, and the optional fourth gRNA molecule are complementary to target sequences not more than 20000 nucleotides, not more than 10000 nucleotides, not more than 6000, not more than 5000 nucleotides, not more than 4000, not more than 1000 nucleotides, not more than 500 nucleotides, not more than 400 nucleotides, not more than 300 nucleotides, not more than 200 nucleotides, not more than 100 nucleotides, not more than 90 nucleotides, not more than 80 nucleotides, not more than 70 nucleotides, not more than 60 nucleotides, not more than 50 nucleotides, not more than 40 nucleotides, not more than 30 nucleotides, not more than 20 nucleotides or not more than 10 nucleotides apart.

In other embodiments, two or more of the first gRNA molecule, the second gRNA molecule, the optional third gRNA molecule, and the optional fourth gRNA molecule are complementary to target sequence within different genes or regions.

In embodiments, including in any of the aforementioned composition aspects and embodiments, the invention provides a composition including a first gRNA molecule and a second gRNA molecule, wherein the first gRNA molecule and second gRNA molecule are:
(a) independently selected from the gRNA molecules of gRNA molecule embodiment 4, and are complementary to different target sequences;
(b) independently selected from the gRNA molecules of gRNA molecule embodiment 5, and are complementary to different target sequences;
c) independently selected from the gRNA molecules of gRNA molecule embodiment 6, and are complementary to different target sequences; or
(d) independently selected from the gRNA molecules of gRNA molecule embodiment 7, and are complementary to different target sequences; or
(e) independently selected from the gRNA molecules of any of gRNA molecule embodiments 41-56, and are complementary to different target sequences.

In embodiments, including in any of the aforementioned composition aspects and embodiments, the invention provides a composition including a first gRNA molecule and a second gRNA molecule, wherein the first gRNA molecule and second gRNA molecule are:
(a) independently selected from the gRNA molecules of gRNA molecule embodiment 10, and are complementary to different target sequences; or
(b) independently selected from the gRNA molecules of gRNA molecule embodiment 11, and are complementary to different target sequences.

In embodiments, including in any of the aforementioned composition aspects and embodiments, the invention provides a composition, including a first gRNA molecule and a second gRNA molecule, wherein the first gRNA molecule and second gRNA molecule are:
(a) independently selected from the gRNA molecules of gRNA molecule embodiment 13, and are complementary to different target sequences; or
(b) independently selected from the gRNA molecules of gRNA molecule embodiment 14, and are complementary to different target sequences.

In embodiments, including in any of the aforementioned composition aspects and embodiments, the invention provides a composition, including a first gRNA molecule and a second gRNA molecule, wherein the first gRNA molecule and second gRNA molecule are:
(a) independently selected from the gRNA molecules of gRNA molecule embodiment 16, and are complementary to different target sequences;
(b) independently selected from the gRNA molecules of gRNA molecule embodiment 17, and are complementary to different target sequences;
(c) independently selected from the gRNA molecules of gRNA molecule embodiment 18, and are complementary to different target sequences; or
(d) independently selected from the gRNA molecules of any of gRNA molecule embodiments 57-68, and are complementary to different target sequences.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 4, (b) selected from the gRNA molecules of gRNA molecule embodiment 5, (c) selected from the gRNA molecules of gRNA molecule embodiment 6, (d) selected from the gRNA molecules of gRNA molecule embodiment 7, or (e) selected from the gRNA molecules of any of gRNA molecule embodiments 41-56; and
(2) the second gRNA molecule is: (a) selected from the gRNA molecules of gRNA molecule embodiment 10, or (b) selected from the gRNA molecules of gRNA molecule embodiment 11.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 4, (b) selected from the gRNA molecules of gRNA molecule embodiment 5, (c) selected from the gRNA molecules of gRNA molecule embodiment 6, (d) selected from the gRNA molecules of gRNA molecule embodiment 7, or (e) selected from the gRNA molecules of any of gRNA molecule embodiments 41-56; and
(2) the second gRNA molecule is: (a) selected from the gRNA molecules of gRNA molecule embodiment 13, (b) selected from the gRNA molecules of gRNA molecule embodiment 14.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 4, (b) selected from the gRNA molecules of gRNA molecule embodiment 5, (c) selected from the gRNA molecules of gRNA molecule embodiment 6, (d) selected from the gRNA molecules of gRNA molecule embodiment 7, or (e) selected from the gRNA molecules of any of gRNA molecule embodiments 41-56; and
(2) the second gRNA molecule is: (a) selected from the gRNA molecules of gRNA molecule embodiment 16, (b) selected from the gRNA molecules of gRNA molecule embodiment 17, (c) selected from the gRNA molecules of gRNA molecule embodiment 18, or (d) selected from the gRNA molecules of any of gRNA molecule embodiments 57-68.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 10, or (b) selected from the gRNA molecules of gRNA molecule embodiment 11; and
(2) the second gRNA molecule is: (a) selected from the gRNA molecules of gRNA molecule embodiment 13, (b) selected from the gRNA molecules of gRNA molecule embodiment 14.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 10, or (b) selected from the gRNA molecules of gRNA molecule embodiment 11; and
(2) the second gRNA molecule is: (a) selected from the gRNA molecules of gRNA molecule embodiment 16, (b) selected from the gRNA molecules of gRNA molecule embodiment 17, (c) selected from the gRNA molecules of gRNA molecule embodiment 18, or (d) selected from the gRNA molecules of any of gRNA molecule embodiments 57-68.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 13, (b) selected from the gRNA molecules of gRNA molecule embodiment 14; and
(2) the second gRNA molecule is: (a) selected from the gRNA molecules of gRNA molecule embodiment 16, (b) selected from the gRNA molecules of gRNA molecule embodiment 17, (c) selected from the gRNA molecules of gRNA molecule embodiment 18, or (d) selected from the gRNA molecules of any of gRNA molecule embodiments 57-68.

In embodiments, including in any of the aforementioned composition aspects and embodiments, the invention provides a composition, including a first gRNA molecule and a second gRNA molecule, wherein:
(1) the first gRNA molecule is: (a) selected from the gRNA molecules of gRNA molecule embodiment 16, (b) selected from the gRNA molecules of gRNA molecule embodiment 17, (c) selected from the gRNA molecules of gRNA molecule embodiment 18, or (d) selected from the gRNA molecules of any of gRNA molecule embodiments 57-68; and (2) the second gRNA molecule includes a targeting domain that is complementary to a target sequence of the beta globin gene; or
(1) the first gRNA molecule is: (a) selected from the gRNA molecules of gRNA molecule embodiment 4, (b) selected from the gRNA molecules of gRNA molecule embodiment 5, (c) selected from the gRNA molecules of gRNA molecule embodiment 6, (d) selected from the gRNA molecules of gRNA molecule embodiment 7, (e) selected from the gRNA molecules of any of gRNA molecule embodiments 41-56, (f) selected from the gRNA molecules of gRNA molecule embodiment 10, (g) selected from the gRNA molecules of gRNA molecule embodiment 11, (h) selected from the gRNA molecules of gRNA molecule embodiment 13, or (i) selected from the gRNA molecules of gRNA molecule embodiment 14; and (2) the second gRNA molecule includes a targeting domain that is complementary to a target sequence of the beta globin gene.

In embodiments, including in any of the aforementioned composition aspects and embodiments, the invention provides a composition, wherein the first gRNA molecule and the second gRNA molecule are independently selected from the gRNA molecules of any of gRNA molecule embodiments 41-68.

In embodiments of the composition, with respect to the gRNA molecule components of the composition, the composition consists of a first gRNA molecule and a second gRNA molecule.

In embodiments of the composition, each of said gRNA molecules is in a ribonuclear protein complex (RNP) with a Cas9 molecule described herein.

In embodiments, including in any of the aforementioned composition aspects and embodiments, the invention provides a composition, further including a template nucleic acid, wherein the template nucleic acid includes a nucleotide that corresponds to a nucleotide at or near the target sequence of the first gRNA molecule. In embodiments, the template nucleic acid includes nucleic acid encoding: (a) human beta globin, e.g., human beta globin including one or more of the mutations G16D, E22A and T87Q, or fragment thereof; or (b) human gamma globin, or fragment thereof.

In embodiments, including in any of the aforementioned composition aspects and embodiments, the composition is formulated in a medium suitable for electroporation, for example, suitable for electroporation into HSPC cells. In embodiments of the composition which include one or more gRNA molecules, each of said gRNA molecules is in a RNP with a Cas9 molecule described herein, and wherein each of said RNP is at a concentration of less than about 10uM, e.g., less than about 3uM, e.g., less than about 1uM, e.g., less than about 0.5uM, e.g., less than about 0.3uM, e.g., less than about 0.1uM.

In another aspect, the invention provides a nucleic acid sequence that encodes one or more gRNA molecules described herein, for example, a gRNA molecule of any of the previous aspects and embodiments. In embodiments, the nucleic acid includes a promoter operably linked to the sequence that encodes the one or more gRNA molecules, for example, a promoter recognized by an RNA polymerase II or RNA polymerase III, for example, a U6 promoter or an HI promoter. In embodiments, the nucleic acid further encodes a Cas9 molecule, for example, described herein, for example, a Cas9 molecule that includes (e.g., consists of) any of SEQ ID NO: 6611, SEQ ID NO: 7821, SEQ ID NO: 7822, SEQ ID NO: 7823, SEQ ID NO: 7824, SEQ ID NO: 7825, SEQ ID NO: 7826, SEQ ID NO: 7827, SEQ ID NO: 7828, SEQ ID NO: 7829, SEQ ID NO: 7830, or SEQ ID NO: 7831. In embodiments, the nucleic acid includes a promoter operably linked to the sequence that encodes a Cas9 molecule, for example, an EF-1 promoter, a CMV IE gene promoter, an EF-1α promoter, an ubiquitin C promoter, or a phosphoglycerate kinase (PGK) promoter.

In another aspect, the invention provides a vector including the nucleic acid of any of the previous nucleic acid aspects and embodiments. In embodiments, the vector is selected from the group consisting of a lentiviral vector, an adenoviral vector, an adeno-associated viral (AAV) vector, a herpes simplex virus (HSV) vector, a plasmid, a minicircle, a nanoplasmid, and an RNA vector.

In another aspect, the invention provides a method of altering a cell (e.g., a population of cells), (e.g., altering the structure (e.g., sequence) of nucleic acid) at or near a target sequence within said cell, including contacting (e.g., introducing into) said cell (e.g., population of cells) with: 1) one or more gRNA molecules of any of the previous gRNA molecule aspects and embodiments and a Cas9 molecule (e.g., described herein); 2) one or more gRNA molecules of any of the previous gRNA molecule aspects and embodiments and nucleic acid encoding a Cas9 molecule (e.g., described herein); 3) nucleic acid encoding one or more gRNA molecules of any of the previous gRNA molecule aspects and embodiments and a Cas9 molecule (e.g., described herein); 4) nucleic acid encoding one or more gRNA molecules of any of the previous gRNA molecule aspects and embodiments and nucleic acid encoding a Cas9 molecule (e.g., described herein); 5) any of 1) to 4), above, and a template nucleic acid; 6) any of 1) to 4) above, and nucleic acid including sequence encoding a template nucleic acid; 7) the composition of any of the previous composition aspects and embodiments; or 8) the vector of any of the previous vector aspects and embodiments. In embodiments, the gRNA molecule or nucleic acid encoding the gRNA molecule, and the Cas9 molecule or nucleic acid encoding the Cas9 molecule, are formulated in a single composition. In other embodiments, the gRNA molecule or nucleic acid encoding the gRNA molecule, and the Cas9 molecule or nucleic acid encoding the Cas9 molecule, are formulated in more than one composition. In embodiments that include more than one composition, the more than one composition are delivered simultaneously or sequentially. In embodiments, the cell is an animal cell, for example, a mammalian, primate, or human cell. In embodiments, the cell, is a hematopoietic stem or progenitor cell (HSPC) (e.g., a population of HSPCs), for example, the cell is a CD34+ cell, for example, the cell is a CD34+CD90+ cell. In embodiments, the cell is disposed in a composition including a population of cells that has been enriched for CD34+ cells. In embodiments, the cell (e.g. population of cells) has been isolated from bone marrow, mobilized peripheral blood, or umbilical cord blood. In embodiments, the cell is autologous with respect to a patient to be administered said cell. In other embodiments, the cell is allogeneic with respect to a patient to be administered said cell. In embodiments, the method results in an indel at or near a genomic DNA sequence complementary to the targeting domain of the one or more gRNA molecules, for example, an indel shown on Figure 25, Table 15, Table 26, Table 27 or Table 37, for example an indel associated with the targeting domain of the gRNA molecule as shown in Figure 25, Table 15, Table 26, Table 27 or Table 37. In embodiments, the indel is an insertion or deletion of less than about 40 nucleotides, e.g., less than 30 nucleotides, e.g., less than 20 nucleotides, e.g., less than 10 nucleotides, for example, is a single nucleotide deletion. In embodiments, the method results in a population of cells wherein at least about 50%, e.g., at least about 60%, e.g., at least about 70%, e.g., at least about 80%, e.g., at least about 90% (e.g., at least about 95%, at least about 96%, at least about 97%, at least about 98%, or at least about 99%) of the cells of the population have been altered, e.g., include an indel. In embodiments, the method (e.g., the method performed on a population of cells, e.g., described herein) results in a cell (e.g., population of cells) that is capable of differentiating into a differentiated cell of an erythroid lineage (e.g., a red blood cell), and wherein said differentiated cell exhibits an increased level of fetal hemoglobin, e.g., relative to an unaltered cell (e.g., population of cells). In embodiments, the method results in a population of cells that is capable of differentiating into a population of differentiated cells, e.g., a population of cells of an erythroid lineage (e.g., a population of red blood cells), and wherein said population of differentiated cells has an increased fraction of F cells (e.g., at least about 15%, at least about 20% , at least about 25%, at least about 30%, or at least about 40% higher) e.g., relative to a population of unaltered cells. In embodiments, the method results in a cell that is capable of differentiating into a differentiated cell, e.g., a cell of an erythroid lineage (e.g., a red blood cell), and wherein said differentiated cell produces at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin per cell. In embodiments, the method is performed ex vivo. In other embodiments, the method is performed in vivo.

In another aspect, the invention provides a cell, altered by the method of altering a cell of any of the previous method aspects and embodiments.

In another aspect, the invention provides a cell, obtainable the method of altering a cell of any of the previous method aspects and embodiments.

In another aspect, the invention provides a cell, including a first gRNA molecule, e.g., described herein, e.g., of any of the previous gRNA molecule aspects and embodiments, or a composition, e.g., described herein, e.g., of any of the previous composition aspects and embodiments, a nucleic acid, e.g., described herein, e.g., of any of the previous nucleic acid aspects and embodiments, or a vector, e.g., described herein, e.g., of any of the previous vector aspects and embodiments. In embodiments, the cell further includes a Cas9 molecule, e.g., described herein, for example, a Cas9 molecule that includes, e.g., consists of, any of SEQ ID NO: 6611, SEQ ID NO: 7821, SEQ ID NO: 7822, SEQ ID NO: 7823, SEQ ID NO: 7824, SEQ ID NO: 7825, SEQ ID NO: 7826, SEQ ID NO: 7827, SEQ ID NO: 7828, SEQ ID NO: 7829, SEQ ID NO: 7830, or SEQ ID NO: 7831. In embodiments, the cell includes, has included, or will include a second gRNA molecule, e.g., described herein, e.g., of any of the previous gRNA molecule aspects and embodiments, or a nucleic acid encoding a second gRNA molecule, e.g., described herein, e.g., of any of the previous gRNA molecule aspects and embodiments, wherein the first gRNA molecule and second gRNA molecule include nonidentical targeting domains. In embodiments, expression of fetal hemoglobin is increased in said cell or its progeny (e.g., its erythroid progeny, e.g., its red blood cell progeny) relative to a cell or its progeny of the same cell type that has not been modified to include a gRNA molecule. In embodiments, the cell is capable of differentiating into a differentiated cell, e.g., a cell of an erythroid lineage (e.g., a red blood cell), and wherein said differentiated cell exhibits an increased level of fetal hemoglobin, e.g., relative to a cell of the same type that has not been modified to include a gRNA molecule. In embodiments, the differentiated cell (e.g., cell of an erythroid lineage, e.g., red blood cell) produces at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin, e.g., relative to a cell of the same type that has not been modified to include a gRNA molecule. In embodiments, the cell has been contacted, e.g., contacted ex vivo, with a stem cell expander, e.g., described herein, e.g., a stem cell expander that is compound 1, compound 2, compound 3, compound 4, or a combination thereof (e.g., compound 1 and compound 4), for example, a stem cell expander that is compound 4. In embodiments, including in any of the aforementioned aspects and embodiments, the cell includes an indel at or near a genomic DNA sequence complementary to the targeting domain of the gRNA molecule (as described herein, e.g., a gRNA molecule of any of the aforementioned aspects and embodiments) introduced therein, for example, an indel shown on Figure 25, Table 15, Table 26, Table 27 or Table 37, for example an indel shown on Figure 25, Table 15, Table 26, Table 27 or Table 37 that is associated with the gRNA molecule (as described herein, e.g., a gRNA molecule of any of the aforementioned aspects and embodiments) introduced therein. In embodiments, the indel is an insertion or deletion of less than about 40 nucleotides, e.g., less than 30 nucleotides, e.g., less than 20 nucleotides, e.g., less than 10 nucleotides, for example, the indel is a single nucleotide deletion. In embodiments, including in any of the aforementioned cell aspects and embodiments, the cell is an animal cell, for example, the cell is a mammalian, a primate, or a human cell. In embodiments, including in any of the aforementioned cell aspects and embodiments, the cell is a hematopoietic stem or progenitor cell (HSPC) (e.g., a population of HSPCs), for example, the cell is a CD34+ cell, for example, the cell is a CD34+CD90+ cell. In embodiments, including in any of the aforementioned cell aspects and embodiments, the cell (e.g. population of cells) has been isolated from bone marrow, mobilized peripheral blood, or umbilical cord blood. In embodiments, including in any of the aforementioned cell aspects and embodiments, the cell is autologous with respect to a patient to be administered said cell. In embodiments, the cell is allogeneic with respect to a patient to be administered said cell.

In another aspect, the invention provides a population of cells including the cell of any of the previous cell aspects and embodiments. In embodiments, at least about 50%, e.g., at least about 60%, e.g., at least about 70%, e.g., at least about 80%, e.g., at least about 90% (e.g., at least about 95%, at least about 96%, at least about 97%, at least about 98%, or at least about 99%) of the cells of the population are a cell according to any of the previous cell aspects and embodiments. In embodiments, the population of cells is capable of differentiating into a population of differentiated cells, e.g., a population of cells of an erythroid lineage (e.g., a population of red blood cells), and wherein said population of differentiated cells has an increased fraction of F cells (e.g., at least about 15%, at least about 20%, at least about 25%, at least about 30%, or at least about 40% higher) e.g., relative to a population of unmodified cells of the same type. In embodiments, the F cells of the population of differentiated cells produce an average of at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin per cell. In embodiments, the population includes: 1) at least 1e6 CD34+ cells/kg body weight of the patient to whom the cells are to be administered; 2) at least 2e6 CD34+ cells/kg body weight of the patient to whom the cells are to be administered; 3) at least 3e6 CD34+ cells/kg body weight of the patient to whom the cells are to be administered; 4) at least 4e6 CD34+ cells/kg body weight of the patient to whom the cells are to be administered; or 5) from 2e6 to 10e6 CD34+ cells/kg body weight of the patient to whom the cells are to be administered. In embodiments, at least about 40%, e.g., at least about 50%, (e.g., at least about 60%, at least about 70%, at least about 80%, or at least about 90%) of the cells of the population are CD34+ cells. In embodiments, at least about 10%, e.g., at least about 15%, e.g., at least about 20%, e.g., at least about 30% of the cells of the population are CD34+CD90+ cells. In embodiments, the population of cells is derived from bone marrow, peripheral blood (e.g., mobilized peripheral blood), umbilical cord blood, or induced pluripotent stem cells (iPSCs). In a preferred embodiment, the population of cells is derived from bone marrow. In embodiments, the population of cells includes, e.g., consists of, mammalian cells, e.g., human cells. In embodiments, the population of cells is autologous relative to a patient to which it is to be administered. In other embodiments, the population of cells is allogeneic relative to a patient to which it is to be administered.

In another aspect, the invention provides a composition including a cell of any of the previous cell aspects and embodiments, or the population of cells of any of previous population of cell aspects and embodiments. In embodiments, the composition includes a pharmaceutically acceptable medium, e.g., a pharmaceutically acceptable medium suitable for cryopreservation.

In another aspect, the invention provides a method of treating a hemoglobinopathy, including administering to a patient a cell of any of the previous cell aspects and embodiments, a population of cells of any of previous population of cell aspects and embodiments, or a composition of any of the previous composition aspects and embodiments. In embodiments, the hemoglobinopathy is a thalassemia, for example, beta-thalassemia, or sickle cell disease.

In another aspect, the invention provides a method of increasing fetal hemoglobin expression in a mammal, including administering to a patient a cell of any of the previous cell aspects and embodiments, a population of cells of any of previous population of cell aspects and embodiments, or a composition of any of the previous composition aspects and embodiments.

In another aspect, the invention provides a method of preparing a cell (e.g., a population of cells) including: (a) providing a cell (e.g., a population of cells) (e.g., a HSPC (e.g., a population of HSPCs)); (b) culturing said cell (e.g., said population of cells) ex vivo in a cell culture medium including a stem cell expander; and (c) introducing into said cell a first gRNA molecule (e.g., described herein, for example, a gRNA molecule of any of the previous gRNA molecule aspects and embodiments), a nucleic acid molecule encoding a first gRNA molecule (e.g., described herein, for example, a gRNA molecule of any of the previous gRNA molecule aspects and embodiments), a composition (e.g., described herein, for example, a composition of any of the previous composition aspects and embodiments), a nucleic acid (e.g., described herein, for example, a nucleic acid of any of the previous nucleic acid aspects and embodiments), or a vector (e.g., described herein, for example, a vector of any of the previous vector aspects and embodiments). In embodiments of said method, after said introducing of step (c), said cell (e.g., population of cells) is capable of differentiating into a differentiated cell (e.g., population of differentiated cells), e.g., a cell of an erythroid lineage (e.g., population of cells of an erythroid lineage), e.g., a red blood cell (e.g., a population of red blood cells), and wherein said differentiated cell (e.g., population of differentiated cells) produces increased fetal hemoglobin, e.g., relative to the same cells which have not been subjected to step (c). In embodiments, including in any of the aforementioned method aspects and embodiments, the stem cell expander is compound 1, compound 2, compound 3, compound 4 or a combination thereof (e.g., compound 1 and compound 4), for example, is compound 4. In embodiments, including in any of the aforementioned method aspects and embodiments, the cell culture medium includes thrombopoietin (Tpo), Flt3 ligand (Flt-3L), and human stem cell factor (SCF). In embodiments, including in any of the aforementioned method aspects and embodiments, the cell culture medium further includes human interleukin-6 (IL-6). In embodiments, including in any of the aforementioned method aspects and embodiments, the cell culture medium includes thrombopoietin (Tpo), Flt3 ligand (Flt-3L), and human stem cell factor (SCF) each at a concentration ranging from about 10 ng/mL to about 1000 ng/mL, for example, each at a concentration of about 50 ng/mL, e.g., at a concentration of 50 ng/mL. In embodiments, including in any of the aforementioned method aspects and embodiments, the cell culture medium includes human interleukin-6 (IL-6) at a concentration ranging from about 10 ng/mL to about 1000 ng/mL, for example, at a concentration of about 50 ng/mL, e.g., at a concentration of 50 ng/mL. In embodiments, including in any of the aforementioned method aspects and embodiments, the cell culture medium includes a stem cell expander at a concentration ranging from about 1 nM to about 1 mM, for example, at a concentration ranging from about 1 uM to about 100 uM, for example, .at a concentration ranging from about 50 uM to about 75 uM, for example, at a concentration of about 50 uM, e.g., at a concentration of 50 uM, or at a concentration of about 75 uM, e.g., at a concentration of 75 uM. In embodiments, including in any of the aforementioned method aspects and embodiments, the culturing of step (b) includes a period of culturing before the introducing of step (c), for example, the period of culturing before the introducing of step (c) is at least 12 hours, e.g., is for a period of about 1 day to about 3 days, e.g., is for a period of about 1 day to about 2 days, e.g., is for a period of about 2 days. In embodiments, including in any of the aforementioned method aspects and embodiments, the culturing of step (b) includes a period of culturing after the introducing of step (c), for example, the period of culturing after the introducing of step (c) is at least 12 hours, e.g., is for a period of about 1 day to about 10 days, e.g., is for a period of about 1 day to about 5 days, e.g., is for a period of about 2 days to about 4 days, e.g., is for a period of about 2 days or is for a period of about 3 days or is for a period of about 4 days. In embodiments, including in any of the aforementioned method aspects and embodiments, the population of cells is expanded at least 4-fold, e.g., at least 5-fold, e.g., at least 10-fold, e.g., relative to cells which are not cultured according to step (b). In embodiments, including in any of the aforementioned method aspects and embodiments, the introducing of step (c) includes an electroporation, for example, an electroporation that includes 1 to 5 pulses, e.g., 1 pulse, and wherein each pulse is at a pulse voltage ranging from 700 volts to 2000 volts and has a pulse duration ranging from 10 ms to 100 ms. In embodiments, including in any of the aforementioned method aspects and embodiments, the electroporation includes, for example, consists of, 1 pulse. In embodiments, including in any of the aforementioned method aspects and embodiments, the pulse voltage ranges from 1500 to 1900 volts, e.g., is 1700 volts. In embodiments, including in any of the aforementioned method aspects and embodiments, the pulse duration ranges from 10 ms to 40 ms, e.g., is 20 ms. In embodiments, including in any of the aforementioned method aspects and embodiments, the cell (e.g., population of cells) provided in step (a) is a human cell (e.g., a population of human cells). In embodiments, including in any of the aforementioned method aspects and embodiments, the cell (e.g., population of cells) provided in step (a) is isolated from bone marrow, peripheral blood (e.g., mobilized peripheral blood), umbilical cord blood, or induced pluripotent stem cells (iPSCs), preferably bone marrow. In embodiments, including in any of the aforementioned method aspects and embodiments, the cell (e.g., population of cells) provided in step (a) is isolated from bone marrow, e.g., is isolated from bone marrow of a patient suffering from a hemoglobinopathy. In embodiments, including in any of the aforementioned method aspects and embodiments, the population of cells provided in step (a) is enriched for HSPCs, e.g., CD34+ cells. In embodiments, including in any of the aforementioned method aspects and embodiments, subsequent to the introducing of step (c), the cell (e.g., population of cells) is cryopreserved. In embodiments, including in any of the aforementioned method aspects and embodiments, subsequent to the introducing of step (c), the cell (e.g., population of cells) includes an indel at or near a genomic DNA sequence complementary to the targeting domain of the first gRNA molecule, for example, an indel shown on Figure 25, Table 15, Table 26, Table 27 or Table 37, for example an indel shown in Figure 25, Table 15, Table 26, Table 27 or Table 37 as associated with the first gRNA molecule. In embodiments, including in any of the aforementioned method aspects and embodiments, after the introducing of step (c), at least about 50%, at least about 60%, at least about 70%, at least about 80%, at least about 90%, at least about 95%, at least about 96%, at least about 97%, at least about 98% or at least about 99% of the cells of the population of cells include an indel at or near a genomic DNA sequence complementary to the targeting domain of the first gRNA molecule, for example, an indel shown on Figure 25, Table 15, Table 26, Table 27 or Table 37, for example an indel shown in Figure 25, Table 15, Table 26, Table 27 or Table 37 as associated with the first gRNA molecule.

In another aspect, the invention provides a cell (e.g., population of cells), obtainable by the method of preparing a cell of any of the previous aspects and embodiments. In another aspect, the invention provides a method of treating a hemoglobinopathy (for example a thalassemia (e.g., beta-thalassemia) or sickle cell disease), including administering to a human patient a composition including said cell (e.g., population of cells). In another aspect, the invention provides a method of increasing fetal hemoglobin expression in a human patient, including administering to said human patient a composition including said cell (e.g., population of cells). In embodiments, the human patient is administered a composition including at least about 1e6 cells (e.g., CD34+ cells, e.g., cells obtainable by a method of preparing a cell of any of the previous aspects and embodiments) per kg body weight of the human patient, e.g., at least about 1e6 CD34+ cells obtainable by a method of preparing a cell of any of the previous aspects and embodiments per kg body weight of the human patient. In embodiments, the human patient is administered a composition including at least about 2e6 cells (e.g., CD34+ cells, e.g., cells obtainable by a method of preparing a cell of any of the previous aspects and embodiments) per kg body weight of the human patient, e.g., at least about 2e6 CD34+ cells obtainable by a method of preparing a cell of any of the previous aspects and embodiments per kg body weight of the human patient. In embodiments, the human patient is administered a composition including from about 2e6 to about 10e6cells (e.g., CD34+ cells, e.g., cells obtainable by a method of preparing a cell of any of the previous aspects and embodiments) per kg body weight of the human patient, e.g., at least about 2e6 to about 10e6 CD34+ cells obtainable by a method of preparing a cell of any of the previous aspects and embodiments per kg body weight of the human patient.

In another aspect, the invention provides a gRNA molecule described herein, for example, a gRNA molecule of any of the previous gRNA molecule aspects and embodiments, a composition described herein, for example, a composition of any of the previous composition aspects and embodiments, a nucleic acid described herein, for example, a nucleic acid of any of the previous nucleic acid aspects and embodiments, a vector described herein, for example, a vector of any of the previous vector aspects and embodiments, a cell described herein, for example, a cell of any of the previous cell aspects and embodiments, or a population of cells described herein, for example, a population of cells of any of the previous population of cell aspects and embodiments, for use as a medicament.

In another aspect, the invention provides a gRNA molecule described herein, for example, a gRNA molecule of any of the previous gRNA molecule aspects and embodiments, a composition described herein, for example, a composition of any of the previous composition aspects and embodiments, a nucleic acid described herein, for example, a nucleic acid of any of the previous nucleic acid aspects and embodiments, a vector described herein, for example, a vector of any of the previous vector aspects and embodiments, a cell described herein, for example, a cell of any of the previous cell aspects and embodiments, or a population of cells described herein, for example, a population of cells of any of the previous population of cell aspects and embodiments, for use in the manufacture of a medicament.

In another aspect, the invention provides a gRNA molecule described herein, for example, a gRNA molecule of any of the previous gRNA molecule aspects and embodiments, a composition described herein, for example, a composition of any of the previous composition aspects and embodiments, a nucleic acid described herein, for example, a nucleic acid of any of the previous nucleic acid aspects and embodiments, a vector described herein, for example, a vector of any of the previous vector aspects and embodiments, a cell described herein, for example, a cell of any of the previous cell aspects and embodiments, or a population of cells described herein, for example, a population of cells of any of the previous population of cell aspects and embodiments, for use in the treatment of a disease.

In another aspect, the invention provides a gRNA molecule described herein, for example, a gRNA molecule of any of the previous gRNA molecule aspects and embodiments, a composition described herein, for example, a composition of any of the previous composition aspects and embodiments, a nucleic acid described herein, for example, a nucleic acid of any of the previous nucleic acid aspects and embodiments, a vector described herein, for example, a vector of any of the previous vector aspects and embodiments, a cell described herein, for example, a cell of any of the previous cell aspects and embodiments, or a population of cells described herein, for example, a population of cells of any of the previous population of cell aspects and embodiments, for use in the treatment of a disease, wherein the disease is a hemoglobinopathy, for example a thalassemia (e.g., beta-thalassemia) or sickle cell disease.

### BRIEF DESCRIPTION OF THE DRAWINGS

Figure 1: Cas9 editing of the Bcl11a +58 erythroid enhancer region. Fraction of editing detected by NGS in HEK-293 Cas9GFP 24h post-delivery of crRNA targeting the +58 enhancer region and trRNA by lipofection. Each dot indicates a different crRNA, the trRNA was held constant. Genomic coordinates indicate location on Chromosome 2, e.g., in reference to hg38. (n=1)
Figure 2: Cas9 editing of the Bcl11a +62 erythroid enhancer region. Fraction of editing detected by NGS in HEK-293 Cas9GFP 24h post-delivery of crRNA targeting the +62 enhancer region and trRNA by lipofection. Each dot indicates a different crRNA, the trRNA was held constant. Genomic coordinates indicate location on Chromosome 2, e.g., in reference to hg38. (n=1)
Figure 3. Gating strategy for selection of cells after introduction of the Cas9 editing system.

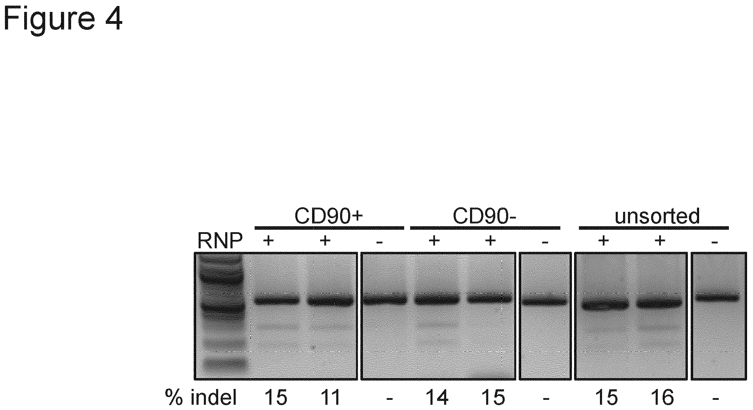

Figure 4. Agarose gel electrophoresis of gene fragments from Cas9 editing system-treated CD34+ cells (shown are both CD34+CD90+ and CD34+CD90- cell populations, together with reference unsorted cells). The upper band represents uncleaved homoduplex DNA and the lower bands indicate cleavage products resulting from heteroduplex DNA. The far left lane is a DNA ladder. Band intensity was calculated by peak integration of unprocessed images by ImageJ software. % gene modification (indel) was calculated as follows: % gene modification = 100 x (1- (1- fraction cleaved)^{1/2}) , and is indicated below each corresponding lane of the gel.
Figure 5. Erythroid enhancer region showing the sites of genomic DNA targeted by sgRNA molecules comprising targeting domains complimentary to the underlined nucleotides.
Figure 6A/6B. NGS results for indel formation by sgEH1 (CR00276) (A) or sgEH2 (CR00275) (B) in CD34+ HSC cells. Insertions are in uppercase. Deletions are indicated by a dashed line. Regions of microhomology near the cutting site are highlighted in bold underline.
Figure 6C/6D. NGS results for indel formation by sgEH8 (CR00273) (C) or sgEH9 (CR00277) (D) in CD34+ HSC cells. Insertions are in uppercase. Deletions are indicated by a dashed line. Regions of microhomology near the cutting site are highlighted in bold underline.
Figure 7. NGS results and indel pattern formation across multiple donors for g7, g8 and g2. The sequences of the top 3 indels from two biological replicate experiments using g7 and g8 were identical, and the sequence of the top 3 indels using g2 were identical to those from a previous experiment.
Figure 8. NGS results and indel pattern formation using a modified gRNA scaffold (BC). The sequences of the top 3 indels from these experiments are identical to those formed when using the standard gRNA scaffold.
Figure 9. Comparison of indel pattern across different delivery methods. AVG % refers to the % of NGS reads exhibiting the indel indicated (Average of 2 experiments).
Figure 10. Indel formation by co-introduction of g7 gRNA and g8 gRNA with either a non-extended flagpole region ("reg") or with a first flagpole extension and first tracr extension ("BC"). PAM sequences for the two gRNAs are boxed.
Figure 11: Top gRNA sequences directed to +58 enhancer region of BCL11a gene in CD34+ cells, as measured by NGS (n=3).
Figure 12: Top gRNA sequences directed to +62 enhancer region of BCL11a gene in CD34+ cells, as measured by NGS (n=3).

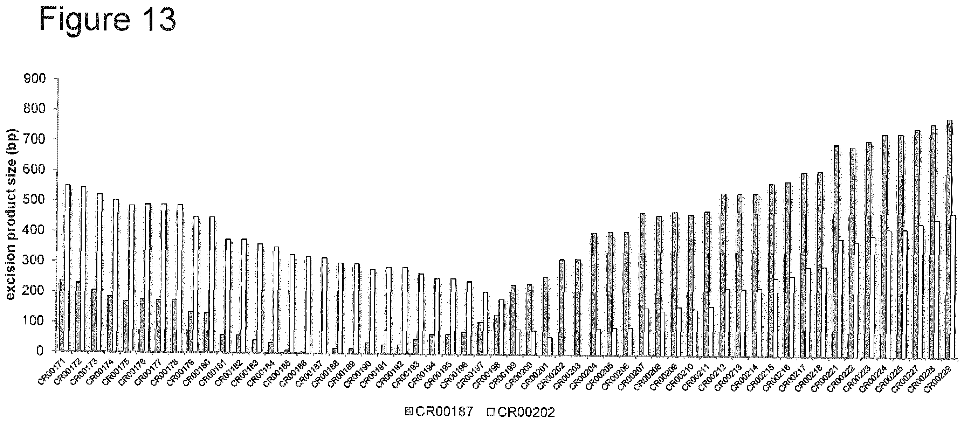

Figure 13: Predicted excision sizes when 2 gRNA molecules targeting the BCL11a +62 enhancer locus are introduced into HEK293_Cas9 cells, when either CR00187 (light grey bars) or CR00202 (dark grey bars) is held constant and co-inserted into the cells with a second gRNA molecule including the targeting domain of the gRNA indicated.

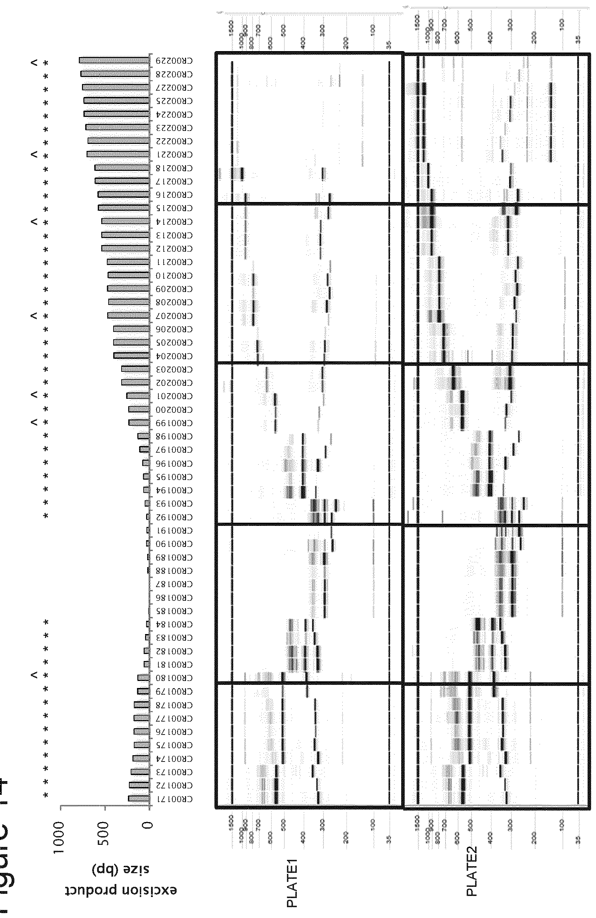

Figure 14: Excision of genomic DNA within the +62 enhancer of BCL11a by addition of gRNA molecules including the targeting domain of CR00187 and the second gRNA molecule indicated in the graph. Stars (^{∗}) indicate excision observed at a frequency greater than 30%; Carats (^) indicate excision observed at a lower frequency (<30%). Those predicted excision products resulting in a fragment less than 50 nt could not be distinguished.

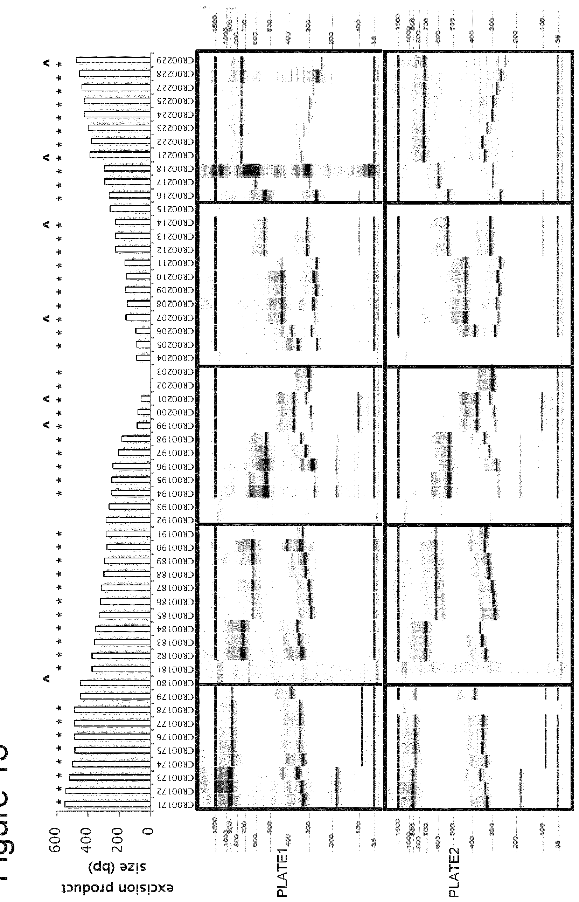

Figure 15: Excision of genomic DNA within the +62 enhancer of BCL11a by addition of gRNA molecules including the targeting domain of CR00202 and the second gRNA molecule indicated in the graph. Stars (^{∗}) indicate excision observed at a frequency greater than 30%; Carats (^) indicate excision observed at lower frequency (<30%).

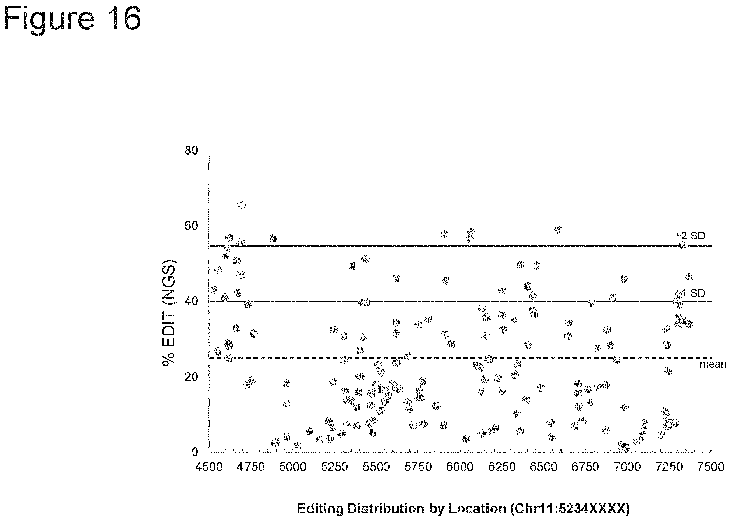

Figure 16: % indel formation by 192 gRNA molecules targeted to the French HPFH region (*Sankaran VG et al. A functional element necessary for fetal hemoglobin silencing. NEJM (2011) 365:807-814*.)
Figure 17. Experimental scheme of Cas9:gRNA ribonucleoprotein (Cas9-RNP) delivery to primary human CD34+ HSPC for genome editing, followed by genetic and phenotypic characterization.
Figure 18. Cell viability following mock electroporation or electroporation of Cas9-RNP complexes into CD34+ HSPC. The HSPC were expanded *in vitro* for 48 hours after electroporation of RNP complexes and the cell viability monitored and percent viability determined. The names of gRNAs used to make RNP complexes are shown on x- axis and the corresponding cell viabilities on y-axis. The CRxxxx identifier indicates the targeting domain of the gRNA molecule.

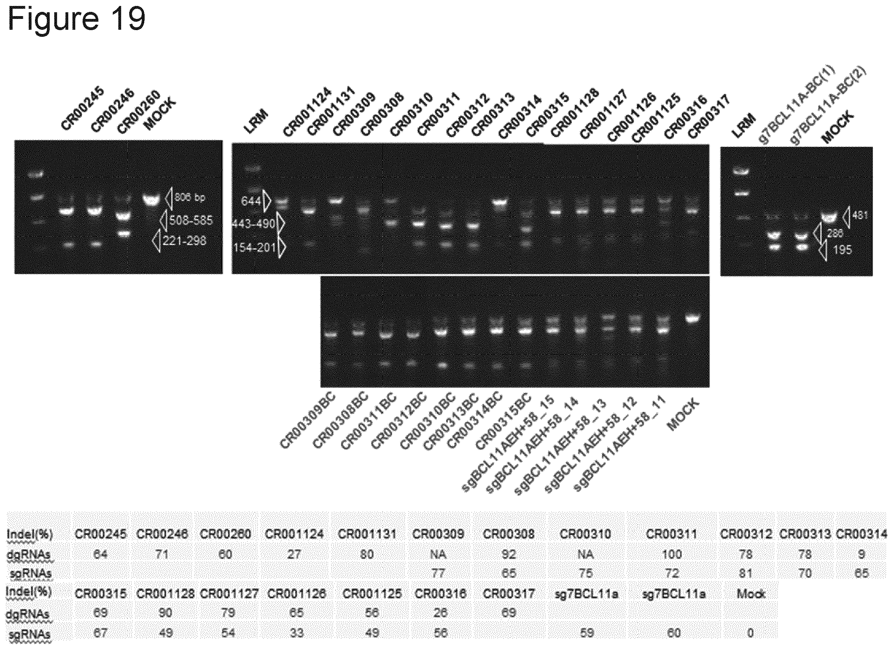

Figure 19. Mismatch detection using T7 endonuclease assay. Human HSC were electroporated to deliver RNP complexes to introduce indels into +58 DHS region of BCL11A erythroid enhancer by NHEJ. PCR amplicons spanning the target region was subjected to the T7E1 assay and the resulting fragments analyzed by 2% agarose gel electrophoresis. Regions of +58 erythroid enhancer BCL11A were disrupted by both guide RNA formats (dual guide RNA - black; single guide RNA -gray (bottom graph and g7BCL11a-BC(1) and g7BCL11A-BC(2) from top graph)). The intensities of DNA bands were estimated by the ImageJ software (http://rsb.info.nih.gov/ij/) to determine the percent of edited alleles. Names of gRNAs used and corresponding gene editing efficiencies determined from mismatch detection assay are also shown in the table below the gel image.
Figure 20: Percent allele editing by next generation sequencing. Labels are as described for Figures 18 and 19.

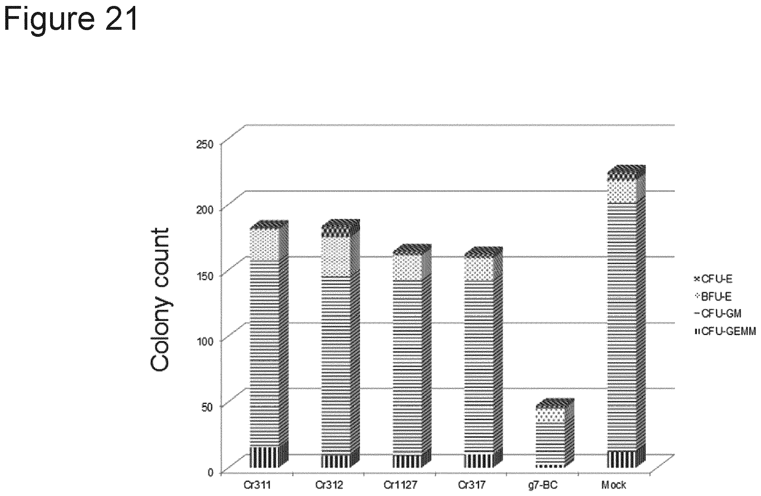

Figure 21. Colony forming cell (CFC) unit assay showing colony number and types of colonies observed for five select gRNAs. The HSPCs genome edited at the +58 erythroid enhancer were plated in methylcellulose and clonal colonies classified and counted based on the number and types of mature cells using morphological and phenotypic criteria using STEMvision. The colonies were classified into colony forming unit-erythroid (CFU-E), burst forming unit-erythroid (BFU-E), colony-forming unit - granulocyte/macrophage (CFU-GM) and colony-forming unit - granulocyte/erythrocyte/macrophage/megakaryocyte (CFU-GEMM).
Figure 22. Kinetics of cell division during erythroid differentiation. Total number of cells determined on day 0, 7, 14 and 21 during erythroid differentiation and the fold cell expansion calculated.
Figure 23. BCL11A, gamma and beta- globin mRNA levels in genome edited and erythroid differentiated HSC. Relative mRNA expression of BCL11A, gamma- and beta-globin chains in unilineage erythroid cultures were quantified by real time PCR. Transcript levels were normalized against human GAPDH transcript levels.
Figure 24A. Efficient editing of HSC obtained with dual gRNA/cas9 system for disruption of BCL11a enhancer (+58) resulted in high levels of induction of HbF. The HSC 48 hours after electroporation were subjected to *in vitro* erythroid differentiation and HbF expression was measured. The percent of HbF positive cells (F-cells) was monitored by FACS analysis at days 7, 14 and 21. Data shown in this figure was generated with dgRNA systems where the dgRNA included the indicated targeting domain.
Figure 24B. Efficient editing of HSC obtained with sgRNA/cas9 system for disruption of BCL11a enhancer (+58) resulted in high levels of induction of HbF. The HSC 48 hours after electroporation were subjected to *in vitro* erythroid differentiation and HbF expression was measured. The percent of HbF positive cells (F-cells) was monitored by FACS analysis at days 7, 14 and 21. Data shown in this figure was generated with sgRNA systems where the sgRNA included the indicated targeting domain.
Figure 25: Indel pattern produced in HSPCs by gRNAs including the indicated targeting domain.
Figure 26A: Phenotyping of edited and unedited cultures in CD34+ cell expansion medium by cell surface marker staining. All gRNA molecules were tested in the dgRNA format. The Tracr used was SEQ ID NO: 7808, and the crRNA had the following format and sequence with 2'O-Methyl (m) and Phosphorothioate Bond (^{∗}) modifications indicated:
   mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the residues of the indicated targeting domain. Shown are representative dot plots showing the fluorescence of cells stained with each antibody panel or corresponding isotype controls as described in Materials and Methods. Cells shown were pre-gated on the viable cell population by forward and side scatter properties and DAPI (4',6-Diamidino-2-Phenylindole) discrimination. The percentage of each cell population named at the top of the plot was determined by the gate indicated by the bold lined box.

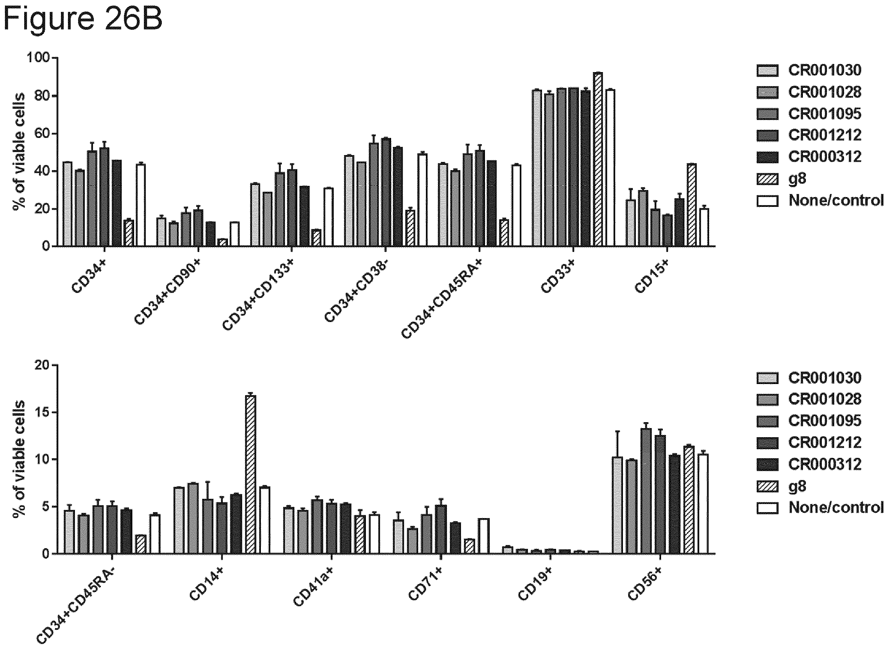

Figure 26B: Phenotyping of edited and unedited cultures in CD34+ cell expansion medium by cell surface marker staining. All gRNA molecules were tested in the dgRNA format. The Tracr used was SEQ ID NO: 7808, and the crRNA had the following format and sequence with 2'O-Methyl (m) and Phosphorothioate Bond (^{∗}) modifications indicated:
   mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the residues of the indicated targeting domain. Shown is the percentage of each named cell population in edited and unedited cultures. Targeting domain of the edited cultures is as indicated.

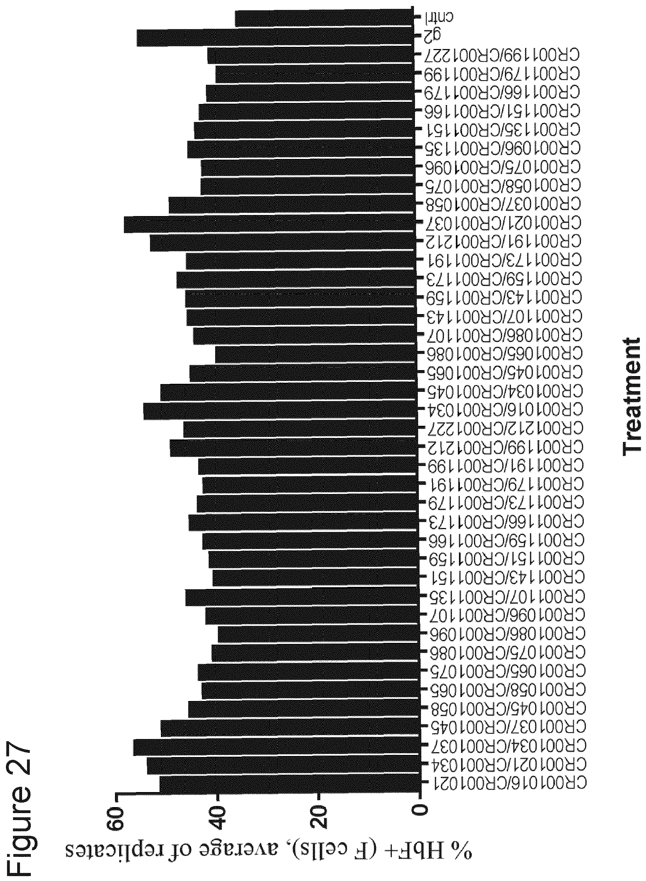

Figure 27. % F cells in erythrocytes differentiated from populations of CD34+ cells edited with RNPs comprising dgRNAs targeting two sites within the HPFH region. "g2" is the positive control (targeting domain to a coding region of BCL11a gene); "cntrl" is negative control (introduction of Cas9 only).

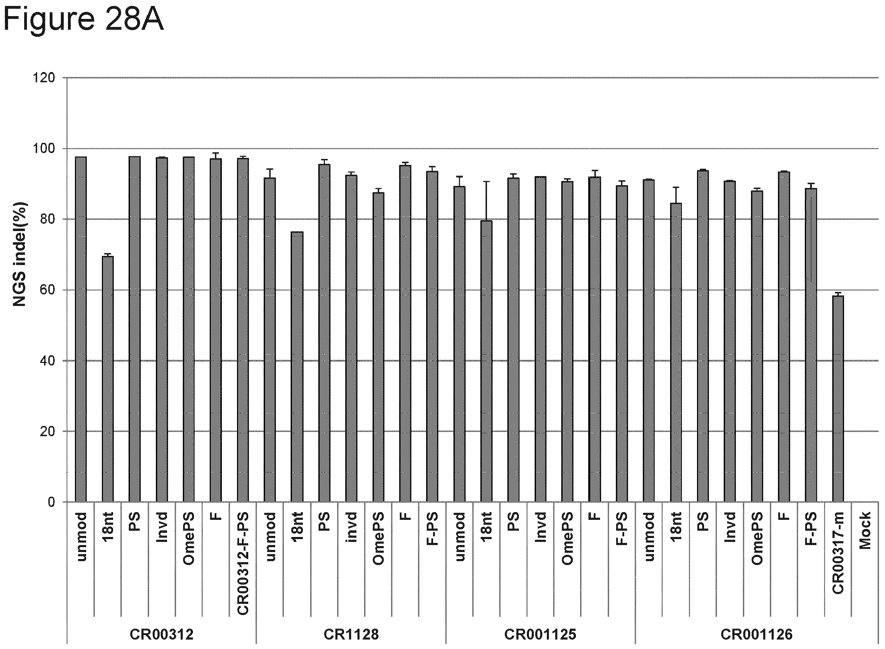

Figure 28A. % Editing as determined by NGS in CD34+ HSPCs at day 2 following electroporation of RNP comprising the indicated dgRNA (unmod or modified as indicated). Controls include dgRNA comprising the targeting domain of CR00317 unmodified (CR00317-m), or electroporation in buffer only (No Cas9 or gRNA; "Mock").

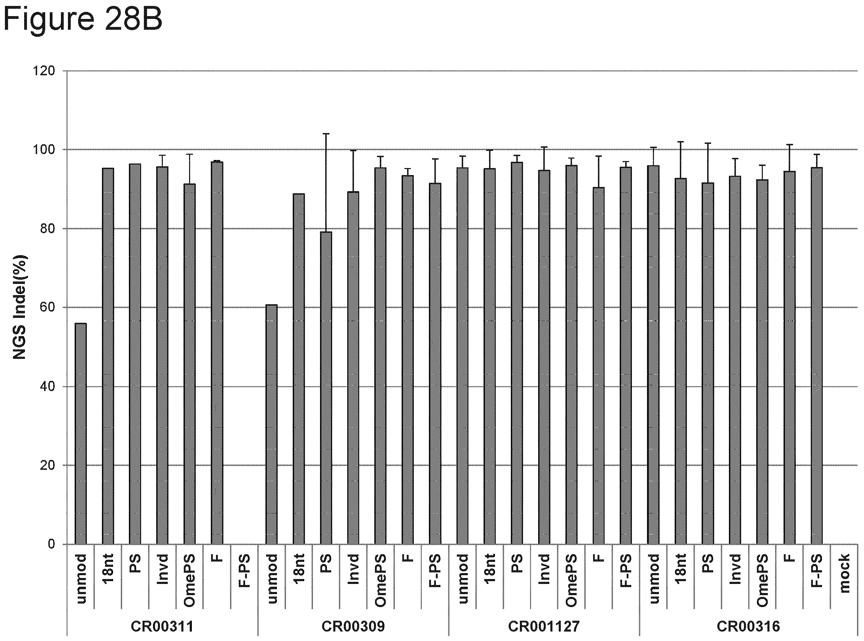

Figure 28B. % Editing as determined by NGS in CD34+ HSPCs at day 2 following electroporation of RNP comprising the indicated dgRNA (unmod or modified as indicated). Controls include electroporation in buffer only (No Cas9 or gRNA; "Mock").

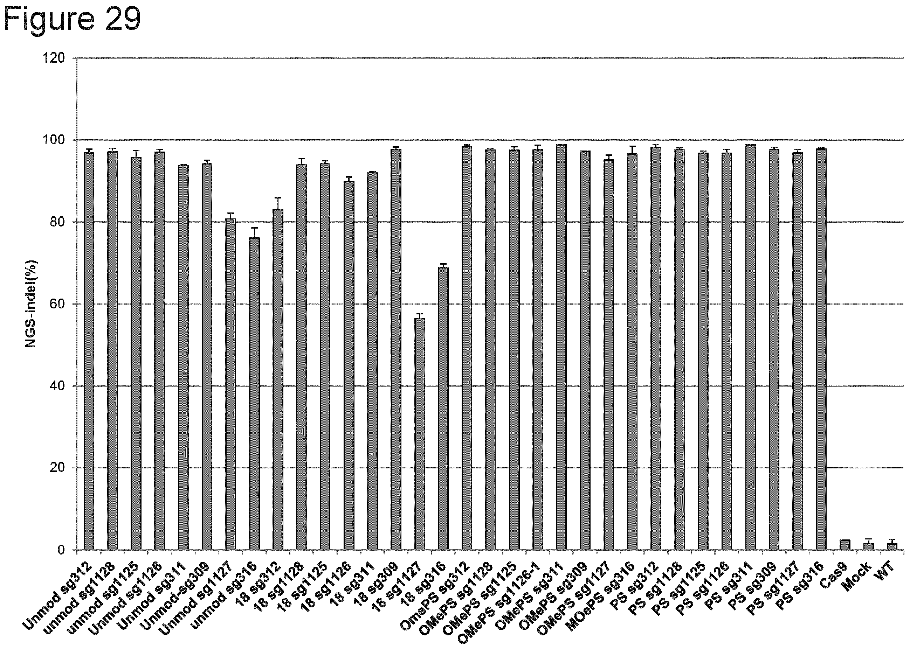

Figure 29. % Editing as determined by NGS in CD34+ HSPCs at day 2 following electroporation of RNP comprising the indicated sgRNA (unmod or modified as indicated. In each case the number corresponds to the CRxxxxxx identifier of the targeting domain, for example, Unmod sg312 refers to unmodified sgRNA comprising the targeting domain of CR00312). Controls include electroporation of Cas9 protein only ("Cas9"), electroporation with buffer only ("mock"), or with no electroporation ("WT").

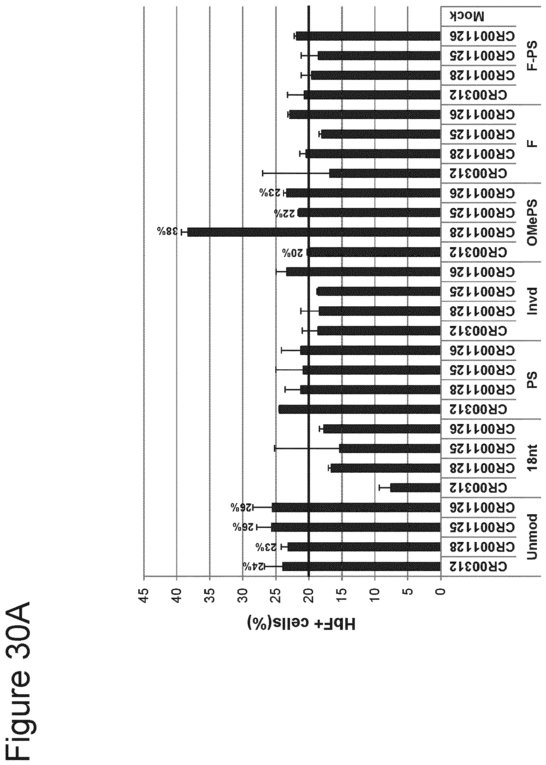

Figure 30A. Normalized % Erythrocytes (% F cells in "Mock" subtracted) that are HbF+ after electroporation of CD34+ HSPCs with RNP comprising the indicated dgRNA (unmod or modified as indicated) and then induced to differentiate by culture in erythroid differentiation medium. Controls include electroporation of buffer only ("mock").

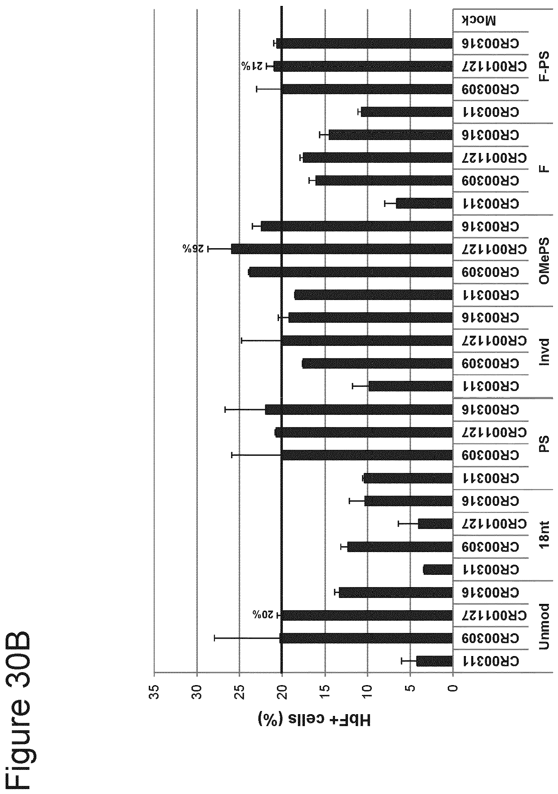

Figure 30B. Normalized % Erythrocytes (% F cells in "Mock" subtracted) that are HbF+ after electroporation of CD34+ HSPCs with RNP comprising the indicated dgRNA (unmod or modified as indicated) and then in induced to differentiate by culture in erythroid differentiation medium. Controls include electroporation of buffer only ("mock").

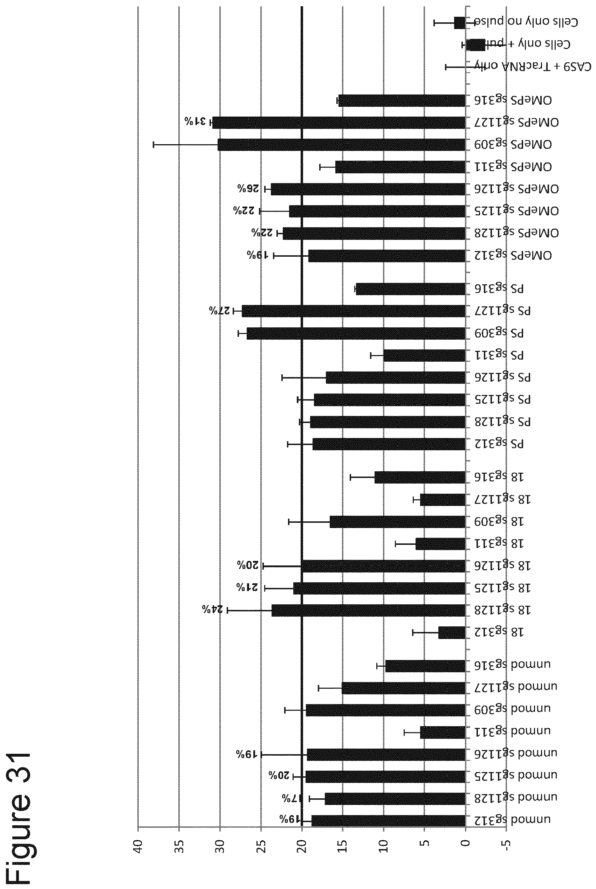

Figure 31. Normalized % Erythrocytes (% F cells in "Mock" subtracted) that are HbF+ after electroporation of CD34+ HSPCs with RNP comprising the indicated sgRNA (unmod or modified as indicated. In each case the number corresponds to the CRxxxxxx identifier of the targeting domain, for example, Unmod sg312 refers to unmodified sgRNA comprising the targeting domain of CR00312) and then in induced to differentiate by culture in erythroid differentiation medium. Controls include electroporation of Cas9 protein and tracr only ("Cas9 + TracRNA only"), electroporation with buffer only ("Cells only + Pulse"), or with no electroporation ("Cells only no pulse").

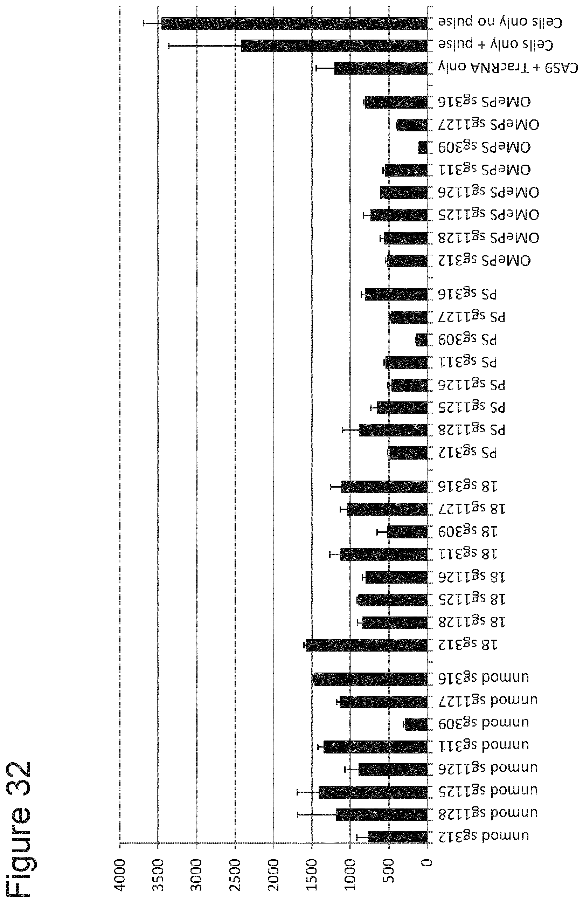

Figure 32. Fold total cell expansion in culture at day 14 after erythroid differentiation after electroporation of RNP comprising the indicated sgRNA (in each case, the number corresponds to the CRxxxxxx identifier of the targeting domain, for example, Unmod sg312 refers to unmodified sgRNA comprising the targeting domain of CR00312) into CD34+ cells. Controls include electroporation of Cas9 protein and tracr only ("Cas9 + TracRNA only"), electroporation with buffer only ("Cells only + Pulse"), or with no electroporation ("Cells only no pulse").

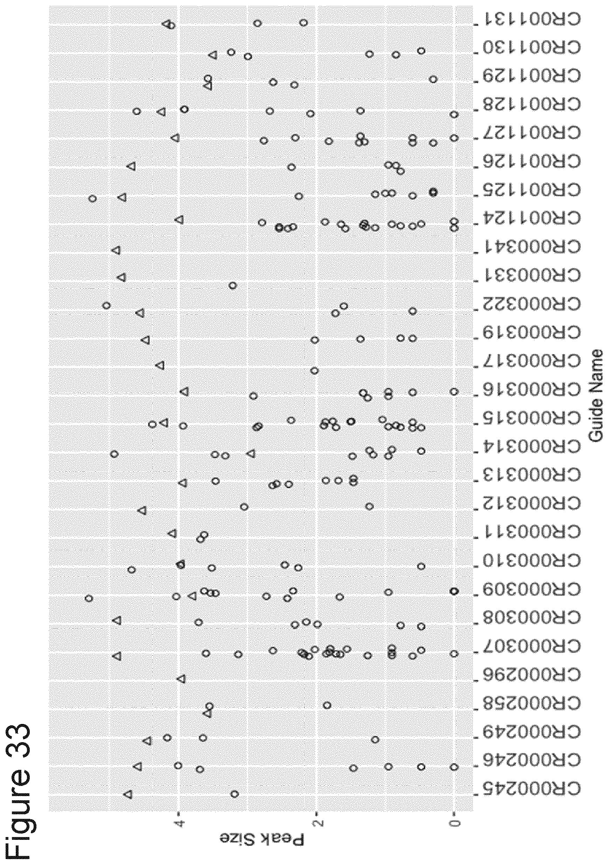

Figure 33. Assessment of potential off-target sites cleaved by Cas9 directed by dgRNA molecules targeting the BCL11a enhancer region. Triangles represent the on-target site while open circles represent a potential off-target site for each guide RNA tested.

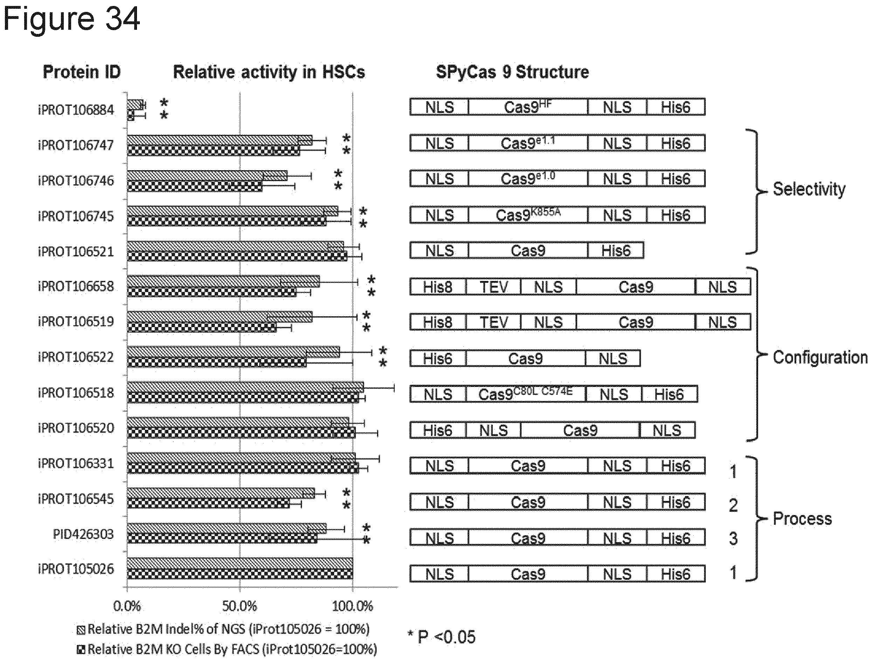

Figure 34. Editing efficiency at targeted B2M locus in CD34+ hematopoietic stem cells by different Cas9 variants, as evaluated by NGS and Flow cytometry. NLS = SV40 NLS; His6 or His8 refers to 6 or 8 histidine residues, respectively; TEV = tobacco etch virus cleavage site; Cas9 = wild type S. pyogenes Cas9 - mutations or variants are as indicated).

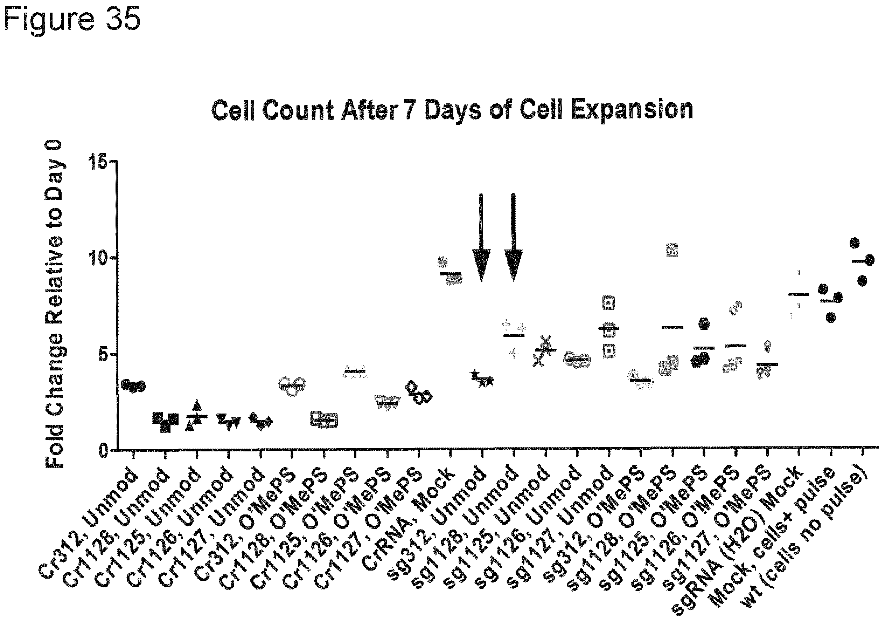

Figure 35. Data show fold increase in cell number after a total of 10 days of culturing in expansion medium (3 days of culturing prior to electroporation and 7 days of culturing following electroporation). With all the guide RNAs tested, including both single and dual guide RNA formats, cell expansion ranged from 2-7 fold. CR00312 and CR001128, indicated by arrows, demonstrated a 3-6 fold increase after 10 days of cell expansion. Lables "Crxxxx" refer to dgRNA having the indicated targeting domain; "sgxxxx" refer to sgRNA having the targeting domain of the CRxxxxx identifier with the same number (e.g., sg312 has the targeting domain of CR00312); "Unmod" indicates no modification to theRNA(s); "O'MePS", when used in relation to a dgRNA refers to a crRNA which has three 3' and three 5' 2'-OMe modifications and phosphorothioate bonds, paired with an unmodified tracr; "O'MePS", when used in relation to a sgRNA refers to gRNA which has three 5'-terminal 2'-OMe modifications and phosphorothioate bonds, three 3'-terminal phosphorothioate bonds, and three 2'OMe modifications of the 4^{th}-to-last, 3^{rd}-to-last and 2^{nd}-to-last 3' nucelotides.

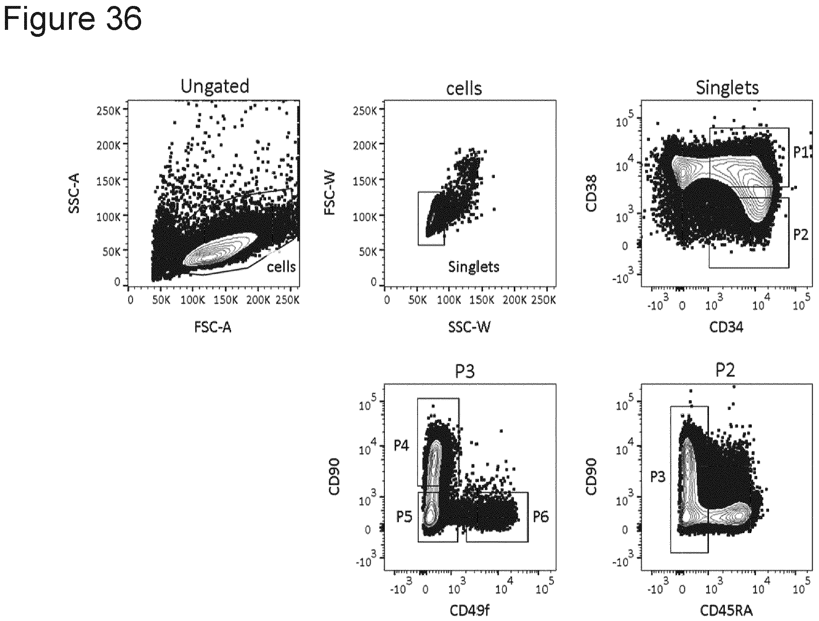

Figure 36. Gating strategy to distinguish different HSPC subpopulations.

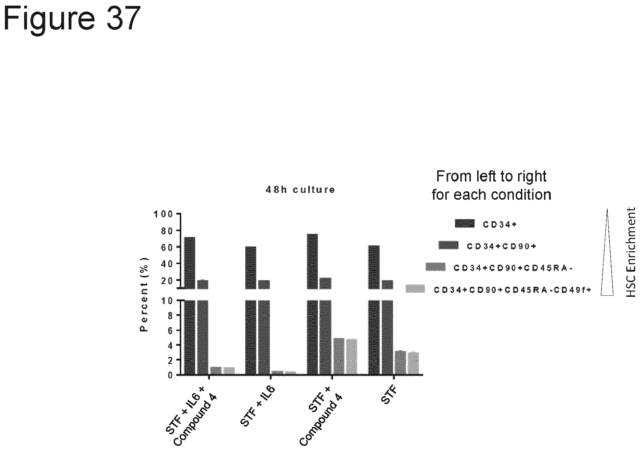

Figure 37. Frequency of each hematopoietic subset after 48 hours ex vivo culture in the indicated medium (STF modified with IL6, Compound 4, or both IL6 and Compound 4), but before electroporation of a CRISPR system. STF = StemSpan SFEM.

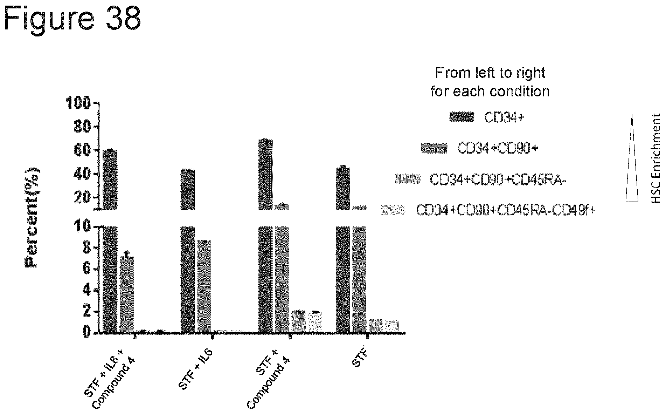

Figure 38. Frequency of each hematopoietic subset 7 days after electroporation introducing a CRISPR system targeting the +58 enhancer of BCL1 1a, cultured in the indicated medium (STF modified with IL6, Compound 4, or both IL6 and Compound 4). STF = StemSpan SFEM.

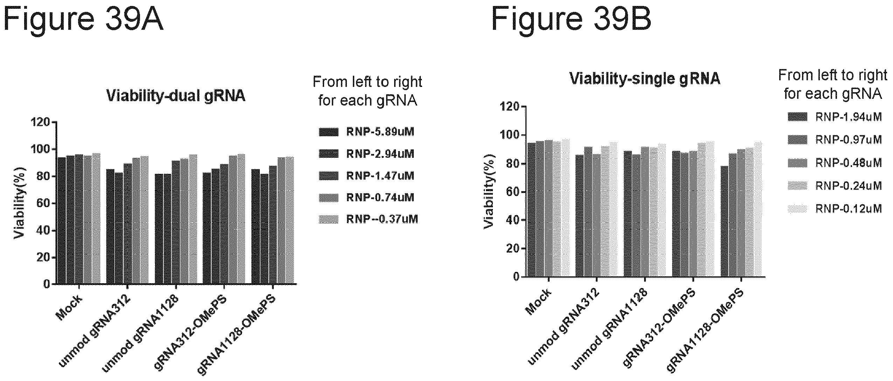

Figure 39A. Cell viability upon gene editing using different RNP concentrations, with RNP containing dgRNAs targeting the +58 region.
Figure 39B. Cell viability upon gene editing using different RNP concentrations, with RNP containing sgRNAs targeting the +58 region.

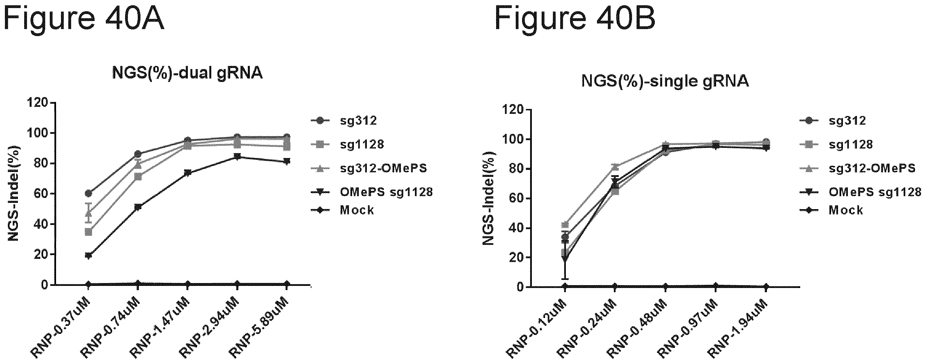

Figure 40A. Gene editing efficiency measured by NGS upon gene editing using different RNP concentrations, with RNP containing dgRNAs targeting the +58 region.
Figure 40B Gene editing efficiency measured by NGS upon gene editing using different RNP concentrations, with RNP containing sgRNAs targeting the +58 region.

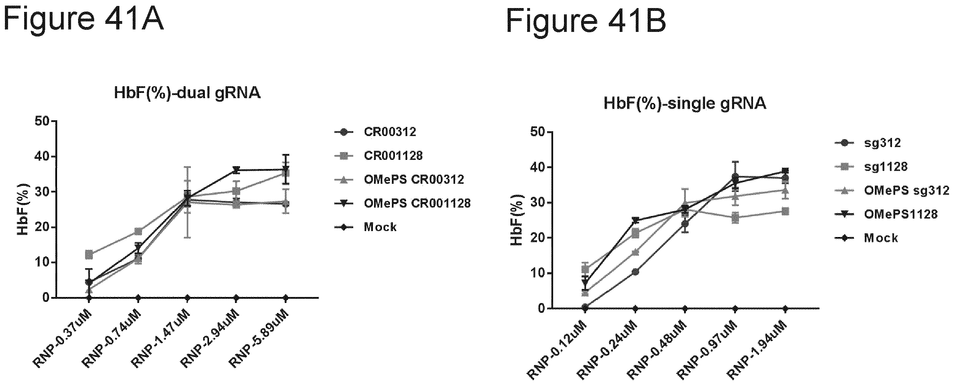

Figure 41A. Percentage of HbF induction measured by flow cytometry upon gene editing using different RNP concentrations, with RNP containing dgRNAs targeting the +58 region.
Figure 41B Percentage of HbF induction measured by flow cytometry upon gene editing using different RNP concentrations, with RNP containing sgRNAs targeting the +58 region.

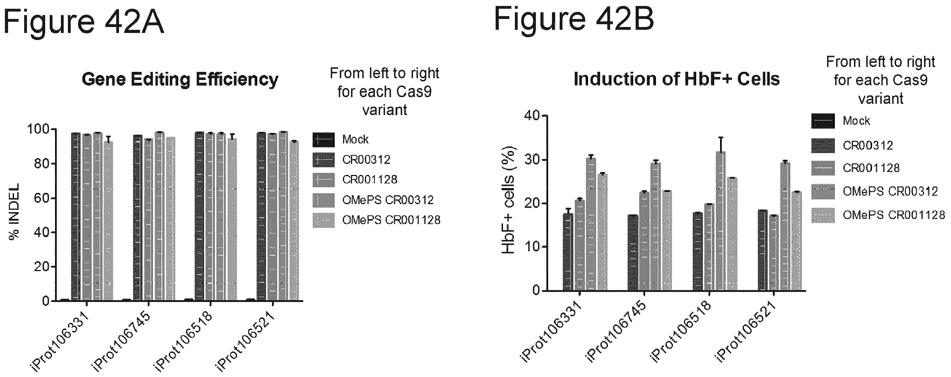

Figure 42A. Gene editing efficiencies of RNPs with different Cas9 proteins. Gene editing was performed using different Cas9 variants (listed on the X-axis) coupled with either the unmodified version of sgRNA CR00312 and sgRNA CR001128, or a modified version of sgRNA CR00312 and sgRNA CR001128. Edited cells were subjected to NGS to determine % edit (Y-axis).
Figure 42B. Induction of HbF+ cells upon gene editing using different Cas9 proteins. Gene editing was performed using different Cas9 variants (listed on the X-axis) coupled with either the unmodified version of sgRNA CR00312 and sgRNA CR001128, or a modified version of sgRNA CR00312 and sgRNA CR001128. Edited cells were differentiated into erythroid lineage and HbF production assessed by flow cytometry using an anti-HbF antibody conjugated with fluorophore.

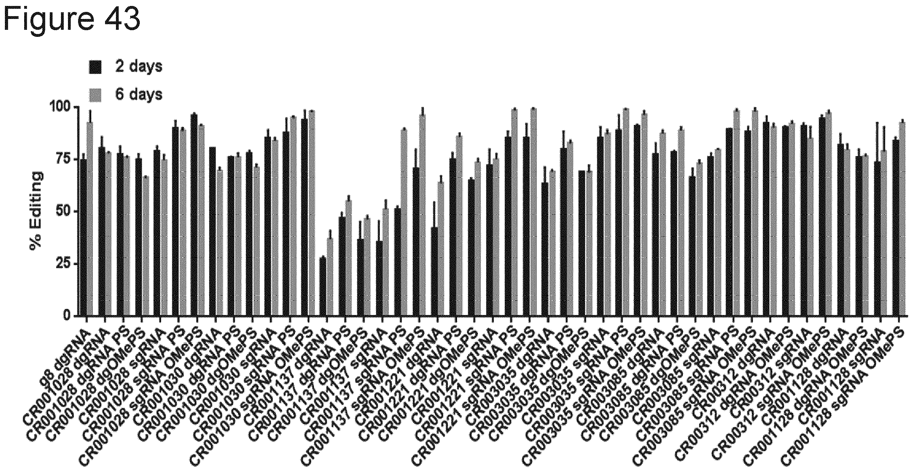

Figure 43. % Editing as determined by NGS in CD34+ HSPCs by electroporation of RNP comprising the indicated dgRNA or sgRNA (unmod or modified as indicated) (labels refer to the gRNA sequences as indicated in Table 36). Editing was determined at either 2 days (black bars) or 6 days (gray bars) post-electroporation. Less than 1.5% editing was detected at each site after control electroporation of Cas9 protein and Tracr only ("none", data not shown). Mean + standard deviation of 2 electroporation replicates is shown.

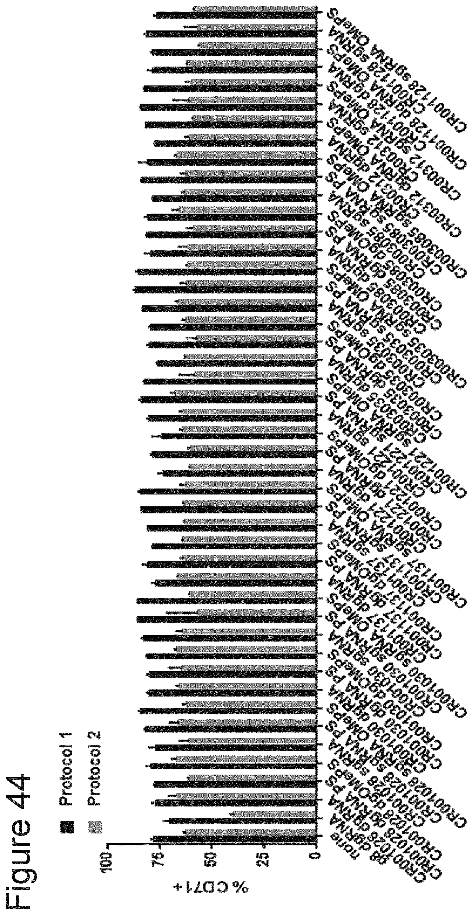

Figure 44. Percent of viable cells that are CD71+ after electroporation into CD34+ HSPCs of RNP comprising the indicated dgRNA or sgRNA (unmod or modified as indicated) (labels refer to the gRNA sequences as indicated in Table 36) and culture in erythroid differentiation conditions for 7 days. After electroporation, cells were maintained by Protocol 1 (black bars) or Protocol 2 (gray bars), as described in Example 4.7. Controls include electroporation of Cas9 protein and Tracr only ("none"). Mean + standard deviation of 2 electroporation replicates is shown.

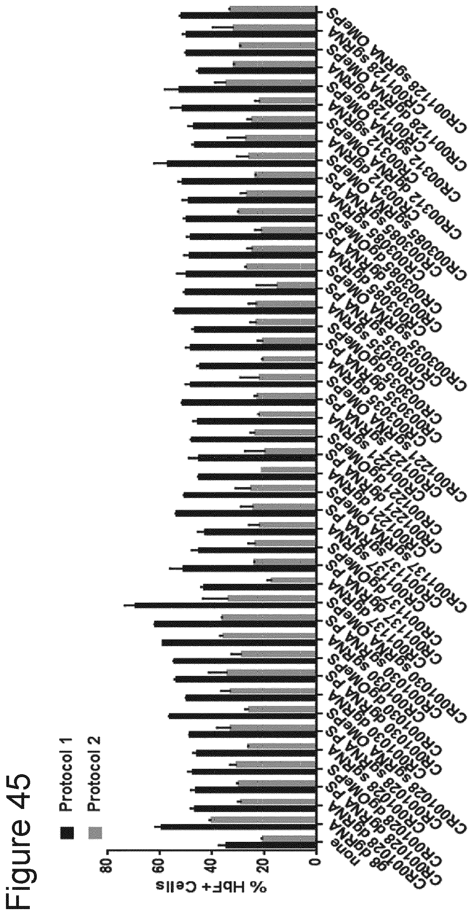

Figure 45. Percent of erythroid cells that are HbF+ after electroporation into CD34+ HSPCs of RNP comprising the indicated dgRNA or sgRNA (unmod or modified as indicated) (labels refer to the gRNA sequences as indicated in Table 36) and culture in erythroid differentiation conditions for 7 days. After electroporation, cells were maintained by Protocol 1 (black bars) or Protocol 2 (gray bars), as described in Example 4.7. Controls include electroporation of Cas9 protein and Tracr only ("none"). Mean + standard deviation of 2 electroporation replicates is shown.

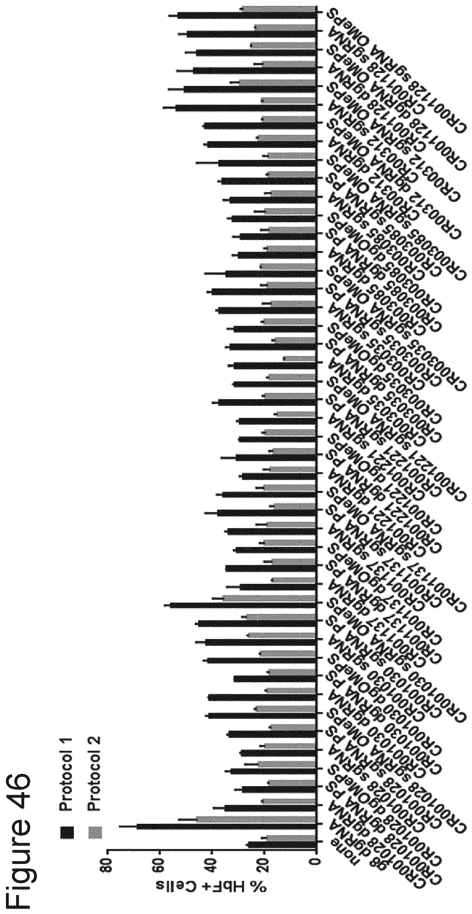

Figure 46. Percent of cells that are HbF+ after electroporation into CD34+ HSPCs of RNP comprising the indicated dgRNA or sgRNA (unmod or modified as indicated) (labels refer to the gRNA sequences as indicated in Table 36) and cultured in erythroid differentiation conditions for 14 days. After electroporation, cells were maintained by Protocol 1 (black bars) or Protocol 2 (gray bars), as described in Example 4.7. Controls include electroporation of Cas9 protein and Tracr only ("none"). Mean + standard deviation of 2 electroporation replicates is shown.

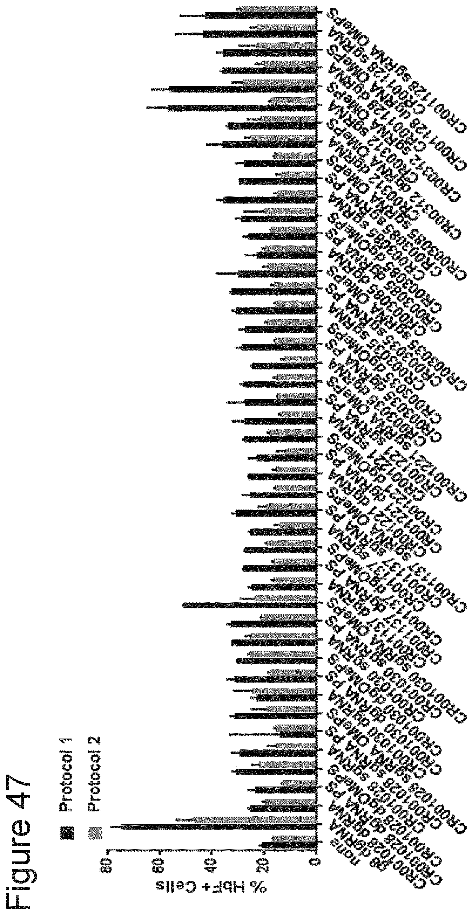

Figure 47. Percent of cells that are HbF+ after electroporation into CD34+ HSPCs of RNP comprising the indicated dgRNA or sgRNA (unmod or modified as indicated) (labels refer to the gRNA sequences as indicated in Table 36) and cultured in erythroid differentiation conditions for 21 days. After electroporation, cells were maintained by Protocol 1 (black bars) or Protocol 2 (gray bars), as described in Example 4.7. Controls include electroporation of Cas9 protein and Tracr only ("none"). Mean + standard deviation of 2 electroporation replicates is shown.

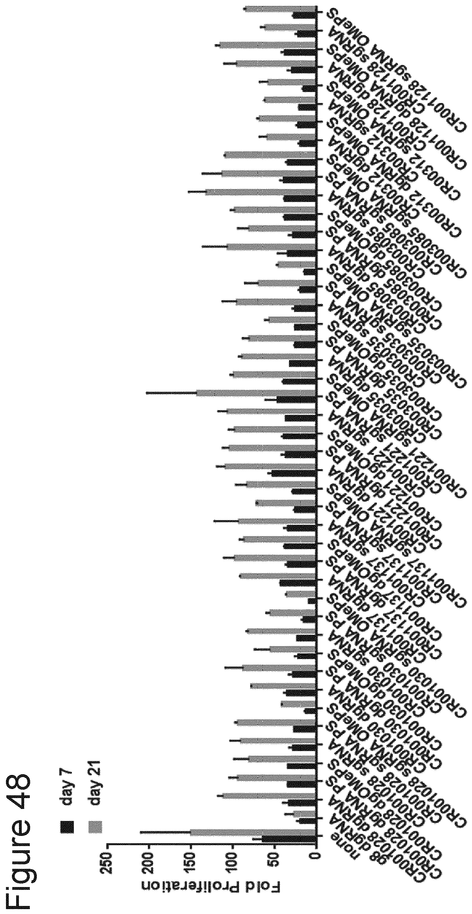

Figure 48. Fold total cell expansion in erythroid differentiation culture for 7 (black bars) or 21 days (black bars) after electroporation into CD34+ HSPCs of RNP comprising the indicated dgRNA or sgRNA (unmod or modified as indicated) (labels refer to the gRNA sequences as indicated in Table 36). After electroporation, cells were maintained by Protocol 2, as described in Example 4.7. Controls include electroporation of Cas9 protein and Tracr only ("none"). Mean + standard deviation of 2 electroporation replicates is shown.

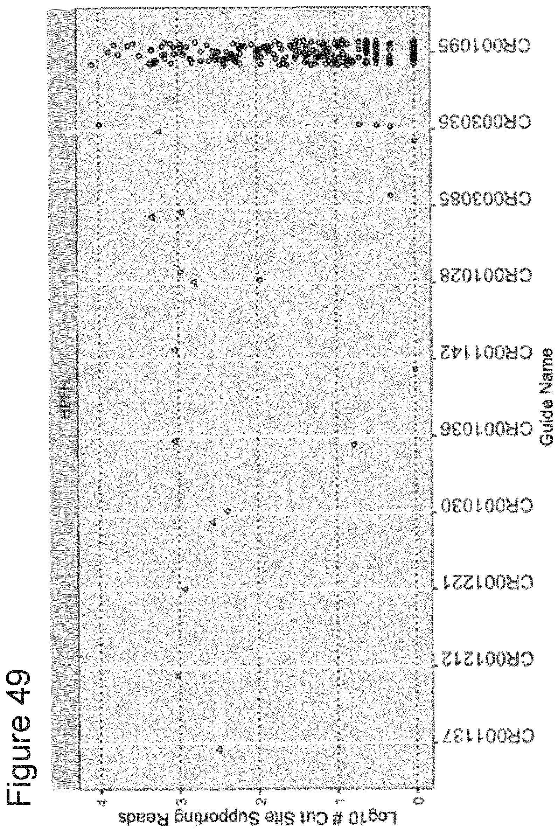

Figure 49. Assessment of potential off-target sites cleaved by Cas9 directed by dgRNA molecules targeting an HPFH region. Triangles represent the on-target site while open circles represent a potential off-target site for each guide RNA tested.

### DEFINITIONS

The terms "CRISPR system," "Cas system" or "CRISPR/Cas system" refer to a set of molecules comprising an RNA-guided nuclease or other effector molecule and a gRNA molecule that together are necessary and sufficient to direct and effect modification of nucleic acid at a target sequence by the RNA-guided nuclease or other effector molecule. In one embodiment, a CRISPR system comprises a gRNA and a Cas protein, e.g., a Cas9 protein. Such systems comprising a Cas9 or modified Cas9 molecule are referred to herein as "Cas9 systems" or "CRISPR/Cas9 systems." In one example, the gRNA molecule and Cas molecule may be complexed, to form a ribonuclear protein (RNP) complex.

The terms "guide RNA," "guide RNA molecule," "gRNA molecule" or "gRNA" are used interchangeably, and refer to a set of nucleic acid molecules that promote the specific directing of a RNA-guided nuclease or other effector molecule (typically in complex with the gRNA molecule) to a target sequence. In some embodiments, said directing is accomplished through hybridization of a portion of the gRNA to DNA (e.g., through the gRNA targeting domain), and by binding of a portion of the gRNA molecule to the RNA-guided nuclease or other effector molecule (e.g., through at least the gRNA tracr). In embodiments, a gRNA molecule consists of a single contiguous polynucleotide molecule, referred to herein as a "single guide RNA" or "sgRNA" and the like. In other embodiments, a gRNA molecule consists of a plurality, usually two, polynucleotide molecules, which are themselves capable of association, usually through hybridization, referred to herein as a "dual guide RNA" or "dgRNA," and the like. gRNA molecules are described in more detail below, but generally include a targeting domain and a tracr. In embodiments the targeting domain and tracr are disposed on a single polynucleotide. In other embodiments, the targeting domain and tracr are disposed on separate polynucleotides.

The term "targeting domain" as the term is used in connection with a gRNA, is the portion of the gRNA molecule that recognizes, e.g., is complementary to, a target sequence, e.g., a target sequence within the nucleic acid of a cell, e.g., within a gene.

The term "crRNA" as the term is used in connection with a gRNA molecule, is a portion of the gRNA molecule that comprises a targeting domain and a region that interacts with a tracr to form a flagpole region.

The term "target sequence" refers to a sequence of nucleic acids complimentary, for example fully complementary, to a gRNA targeting domain. In embodiments, the target sequence is disposed on genomic DNA. In an embodiment the target sequence is adjacent to (either on the same strand or on the complementary strand of DNA) a protospacer adjacent motif (PAM) sequence recognized by a protein having nuclease or other effector activity, e.g., a PAM sequence recognized by Cas9. In embodiments, the target sequence is a target sequence within a gene or locus that affects expression of a globin gene, e.g., that affects expression of beta globin or fetal hemoglobin (HbF). In embodiments, the target sequence is a target sequence within the globin locus. In embodiments, the target sequence is a target sequence within the BCL11a gene. In embodiments, the target sequence is a target sequence within the BCL11a ehancer region. In embodiments, the target sequence is a target sequence within a HPFH region.

The term "flagpole" as used herein in connection with a gRNA molecule, refers to the portion of the gRNA where the crRNA and the tracr bind to, or hybridize to, one another.

The term "tracr" as used herein in connection with a gRNA molecule, refers to the portion of the gRNA that binds to a nuclease or other effector molecule. In embodiements, the tracr comprises nucleic acid sequence that binds specifically to Cas9. In embodiments, the tracr comprises nucleic acid sequence that forms part of the flagpole.

The terms "Cas9" or "Cas9 molecule" refer to an enzyme from bacterial Type II CRISPR/Cas system responsible for DNA cleavage. Cas9 also includes wild-type protein as well as functional and non-functinal mutants thereof. In embodiments, the Cas9 is a Cas9 of *S. pyogenes.*

The term "complementary" as used in connection with nucleic acid, refers to the pairing of bases, A with T or U, and G with C. The term complementary refers to nucleic acid molecules that are completely complementary, that is, form A to T or U pairs and G to C pairs across the entire reference sequence, as well as molecules that are at least 80%, 85%, 90%, 95%, 99% complementary.

"Template Nucleic Acid" as used in connection with homology-directed repair or homologous recombination, refers to nucleic acid to be inserted at the site of modification by the CRISPR system donor sequence for gene repair (insertion) at site of cutting.

An "indel," as the term is used herein, refers to a nucleic acid comprising one or more insertions of nucleotides, one or more deletions of nucleotides, or a combination of insertions and delections of nucleotides, relative to a reference nucleic acid, that results after being exposed to a composition comprising a gRNA molecule, for example a CRISPR system. Indels can be determined by sequencing nucleic acid after being exposed to a composition comprising a gRNA molecule, for example, by NGS. With respect to the site of an indel, an indel is said to be "at or near" a reference site (e.g., a site complementary to a targeting domain of a gRNA molecule) if it comprises at least one insertion or deletion within about 10, 9 , 8, 7, 6, 5, 4, 3, 2, or 1 nucleotide(s) of the reference site, or is overlapping with part or all of said refrence site (e.g., comprises at least one insertion or deletion overlapping with, or within 10, 9, 8, 7, 6, 5, 4, 3, 2, or 1 nucelotides of a site complementary to the targeting domain of a gRNA molecule, e.g., a gRNA molecule described herein).

An "indel pattern," as the term is used herein, refers to a set of indels that results after exposure to a composition comprising a gRNA molecule. In an embodiment, the indel pattern consists of the top three indels, by frequency of appearance. In an embodiment, the indel pattern consists of the top five indels, by frequency of appearance. In an embodiment, the indel pattern consists of the indels which are present at greater than about 5% frequency relative to all sequencing reads. In an embodiment, the indel pattern consists of the indels which are present at greater than about 10% frequency relative to to total number of indel sequencing reads (i.e., those reads that do not consist of the unmodified reference nucleic acid sequence). In an embodiment, the indel pattern includes of any 3 of the top five most frequently observed indels. The indel pattern may be determined, for example, by sequencing cells of a population of cells which were exposed to the gRNA molecule.

An "off-target indel," as the term I used herein, refers to an indel at or near a site other than the target sequence of the targeting domain of the gRNA molecule. Such sites may comprise, for example, 1, 2, 3, 4, 5 or more mismatch nucleotides relative to the sequence of the targeting domain of the gRNA. In exemplary embodiments, such sites are detected using targeted sequencing of in silico predicted off-target sites, or by an insertional method known in the art.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be synthesized or can be derived from a biological sample, or might be macromolecule besides a polypeptide. Such a biological sample can include, but is not limited to a tissue sample, a cell or a fluid with other biological components.

The term "autologous" refers to any material derived from the same individual into whom it is later to be re-introduced.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically

The term "xenogeneic" refers to a graft derived from an animal of a different species.

"Derived from" as that term is used herein, indicates a relationship between a first and a second molecule. It generally refers to structural similarity between the first molecule and a second molecule and does not connotate or include a process or source limitation on a first molecule that is derived from a second molecule.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

As used herein in connection with a messenger RNA (mRNA), a 5' cap (also termed an RNA cap, an RNA 7-methylguanosine cap or an RNA m7G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In the preferred embodiment of a construct for transient expression, the polyA is between 50 and 5000 (SEQ ID NO: 6596), preferably greater than 64, more preferably greater than 100, most preferably greater than 300 or 400. poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein, the terms "treat", "treatment" and "treating" refer to the reduction or amelioration of the progression, severity and/or duration of a disorder, e.g., a hemoglobinopathy, or the amelioration of one or more symptoms (preferably, one or more discernible symptoms) of a disorder, e.g., a hemoglobinopathy, resulting from the administration of one or more therapies (e.g., one or more therapeutic agents such as a gRNA molecule, CRISPR system, or modified cell of the invention). In specific embodiments, the terms "treat", "treatment" and "treating" refer to the amelioration of at least one measurable physical parameter of a hemoglobinopathy disorder, not discernible by the patient. In other embodiments the terms "treat", "treatment" and "treating" refer to the inhibition of the progression of a disorder, either physically by, e.g., stabilization of a discernible symptom, physiologically by, e.g., stabilization of a physical parameter, or both. In other embodiments the terms "treat", "treatment" and "treating" refer to the reduction or stabilization of a symptom of a hemoglobinopathy, e.g., sickle cell disease or beta-thalassemia.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "therapeutic" as used herein means a treatment. A therapeutic effect is obtained by reduction, suppression, remission, or eradication of a disease state.

The term "prophylaxis" as used herein means the prevention of or protective treatment for a disease or disease state.

The term "transfected" or "transformed" or "transduced" refers to a process by which exogenous nucleic acid and/or ptotein is transferred or introduced into the host cell. A "transfected" or "transformed" or "transduced" cell is one which has been transfected, transformed or transduced with exogenous nucleic acid and/or protein. The cell includes the primary subject cell and its progeny.

The term "specifically binds," refers to a molecule recognizing and binding with a binding partner (e.g., a protein or nucleic acid) present in a sample, but which molecule does not substantially recognize or bind other molecules in the sample.

The term "bioequivalent" refers to an amount of an agent other than the reference compound, required to produce an effect equivalent to the effect produced by the reference dose or reference amount of the reference compound.

"Refractory" as used herein refers to a disease, e.g., a hemoglobinopathy, that does not respond to a treatment. In embodiments, a refractory hemoglobinopathy can be resistant to a treatment before or at the beginning of the treatment. In other embodiments, the refractory hemoglobinopathy can become resistant during a treatment. A refractory hemoglobinopathy is also called a resistant hemoglobinopathy.

"Relapsed" as used herein refers to the return of a disease (e.g., hemoglobinopathy) or the signs and symptoms of a disease such as a hemoglobinopathy after a period of improvement, e.g., after prior treatment of a therapy, e.g., hemoglobinopathy therapy.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

The term "BCL11a" refers to B-cell lymphoma/leukemia 11A, a RNA polymerase II core promoter proximal region sequence-sepecific DNA binding protein, and the gene encoding said protein, together with all introns and exons. This gene encodes a C2H2 type zinc-finger protein. BCL11A has been found to play a role in the suppression of fetal hemoglobin production. BCL11a is also known as B-Cell CLL/Lymphoma 11A (Zinc Finger Protein), CTIP1, EVI9, Ecotropic Viral Integration Site 9 Protein Homolog, COUP-TF-Interacting Protein 1, Zinc Finger Protein 856, KIAA1809, BCL-11A, ZNF856, EVI-9, and B-Cell CLL/Lymphoma 11A. The term encompasses all isoforms and splice variants of BLC11a. The human gene encoding BCL11a is mapped to chromosomal location 2p16.1 (by Ensembl). The human and murine amino acid and nucleic acid sequences can be found in a public database, such as GenBank, UniProt and Swiss-Prot., and the genomic sequence of human BCL11a can be found in GenBank at NC_000002.12. The BCL11a gene refers to this genomic location, including all introns and exons. There are multiple known isotypes of BCL11a.

The sequence of mRNA encoding isoform 1 of human BCL11a can be found at NM_022893.

The peptide sequence of isoform 1 of human BCL11a is: 
10 20 30 40 50

60 70 80 90 100

110 120 130 140 150

160 170 180 190 200

210 220 230 240 250

260 270 280 290 300

310 320 330 340 350

360 370 380 390 400

410 420 430 440 450

460 470 480 490 500

510 520 530 540 550

560 570 580 590 600

610 620 630 640 650

660 670 680 690 700

710 720 730 740 750

760 770 780 790 800

810 820 830

 SEQ ID NO: 21-1 (Identifier Q9H165-1; and NM_022893.3; and accession ADL14508.1).

The sequences of other BCL11a protein isoforms are provided at:
Isoform 2: Q9H165-2
Isoform 3: Q9H165-3
Isoform 4: Q9H165-4
Isoform 5: Q9H165-5
Isoform 6: Q9H165-6

As used herein, a human BCL11a protein also encompasses proteins that have over its full length at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity with BCL11a isolorm 1-6, wherein such proteins still have at least one of the functions of BCL11a.

The term "globin locus" as used herein refers to the region of human chromosome 11 comprising genes for embryonic (ε), fetal (G(γ) and A(γ)), adult globin genes (γ and β), locus control regions and DNase I hypersensitivity sites.

The term "complementary" as used in connection with nucleic acid, refers to the pairing of bases, A with T or U, and G with C. The term complementary refers to nucleic acid molecules that are completely complementary, that is, form A to T or U pairs and G to C pairs across the entire reference sequence, as well as molecules that are at least 80%, 85%, 90%, 95%, 99% complementary.

The term "HPFH" refers to hereditary persistence of fetal hemoglobin, and is characterized in increased fetal hemoglobin in adult red blood cells. The term "HPFH region" refers to a genomic site which, when modified (e.g., mutated or deleted), causes increased HbF production in adult red blood cells, and includes HPFH sites identified in the literature (see e.g., the Online Mendelian Inheritance in Man: http://www.omim.org/entry/141749). In an exemplary embodiment, the HPFH region is a region within or encompassing the beta globin gene cluster on chromosome 11p15. In an exemplary embodiment, the HPFH region is within or emcompasses at least part of the delta globin gene. In an exemplary embodiment, the HPFH region is a region of the promoter of HBG1. In an exemplary embodiment, the HPFH region is a region of the promoter of HBG1. In an exemplary embodiment, the HPFH region is a region described in Sankaran VG et al. NEJM (2011) 365:807-814. In an exemplary embodiment, the HPFH region is the French breakpoint deletional HPFH as described in Sankaran VG et al. NEJM (2011) 365:807-814. In an exemplary embodiment, the HPFH region is the Algerian HPFH as described in Sankaran VG et al. NEJM (2011) 365:807-814. In an exemplary embodiment, the HPFH region is the Sri Lankan HPFH as described in Sankaran VG et al. NEJM (2011) 365:807-814. In an exemplary embodiment, the HPFH region is the HPFH-3 as described in Sankaran VG et al. NEJM (2011) 365:807-814. In an exemplary embodiment, the HPFH region is the HPFH-2 as described in Sankaran VG et al. NEJM (2011) 365:807-814. In an embodiment, the HPFH-1 region is the HPFH-3 as described in Sankaran VG et al. NEJM (2011) 365:807-814. In an exemplary embodiment, the HPFH region is the Sri Lankan (δβ)⁰-thalassemia HPFH as described in Sankaran VG et al. NEJM (2011) 365:807-814. In an exemplary embodiment, the HPFH region is the Sicilian (δβ)⁰-thalassemia HPFH as described in Sankaran VG et al. NEJM (2011) 365:807-814. In an exemplary embodiment, the HPFH region is the Macedonian (δβ)⁰-thalassemia HPFH as described in Sankaran VG et al. NEJM (2011) 365:807-814. In an exemplary embodiment, the HPFH region is the Kurdish β⁰-thalassemia HPFH as described in Sankaran VG et al. NEJM (2011) 365:807-814. In an exemplary embodiment, the HPFH region is the region located at Chr11:5213874-5214400 (hg18). In an exemplary embodiment, the HPFH region is the region located at Chr11:5215943-5215046 (hg18). In an exemplary embodiment, the HPFH region is the region located at Chr11:5234390-5238486 (hg38).

"BCL11a enhancer" as the term is used herein, refers to nucleic acid sequence which affects, e.g., enhances, expression or function of BCL11a. See e.g., Bauer et al., Science, vol. 342, 2013, pp. 253-257. The BCL11a enhancer may be, for example, operative only in certain cell types, for example, cells of the erythroid lineage. One example of a BCL11a enhancer is the nucleic acid sequence between exon 2 and exon 3 of the BCL11a gene gene (e.g., the nucleic acid at or corresponding to positions +55: Chr2:60497676- 60498941; +58: Chr2:60494251- 60495546; +62: Chr2:60490409-60491734 as recorded in hg38). In an embodiment, the BCL11a Enhancer is the +62 region of the nucleic acid sequence between exon 2 and exon 3 of the BCL11a gene. In an embodiment, the BCL11a Enhancer is the +58 region of the nucleic acid sequence between exon 2 and exon 3 of the BCL11a gene. In an embodiment, the BCL11a Enhancer is the +55 region of the nucleic acid sequence between exon 2 and exon 3 of the BCL11a gene.

The terms "hematopoietic stem and progenitor cell" or "HSPC" are used interchangeably, and refer to a population of cells comprising both hematopoietic stem cells ("HSCs") and hematopoietic progenitor cells ("HPCs"). Such cells are characterized, for example, as CD34+. In exemplary embodiments, HSPCs are isolated from bone marrow. In other exemplary embodiments, HSPCs are isolated from peripheral blood. In other exemplary embodiments, HSPCs are isolated from umbilical cord blood.

"Stem cell expander" as used herein refers to a compound which causes cells, e.g., HSPCs, HSCs and/or HPCs to proliferate, e.g., increase in number, at a faster rate relative to the same cell types absent said agent. In one exemplary aspect, the stem cell expander is an inhibitor of the aryl hydrocarbon receptor pathway. Additional examples of stem cell expanders are provided below. In embodiments, the proliferation, e.g., increase in number, is accomplished *ex vivo.*

"Engraftment" or "engraft" refers to the incorporation of a cell or tissue, e.g., a population of HSPCs, into the body of a recipient, e.g., a mammal or human subject. In one example, engraftment includes the growth, expansion and/or differention of the engrafted cells in the recipient. In an example, engraftment of HSPCs includes the differentiation and growth of said HSPCs into erythroid cells within the body of the recipient.

The term "Hematopoietic progenitor cells" (HPCs) as used herein refers to primitive hematopoietic cells that have a limited capacity for self-renewal and the potential for multilineage differentiation (e.g., myeloid, lymphoid), mono-lineage differentiation (e.g., myeloid or lymphoid) or cell-type restricted differentiation (e.g., erythroid progenitor) depending on placement within the hematopoietic hierarchy (Doulatov et al., Cell Stem Cell 2012).

"Hematopoietic stem cells" (HSCs) as used herein refer to immature blood cells having the capacity to self-renew and to differentiate into more mature blood cells comprising granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), and monocytes (e.g., monocytes, macrophages). HSCs are interchangeably described as stem cells throughout the specification. It is known in the art that such cells may or may not include CD34+ cells. CD34+ cells are immature cells that express the CD34 cell surface marker. CD34+ cells are believed to include a subpopulation of cells with the stem cell properties defined above. It is well known in the art that HSCs are multipotent cells that can give rise to primitive progenitor cells (e.g., multipotent progenitor cells) and/or progenitor cells committed to specific hematopoietic lineages (e.g., lymphoid progenitor cells). The stem cells committed to specific hematopoietic lineages may be of T cell lineage, B cell lineage, dendritic cell lineage, Langerhans cell lineage and/or lymphoid tissue-specific macrophage cell lineage. In addition, HSCs also refer to long term HSC (LT-HSC) and short term HSC (ST-HSC). ST-HSCs are more active and more proliferative than LT-HSCs. However, LT-HSC have unlimited self renewal (i.e., they survive throughout adulthood), whereas ST-HSC have limited self renewal (i.e., they survive for only a limited period of time). Any of these HSCs can be used in any of the methods described herein. Optionally, ST-HSCs are useful because they are highly proliferative and thus, quickly increase the number of HSCs and their progeny. Hematopoietic stem cells are optionally obtained from blood products. A blood product includes a product obtained from the body or an organ of the body containing cells of hematopoietic origin. Such sources include un-fractionated bone marrow, umbilical cord, peripheral blood (e.g., mobilized peripheral blood, e.g., moblized with a mobilization agent such as G-CSF or Plerixafor^{®} (AMD3100)), liver, thymus, lymph and spleen. All of the aforementioned crude or un-fractionated blood products can be enriched for cells having hematopoietic stem cell characteristics in ways known to those of skill in the art. In an embodiment, HSCs are characterized as CD34+/CD38-/CD90+/CD45RA-. In embodiments, the HSC s are characterized as CD34+/CD90+/CD49f+ cells.

"Expansion" or "Expand" in the context of cells refers to an increase in the number of a characteristic cell type, or cell types, from an initial cell population of cells, which may or may not be identical. The initial cells used for expansion may not be the same as the cells generated from expansion.

"Cell population" refers to eukaryotic mammalian, preferably human, cells isolated from biological sources, for example, blood product or tissues and derived from more than one cell.

"Enriched" when used in the context of cell population refers to a cell population selected based on the presence of one or more markers, for example, CD34+.

The term "CD34+ cells" refers to cells that express at their surface CD34 marker. CD34+ cells can be detected and counted using for example flow cytometry and fluorescently labeled anti-CD34 antibodies.

"Enriched in CD34+ cells" means that a cell population has been selected based on the presence of CD34 marker. Accordingly, the percentage of CD34+ cells in the cell population after selection method is higher than the percentage of CD34+ cells in the initial cell population before selecting step based on CD34 markers. For example, CD34+ cells may represent at least 50%, 60%, 70%, 80% or at least 90% of the cells in a cell population enriched in CD34+ cells.

The terms "F cell" and "F-cell" refer to cells, ususally erythrocytes (e.g., red blood cells) which contain and/or produce (e.g., express) fetal hemoglobin. For example, an F-cell is a cell that contains or produces detectible levels of fetal hemoglobin. For example, an F-cell is a cell that contains or produces at least 5 picograms of fetal hemoglobin. In another example, an F-cell is a cell that contains or produces at least 6 picograms of fetal hemoglobin. In another example, an F-cell is a cell that contains or produces at least 7 picograms of fetal hemoglobin. In another example, an F-cell is a cell that contains or produces at least 8 picograms of fetal hemoglobin. In another example, an F-cell is a cell that contains or produces at least 9 picograms of fetal hemoglobin. In another example, an F-cell is a cell that contains or produces at least 10 picograms of fetal hemoglobin. Levels of fetal hemoglobin may be measured using an assay described herein or by other method known in the art, for example, flow cytometry using an anti-fetal hemoglobin detection reagent, high performance liquid chromatography, mass spectrometry, or enzyme-linked immunoabsorbent assay.

Unless otherwise stated, all genome or chromosome coordinates are are according to hg38.

### DETAILED DESCRIPTION

The gRNA molecules, compositions and methods described herein relate to genome editing in eukaryotic cells using the CRISPR/Cas9 system. In particular, the gRNA molecules, compositions and methods described herein relate to regulation of globin levels and are useful, for example, in regulating expression and production of globin genes and protein. The gRNA molecules, compositions and methods can be useful in the treatment of hemoglobinopathies.

### I. gRNA Molecules

A gRNA molecule may have a number of domains, as described more fully below, however, a gRNA molecule typically comprises at least a crRNA domain (comprising a targeting domain) and a tracr. The gRNA molecules of the invention, used as a component of a CRISPR system, are useful for modifying (e.g., modifying the sequence) DNA at or near a target site. Such modifications include deletions and or insertions that result in, for example, reduced or eliminated expression of a functional product of the gene comprising the target site. These uses, and additional uses, are described more fully below.

In an embodiment, a unimolecular, or sgRNA comprises, preferably from 5' to 3' : a crRNA (which contains a targeting domain complementary to a target sequence and a region that forms part of a flagpole (i.e., a crRNA flagpole region)); a loop; and a tracr (which contains a domain complementary to the crRNA flagpole region, and a domain which additionally binds a nuclease or other effector molecule, e.g., a Cas molecule, e.g., aCas9 molecule), and may take the following format (from 5' to 3'):
[targeting domain] - [crRNA flagpole region] - [optional first flagpole extension] - [loop] - [optional first tracr extension] - [tracr flagpole region] - [tracr nuclease binding domain].

In embodiments, the tracr nuclease binding domain binds to a Cas protein, e.g., a Cas9 protein.

In an embodiment, a bimolecular, or dgRNA comprises two polynucleotides; the first, preferably from 5' to 3': a crRNA (which contains a targeting domain complementary to a target sequence and a region that forms part of a flagpole; and the second, preferrably from 5' to 3': a tracr (which contains a domain complementary to the crRNA flagpole region, and a domain which additionally binds a nuclease or other effector molecule, e.g., a Cas molecule, e.g., Cas9 molecule), and may take the following format (from 5' to 3'):
Polynucleotide 1 (crRNA): [targeting domain] - [crRNA flagpole region] - [optional first flagpole extension] - [optional second flagpole extension]
Polynucleotide 2 (tracr): [optional first tracr extension] - [tracr flagpole region] - [tracr nuclease binding domain]

In embodiments, the tracr nuclease binding domain binds to a Cas protein, e.g., a Cas9 protein.

In some aspects, the targeting domain comprises or consists of a targeting domain sequence described herein, e.g., a targeting domain described in Table 1, 2, 3, 4, 5, or 6, or a targeting domain comprising or consisting of 17, 18, 19, 20, 21, 22, 23, 24, or 25 (preferably 20) consecutive nucleotides of a targeting domain sequence described in Table 1, 2, 3, 4, 5, or 6.

In some aspects, the flagpole, e.g., the crRNA flagpole region, comprises, from 5' to 3': GUUUUAGAGCUA (SEQ ID NO: 6584).

In some aspects, the flagpole, e.g., the crRNA flagpole region, comprises, from 5' to 3': GUUUAAGAGCUA (SEQ ID NO: 6585).

In some aspects the loop comprises, from 5' to 3': GAAA (SEQ ID NO: 6588).

In some aspects the tracr comprises, from 5' to 3':
UAGCAAGUUAAAAUAAGGCUAGUCCGUUAUCAACUUGAAAAAGUGGCACCGAGUCGG UGC (SEQ ID NO: 6589) and is preferably used in a gRNA molecule comprising SEQ ID NO 6584.

In some aspects the tracr comprises, from 5' to 3':
UAGCAAGUUUAAAUAAGGCUAGUCCGUUAUCAACUUGAAAAAGUGGCACCGAGUCGG UGC (SEQ ID NO: 6590) and is preferably used in a gRNA molecule comprising SEQ ID NO 6585.

In some aspects, the gRNA may also comprise, at the 3' end, additional U nucleic acids. For example the gRNA may comprise an additional 1, 2, 3, 4, 5, 6, 7, 8, 9, or 10 U nucleic acids at the 3' end. In an embodiment, the gRNA comprises an additional 4 U nucleic acids at the 3' end. In the case of dgRNA, one or more of the polynucleotides of the dgRNA (e.g., the polynucleotide comprising the targeting domain and the polynucleotide comprising the tracr) may comprise, at the 3' end, additional U nucleic acids. For example, the case of dgRNA, one or more of the polynucleotides of the dgRNA (e.g., the polynucleotide comprising the targeting domain and the polynucleotide comprising the tracr) may comprise an additional 1, 2, 3, 4, 5, 6, 7, 8, 9, or 10 U nucleic acids at the 3' end. In an embodiment, in the case of dgRNA, one or more of the polynucleotides of the dgRNA (e.g., the polynucleotide comprising the targeting domain and the polynucleotide comprising the tracr) comprises an additional 4 U nucleic acids at the 3' end. In an embodiment of a dgRNA, only the polynucleotide comprising the tracr comprises the additional U nucleic acid(s), e.g., 4 U nucleic acids. In an emebodiment of a dgRNA, only the polynucleotide comprising the targeting domain comprises the additional U nucleic acid(s). In an embodiment of a dgRNA, both the polynucleotide comprising the targeting domain and the polynucleotide comprising the tracr comprise the additional U nucleic acids, e.g., 4 U nucleic acids.

In some aspects, the gRNA may also comprise, at the 3' end, additional A nucleic acids. For example the gRNA may comprise an additional 1, 2, 3, 4, 5, 6, 7, 8, 9, or 10 A nucleic acids at the 3' end. In an embodiment, the gRNA comprises an additional 4 A nucleic acids at the 3' end. In the case of dgRNA, one or more of the polynucleotides of the dgRNA (e.g., the polynucleotide comprising the targeting domain and the polynucleotide comprising the tracr) may comprise, at the 3' end, additional A nucleic acids. For example, the case of dgRNA, one or more of the polynucleotides of the dgRNA (e.g., the polynucleotide comprising the targeting domain and the polynucleotide comprising the tracr) may comprise an additional 1, 2, 3, 4, 5, 6, 7, 8, 9, or 10 A nucleic acids at the 3' end. In an embodiment, in the case of dgRNA, one or more of the polynucleotides of the dgRNA (e.g., the polynucleotide comprising the targeting domain and the polynucleotide comprising the tracr) comprises an additional 4 A nucleic acids at the 3' end. In an embodiment of a dgRNA, only the polynucleotide comprising the tracr comprises the additional A nucleic acid(s), e.g., 4 A nucleic acids. In an emebodiment of a dgRNA, only the polynucleotide comprising the targeting domain comprises the additional A nucleic acid(s). In an embodiment of a dgRNA, both the polynucleotide comprising the targeting domain and the polynucleotide comprising the tracr comprise the additional U nucleic acids, e.g., 4 A nucleic acids.

In embodiments, one or more of the polynucleotides of the gRNA molecule may comprise a cap at the 5' end.

In an embodiment, a unimolecular, or sgRNA comprises, preferably from 5' to 3': a crRNA (which contains a targeting domain complementary to a target sequence; a crRNA flagpole region; first flagpole extension; a loop; a first tracr extension (which contains a domain complementary to at least a portion of the first flagpole extension); and a tracr (which contains a domain complementary to the crRNA flagpole region, and a domain which additionally binds a Cas9 molecule). In some aspects, the targeting domain comprises a targeting domain sequence described herein, e.g., a targeting domain described in Table 1, 2, 3, 4, 5, or 6, or a targeting domain comprising or consisting of 17, 18, 19, 20, 21, 22, 23, 24, or 25 (preferably 20) consecutive nucleotides of a targeting domain sequence described in Table 1, 2, 3, 4, 5, or 6, for example the 3' 17, 18, 19, 20, 21, 22, 23, 24 or 25 (preferably 20) consecutive nucleotides of a targeting domain sequence described in Table 1, 2, 3, 4, 5, or 6.

In aspects comprising a first flagpole extension and/or a first tracr extension, the flagpole, loop and tracr sequences may be as described above. In general any first flagpole extension and first tracr extension may be employed, provided that they are complementary. In embodiments, the first flagpole extension and first tracr extension consist of 3, 4, 5, 6, 7, 8, 9, 10 or more complementary nucleotides.

In some aspects, the first flagpole extension comprises, from 5' to 3': UGCUG (SEQ ID NO: 6586). In some aspects, the first flagpole extension consists of SEQ ID NO: 6586.

In some aspects, the first tracr extension comprises, from 5' to 3': CAGCA (SEQ ID NO: 6591). In some aspects, the first tracr extension consists of SEQ ID NO: 6591.

In an embodiment, a dgRNA comprises two nucleic acid molecules. In some aspects, the dgRNA comprises a first nucleic acid which contains, preferably from 5' to 3' : a targeting domain complementary to a target sequence; a crRNA flagpole region; optionally a first flagpole extension; and, optionally, a second flagpole extension; and a second nucleic acid (which may be referred to herein as a tracr), and comprises at least a domain which binds a Cas molecule, e.g., a Cas9 molecule) comprising preferably from 5' to 3': optionally a first tracr extension; and a tracr (which contains a domain complementary to the crRNA flagpole region, and a domain which additionally binds a Cas, e.g., Cas9, molecule). The second nucleic acid may additionally comprise, at the 3' end (e.g., 3' to the tracr) additional U nucleic acids. For example the tracr may comprise an additional 1, 2, 3, 4, 5, 6, 7, 8, 9, or 10 U nucleic acids at the 3' end (e.g., 3' to the tracr). The second nucleic acid may additionally or alternately comprise, at the 3' end (e.g., 3' to the tracr) additional A nucleic acids. For example the tracr may comprise an additional 1, 2, 3, 4, 5, 6, 7, 8, 9, or 10 A nucleic acids at the 3' end (e.g., 3' to the tracr). In some aspects, the targeting domain comprises a targeting domain sequence described herein, e.g., a targeting domain described in Table 1, 2, 3, 4, 5, or 6, or a targeting domain comprising or consisting of 17, 18, 19, 20, 21, 22, 23, 24, or 25 (preferably 20) consecutive nucleotides of a targeting domain sequence described in Table 1, 2, 3, 4, 5, or 6.

In aspects involving a dgRNA, the crRNA flagpole region, optional first flagpole extension, optional first tracr extension and tracr sequences may be as described above.

In some aspects, the optional second flagpole extension comprises, from 5' to 3': UUUUG (SEQ ID NO: 6587).

In embodiments, the 3' 1, 2, 3, 4, or 5 nucleotides, the 5' 1, 2, 3, 4, or 5 nucleotides, or both the 3' and 5' 1, 2, 3, 4, or 5 nucleotides of the gRNA molecule (and in the case of a dgRNA molecule, the polynucleotide comprising the targeting domain and/or the polynucleotide comprising the tracr) are modified nucleic acids, as described more fully in section XIII, below.

The domains are discussed briefly below:
1) The Targeting Domain:
   Guidance on the selection of targeting domains can be found, e.g., in Fu Y el al. NAT BIOTECHNOL 2014 (doi: 10.1038/nbt.2808) and Sternberg SH el al. NATURE 2014 (doi: 10.1038/naturel3011).

The targeting domain comprises a nucleotide sequence that is complementary, e.g., at least 80, 85, 90, 95, or 99% complementary, e.g., fully complementary, to the target sequence on the target nucleic acid. The targeting domain is part of an RNA molecule and will therefore comprise the base uracil (U), while any DNA encoding the gRNA molecule will comprise the base thymine (T). While not wishing to be bound by theory, it is believed that the complementarity of the targeting domain with the target sequence contributes to specificity of the interaction of the gRNA molecule/Cas9 molecule complex with a target nucleic acid. It is understood that in a targeting domain and target sequence pair, the uracil bases in the targeting domain will pair with the adenine bases in the target sequence.

In an embodiment, the targeting domain is 5 to 50, e.g., 10 to 40, e.g., 10 to 30, e.g., 15 to 30, e.g., 15 to 25 nucleotides in length. In an embodiment, the targeting domain is 15, 16, 17, 18, 19, 20, 21, 22, 23, 24 or 25 nucleotides in length. In an embodiment, the targeting domain is 16 nucleotides in length. In an embodiment, the targeting domain is 17 nucleotides in length. In an embodiment, the targeting domain is 18 nucleotides in length. In an embodiment, the targeting domain is 19 nucleotides in length. In an embodiment, the targeting domain is 20 nucleotides in length. In an embodiment, the targeting domain is 21 nucleotides in length. In an embodiment, the targeting domain is 22 nucleotides in length. In an embodiment, the targeting domain is 23 nucleotides in length. In an embodiment, the targeting domain is 24 nucleotides in length. In an embodiment, the targeting domain is 25 nucleotides in length. In embodiments, the aforementioned 16, 17, 18, 19, 20, 21, 22, 23, 24, or 25 nucleotides comprise the 5'- 16, 17, 18, 19, 20, 21, 22, 23, 24, or 25 nucleotides from a targeting domain described in Table 1, 2, 3, 4, 5, or 6. In embodiments, the aforementioned 16, 17, 18, 19, 20, 21, 22, 23, 24, or 25 nucleotides comprise the 3'- 16, 17, 18, 19, 20, 21, 22, 23, 24, or 25 nucleotides from a targeting domain described in Table 1, 2, 3, 4, 5, or 6.

Without being bound by theory, it is believed that the 8, 9, 10, 11 or 12 nucleic acids of the targeting domain disposed at the 3' end of the targeting domain is important for targeting the target sequence, and may thus be referred to as the "core" region of the targeting domain. In an embodiment, the core domain is fully complementary with the target sequence.

The strand of the target nucleic acid with which the targeting domain is complementary is referred to herein as the target sequence. In some aspects, the target sequence is disposed on a chromosome, e.g., is a target within a gene. In some aspects the target sequence is disposed within an exon of a gene. In some aspects the target sequence is disposed within an intron of a gene. In some aspects, the target sequence comprises, or is proximal (e.g., within 10, 20, 30, 40, 50, 100, 200, 300, 400, 500, or 1000 nucleic acids) to a binding site of a regulatory element, e.g., a promoter or transcription factor binding site, of a gene of interest. Some or all of the nucleotides of the domain can have a modification, e.g., modification found in Section XIII herein.

### 2) crRNA Flagpole Region:

The flagpole contains portions from both the crRNA and the tracr. The crRNA flagpole region is complementary with a portion of the tracr, and in an embodiment, has sufficient complementarity to a portion of the tracr to form a duplexed region under at least some physiological conditions, for example, normal physiological conditions. In an embodiment, the crRNA flagpole region is 5 to 30 nucleotides in length. In an embodiment, the crRNA flagpole region is 5 to 25 nucleotides in length. The crRNA flagpole region can share homology with, or be derived from, a naturally occurring portion of the repeat sequence from a bacterial CRISPR array. In an embodiment, it has at least 50% homology with a crRNA flagpole region disclosed herein, e.g., an S. pyogenes, or S. thermophilus, crRNA flagpole region.

In an embodiment, the flagpole, e.g., the crRNA flagpole region, comprises SEQ ID NO: 6584. In an embodiment, the flagpole, e.g., the crRNA flagpole region, comprises sequence having at least 50%, 60%, 70%, 80%, 85%, 90%, 95% or 99% homology with SEQ ID NO: 6584. In an embodiment, the flagpole, e.g., the crRNA flagpole region, comprises at least 5, 6, 7, 8, 9, 10, or 11 nucleotides of SEQ ID NO: 6584. In an embodiment, the flagpole, e.g., the crRNA flagpole region, comprises SEQ ID NO: 6585. In an embodiment, the flagpole comprises sequence having at least 50%, 60%, 70%, 80%, 85%, 90%, 95% or 99% homology with SEQ ID NO: 6585. In an embodiment, the flagpole, e.g., the crRNA flagpole region, comprises at least 5, 6, 7, 8, 9, 10, or 11 nucleotides of SEQ ID NO: 6585.

Some or all of the nucleotides of the domain can have a modification, e.g., modification described in Section XIII herein.

### 3) First Flagpole Extension

When a tracr comprising a first tracr extension is used, the crRNA may comprise a first flagpole extension. In general any first flagpole extension and first tracr extension may be employed, provided that they are complementary. In embodiments, the first flagpole extension and first tracr extension consist of 3, 4, 5, 6, 7, 8, 9, 10 or more complementary nucleotides.

The first flagpole extension may comprise nucleotides that are complementary, e.g., 80%, 85%, 90%, 95% or 99%, e.g., fully complementary, with nucleotides of the first tracr extension. In some aspects, the first flagpole extension nucleotides that hybridize with complementary nucleotides of the first tracr extension are contiguous. In some aspects, the first flagpole extension nucleotides that hybridize with complementary nucleotides of the first tracr extension are discontinuous, e.g., comprises two or more regions of hybridization separated by nucleotides that do not base pair with nucleotides of the first tracr extension. In some aspects, the first flagpole extension comprises at least 2, 3, 4, 5, 6, 7, 8, 9, 10, 11, 12, 13, 14, 15, 16, 17, 18, 19, 20 or more nucleotides. In some aspects, the first flagpole extension comprises, from 5' to 3': UGCUG (SEQ ID NO: 6586). In some aspects, the first flagpole extension consists of SEQ ID NO: 6586. In some aspects the first flagpole extension comprises nucleic acid that is at least 80%, 85%, 90%, 95% or 99% homology to SEQ ID NO: 6586.

Some or all of the nucleotides of the first tracr extension can have a modification, e.g., modification found in Section XIII herein.

### 3) The Loop

A loop serves to link the crRNA flagpole region (or optionally the first flagpole extension, when present) with the tracr (or optionally the first tracr extension, when present) of a sgRNA. The loop can link the crRNA flagpole region and tracr covalently or non-covalently. In an embodiment, the linkage is covalent. In an embodiment, the loop covalently couples the crRNA flagpole region and tracr. In an embodiment, the loop covalently couples the first flagpole extension and the first tracr extension. In an embodiment, the loop is, or comprises, a covalent bond interposed between the crRNA flagpole region and the domain of the tracr which hybridizes to the crRNA flagpole region. Typically, the loop comprises one or more, e.g., 2, 3, 4, 5, 6, 7, 8, 9, or 10 nucleotides.

In dgRNA molecules the two molecules can be associated by virtue of the hybridization between at least a portion of the crRNA (e.g., the crRNA flagpole region) and at least a portion of the tracr (e.g., the domain of the tracr which is complementary to the crRNA flagpole region).

A wide variety of loops are suitable for use in sgRNAs. Loops can consist of a covalent bond, or be as short as one or a few nucleotides, e.g., 1 , 2, 3, 4, or 5 nucleotides in length. In an embodiment, a loop is 2, 3, 4, 5, 6, 7, 8, 9, 10, 15, 20, or 25 or more nucleotides in length. In an embodiment, a loop is 2 to 50, 2 to 40, 2 to 30, 2 to 20, 2 to 10, or 2 to 5 nucleotides in length. In an embodiment, a loop shares homology with, or is derived from, a naturally occurring sequence. In an embodiment, the loop has at least 50% homology with a loop disclosed herein. In an embodiment, the loop comprises SEQ ID NO: 6588.

Some or all of the nucleotides of the domain can have a modification, e.g., modification described in Section XIII herein.

### 4) The Second Flagpole Extension

In an embodiment, a dgRNA can comprise additional sequence, 3' to the crRNA flagpole region or, when present, the first flagpole extension, referred to herein as the second flagpole extension. In an embodiment, the second flagpole extension is, 2-10, 2-9, 2-8, 2-7, 2-6, 2-5, or 2-4 nucleotides in length. In an embodiment, the second flagpole extension is 2, 3, 4, 5, 6, 7, 8, 9, or 10 or more nucleotides in length. In an embodiment, the second flagpole extension comprises SEQ ID NO: 6587.

### 5) The Tracr:

The tracr is the nucleic acid sequence required for nuclease, e.g., Cas9, binding. Without being bound by theory, it is believed that each Cas9 species is associated with a particular tracr sequence. Tracr sequences are utilized in both sgRNA and in dgRNA systems. In an embodiment, the tracr comprises sequence from, or derived from, an *S*. *pyogenes* tracr. In some aspects, the tracr has a portion that hybridizes to the flagpole portion of the crRNA, e.g., has sufficient complementarity to the crRNA flagpole region to form a duplexed region under at least some physiological conditions (sometimes referred to herein as the tracr flagpole region or a tracr domain complementary to the crRNA flagpole region). In embodiments, the domain of the tracr that hybridizes with the crRNA flagpole region comprises at least 5, 6, 7, 8, 9, 10, 11, 12, 13, 14, 15, 16, 17, 18, 19, or 20 nucleotides that hybridize with complementary nucleotides of the crRNA flagpole region. In some aspects, the tracr nucleotides that hybridize with complementary nucleotides of the crRNA flagpole region are contiguous. In some aspects, the tracr nucleotides that hybridize with complementary nucleotides of the crRNA flagpole region are discontinuous, e.g., comprises two or more regions of hybridization separated by nucleotides that do not base pair with nucleotides of the crRNA flagpole region. In some aspects, the portion of the tracr that hybridizes to the crRNA flagpole region comprises, from 5' to 3': UAGCAAGUUAAAA (SEQ ID NO: 6597). In some aspects, the portion of the tracr that hybridizes to the crRNA flagpole regioncomprises, from 5' to 3': UAGCAAGUUUAAA (SEQ ID NO: 6598). In embodiments, the sequence that hybridizes with the crRNA flagpole region is disposed on the tracr 5'- to the sequence of the tracr that additionally binds a nuclease, e.g., a Cas molecule, e.g., a Cas9 molecule.

The tracr further comprises a domain that additionally binds to a nuclease, e.g., a Cas molecule, e.g., a Cas9 molecule. Without being bound by theory, it is believed that Cas9 from different species bind to different tracr sequences. In some aspects, the tracr comprises sequence that binds to a *S*. *pyogenes* Cas9 molecule. In some aspects, the tracr comprises sequence that binds to a Cas9 molecule disclosed herein. In some aspects, the domain that additionally binds a Cas9 molecule comprises, from 5' to 3': UAAGGCUAGUCCGUUAUCAACUUGAAAAAGUGGCACCGAGUCGGUGC (SEQ ID NO: 6599). In some aspects the domain that additionally binds a Cas9 molecule comprises, from 5' to 3': UAAGGCUAGUCCGUUAUCAACUUGAAAAAGUGGCACCGAGUCGGUGCUUUU (SEQ ID NO: 6600).

In some embodiments, the tracr comprises SEQ ID NO: 6589. In some embodiments, the tracr comprises SEQ ID NO: 6590.

Some or all of the nucleotides of the tracr can have a modification, e.g., modification found in Section XIII herein. In embodiments, the gRNA (e.g., the sgRNA or the tracr and/or crRNA of a dgRNA), e.g., any of the gRNA or gRNA components described above, comprises an inverted abasic residue at the 5' end, the 3' end or both the 5' and 3' end of the gRNA. In embodiments, the gRNA (e.g., the sgRNA or the tracr and/or crRNA of a dgRNA), e.g., any of the gRNA or gRNA components described above, comprises one or more phosphorothioate bonds between residues at the 5' end of the polynucleotide, for example, a phosphrothioate bond between the first two 5' residues, between each of the first three 5' residues, between each of the first four 5' residues, or between each of the first five 5' residues. In embodiments, the gRNA or gRNA component may alternatviely or additionally comprise one or more phosphorothioate bonds between residues at the 3' end of the polynucleotide, for example, a phosphrothioate bond between the first two 3' residues, between each of the first three 3' residues, between each of the first four 3' residues, or between each of the first five 3' residues. In an embodiment, the gRNA (e.g., the sgRNA or the tracr and/or crRNA of a dgRNA), e.g., any of the gRNA or gRNA components described above, comprises a phosphorothioate bond between each of the first four 5' residues (e.g., comprises, e.g., consists of, three phosphorothioate bonds at the 5' end(s)), and a phosphorothioate bond between each of the first four 3' residues (e.g., comprises, e.g., consists of, three phosphorothioate bonds at the 3' end(s)). In an embodiment, any of the phosphorothioate modificaitons described above are combined with an inverted abasic residue at the 5' end, the 3' end, or both the 5' and 3' ends of the polynucleotide. In such embodiments, the inverted abasic nucleotide may be linked to the 5' and/or 3' nucelotide by a phosphate bond or a phosphorothioate bond. In embodiments, the gRNA (e.g., the sgRNA or the tracr and/or crRNA of a dgRNA), e.g., any of the gRNA or gRNA components described above, comprises one or more nucleotides that include a 2' O-methyl modification. In embodiments, each of the first 1, 2, 3, or more of the 5' residues comprise a 2' O-methyl modification. In embodiments, each of the first 1, 2, 3, or more of the 3' residues comprise a 2' O-methyl modification. In embodiments, the 4^{th}-to-terminal, 3^{rd}-to-terminal, and 2^{nd}-to-terminal 3' residues comprise a 2' O-methyl modification. In embodiments, each of the first 1, 2, 3 or more of the 5' residues comprise a 2' O-methyl modification, and each of the first 1, 2, 3 or more of the 3' residues comprise a 2' O-methyl modification. In an embodiment, each of the first 3 of the 5' residues comprise a 2' O-methyl modification, and each of the first 3 of the 3' residues comprise a 2' O-methyl modification. In embodiments, each of the first 3 of the 5' residues comprise a 2' O-methyl modification, and the 4^{th}-to-terminal, 3^{rd}-to-terminal, and 2^{nd}-to-terminal 3' residues comprise a 2' O-methyl modification. In embodiments, any of the 2' O-methyl modfications, e.g., as described above, may be combined with one or more phosphorothioate modifications, e.g., as described above, and/or one or more inverted abasic modifications, e.g., as described above. In an embodiment, the gRNA (e.g., the sgRNA or the tracr and/or crRNA of a dgRNA), e.g., any of the gRNA or gRNA components described above, comprises, e.g., consists of, a phosphorothioate bond between each of the first four 5' residues (e.g., comprises, e.g., consists of three phosphorothioate bonds at the 5' end of the polynucleotide(s)), a phosphorothioate bond between each of the first four 3' residues (e.g., comprises, e.g., consists of three phosphorothioate bonds at the 5' end of the polynucleotide(s)), a 2' O-methyl modification at each of the first three 5' residues, and a 2' O-methyl modification at each of the first three 3' residues. In an embodiment, the gRNA (e.g., the sgRNA or the tracr and/or crRNA of a dgRNA), e.g., any of the gRNA or gRNA components described above, comprises, e.g., consists of, a phosphorothioate bond between each of the first four 5' residues (e.g., comprises, e.g., consists of three phosphorothioate bonds at the 5' end of the polynucleotide(s)), a phosphorothioate bond between each of the first four 3' residues (e.g., comprises, e.g., consists of three phosphorothioate bonds at the 5' end of the polynucleotide(s)), a 2' O-methyl modification at each of the first three 5' residues, and a 2' O-methyl modification at each of the 4^{th}-to-terminal, 3^{rd}-to-terminal, and 2^{nd}-to-terminal 3' residues.

In an embodiment, the gRNA (e.g., the sgRNA or the tracr and/or crRNA of a dgRNA), e.g., any of the gRNA or gRNA components described above, comprises, e.g., consists of, a phosphorothioate bond between each of the first four 5' residues (e.g., comprises, e.g., consists of three phosphorothioate bonds at the 5' end of the polynucleotide(s)), a phosphorothioate bond between each of the first four 3' residues (e.g., comprises, e.g., consists of three phosphorothioate bonds at the 5' end of the polynucleotide(s)), a 2' O-methyl modification at each of the first three 5' residues, a 2' O-methyl modification at each of the first three 3' residues, and an additional inverted abasic residue at each of the 5' and 3' ends.

In an embodiment, the gRNA (e.g., the sgRNA or the tracr and/or crRNA of a dgRNA), e.g., any of the gRNA or gRNA components described above, comprises, e.g., consists of, a phosphorothioate bond between each of the first four 5' residues (e.g., comprises, e.g., consists of three phosphorothioate bonds at the 5' end of the polynucleotide(s)), a phosphorothioate bond between each of the first four 3' residues (e.g., comprises, e.g., consists of three phosphorothioate bonds at the 5' end of the polynucleotide(s)), a 2' O-methyl modification at each of the first three 5' residues, and a 2' O-methyl modification at each of the 4^{th}-to-terminal, 3^{rd}-to-terminal, and 2^{nd}-to-terminal 3' residues, and an additional inverted abasic residue at each of the 5' and 3' ends.

In an embodiment, the gRNA is a dgRNA and comprises, e.g., consists of:
crRNA:
   mN^{∗}mN^{∗}mN^{∗}NNNNNNNNNNNNNNNNNGUUUUAGAGCUAU^{∗}mG^{∗}mC^{∗}mU, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus); and
tracr:
   AACAGCAUAGCAAGUUAAAAUAAGGCUAGUCCGUUAUCAACUUGAAAAAGUGGCACC GAGUCGGUGCUUUUUUU (SEQ ID NO: 6660) (optionally with an inverted abasic residue at the 5' and/or 3' terminus).

In an embodiment, the gRNA is a dgRNA and comprises, e.g., consists of:
crRNA:
   mN^{∗}mN^{∗}mN^{∗}NNNNNNNNNNNNNNNNNGUUUUAGAGCUAU^{∗}mG^{∗}mC^{∗}mU, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus); and
tracr:
   mA^{∗}mA^{∗}mC^{∗}AGCAUAGCAAGUUAAAAUAAGGCUAGUCCGUUAUCAACUUGAAAAAGUG GCACCGAGUCGGUGCUUUU^{∗}mU^{∗}mU^{∗}mU (SEQ ID NO: 346), where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus).

In an embodiment, the gRNA is a dgRNA and comprises, e.g., consists of:
crRNA:
   mN^{∗}mN^{∗}mN^{∗}NNNNNNNNNNNNNNNNNGUUUUAGAGCUAUGCUGUU^{∗}mU^{∗}mU^{∗}mG, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus); and
tracr:
   AACAGCAUAGCAAGUUAAAAUAAGGCUAGUCCGUUAUCAACUUGAAAAAGUGGCACC GAGUCGGUGCUUUUUUU (SEQ ID NO: 6660) (optionally with an inverted abasic residue at the 5' and/or 3' terminus).

In an embodiment, the gRNA is a dgRNA and comprises, e.g., consists of:
crRNA:
   mN^{∗}mN^{∗}mN^{∗}NNNNNNNNNNNNNNNNNGUUUUAGAGCUAUGCUGUU^{∗}mU^{∗}mU^{∗}mG, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus); and
tracr:
   mA^{∗}mA^{∗}mC^{∗}AGCAUAGCAAGUUAAAAUAAGGCUAGUCCGUUAUCAACUUGAAAAAGUG GCACCGAGUCGGUGCUUUU^{∗}mU^{∗}mU^{∗}mU (SEQ ID NO: 346), where m indicates a base with 2'O-Methyl modification, and ^{∗} indicates a phosphorothioate bond (optionally with an inverted abasic residue at the 5' and/or 3' terminus).

In an embodiment, the gRNA is a dgRNA and comprises, e.g., consists of:
crRNA:
   NNNNNNNNNNNNNNNNNNNNGUUUUAGAGCUAUGCUGUUUUG, where N's indicate the residues of the targeting domain, e.g., as described herein, (optionally with an inverted abasic residue at the 5' and/or 3' terminus); and
tracr:
   mA^{∗}mA^{∗}mC^{∗}AGCAUAGCAAGUUAAAAUAAGGCUAGUCCGUUAUCAACUUGAAAAAGUG GCACCGAGUCGGUGCUUUU^{∗}mU^{∗}mU^{∗}mU (SEQ ID NO: 346), where m indicates a base with 2'O-Methyl modification, and ^{∗} indicates a phosphorothioate bond (optionally with an inverted abasic residue at the 5' and/or 3' terminus).

In an embodiment, the gRNA is a sgRNA and comprises, e.g., consists of:
NNNNNNNNNNNNNNNNNNNNGUUUUAGAGCUAGAAAUAGCAAGUUAAAAUAAGGCUA GUCCGUUAUCAACUUGAAAAAGUGGCACCGAGUCGGUGCUUUU, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus).

In an embodiment, the gRNA is a sgRNA and comprises, e.g., consists of:
mN^{∗}mN^{∗}mN^{∗}NNNNNNNNNNNNNNNNNGUUUUAGAGCUAGAAAUAGCAAGUUAAAAUA AGGCUAGUCCGUUAUCAACUUGAAAAAGUGGCACCGAGUCGGUGCU^{∗}mU^{∗}mU^{∗}mU, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus).

In an embodiment, the gRNA is a sgRNA and comprises, e.g., consists of:
mN^{∗}mN^{∗}mN^{∗}NNNNNNNNNNNNNNNNNGUUUUAGAGCUAGAAAUAGCAAGUUAAAAUA AGGCUAGUCCGUUAUCAACUUGAAAAAGUGGCACCGAGUCGGUGCmU^{∗}mU^{∗}mU^{∗}U, where m indicates a base with 2'O-Methyl modification, ^{∗} indicates a phosphorothioate bond, and N's indicate the residues of the targeting domain, e.g., as described herein, (optionally with an inverted abasic residue at the 5' and/or 3' terminus).

### 6) First Tracr Extension

Where the gRNA comprises a first flagpole extension, the tracr may comprise a first tracr extension. The first tracr extension may comprise nucleotides that are complementary, e.g., 80%, 85%, 90%, 95% or 99%, e.g., fully complementary, with nucleotides of the first flagpole extension. In some aspects, the first tracr extension nucleotides that hybridize with complementary nucleotides of the first flagpole extension are contiguous. In some aspects, the first tracr extension nucleotides that hybridize with complementary nucleotides of the first flagpole extension are discontinuous, e.g., comprises two or more regions of hybridization separated by nucleotides that do not base pair with nucleotides of the first flagpole extension. In some aspects, the first tracr extension comprises at least 2, 3, 4, 5, 6, 7, 8, 9, 10, 11, 12, 13, 14, 15, 16, 17, 18, 19, 20 or more nucleotides. In some aspects, the first tracr extension comprises SEQ ID NO: 6591. In some aspects the first tracr extension comprises nucleic acid that is at least 80%, 85%, 90%, 95% or 99% homology to SEQ ID NO: 6591.

Some or all of the nucleotides of the first tracr extension can have a modification, e.g., modification found in Section XIII herein.

In some embodiments, the sgRNA may comprise, from 5' to 3', disposed 3' to the targeting domain:
a) 
GUUUUAGAGCUAGAAAUAGCAAGUUAAAAUAAGGCUAGUCCGUUAUCAACUUGAAAA
AGUGGCACCGAGUCGGUGC (SEQ ID NO: 6601);
b) 
GUUUAAGAGCUAGAAAUAGCAAGUUUAAAUAAGGCUAGUCCGUUAUCAACUUGAAAA
AGUGGCACCGAGUCGGUGC (SEQ ID NO: 6602);
c) 
GUUUUAGAGCUAUGCUGGAAACAGCAUAGCAAGUUAAAAUAAGGCUAGUCCGUUAUC
AACUUGAAAAAGUGGCACCGAGUCGGUGC (SEQ ID NO: 6603);
d) 
GUUUAAGAGCUAUGCUGGAAACAGCAUAGCAAGUUUAAAUAAGGCUAGUCCGUUAUC
AACUUGAAAAAGUGGCACCGAGUCGGUGC (SEQ ID NO: 6604);
e) any of a) to d), above, further comprising, at the 3' end, at least 1, 2, 3, 4, 5, 6 or 7 uracil (U) nucleotides, e.g., 1, 2, 3, 4, 5, 6, or 7 uracil (U) nucleotides;
f) any of a) to d), above, further comprising, at the 3' end, at least 1, 2, 3, 4, 5, 6 or 7 adenine (A) nucleotides, e.g., 1, 2, 3, 4, 5, 6, or 7 adenine (A) nucleotides; or
g) any of a) to f), above, further comprising, at the 5' end (e.g., at the 5' terminus, e.g., 5' to the targeting domain), at least 1, 2, 3, 4, 5, 6 or 7 adenine (A) nucleotides, e.g., 1, 2, 3, 4, 5, 6, or 7 adenine (A) nucleotides. In embodiments, any of a) to g) above is disposed directly 3' to the targeting domain.

In an embodiment, a sgRNA of the invention comprises, e.g., consists of, from 5' to 3': [targeting domain]-

GUUUUAGAGCUAGAAAUAGCAAGUUAAAAUAAGGCUAGUCCGUUAUCAACUUGAAAA
AGUGGCACCGAGUCGGUGCUUUU (SEQ ID NO: 7811).

In an embodiment, a sgRNA of the invention comprises, e.g., consists of, from 5' to 3': [targeting domain]-

GUUUAAGAGCUAUGCUGGAAACAGCAUAGCAAGUUUAAAUAAGGCUAGUCCGUUAUC
AACUUGAAAAAGUGGCACCGAGUCGGUGCUUUU (SEQ ID NO: 7807).

In some embodiments, the dgRNA may comprise:
A crRNA comprising, from 5' to 3', preferrably disposed directly 3' to the targeting domain:
   a) GUUUUAGAGCUA (SEQ ID NO: 6584);
   b) GUUUAAGAGCUA (SEQ ID NO: 6585);
   c) GUUUUAGAGCUAUGCUG (SEQ ID NO: 6605);
   d) GUUUAAGAGCUAUGCUG (SEQ ID NO: 6606);
   e) GUUUUAGAGCUAUGCUGUUUUG (SEQ ID NO: 6607);
   f) GUUUAAGAGCUAUGCUGUUUUG (SEQ ID NO: 6608); or
   g) GUUUUAGAGCUAUGCU (SEQ ID NO: 7806):
and a tracr comprising, from 5' to 3':
   a) 
UAGCAAGUUAAAAUAAGGCUAGUCCGUUAUCAACUUGAAAAAGUGGCACCGAGUCGG
UGC (SEQ ID NO: 6589);
   b) 
UAGCAAGUUAAAUAAGGCUAGUCCGUUAUCAACUUGAAAAAGUGGCACCGAGUCGG
UGC (SEQ ID NO: 6590);
   c) 
CAGCAUAGCAAGUUAAAAUAAGGCUAGUCCGUUAUCAACUUGAAAAAGUGGCACCGA
GUCGGUGC (SEQ ID NO: 6609);
   d) 
CAGCAUAGCAAGUUUAAAUAAGGCUAGUCCGUUAUCAACUUGAAAAAGUGGCACCGA
GUCGGUGC (SEQ ID NO: 6610);
   e) 
AACAGCAUAGCAAGUUAAAAUAAGGCUAGUCCGUUAUCAACUUGAAAAAGUGGCACC
GAGUCGGUGCUUUUUUU (SEQ ID NO: 6660);
   f) 
AACAGCAUAGCAAGUUAAAAUAAGGCUAGUCCGUUAUCAACUUGAAAAAGUGGCACC
GAGUCGGUGCUUUUUUU (SEQ ID NO: 6661);
   g) 
AACAGCAUAGCAAGUUAAAAUAAGGCUAGUCCGUUAUCAACUUGAAAAAGUGGCACC
GAGUCGGUGC (SEQ ID NO: 7812)
   h) 
GUUUAAGAGCUAUGCUGGAAACAGCAUAGCAAGUUUAAAUAAGGCUAGUCCGUUAUC
AACUUGAAAAAGUGGCACCGAGUCGGUGCUUUU (SEQ ID NO: 7807);
   i) 
AGCAUAGCAAGUUAAAAUAAGGCUAGUCCGUUAUCAACUUGAAAAAGUGGCACCGAG
UCGGUGCUUU (SEQ ID NO: 7808);
   j) 
GUUGGAACCAUUCAAAACAGCAUAGCAAGUUAAAAUAAGGCUAGUCCGUUAUCAACU
UGAAAAAGUGGCACCGAGUCGGUGCUUU (SEQ ID NO: 7809);
   k) any of a) to j), above, further comprising, at the 3' end, at least 1, 2, 3, 4, 5, 6 or 7 uracil (U) nucleotides, e.g., 1, 2, 3, 4, 5, 6, or 7 uracil (U) nucleotides;
   l) any of a) to j), above, further comprising, at the 3' end, at least 1, 2, 3, 4, 5, 6 or 7 adenine (A) nucleotides, e.g., 1, 2, 3, 4, 5, 6, or 7 adenine (A) nucleotides; or
   m) any of a) to l), above, further comprising, at the 5' end (e.g., at the 5' terminus), at least 1, 2, 3, 4, 5, 6 or 7 adenine (A) nucleotides, e.g., 1, 2, 3, 4, 5, 6, or 7 adenine (A) nucleotides.

In an embodiment, the sequence of k), above comprises the 3' sequence UUUUUU, e.g., if a U6 promoter is used for transcription. In an embodiment, the sequence of k), above, comprises the 3' sequence UUUU, e.g., if an HI promoter is used for transcription. In an embodiment, sequence of k), above, comprises variable numbers of 3' U's depending, e.g., on the termination signal of the pol-III promoter used. In an embodiment, the sequence of k), above, comprises variable 3' sequence derived from the DNA template if a T7 promoter is used. In an embodiment, the sequence of k), above, comprises variable 3' sequence derived from the DNA template, e.g., if in vitro transcription is used to generate the RNA molecule. In an embodiment, the sequence of k), above, comprises variable 3' sequence derived from the DNA template, e.g, if a pol-II promoter is used to drive transcription.

In an embodiment, the crRNA comprises, e.g., consists of, a targeting domain and, disposed 3' to the targeting domain (e.g., disposed directly 3' to the targeting domain), a sequence comprising, e.g., consisting of, SEQ ID NO: 6607, and the tracr comprises, e.g., consists of

AACAGCAUAGCAAGUUAAAAUAAGGCUAGUCCGUUAUCAACUUGAAAAAGUGGCACC
GAGUCGGUGCUUUUUUU (SEQ ID NO: 6660).

In an embodiment, the crRNA comprises, e.g., consists of, a targeting domain and, disposed 3' to the targeting domain (e.g., disposed directly 3' to the targeting domain), a sequence comprising, e.g., consisting of, SEQ ID NO: 6608, and the tracr comprises, e.g., consists of,

AACAGCAUAGCAAGUUAAAAUAAGGCUAGUCCGUUAUCAACUUGAAAAAGUGGCACC
GAGUCGGUGCUUUUUUU (SEQ ID NO: 6661).

In an embodiment, the crRNA comprises, e.g., consists of, a targeting domain and, disposed 3' to the targeting domain (e.g., disposed directly 3' to the targeting domain), a sequence comprising, e.g., consisting of, GUUUUAGAGCUAUGCU (SEQ ID NO: 7806), and the tracr comprises, e.g., consists of,

GUUUAAGAGCUAUGCUGGAAACAGCAUAGCAAGUUUAAAUAAGGCUAGUCCGUUAUC
AACUUGAAAAAGUGGCACCGAGUCGGUGCUUUU (SEQ ID NO: 7807).

In an embodiment, the crRNA comprises, e.g., consists of, a targeting domain and, disposed 3' to the targeting domain (e.g., disposed directly 3' to the targeting domain), a sequence comprising, e.g., consisting of, GUUUUAGAGCUAUGCU (SEQ ID NO: 7806), and the tracr comprises, e.g., consists of,

AGCAUAGCAAGUUAAAAUAAGGCUAGUCCGUUAUCAACUUGAAAAAGUGGCACCGAG
UCGGUGCUUU (SEQ ID NO: 7808).

In an embodiment, the crRNA comprises, e.g., consists of, a targeting domain and, disposed 3' to the targeting domain (e.g., disposed directly 3' to the targeting domain), a sequence comprising, e.g., consisting of, GUUUUAGAGCUAUGCUGUUUUG (SEQ ID NO: 6607), and the tracr comprises, e.g., consists of,

GUUGGAACCAUUCAAAACAGCAUAGCAAGUUAAAAUAAGGCUAGUCCGUUAUCAACU
UGAAAAAGUGGCACCGAGUCGGUGCUUU (SEQ ID NO: 7809).

### II. Targeting Domains Useful for Altering Expression of Globin Genes

Provided in the tables below are targeting domains for gRNA molecules for use in the various aspect of the present invention, for example, in altering expression of globin genes, for example, a fetal hemoglobin gene or a hemoglobin beta gene.

**Table 1: gRNA targeting domains directed to BLC11a exon regions**

| **Id.** | **Target** | **Target Region** | **Strand** | **Targeting Site (hg38)** | **gRNA Targeting Domain** | **SEQ ID NO:** |
|---|---|---|---|---|---|---|
| 53335_5_1 | BCL11 a | Exon 5 | + | chr2:60451170-60451195 | CAGUCAUUAUUUAUUAUGA AUAAGC | 400 |
| 53335_5_2 | BCL11 a | Exon 5 | + | chr2:60451196-60451221 | GGAAAUAAUUCACAUGCCA AUUAUU | 401 |
| 53335_5_3 | BCL11 a | Exon 5 | + | chr2:60451217-60451242 | UAUUGGCUAUCUUUCAC UAAGCU | 402 |
| 53335_5_4 | BCL11 a | Exon 5 | + | chr2:60451233-60451258 | CACUAAGCUAGGUAAUCUA GCCAGA | 403 |
| 53335_5_5 | BCL11 a | Exon 5 | + | chr2:60451236-60451261 | UAAGCUAGGUAAUCUAGCC AGAAGG | 404 |
| 53335_5_6 | BCL11 a | Exon 5 | + | chr2:60451241-60451266 | UAGGUAAUCUAGCCAGAAG GUGGAU | 405 |
| 53335_5_7 | BCL11 a | Exon 5 | + | chr2:60451245-60451270 | UAAUCUAGCCAGAAGGUGG AUAGGU | 406 |
| 53335_5_8 | BCL11 a | Exon 5 | + | chr2:60451261-60451286 | UGGAUAGGUAGGAUUUUCC CCACUU | 407 |
| 53335_5_9 | BCL11 a | Exon 5 | + | chr2:60451268-60451293 | GUAGGAUUUUCCCCACUUA GGUUCA | 408 |
| 53335_5_10 | BCL11 a | Exon 5 | + | chr2:60451295-60451320 | GCCUGUAUGUGAAUCUACA GCACAC | 409 |
| 53335_5_11 | BCL11 a | Exon 5 | + | chr2:60451359-60451384 | UGCUAUGUUGUUUCUAAUU CUCUUC | 410 |
| 53335_5_12 | BCL11 a | Exon 5 | + | chr2:60451414-60451439 | UGAGAAAGUUCUGUGCAA AUUAAC | 411 |
| 53335_5_13 | BCL11 a | Exon 5 | + | chr2:60451415-60451440 | GAGAAAAGUUCUGUGCAAA UUAACU | 412 |
| 53335_5_14 | BCL11 a | Exon 5 | + | chr2:60451462-60451487 | CCUGUUUGUAGAUGUAACU UAUUG | 413 |
| 53335_5_15 | BCL11 a | Exon 5 | + | chr2:60451477-60451502 | AACUUAUUUGUGGCACUAA UUUCUA | 414 |
| 53335_5_16 | BCL11 a | Exon 5 | + | chr2:60451625-60451650 | GUCUGCAUUAAGAUAUAUU UCCUGA | 415 |
| 53335_5_17 | BCL11 a | Exon 5 | + | chr2:60451630-60451655 | CAUUAAGAUAUAUUUCCUG AAGGUU | 416 |
| 53335_5_18 | BCL11 a | Exon 5 | + | chr2:60451631-60451656 | AUUAAGAUAUAUUUCCUGA AGGUUU | 417 |
| 53335_5_19 | BCL11 a | Exon 5 | + | chr2:60451687-60451712 | ACUACUGACAUUUAUCACC UUCUUU | 418 |
| 53335_5_20 | BCL11 a | Exon 5 | + | chr2:60451754-60451779 | UUAAAAGACAAAUUCAAAU CCUGCA | 419 |
| 53335_5_21 | BCL11 a | Exon 5 | + | chr2:60451786-60451811 | CACCAAAAGCAUAUAUUUG AAAAAC | 420 |
| 53335_5_22 | BCL11 a | Exon 5 | + | chr2:60451792-60451817 | AAGCAUAUAUUUGAAAAC AGGAUU | 421 |
| 53335_5_23 | BCL11 a | Exon 5 | + | chr2:60451819-60451844 | GCGUGCCAUAUUAUGCAUU AUUUAA | 422 |
| 53335_5_24 | BCL11 a | Exon 5 | + | chr2:60451847-60451872 | UAUGCAAAUUAUAAGUCAG ACAGUU | 423 |
| 53335_5_25 | BCL11 a | Exon 5 | + | chr2:60451971-60451996 | UUGAAAAUUUAUGCCAUCU GAUAAG | 424 |
| 53335_5_26 | BCL11 a | Exon 5 | + | chr2:60452037-60452062 | AAAUCUGUUCCUCCUACCC ACCCGA | 425 |
| 53335_5_27 | BCL11 a | Exon 5 | + | chr2:60452038-60452063 | AAUCUGUUCCUCCUACCCA CCCGAU | 426 |
| 53335_5_28 | BCL11 a | Exon 5 | + | chr2:60452048-60452073 | UCCUACCCACCCGAUGGGU GUCUGU | 427 |
| 53335_5_29 | BCL11 a | Exon 5 | + | chr2:60452055-60452080 | CACCCGAUGGGUGUCUGUA GGAAAC | 428 |
| 53335_5_30 | BCL11 a | Exon 5 | + | chr2:60452203-60452228 | AGACAACAUAGAAUGUAUA GAAACA | 429 |
| 53335_5_31 | BCL11 a | Exon 5 | + | chr2:60452204-60452229 | GACAACAUAGAAUGUAUAG AAACAA | 430 |
| 53335_5_32 | BCL11 a | Exon 5 | + | chr2:60452205-60452230 | ACAACAUAGAAUGUAUAGA AACAAG | 431 |
| 53335_5_33 | BCL11 a | Exon 5 | + | chr2:60452209-60452234 | CAUAGAAUGUAUAGAAACA AGGGGU | 432 |
| 53335_5_34 | BCL11 a | Exon 5 | + | chr2:60452210-60452235 | AUAGAAUGUAUAGAAACAA GGGGUU | 433 |
| 53335_5_35 | BCL11 a | Exon 5 | + | chr2:60452211-60452236 | UAGAAUGUAUAGAAACAAG GGGUUG | 434 |
| 53335_5_36 | BCL11 a | Exon 5 | + | chr2:60452237-60452262 | GGACUCAUGCGCAUUUCCA CAAUAC | 435 |
| 53335_5_37 | BCL11 a | Exon 5 | + | chr2:60452245-60452270 | GCGCAUUUCCACAAUACAG GUAAUU | 436 |
| 53335_5_38 | BCL11 a | Exon 5 | + | chr2:60452249-60452274 | AUUUCCACAAUACAGGUAA UUAGGU | 437 |
| 53335_5_39 | BCL11 a | Exon 5 | + | chr2:60452253-60452278 | CCACAAUACAGGUAAUUAG GUUGGC | 438 |
| 53335_5_40 | BCL11 a | Exon 5 | + | chr2:60452263-60452288 | GGUAAUUAGGUUGGCUGGU UUCAGA | 439 |
| 53335_5_41 | BCL11 a | Exon 5 | + | chr2:60452264-60452289 | GUAAUUAGGUUGGCUGGUU UCAGAA | 440 |
| 53335_5_42 | BCL11 a | Exon 5 | + | chr2:60452269-60452294 | UAGGUUGGCUGGUUUCAGA AGGGCC | 441 |
| 53335_5_43 | BCL11 a | Exon 5 | + | chr2:60452270-60452295 | AGGUUGGCUGGUUUCAGAA GGGCCA | 442 |
| 53335_5_44 | BCL11 a | Exon 5 | + | chr2:60452291-60452316 | GCCAGGGCAUCACUCAUGA CAGCGA | 443 |
| 53335_5_45 | BCL11 a | Exon 5 | + | chr2:60452298-60452323 | CAUCACUCAUGACAGCGAU GGUCCA | 444 |
| 53335_5_46 | BCL11 a | Exon 5 | + | chr2:60452299-60452324 | AUCACUCAUGACAGCGAUG GUCCAC | 445 |
| 53335_5_47 | BCL11 a | Exon 5 | + | chr2:60452311-60452336 | AGCGAUGGUCCACGGGCCC UCUCUA | 446 |
| 53335_5_48 | BCL11 a | Exon 5 | + | chr2:60452312-60452337 | GCGAUGGUCCACGGCCCU CUCUAU | 447 |
| 53335_5_49 | BCL11 a | Exon 5 | + | chr2:60452335-60452360 | AUGGGACUGAUUCACUGUU CCAAUG | 448 |
| 53335_5_50 | BCL11 a | Exon 5 | + | chr2:60452336-60452361 | UGGGACUGAUUCACUGUUC CAAUGU | 449 |
| 53335_5_51 | BCL11 a | Exon 5 | + | chr2:60452383-60452408 | UUUUUAUUAAAAAAUAUAA AUAAAA | 450 |
| 53335_5_52 | BCL11 a | Exon 5 | + | chr2:60452392-60452417 | AAAAAUAUAAAUAAAAUGG CGCUGC | 451 |
| 53335_5_53 | BCL11 a | Exon 5 | + | chr2:60452398-60452423 | AUAAAUAAAAUGGCGCUGC AGGCCU | 452 |
| 53335_5_54 | BCL11 a | Exon 5 | + | chr2:60452402-60452427 | AUAAAAUGGCGCUGCAGGC CUAGGC | 453 |
| 53335_5_55 | BCL11 a | Exon 5 | + | chr2:60452406-60452431 | AAUGGCGCUGCAGGCCUAG GCUGGA | 454 |
| 53335_5_56 | BCL11 a | Exon 5 | + | chr2:60452416-60452441 | CAGGCCUAGGCUGGAAGGA CUCUGC | 455 |
| 53335_5_57 | BCL11 a | Exon 5 | + | chr2:60452436-60452461 | UCUGCAGGACUCUGUCUUC GCACAA | 456 |
| 53335_5_58 | BCL11 a | Exon 5 | + | chr2:60452444-60452469 | ACUCUGUCUUCGCACAACG GCUUCU | 457 |
| 53335_5_59 | BCL11 a | Exon 5 | + | chr2:60452447-60452472 | CUGUCUUCGCACAACGGCU UCUUGG | 458 |
| 53335_5_60 | BCL11 a | Exon 5 | + | chr2:60452478-60452503 | CUGUCAGAAAACAUCACAA ACUAGC | 459 |
| 53335_5_61 | BCL11 a | Exon 5 | + | chr2:60452505-60452530 | GAUGACAGACCACGCUGAC GUCGAC | 460 |
| 53335_5_62 | BCL11 a | Exon 5 | + | chr2:60452506-60452531 | AUGACAGACCACGCUGACG UCGACU | 461 |
| 53335_5_63 | BCL11 a | Exon 5 | + | chr2:60452509-60452534 | ACAGACCACGCUGACGUCG ACUGGG | 462 |
| 53335_5_64 | BCL11 a | Exon 5 | + | chr2:60452531-60452556 | GGGCGGCACGCGUCCACCC CACCCC | 463 |
| 53335_5_65 | BCL11 a | Exon 5 | + | chr2:60452532-60452557 | GGCGGCACGCGUCCACCCC ACCCCU | 464 |
| 53335_5_66 | BCL11 a | Exon 5 | + | chr2:60452533-60452558 | GCGGCACGCGUCCACCCCA CCCCUG | 465 |
| 53335_5_67 | BCL11 a | Exon 5 | + | chr2:60452534-60452559 | CGGCACGCGUCCACCCCACC CCUGG | 466 |
| 53335_5_68 | BCL11 a | Exon 5 | + | chr2:60452559-60452584 | GGGCUUCAAAUUUUCUCAG AACUUA | 467 |
| 53335_5_69 | BCL11 a | Exon 5 | + | chr2:60452560-60452585 | GGCUUCAAAUUUUCUCAGA ACUUAA | 468 |
| 53335_5_70 | BCL11 a | Exon 5 | + | chr2:60452607-60452632 | AAACUGCCACACAUCUUGA GCUCUC | 469 |
| 53335_5_71 | BCL11 a | Exon 5 | + | chr2:60452608-60452633 | AACUGCCACACAUCUUGAG CUCUCU | 470 |
| 53335_5_72 | BCL11 a | Exon 5 | + | chr2:60452623-60452648 | UGAGCUCUCUGGGUACUAC GCCGAA | 471 |
| 53335_5_73 | BCL11 a | Exon 5 | + | chr2:60452624-60452649 | GAGCUCUCUGGGUACUACG CCGAAU | 472 |
| 53335_5_74 | BCL11 a | Exon 5 | + | chr2:60452625-60452650 | AGCUCUCUGGGUACUACGC CGAAUG | 473 |
| 53335_5_75 | BCL11 a | Exon 5 | + | chr2:60452626-60452651 | GCUCUCUGGGUACUACGCC GAAUGG | 474 |
| 53335_5_76 | BCL11 a | Exon 5 | + | chr2:60452649-60452674 | GGGGGUGUGUGAAGAACCU AGAAAG | 475 |
| 53335_5_77 | BCL11 a | Exon 5 | + | chr2:60452653-60452678 | GUGUGUGAAGAACCUAGAA AGAGGU | 476 |
| 53335_5_78 | BCL11 a | Exon 5 | + | chr2:60452662-60452687 | GAACCUAGAAAGAGGUUGG AGACAG | 477 |
| 53335_5_79 | BCL11 a | Exon 5 | - | chr2:60451215-60451240 | CUUAGUGAAAGAUAGCCA AAUAAU | 478 |
| 53335_5_80 | BCL11 a | Exon 5 | - | chr2:60451256-60451281 | GGGAAAAUCCUACCUAUCC ACCUUC | 479 |
| 53335_5_81 | BCL11 a | Exon 5 | - | chr2:60451281-60451306 | CACAUACAGGCCGUGAACC UAAGUG | 480 |
| 53335_5_82 | BCL11 a | Exon 5 | - | chr2:60451282-60451307 | UCACAUACAGGCCGUGAAC CUAAGU | 481 |
| 53335_5_83 | BCL11 a | Exon 5 | - | chr2:60451283-60451308 | UUCACAUACAGGCCGUGAA CCUAAG | 482 |
| 53335_5_84 | BCL11 a | Exon 5 | - | chr2:60451299-60451324 | ACCUGUGUGCUGUAGAUUC ACAUAC | 483 |
| 53335_5_85 | BCL11 a | Exon 5 | - | chr2:60451350-60451375 | UAGAAACAACAUAGCAAAU UAAAAU | 484 |
| 53335_5_86 | BCL11 a | Exon 5 | - | chr2:60451394-60451419 | UCUCAGUUUGGUAUUUUUU ACUGCU | 485 |
| 53335_5_87 | BCL11 a | Exon 5 | - | chr2:60451411-60451436 | AAUUUGCACAGAACUUUUC UCAGUU | 486 |
| 53335_5_88 | BCL11 a | Exon 5 | - | chr2:60451446-60451471 | ACAAACAGGUGGUAAAAU UCUUUC | 487 |
| 53335_5_89 | BCL11 a | Exon 5 | - | chr2:60451462-60451487 | CAAAUAAGUUACAUCUACA AACAGG | 488 |
| 53335_5_90 | BCL11 a | Exon 5 | - | chr2:60451465-60451490 | CCACAAAUAAGUUACAUCU ACAAAC | 489 |
| 53335_5_91 | BCL11 a | Exon 5 | - | chr2:60451552-60451577 | GAUUUUAGAGGGGGAAAU UAUAGG | 490 |
| 53335_5_92 | BCL11 a | Exon 5 | - | chr2:60451555-60451580 | GCAGAUUUUAGAGGGGGA AAUUAU | 491 |
| 53335_5_93 | BCL11 a | Exon 5 | - | chr2:60451565-60451590 | GAAAUUUGGGCAGAUUUU AGAGGG | 492 |
| 53335_5_94 | BCL11 a | Exon 5 | - | chr2:60451566-60451591 | GGAAAUUUGGGGCAGAUUU UAGAGG | 493 |
| 53335_5_95 | BCL11 a | Exon 5 | - | chr2:60451567-60451592 | AGGAAAUUUGGGCAGAUU UUAGAG | 494 |
| 53335_5_96 | BCL11 a | Exon 5 | - | chr2:60451568-60451593 | CAGGAAAUUUGGGGCAGAU UUUAGA | 495 |
| 53335_5_97 | BCL11 a | Exon 5 | - | chr2:60451569-60451594 | UCAGGAAAUUUGGGCAGA UUUUAG | 496 |
| 53335_5_98 | BCL11 a | Exon 5 | - | chr2:60451582-60451607 | UUUAGUACUUACAUCAGGA AAUUUG | 497 |
| 53335_5_99 | BCL11 a | Exon 5 | - | chr2:60451583-60451608 | CUUUAGUACUUACAUCAGG AAAUUU | 498 |
| 53335_5_10 0 | BCL11 a | Exon 5 | - | chr2:60451584-60451609 | UCUUUAGUACUUACAUCAG GAAAUU | 499 |
| 53335_5_10 1 | BCL11 a | Exon 5 | - | chr2:60451592-60451617 | AUAAAACUUCUUUAGUACU UACAUC | 500 |
| 53335_5_10 2 | BCL11 a | Exon 5 | - | chr2:60451648-60451673 | UCUAAUUUUAGGUUCCCAA ACCUUC | 501 |
| 53335_5_10 3 | BCL11 a | Exon 5 | - | chr2:60451664-60451689 | GUUUCUUAUUAAAUAUUCU AAUUUU | 502 |
| 53335_5_10 4 | BCL11 a | Exon 5 | - | chr2:60451707-60451732 | CUGGGCGAGCGGUAAAUCC UAAAGA | 503 |
| 53335_5_10 5 | BCL11 a | Exon 5 | - | chr2:60451723-60451748 | UUAGGAAAGAACAUCACUG GGCGAG | 504 |
| 53335_5_10 6 | BCL11 a | Exon 5 | - | chr2:60451730-60451755 | AUUUAGCUUAGGAAAGAAC AUCACU | 505 |
| 53335_5_10 7 | BCL11 a | Exon 5 | - | chr2:60451731-60451756 | AAUUUAGCUUAGGAAAGAA CAUCAC | 506 |
| 53335_5_10 8 | BCL11 a | Exon 5 | - | chr2:60451746-60451771 | UUGAAUUUGUCUUUUAAUU UAGCUU | 507 |
| 53335_5_10 9 | BCL11 a | Exon 5 | - | chr2:60451776-60451801 | UAUAUGCUUUUGGUGGCUA CCAUGC | 508 |
| 53335_5_11 0 | BCL11 a | Exon 5 | - | chr2:60451788-60451813 | CUGUUUUCAAAUAUAUGC UUUUGG | 509 |
| 53335_5_11 1 | BCL11 a | Exon 5 | - | chr2:60451791-60451816 | AUCCUGUUUUCAAAUAUA UGCUUU | 510 |
| 53335_5_11 2 | BCL11 a | Exon 5 | - | chr2:60451827-60451852 | GCAUACCAUUAAAUAAUGC AUAAUA | 511 |
| 53335_5_11 3 | BCL11 a | Exon 5 | - | chr2:60451882-60451907 | AUUGCUGUUUAUUAAUGCU GAAGUG | 512 |
| 53335_5_11 4 | BCL11 a | Exon 5 | - | chr2:60451949-60451974 | CAAGGAGAAUCAAAAUGCA AAACUU | 513 |
| 53335_5_11 5 | BCL11 a | Exon 5 | - | chr2:60451950-60451975 | UCAAGGAGAAUCAAAAUGC AAAACU | 514 |
| 53335_5_11 6 | BCL11 a | Exon 5 | - | chr2:60451972-60451997 | GCUUAUCAGAUGGCAUAAA UUUUCA | 515 |
| 53335_5_11 7 | BCL11 a | Exon 5 | - | chr2:60451987-60452012 | GGGAACUAGGUUACCGCUU AUCAGA | 516 |
| 53335_5_11 8 | BCL11 a | Exon 5 | - | chr2:60452005-60452030 | GGGCAGGAGAUGUAGGAGG GGAACU | 517 |
| 53335_5_11 9 | BCL11 a | Exon 5 | - | chr2:60452012-60452037 | GAGAAAUGGGCAGGAGAUG UAGGAG | 518 |
| 53335_5_12 0 | BCL11 a | Exon 5 | - | chr2:60452013-60452038 | UGAGAAAUGGGCAGGAGAU GUAGGA | 519 |
| 53335_5_12 1 | BCL11 a | Exon 5 | - | chr2:60452014-60452039 | UUGAGAAAUGGGCAGGAGA UGUAGG | 520 |
| 53335_5_12 2 | BCL11 a | Exon 5 | - | chr2:60452017-60452042 | GAUUUGAGAAAUGGGCAGG AGAUGU | 521 |
| 53335_5_12 3 | BCL11 a | Exon 5 | - | chr2:60452026-60452051 | GGAGGAACAGAUUUGAGAA AUGGGC | 522 |
| 53335_5_12 4 | BCL11 a | Exon 5 | - | chr2:60452030-60452055 | GGUAGGAGGAACAGAUUUG AGAAAU | 523 |
| 53335_5_12 5 | BCL11 a | Exon 5 | - | chr2:60452031-60452056 | GGGUAGGAGGAACAGAUUU GAGAAA | 524 |
| 53335_5_12 6 | BCL11 a | Exon 5 | - | chr2:60452049-60452074 | UACAGACACCCAUCGGGUG GGUAGG | 525 |
| 53335_5_12 7 | BCL11 a | Exon 5 | - | chr2:60452052-60452077 | UCCUACAGACACCCAUCGG GUGGGU | 526 |
| 53335_5_12 8 | BCL11 a | Exon 5 | - | chr2:60452056-60452081 | UGUUUCCUACAGACACCCA UCGGGU | 527 |
| 53335_5_12 9 | BCL11 a | Exon 5 | - | chr2:60452057-60452082 | CUGUUUCCUACAGACACCC AUCGGG | 528 |
| 53335_5_13 0 | BCL11 a | Exon 5 | - | chr2:60452060-60452085 | UACCUGUUUCCUACAGACA CCCAUC | 529 |
| 53335_5_13 1 | BCL11 a | Exon 5 | - | chr2:60452061-60452086 | GUACCUGUUUCCUACAGAC ACCCAU | 530 |
| 53335_5_13 2 | BCL11 a | Exon 5 | - | chr2:60452111-60452136 | AAUUUUUAAUGCUUAUAAG ACAAUG | 531 |
| 53335_5_13 3 | BCL11 a | Exon 5 | - | chr2:60452155-60452180 | ACACAAUAAAUGUUGGAGC UUUAGG | 532 |
| 53335_5_13 4 | BCL11 a | Exon 5 | - | chr2:60452158-60452183 | UUGACACAAUAAAUGUUGG AGCUUU | 533 |
| 53335_5_13 5 | BCL11 a | Exon 5 | - | chr2:60452167-60452192 | UGCUUAACAUUGACACAAU AAAUGU | 534 |
| 53335_5_13 6 | BCL11 a | Exon 5 | - | chr2:60452256-60452281 | CCAGCCAACCUAAUUACCU GUAUUG | 535 |
| 53335_5_13 7 | BCL11 a | Exon 5 | - | chr2:60452295-60452320 | ACCAUCGCUGUCAUGAGUG AUGCCC | 536 |
| 53335_5_13 8 | BCL11 a | Exon 5 | - | chr2:60452323-60452348 | GAAUCAGUCCCAUAGAGAG GGCCCG | 537 |
| 53335_5_13 9 | BCL11 a | Exon 5 | - | chr2:60452330-60452355 | GAACAGUGAAUCAGUCCCA UAGAGA | 538 |
| 53335_5_14 0 | BCL11 a | Exon 5 | - | chr2:60452331-60452356 | GGAACAGUGAAUCAGUCCC AUAGAG | 539 |
| 53335_5_14 1 | BCL11 a | Exon 5 | - | chr2:60452357-60452382 | UAAAAACAAAAAAACAGAC CCACAU | 540 |
| 53335_5_14 2 | BCL11 a | Exon 5 | - | chr2:60452423-60452448 | GAGUCCUGCAGAGUCCUUC CAGCCU | 541 |
| 53335_5_14 3 | BCL11 a | Exon 5 | - | chr2:60452517-60452542 | GCGUGCCGCCCAGUCGACG UCAGCG | 542 |
| 53335_5_14 4 | BCL11 a | Exon 5 | - | chr2:60452547-60452572 | AAAUUUGAAGCCCCCAGGG GUGGGG | 543 |
| 53335_5_14 5 | BCL11 a | Exon 5 | - | chr2:60452550-60452575 | AGAAAAUUUGAAGCCCCCA GGGGUG | 544 |
| 53335_5_14 6 | BCL11 a | Exon 5 | - | chr2:60452551-60452576 | GAGAAAAUUUGAAGCCCCC AGGGGU | 545 |
| 53335_5_14 7 | BCL11 a | Exon 5 | - | chr2:60452552-60452577 | UGAGAAAAUUUGAAGCCCC CAGGGG | 546 |
| 53335_5_14 8 | BCL11 a | Exon 5 | - | chr2:60452555-60452580 | UUCUGAGAAAAUUUGAAGC CCCCAG | 547 |
| 53335_5_14 9 | BCL11 a | Exon 5 | - | chr2:60452556-60452581 | GUUCUGAGAAAAUUUGAAG CCCCCA | 548 |
| 53335_5_15 0 | BCL11 a | Exon 5 | - | chr2:60452557-60452582 | AGUUCUGAGAAAUUUGAA GCCCCC | 549 |
| 53335_5_15 1 | BCL11 a | Exon 5 | - | chr2:60452602-60452627 | CUCAAGAUGUGUGGCAGUU UUCGGA | 550 |
| 53335_5_15 2 | BCL11 a | Exon 5 | - | chr2:60452606-60452631 | AGAGCUCAAGAUGUGUGGC AGUUUU | 551 |
| 53335_5_15 3 | BCL11 a | Exon 5 | - | chr2:60452616-60452641 | UAGUACCCAGAGAGCUCAA GAUGUG | 552 |
| 53335_5_15 4 | BCL11 a | Exon 5 | - | chr2:60452646-60452671 | UCUAGGUUCUUCACACACC CCCAUU | 553 |
| 53335_4_1 | BCL11 a | Exon 4 | + | chr2:60457199-60457224 | UUAAUUUUUUUUAUUUUUU CAAUAA | 554 |
| 53335_4_2 | BCL11 a | Exon 4 | + | chr2:60457200-60457225 | UAAUUUUUUUUAUUUUUUC AAUAAA | 555 |
| 53335_4_3 | BCL11 a | Exon 4 | + | chr2:60457209-60457234 | UUAUUUUUUCAAUAAAGGG ACAAAA | 556 |
| 53335_4_4 | BCL11 a | Exon 4 | + | chr2:60457210-60457235 | UAUUUUUUCAAUAAAGGGA CAAAAU | 557 |
| 53335_4_5 | BCL11 a | Exon 4 | + | chr2:60457222-60457247 | AAAGGGACAAAAUGGGUGU AUGAAC | 558 |
| 53335_4_6 | BCL11 a | Exon 4 | + | chr2:60457259-60457284 | ACAACUGCCAAAAAAACAC AGACAG | 559 |
| 53335_4_7 | BCL11 a | Exon 4 | + | chr2:60457312-60457337 | CCAGAAACAAAUACAAUAA AAAGCC | 560 |
| 53335_4_8 | BCL11 a | Exon 4 | + | chr2:60457328-60457353 | UAAAAAGCCAGGUUGUAAU GACCUU | 561 |
| 53335_4_9 | BCL11 a | Exon 4 | + | chr2:60457401-60457426 | CAAAUUAAGUGCCUCUGUU UUGAAC | 562 |
| 53335_4_10 | BCL11 a | Exon 4 | + | chr2:60457402-60457427 | AAAUUAAGUGCCUCUGUUU UGAACA | 563 |
| 53335_4_11 | BCL11 a | Exon 4 | + | chr2:60457480-60457505 | AGUUACGACAAACAGCUUU CAUUAC | 564 |
| 53335_4_12 | BCL11 a | Exon 4 | + | chr2:60457491-60457516 | ACAGCUUUCAUUACAGGAA UAGAAA | 565 |
| 53335_4_13 | BCL11 a | Exon 4 | + | chr2:60457545-60457570 | AUUUACAAAAAAGUAUUGA CUAAAG | 566 |
| 53335_4_14 | BCL11 a | Exon 4 | + | chr2:60457546-60457571 | UUUACAAAAAAGUAUUGAC UAAAGC | 567 |
| 53335_4_15 | BCL11 a | Exon 4 | + | chr2:60457616-60457641 | UAAAUAUAAAGCACCAUUU AGUUUU | 568 |
| 53335_4_16 | BCL11 a | Exon 4 | + | chr2:60457642-60457667 | GGCAAUGAAAAAAACUGCA AAACAU | 569 |
| 53335_4_17 | BCL11 a | Exon 4 | + | chr2:60457695-60457720 | UCUUUCUUUCUUUUACUGC AUAUGA | 570 |
| 53335_4_18 | BCL11 a | Exon 4 | + | chr2:60457706-60457731 | UUUACUGCAUAUGAAGGUA AGAUGC | 571 |
| 53335_4_19 | BCL11 a | Exon 4 | + | chr2:60457714-60457739 | AUAUGAAGGUAAGAUGCUG GAAUGU | 572 |
| 53335_4 | BCL11 a | Exon 4 | + | chr2:60457715-60457740 | UAUGAAGGUAAGAUGCUGG AAUGUA | 573 |
| 53335_4_21 | BCL11 a | Exon 4 | + | chr2:60457725-60457750 | AGAUGCUGGAAUGUAGGGU GAUAGA | 574 |
| 53335 _4_22 | BCL11 a | Exon 4 | + | chr2:60457730-60457755 | CUGGAAUGUAGGGUGAUAG AAGGAA | 575 |
| 53335_4_23 | BCL11 a | Exon 4 | + | chr2:6045773 1-60457756 | UGGAAUGUAGGGUGAUAGA AGGAAA | 576 |
| 53335_4_24 | BCL11 a | Exon 4 | + | chr2:60457792-60457817 | UUAGCUUGCAGUACUGCAU ACAGUA | 577 |
| 53335_4_25 | BCL11 a | Exon 4 | + | chr2:60457799-60457824 | GCAGUACUGCAUACAGUAU GGCAGC | 578 |
| 53335_4_26 | BCL11 a | Exon 4 | + | chr2:60457806-60457831 | UGCAUACAGUAUGGCAGCA GGAAAA | 579 |
| 53335 _4_27 | BCL11 a | Exon 4 | + | chr2:60457817-60457842 | UGGCAGCAGGAAAAAGGAA CAAAAA | 580 |
| 53335_4_28 | BCL11 a | Exon 4 | + | chr2:60457863-60457888 | ACAGCCAUCCAUGUGACAU UCUAGC | 581 |
| 53335 _4_29 | BCL11 a | Exon 4 | + | chr2:60457887-60457912 | CAGGCUCCCCCAAACCGCC AUUAUA | 582 |
| 53335_4_30 | BCL11 a | Exon 4 | + | chr2:60457996-60458021 | CCUGCCAAAUUAAAAAAAU AUACUG | 583 |
| 53335_4_31 | BCL11 a | Exon 4 | + | chr2:60458031-60458056 | UCUUUUUUUUUCCACUAC CAAAAA | 584 |
| 53335_4_32 | BCL11 a | Exon 4 | + | chr2:60458178-60458203 | AAAGACCAUAAAUGUAUUU UAGCAU | 585 |
| 53335_4_33 | BCL11 a | Exon 4 | + | chr2:60458274-60458299 | UUUUUUUUUUUACAACCU GAAGAG | 586 |
| 53335_4_34 | BCL11 a | Exon 4 | + | chr2:60458287-60458312 | CAACCUGAAGAGCGGUGUG UAUCCA | 587 |
| 53335_4_35 | BCL11 a | Exon 4 | + | chr2:60458317-60458342 | UAGAAUUUCCACUACCAUU UUUAAA | 588 |
| 53335_4_36 | BCL11 a | Exon 4 | + | chr2:60458350-60458375 | AAGUCUUGUAACACCACCA AGACAA | 589 |
| 53335_4_37 | BCL11 a | Exon 4 | + | chr2:60458564-60458589 | UAAGUAAGCUCAAUAGUCA AGUAAA | 590 |
| 53335_4_38 | BCL11 a | Exon 4 | + | chr2:60458568-60458593 | UAAGCUCAAUAGUCAAGUA AAUGGC | 591 |
| 53335_4_39 | BCL11 a | Exon 4 | + | chr2:60458677-60458702 | UUCCCUUAAGUAUAGACCU GUAAAC | 592 |
| 53335_4_40 | BCL11 a | Exon 4 | + | chr2:60458678-60458703 | UCCCUUAAGUAUAGACCUG UAAACU | 593 |
| 53335_4_41 | BCL11 a | Exon 4 | + | chr2:60458717-60458742 | GUGCACUUAAUUGUCCUAU CUGAGC | 594 |
| 53335_4_42 | BCL11 a | Exon 4 | + | chr2:60458775-60458800 | AUUAGAGAAAAGAUACAGA UAUCAC | 595 |
| 53335_4_43 | BCL11 a | Exon 4 | + | chr2:60458806-60458831 | AGUCAAGUGCUAUUUGAAC ACCAAC | 596 |
| 53335_4_44 | BCL11 a | Exon 4 | + | chr2:60458807-60458832 | GUCAAGUGCUAUUUGAACA CCAACU | 597 |
| 53335_4_45 | BCL11 a | Exon 4 | + | chr2:60458808-60458833 | UCAAGUGCUAUUUGAACAC CAACUG | 598 |
| 53335_4_46 | BCL11 a | Exon 4 | + | chr2:60458904-60458929 | GUUAACACAAAUAGCACAC AGUGUA | 599 |
| 53335_4_47 | BCL11 a | Exon 4 | + | chr2:60458930-60458955 | GGAAAAGAAAUGAAGUACA ACUUUU | 600 |
| 53335_4_48 | BCL11 a | Exon 4 | + | chr2:60458931-60458956 | GAAAAGAAAUGAAGUACAA CUUUUA | 601 |
| 53335_4_49 | BCL11 a | Exon 4 | + | chr2:60459074-60459099 | CUUAUAUACCUGUUCUAGU UUUAAA | 602 |
| 53335_4_50 | BCL11 a | Exon 4 | + | chr2:60459165-60459190 | UAUUGUCAGCCUCUUCCUU UCAAUA | 603 |
| 53335_4_51 | BCL11 a | Exon 4 | + | chr2:60459174-60459199 | CCUCUUCCUUUCAAUAUGG UAUACA | 604 |
| 53335_4_52 | BCL11 a | Exon 4 | + | chr2:60459223-60459248 | UGUCCACUUGACAACCAAG UAGAUC | 605 |
| 53335_4_53 | BCL11 a | Exon 4 | + | chr2:60459238-60459263 | CAAGUAGAUCUGGAUCUAU UUCUUU | 606 |
| 53335_4_54 | BCL11 a | Exon 4 | + | chr2:60459322-60459347 | UUACUAGUGUAUUUAAUUG CGUUCC | 607 |
| 53335_4_55 | BCL11 a | Exon 4 | + | chr2:60459323-60459348 | UACUAGUGUAUUUAAUUGC GUUCCA | 608 |
| 53335_4_56 | BCL11 a | Exon 4 | + | chr2:60459378-60459403 | UCAUUGUUUAAAAAAAAUA AAACUU | 609 |
| 53335_4_57 | BCL11 a | Exon 4 | + | chr2:60459379-60459404 | CAUUGUUUAAAAAAAAUAA AACUUU | 610 |
| 53335_4_58 | BCL11 a | Exon 4 | + | chr2:60459413-60459438 | AGCCCAUUUCUUUUAAGCU CUCACC | 611 |
| 53335_4_59 | BCL11 a | Exon 4 | + | chr2:60459435-60459460 | ACCAGGAGCAAAGUAGCUU UUAUAC | 612 |
| 53335_4_60 | BCL11 a | Exon 4 | + | chr2:60459470-60459495 | AGUUUUGUUUAUAAAAUUA AACUAA | 613 |
| 53335_4_61 | BCL11 a | Exon 4 | + | chr2:60459490-60459515 | ACUAAAGGAAAAAUGAUGA UUAACU | 614 |
| 53335_4_62 | BCL11 a | Exon 4 | + | chr2:60459499-60459524 | AAAAUGAUGAUUAACUAGG ACAUAA | 615 |
| 53335_4_63 | BCL11 a | Exon 4 | + | chr2:60459500-60459525 | AAAUGAUGAUUAACUAGGA CAUAAU | 616 |
| 53335_4_64 | BCL11 a | Exon 4 | + | chr2:60459513-60459538 | CUAGGACAUAAUGGGUCAU CUUUUU | 617 |
| 53335_4_65 | BCL11 a | Exon 4 | + | chr2:60459564-60459589 | AUAUAGAAUUAUAUGCUAG UUCCUA | 618 |
| 53335_4_66 | BCL11 a | Exon 4 | + | chr2:60459632-60459657 | UAACAAGUAGAAAGAACCA UCGAUG | 619 |
| 53335_4_67 | BCL11 a | Exon 4 | + | chr2:60459649-60459674 | CAUCGAUGUGGUUUUAAUA GAUCCA | 620 |
| 53335_4_68 | BCL11 a | Exon 4 | + | chr2:60459718-60459743 | GUUUUUCUGUUAAUUUGUC AAUUCA | 621 |
| 53335_4_69 | BCL11 a | Exon 4 | + | chr2:60459818-60459843 | UCAGUGCUAUCUAUUCUGU CUAUAG | 622 |
| 53335_4_70 | BCL11 a | Exon 4 | + | chr2:60459819-60459844 | CAGUGCUAUCUAUUCUGUC UAUAGA | 623 |
| 53335_4_71 | BCL11 a | Exon 4 | + | chr2:60459900-60459925 | UAUGUAUUACAGAAUGUAU GCAGCA | 624 |
| 53335_4_72 | BCL11 a | Exon 4 | + | chr2:60459937-60459962 | CUCUCUCUCUCUUUUUCUC UCAGAA | 625 |
| 53335_4_73 | BCL11 a | Exon 4 | + | chr2:60459943-60459968 | CUCUCUUUUCUCUCAGAA CGGAAC | 626 |
| 53335_4_74 | BCL11 a | Exon 4 | + | chr2:60459977-60460002 | CAACAUGUUUGCUCAGCAA CGAAUU | 627 |
| 53335_4_75 | BCL11 a | Exon 4 | + | chr2:60459978-60460003 | AACAUGUUUGCUCAGCAAC GAAUUA | 628 |
| 53335 4 76 | BCL11 a | Exon 4 | + | chr2:60460068-60460093 | ACAUGUAAAUUAUUGCACA AGAGAA | 629 |
| 53335_4_77 | BCL11 a | Exon 4 | + | chr2:60460126-60460151 | GCCCCAUACAGAUCAUGCA UUCAAA | 630 |
| 53335_4_78 | BCL11 a | Exon 4 | + | chr2:60460140-60460165 | AUGCAUUCAAACGGUGAGA ACAUAA | 631 |
| 53335_4_79 | BCL11 a | Exon 4 | + | chr2:60460193-60460218 | AAAGAAAAAGAAAAAGAAA AAAAAC | 632 |
| 53335_4_80 | BCL11 a | Exon 4 | + | chr2:60460201-60460226 | AGAAAAAGAAAAAAAACAG GUGUGC | 633 |
| 53335_4_81 | BCL11 a | Exon 4 | + | chr2:60460269-60460294 | UGUUUGUUUGUUUGUUUAA AUCACA | 634 |
| 53335_4_82 | BCL11 a | Exon 4 | + | chr2:60460270-60460295 | GUUUGUUUGUUUGUUUAAA UCACAU | 635 |
| 53335_4_83 | BCL11 a | Exon 4 | + | chr2:60460291-60460316 | ACAUGGACUAGAAAAAAA UCCUAC | 636 |
| 53335_4_84 | BCL11 a | Exon 4 | + | chr2:60460292-60460317 | CAUGGGACUAGAAAAAAAU CCUACA | 637 |
| 53335_4_85 | BCL11 a | Exon 4 | + | chr2:60460297-60460322 | GACUAGAAAAAAUCCUAC AGGGAG | 638 |
| 53335_4_86 | BCL11 a | Exon 4 | + | chr2:60460298-60460323 | ACUAGAAAAAAUCCUACA GGGAGU | 639 |
| 53335_4_87 | BCL11 a | Exon 4 | + | chr2:60460299-60460324 | CUAGAAAAAAUCCUACAG GGAGUG | 640 |
| 53335_4_88 | BCL11 a | Exon 4 | + | chr2:60460303-60460328 | AAAAAAAUCCUACAGGGAG UGGGGC | 641 |
| 53335_4_89 | BCL11 a | Exon 4 | + | chr2:60460306-60460331 | AAAAUCCUACAGGGAGUGG GGCUGG | 642 |
| 53335_4_90 | BCL11 a | Exon 4 | + | chr2:60460307-60460332 | AAAUCCUACAGGGAGUGGG GCUGGA | 643 |
| 53335_4_91 | BCL11 a | Exon 4 | + | chr2:60460313-60460338 | UACAGGGAGUGGGCUGGA GGGCGA | 644 |
| 53335_4_92 | BCL11 a | Exon 4 | + | chr2:60460314-60460339 | ACAGGGAGUGGGCUGGAG GGCGAU | 645 |
| 53335_4_93 | BCL11 a | Exon 4 | + | chr2:60460315-60460340 | CAGGGAGUGGGGCUGGAGG GCGAUG | 646 |
| 53335_4_94 | BCL11 a | Exon 4 | + | chr2:60460319-60460344 | GAGUGGGGCUGGAGGGCGA UGGGGA | 647 |
| 53335_4_95 | BCL11 a | Exon 4 | + | chr2:60460320-60460345 | AGUGGGGCUGGAGGGCGAU GGGGAA | 648 |
| 53335_4_96 | BCL11 a | Exon 4 | + | chr2:60460321-60460346 | GUGGGGCUGGAGGGCGAUG GGGAAG | 649 |
| 53335_4_97 | BCL11 a | Exon 4 | + | chr2:60460326-60460351 | GCUGGAGGGCGAUGGGGAA GGGGAG | 650 |
| 53335_4_98 | BCL11 a | Exon 4 | + | chr2:60460335-60460360 | CGAUGGGAAGGGAGUGG UGAAAA | 651 |
| 53335_4_99 | BCL11 a | Exon 4 | + | chr2:60460336-60460361 | GAUGGGAAGGGAGUGGU GAAAAA | 652 |
| 53335_4_10 0 | BCL11 a | Exon 4 | + | chr2:60460337-60460362 | AUGGGAAGGGAGUGGUG AAAAAG | 653 |
| 53335_4_10 1 | BCL11 a | Exon 4 | + | chr2:60460338-60460363 | UGGGGAAGGGGAGUGGUGA AAAAGG | 654 |
| 53335_4_10 2 | BCL11 a | Exon 4 | + | chr2:60460345-60460370 | GGGGAGUGGUGAAAAGGG GGUGUC | 655 |
| 53335_4_10 3 | BCL11 a | Exon 4 | + | chr2:60460348-60460373 | GAGUGGUGAAAAAGGGGU GUCAGG | 656 |
| 53335_4_10 4 | BCL11 a | Exon 4 | + | chr2:60460349-60460374 | AGUGGUGAAAAGGGGGUG UCAGGU | 657 |
| 53335_4_10 5 | BCL11 a | Exon 4 | + | chr2:60460356-60460381 | AAAAAGGGGUGUCAGGUG GGAGUG | 658 |
| 53335_4_10 6 | BCL11 a | Exon 4 | + | chr2:60460357-60460382 | AAAAGGGGGUGUCAGGUGG GAGUGA | 659 |
| 53335_4_10 7 | BCL11 a | Exon 4 | + | chr2:60460360-60460385 | AGGGGGUGUCAGGUGGGAG UGAGGG | 660 |
| 53335_4_10 8 | BCL11 a | Exon 4 | + | chr2:60460361-60460386 | GGGGGUGUCAGGUGGGAGU GAGGGA | 661 |
| 53335_4_10 9 | BCL11 a | Exon 4 | + | chr2:60460362-60460387 | GGGGUGUCAGGUGGGAGUG AGGGAG | 662 |
| 53335_4_11 0 | BCL11 a | Exon 4 | + | chr2:60460442-60460467 | GUGCCAUUUUUUCAUGUGU UUCUCC | 663 |
| 53335_4_11 1 | BCL11 a | Exon 4 | + | chr2:60460443-60460468 | UGCCAUUUUUCAUGUGUU UCUCCA | 664 |
| 53335_4_11 2 | BCL11 a | Exon 4 | + | chr2:60460462-60460487 | UCUCCAGGGUACUGUACAC GCUAAA | 665 |
| 53335_4_11 3 | BCL11 a | Exon 4 | + | chr2:60460505-60460530 | ACAUUUGUAAACGUCCUUC CCCACC | 666 |
| 53335_4_11 4 | BCL11 a | Exon 4 | + | chr2:60460527-60460552 | ACCUGGCCAUGCGUUUUCA UGUGCC | 667 |
| 53335_4_11 5 | BCL11 a | Exon 4 | + | chr2:60460544-60460569 | CAUGUGCCUGGUGAGCUUG CUACUC | 668 |
| 53335_4_11 6 | BCL11 a | Exon 4 | + | chr2:60460545-60460570 | AUGUGCCUGGUGAGCUUGC UACUCU | 669 |
| 53335_4_11 7 | BCL11 a | Exon 4 | + | chr2:60460551-60460576 | CUGGUGAGCUUGCUACUCU GGGCAC | 670 |
| 53335_4_11 8 | BCL11 a | Exon 4 | + | chr2:60460579-60460604 | CAUAGUUGCACAGCUCGCA UUUAUA | 671 |
| 53335_4_11 9 | BCL11 a | Exon 4 | + | chr2:60460595-60460620 | GCAUUUAUAAGGCCUUUCG CCCGUG | 672 |
| 53335_4_12 0 | BCL11 a | Exon 4 | + | chr2:60460607-60460632 | CCUUUCGCCCGUGUGGCUU CUCCUG | 673 |
| 53335_4_12 1 | BCL11 a | Exon 4 | + | chr2:60460686-60460711 | UCGCUGCGUCUGCCCUCUU UUGAGC | 674 |
| 53335_4_12 2 | BCL11 a | Exon 4 | + | chr2:60460687-60460712 | CGCUGCGUCUGCCCUCUUU UGAGCU | 675 |
| 53335_4_12 3 | BCL11 a | Exon 4 | + | chr2:60460696-60460721 | UGCCCUCUUUUGAGCUGGG CCUGCC | 676 |
| 53335_4_12 4 | BCL11 a | Exon 4 | + | chr2:60460697-60460722 | GCCCUCUUUGAGCUGGGC CUGCCC | 677 |
| 53335_4_12 5 | BCL11 a | Exon 4 | + | chr2:60460702-60460727 | CUUUUGAGCUGGGCCUGCC CGGGCC | 678 |
| 53335_4_12 6 | BCL11 a | Exon 4 | + | chr2:60460715-60460740 | CCUGCCCGGGCCCGGACCA CUAAUA | 679 |
| 53335_4_12 7 | BCL11 a | Exon 4 | + | chr2:60460716-60460741 | CUGCCCGGGCCCGGACCAC UAAUAU | 680 |
| 53335_4_12 8 | BCL11 a | Exon 4 | + | chr2:60460717-60460742 | UGCCCGGGCCCGGACCACU AAUAUG | 681 |
| 53335_4_12 9 | BCL11 a | Exon 4 | + | chr2:60460776-60460801 | CCCGAGAUCCCUCCGUCCA GCUCCC | 682 |
| 53335_4_13 0 | BCL11 a | Exon 4 | + | chr2:60460777-60460802 | CCGAGAUCCCUCCGUCCAG CUCCCC | 683 |
| 53335_4_13 1 | BCL11 a | Exon 4 | + | chr2:60460780-60460805 | AGAUCCCUCCGUCCAGCUC CCCGGG | 684 |
| 53335_4_13 2 | BCL11 a | Exon 4 | + | chr2:60460785-60460810 | CCUCCGUCCAGCUCCCCGG GCGGUG | 685 |
| 53335_4_13 3 | BCL11 a | Exon 4 | + | chr2:60460812-60460837 | GAGAAGCGCAAACUCCCGU UCUCCG | 686 |
| 53335_4_13 4 | BCL11 a | Exon 4 | + | chr2:60460827-60460852 | CCGUUCUCCGAGGAGUGCU CCGACG | 687 |
| 53335_4_13 5 | BCL11 a | Exon 4 | + | chr2:60460830-60460855 | UUCUCCGAGGAGUGCUCCG ACGAGG | 688 |
| 53335_4_13 6 | BCL11 a | Exon 4 | + | chr2:60460837-60460862 | AGGAGUGCUCCGACGAGGA GGCAAA | 689 |
| 53335_4_13 7 | BCL11 a | Exon 4 | + | chr2:60460848-60460873 | GACGAGGAGGCAAAAGGCG AUUGUC | 690 |
| 53335_4_13 8 | BCL11 a | Exon 4 | + | chr2:60460865-60460890 | CGAUUGUCUGGAGUCUCCG AAGCUA | 691 |
| 53335_4_13 9 | BCL11 a | Exon 4 | + | chr2:60460869-60460894 | UGUCUGGAGUCUCCGAAGC UAAGGA | 692 |
| 53335_4_14 0 | BCL11 a | Exon 4 | + | chr2:60460870-60460895 | GUCUGGAGUCUCCGAAGCU AAGGAA | 693 |
| 53335_4_14 1 | BCL11 a | Exon 4 | + | chr2:60460887-60460912 | CUAAGGAAGGGAUCUUUGA GCUGCC | 694 |
| 53335_4_14 2 | BCL11 a | Exon 4 | + | chr2:60460890-60460915 | AGGAAGGGAUCUUUGAGCU GCCUGG | 695 |
| 53335_4_14 3 | BCL11 a | Exon 4 | + | chr2:60460902-60460927 | UUGAGCUGCCUGGAGGCCG CGUAGC | 696 |
| 53335_4_14 4 | BCL11 a | Exon 4 | + | chr2:60460935-60460960 | CACUGCGAGUACACGUUCU CCGUGU | 697 |
| 53335_4_14 5 | BCL11 a | Exon 4 | + | chr2:60460936-60460961 | ACUGCGAGUACACGUUCUC CGUGUU | 698 |
| 53335_4_14 6 | BCL11 a | Exon 4 | + | chr2:60460944-60460969 | UACACGUUCUCCGUGUUGG GCAUCG | 699 |
| 53335_4_14 7 | BCL11 a | Exon 4 | + | chr2:60460948-60460973 | CGUUCUCCGUGUUGGGCAU CGCGGC | 700 |
| 53335_4_14 8 | BCL11 a | Exon 4 | + | chr2:60460949-60460974 | GUUCUCCGUGUUGGGCAUC GCGGCC | 701 |
| 53335_4_14 9 | BCL11 a | Exon 4 | + | chr2:60460950-60460975 | UUCUCCGUGUUGGGCAUCG CGGCCG | 702 |
| 53335_4_15 0 | BCL11 a | Exon 4 | + | chr2:60460951-60460976 | UCUCCGUGUUGGGCAUCGC GGCCGG | 703 |
| 53335_4_15 1 | BCL11 a | Exon 4 | + | chr2:60460955-60460980 | CGUGUUGGGCAUCGCGGCC GGGGGC | 704 |
| 53335_4_15 2 | BCL11 a | Exon 4 | + | chr2:60460992-60461017 | UUCUCGAGCUUGAUGCGCU UAGAGA | 705 |
| 53335_4_15 3 | BCL11 a | Exon 4 | + | chr2:60460993-60461018 | UCUCGAGCUUGAUGCGCUU AGAGAA | 706 |
| 53335_4_15 4 | BCL11 a | Exon 4 | + | chr2:60460994-60461019 | CUCGAGCUUGAUGCGCUUA GAGAAG | 707 |
| 53335_4_15 5 | BCL11 a | Exon 4 | + | chr2:60461007-60461032 | CGCUUAGAGAAGGGGCUCA GCGAGC | 708 |
| 53335_4_15 6 | BCL11 a | Exon 4 | + | chr2:60461008-60461033 | GCUUAGAGAAGGGGCUCAG CGAGCU | 709 |
| 53335_4_15 7 | BCL11 a | Exon 4 | + | chr2:60461009-60461034 | CUUAGAGAAGGGCUCAGC GAGCUG | 710 |
| 53335_4_15 8 | BCL11 a | Exon 4 | + | chr2:60461031-60461056 | CUGGGGCUGCCCAGCAGCA GCUUUU | 711 |
| 53335_4_15 9 | BCL11 a | Exon 4 | + | chr2:60461036-60461061 | GCUGCCCAGCAGCAGCUUU UUGGAC | 712 |
| 53335_4_16 0 | BCL11 a | Exon 4 | + | chr2:60461046-60461071 | AGCAGCUUUUUGGACAGGC CCCCCG | 713 |
| 53335_4_16 1 | BCL11 a | Exon 4 | + | chr2:60461059-60461084 | ACAGGCCCCCGAGGCCGA CUCGCC | 714 |
| 53335_4_16 2 | BCL11 a | Exon 4 | + | chr2:60461060-60461085 | CAGGCCCCCGAGGCCGAC UCGCCC | 715 |
| 53335_4_16 3 | BCL11 a | Exon 4 | + | chr2:60461061-60461086 | AGGCCCCCGAGGCCGACU CGCCCG | 716 |
| 53335_4_16 4 | BCL11 a | Exon 4 | + | chr2:60461072-60461097 | GGCCGACUCGCCCGGGGAG CAGCCG | 717 |
| 53335_4_16 5 | BCL11 a | Exon 4 | + | chr2:60461099-60461124 | GCCAUUAACAGUGCCAUCG UCUAUG | 718 |
| 53335_4_16 6 | BCL11 a | Exon 4 | + | chr2:60461112-60461137 | CCAUCGUCUAUGCGGUCCG ACUCGC | 719 |
| 53335_4_16 7 | BCL11 a | Exon 4 | + | chr2:60461144-60461169 | CGAGUCUUCGUCGCAAGUG UCCCUG | 720 |
| 53335_4_16 8 | BCL11 a | Exon 4 | + | chr2:60461151-60461176 | UCGUCGCAAGUGUCCCUGU GGCCCU | 721 |
| 53335_4_16 9 | BCL11 a | Exon 4 | + | chr2:60461157-60461182 | CAAGUGUCCCUGUGGCCCU CGGCCU | 722 |
| 53335_4_17 0 | BCL11 a | Exon 4 | + | chr2:60461162-60461187 | GUCCCUGUGGCCCUCGGCC UCGGCC | 723 |
| 53335_4_17 1 | BCL11 a | Exon 4 | + | chr2:60461165-60461190 | CCUGUGGCCCUCGGCCUCG GCCAGG | 724 |
| 53335_4_17 2 | BCL11 a | Exon 4 | + | chr2:60461189-60461214 | GUGGCCGCGCUUAUGCUUC UCGCCC | 725 |
| 53335_4_17 3 | BCL11 a | Exon 4 | + | chr2:60461195-60461220 | GCGCUUAUGCUUCUCGCCC AGGACC | 726 |
| 53335_4_17 4 | BCL11 a | Exon 4 | + | chr2:60461198-60461223 | CUUAUGCUUCUCGCCCAGG ACCUGG | 727 |
| 53335_4_17 5 | BCL11 a | Exon 4 | + | chr2:60461202-60461227 | UGCUUCUCGCCCAGGACCU GGUGGA | 728 |
| 53335_4_17 6 | BCL11 a | Exon 4 | + | chr2:60461223-60461248 | UGGAAGGCCUCGCUGAAGU GCUGCA | 729 |
| 53335_4_17 7 | BCL11 a | Exon 4 | + | chr2:60461253-60461278 | CUGAGCACCAUGCCCUGCA UGACGU | 730 |
| 53335_4_17 8 | BCL11 a | Exon 4 | + | chr2:60461254-60461279 | UGAGCACCAUGCCCUGCAU GACGUC | 731 |
| 53335_4_17 9 | BCL11 a | Exon 4 | + | chr2:60461258-60461283 | CACCAUGCCCUGCAUGACG UCGGGC | 732 |
| 53335_4_18 0 | BCL11 a | Exon 4 | + | chr2:60461259-60461284 | ACCAUGCCCUGCAUGACGU CGGGCA | 733 |
| 53335_4_18 1 | BCL11 a | Exon 4 | + | chr2:60461264-60461289 | GCCCUGCAUGACGUCGGGC AGGGCG | 734 |
| 53335_4_18 2 | BCL11 a | Exon 4 | + | chr2:60461312-60461337 | GACCGCGCCCCGCGAGCUG UUCUCG | 735 |
| 53335_4_18 3 | BCL11 a | Exon 4 | + | chr2:60461315-60461340 | CGCGCCCGCGAGCUGUUC UCGUGG | 736 |
| 53335_4_18 4 | BCL11 a | Exon 4 | + | chr2:60461330-60461355 | GUUCUCGUGGUGGCGCGCC GCCUCC | 737 |
| 53335_4_18 5 | BCL11 a | Exon 4 | + | chr2:60461446-60461471 | CCUCUUCCUCCUCGUCCCCG UUCUC | 738 |
| 53335_4_18 6 | BCL11 a | Exon 4 | + | chr2:60461447-60461472 | CUCUUCCUCCUCGUCCCCG UUCUCC | 739 |
| 53335_4_18 7 | BCL11 a | Exon 4 | + | chr2:60461453-60461478 | CUCCUCGUCCCCGUUCUCC GGGAUC | 740 |
| 53335_4_18 8 | BCL11 a | Exon 4 | + | chr2:60461457-60461482 | UCGUCCCCGUUCUCCGGGA UCAGGU | 741 |
| 53335_4_18 9 | BCL11 a | Exon 4 | + | chr2:60461458-60461483 | CGUCCCCGUUCUCCGGGAU CAGGUU | 742 |
| 53335_4_19 0 | BCL11 a | Exon 4 | + | chr2:60461459-60461484 | GUCCCCGUUCUCCGGGAUC AGGUUG | 743 |
| 53335_4_19 1 | BCL11 a | Exon 4 | + | chr2:60461481-60461506 | UUGGGGUCGUUCUCGCUCU UGAACU | 744 |
| 53335_4_19 2 | BCL11 a | Exon 4 | + | chr2:60461490-60461515 | UUCUCGCUCUUGAACUUGG CCACCA | 745 |
| 53335_4_19 3 | BCL11 a | Exon 4 | + | chr2:60461508-60461533 | GCCACCACGGACUUGAGCG CGCUGC | 746 |
| 53335_4_19 4 | BCL11 a | Exon 4 | + | chr2:60461529-60461554 | CUGCUGGCGCUGCCCACCA AGUCGC | 747 |
| 53335_4_19 5 | BCL11 a | Exon 4 | + | chr2:60461535-60461560 | GCGCUGCCCACCAAGUCGC UGGUGC | 748 |
| 53335_4_19 6 | BCL11 a | Exon 4 | + | chr2:60461536-60461561 | CGCUGCCCACCAAGUCGCU GGUGCC | 749 |
| 53335_4_19 7 | BCL11 a | Exon 4 | + | chr2:60461542-60461567 | CCACCAAGUCGCUGGUGCC GGGUUC | 750 |
| 53335_4_19 8 | BCL11 a | Exon 4 | + | chr2:60461543-60461568 | CACCAAGUCGCUGGUGCCG GGUUCC | 751 |
| 53335_4_19 9 | BCL11 a | Exon 4 | + | chr2:60461544-60461569 | ACCAAGUCGCUGGUGCCGG GUUCCG | 752 |
| 53335_4_20 0 | BCL11 a | Exon 4 | + | chr2:60461550-60461575 | UCGCUGGUGCCGGUUCCG GGGAGC | 753 |
| 53335_4_20 1 | BCL11 a | Exon 4 | + | chr2:60461553-60461578 | CUGGUGCCGGGUUCCGGGG AGCUGG | 754 |
| 53335_4_20 2 | BCL11 a | Exon 4 | + | chr2:60461556-60461581 | GUGCCGGGUUCCGGGGAGC UGGCGG | 755 |
| 53335_4_20 3 | BCL11 a | Exon 4 | + | chr2:60461571-60461596 | GAGCUGGCGGUGGAGAGAC CGUCGU | 756 |
| 53335_4_20 4 | BCL11 a | Exon 4 | + | chr2:60461586-60461611 | AGACCGUCGUCGGACUUGA CCGUCA | 757 |
| 53335_4_20 5 | BCL11 a | Exon 4 | + | chr2:60461587-60461612 | GACCGUCGUCGGACUUGAC CGUCAU | 758 |
| 53335_4_20 6 | BCL11 a | Exon 4 | + | chr2:60461588-60461613 | ACCGUCGUCGGACUUGACC GUCAUG | 759 |
| 53335_4_20 7 | BCL11 a | Exon 4 | + | chr2:60461589-60461614 | CCGUCGUCGGACUUGACCG UCAUGG | 760 |
| 53335_4_20 8 | BCL11 a | Exon 4 | + | chr2:60461618-60461643 | CGAUUUGUGCAUGUGCGUC UUCAUG | 761 |
| 53335_4_20 9 | BCL11 a | Exon 4 | + | chr2:60461634-60461659 | GUCUUCAUGUGGCGCUUCA GCUUGC | 762 |
| 53335_4_21 0 | BCL11 a | Exon 4 | + | chr2:60461639-60461664 | CAUGUGGCGCUUCAGCUUG CUGGCC | 763 |
| 53335_4_21 1 | BCL11 a | Exon 4 | + | chr2:60461640-60461665 | AUGUGGCGCUUCAGCUUGC UGGCCU | 764 |
| 53335_4_21 2 | BCL11 a | Exon 4 | + | chr2:60461651-60461676 | CAGCUUGCUGGCCUGGGUG CACGCG | 765 |
| 53335_4_21 3 | BCL11 a | Exon 4 | + | chr2:60461660-60461685 | GGCCUGGGUGCACGCGUGG UCGCAC | 766 |
| 53335_4_21 4 | BCL11 a | Exon 4 | + | chr2:60461673-60461698 | GCGUGGUCGCACAGGUUGC ACUUGU | 767 |
| 53335_4_21 5 | BCL11 a | Exon 4 | + | chr2:60461674-60461699 | CGUGGUCGCACAGGUUGCA CUUGUA | 768 |
| 53335_4_21 6 | BCL11 a | Exon 4 | + | chr2:60461690-60461715 | GCACUUGUAGGGCUUCUCG CCCGUG | 769 |
| 53335_4_21 7 | BCL11 a | Exon 4 | + | chr2:60461699-60461724 | GGGCUUCUCGCCCGUGUGG CUGCGC | 770 |
| 53335_4_21 8 | BCL11 a | Exon 4 | + | chr2:60461711-60461736 | CGUGUGGCUGCGCCGGUGC ACCACC | 771 |
| 53335_4_21 9 | BCL11 a | Exon 4 | + | chr2:60461757-60461782 | GUCUUGCCGCAGAACUCGC AUGACU | 772 |
| 53335_4_22 0 | BCL11 a | Exon 4 | + | chr2:60461767-60461792 | AGAACUCGCAUGACUUGGA CUUGAC | 773 |
| 53335_4_22 1 | BCL11 a | Exon 4 | + | chr2:60461768-60461793 | GAACUCGCAUGACUUGGAC UUGACC | 774 |
| 53335_4_22 2 | BCL11 a | Exon 4 | + | chr2:60461769-60461794 | AACUCGCAUGACUUGGACU UGACCG | 775 |
| 53335_4_22 3 | BCL11 a | Exon 4 | + | chr2:60461770-60461795 | ACUCGCAUGACUUGGACUU GACCGG | 776 |
| 53335_4_22 4 | BCL11 a | Exon 4 | + | chr2:60461774-60461799 | GCAUGACUUGGACUUGACC GGGGGC | 777 |
| 53335_4_22 5 | BCL11 a | Exon 4 | + | chr2:60461775-60461800 | CAUGACUUGGACUUGACCG GGGGCU | 778 |
| 53335_4_22 6 | BCL11 a | Exon 4 | + | chr2:60461778-60461803 | GACUUGGACUUGACCGGGG GCUGGG | 779 |
| 53335_4_22 7 | BCL11 a | Exon 4 | + | chr2:60461779-60461804 | ACUUGGACUUGACCGGGGG CUGGGA | 780 |
| 53335_4_22 8 | BCL11 a | Exon 4 | + | chr2:60461782-60461807 | UGGACUUGACCGGGGGCUG GGAGGG | 781 |
| 53335_4_22 9 | BCL11 a | Exon 4 | + | chr2:60461785-60461810 | ACUUGACCGGGGGCUGGGA GGGAGG | 782 |
| 53335_4_23 0 | BCL11 a | Exon 4 | + | chr2:60461786-60461811 | CUUGACCGGGGGCUGGGAG GGAGGA | 783 |
| 53335_4_23 1 | BCL11 a | Exon 4 | + | chr2:60461787-60461812 | UUGACCGGGGGCUGGGAGG GAGGAG | 784 |
| 53335_4_23 2 | BCL11 a | Exon 4 | + | chr2:60461790-60461815 | ACCGGGGGCUGGGAGGGAG GAGGGG | 785 |
| 53335_4_23 3 | BCL11 a | Exon 4 | + | chr2:60461800-60461825 | GGGAGGGAGGAGGGCGGA UUGCAG | 786 |
| 53335_4_23 4 | BCL11 a | Exon 4 | + | chr2:60461803-60461828 | AGGGAGGAGGGGCGGAUUG CAGAGG | 787 |
| 53335_4_23 5 | BCL11 a | Exon 4 | + | chr2:60461804-60461829 | GGGAGGAGGGCGGAUUGC AGAGGA | 788 |
| 53335_4_23 6 | BCL11 a | Exon 4 | + | chr2:60461807-60461832 | AGGAGGGGCGGAUUGCAGA GGAGGG | 789 |
| 53335_4_23 7 | BCL11 a | Exon 4 | + | chr2:60461808-60461833 | GGAGGGGCGGAUUGCAGAG GAGGGA | 790 |
| 53335_4_23 8 | BCL11 a | Exon 4 | + | chr2:60461809-60461834 | GAGGGGCGGAUUGCAGAGG AGGGAG | 791 |
| 53335_4_23 9 | BCL11 a | Exon 4 | + | chr2:60461810-60461835 | AGGGGCGGAUUGCAGAGGA GGGAGG | 792 |
| 53335_4_24 0 | BCL11 a | Exon 4 | + | chr2:60461811-60461836 | GGGGCGGAUUGCAGAGGAG GGAGGG | 793 |
| 53335_4_24 1 | BCL11 a | Exon 4 | + | chr2:60461812-60461837 | GGGCGGAUUGCAGAGGAGG GAGGGG | 794 |
| 53335_4_24 2 | BCL11 a | Exon 4 | + | chr2:60461822-60461847 | CAGAGGAGGGAGGGGGGGC GUCGCC | 795 |
| 53335_4_24 3 | BCL11 a | Exon 4 | + | chr2:60461826-60461851 | GGAGGGAGGGGGGCGUCG CCAGGA | 796 |
| 53335_4_24 4 | BCL11 a | Exon 4 | + | chr2:60461827-60461852 | GAGGGAGGGGGGCGUCGC CAGGAA | 797 |
| 53335_4_24 5 | BCL11 a | Exon 4 | + | chr2:60461830-60461855 | GGAGGGGGGCGUCGCCAG GAAGGG | 798 |
| 53335_4_24 6 | BCL11 a | Exon 4 | + | chr2:60461842-60461867 | UCGCCAGGAAGGGCGGCUU GCUACC | 799 |
| 53335_4_24 7 | BCL11 a | Exon 4 | + | chr2:60461846-60461871 | CAGGAAGGGCGGCUUGCUA CCUGGC | 800 |
| 53335_4_24 8 | BCL11 a | Exon 4 | + | chr2:60461851-60461876 | AGGGCGGCUUGCUACCUGG CUGGAA | 801 |
| 53335_4_24 9 | BCL11 a | Exon 4 | + | chr2:60461872-60461897 | GGAAUGGUUGCAGUAACCU UUGCAU | 802 |
| 53335_4_25 0 | BCL11 a | Exon 4 | + | chr2:60461873-60461898 | GAAUGGUUGCAGUAACCUU UGCAUA | 803 |
| 53335_4_25 1 | BCL11 a | Exon 4 | + | chr2:60461877-60461902 | GGUUGCAGUAACCUUUGCA UAGGGC | 804 |
| 53335_4_25 2 | BCL11 a | Exon 4 | + | chr2:60461878-60461903 | GUUGCAGUAACCUUUGCAU AGGGCU | 805 |
| 53335_4_25 3 | BCL11 a | Exon 4 | + | chr2:60461882-60461907 | CAGUAACCUUUGCAUAGGG CUGGGC | 806 |
| 53335_4_25 4 | BCL11 a | Exon 4 | + | chr2:60461887-60461912 | ACCUUUGCAUAGGGCUGGG CCGGCC | 807 |
| 53335_4_25 5 | BCL11 a | Exon 4 | + | chr2:60461888-60461913 | CCUUUGCAUAGGGCUGGGC CGGCCU | 808 |
| 53335_4_25 6 | BCL11 a | Exon 4 | + | chr2:60461889-60461914 | CUUUGCAUAGGGCUGGGCC GGCCUG | 809 |
| 53335_4_25 7 | BCL11 a | Exon 4 | + | chr2:60461896-60461921 | UAGGGCUGGGCCGGCCUGG GGACAG | 810 |
| 53335_4_25 8 | BCL11 a | Exon 4 | + | chr2:60461899-60461924 | GGCUGGGCCGGCCUGGGGA CAGCGG | 811 |
| 53335_4_25 9 | BCL11 a | Exon 4 | + | chr2:60461900-60461925 | GCUGGGCCGGCCUGGGGAC AGCGGU | 812 |
| 53335_4_26 0 | BCL11 a | Exon 4 | + | chr2:60461949-60461974 | UCUCUAAGUCUCCUAGAGA AAUCCA | 813 |
| 53335_4_26 1 | BCL11 a | Exon 4 | + | chr2:60461952-60461977 | CUAAGUCUCCUAGAGAAAU CCAUGG | 814 |
| 53335_4_26 2 | BCL11 a | Exon 4 | + | chr2:60461953-60461978 | UAAGUCUCCUAGAGAAAUC CAUGGC | 815 |
| 53335_4_26 3 | BCL11 a | Exon 4 | + | chr2:60461956-60461981 | GUCUCCUAGAGAAAUCCAU GGCGGG | 816 |
| 53335_4_26 4 | BCL11 a | Exon 4 | + | chr2:60461971-60461996 | CCAUGGCGGGAGGCUCCAU AGCCAU | 817 |
| 53335_4_26 5 | BCL11 a | Exon 4 | + | chr2:60461997-60462022 | GGAUUCAACCGCAGCACCC UGUCAA | 818 |
| 53335_4_26 6 | BCL11 a | Exon 4 | + | chr2:60462004-60462029 | ACCGCAGCACCCUGUCAAA GGCACU | 819 |
| 53335_4_26 7 | BCL11 a | Exon 4 | + | chr2:60462005-60462030 | CCGCAGCACCCUGUCAAAG GCACUC | 820 |
| 53335_4_26 8 | BCL11 a | Exon 4 | + | chr2:60462011-60462036 | CACCCUGUCAAAGGCACUC GGGUGA | 821 |
| 53335_4_26 9 | BCL11 a | Exon 4 | + | chr2:60462012-60462037 | ACCCUGUCAAAGGCACUCG GGUGAU | 822 |
| 53335_4_27 0 | BCL11 a | Exon 4 | + | chr2:60462015-60462040 | CUGUCAAAGGCACUCGGGU GAUGGG | 823 |
| 53335_4_27 1 | BCL11 a | Exon 4 | + | chr2:60462020-60462045 | AAAGGCACUCGGGUGAUGG GUGGCC | 824 |
| 53335_4_272 2 | BCL11 a | Exon 4 | + | chr2:60462021-60462046 | AAGGCACUCGGGUGAUGGG UGGCCA | 825 |
| 53335_4_27 3 | BCL11 a | Exon 4 | + | chr2:60462041-60462066 | GGCCAGGGCCAUCUCUUCC GCCCCC | 826 |
| 53335_4_27 4 | BCL11 a | Exon 4 | + | chr2:60462053-60462078 | CUCUUCCGCCCCCAGGCGC UCUAUG | 827 |
| 53335_4_27 5 | BCL11 a | Exon 4 | + | chr2:60462056-60462081 | UUCCGCCCCCAGGCGCUCU AUGCGG | 828 |
| 53335_4_27 6 | BCL11 a | Exon 4 | + | chr2:60462057-60462082 | UCCGCCCCCAGGCGCUCUA UGCGGU | 829 |
| 53335_4_27 7 | BCL11 a | Exon 4 | + | chr2:60462058-60462083 | CCGCCCCCAGGCGCUCUAU GCGGUG | 830 |
| 53335_4_27 8 | BCL11 a | Exon 4 | + | chr2:60462059-60462084 | CGCCCCCAGGCGCUCUAUG CGGUGG | 831 |
| 53335_4_27 9 | BCL11 a | Exon 4 | + | chr2:60462076-60462101 | UGCGGUGGGGUCCAAGUG AUGUCU | 832 |
| 53335_4_28 0 | BCL11 a | Exon 4 | + | chr2:60462079-60462104 | GGUGGGGUCCAAGUGAUG UCUCGG | 833 |
| 53335_4_28 1 | BCL11 a | Exon 4 | + | chr2:60462082-60462107 | GGGGGUCCAAGUGAUGUCU CGGUGG | 834 |
| 53335_4_28 2 | BCL11 a | Exon 4 | + | chr2:60462092-60462117 | GUGAUGUCUCGGUGGUGGA CUAAAC | 835 |
| 53335_4_28 3 | BCL11 a | Exon 4 | + | chr2:60462093-60462118 | UGAUGUCUCGGUGGUGGAC UAAACA | 836 |
| 53335_4_28 4 | BCL11 a | Exon 4 | + | chr2:60462094-60462119 | GAUGUCUCGGUGGUGGACU AAACAG | 837 |
| 53335_4_28 5 | BCL11 a | Exon 4 | + | chr2:60462095-60462120 | AUGUCUCGGUGGUGGACUA AACAGG | 838 |
| 53335_4_28 6 | BCL11 a | Exon 4 | + | chr2:60462096-60462121 | UGUCUCGGUGGUGGACUAA ACAGGG | 839 |
| 53335_4_28 7 | BCL11 a | Exon 4 | + | chr2:60462097-60462122 | GUCUCGGUGGUGGACUAAA CAGGGG | 840 |
| 53335_4_28 8 | BCL11 a | Exon 4 | + | chr2:60462102-60462127 | GGUGGUGGACUAAACAGGG GGGGAG | 841 |
| 53335_4_28 9 | BCL11 a | Exon 4 | + | chr2:60462103-60462128 | GUGGUGGACUAAACAGGGG GGGAGU | 842 |
| 53335_4_29 0 | BCL11 a | Exon 4 | + | chr2:60462106-60462131 | GUGGACUAAACAGGGGGG AGUGGG | 843 |
| 53335_4_29 1 | BCL11 a | Exon 4 | + | chr2:60462125-60462150 | AGUGGGUGGAAAGCGCCCU UCUGCC | 844 |
| 53335_4_29 2 | BCL11 a | Exon 4 | + | chr2:60462129-60462154 | GGUGGAAAGCGCCCUUCUG CCAGGC | 845 |
| 53335_4_29 3 | BCL11 a | Exon 4 | + | chr2:60462154-60462179 | CGGAAGCCUCUCUCGAUAC UGAUCC | 846 |
| 53335_4_29 4 | BCL11 a | Exon 4 | + | chr2:60462167-60462192 | CGAUACUGAUCCUGGUAUU CUUAGC | 847 |
| 53335_4_29 5 | BCL11 a | Exon 4 | + | chr2:60462174-60462199 | GAUCCUGGUAUUCUUAGCA GGUUAA | 848 |
| 53335_4_29 6 | BCL11 a | Exon 4 | + | chr2:60462175-60462200 | AUCCUGGUAUUCUUAGCAG GUUAAA | 849 |
| 53335_4_29 7 | BCL11 a | Exon 4 | + | chr2:60462176-60462201 | UCCUGGUAUUCUUAGCAGG UUAAAG | 850 |
| 53335_4_29 8 | BCL11 a | Exon 4 | + | chr2:60462203-60462228 | GUUAUUGUCUGCAAUAUGA AUCCCA | 851 |
| 53335_4_29 9 | BCL11 a | Exon 4 | + | chr2:60462208-60462233 | UGUCUGCAAUAUGAAUCCC AUGGAG | 852 |
| 53335_4_30 0 | BCL11 a | Exon 4 | + | chr2:60462211-60462236 | CUGCAAUAUGAAUCCCAUG GAGAGG | 853 |
| 53335_4_30 1 | BCL11 a | Exon 4 | + | chr2:60462215-60462240 | AAUAUGAAUCCCAUGGAGA GGUGGC | 854 |
| 53335_4_30 2 | BCL11 a | Exon 4 | + | chr2:60462216-60462241 | AUAUGAAUCCCAUGGAGAG GUGGCU | 855 |
| 53335_4_30 3 | BCL11 a | Exon 4 | + | chr2:60462220-60462245 | GAAUCCCAUGGAGAGGUGG CUGGGA | 856 |
| 53335_4_30 4 | BCL11 a | Exon 4 | + | chr2:60462244-60462269 | AAGGACAUUCUGCACCUAG UCCUGA | 857 |
| 53335_4_30 5 | BCL11 a | Exon 4 | + | chr2:60462245-60462270 | AGGACAUUCUGCACCUAGU CCUGAA | 858 |
| 53335_4_30 6 | BCL11 a | Exon 4 | + | chr2:60462259-60462284 | CUAGUCCUGAAGGAUACC AACCCG | 859 |
| 53335_4_30 7 | BCL11 a | Exon 4 | + | chr2:60462260-60462285 | UAGUCCUGAAGGGAUACCA ACCCGC | 860 |
| 53335_4_30 8 | BCL11 a | Exon 4 | + | chr2:60462261-60462286 | AGUCCUGAAGGAUACCAA CCCGCG | 861 |
| 53335_4_30 9 | BCL11 a | Exon 4 | + | chr2:60462266-60462291 | UGAAGGGAUACCAACCCGC GGGGUC | 862 |
| 53335_4_31 0 | BCL11 a | Exon 4 | + | chr2:60462267-60462292 | GAAGGGAUACCAACCCGCG GGGUCA | 863 |
| 53335_4_31 1 | BCL11 a | Exon 4 | + | chr2:60462268-60462293 | AAGGGAUACCAACCCGCGG GGUCAG | 864 |
| 53335_4_31 2 | BCL11 a | Exon 4 | + | chr2:60462345-60462370 | GCGUGUUGCAAGAGAAACC AUGCAC | 865 |
| 53335_4_31 3 | BCL11 a | Exon 4 | + | chr2:60462352-60462377 | GCAAGAGAAACCAUGCACU GGUGAA | 866 |
| 53335_4_31 4 | BCL11 a | Exon 4 | + | chr2:60462384-60462409 | UUGCAAGUUGUACAUGUGU AGCUGC | 867 |
| 53335_4_31 5 | BCL11 a | Exon 4 | + | chr2:60462385-60462410 | UGCAAGUUGUACAUGUGUA GCUGCU | 868 |
| 53335_4_31 6 | BCL11 a | Exon 4 | - | chr2:60457269-60457294 | GGAAAAACCACUGUCUGUG UUUUUU | 869 |
| 53335_4_31 7 | BCL11 a | Exon 4 | - | chr2:60457295-60457320 | GUUUCUGGUCUUUGUUAAG UUCUAU | 870 |
| 53335_4_31 8 | BCL11 a | Exon 4 | - | chr2:60457315-60457340 | CCUGGCUUUUUAUUGUAUU UGUUUC | 871 |
| 53335_4_31 9 | BCL11 a | Exon 4 | - | chr2:60457338-60457363 | AAGAUGACCAAAGGUCAUU ACAACC | 872 |
| 53335_4_32 0 | BCL11 a | Exon 4 | - | chr2:60457352-60457377 | AAAAAAAAAAAUAAAAGAU GACCAA | 873 |
| 53335_4_32 1 | BCL11 a | Exon 4 | - | chr2:60457415-60457440 | UGCUUAUGUGCCCUGUUCA AAACAG | 874 |
| 53335_4_32 2 | BCL11 a | Exon 4 | - | chr2:60457459-60457484 | AACUUGAAAUUUUAUCUUU UACUAU | 875 |
| 53335_4_32 3 | BCL11 a | Exon 4 | - | chr2:60457460-60457485 | UAACUUGAAAUUUUAUCUU UUACUA | 876 |
| 53335_4_32 4 | BCL11 a | Exon 4 | - | chr2:60457522-60457547 | AUGGCAAUGCAGAAUAUUU UGUUAU | 877 |
| 53335_4_32 5 | BCL11 a | Exon 4 | - | chr2:60457546-60457571 | GCUUUAGUCAAUACUUUUU UGUAAA | 878 |
| 53335_4_32 6 | BCL11 a | Exon 4 | - | chr2:60457613-60457638 | ACUAAAUGGUGCUUUAUAU UUAGAU | 879 |
| 53335_4_32 7 | BCL11 a | Exon 4 | - | chr2:60457632-60457657 | GUUUUUUUCAUUGCCAAAA ACUAAA | 880 |
| 53335_4_32 8 | BCL11 a | Exon 4 | - | chr2:60457786-60457811 | AUGCAGUACUGCAAGCUAA UAACGU | 881 |
| 53335_4_32 9 | BCL11 a | Exon 4 | - | chr2:60457858-60457883 | AAUGUCACAUGGAUGGCUG UCAUAG | 882 |
| 53335_4_33 0 | BCL11 a | Exon 4 | - | chr2:60457859-60457884 | GAAUGUCACAUGGAUGGCU GUCAUA | 883 |
| 53335_4_33 1 | BCL11 a | Exon 4 | - | chr2:60457860-60457885 | AGAAUGUCACAUGGAUGGC UGUCAU | 884 |
| 53335_4_33 2 | BCL11 a | Exon 4 | - | chr2:60457870-60457895 | GGAGCCUGCUAGAAUGUCA CAUGGA | 885 |
| 53335_4_33 3 | BCL11 a | Exon 4 | - | chr2:60457874-60457899 | UGGGGGAGCCUGCUAGAAU GUCACA | 886 |
| 53335_4_33 4 | BCL11 a | Exon 4 | - | chr2:60457896-60457921 | GAGAAGCCAUAUAAUGGCG GUUUGG | 887 |
| 53335_4_33 5 | BCL11 a | Exon 4 | - | chr2:60457897-60457922 | UGAGAAGCCAUAUAAUGGC GGUUUG | 888 |
| 53335_4_33 6 | BCL11 a | Exon 4 | - | chr2:60457898-60457923 | AUGAGAAGCCAUAUAAUGG CGGUUU | 889 |
| 53335_4_33 7 | BCL11 a | Exon 4 | - | chr2:60457899-60457924 | GAUGAGAAGCCAUAUAAUG GCGGUU | 890 |
| 53335_4_33 8 | BCL11 a | Exon 4 | - | chr2:60457904-60457929 | UUACAGAUGAGAAGCCAUA UAAUGG | 891 |
| 53335_4_33 9 | BCL11 a | Exon 4 | - | chr2:60457907-60457932 | ACAUUACAGAUGAGAAGCC AUAUAA | 892 |
| 53335_4_34 0 | BCL11 a | Exon 4 | - | chr2:60457999-60458024 | CCACAGUAUAUUUUUUUAA UUUGGC | 893 |
| 53335_4_34 1 | BCL11 a | Exon 4 | - | chr2:60458003-60458028 | GCUGCCACAGUAUAUUUUU UUAAUU | 894 |
| 53335_4_34 2 | BCL11 a | Exon 4 | - | chr2:60458030-60458055 | UUUUGGUAGUGGAAAAAA AAAGAC | 895 |
| 53335_4_34 3 | BCL11 a | Exon 4 | - | chr2:60458046-60458071 | GGUAUCAAUGUACCUUUUU UGGUAG | 896 |
| 53335_4_34 4 | BCL11 a | Exon 4 | - | chr2:60458052-60458077 | UUAAAAGGUAUCAAUGUAC CUUUUU | 897 |
| 53335_4_34 5 | BCL11 a | Exon 4 | - | chr2:60458072-60458097 | UUUAACUGUUGCUUGUUCU CUUAAA | 898 |
| 53335_4_34 6 | BCL11 a | Exon 4 | - | chr2:60458130-60458155 | UUGAAUUAAAUGUUCAUCU AGUGUU | 899 |
| 53335_4_34 7 | BCL11 a | Exon 4 | - | chr2:60458161-60458186 | UGGUCUUUUUCUGUAUUU CUAGAA | 900 |
| 53335_4_34 8 | BCL11 a | Exon 4 | - | chr2:60458186-60458211 | UGAUUCCUAUGCUAAAAUA CAUUUA | 901 |
| 53335_4_34 9 | BCL11 a | Exon 4 | - | chr2:60458231-60458256 | UUAAGAUUAUAUAGUACUU AAAUAU | 902 |
| 53335_4_35 0 | BCL11 a | Exon 4 | - | chr2:60458260-60458285 | AAAAAAAAAAACAUACAUU GGGGAA | 903 |
| 53335_4_35 1 | BCL11 a | Exon 4 | - | chr2:60458265-60458290 | UUGUAAAAAAAAAAAACAU ACAUUG | 904 |
| 53335_4_35 2 | BCL11 a | Exon 4 | - | chr2:60458266-60458291 | GUUGUAAAAAAAAAAAACA UACAUU | 905 |
| 53335_4_35 3 | BCL11 a | Exon 4 | - | chr2:60458267-60458292 | GGUUGUAAAAAAAAAAAAC AUACAU | 906 |
| 53335_4_35 4 | BCL11 a | Exon 4 | - | chr2:60458293-60458318 | AUGCCUUGGAUACACACCG CUCUUC | 907 |
| 53335_4_35 5 | BCL11 a | Exon 4 | - | chr2:60458312-60458337 | AAAUGGUAGUGGAAAUUCU AUGCCU | 908 |
| 53335_4_35 6 | BCL11 a | Exon 4 | - | chr2:60458328-60458353 | CUUGUUAUCCAUUUAAAAA UGGUAG | 909 |
| 53335_4_35 7 | BCL11 a | Exon 4 | - | chr2:60458334-60458359 | ACAAGACUUGUUAUCCAUU UAAAAA | 910 |
| 53335_4_35 8 | BCL11 a | Exon 4 | - | chr2:60458366-60458391 | GCAUUUUAGGUUCCAUUG UCUUGG | 911 |
| 53335_4_35 9 | BCL11 a | Exon 4 | - | chr2:60458369-60458394 | ACUGCAUUUUAGGGUUCCA UUGUCU | 912 |
| 53335_4_36 0 | BCL11 a | Exon 4 | - | chr2:60458383-60458408 | AUGUUUAGGGGGAACUGC AUUUUA | 913 |
| 53335_4_36 1 | BCL11 a | Exon 4 | - | chr2:60458384-60458409 | UAUGUUUAGGGGGGAACUG CAUUUU | 914 |
| 53335_4_36 2 | BCL11 a | Exon 4 | - | chr2:60458398-60458423 | AAAAAACACUUCAUUAUGU UUAGGG | 915 |
| 53335_4_36 3 | BCL11 a | Exon 4 | - | chr2:60458399-60458424 | UAAAAAACACUUCAUUAUG UUUAGG | 916 |
| 53335_4_36 4 | BCL11 a | Exon 4 | - | chr2:60458400-60458425 | UUAAAAAACACUUCAUUAU GUUUAG | 917 |
| 53335_4_36 5 | BCL11 a | Exon 4 | - | chr2:60458401-60458426 | UUUAAAAAACACUUCAUUA UGUUUA | 918 |
| 53335_4_36 6 | BCL11 a | Exon 4 | - | chr2:60458402-60458427 | UUUUAAAAAACACUUCAUU AUGUUU | 919 |
| 53335_4_36 7 | BCL11 a | Exon 4 | - | chr2:60458478-60458503 | UGAUUGCUUUCGCUUCUAC AGUGCA | 920 |
| 53335_4_36 8 | BCL11 a | Exon 4 | - | chr2:60458535-60458560 | GACGCAACAUUGCAAGCGC UGUGAA | 921 |
| 53335_4_36 9 | BCL11 a | Exon 4 | - | chr2:60458562-60458587 | UACUUGACUAUUGAGCUUA CUUACU | 922 |
| 53335_4_37 0 | BCL11 a | Exon 4 | - | chr2:60458634-60458659 | AAAAAAAUUGAACACAAUC UCAUUG | 923 |
| 53335_4_37 1 | BCL11 a | Exon 4 | - | chr2:60458682-60458707 | UCCCAGUUUACAGGUCUAU ACUUAA | 924 |
| 53335_4_37 2 | BCL11 a | Exon 4 | - | chr2:60458683-60458708 | UUCCCAGUUUACAGGUCUA UACUUA | 925 |
| 53335_4_37 3 | BCL11 a | Exon 4 | - | chr2:60458696-60458721 | GCACUGUACAAUUUUCCCA GUUUAC | 926 |
| 53335_4_37 4 | BCL11 a | Exon 4 | - | chr2:60458734-60458759 | GAGUAUAAAAUAAACCUGC UCAGAU | 927 |
| 53335_4_37 5 | BCL11 a | Exon 4 | - | chr2:60458764-60458789 | AUCUUUUCUCUAAUCAGAG AUACAG | 928 |
| 53335_4_37 6 | BCL11 a | Exon 4 | - | chr2:60458829-60458854 | AAUAAAAGCUAGCAUCUGC CCCAGU | 929 |
| 53335_4_37 7 | BCL11 a | Exon 4 | - | chr2:60458897-60458922 | UGUGCUAUUUGUGUUAACA UGGAAG | 930 |
| 53335_4_37 8 | BCL11 a | Exon 4 | - | chr2:60458903-60458928 | ACACUGUGUGCUAUUUGUG UUAACA | 931 |
| 53335_4_37 9 | BCL11 a | Exon 4 | - | chr2:60459085-60459110 | ACUAUUUGCCAUUUAAAAC UAGAAC | 932 |
| 53335_4_38 0 | BCL11 a | Exon 4 | - | chr2:60459114-60459139 | AAUAAUAUGAUUUAUUAGC ACAACG | 933 |
| 53335_4_38 1 | BCL11 a | Exon 4 | - | chr2:60459152-60459177 | AGGCUGACAAUAAGGUUUG ACAGAG | 934 |
| 53335_4_38 2 | BCL11 a | Exon 4 | - | chr2:60459153-60459178 | GAGGCUGACAAUAAGGUUU GACAGA | 935 |
| 53335_4_38 3 | BCL11 a | Exon 4 | - | chr2:60459154-60459179 | AGAGGCUGACAAUAAGGUU UGACAG | 936 |
| 53335_4_38 4 | BCL11 a | Exon 4 | - | chr2:60459165-60459190 | UAUUGAAAGGAAGAGGCUG ACAAUA | 937 |
| 53335_4_38 5 | BCL11 a | Exon 4 | - | chr2:60459177-60459202 | CCUUGUAUACCAUAUUGAA AGGAAG | 938 |
| 53335_4_38 6 | BCL11 a | Exon 4 | - | chr2:60459183-60459208 | UUAAGACCUUGUAUACCAU AUUGAA | 939 |
| 53335_4_38 7 | BCL11 a | Exon 4 | - | chr2:60459229-60459254 | GAUCCAGAUCUACUUGGUU GUCAAG | 940 |
| 53335_4_38 8 | BCL11 a | Exon 4 | - | chr2:60459240-60459265 | CAAAAGAAAUAGAUCCAGA UCUACU | 941 |
| 53335_4_38 9 | BCL11 a | Exon 4 | - | chr2:60459271-60459296 | UUUAAUAAUGUCUUUUUAA AAAUAC | 942 |
| 53335_4_39 0 | BCL11 a | Exon 4 | - | chr2:60459323-60459348 | UGGAACGCAAUUAAAUACA CUAGUA | 943 |
| 53335_4_39 1 | BCL11 a | Exon 4 | - | chr2:60459348-60459373 | UUGAAUGUGUAAUGUGCAA AAGCCC | 944 |
| 53335_4_39 2 | BCL11 a | Exon 4 | - | chr2:60459418-60459443 | CUCCUGGUGAGAGCUUAAA AGAAAU | 945 |
| 53335_4_39 3 | BCL11 a | Exon 4 | - | chr2:60459419-60459444 | GCUCCUGGUGAGAGCUUAA AAGAAA | 946 |
| 53335_4_39 4 | BCL11 a | Exon 4 | - | chr2:60459439-60459464 | ACCAGUAUAAAAGCUACUU UGCUCC | 947 |
| 53335_4_39 5 | BCL11 a | Exon 4 | - | chr2:60459547-60459572 | UUCUAUAUUGUAUUUCUCA CAACAA | 948 |
| 53335_4_39 6 | BCL11 a | Exon 4 | - | chr2:60459588-60459613 | AGCAUUGGUGAGGUAAUA AACCUU | 949 |
| 53335_4_39 7 | BCL11 a | Exon 4 | - | chr2:60459602-60459627 | UUGUAGCUUAAUUCAGCAU UGGGUG | 950 |
| 53335_4_39 8 | BCL11 a | Exon 4 | - | chr2:60459607-60459632 | UAAACUUGUAGCUUAAUUC AGCAUU | 951 |
| 53335_4_39 9 | BCL11 a | Exon 4 | - | chr2:60459608-60459633 | AUAAACUUGUAGCUUAAUU CAGCAU | 952 |
| 53335_4_40 0 | BCL11 a | Exon 4 | - | chr2:60459651-60459676 | CUUGGAUCUAUUAAAACCA CAUCGA | 953 |
| 53335_4_40 1 | BCL11 a | Exon 4 | - | chr2:60459674-60459699 | AUUUGGUUUUAAAAUAUGA GUGCCU | 954 |
| 53335_4_40 2 | BCL11 a | Exon 4 | - | chr2:60459696-60459721 | AACAGAACAAGUUUAUUCU AUCAUU | 955 |
| 53335_4_40 3 | BCL11 a | Exon 4 | - | chr2:60459749-60459774 | UGUAUCAAUUGGAAAGGAA GAAAAA | 956 |
| 53335_4_40 4 | BCL11 a | Exon 4 | - | chr2:60459760-60459785 | AAAGGGUUAAAUGUAUCAA UUGGAA | 957 |
| 53335_4_40 5 | BCL11 a | Exon 4 | - | chr2:60459765-60459790 | UCUCUAAAGGUUAAAUGU AUCAAU | 958 |
| 53335_4_40 6 | BCL11 a | Exon 4 | - | chr2:60459782-60459807 | UAUGAGCUAAAUGUCUGUC UCUAAA | 959 |
| 53335_4_40 7 | BCL11 a | Exon 4 | - | chr2:60459783-60459808 | CUAUGAGCUAAAUGUCUGU CUCUAA | 960 |
| 53335_4_410 8 | BCL11 a | Exon 4 | - | chr2:60459855-60459880 | UAACGUGAGGAGGAAAAAC AGUCUU | 961 |
| 53335_4_40 9 | BCL11 a | Exon 4 | - | chr2:60459870-60459895 | UACAGUUUUAUUUUAUAAC GUGAGG | 962 |
| 53335_4_41 0 | BCL11 a | Exon 4 | - | chr2:60459873-60459898 | AUGUACAGUUUUAUUUUAU AACGUG | 963 |
| 53335_4_41 1 | BCL11 a | Exon 4 | - | chr2:60460023-60460048 | CUUAGACAGCAUGUAUGGU AUGUUA | 964 |
| 53335_4_41 2 | BCL11 a | Exon 4 | - | chr2:60460033-60460058 | UUUUUUUAAACUUAGACAG CAUGUA | 965 |
| 53335_4_41 3 | BCL11 a | Exon 4 | - | chr2:60460130-60460155 | ACCGUUUGAAUGCAUGAUC UGUAUG | 966 |
| 53335_4_41 4 | BCL11 a | Exon 4 | - | chr2:60460131-60460156 | CACCGUUUGAAUGCAUGAU CUGUAU | 967 |
| 53335_4_41 5 | BCL11 a | Exon 4 | - | chr2:60460132-60460157 | UCACCGUUUGAAUGCAUGA UCUGUA | 968 |
| 53335_4_41 6 | BCL11 a | Exon 4 | - | chr2:60460314-60460339 | AUCGCCCUCCAGCCCCACUC CCUGU | 969 |
| 53335_4_41 7 | BCL11 a | Exon 4 | - | chr2:60460403-60460428 | GAAUAAUGAUAUAAAAACU GAAUAG | 970 |
| 53335_4_41 8 | BCL11 a | Exon 4 | - | chr2:60460448-60460473 | UACCCUGGAGAAACACAUG AAAAAA | 971 |
| 53335_4_41 9 | BCL11 a | Exon 4 | - | chr2:60460468-60460493 | AUGCCUUUUAGCGUGUACA GUACCC | 972 |
| 53335_4_42 0 | BCL11 a | Exon 4 | - | chr2:60460522-60460547 | AUGAAAACGCAUGGCCAGG UGGGGA | 973 |
| 53335_4_42 1 | BCL11 a | Exon 4 | - | chr2:60460526-60460551 | GCACAUGAAAACGCAUGGC CAGGUG | 974 |
| 53335_4_42 2 | BCL11 a | Exon 4 | - | chr2:60460527-60460552 | GGCACAUGAAAACGCAUGG CCAGGU | 975 |
| 53335_4_42 3 | BCL11 a | Exon 4 | - | chr2:60460528-60460553 | AGGCACAUGAAAACGCAUG GCCAGG | 976 |
| 53335_4_42 4 | BCL11 a | Exon 4 | - | chr2:60460531-60460556 | ACCAGGCACAUGAAAACGC AUGGCC | 977 |
| 53335_4_42 5 | BCL11 a | Exon 4 | - | chr2:60460536-60460561 | AGCUCACCAGGCACAUGAA AACGCA | 978 |
| 53335_4_42 6 | BCL11 a | Exon 4 | - | chr2:60460553-60460578 | CUGUGCCCAGAGUAGCAAG CUCACC | 979 |
| 53335_4_42 7 | BCL11 a | Exon 4 | - | chr2:60460610-60460635 | CCACAGGAGAAGCCACACG GGCGAA | 980 |
| 53335_4_42 8 | BCL11 a | Exon 4 | - | chr2:60460617-60460642 | UCACUGUCCACAGGAGAAG CCACAC | 981 |
| 53335_4_42 9 | BCL11 a | Exon 4 | - | chr2:60460618-60460643 | CUCACUGUCCACAGGAGAA GCCACA | 982 |
| 53335_4_43 0 | BCL11 a | Exon 4 | - | chr2:60460631-60460656 | GAACUGUAGCAAUCUCACU GUCCAC | 983 |
| 53335_4_43 1 | BCL11 a | Exon 4 | - | chr2:60460670-60460695 | ACGCAGCGACACUUGUGAG UACUGU | 984 |
| 53335_4_43 2 | BCL11 a | Exon 4 | - | chr2:60460671-60460696 | GACGCAGCGACACUUGUGA GUACUG | 985 |
| 53335_4_43 3 | BCL11 a | Exon 4 | - | chr2:60460701-60460726 | GCCCGGGCAGGCCCAGCUC AAAAGA | 986 |
| 53335_4_43 4 | BCL11 a | Exon 4 | - | chr2:60460702-60460727 | GGCCCGGGCAGGCCCAGCU CAAAAG | 987 |
| 53335_4_43 5 | BCL11 a | Exon 4 | - | chr2:60460718-60460743 | CCAUAUUAGUGGUCCGGGC CCGGGC | 988 |
| 53335_4_43 6 | BCL11 a | Exon 4 | - | chr2:60460722-60460747 | CGCCCCAUAUUAGUGGUCC GGGCCC | 989 |
| 53335_4_43 7 | BCL11 a | Exon 4 | - | chr2:60460723-60460748 | ACGCCCCAUAUUAGUGGUC CGGGCC | 990 |
| 53335_4_43 8 | BCL11 a | Exon 4 | - | chr2:60460728-60460753 | GGAGCACGCCCCAUAUUAG UGGUCC | 991 |
| 53335_4_43 9 | BCL11 a | Exon 4 | - | chr2:60460729-60460754 | GGGAGCACGCCCCAUAUUA GUGGUC | 992 |
| 53335_4_44 0 | BCL11 a | Exon 4 | - | chr2:60460734-60460759 | GUGGAGGGAGCACGCCCCA UAUUAG | 993 |
| 53335_4_44 1 | BCL11 a | Exon 4 | - | chr2:60460754-60460779 | GGGGCGCAGCGGCACGGGA AGUGGA | 994 |
| 53335_4_44 2 | BCL11 a | Exon 4 | - | chr2:60460755-60460780 | CGGGGCGCAGCGGCACGGG AAGUGG | 995 |
| 53335_4_44 3 | BCL11 a | Exon 4 | - | chr2:60460758-60460783 | UCUCGGGGCGCAGCGGCAC GGGAAG | 996 |
| 53335_4_44 4 | BCL11 a | Exon 4 | - | chr2:60460764-60460789 | GAGGGAUCUCGGGGCGCAG CGGCAC | 997 |
| 53335_4_44 5 | BCL11 a | Exon 4 | - | chr2:60460765-60460790 | GGAGGGAUCUCGGGGCGCA GCGGCA | 998 |
| 53335_4_44 6 | BCL11 a | Exon 4 | - | chr2:60460770-60460795 | UGGACGGAGGGAUCUCGGG GCGCAG | 999 |
| 53335_4_44 7 | BCL11 a | Exon 4 | - | chr2:60460778-60460803 | CGGGGAGCUGGACGGAGGG AUCUCG | 1000 |
| 53335_4_44 8 | BCL11 a | Exon 4 | - | chr2:60460779-60460804 | CCGGGGAGCUGGACGGAGG GAUCUC | 1001 |
| 53335_4_44 9 | BCL11 a | Exon 4 | - | chr2:60460780-60460805 | CCCGGGAGCUGGACGGAG GGAUCU | 1002 |
| 53335_4_45 0 | BCL11 a | Exon 4 | - | chr2:60460787-60460812 | CACACCGCCCGGGGAGCUG GACGGA | 1003 |
| 53335_4_45 1 | BCL11 a | Exon 4 | - | chr2:60460788-60460813 | CCACACCGCCCGGGAGCU GGACGG | 1004 |
| 53335_4_45 2 | BCL11 a | Exon 4 | - | chr2:60460791-60460816 | UCUCCACACCGCCCGGGGA GCUGGA | 1005 |
| 53335_4_45 3 | BCL11 a | Exon 4 | - | chr2:60460795-60460820 | CGCUUCUCCACACCGCCCG GGGAGC | 1006 |
| 53335_4_45 4 | BCL11 a | Exon 4 | - | chr2:60460801-60460826 | AGUUUGCGCUUCUCCACAC CGCCCG | 1007 |
| 53335_4_45 5 | BCL11 a | Exon 4 | - | chr2:60460802-60460827 | GAGUUUGCGCUUCUCCACA CCGCCC | 1008 |
| 53335_4_45 6 | BCL11 a | Exon 4 | - | chr2:60460803-60460828 | GGAGUUUGCGCUUCUCCAC ACCGCC | 1009 |
| 53335_4_45 7 | BCL11 a | Exon 4 | - | chr2:60460829-60460854 | CUCGUCGGAGCACUCCUCG GAGAAC | 1010 |
| 53335_4_45 8 | BCL11 a | Exon 4 | - | chr2:60460830-60460855 | CCUCGUCGGAGCACUCCUC GGAGAA | 1011 |
| 53335_4_45 9 | BCL11 a | Exon 4 | - | chr2:60460837-60460862 | UUUGCCUCCUCGUCGGAGC ACUCCU | 1012 |
| 53335_4_46 0 | BCL11 a | Exon 4 | - | chr2:60460849-60460874 | AGACAAUCGCCUUUUGCCU CCUCGU | 1013 |
| 53335_4_46 1 | BCL11 a | Exon 4 | - | chr2:60460884-60460909 | AGCUCAAAGAUCCCUUCCU UAGCUU | 1014 |
| 53335_4_46 2 | BCL11 a | Exon 4 | - | chr2:60460913-60460938 | GUGGCUCGCCGGCUACGCG GCCUCC | 1015 |
| 53335_4_46 3 | BCL11 a | Exon 4 | - | chr2:60460921-60460946 | UACUCGCAGUGGCUCGCCG GCUACG | 1016 |
| 53335_4_46 4 | BCL11 a | Exon 4 | - | chr2:60460929-60460954 | AGAACGUGUACUCGCAGUG GCUCGC | 1017 |
| 53335_4_46 5 | BCL11 a | Exon 4 | - | chr2:60460937-60460962 | CAACACGGAGAACGUGUAC UCGCAG | 1018 |
| 53335_4_46 6 | BCL11 a | Exon 4 | - | chr2:60460957-60460982 | CUGCCCCGGCCGCGAUGC CCAACA | 1019 |
| 53335_4_46 7 | BCL11 a | Exon 4 | - | chr2:60460975-60461000 | CUCGAGAAGGAGUUCGACC UGCCCC | 1020 |
| 53335_4_46 8 | BCL11 a | Exon 4 | - | chr2:60460993-60461018 | UUCUCUAAGCGCAUCAAGC UCGAGA | 1021 |
| 53335_4_46 9 | BCL11 a | Exon 4 | - | chr2:60461043-60461068 | GGGGCCUGUCCAAAAAGCU GCUGCU | 1022 |
| 53335_4_47 0 | BCL11 a | Exon 4 | - | chr2:60461044-60461069 | GGGGGCCUGUCCAAAAGC UGCUGC | 1023 |
| 53335_4_47 1 | BCL11 a | Exon 4 | - | chr2:60461067-60461092 | GCUCCCCGGGCGAGUCGGC CUCGGG | 1024 |
| 53335_4_47 2 | BCL11 a | Exon 4 | - | chr2:60461068-60461093 | UGCUCCCCGGGCGAGUCGG CCUCGG | 1025 |
| 53335_4_47 3 | BCL11 a | Exon 4 | - | chr2:60461069-60461094 | CUGCUCCCCGGGCGAGUCG GCCUCG | 1026 |
| 53335_4_47 4 | BCL11 a | Exon 4 | - | chr2:60461070-60461095 | GCUGCUCCCCGGGCGAGUC GGCCUC | 1027 |
| 53335_4_47 5 | BCL11 a | Exon 4 | - | chr2:60461071-60461096 | GGCUGCUCCCGGGCGAGU CGGCCU | 1028 |
| 53335_4_47 6 | BCL11 a | Exon 4 | - | chr2:60461077-60461102 | GGCCGCGGCUGCUCCCCGG GCGAGU | 1029 |
| 53335_4_47 7 | BCL11 a | Exon 4 | - | chr2:60461085-60461110 | CUGUUAAUGGCCGCGGCUG CUCCCC | 1030 |
| 53335_4_47 8 | BCL11 a | Exon 4 | - | chr2:60461086-60461111 | ACUGUUAAUGGCCGCGGCU GCUCCC | 1031 |
| 53335_4_47 9 | BCL11 a | Exon 4 | - | chr2:60461097-60461122 | UAGACGAUGGCACUGUUAA UGGCCG | 1032 |
| 53335_4_48 0 | BCL11 a | Exon 4 | - | chr2:60461103-60461128 | ACCGCAUAGACGAUGGCAC UGUUAA | 1033 |
| 53335_4_48 1 | BCL11 a | Exon 4 | - | chr2:60461115-60461140 | CCGGCGAGUCGGACCGCAU AGACGA | 1034 |
| 53335_4_48 2 | BCL11 a | Exon 4 | - | chr2:60461131-60461156 | GACGAAGACUCGGUGGCCG GCGAGU | 1035 |
| 53335_4_48 3 | BCL11 a | Exon 4 | - | chr2:60461139-60461164 | ACACUUGCGACGAAGACUC GGUGGC | 1036 |
| 53335_4_48 4 | BCL11 a | Exon 4 | - | chr2:60461143-60461168 | AGGGACACUUGCGACGAAG ACUCGG | 1037 |
| 53335_4_48 5 | BCL11 a | Exon 4 | - | chr2:60461146-60461171 | CACAGGGACACUUGCGACG AAGACU | 1038 |
| 53335_4_48 6 | BCL11 a | Exon 4 | - | chr2:60461167-60461192 | CACCUGGCCGAGGCCGAGG GCCACA | 1039 |
| 53335_4_48 7 | BCL11 a | Exon 4 | - | chr2:60461168-60461193 | CCACCUGGCCGAGGCCGAG GGCCAC | 1040 |
| 53335_4_48 8 | BCL11 a | Exon 4 | - | chr2:60461175-60461200 | AGCGCGGCCACCUGGCCGA GGCCGA | 1041 |
| 53335_4_48 9 | BCL11 a | Exon 4 | - | chr2:60461176-60461201 | AAGCGCGGCCACCUGGCCG AGGCCG | 1042 |
| 53335_4_49 0 | BCL11 a | Exon 4 | - | chr2:60461182-60461207 | AAGCAUAAGCGCGGCCACC UGGCCG | 1043 |
| 53335_4_49 1 | BCL11 a | Exon 4 | - | chr2:60461188-60461213 | GGCGAGAAGCAUAAGCGCG GCCACC | 1044 |
| 53335_4_49 2 | BCL11 a | Exon 4 | - | chr2:60461196-60461221 | AGGUCCUGGGCGAGAAGCA UAAGCG | 1045 |
| 53335_4_49 3 | BCL11 a | Exon 4 | - | chr2:60461214-60461239 | UCAGCGAGGCCUUCCACCA GGUCCU | 1046 |
| 53335_4_49 4 | BCL11 a | Exon 4 | - | chr2:60461215-60461240 | UUCAGCGAGGCCUUCCACC AGGUCC | 1047 |
| 53335_4_49 5 | BCL11 a | Exon 4 | - | chr2:60461221-60461246 | CAGCACUUCAGCGAGGCCU UCCACC | 1048 |
| 53335_4_49 6 | BCL11 a | Exon 4 | - | chr2:60461233-60461258 | CUCAGCUCCAUGCAGCACU UCAGCG | 1049 |
| 53335_4_49 7 | BCL11 a | Exon 4 | - | chr2:60461263-60461288 | GCCCUGCCCGACGUCAUGC AGGGCA | 1050 |
| 53335_4_49 8 | BCL11 a | Exon 4 | - | chr2:60461268-60461293 | GCCGCGCCCUGCCCGACGU CAUGCA | 1051 |
| 53335_4_49 9 | BCL11 a | Exon 4 | - | chr2:60461269-60461294 | AGCCGCGCCCUGCCCGACG UCAUGC | 1052 |
| 53335_4_50 0 | BCL11 a | Exon 4 | - | chr2:60461304-60461329 | GCUCGCGGGCGCGGUCGU GGGCGU | 1053 |
| 53335_4_50 1 | BCL11 a | Exon 4 | - | chr2:60461305-60461330 | AGCUCGCGGGCGCGGUCG UGGGCG | 1054 |
| 53335_4_50 2 | BCL11 a | Exon 4 | - | chr2:60461310-60461335 | AGAACAGCUCGCGGGGCGC GGUCGU | 1055 |
| 53335_4_50 3 | BCL11 a | Exon 4 | - | chr2:60461311-60461336 | GAGAACAGCUCGCGGGCG CGGUCG | 1056 |
| 53335_4_50 4 | BCL11 a | Exon 4 | - | chr2:60461317-60461342 | CACCACGAGAACAGCUCGC GGGGCG | 1057 |
| 53335_4_50 5 | BCL11 a | Exon 4 | - | chr2:60461322-60461347 | CGCGCCACCACGAGAACAG CUCGCG | 1058 |
| 53335_4_50 6 | BCL11 a | Exon 4 | - | chr2:60461323-60461348 | GCGCGCCACCACGAGAACA GCUCGC | 1059 |
| 53335_4_50 7 | BCL11 a | Exon 4 | - | chr2:60461324-60461349 | GGCGCGCCACCACGAGAAC AGCUCG | 1060 |
| 53335_4_50 8 | BCL11 a | Exon 4 | - | chr2:60461350-60461375 | UACGGCUUCGGGCUGAGCC UGGAGG | 1061 |
| 53335_4_50 9 | BCL11 a | Exon 4 | - | chr2:60461353-60461378 | GACUACGGCUUCGGGCUGA GCCUGG | 1062 |
| 53335_4_51 0 | BCL11 a | Exon 4 | - | chr2:60461356-60461381 | CUGGACUACGGCUUCGGGC UGAGCC | 1063 |
| 53335_4_51 1 | BCL11 a | Exon 4 | - | chr2:60461366-60461391 | CUCGCUCUCCCUGGACUAC GGCUUC | 1064 |
| 53335_4_51 2 | BCL11 a | Exon 4 | - | chr2:60461367-60461392 | UCUCGCUCUCCCUGGACUA CGGCUU | 1065 |
| 53335_4_51 3 | BCL11 a | Exon 4 | - | chr2:60461373-60461398 | ACUGCCUCUCGCUCUCCCU GGACUA | 1066 |
| 53335_4_51 4 | BCL11 a | Exon 4 | - | chr2:60461380-60461405 | CUCCUCGACUGCCUCUCGC UCUCCC | 1067 |
| 53335_4_51 5 | BCL11 a | Exon 4 | - | chr2:60461512-60461537 | GCCAGCAGCGCUCAAGU CCGUGG | 1068 |
| 53335_4_51 6 | BCL11 a | Exon 4 | - | chr2:60461515-60461540 | AGCGCCAGCAGCGCGCUCA AGUCCG | 1069 |
| 53335_4_51 7 | BCL11 a | Exon 4 | - | chr2:60461544-60461569 | CGGAACCCGGCACCAGCGA CUUGGU | 1070 |
| 53335_4_51 8 | BCL11 a | Exon 4 | - | chr2:60461545-60461570 | CCGGAACCCGGCACCAGCG ACUUGG | 1071 |
| 53335_4_51 9 | BCL11 a | Exon 4 | - | chr2:60461548-60461573 | UCCCCGGAACCCGGCACCA GCGACU | 1072 |
| 53335_4_52 0 | BCL11 a | Exon 4 | - | chr2:60461562-60461587 | UCUCCACCGCCAGCUCCCCG GAACC | 1073 |
| 53335_4_52 1 | BCL11 a | Exon 4 | - | chr2:60461569-60461594 | GACGGUCUCUCCACCGCCA GCUCCC | 1074 |
| 53335_4_52 2 | BCL11 a | Exon 4 | - | chr2:60461592-60461617 | CCCCCAUGACGGUCAAGUC CGACGA | 1075 |
| 53335_4_52 3 | BCL11 a | Exon 4 | - | chr2:60461608-60461633 | CACAUGCACAAAUCGUCCC CCAUGA | 1076 |
| 53335_4_52 4 | BCL11 a | Exon 4 | - | chr2:60461665-60461690 | AACCUGUGCGACCACGCGU GCACCC | 1077 |
| 53335_4_52 5 | BCL11 a | Exon 4 | - | chr2:60461712-60461737 | UGGUGGUGCACCGGCGCAG CCACAC | 1078 |
| 53335_4_52 6 | BCL11 a | Exon 4 | - | chr2:60461713-60461738 | CUGGUGGUGCACCGGCGCA GCCACA | 1079 |
| 53335_4_52 7 | BCL11 a | Exon 4 | - | chr2:60461726-60461751 | AUUUCAGAGCAACCUGGUG GUGCAC | 1080 |
| 53335_4_52 8 | BCL11 a | Exon 4 | - | chr2:60461734-60461759 | ACGUUCAAAUUUCAGAGCA ACCUGG | 1081 |
| 53335_4_52 9 | BCL11 a | Exon 4 | - | chr2:60461737-60461762 | AAGACGUUCAAAUUUCAGA GCAACC | 1082 |
| 53335_4_53 0 | BCL11 a | Exon 4 | - | chr2:60461766-60461791 | UCAAGUCCAAGUCAUGCGA GUUCUG | 1083 |
| 53335_4_53 1 | BCL11 a | Exon 4 | - | chr2:60461794-60461819 | UCCGCCCCUCCUCCCUCCCA GCCCC | 1084 |
| 53335_4_53 2 | BCL11 a | Exon 4 | - | chr2:60461848-60461873 | CAGCCAGGUAGCAAGCCGC CCUUCC | 1085 |
| 53335_4_53 3 | BCL11 a | Exon 4 | - | chr2:60461868-60461893 | AAAGGUUACUGCAACCAUU CCAGCC | 1086 |
| 53335_4_53 4 | BCL11 a | Exon 4 | - | chr2:60461891-60461916 | CCCAGGCCGGCCCAGCCCU AUGCAA | 1087 |
| 53335_4_53 5 | BCL11 a | Exon 4 | - | chr2:60461909-60461934 | GUCUAGCCCACCGCUGUCC CCAGGC | 1088 |
| 53335_4_53 6 | BCL11 a | Exon 4 | - | chr2:60461913-60461938 | ACACGUCUAGCCCACCGCU GUCCCC | 1089 |
| 53335_4_53 7 | BCL11 a | Exon 4 | - | chr2:60461942-60461967 | CUCUAGGAGACUUAGAGAG CUGGCA | 1090 |
| 53335_4_53 8 | BCL11 a | Exon 4 | - | chr2:60461943-60461968 | UCUCUAGGAGACUUAGAGA GCUGGC | 1091 |
| 53335_4_53 9 | BCL11 a | Exon 4 | - | chr2:60461947-60461972 | GAUUUCUCUAGGAGACUUA GAGAGC | 1092 |
| 53335_4_54 0 | BCL11 a | Exon 4 | - | chr2:60461963-60461988 | GGAGCCUCCCGCCAUGGAU UUCUCU | 1093 |
| 53335_4_54 1 | BCL11 a | Exon 4 | - | chr2:60461974-60461999 | CCAAUGGCUAUGGAGCCUC CCGCCA | 1094 |
| 53335_4_54 2 | BCL11 a | Exon 4 | - | chr2:60461989-60462014 | GUGCUGCGGUUGAAUCCAA UGGCUA | 1095 |
| 53335_4_54 3 | BCL11 a | Exon 4 | - | chr2:60461995-60462020 | GACAGGGUGCUGCGGUUGA AUCCAA | 1096 |
| 53335_4_54 4 | BCL11 a | Exon 4 | - | chr2:60462008-60462033 | CCCGAGUGCCUUUGACAGG GUGCUG | 1097 |
| 53335_4_54 5 | BCL11 a | Exon 4 | - | chr2:60462016-60462041 | ACCCAUCACCCGAGUGCCU UUGACA | 1098 |
| 53335_4_54 6 | BCL11 a | Exon 4 | - | chr2:60462017-60462042 | CACCCAUCACCCGAGUGCC UUUGAC | 1099 |
| 53335_4_54 7 | BCL11 a | Exon 4 | - | chr2:60462046-60462071 | CGCCUGGGGGCGGAAGAGA UGGCCC | 1100 |
| 53335_4_54 8 | BCL11 a | Exon 4 | - | chr2:60462052-60462077 | AUAGAGCGCCUGGGGGCGG AAGAGA | 1101 |
| 53335_4_54 9 | BCL11 a | Exon 4 | - | chr2:60462061-60462086 | CCCCACCGCAUAGAGCGCC UGGGGG | 1102 |
| 53335_4_55 0 | BCL11 a | Exon 4 | - | chr2:60462064-60462089 | GACCCCCACCGCAUAGAGC GCCUGG | 1103 |
| 53335_4_55 1 | BCL11 a | Exon 4 | - | chr2:60462065-60462090 | GGACCCCCACCGCAUAGAG CGCCUG | 1104 |
| 53335_4_55 2 | BCL11 a | Exon 4 | - | chr2:60462066-60462091 | UGGACCCCCACCGCAUAGA GCGCCU | 1105 |
| 53335_4_55 3 | BCL11 a | Exon 4 | - | chr2:60462067-60462092 | UUGGACCCCCACCGCAUAG AGCGCC | 1106 |
| 53335_4_55 4 | BCL11 a | Exon 4 | - | chr2:60462091-60462116 | UUUAGUCCACCACCGAGAC AUCACU | 1107 |
| 53335_4_55 5 | BCL11 a | Exon 4 | - | chr2:60462143-60462168 | GAGAGAGGCUUCCGGCCUG GCAGAA | 1108 |
| 53335_4_55 6 | BCL11 a | Exon 4 | - | chr2:60462144-60462169 | CGAGAGAGGCUUCCGGCCU GGCAGA | 1109 |
| 53335_4_55 7 | BCL11 a | Exon 4 | - | chr2:60462151-60462176 | UCAGUAUCGAGAGAGGCUU CCGGCC | 1110 |
| 53335_4_55 8 | BCL11 a | Exon 4 | - | chr2:60462156-60462181 | CAGGAUCAGUAUCGAGAGA GGCUUC | 1111 |
| 53335_4_55 9 | BCL11 a | Exon 4 | - | chr2:60462163-60462188 | AGAAUACCAGGAUCAGUAU CGAGAG | 1112 |
| 53335_4_56 0 | BCL11 a | Exon 4 | - | chr2:60462180-60462205 | ACCCCUUUAACCUGCUAAG AAUACC | 1113 |
| 53335_4_56 1 | BCL11 a | Exon 4 | - | chr2:60462227-60462252 | AUGUCCUUCCCAGCCACCU CUCCAU | 1114 |
| 53335_4_56 2 | BCL11 a | Exon 4 | - | chr2:60462228-60462253 | AAUGUCCUUCCCAGCCACC UCUCCA | 1115 |
| 53335_4_56 3 | BCL11 a | Exon 4 | - | chr2:60462261-60462286 | CGCGGGUUGGUAUCCCUUC AGGACU | 1116 |
| 53335_4_56 4 | BCL11 a | Exon 4 | - | chr2:60462267-60462292 | UGACCCCGCGGUUGGUAU CCCUUC | 1117 |
| 53335_4_56 5 | BCL11 a | Exon 4 | - | chr2:60462279-60462304 | ACGGAAGUCCCCUGACCCC GCGGGU | 1118 |
| 53335_4_56 6 | BCL11 a | Exon 4 | - | chr2:60462283-60462308 | GAACACGGAAGUCCCCUGA CCCCGC | 1119 |
| 53335_4_56 7 | BCL11 a | Exon 4 | - | chr2:60462284-60462309 | CGAACACGGAAGUCCCCUG ACCCCG | 1120 |
| 53335_4_56 8 | BCL11 a | Exon 4 | - | chr2:60462303-60462328 | UAAGAAUCUACUUAGAAAG CGAACA | 1121 |
| 53335_4_56 9 | BCL11 a | Exon 4 | - | chr2:60462333-60462358 | UCUUGCAACACGCACAGAA CACUCA | 1122 |
| 53335_4_57 0 | BCL11 a | Exon 4 | - | chr2:60462365-60462390 | UUGCAAACAGCCAUUCACC AGUGCA | 1123 |
| 53335_3_1 | BCL11 a | Exon 3 | + | chr2:60468716-60468741 | UAAGGCUCAACUUACAAAU ACCCUG | 1124 |
| 53335_3_2 | BCL11 a | Exon 3 | + | chr2:60468717-60468742 | AAGGCUCAACUUACAAAUA CCCUGC | 1125 |
| 53335_3_3 | BCL11 a | Exon 3 | + | chr2:60468718-60468743 | AGGCUCAACUUACAAAUAC CCUGCG | 1126 |
| 53335_3_4 | BCL11 a | Exon 3 | + | chr2:60468740-60468765 | GCGGGCAUAUUCUGCACU CAUCCC | 1127 |
| 53335_3_5 | BCL11 a | Exon 3 | + | chr2:60468745-60468770 | GCAUAUUCUGCACUCAUCC CAGGCG | 1128 |
| 53335_3_6 | BCL11 a | Exon 3 | + | chr2:60468746-60468771 | CAUAUUCUGCACUCAUCCC AGGCGU | 1129 |
| 53335_3_7 | BCL11 a | Exon 3 | + | chr2:60468747-60468772 | AUAUUCUGCACUCAUCCCA GGCGUG | 1130 |
| 53335_3_8 | BCL11 a | Exon 3 | + | chr2:60468773-60468798 | GGAUUAGAGCUCCAUGUGC AGAACG | 1131 |
| 53335_3_9 | BCL11 a | Exon 3 | + | chr2:60468774-60468799 | GAUUAGAGCUCCAUGUGCA GAACGA | 1132 |
| 53335_3_10 | BCL11 a | Exon 3 | + | chr2:60468775-60468800 | AUUAGAGCUCCAUGUGCAG AACGAG | 1133 |
| 53335_3_11 | BCL11 a | Exon 3 | + | chr2:60468778-60468803 | AGAGCUCCAUGUGCAGAAC GAGGGG | 1134 |
| 53335_3_12 | BCL11 a | Exon 3 | + | chr2:60468783-60468808 | UCCAUGUGCAGAACGAGGG GAGGAG | 1135 |
| 53335_3_13 | BCL11 a | Exon 3 | - | chr2:60468739-60468764 | GGAUGAGUGCAGAAUAUGC CCCGCA | 1136 |
| 53335_3_14 | BCL11 a | Exon 3 | - | chr2:60468740-60468765 | GGGAUGAGUGCAGAAUAUG CCCCGC | 1137 |
| 53335_3_15 | BCL11 a | Exon 3 | - | chr2:60468765-60468790 | ACAUGGAGCUCUAAUCCCC ACGCCU | 1138 |
| 53335_3_16 | BCL11 a | Exon 3 | - | chr2:60468766-60468791 | CACAUGGAGCUCUAAUCCC CACGCC | 1139 |
| 53335_3_17 | BCL11 a | Exon 3 | - | chr2:60468787-60468812 | GCCUCUCCUCCCCUCGUUC UGCACA | 1140 |
| 53335_3_18 | BCL11 a | Exon 3 | - | chr2:60468814-60468839 | UCACAGAUAAACUUCUGCA CUGGAG | 1141 |
| 53335_3_19 | BCL11 a | Exon 3 | - | chr2:60468815-60468840 | UUCACAGAUAAACUUCUGC ACUGGA | 1142 |
| 53335_3_20 | BCL11 a | Exon 3 | - | chr2:60468816-60468841 | UUUCACAGAUAAACUUCUG CACUGG | 1143 |
| 53335_3_21 | BCL11 a | Exon 3 | - | chr2:60468819-60468844 | UUCUUUCACAGAUAAACUU CUGCAC | 1144 |
| 53335_2_1 | BCL11 a | Exon 2 | + | chr2:60545963-60545988 | CAUUUACCUGCUAUGUGUU CCUGUU | 1145 |
| 53335_2_2 | BCL11 a | Exon 2 | + | chr2:60545964-60545989 | AUUUACCUGCUAUGUGUUC CUGUUU | 1146 |
| 53335_2_3 | BCL11 a | Exon 2 | + | chr2:60545965-60545990 | UUUACCUGCUAUGUGUUCC UGUUUG | 1147 |
| 53335_2_4 | BCL11 a | Exon 2 | + | chr2:60546009-60546034 | ACGUUGAUAAACAAUCGUC AUCCUC | 1148 |
| 53335_2_5 | BCL11 a | Exon 2 | + | chr2:60546019-60546044 | ACAAUCGUCAUCCUCUGGC GUGACC | 1149 |
| 53335_2_6 | BCL11 a | Exon 2 | + | chr2:60546035-60546060 | GGCGUGACCUGGAUGCCAA CCUCCA | 1150 |
| 53335_2_7 | BCL11 a | Exon 2 | + | chr2:60546036-60546061 | GCGUGACCUGGAUGCCAAC CUCCAC | 1151 |
| 53335_2_8 | BCL11 a | Exon 2 | + | chr2:60546041-60546066 | ACCUGGAUGCCAACCUCCA CGGGAU | 1152 |
| 53335_2_9 | BCL11 a | Exon 2 | + | chr2:60546063-60546088 | GAUUGGAUGCUUUUUUCAU CUCGAU | 1153 |
| 53335_2_10 | BCL11 a | Exon 2 | + | chr2:60546069-60546094 | AUGCUUUUUUCAUCUCGAU UGGUGA | 1154 |
| 53335_2_11 | BCL11 a | Exon 2 | + | chr2:60546070-60546095 | UGCUUUUUUCAUCUCGAUU GGUGAA | 1155 |
| 53335_2_12 | BCL11 a | Exon 2 | + | chr2:60546071-60546096 | GCUUUUUUCAUCUCGAUUG GUGAAG | 1156 |
| 53335_2_13 | BCL11 a | Exon 2 | + | chr2:60546075-60546100 | UUUUCAUCUCGAUUGGUGA AGGGGA | 1157 |
| 53335_2_14 | BCL11 a | Exon 2 | + | chr2:60546078-60546103 | UCAUCUCGAUUGGUGAAGG GGAAGG | 1158 |
| 53335_2_15 | BCL11 a | Exon 2 | + | chr2:60546106-60546131 | CUUAUCCACAGCUUUUUCU AAGCAG | 1159 |
| 53335_2_16 | BCL11 a | Exon 2 | + | chr2:60546162-60546187 | CGAUAAAAUAAGAAUGUC CCCCAA | 1160 |
| 53335_2_17 | BCL11 a | Exon 2 | + | chr2:60546163-60546188 | GAUAAAAAUAAGAAUGUCC CCCAAU | 1161 |
| 53335_2_18 | BCL11 a | Exon 2 | + | chr2:60546175-60546200 | AAUGUCCCCCAAUGGGAAG UUCAUC | 1162 |
| 53335_2_19 | BCL11 a | Exon 2 | + | chr2:60546188-60546213 | GGGAAGUUCAUCUGGCACU GCCCAC | 1163 |
| 53335_2_20 | BCL11 a | Exon 2 | + | chr2:60546193-60546218 | GUUCAUCUGGCACUGCCCA CAGGUG | 1164 |
| 53335_2_21 | BCL11 a | Exon 2 | + | chr2:60546196-60546221 | CAUCUGGCACUGCCCACAG GUGAGG | 1165 |
| 53335_2_22 | BCL11 a | Exon 2 | + | chr2:60546213-60546238 | AGGUGAGGAGGUCAUGAUC CCCUUC | 1166 |
| 53335_2_23 | BCL11 a | Exon 2 | + | chr2:60546225-60546250 | CAUGAUCCCCUUCUGGAGC UCCCAA | 1167 |
| 53335_2_24 | BCL11 a | Exon 2 | + | chr2:60546226-60546251 | AUGAUCCCCUUCUGGAGCU CCCAAC | 1168 |
| 53335_2_25 | BCL11 a | Exon 2 | + | chr2:60546232-60546257 | CCCUUCUGGAGCUCCCAAC GGGCCG | 1169 |
| 53335_2_26 | BCL11 a | Exon 2 | + | chr2:60546237-60546262 | CUGGAGCUCCCAACGGGCC GUGGUC | 1170 |
| 53335_2_27 | BCL11 a | Exon 2 | + | chr2:60546257-60546282 | UGGUCUGGUUCAUCAUCUG UAAGAA | 1171 |
| 53335_2_28 | BCL11 a | Exon 2 | + | chr2:60546267-60546292 | CAUCAUCUGUAAGAAUGGC UUCAAG | 1172 |
| 53335_2_29 | BCL11 a | Exon 2 | + | chr2:60546272-60546297 | UCUGUAAGAAUGGCUUCAA GAGGCU | 1173 |
| 53335_2_30 | BCL11 a | Exon 2 | + | chr2:60546278-60546303 | AGAAUGGCUUCAAGAGGCU CGGCUG | 1174 |
| 53335_2_31 | BCL11 a | Exon 2 | + | chr2:60546282-60546307 | UGGCUUCAAGAGGCUCGGC UGUGGU | 1175 |
| 53335_2_32 | BCL11 a | Exon 2 | - | chr2:60545956-60545981 | ACACAUAGCAGGUAAAUGA GAAGCA | 1176 |
| 53335_2_33 | BCL11 a | Exon 2 | - | chr2:60545972-60545997 | UUUGCCCCAAACAGGAACA CAUAGC | 1177 |
| 53335_2_34 | BCL11 a | Exon 2 | - | chr2:60545985-60546010 | UCAUCUAGAGGAAUUUGCC CCAAAC | 1178 |
| 53335_2_35 | BCL11 a | Exon 2 | - | chr2:60546002-60546027 | ACGAUUGUUUAUCAACGUC AUCUAG | 1179 |
| 53335_2_36 | BCL11 a | Exon 2 | - | chr2:60546033-60546058 | GAGGUUGGCAUCCAGGUCA CGCCAG | 1180 |
| 53335_2_37 | BCL11 a | Exon 2 | - | chr2:60546045-60546070 | UCCAAUCCCGUGGAGGUUG GCAUCC | 1181 |
| 53335_2_38 | BCL11 a | Exon 2 | - | chr2:60546053-60546078 | AAAAAGCAUCCAAUCCCGU GGAGGU | 1182 |
| 53335_2_39 | BCL11 a | Exon 2 | - | chr2:60546057-60546082 | AUGAAAAAGCAUCCAAUC CCGUGG | 1183 |
| 53335_2_40 | BCL11 a | Exon 2 | - | chr2:60546060-60546085 | GAGAUGAAAAAGCAUCCA AUCCCG | 1184 |
| 53335_2_41 | BCL11 a | Exon 2 | - | chr2:60546114-60546139 | GGCAGCCUCUGCUUAGAAA AAGCUG | 1185 |
| 53335_2_42 | BCL11 a | Exon 2 | - | chr2:60546140-60546165 | UCGAGCACAAACGGAAACA AUGCAA | 1186 |
| 53335_2_43 | BCL11 a | Exon 2 | - | chr2:60546154-60546179 | CAUUCUUAUUUUUAUCGAG CACAAA | 1187 |
| 53335_2_44 | BCL11 a | Exon 2 | - | chr2:60546183-60546208 | CAGUGCCAGAUGAACUUCC CAUUGG | 1188 |
| 53335_2_45 | BCL11 a | Exon 2 | - | chr2:60546184-60546209 | GCAGUGCCAGAUGAACUUC CCAUUG | 1189 |
| 53335_2_46 | BCL11 a | Exon 2 | - | chr2:60546185-60546210 | GGCAGUGCCAGAUGAACUU CCCAUU | 1190 |
| 53335_2_47 | BCL11 a | Exon 2 | - | chr2:60546186-60546211 | GGGCAGUGCCAGAUGAACU UCCCAU | 1191 |
| 53335_2_48 | BCL11 a | Exon 2 | - | chr2:60546211-60546236 | AGGGGAUCAUGACCUCCUC ACCUGU | 1192 |
| 53335_2_49 | BCL11 a | Exon 2 | - | chr2:60546212-60546237 | AAGGGGAUCAUGACCUCCU CACCUG | 1193 |
| 53335_2_50 | BCL11 a | Exon 2 | - | chr2:60546234-60546259 | CACGGCCCGUUGGGAGCUC CAGAAG | 1194 |
| 53335_2_51 | BCL11 a | Exon 2 | - | chr2:60546235-60546260 | CCACGGCCCGUUGGGAGCU CCAGAA | 1195 |
| 53335_2_52 | BCL11 a | Exon 2 | - | chr2:60546236-60546261 | ACCACGGCCCGUUGGGAGC UCCAGA | 1196 |
| 53335_2_53 | BCL11 a | Exon 2 | - | chr2:60546248-60546273 | AUGAUGAACCAGACCACGG CCCGUU | 1197 |
| 53335_2_54 | BCL11 a | Exon 2 | - | chr2:60546249-60546274 | GAUGAUGAACCAGACCACG GCCCGU | 1198 |
| 53335_2_55 | BCL11 a | Exon 2 | - | chr2:60546257-60546282 | UUCUUACAGAUGAUGAACC AGACCA | 1199 |
| 53335_1_1 | BCL11 a | Exon 1 | + | chr2:60553216-60553241 | CGAGAAUUCCCGUUUGCUU AAGUGC | 1200 |
| 53335_1_2 | BCL11 a | Exon 1 | + | chr2:60553217-60553242 | GAGAAUUCCCGUUUGCUUA AGUGCU | 1201 |
| 53335_1_3 | BCL11 a | Exon 1 | + | chr2:60553218-60553243 | AGAAUUCCCGUUUGCUUAA GUGCUG | 1202 |
| 53335_1_4 | BCL11 a | Exon 1 | + | chr2:60553234-60553259 | UAAGUGCUGGGUUUGCCU UGCUUG | 1203 |
| 53335_1_5 | BCL11 a | Exon 1 | + | chr2:60553244-60553269 | GGUUUGCCUUGCUUGCGGC GAGACA | 1204 |
| 53335_1_6 | BCL11 a | Exon 1 | + | chr2:60553247-60553272 | UUGCCUUGCUUGCGGCGAG ACAUGG | 1205 |
| 53335_1_7 | BCL11 a | Exon 1 | + | chr2:60553248-60553273 | UGCCUUGCUUGCGGCGAGA CAUGGU | 1206 |
| 53335_1_8 | BCL11 a | Exon 1 | + | chr2:60553254-60553279 | GCUUGCGGCGAGACAUGGU GGGCUG | 1207 |
| 53335_1_9 | BCL11 a | Exon 1 | + | chr2:60553255-60553280 | CUUGCGGCGAGACAUGGUG GGCUGC | 1208 |
| 53335_1_10 | BCL11 a | Exon 1 | + | chr2:60553256-60553281 | UUGCGGCGAGACAUGGUGG GCUGCG | 1209 |
| 53335_1_11 | BCL11 a | Exon 1 | + | chr2:60553291-60553316 | CGGCGGCGGCGGCGGCGGC GGCGGG | 1210 |
| 53335_1_12 | BCL11 a | Exon 1 | + | chr2:60553298-60553323 | GGCGGCGGCGGCGGCGGGC GGACGA | 1211 |
| 53335_1_13 | BCL11 a | Exon 1 | + | chr2:60553303-60553328 | CGGCGGCGGCGGGCGGACG ACGGCU | 1212 |
| 53335_1_14 | BCL11 a | Exon 1 | + | chr2:60553313-60553338 | GGGCGGACGACGGCUCGGU UCACAU | 1213 |
| 53335_1_15 | BCL11 a | Exon 1 | + | chr2:60553314-60553339 | GGCGGACGACGGCUCGGUU CACAUC | 1214 |
| 53335_1_16 | BCL11 a | Exon 1 | + | chr2:60553322-60553347 | ACGGCUCGGUUCACAUCGG GAGAGC | 1215 |
| 53335_1_17 | BCL11 a | Exon 1 | + | chr2:60553323-60553348 | CGGCUCGGUUCACAUCGGG AGAGCC | 1216 |
| 53335_1_18 | BCL11 a | Exon 1 | + | chr2:60553335-60553360 | CAUCGGGAGAGCCGGGUUA GAAAGA | 1217 |
| 53335_1_19 | BCL11 a | Exon 1 | + | chr2:60553406-60553431 | AAAUAAAUUAGAAAUAAUA CAAAGA | 1218 |
| 53335_1_20 | BCL11 a | Exon 1 | + | chr2:60553412-60553437 | AUUAGAAAUAAUACAAAGA UGGCGC | 1219 |
| 53335_1_21 | BCL11 a | Exon 1 | + | chr2:60553413-60553438 | UUAGAAAUAAUACAAAGAU GGCGCA | 1220 |
| 53335_1_22 | BCL11 a | Exon 1 | + | chr2:60553427-60553452 | AAGAUGGCGCAGGGAAGAU GAAUUG | 1221 |
| 53335_1_23 | BCL11 a | Exon 1 | + | chr2:60553428-60553453 | AGAUGGCGCAGGGAAGAUG AAUUGU | 1222 |
| 53335_1_24 | BCL11 a | Exon 1 | + | chr2:60553441-60553466 | AAGAUGAAUUGUGGGAGAG CCGUCA | 1223 |
| 53335_1_25 | BCL11 a | Exon 1 | - | chr2:60553227-60553252 | GGCAAACCCCAGCACUUAA GCAAAC | 1224 |
| 53335_1_26 | BCL11 a | Exon 1 | - | chr2:60553228-60553253 | AGGCAAACCCCAGCACUUA AGCAAA | 1225 |
| 53335_1_27 | BCL11 a | Exon 1 | - | chr2:60553253-60553278 | AGCCCACCAUGUCUCGCCG CAAGCA | 1226 |
| 53335_1_28 | BCL11 a | Exon 1 | - | chr2:60553349-60553374 | CUCUGGAGUCUCCUUCUUU CUAACC | 1227 |
| 53335_1_29 | BCL11 a | Exon 1 | - | chr2:60553371-60553396 | AAGGCACUGAUGAAGAUAU UUUCUC | 1228 |
| 53335_1_30 | BCL11 a | Exon 1 | - | chr2:60553395-60553420 | UUUCUAAUUUAUUUUGGAU GUCAAA | 1229 |
| 53335_1_31 | BCL11 a | Exon 1 | - | chr2:60553406-60553431 | UCUUUGUAUUAUUUCUAAU UUAUUU | 1230 |
| 53335_1_32 | BCL11 a | Exon 1 | - | chr2:60553463-60553488 | AAAAAAGCUUAAAAAAAAG CCAUGA | 1231 |

**Table 2: gRNA targeting domains targeting BCL11a Intron 2 (e.g., to a BCL11a Enhancer)**

| **Id.** | Target | Target Region | Strand | Targeting Site **(hg38)** | **gRNA** Targeting Domain | SEQ ID NO: |
|---|---|---|---|---|---|---|
| 53335_I2_1 | BCL11a | Intron 2 | + | chr2:60494277-60494302 | GGGAGUUUGGCUUCUCA UCUGUGCA | 1232 |
| 53335_I2_2 | BCL11a | Intron 2 | + | chr2:60494289-60494314 | UCUCAUCUGUGCAUGGC CUCUAAAC | 1233 |
| 53335_I2_3 | BCL11a | Intron 2 | + | chr2:60494290-60494315 | CUCAUCUGUGCAUGGCC UCUAAACU | 1234 |
| 53335_I2_4 | BCL11a | Intron 2 | + | chr2:60494302-60494327 | UGGCCUCUAAACUGGGC AGUGACCA | 1235 |
| 53335_I2_5 | BCL11a | Intron 2 | + | chr2:60494307-60494332 | UCUAAACUGGGCAGUGA CCAUGGCC | 1236 |
| 53335_I2_6 | BCL11a | Intron 2 | + | chr2:60494324-60494349 | CCAUGGCCUGGUCACCU CCCCACUC | 1237 |
| 53335_I2_7 | BCL11a | Intron 2 | + | chr2:60494330-60494355 | CCUGGUCACCUCCCCACU CUGGACC | 1238 |
| 53335_I2_8 | BCL11a | Intron 2 | + | chr2:60494331-60494356 | CUGGUCACCUCCCCACUC UGGACCU | 1239 |
| 53335_I2_9 | BCL11a | Intron 2 | + | chr2:60494351-60494376 | GACCUGGGUUGCCCCUC UGUAAACA | 1240 |
| 53335_I2_10 | BCL11a | Intron 2 | + | chr2:60494354-60494379 | CUGGGUUGCCCCUCUGU AAACAAGG | 1241 |
| 53335_I2_11 | BCL11a | Intron 2 | + | chr2:60494418-60494443 | AAAUCUUAUGCAAUUUU UGCCAAGA | 1242 |
| 53335_I2_12 | BCL11a | Intron 2 | + | chr2:60494419-60494444 | AAUCUUAUGCAAUUUUU GCCAAGAU | 1243 |
| 53335_I2_13 | BCL11a | Intron 2 | + | chr2:60494426-60494451 | UGCAAUUUUUGCCAAGA UGGGAGUA | 1244 |
| 53335_I2_14 | BCL11a | Intron 2 | + | chr2:60494427-60494452 | GCAAUUUUGCCAAGAU GGGAGUAU | 1245 |
| 53335_I2_15 | BCL11a | Intron 2 | + | chr2:60494428-60494453 | CAAUUUUUGCCAAGAUG GGAGUAUG | 1246 |
| 53335_I2_16 | BCL11a | Intron 2 | + | chr2:60494440-60494465 | AGAUGGGAGUAUGGGGA GAGAAGAG | 1247 |
| 53335_I2_17 | BCL11a | Intron 2 | + | chr2:60494446-60494471 | GAGUAUGGGAGAGAAG AGUGGAAA | 1248 |
| 53335_I2_18 | BCL11a | Intron 2 | + | chr2:60494477-60494502 | AGAGCUCAGUGAGAUGA GAUAUCAA | 1249 |
| 53335_I2_19 | BCL11a | Intron 2 | + | chr2:60494478-60494503 | GAGCUCAGUGAGAUGAG AUAUCAAA | 1250 |
| 53335_I2_20 | BCL11a | Intron 2 | + | chr2:60494479-60494504 | AGCUCAGUGAGAUGAGA UAUCAAAG | 1251 |
| 53335_I2_21 | BCL11a | Intron 2 | + | chr2:60494518-60494543 | UCAUUCCAUCUCCCUAA UCUCCAAU | 1252 |
| 53335_I2_22 | BCL11a | Intron 2 | + | chr2:60494533-60494558 | AAUCUCCAAUUGGCAAA GCCAGACU | 1253 |
| 53335_I2_23 | BCL11a | Intron 2 | + | chr2:60494534-60494559 | AUCUCCAAUUGGCAAAG CCAGACUU | 1254 |
| 53335_I2_24 | BCL11a | Intron 2 | + | chr2:60494535-60494560 | UCUCCAAUUGGCAAAGC CAGACUUG | 1255 |
| 53335_I2_25 | BCL11a | Intron 2 | + | chr2:60494548-60494573 | AAGCCAGACUUGGGGCA AUACAGAC | 1256 |
| 53335_I2_26 | BCL11a | Intron 2 | + | chr2:60494617-60494642 | UAUCACCAAAUGUUCUU UCUUCAGC | 1257 |
| 53335_I2_27 | BCL11a | Intron 2 | + | chr2:60494631-60494656 | CUUUCUUCAGCUGGAAU UUAAAAUA | 1258 |
| 53335_I2_28 | BCL11a | Intron 2 | + | chr2:60494649-60494674 | UAAAAUAUGGACUCAUC CGUAAAAU | 1259 |
| 53335_I2_29 | BCL11a | Intron 2 | + | chr2:60494675-60494700 | GGAAUAAUAAUAGUAUA UGCUUCAU | 1260 |
| 53335_I2_30 | BCL11a | Intron 2 | + | chr2:60494676-60494701 | GAAUAAUAAUAGUAUAU GCUUCAUA | 1261 |
| 53335_I2_31 | BCL11a | Intron 2 | + | chr2:60494766-60494791 | GCUUGUGAACUAAAAUG CUGCCUCC | 1262 |
| 53335_I2_32 | BCL11a | Intron 2 | + | chr2:60494806-60494831 | ACACCUCAGCAGAAACA AAGUUAUC | 1263 |
| 53335_I2_33 | BCL11a | Intron 2 | + | chr2:60494830-60494855 | CAGGCCCUUUCCCCAAU UCCUAGUU | 1264 |
| 53335_I2_34 | BCL11a | Intron 2 | + | chr2:60494831-60494856 | AGGCCCUUUCCCCAAUU CCUAGUUU | 1265 |
| 53335_I2_35 | BCL11a | Intron 2 | + | chr2:60494844-60494869 | AAUUCCUAGUUUGGUC AGAAGAAA | 1266 |
| 53335_I2_36 | BCL11a | Intron 2 | + | chr2:60494845-60494870 | AUUCCUAGUUUGGUCA GAAGAAAA | 1267 |
| 53335_I2_37 | BCL11a | Intron 2 | + | chr2:60494851-60494876 | AGUUUGGGUCAGAAGAA AAGGGAAA | 1268 |
| 53335_I2_38 | BCL11a | Intron 2 | + | chr2:60494852-60494877 | GUUUGGGUCAGAAGAAA AGGGAAAA | 1269 |
| 53335_I2_39 | BCL11a | Intron 2 | + | chr2:60494857-60494882 | GGUCAGAAGAAAAGGGA AAAGGGAG | 1270 |
| 53335_I2_40 | BCL11a | Intron 2 | + | chr2:60494864-60494889 | AGAAAAGGAAAAGGA GAGGAAAA | 1271 |
| 53335_I2_41 | BCL11a | Intron 2 | + | chr2:60494883-60494908 | GGAAAAAGGAAAAGAAU AUGACGUC | 1272 |
| 53335_I2_42 | BCL11a | Intron 2 | + | chr2:60494884-60494909 | GAAAAAGGAAAAGAAUA UGACGUCA | 1273 |
| 53335_I2_43 | BCL11a | Intron 2 | + | chr2:60494885-60494910 | AAAAAGGAAAAGAAUAU GACGUCAG | 1274 |
| 53335_I2_44 | BCL11a | Intron 2 | + | chr2:60494886-60494911 | AAAAGGAAAAGAAUAUG ACGUCAGG | 1275 |
| 53335_I2_45 | BCL11a | Intron 2 | + | chr2:60494889-60494914 | AGGAAAAGAAUAUGACG UCAGGGGG | 1276 |
| 53335_I2_46 | BCL11a | Intron 2 | + | chr2:60494901-60494926 | UGACGUCAGGGGGAGGC AAGUCAGU | 1277 |
| 53335_I2_47 | BCL11a | Intron 2 | + | chr2:60494902-60494927 | GACGUCAGGGGAGGCA AGUCAGUU | 1278 |
| 53335_I2_48 | BCL11a | Intron 2 | + | chr2:60494928-60494953 | GGAACACAGAUCCUAAC ACAGUAGC | 1279 |
| 53335_I2_49 | BCL11a | Intron 2 | + | chr2:60494939-60494964 | CCUAACACAGUAGCUGG UACCUGAU | 1280 |
| 53335_I2_50 | BCL11a | Intron 2 | + | chr2:60494955-60494980 | GUACCUGAUAGGUGCCU AUAUGUGA | 1281 |
| 53335_I2_51 | BCL11a | Intron 2 | + | chr2:60494959-60494984 | CUGAUAGGUGCCUAUAU GUGAUGGA | 1282 |
| 53335_I2_52 | BCL11a | Intron 2 | + | chr2:60494960-60494985 | UGAUAGGUGCCUAUAUG UGAUGGAU | 1283 |
| 53335_I2_53 | BCL11a | Intron 2 | + | chr2:60494963-60494988 | UAGGUGCCUAUAUGUGA UGGAUGGG | 1284 |
| 53335_I2_54 | BCL11a | Intron 2 | + | chr2:60494986-60495011 | GGUGGACAGCCCGACAG AUGAAAAA | 1285 |
| 53335_I2_55 | BCL11a | Intron 2 | + | chr2:60494998-60495023 | GACAGAUGAAAAAUGGA CAAUUAUG | 1286 |
| 53335_I2_56 | BCL11a | Intron 2 | + | chr2:60495001-60495026 | AGAUGAAAAAUGGACAA UUAUGAGG | 1287 |
| 53335_I2_57 | BCL11a | Intron 2 | + | chr2:60495002-60495027 | GAUGAAAAAUGGACAAU UAUGAGGA | 1288 |
| 53335_I2_58 | BCL11a | Intron 2 | + | chr2:60495003-60495028 | AUGAAAAAUGGACAAUU AUGAGGAG | 1289 |
| 53335_I2_59 | BCL11a | Intron 2 | + | chr2:60495017-60495042 | AUUAUGAGGAGGGAGA GUGCAGAC | 1290 |
| 53335_I2_60 | BCL11a | Intron 2 | + | chr2:60495018-60495043 | UUAUGAGGAGGGAGAG UGCAGACA | 1291 |
| 53335_I2_61 | BCL11a | Intron 2 | + | chr2:60495019-60495044 | UAUGAGGAGGGAGAGU GCAGACAG | 1292 |
| 53335_I2_62 | BCL11a | Intron 2 | + | chr2:60495045-60495070 | GGAAGCUUCACCUCCUU UACAAUUU | 1293 |
| 53335_I2_63 | BCL11a | Intron 2 | + | chr2:60495046-60495071 | GAAGCUUCACCUCCUUU ACAAUUUU | 1294 |
| 53335_I2_64 | BCL11a | Intron 2 | + | chr2:60495057-60495082 | UCCUUUACAAUUUUGGG AGUCCACA | 1295 |
| 53335_I2_65 | BCL11a | Intron 2 | + | chr2:60495062-60495087 | UACAAUUUUGGGAGUCC ACACGGCA | 1296 |
| 53335_I2_66 | BCL11a | Intron 2 | + | chr2:60495135-60495160 | CAUCACCAAGAGAGCCU UCCGAAAG | 1297 |
| 53335_I2_67 | BCL11a | Intron 2 | + | chr2:60495144-60495169 | GAGAGCCUUCCGAAAGA GGCCCCCC | 1298 |
| 53335_I2_68 | BCL11a | Intron 2 | + | chr2:60495145-60495170 | AGAGCCUUCCGAAAGAG GCCCCCCU | 1299 |
| 53335_I2_69 | BCL11a | Intron 2 | + | chr2:60495152-60495177 | UCCGAAAGAGGCCCCCC UGGGCAAA | 1300 |
| 53335_I2_70 | BCL11a | Intron 2 | + | chr2:60495162-60495187 | GCCCCCCUGGGCAAACG GCCACCGA | 1301 |
| 53335_I2_71 | BCL11a | Intron 2 | + | chr2:60495167-60495192 | CCUGGGCAAACGGCCAC CGAUGGAG | 1302 |
| 53335_I2_72 | BCL11a | Intron 2 | + | chr2:60495215-60495240 | CCACCCACGCCCCCACCC UAAUCAG | 1303 |
| 53335_I2_73 | BCL11a | Intron 2 | + | chr2:60495230-60495255 | CCCUAAUCAGAGGCCAA ACCCUUCC | 1304 |
| 53335_I2_74 | BCL11a | Intron 2 | + | chr2:60495293-60495318 | ACUAGCUUCAAAGUUGU AUUGACCC | 1305 |
| 53335_I2_75 | BCL11a | Intron 2 | + | chr2:60495333-60495358 | AAGAGUAGAUGCCAUAU CUCUUUC | 1306 |
| 53335_I2_76 | BCL11a | Intron 2 | + | chr2:60495354-60495379 | UUUCUGGCCUAUGUUAU UACCUGUA | 1307 |
| 53335_I2_77 | BCL11a | Intron 2 | + | chr2:60495366-60495391 | GUUAUUACCUGUAUGGA CUUUGCAC | 1308 |
| 53335_I2_78 | BCL11a | Intron 2 | + | chr2:60495400-60495425 | CUAUCUGCUCUUACUUA UGCACACC | 1309 |
| 53335_I2_79 | BCL11a | Intron 2 | + | chr2:60495401-60495426 | UAUCUGCUCUUACUUAU GCACACCU | 1310 |
| 53335_I2_80 | BCL11a | Intron 2 | + | chr2:60495402-60495427 | AUCUGCUCUUACUUAUG CACACCUG | 1311 |
| 53335_I2_81 | BCL11a | Intron 2 | + | chr2:60495486-60495511 | CCUGUCUAGCUGCCUUC CUUAUCAC | 1312 |
| 53335_I2_82 | BCL11a | Intron 2 | + | chr2:60495500-60495525 | UUCCUUAUCACAGGAAU AGCACCCA | 1313 |
| 53335_I2_83 | BCL11a | Intron 2 | + | chr2:60495543-60495568 | GAGUAGAACCCCCUAUA AACUAGUC | 1314 |
| 53335_I2_84 | BCL11a | Intron 2 | + | chr2:60495554-60495579 | CCUAUAAACUAGUCUGG UUUGCCCA | 1315 |
| 53335_I2_85 | BCL11a | Intron 2 | + | chr2:60495555-60495580 | CUAUAAACUAGUCUGGU UUGCCCAU | 1316 |
| 53335_I2_86 | BCL11a | Intron 2 | + | chr2:60495556-60495581 | UAUAAACUAGUCUGGUU UGCCCAUG | 1317 |
| 53335_I2_87 | BCL11a | Intron 2 | + | chr2:60495566-60495591 | UCUGGUUUGCCCAUGGG GCACAGUC | 1318 |
| 53335_I2_88 | BCL11a | Intron 2 | + | chr2:60495578-60495603 | AUGGGCACAGUCAGGC UGUUUCC | 1319 |
| 53335_I2_89 | BCL11a | Intron 2 | + | chr2:60495579-60495604 | UGGGGCACAGUCAGGCU GUUUUCCA | 1320 |
| 53335_I2_90 | BCL11a | Intron 2 | + | chr2:60495582-60495607 | GGCACAGUCAGGCUGUU UUCCAGGG | 1321 |
| 53335_I2_91 | BCL11a | Intron 2 | + | chr2:60495583-60495608 | GCACAGUCAGGCUGUUU UCCAGGGU | 1322 |
| 53335_I2_92 | BCL11a | Intron 2 | + | chr2:60495584-60495609 | CACAGUCAGGCUGUUUU CCAGGGUG | 1323 |
| 53335_I2_93 | BCL11a | Intron 2 | + | chr2:60495661-60495686 | ACACACGUAUGUGUUGU GAUCCCUG | 1324 |
| 53335_I2_94 | BCL11a | Intron 2 | + | chr2:60495674-60495699 | UUGUGAUCCCUGUGGUU UGAGAGUU | 1325 |
| 53335_I2_95 | BCL11a | Intron 2 | + | chr2:60495706-60495731 | UCCCUAAAAGUCAAAAU AUUCUCAA | 1326 |
| 53335_I2_96 | BCL11a | Intron 2 | + | chr2:60495707-60495732 | CCCUAAAAGUCAAAAUA UUCUCAAU | 1327 |
| 53335_I2_97 | BCL11a | Intron 2 | + | chr2:60495735-60495760 | CCCUCAAUCAGCACAUA CACACAAA | 1328 |
| 53335_I2_98 | BCL11a | Intron 2 | + | chr2:60495742-60495767 | UCAGCACAUACACACAA AAGGUACC | 1329 |
| 53335_I2_99 | BCL11a | Intron 2 | + | chr2:60495775-60495800 | UGUAAUUCUUUUCCUGC UCAAAGAC | 1330 |
| 53335_I2_100 | BCL11a | Intron 2 | + | chr2:60495 820-60495845 | CCCCAACCAAAAACCCUU GCCACCA | 1331 |
| 53335_I2_101 | BCL11a | Intron 2 | + | chr2:60495821-60495846 | CCCAACCAAAAACCCUU GCCACCAU | 1332 |
| 53335_I2_102 | BCL11a | Intron 2 | + | chr2:60495828-60495853 | AAAAACCCUUGCCACCA UGGGAGCC | 1333 |
| 53335_I2_103 | BCL11a | Intron 2 | + | chr2:60495829-60495854 | AAAACCCUUGCCACCAU GGGAGCCU | 1334 |
| 53335_I2_104 | BCL11a | Intron 2 | + | chr2:60495830-60495855 | AAACCCUUGCCACCAUG GGAGCCUG | 1335 |
| 53335_I2_105 | BCL11a | Intron 2 | + | chr2:60495839-60495864 | CCACCAUGGGAGCCUGG GGCAGAGA | 1336 |
| 53335_I2_106 | BCL11a | Intron 2 | + | chr2:60495867-60495892 | CACAGUGAAGUCAAACU GUAAUUCC | 1337 |
| 53335_I2_107 | BCL11a | Intron 2 | + | chr2:60495877-60495902 | UCAAACUGUAAUUCCAG GCUCUAAA | 1338 |
| 53335_I2_108 | BCL11a | Intron 2 | + | chr2:60495913-60495938 | UUUUUCUGAGAGUCUCU AAAUUACA | 1339 |
| 53335_I2_109 | BCL11a | Intron 2 | + | chr2:60495914-60495939 | UUUUCUGAGAGUCUCUA AAUUACAA | 1340 |
| 53335_I2_110 | BCL11a | Intron 2 | + | chr2:60495972-60495997 | ACACCUAAGAAACAUAC UGCAGCUC | 1341 |
| 53335_I2_111 | BCL11a | Intron 2 | + | chr2:60496001-60496026 | AAAGAGAACAAACAAAC CAAAGAGA | 1342 |
| 53335_I2_112 | BCL11a | Intron 2 | + | chr2:60496002-60496027 | AAGAGAACAAACAAACC AAAGAGAA | 1343 |
| 53335_I2_113 | BCL11a | Intron 2 | + | chr2:60496011-60496036 | AACAAACCAAAGAGAAG GGAUCCAG | 1344 |
| 53335_I2_114 | BCL11a | Intron 2 | + | chr2:60496048-60496073 | UAUGUGAAAAGUCAAUU GAUAAUGA | 1345 |
| 53335_I2_115 | BCL11a | Intron 2 | + | chr2:60496055-60496080 | AAAGUCAAUUGAUAAUG AAGGCUUU | 1346 |
| 53335_I2_116 | BCL11a | Intron 2 | + | chr2:60496063-60496088 | UUGAUAAUGAAGGCUUU AGGAUAAC | 1347 |
| 53335_I2_117 | BCL11a | Intron 2 | + | chr2:60496066-60496091 | AUAAUGAAGGCUUUAGG AUAACCGG | 1348 |
| 53335_I2_118 | BCL11a | Intron 2 | + | chr2:60496067-60496092 | UAAUGAAGGCUUUAGGA UAACCGGA | 1349 |
| 53335_I2_119 | BCL11a | Intron 2 | + | chr2:60496068-60496093 | AAUGAAGGCUUUAGGAU AACCGGAG | 1350 |
| 53335_I2_120 | BCL11a | Intron 2 | + | chr2:60496098-60496123 | AUGAUUGAAAGCAAUGC ACCUGUGC | 1351 |
| 53335_I2_121 | BCL11a | Intron 2 | + | chr2:60496104-60496129 | GAAAGCAAUGCACCUGU GCAGGAAA | 1352 |
| 53335_I2_122 | BCL11a | Intron 2 | + | chr2:60496111-60496136 | AUGCACCUGUGCAGGAA AUGGAUUA | 1353 |
| 53335_I2_123 | BCL11a | Intron 2 | + | chr2:60496118-60496143 | UGUGCAGGAAAUGGAUU ACGGAAAC | 1354 |
| 53335_I2_124 | BCL11a | Intron 2 | + | chr2:60496119-60496144 | GUGCAGGAAAUGGAUUA CGGAAACA | 1355 |
| 53335_I2_125 | BCL11a | Intron 2 | + | chr2:60496153-60496178 | UCAUGAAAUCCCAGAAA ACCAGAAC | 1356 |
| 53335_I2_126 | BCL11a | Intron 2 | + | chr2:60496154-60496179 | CAUGAAAUCCCAGAAAA CCAGAACC | 1357 |
| 53335_I2_127 | BCL11a | Intron 2 | + | chr2:60496164-60496189 | CAGAAAACCAGAACCGG GAAAGUUC | 1358 |
| 53335_I2_128 | BCL11a | Intron 2 | + | chr2:60496171-60496196 | CCAGAACCGGGAAAGUU CUGGAAGU | 1359 |
| 53335_I2_129 | BCL11a | Intron 2 | + | chr2:60496198-60496223 | GAAAAACAAAUCAUGAC UUAAGCAA | 1360 |
| 53335_I2_130 | BCL11a | Intron 2 | + | chr2:60496238-60496263 | CGUUUACAGAAUGCCUU GUCCCACG | 1361 |
| 53335_I2_131 | BCL11a | Intron 2 | - | chr2:60494308-60494333 | AGGCCAUGGUCACUGCC CAGUUUAG | 1362 |
| 53335_I2_132 | BCL11a | Intron 2 | - | chr2:60494327-60494352 | CCAGAGUGGGAGGUGA CCAGGCCA | 1363 |
| 53335_I2_133 | BCL11a | Intron 2 | - | chr2:60494333-60494358 | CCAGGUCCAGAGUGGGG AGGUGACC | 1364 |
| 53335_I2_134 | BCL11a | Intron 2 | - | chr2:60494341-60494366 | GGGGCAACCCAGGUCCA GAGUGGGG | 1365 |
| 53335_I2_135 | BCL11a | Intron 2 | - | chr2:60494344-60494369 | AGAGGGGCAACCCAGGU CCAGAGUG | 1366 |
| 53335_I2_136 | BCL11a | Intron 2 | - | chr2:60494345-60494370 | CAGAGGGGCAACCCAGG UCCAGAGU | 1367 |
| 53335_I2_137 | BCL11a | Intron 2 | - | chr2:60494346-60494371 | ACAGAGGGGCAACCCAG GUCCAGAG | 1368 |
| 53335_I2_138 | BCL11a | Intron 2 | - | chr2:60494356-60494381 | CUCCUUGUUUACAGAGG GGCAACCC | 1369 |
| 53335_I2_139 | BCL11a | Intron 2 | - | chr2:60494365-60494390 | UAUUACAACCUCCUUGU UUACAGAG | 1370 |
| 53335_I2_140 | BCL11a | Intron 2 | - | chr2:60494366-60494391 | UUAUUACAACCUCCUUG UUUACAGA | 1371 |
| 53335_I2_141 | BCL11a | Intron 2 | - | chr2:60494367-60494392 | UUUAUUACAACCUCCUU GUUUACAG | 1372 |
| 53335_I2_142 | BCL11a | Intron 2 | - | chr2:60494401-60494426 | UAAGAUUUAUAAGACAU UAGGGUAU | 1373 |
| 53335_I2_143 | BCL11a | Intron 2 | - | chr2:60494407-60494432 | AUUGCAUAAGAUUUAUA AGACAUUA | 1374 |
| 53335_I2_144 | BCL11a | Intron 2 | - | chr2:60494408-60494433 | AAUUGCAUAAGAUUUAU AAGACAUU | 1375 |
| 53335_I2_145 | BCL11a | Intron 2 | - | chr2:60494440-60494465 | CUCUUCUCUCCCCAUACU CCCAUCU | 1376 |
| 53335_I2_146 | BCL11a | Intron 2 | - | chr2:60494477-60494502 | UUGAUAUCUCAUCUCAC UGAGCUCU | 1377 |
| 53335_I2_147 | BCL11a | Intron 2 | - | chr2:60494478-60494503 | UUUGAUAUCUCAUCUCA CUGAGCUC | 1378 |
| 53335_I2_148 | BCL11a | Intron 2 | - | chr2:60494526-60494551 | CUUUGCCAAUUGGAGAU UAGGGAGA | 1379 |
| 53335_I2_149 | BCL11a | Intron 2 | - | chr2:60494532-60494557 | GUCUGGCUUUGCCAAUU GGAGAUUA | 1380 |
| 53335_I2_150 | BCL11a | Intron 2 | - | chr2:60494533-60494558 | AGUCUGGCUUUGCCAAU UGGAGAUU | 1381 |
| 53335_I2_151 | BCL11a | Intron 2 | - | chr2:60494541-60494566 | UUGCCCCAAGUCUGGCU UUGCCAAU | 1382 |
| 53335_I2_152 | BCL11a | Intron 2 | - | chr2:60494554-60494579 | GAACCAGUCUGUAUUGC CCCAAGUC | 1383 |
| 53335_I2_153 | BCL11a | Intron 2 | - | chr2:60494599-60494624 | GGUGAUAAAUCAUUAGG AAUGAGCU | 1384 |
| 53335_I2_154 | BCL11a | Intron 2 | - | chr2:60494610-60494635 | AAAGAACAUUUGGUGAU AAAUCAUU | 1385 |
| 53335_I2_155 | BCL11a | Intron 2 | - | chr2:60494625-60494650 | AAAUUCCAGCUGAAGAA AGAACAUU | 1386 |
| 53335_I2_156 | BCL11a | Intron 2 | - | chr2:60494668-60494693 | AUAUACUAUUAUUAUUC CUAUUUUA | 1387 |
| 53335_I2_157 | BCL11a | Intron 2 | - | chr2:60494745-60494770 | AAGCUCGGAGCACUUAC UCUGCUCU | 1388 |
| 53335_I2_158 | BCL11a | Intron 2 | - | chr2:60494765-60494790 | GAGGCAGCAUUUUAGUU CACAAGCU | 1389 |
| 53335_I2_159 | BCL11a | Intron 2 | - | chr2:60494789-60494814 | UGAGGUGUAACUAAUAA AUACCAGG | 1390 |
| 53335_I2_160 | BCL11a | Intron 2 | - | chr2:60494792-60494817 | UGCUGAGGUGUAACUAA UAAAUACC | 1391 |
| 53335_I2_161 | BCL11a | Intron 2 | - | chr2:60494812-60494837 | GGGCCUGAUAACUUUGU UUCUGCUG | 1392 |
| 53335_I2_162 | BCL11a | Intron 2 | - | chr2:60494837-60494862 | UGACCCAAACUAGGAAU UGGGGAAA | 1393 |
| 53335_I2_163 | BCL11a | Intron 2 | - | chr2:60494838-60494863 | CUGACCCAAACUAGGAA UUGGGGAA | 1394 |
| 53335_I2_164 | BCL11a | Intron 2 | - | chr2:60494843-60494868 | UUCUUCUGACCCAAACU AGGAAUUG | 1395 |
| 53335_I2_165 | BCL11a | Intron 2 | - | chr2:60494844-60494869 | UUUCUUCUGACCCAAAC UAGGAAUU | 1396 |
| 53335_I2_166 | BCL11a | Intron 2 | - | chr2:60494845-60494870 | UUUUCUUCUGACCCAAA CUAGGAAU | 1397 |
| 53335_I2_167 | BCL11a | Intron 2 | - | chr2:60494851-60494876 | UUUCCCUUUUCUUCUGA CCCAAACU | 1398 |
| 53335_I2_168 | BCL11a | Intron 2 | - | chr2:60494942-60494967 | CCUAUCAGGUACCAGCU ACUGUGUU | 1399 |
| 53335_I2_169 | BCL11a | Intron 2 | - | chr2:60494961-60494986 | CAUCCAUCACAUAUAGG CACCUAUC | 1400 |
| 53335_I2_170 | BCL11a | Intron 2 | - | chr2:60494972-60494997 | GGCUGUCCACCCAUCCA UCACAUAU | 1401 |
| 53335_I2_171 | BCL11a | Intron 2 | - | chr2:60494998-60495023 | CAUAAUUGUCCAUUUUU CAUCUGUC | 1402 |
| 53335_I2_172 | BCL11a | Intron 2 | - | chr2:60494999-60495024 | UCAUAAUUGUCCAUUUU UCAUCUGU | 1403 |
| 53335_I2_173 | BCL11a | Intron 2 | - | chr2:60495058-60495083 | GUGUGGACUCCCAAAAU UGUAAAGG | 1404 |
| 53335_I2_174 | BCL11a | Intron 2 | - | chr2:60495061-60495086 | GCCGUGUGGACUCCCAA AAUUGUAA | 1405 |
| 53335_I2_175 | BCL11a | Intron 2 | - | chr2:60495080-60495105 | GAAAUAAUUUGUAUGCC AUGCCGUG | 1406 |
| 53335_I2_176 | BCL11a | Intron 2 | - | chr2:60495111-60495136 | GGAGAAUUGGAUUUUAU UUCUCAAU | 1407 |
| 53335_I2_177 | BCL11a | Intron 2 | - | chr2:60495112-60495137 | UGGAGAAUUGGAUUUUA UUUCUCAA | 1408 |
| 53335_I2_178 | BCL11a | Intron 2 | - | chr2:60495129-60495154 | GAAGGCUCUCUUGGUGA UGGAGAAU | 1409 |
| 53335_I2_179 | BCL11a | Intron 2 | - | chr2:60495137-60495162 | CUCUUUCGGAAGGCUCU CUUGGUGA | 1410 |
| 53335_I2_180 | BCL11a | Intron 2 | - | chr2:60495143-60495168 | GGGGGCCUCUUUCGGAA GGCUCUCU | 1411 |
| 53335_I2_181 | BCL11a | Intron 2 | - | chr2:60495152-60495177 | UUUGCCCAGGGGGCCU CUUUCGGA | 1412 |
| 53335_I2_182 | BCL11a | Intron 2 | - | chr2:60495156-60495181 | GCCGUUUGCCCAGGGGG GCCUCUUU | 1413 |
| 53335_I2_183 | BCL11a | Intron 2 | - | chr2:60495166-60495191 | UCCAUCGGUGGCCGUUU GCCCAGGG | 1414 |
| 53335_I2_184 | BCL11a | Intron 2 | - | chr2:60495167-60495192 | CUCCAUCGGUGGCCGUU UGCCCAGG | 1415 |
| 53335_I2_185 | BCL11a | Intron 2 | - | chr2:60495168-60495193 | UCUCCAUCGGUGGCCGU UUGCCCAG | 1416 |
| 53335_I2_186 | BCL11a | Intron 2 | - | chr2:60495169-60495194 | CUCUCCAUCGGUGGCCG UUUGCCCA | 1417 |
| 53335_I2_187 | BCL11a | Intron 2 | - | chr2:60495170-60495195 | CCUCUCCAUCGGUGGCC GUUUGCCC | 1418 |
| 53335_I2_188 | BCL11a | Intron 2 | - | chr2:60495183-60495208 | GAGGACUGGCAGACCUC UCCAUCGG | 1419 |
| 53335_I2_189 | BCL11a | Intron 2 | - | chr2:60495186-60495211 | GAAGAGGACUGGCAGAC CUCUCCAU | 1420 |
| 53335_I2_190 | BCL11a | Intron 2 | - | chr2:60495202-60495227 | GGGCGUGGGUGGGGUAG AAGAGGAC | 1421 |
| 53335_I2_191 | BCL11a | Intron 2 | - | chr2:60495207-60495232 | GGUGGGGCGUGGUGG GGUAGAAG | 1422 |
| 53335_I2_192 | BCL11a | Intron 2 | - | chr2:60495216-60495241 | UCUGAUUAGGGUGGGGG CGUGGGUG | 1423 |
| 53335_I2_193 | BCL11a | Intron 2 | - | chr2:60495217-60495242 | CUCUGAUUAGGGUGGGG GCGUGGGU | 1424 |
| 53335_I2_194 | BCL11a | Intron 2 | - | chr2:60495218-60495243 | CCUCUGAUUAGGGUGGG GGCGUGGG | 1425 |
| 53335_I2_195 | BCL11a | Intron 2 | - | chr2:60495221-60495246 | UGGCCUCUGAUUAGGGU GGGGGCGU | 1426 |
| 53335_I2_196 | BCL11a | Intron 2 | - | chr2:60495222-60495247 | UUGGCCUCUGAUUAGGG UGGGGGCG | 1427 |
| 53335_I2_197 | BCL11a | Intron 2 | - | chr2:60495227-60495252 | AGGGUUUGGCCUCUGAU UAGGGUGG | 1428 |
| 53335_I2_198 | BCL11a | Intron 2 | - | chr2:60495228-60495253 | AAGGGUUUGGCCUCUGA UUAGGGUG | 1429 |
| 53335_I2_199 | BCL11a | Intron 2 | - | chr2:60495229-60495254 | GAAGGGUUUGGCCUCUG AUUAGGGU | 1430 |
| 53335_I2_200 | BCL11a | Intron 2 | - | chr2:60495230-60495255 | GGAAGGGUUUGGCCUCU GAUUAGGG | 1431 |
| 53335_I2_201 | BCL11a | Intron 2 | - | chr2:60495233-60495258 | CCAGGAAGGGUUUGGCC UCUGAUUA | 1432 |
| 53335_I2_202 | BCL11a | Intron 2 | - | chr2:60495234-60495259 | UCCAGGAAGGGUUUGGC CUCUGAUU | 1433 |
| 53335_I2_203 | BCL11a | Intron 2 | - | chr2:60495246-60495271 | UUUAUCACAGGCUCCAG GAAGGGUU | 1434 |
| 53335_I2_204 | BCL11a | Intron 2 | - | chr2:60495251-60495276 | UUGCUUUUAUCACAGGC UCCAGGAA | 1435 |
| 53335_I2_205 | BCL11a | Intron 2 | - | chr2:60495252-60495277 | GUUGCUUUUAUCACAGG CUCCAGGA | 1436 |
| 53335_I2_206 | BCL11a | Intron 2 | - | chr2:60495256-60495281 | AACAGUUGCUUUUAUCA CAGGCUCC | 1437 |
| 53335_I2_207 | BCL11a | Intron 2 | - | chr2:60495263-60495288 | GCAAGCUAACAGUUGCU UUUAUCAC | 1438 |
| 53335_I2_208 | BCL11a | Intron 2 | - | chr2:60495318-60495343 | AUCUACUCUUAGACAUA ACACACCA | 1439 |
| 53335_I2_209 | BCL11a | Intron 2 | - | chr2:604953 19-60495344 | CAUCUACUCUUAGACAU AACACACC | 1440 |
| 53335_I2_210 | BCL11a | Intron 2 | - | chr2:60495347-60495372 | AAUAACAUAGGCCAGAA AAGAGAUA | 1441 |
| 53335_I2_211 | BCL11a | Intron 2 | - | chr2:60495364-60495389 | GCAAAGUCCAUACAGGU AAUAACAU | 1442 |
| 53335_I2_212 | BCL11a | Intron 2 | - | chr2:60495376-60495401 | GCUGAUUCCAGUGCAAA GUCCAUAC | 1443 |
| 53335_I2_213 | BCL11a | Intron 2 | - | chr2:60495426-60495451 | CGAUACAGGGCUGGCUC UAUGCCCC | 1444 |
| 53335_I2_214 | BCL11a | Intron 2 | - | chr2:60495440-60495465 | AGAUGGCUGAAAAGCGA UACAGGGC | 1445 |
| 53335_I2_215 | BCL11a | Intron 2 | - | chr2:60495444-60495469 | AGUGAGAUGGCUGAAAA GCGAUACA | 1446 |
| 53335_I2_216 | BCL11a | Intron 2 | - | chr2:60495445-60495470 | UAGUGAGAUGGCUGAAA AGCGAUAC | 1447 |
| 53335_I2_217 | BCL11a | Intron 2 | - | chr2:60495462-60495487 | GACUUGGGAGUUAUCUG UAGUGAGA | 1448 |
| 53335_I2_218 | BCL11a | Intron 2 | - | chr2:60495482-60495507 | UAAGGAAGGCAGCUAGA CAGGACUU | 1449 |
| 53335_I2_219 | BCL11a | Intron 2 | - | chr2:60495483-60495508 | AUAAGGAAGGCAGCUAG ACAGGACU | 1450 |
| 53335_I2_220 | BCL11a | Intron 2 | - | chr2:60495489-60495514 | CCUGUGAUAAGGAAGGC AGCUAGAC | 1451 |
| 53335_I2_221 | BCL11a | Intron 2 | - | chr2:60495501-60495526 | UUGGGUGCUAUUCCUGU GAUAAGGA | 1452 |
| 53335_I2_222 | BCL11a | Intron 2 | - | chr2:60495505-60495530 | GACCUUGGGUGCUAUUC CUGUGAUA | 1453 |
| 53335_I2_223 | BCL11a | Intron 2 | - | chr2:60495524-60495549 | CUACUCUGAGGUACUGA UGGACCUU | 1454 |
| 53335_I2_224 | BCL11a | Intron 2 | - | chr2:60495525-60495550 | UCUACUCUGAGGUACUG AUGGACCU | 1455 |
| 53335_I2_225 | BCL11a | Intron 2 | - | chr2:60495532-60495557 | AGGGGGUUCUACUCUGA GGUACUGA | 1456 |
| 53335_I2_226 | BCL11a | Intron 2 | - | chr2:60495541-60495566 | CUAGUUUAUAGGGGUU CUACUCUG | 1457 |
| 53335_I2_227 | BCL11a | Intron 2 | - | chr2:60495554-60495579 | UGGGCAAACCAGACUAG UUUAUAGG | 1458 |
| 53335_I2_228 | BCL11a | Intron 2 | - | chr2:60495555-60495580 | AUGGGCAAACCAGACUA GUUUAUAG | 1459 |
| 53335_I2_229 | BCL11a | Intron 2 | - | chr2:60495556-60495581 | CAUGGGCAAACCAGACU AGUUUAUA | 1460 |
| 53335_I2_230 | BCL11a | Intron 2 | - | chr2:60495557-60495582 | CCAUGGGCAAACCAGAC UAGUUUAU | 1461 |
| 53335_I2_231 | BCL11a | Intron 2 | - | chr2:60495578-60495603 | GGAAAACAGCCUGACUG UGCCCCAU | 1462 |
| 53335_I2_232 | BCL11a | Intron 2 | - | chr2:60495579-60495604 | UGGAAAACAGCCUGACU GUGCCCCA | 1463 |
| 53335_I2_233 | BCL11a | Intron 2 | - | chr2:60495604-60495629 | GGCAGAGAAUGUCUGCA CCCCACCC | 1464 |
| 53335_I2_234 | BCL11a | Intron 2 | - | chr2:60495630-60495655 | UGACGUUAUAUGUAAGC AUCACAAC | 1465 |
| 53335_I2_235 | BCL11a | Intron 2 | - | chr2:60495684-60495709 | GGAAGCUCCAAACUCUC AAACCACA | 1466 |
| 53335_I2_236 | BCL11a | Intron 2 | - | chr2:60495685-60495710 | GGGAAGCUCCAAACUCU CAAACCAC | 1467 |
| 53335_I2_237 | BCL11a | Intron 2 | - | chr2:60495710-60495735 | CCCAUUGAGAAUAUUUU GACUUUUA | 1468 |
| 53335_I2_238 | BCL11a | Intron 2 | - | chr2:60495711-60495736 | GCCCAUUGAGAAUAUUU UGACUUUU | 1469 |
| 53335_I2_239 | BCL11a | Intron 2 | - | chr2:60495738-60495763 | CCUUUUGUGUGUAUGUG CUGAUUGA | 1470 |
| 53335_I2_240 | BCL11a | Intron 2 | - | chr2:60495739-60495764 | ACCUUUUGUGUGUAUGU GCUGAUUG | 1471 |
| 53335_I2_241 | BCL11a | Intron 2 | - | chr2:60495768-60495793 | AGCAGGAAAAGAAUUAC AGUUUCC | 1472 |
| 53335_I2_242 | BCL11a | Intron 2 | - | chr2:60495790-60495815 | GGUAUUGAAUUGCCUGU CUUUGAGC | 1473 |
| 53335_I2_243 | BCL11a | Intron 2 | - | chr2:60495816-60495841 | GGCAAGGGUUUUUGGUU GGGGGAAG | 1474 |
| 53335_I2_244 | BCL11a | Intron 2 | - | chr2:60495817-60495842 | UGGCAAGGGUUUUUGGU UGGGGGAA | 1475 |
| 53335_I2_245 | BCL11a | Intron 2 | - | chr2:60495818-60495843 | GUGGCAAGGUUUUUGG UUGGGGA | 1476 |
| 53335_I2_246 | BCL11a | Intron 2 | - | chr2:60495822-60495847 | CAUGGUGGCAAGGGUUU UUGGUUGG | 1477 |
| 53335_I2_247 | BCL11a | Intron 2 | - | chr2:60495823-60495848 | CCAUGGUGGCAAGGGUU UUUGGUUG | 1478 |
| 53335_I2_248 | BCL11a | Intron 2 | - | chr2:60495824-60495849 | CCCAUGGUGGCAAGGGU UUUUGGUU | 1479 |
| 53335_I2_249 | BCL11a | Intron 2 | - | chr2:60495825-60495850 | UCCCAUGGUGGCAAGGG UUUUUGGU | 1480 |
| 53335_I2_250 | BCL11a | Intron 2 | - | chr2:60495829-60495854 | AGGCUCCCAUGGUGGCA AGGGUUUU | 1481 |
| 53335_I2_251 | BCL11a | Intron 2 | - | chr2:60495836-60495861 | CUGCCCCAGGCUCCCAUG GUGGCAA | 1482 |
| 53335_I2_252 | BCL11a | Intron 2 | - | chr2:60495837-60495862 | UCUGCCCCAGGCUCCCAU GGUGGCA | 1483 |
| 53335_I2_253 | BCL11a | Intron 2 | - | chr2:60495842-60495867 | CCUUCUCUGCCCCAGGCU CCCAUGG | 1484 |
| 53335_I2_254 | BCL11a | Intron 2 | - | chr2:60495845-60495870 | GUGCCUUCUCUGCCCCA GGCUCCCA | 1485 |
| 53335_I2_255 | BCL11a | Intron 2 | - | chr2:60495854-60495879 | GACUUCACUGUGCCUUC UCUGCCCC | 1486 |
| 53335_I2_256 | BCL11a | Intron 2 | - | chr2:60495893-60495918 | AAAAAUGACAGCACCAU UUAGAGCC | 1487 |
| 53335_I2_257 | BCL11a | Intron 2 | - | chr2:60495978-60496003 | UUUCCAGAGCUGCAGUA UGUUUCUU | 1488 |
| 53335_I2_258 | BCL11a | Intron 2 | - | chr2:60496020-60496045 | GGGUGACCUCUGGAUCC CUUCUCUU | 1489 |
| 53335_I2_259 | BCL11a | Intron 2 | - | chr2:60496035-60496060 | ACUUUUCACAUAUGAGG GUGACCUC | 1490 |
| 53335_I2_260 | BCL11a | Intron 2 | - | chr2:60496045-60496070 | UUAUCAAUUGACUUUUC ACAUAUGA | 1491 |
| 53335_I2_261 | BCL11a | Intron 2 | - | chr2:60496046-60496071 | AUUAUCAAUUGACUUUU CACAUAUG | 1492 |
| 53335_I2_262 | BCL11a | Intron 2 | - | chr2:60496090-60496115 | GCAUUGCUUUCAAUCAU CUCCCCUC | 1493 |
| 53335_I2_263 | BCL11a | Intron 2 | - | chr2:60496119-60496144 | UGUUUCCGUAAUCCAUU UCCUGCAC | 1494 |
| 53335_I2_264 | BCL11a | Intron 2 | - | chr2:60496165-60496190 | AGAACUUUCCCGGUUCU GGUUUCU | 1495 |
| 53335_I2_265 | BCL11a | Intron 2 | - | chr2:60496166-60496191 | CAGAACUUUCCCGGUUC UGGUUUUC | 1496 |
| 53335_I2_266 | BCL11a | Intron 2 | - | chr2:60496174-60496199 | CCGACUUCCAGAACUUU CCCGGUUC | 1497 |
| 53335_I2_267 | BCL11a | Intron 2 | - | chr2:60496180-60496205 | GUUUUUCCGACUUCCAG AACUUUCC | 1498 |
| 53335_I2_268 | BCL11a | Intron 2 | - | chr2:60496233-60496258 | GACAAGGCAUUCUGUAA ACGUGUAU | 1499 |

In embodiments, the gRNA targeting domain consists of the 20 3' nt of any one of the sequences in the table above.

Exemplary preferred gRNA tareting domains useful in the compositions and methods of the invention are described in the tables below.

**Table 5. Preferred Guide RNA Targeting Domains Directed to Coding Regions of the BCL11a Gene**

| **Id.** | **Target** | **gRNA Targeting Domain** | **Targeting Site (Chr2)** | **SEQ ID NO:** |
|---|---|---|---|---|
| CR000044 | BCL11a | ACAGGCCGUGAACCUAAGUG | 60678414-60678436 | 1 |
| CR000056 | BCL11a | UAGAGGGGGGAAAUUAUAGG | 60678685-60678707 | 2 |
| CR000068 | BCL11a | CGAGCGGUAAAUCCUAAAGA | 60678840-60678862 | 3 |
| CR000079 | BCL11a | AGACAAAUUCAAAUCCUGCA | 60678895-60678917 | 4 |
| CR000091 | BCL11a | GCUUUUGGUGGCUACCAUGC | 60678909-60678931 | 5 |
| CR000102 | BCL11a | AAUUUAUGCCAUCUGAUAAG | 60679112-60679134 | 6 |
| CR000033 | BCL11a | UGUUCCUCCUACCCACCCGA | 60679178-60679200 | 7 |
| CR000045 | BCL11a | AUAAAUGUUGGAGCUUUAGG | 60679288-60679310 | 8 |
| CR000069 | BCL11a | CAACCUAAUUACCUGUAUUG | 60679389-60679411 | 9 |
| CR000080 | BCL11a | CGCUGUCAUGAGUGAUGCCC | 60679428-60679450 | 10 |
| CR000092 | BCL11a | GGCAUCACUCAUGACAGCGA | 60679432-60679454 | 11 |
| CR000103 | BCL11a | AGUCCCAUAGAGAGGGCCCG | 60679456-60679478 | 12 |
| CR000115 | BCL11a | ACUGAUUCACUGUUCCAAUG | 60679476-60679498 | 13 |
| CR000034 | BCL11a | AGGACUCUGUCUUCGCACAA | 60679577-60679599 | 14 |
| CR000058 | BCL11a | CCACGCUGACGUCGACUGGG | 60679650-60679672 | 15 |
| CR000070 | BCL11a | GUUCUUCACACACCCCCAUU | 60679779-60679801 | 16 |
| CR000081 | BCL11a | GCGCUUCUCCACACCGCCCG | 60687934-60687956 | 17 |
| CR000093 | BCL11a | GUCUGGAGUCUCCGAAGCUA | 60688006-60688028 | 18 |
| CR000116 | BCL11a | AAAGAUCCCUUCCUUAGCUU | 60688017-60688039 | 19 |
| CR000035 | BCL11a | UCGCCGGCUACGCGGCCUCC | 60688046-60688068 | 20 |
| CR000047 | BCL11a | CGGAGAACGUGUACUCGCAG | 60688070-60688092 | 21 |
| CR000071 | BCL11a | GAGCUUGAUGCGCUUAGAGA | 60688133-60688155 | 22 |
| CR000082 | BCL11a | CCCCCCGAGGCCGACUCGCC | 60688200-60688222 | 23 |
| CR000094 | BCL11a | CACCAUGCCCUGCAUGACGU | 60688394-60688416 | 24 |
| CR000105 | BCL11a | GAGAGCGAGAGGGUGGACUA | 60688506-60688528 | 25 |
| CR000117 | BCL11a | CACGGACUUGAGCGCGCUGC | 60688649-60688671 | 26 |
| CR000036 | BCL11a | GCCCACCAAGUCGCUGGUGC | 60688676-60688698 | 27 |
| CR000048 | BCL11a | CCCACCAAGUCGCUGGUGCC | 60688677-60688699 | 28 |
| CR000060 | BCL11a | GGCGGUGGAGAGACCGUCGU | 60688712-60688734 | 29 |
| CR000072 | BCL11a | GCCGCAGAACUCGCAUGACU | 60688898-60688920 | 30 |
| CR000083 | BCL11a | CCGCCCCCAGGCGCUCUAUG | 60689194-60689216 | 31 |
| CR000095 | BCL11a | UGGGGGUCCAAGUGAUGUCU | 60689217-60689239 | 32 |
| CR000106 | BCL11a | CUCAACUUACAAAUACCCUG | 60695857-60695879 | 33 |
| CR000118 | BCL11a | AGUGCAGAAUAUGCCCCGCA | 60695872-60695894 | 34 |
| CR000037 | BCL11a | GCAUAUUCUGCACUCAUCCC | 60695881-60695903 | 35 |
| CR000049 | BCL11a | GAGCUCUAAUCCCCACGCCU | 60695898-60695920 | 36 |
| CR000061 | BCL11a | AGAGCUCCAUGUGCAGAACG | 60695914-60695936 | 37 |
| CR000084 | BCL11a | GAUAAACUUCUGCACUGGAG | 60695947-60695969 | 38 |
| CR000096 | BCL11a | UAGCUGUAGUGCUUGAUUUU | 60695981-60696003 | 39 |
| CR000107 | BCL11a | UAUCCAAAUUCAUACAAUAG | 60716451-60716473 | 40 |
| CR000119 | BCL11a | AUGCCCUGAGUAUCCCAACA | 60717068-60717090 | 41 |
| CR000038 | BCL11a | UAGUAACAGACCCAUGUGCU | 60717232-60717254 | 42 |
| CR000050 | BCL11a | AUACUUCAAGGCCUCAAUGA | 60717661-60717683 | 43 |
| CR000062 | BCL11a | GACUAGGUAGACCUUCAUUG | 60717672-60717694 | 44 |
| CR000073 | BCL11a | CUGAGCUAGUGGGGGCUCUU | 60720773-60720795 | 45 |
| CR000085 | BCL11a | CUGAGCACAUUCUUACGCCU | 60721291-60721313 | 46 |
| CR000097 | BCL11a | UUUAUAAGACAUUAGGGUAt | 60721534-60721556 | 47 |
| CR000108 | BCL11a | UGAUAGGUGCCUAUAUGUGA | 60722096-60722118 | 48 |
| CR000120 | BCL11a | UCCACCCAUCCAUCACAUAU | 60722105-60722127 | 49 |
| CR000039 | BCL11a | CUUCAAAGUUGUAUUGACCC | 60722434-60722456 | 50 |
| CR000051 | BCL11a | AAACCAGACUAGUUUAUAGG | 60722687-60722709 | 51 |
| CR000063 | BCL11a | UUUGAGACAGUUACCCCUUC | 60723706-60723728 | 52 |
| CR000074 | BCL11a | AACCUGAGGGCUAGUUUCUA | 60724541-60724563 | 53 |
| CR000086 | BCL11a | GCCUGGACCCACCGCUUCAU | 60725058-60725080 | 54 |
| CR000109 | BCL11a | AAACCAGUGAGGUCAUCUAU | 60725857-60725879 | 55 |
| CR000121 | BCL11a | ACCUGCUAUGUGUUCCUGUU | 60773104-60773126 | 56 |
| CR000040 | BCL11a | UAGAGGAAUUUGCCCCAAAC | 60773118-60773140 | 57 |
| CR000052 | BCL11a | GAUAAACAAUCGUCAUCCUC | 60773150-60773172 | 58 |
| CR000064 | BCL11a | UGGCAUCCAGGUCACGCCAG | 60773166-60773188 | 59 |
| CR000075 | BCL11a | GACCUGGAUGCCAACCUCCA | 60773176-60773198 | 60 |
| CR000087 | BCL11a | AAAAGCAUCCAAUCCCGUGG | 60773190-60773212 | 61 |
| CR000110 | BCL11a | GAUGCUUUUUUCAUCUCGAU | 60773204-60773226 | 62 |
| CR000122 | BCL11a | CCACAGCUUUUUCUAAGCAG | 60773247-60773269 | 63 |
| CR000041 | BCL11a | CCCCCAAUGGGAAGUUCAUC | 60773316-60773338 | 64 |
| CR000053 | BCL11a | AUCAUGACCUCCUCACCUGU | 60773344-60773366 | 65 |
| CR000065 | BCL11a | AGGAGGUCAUGAUCCCCUUC | 60773354-60773376 | 66 |
| CR000088 | BCL11a | CCCCUUCUGGAGCUCCCAAC | 60773367-60773389 | 67 |
| CR000099 | BCL11a | GCUCCCAACGGGCCGUGGUC | 60773378-60773400 | 68 |
| CR000111 | BCL11a | ACAGAUGAUGAACCAGACCA | 60773390-60773412 | 69 |
| CR000123 | BCL11a | UCUGUAAGAAUGGCUUCAAG | 60773408-60773430 | 70 |
| CR000042 | BCL11a | CAAUUAUUAGAGUGCCAGAG | 60773459-60773481 | 71 |
| CR000054 | BCL11a | GCCUUGCUUGCGGCGAGACA | 60780385-60780407 | 72 |
| CR000066 | BCL11a | ACCAUGUCUCGCCGCAAGCA | 60780386-60780408 | 73 |
| CR000077 | BCL11a | GAGUCUCCUUCUUUCUAACC | 60780482-60780504 | 74 |
| CR000089 | BCL11a | GAAUUGUGGGAGAGCCGUCA | 60780582-60780604 | 75 |
| CR000100 | BCL11a | AAAAUAGAGCGAGAGUGCAC | 60781166-60781188 | 76 |
| CR000112 | BCL11a | GCCCCUGGCGUCCACACGCG | 60781306-60781328 | 77 |
| CR000124 | BCL11a | CUCCUCGUUCUUUAUUCCUC | 60781716-60781738 | 78 |
| CR000043 | BCL11a | GACUGCCCGCGCUUUGUCCU | 60781815-60781837 | 79 |
| CR000055 | BCL11a | UUCCCAGGGACUGGGACUCC | 60781838-60781860 | 80 |
| CR000078 | BCL11a | UGGCUCCUGGGUGUGCGCCU | 60782123-60782145 | 81 |
| CR000090 | BCL11a | UGAAGCCCGCUUUUUGACAG | 60782254-60782276 | 82 |
| CR000101 | BCL11a | AGGGUGGAGACGGGUCGCCA | 60782526-60782548 | 83 |
| CR000113 | BCL11a | GCUCAGGGUCUCGCGGGCCA | 60782543-60782565 | 84 |
| CR000059 | BCL11a | UGAGUCCGAGCAGAAGAAGA | 73160981-73161003 | 85 |

**Table 6: Preferred Guide RNA Targeting Domains directed to the French HPFH (French HPFH; Sankaran VG et al. A functional element necessary for fetal hemoglobin silencing. NEJM (2011) 365:807-814.)**

| **Id.** | **Target Name** | **Strand** | **gRNA Targeting Domain** | **Genomic Target Location** | **SEQ ID NO:** |
|---|---|---|---|---|---|
| CR001016 | HPFH | - | UCUUAAACCAACCUGCUCAC | chr11:5234538-5234558 | 86 |
| CR001017 | HPFH | + | CAGGUUGGUUUAAGAUAAGC | chr11:5234543-5234563 | 87 |
| CR001018 | HPFH | + | AGGUUGGUUUAAGAUAAGCA | chr11:5234544-5234564 | 88 |
| CR001019 | HPFH | - | UUAAGGGAAUAGUGGAAUGA | chr11:5234600-5234620 | 89 |
| CR001020 | HPFH | - | AGGGCAAGUUAAGGGAAUAG | chr11:5234608-5234628 | 90 |
| CR001021 | HPFH | + | CCCUUAACUUGCCCUGAGAU | chr11:5234613-5234633 | 91 |
| CR001022 | HPFH | - | CCAAUCUCAGGGCAAGUUAA | chr11:5234616-5234636 | 92 |
| CR001023 | HPFH | - | GCCAAUCUCAGGGCAAGUUA | chr11:5234617-5234637 | 93 |
| CR001024 | HPFH | - | UGACAGAACAGCCAAUCUCA | chr11:5234627-5234647 | 94 |
| CR001025 | HPFH | - | AUGACAGAACAGCCAAUCUC | chr11:5234628-5234648 | 95 |
| CR001026 | HPFH | - | GAGAUAUGUAGAGGAGAACA | chr11:5234670-5234690 | 96 |
| CR001027 | HPFH | - | GGAGAUAUGUAGAGGAGAAC | chr11:5234671-5234691 | 97 |
| CR001028 | HPFH | - | UGCGGUGGGGAGAUAUGUAG | chr11:5234679-5234699 | 98 |
| CR001029 | HPFH | - | CUGCUGAAAGAGAUGCGGUG | chr11:5234692-5234712 | 99 |
| CR001030 | HPFH | - | ACUGCUGAAAGAGAUGCGGU | chr11:5234693-5234713 | 100 |
| CR001031 | HPFH | - | AACUGCUGAAAGAGAUGCGG | chr11:5234694-5234714 | 101 |
| CR001032 | HPFH | - | AACAACUGCUGAAAGAGAUG | chr11:5234697-5234717 | 102 |
| CR001033 | HPFH | - | UCUGCAAAAAUGAAACUAGG | chr11:5234731-5234751 | 103 |
| CR001034 | HPFH | - | ACUUCUGCAAAAAUGAAACU | chr11:5234734-5234754 | 104 |
| CR001035 | HPFH | + | CAUUUUUGCAGAAGUGUUUU | chr11:5234739-5234759 | 105 |
| CR001036 | HPFH | + | AGUGUUUUAGGCUAAUAUAG | chr11:5234751-5234771 | 106 |
| CR001037 | HPFH | - | UUGGAGACAAAAAUCUCUAG | chr11:5234883-5234903 | 107 |
| CR001038 | HPFH | + | UCUAGAGAUUUUUGUCUCCA | chr11:5234882-5234902 | 108 |
| CR001039 | HPFH | + | CUAGAGAUUUUUGUCUCCAA | chr11:5234883-5234903 | 109 |
| CR001040 | HPFH | + | GUCUCCAAGGGAAUUUUGAG | chr11:5234895-5234915 | 110 |
| CR001041 | HPFH | + | CCAAGGGAAUUUUGAGAGGU | chr11:5234899-5234919 | 111 |
| CR001042 | HPFH | - | CCAACCUCUCAAAAUUCCCU | chr11:5234902-5234922 | 112 |
| CR001043 | HPFH | + | GGAAUUUUGAGAGGUUGGAA | chr11:5234904-5234924 | 113 |
| CR001044 | HPFH | + | UGCUUGCUUCCUCCUUCUUU | chr11:5234953-5234973 | 114 |
| CR001045 | HPFH | - | AAGAAUUUACCAAAAGAAGG | chr11:5234965-5234985 | 115 |
| CR001046 | HPFH | - | AGGAAGAAUUUACCAAAAGA | chr11:5234968-5234988 | 116 |
| CR001047 | HPFH | - | AAAAAUUAGAGUUUUAUUAU | chr11:5234988-5235008 | 117 |
| CR001048 | HPFH | - | UUUUUUAAAUAUUCUUUUAA | Chr11:5235023-5235045 | 118 |
| CR001049 | HPFH | + | UAUUUACCAGUUAUUGAAAU | chr11:5235062-5235082 | 119 |
| CR001050 | HPFH | + | CCAGUUAUUGAAAUAGGUUC | chr11:5235068-5235088 | 120 |
| CR001051 | HPFH | - | CCAGAACCUAUUUCAAUAAC | chr11:5235071-5235091 | 121 |
| CR001052 | HPFH | + | UUCUGGAAACAUGAAUUUUA | chr11:5235085-5235105 | 122 |
| CR001053 | HPFH | + | AUUUUGAAUGUUUAAAAUUA | chr11:5235151-5235171 | 123 |
| CR001054 | HPFH | - | AAAUUUAAUCUGGCUGAAUA | chr11:5235216-5235236 | 124 |
| CR001055 | HPFH | - | GAACUUCGUUAAAUUUAAUC | chr11:5235226-5235246 | 125 |
| CR001056 | HPFH | + | AUUAAAUUUAACGAAGUUCC | chr11:5235227-5235247 | 126 |
| CR001057 | HPFH | + | UUAAAUUUAACGAAGUUCCU | chr11:5235228-5235248 | 127 |
| CR001058 | HPFH | - | UUCUGUACUAGCAUAUUCCC | chr11:5235248-5235268 | 128 |
| CR001059 | HPFH | + | UGUGUUCUUAAAAAAAAAUG | Chr11:5235275-5235297 | 129 |
| CR001060 | HPFH | + | AAAAAUGUGGAAUUAGACCC | chr11:5235293-5235313 | 130 |
| CR001061 | HPFH | - | CUACUGGGAUCUUCAUUCCU | chr11:5235313-5235333 | 131 |
| CR001062 | HPFH | - | ACUACUGGGAUCUUCAUUCC | chr11:5235314-5235334 | 132 |
| CR001063 | HPFH | - | GAAAAGAGUGAAAAACUACU | chr11:5235328-5235348 | 133 |
| CR001064 | HPFH | - | AGAAAAGAGUGAAAAACUAC | chr11:5235329-5235349 | 134 |
| CR001065 | HPFH | + | GAAUUCAAAUAAUGCCACAA | chr11:5235349-5235369 | 135 |
| CR001066 | HPFH | - | UGUGUAUUUGUCUGCCAUUG | chr11:5235366-5235386 | 136 |
| CR001067 | HPFH | + | CACCCAUGAGCAUAUCCAAA | chr11:5235384-5235404 | 137 |
| CR001068 | HPFH | - | UUCCUUUUGGAUAUGCUCAU | chr11:5235389-5235409 | 138 |
| CR001069 | HPFH | - | CUUCCUUUUGGAUAUGCUCA | chr11:5235390-5235410 | 139 |
| CR001070 | HPFH | + | CAUGAGCAUAUCCAAAAGGA | chr11:5235388-5235408 | 140 |
| CR001071 | HPFH | + | UAUCCAAAAGGAAGGAUUGA | chr11:5235396-5235416 | 141 |
| CR001072 | HPFH | - | UUUCCUUCAAUCCUUCCUUU | chr11:5235402-5235422 | 142 |
| CR001073 | HPFH | + | AAGGAAGGAUUGAAGGAAAG | chr11:5235403-5235423 | 143 |
| CR001074 | HPFH | + | GAAGGAUUGAAGGAAAGAGG | chr11:5235406-5235426 | 144 |
| CR001075 | HPFH | + | GAGGAGGAAGAAAUGGAGAA | chr11:5235422-5235442 | 145 |
| CR001076 | HPFH | + | AGGAAGAAAUGGAGAAAGGA | chr11:5235426-5235446 | 146 |
| CR001077 | HPFH | + | GAAGGAAGAGGGGAAGAGAG | chr11:5235448-5235468 | 147 |
| CR001078 | HPFH | + | GAAGAGGGGAAGAGAGAGGA | chr11:5235452-5235472 | 148 |
| CR001079 | HPFH | + | AGGGGAAGAGAGAGGAUGGA | chr11:5235456-5235476 | 149 |
| CR001080 | HPFH | + | GGGGAAGAGAGAGGAUGGAA | chr11:5235457-5235477 | 150 |
| CR001081 | HPFH | + | AAGAGAGAGGAUGGAAGGGA | chr11:5235461-5235481 | 151 |
| CR001082 | HPFH | + | AGAGAGGAUGGAAGGGAUGG | chr11:5235464-5235484 | 152 |
| CR001083 | HPFH | + | GGAAGGGAUGGAGGAGAAGA | chr11:5235473-5235493 | 153 |
| CR001084 | HPFH | + | GAAGAAGGAAAAAUAAAUAA | Chr11:5235483-5235505 | 154 |
| CR001085 | HPFH | + | AGGAAAAAUAAAUAAUGGAG | Chr11:5235488-5235510 | 155 |
| CR001086 | HPFH | + | AAAUAAAUAAUGGAGAGGAG | chr11:5235498-5235518 | 156 |
| CR001087 | HPFH | + | UGGAGAGGAGAGGAGAAAAA | chr11:5235508-5235528 | 157 |
| CR001088 | HPFH | + | AGAGGAGAGGAGAAAAAAGG | chr11:5235511-5235531 | 158 |
| CR001089 | HPFH | + | GAGGAGAGGAGAAAAAAGGA | chr11:5235512-5235532 | 159 |
| CR001090 | HPFH | + | AGGAGAGGAGAAAAAAGGAG | chr11:5235513-5235533 | 160 |
| CR001091 | HPFH | + | AGGAGAAAAAAGGAGGGGAG | chr11:5235518-5235538 | 161 |
| CR001092 | HPFH | + | GAGAGGAGAGGAGAAGGGAU | chr11:5235535-5235555 | 162 |
| CR001093 | HPFH | + | AGAGGAGAGGAGAAGGGAUA | chr11:5235536-5235556 | 163 |
| CR001094 | HPFH | + | GAAGAGAAAGAGAAAGGGAA | Chr11:5235553-5235575 | 164 |
| CR001095 | HPFH | + | AAGAGAGGAAAGAAGAGAAG | chr11:5235581-5235601 | 165 |
| CR001096 | HPFH | + | GAGAGAAAAGAAACGAAGAG | Chr11:5235598-5235620 | 166 |
| CR001097 | HPFH | + | AGAGAAAAGAAACGAAGAGA | Chr11:5235599-5235621 | 167 |
| CR001098 | HPFH | + | GAGAAAAGAAACGAAGAGAG | Chr11:5235600-5235622 | 168 |
| CR001099 | HPFH | + | AAAGAAACGAAGAGAGGGGA | chr11:5235609-5235629 | 169 |
| CR001100 | HPFH | + | AAGAAACGAAGAGAGGGGAA | chr11:5235610-5235630 | 170 |
| CR001101 | HPFH | + | GGAAGGGAAGGAAAAAAAAG | chr11:5235626-5235646 | 171 |
| CR001102 | HPFH | + | AAGACUGACAGUUCAAAUUU | chr11:5235672-5235692 | 172 |
| CR001103 | HPFH | + | ACUGACAGUUCAAAUUUUGG | chr11:5235675-5235695 | 173 |
| CR001104 | HPFH | + | UUCAAAUUUUGGUGGUGAUA | chr11:5235683-5235703 | 174 |
| CR001105 | HPFH | + | AAUAGAAACUCAAACUCUGU | chr11:5235709-5235729 | 175 |
| CR001106 | HPFH | + | GUACAAUAGUAUAACCCCUU | chr11:5235739-5235759 | 176 |
| CR001107 | HPFH | - | CUAUUAAAGGUUUUCCAAAG | chr11:5235756-5235776 | 177 |
| CR001108 | HPFH | - | ACUAUUAAAGGUUUUCCAAA | chr11:5235757-5235777 | 178 |
| CR001109 | HPFH | - | UACUAUUAAAGGUUUUCCAA | chr11:5235758-5235778 | 179 |
| CR001110 | HPFH | - | GCAUUUGUGGAUACUAUUAA | chr11:5235769-5235789 | 180 |
| CR001111 | HPFH | + | UUAAUAGUAUCCACAAAUGC | chr11:5235769-5235789 | 181 |
| CR001132 | HPFH | - | UAUCAAGCAUCCAGCAUUUG | chr11:5235782-5235802 | 1500 |
| CR001133 | HPFH | - | UAUCUAAAAAUGUAAUUGCU | chr11:5235814-5235834 | 1501 |
| CR001134 | HPFH | - | AGCAUUUCUAUACAUGUCUU | chr11:5235862-5235882 | 1502 |
| CR001135 | HPFH | + | UAAUCAUAAAAACCUCAAAC | chr11:5235893-5235913 | 1503 |
| CR001136 | HPFH | - | UUUAAGUGGCUACCGGUUUG | chr11:5235908-5235928 | 1504 |
| CR001137 | HPFH | - | GUAAGCAUUUAAGUGGCUAC | chr11:5235915-5235935 | 1505 |
| CR001138 | HPFH | - | ACUGUUGGUAAGCAUUUAAG | chr11:5235922-5235942 | 1506 |
| CR001139 | HPFH | - | UAAUUUAUCAAUUCUACUGU | chr11:5235937-5235957 | 1507 |
| CR001140 | HPFH | + | ACAGUAGAAUUGAUAAAUUA | chr11:5235937-5235957 | 1508 |
| CR001141 | HPFH | + | CAAAUGCAUUUUACAGCAUU | chr11:5236027-5236047 | 1509 |
| CR001142 | HPFH | + | GGUUGAUUAAAAGUAACCAG | chr11:5236048-5236068 | 1510 |
| CR001143 | HPFH | - | AUAUAGUUUGAACUCACCUC | Chr11:5236059-5236081 | 1511 |
| CR001144 | HPFH | + | UUUAUUUGUAUAUAGAAAGA | chr11:5236090-5236110 | 1512 |
| CR001145 | HPFH | + | UGCCUGAGAUUCUGAUCACA | chr11:5236119-5236139 | 1513 |
| CR001146 | HPFH | + | GCCUGAGAUUCUGAUCACAA | chr11:5236120-5236140 | 1514 |
| CR001147 | HPFH | + | CCUGAGAUUCUGAUCACAAG | chr11:5236121-5236141 | 1515 |
| CR001148 | HPFH | - | CCCCUUGUGAUCAGAAUCUC | chr11:5236124-5236144 | 1516 |
| CR001149 | HPFH | + | AAGGGGAAAUGUUAUAAAAU | chr11:5236138-5236158 | 1517 |
| CR001150 | HPFH | + | AGGGGAAAUGUUAUAAAAUA | chr11:5236139-5236159 | 1518 |
| CR001151 | HPFH | + | UGUUAUAAAAUAGGGUAGAG | chr11:5236147-5236167 | 1519 |
| CR001152 | HPFH | - | CAAAGUUUAAAGGUCAUUCA | chr11:5236175-5236195 | 1520 |
| CR001153 | HPFH | - | UAACUUGUAACAAAGUUUAA | chr11:5236185-5236205 | 1521 |
| CR001154 | HPFH | + | CAAGUUAUUUUUCUGUAACC | chr11:5236198-5236218 | 1522 |
| CR001155 | HPFH | - | AAUAUCUUUCGUUGGCUUCC | chr11:5236219-5236239 | 1523 |
| CR001156 | HPFH | - | AAUUAUUCAAUAUCUUUCGU | chr11:5236227-5236247 | 1524 |
| CR001157 | HPFH | + | GAUAUUGAAUAAUUCAAGAA | chr11:5236233-5236253 | 1525 |
| CR001158 | HPFH | + | AUUGAAUAAUUCAAGAAAGG | chr11:5236236-5236256 | 1526 |
| CR001159 | HPFH | + | GAAUAAUUCAAGAAAGGUGG | chr11:5236239-5236259 | 1527 |
| CR001160 | HPFH | + | AUUCAAGAAAGGUGGUGGCA | chr11:5236244-5236264 | 1528 |
| CR001161 | HPFH | + | UAUUUUAGAAGUAGAGAAAA | chr11:5236313-5236333 | 1529 |
| CR001162 | HPFH | + | AUUUUAGAAGUAGAGAAAAU | chr11:5236314-5236334 | 1530 |
| CR001163 | HPFH | + | GAAAAUGGGAGACAAAUAGC | chr11:5236328-5236348 | 1531 |
| CR001164 | HPFH | + | AAAAUGGGAGACAAAUAGCU | chr11:5236329-5236349 | 1532 |
| CR001165 | HPFH | + | AGCUGGGCUUCUGUUGCAGU | chr11:5236345-5236365 | 1533 |
| CR001166 | HPFH | + | GCUGGGCUUCUGUUGCAGUA | chr11:5236346-5236366 | 1534 |
| CR001167 | HPFH | + | GCCAUUUCUAUUAUCAGACU | chr11:5236383-5236403 | 1535 |
| CR001168 | HPFH | - | UCCAAGUCUGAUAAUAGAAA | chr11:5236387-5236407 | 1536 |
| CR001169 | HPFH | + | UUAUCAGACUUGGACCAUGA | chr11:5236393-5236413 | 1537 |
| CR001170 | HPFH | - | CACGACUGACAUCACCGUCA | chr11:5236410-5236430 | 1538 |
| CR001171 | HPFH | + | UCAGUCGUGAACACAAGAAU | chr11:5236421-5236441 | 1539 |
| CR001172 | HPFH | + | CAGUCGUGAACACAAGAAUA | chr11:5236422-5236442 | 1540 |
| CR001173 | HPFH | + | GGCCACAUUUGUGAGUUUAG | chr11:5236443-5236463 | 1541 |
| CR001174 | HPFH | - | UACCACUAAACUCACAAAUG | chr11:5236448-5236468 | 1542 |
| CR001175 | HPFH | + | UAAAAUCAGAAAUACAGUCU | chr11:5236471-5236491 | 1543 |
| CR001176 | HPFH | + | AAAAGAUGUACUUAGAUAUG | chr11:5236528-5236548 | 1544 |
| CR001177 | HPFH | + | UGUACUUAGAUAUGUGGAUC | chr11:5236534-5236554 | 1545 |
| CR001178 | HPFH | + | AGCUCAGAAAGAAUACAACC | chr11:5236557-5236577 | 1546 |
| CR001179 | HPFH | + | ACCAGGUCAAGAAUACAGAA | chr11:5236574-5236594 | 1547 |
| CR001180 | HPFH | - | UCCAUUCUGUAUUCUUGACC | chr11:5236578-5236598 | 1548 |
| CR001181 | HPFH | - | CUGUCAUUUUUAACAGGUAG | chr11:5236646-5236666 | 1549 |
| CR001182 | HPFH | - | CAUCAUCUGUCAUUUUUAAC | chr11:5236652-5236672 | 1550 |
| CR001183 | HPFH | - | AAACACAUUCUAAGAUUUUA | chr11:5236691-5236711 | 1551 |
| CR001184 | HPFH | + | AAUCUUAGAAUGUGUUUGUG | chr11:5236694-5236714 | 1552 |
| CR001185 | HPFH | + | AUCUUAGAAUGUGUUUGUGA | chr11:5236695-5236715 | 1553 |
| CR001186 | HPFH | + | UUAGAAUGUGUUUGUGAGGG | chr11:5236698-5236718 | 1554 |
| CR001187 | HPFH | - | CAAUUUUCUUAUAUAUGAAU | chr11:5236734-5236754 | 1555 |
| CR001188 | HPFH | + | UUGAUUCUAAAAAAAAUGUU | Chr11:5236746-5236768 | 1556 |
| CR001189 | HPFH | + | AAAUGUUAGGUAAAUUCUUA | chr11:5236764-5236784 | 1557 |
| CR001190 | HPFH | + | GGUAAAUUCUUAAGGCCAUG | chr11:5236772-5236792 | 1558 |
| CR001191 | HPFH | - | AGAUCAAAUAACAGUCCUCA | chr11:5236790-5236810 | 1559 |
| CR001192 | HPFH | + | GUCUGUUAAUUCCAAAGACU | chr11:5236812-5236832 | 1560 |
| CR001193 | HPFH | - | AAAGUGAAAAGCCAAGUCUU | chr11:5236826-5236846 | 1561 |
| CR001194 | HPFH | + | CCUGAAAUGAUUUUACACAU | chr11:5236858-5236878 | 1562 |
| CR001195 | HPFH | - | CCAAUGUGUAAAAUCAUUUC | chr11:5236861-5236881 | 1563 |
| CR001196 | HPFH | + | CUGAAAUGAUUUUACACAUU | chr11:5236859-5236879 | 1564 |
| CR001197 | HPFH | + | AUUUUACACAUUGGGAGAUC | chr11:5236867-5236887 | 1565 |
| CR001198 | HPFH | + | GGUUACAUGUUUAUUCUAUA | chr11:5236888-5236908 | 1566 |
| CR001199 | HPFH | + | UCUAUAUGGAUUGCAUUGAG | chr11:5236902-5236922 | 1567 |
| CR001200 | HPFH | + | AGGAUUUGUAUAACAGAAUA | chr11:5236922-5236942 | 1568 |
| CR001201 | HPFH | + | UUUUCUUUUCUCUUCUGAGA | Chr11:5236945-5236967 | 1569 |
| CR001202 | HPFH | - | GCACUCUAGCUUGGGCAAUA | chr11:5236984-5237004 | 1570 |
| CR001203 | HPFH | - | UGCACUCUAGCUUGGGCAAU | chr11:5236985-5237005 | 1571 |
| CR001204 | HPFH | - | UGCACCAUUGCACUCUAGCU | Chr11:5236985-5237007 | 1572 |
| CR001205 | HPFH | - | GCUAUUCAGGUGGCUGAGGC | chr11:5237061-5237081 | 1573 |
| CR001206 | HPFH | + | ACCUGAAUAGCUGGGACUGC | Chr11:5237065-5237087 | 1574 |
| CR001207 | HPFH | + | GCAGGCAUGCACCACACGCC | Chr11:5237083-5237105 | 1575 |
| CR001208 | HPFH | - | UACAAAAUCAGCCGGGCGUG | chr11:5237102-5237122 | 1576 |
| CR001209 | HPFH | - | GGCUUGUAAACCCAGCACUU | chr11:5237208-5237228 | 1577 |
| CR001210 | HPFH | - | CUGGCUGGAUGCGGUGGCUC | chr11:5237229-5237249 | 1578 |
| CR001211 | HPFH | + | CUGAGCCACCGCAUCCAGCC | chr11:5237227-5237247 | 1579 |
| CR001212 | HPFH | - | CUUAUCCUGGCUGGAUGCGG | chr11:5237235-5237255 | 1580 |
| CR001213 | HPFH | + | CACCGCAUCCAGCCAGGAUA | chr11:5237233-5237253 | 1581 |
| CR001214 | HPFH | - | GACCUUAUCCUGGCUGGAUG | chr11:5237238-5237258 | 1582 |
| CR001215 | HPFH | - | CUUUUAGACCUUAUCCUGGC | chr11:5237244-5237264 | 1583 |
| CR001216 | HPFH | + | GCCAGGAUAAGGUCUAAAAG | chr11:5237244-5237264 | 1584 |
| CR001217 | HPFH | - | UCCACUUUUAGACCUUAUCC | chr11:5237248-5237268 | 1585 |
| CR001218 | HPFH | + | AAUAGCAUCUACUCUUGUUC | chr11:5237271-5237291 | 1586 |
| CR001219 | HPFH | + | CUCUUGUUCAGGAAACAAUG | chr11:5237282-5237302 | 1587 |
| CR001220 | HPFH | + | GGAAACAAUGAGGACCUGAC | chr11:5237292-5237312 | 1588 |
| CR001221 | HPFH | + | GAAACAAUGAGGACCUGACU | chr11:5237293-5237313 | 1589 |
| CR001222 | HPFH | + | ACCUGACUGGGCAGUAAGAG | chr11:5237305-5237325 | 1590 |
| CR001223 | HPFH | - | ACCACUCUUACUGCCCAGUC | chr11:5237309-5237329 | 1591 |
| CR001224 | HPFH | + | AAGAGUGGUGAUUAAUAGAU | chr11:5237320-5237340 | 1592 |
| CR001225 | HPFH | + | AGAGUGGUGAUUAAUAGAUA | chr11:5237321-5237341 | 1593 |
| CR001226 | HPFH | + | AGAAUCGAACUGUUGAUUAG | chr11:5237356-5237376 | 1594 |
| CR001227 | HPFH | + | UCGAACUGUUGAUUAGAGGU | chr11:5237360-5237380 | 1595 |
| CR003027 | HPFH | + | CGAACUGUUGAUUAGAGGUA | chr11:5237361-5237381 | 1692 |
| CR003028 | HPFH | + | AUGAUUUUAAUCUGUGACCU | chr11:5237386-5237406 | 1693 |
| CR003029 | HPFH | + | UAAUCUGUGACCUUGGUGAA | chr11:5237393-5237413 | 1694 |
| CR003030 | HPFH | + | AAUCUGUGACCUUGGUGAAU | chr11:5237394-5237414 | 1695 |
| CR003031 | HPFH | - | AGCUACUUGCCCAUUCACCA | chr11:5237406-5237426 | 1696 |
| CR003032 | HPFH | + | UAGCUAUCUAAUGACUAAAA | chr11:5237421-5237441 | 1697 |
| CR003033 | HPFH | + | AUGACUAAAAUGGAAAACAC | chr11:5237431-5237451 | 1698 |
| CR003034 | HPFH | + | AAAUACCCAUGCUGAGUCUG | chr11:5237482-5237502 | 1699 |
| CR003035 | HPFH | - | AGGCACCUCAGACUCAGCAU | chr11:5237490-5237510 | 1700 |
| CR003036 | HPFH | - | UAGGCACCUCAGACUCAGCA | chr11:5237491-5237511 | 1701 |
| CR003037 | HPFH | + | GCUGAGUCUGAGGUGCCUAU | chr11:5237492-5237512 | 1702 |
| CR003038 | HPFH | - | UAUUUAUAUAGAUGUCCUAU | chr11:5237510-5237530 | 1703 |
| CR003039 | HPFH | - | CAUAUAUCAAACAAUGUACU | chr11:5237535-5237555 | 1704 |
| CR003040 | HPFH | + | CCAGUACAUUGUUUGAUAUA | chr11:5237533-5237553 | 1705 |
| CR003041 | HPFH | - | CCAUAUAUCAAACAAUGUAC | chr11:5237536-5237556 | 1706 |
| CR003042 | HPFH | + | CAGUACAUUGUUUGAUAUAU | chr11:5237534-5237554 | 1707 |
| CR003043 | HPFH | + | CAUUGUUUGAUAUAUGGGUU | chr11:5237539-5237559 | 1708 |
| CR003044 | HPFH | + | GAUAUAUGGGUUUGGCACUG | chr11:5237547-5237567 | 1709 |
| CR003045 | HPFH | + | UAUGGGUUUGGCACUGAGGU | chr11:5237551-5237571 | 1710 |
| CR003046 | HPFH | + | GGGUUUGGCACUGAGGUUGG | chr11:5237554-5237574 | 1711 |
| CR003047 | HPFH | + | GCACUGAGGUUGGAGGUCAG | chr11:5237561-5237581 | 1712 |
| CR003048 | HPFH | + | CAGAGGUUAGAAAUCAGAGU | chr11:5237578-5237598 | 1713 |
| CR003049 | HPFH | + | AGAGGUUAGAAAUCAGAGUU | chr11:5237579-5237599 | 1714 |
| CR003050 | HPFH | + | UAGAAAUCAGAGUUGGGAAU | chr11:5237585-5237605 | 1715 |
| CR003051 | HPFH | + | AGAAAUCAGAGUUGGGAAUU | chr11:5237586-5237606 | 1716 |
| CR003052 | HPFH | + | GUUGGGAAUUGGGAUUAUAC | chr11:5237596-5237616 | 1717 |
| CR003053 | HPFH | - | CUUUGUAUUCAUCACACUCU | chr11:5237654-5237674 | 1718 |
| CR003054 | HPFH | + | AUGAAUACAAAGUUAAAUGA | chr11:5237662-5237682 | 1719 |
| CR003055 | HPFH | - | UAAAUGUUGGUGUUCAUUAA | chr11:5237689-5237709 | 1720 |
| CR003056 | HPFH | - | UGAGAUUUCACAUUAAAUGU | chr11:5237702-5237722 | 1721 |
| CR003057 | HPFH | + | ACAUUUAAUGUGAAAUCUCA | chr11:5237702-5237722 | 1722 |
| CR003058 | HPFH | - | UAAAAUCAUCGGGGAUUUUG | chr11:5237749-5237769 | 1723 |
| CR003059 | HPFH | - | CUAAAAUCAUCGGGGAUUUU | chr11:5237750-5237770 | 1724 |
| CR003060 | HPFH | - | UCUAAAAUCAUCGGGGAUUU | chr11:5237751-5237771 | 1725 |
| CR003061 | HPFH | - | ACUGAGUUCUAAAAUCAUCG | chr11:5237758-5237778 | 1726 |
| CR003062 | HPFH | - | UACUGAGUUCUAAAAUCAUC | chr11:5237759-5237779 | 1727 |
| CR003063 | HPFH | - | AUACUGAGUUCUAAAAUCAU | chr11:5237760-5237780 | 1728 |
| CR003064 | HPFH | + | UAAUUAGUGUAAUGCCAAUG | chr11:5237786-5237806 | 1729 |
| CR003065 | HPFH | + | AAUUAGUGUAAUGCCAAUGU | chr11:5237787-5237807 | 1730 |
| CR003066 | HPFH | + | AAUGCCAAUGUGGGUUAGAA | chr11:5237796-5237816 | 1731 |
| CR003067 | HPFH | - | ACUUCCAUUCUAACCCACAU | chr11:5237803-5237823 | 1732 |
| CR003068 | HPFH | + | AAUGGAAGUCAACUUGCUGU | chr11:5237814-5237834 | 1733 |
| CR003069 | HPFH | + | CUUGCUGUUGGUUUCAGAGC | chr11:5237826-5237846 | 1734 |
| CR003070 | HPFH | + | CUGUUGGUUUCAGAGCAGGU | chr11:5237830-5237850 | 1735 |
| CR003071 | HPFH | + | UUCAGAGCAGGUAGGAGAUA | chr11:5237838-5237858 | 1736 |
| CR003072 | HPFH | + | AGUGAAAAGCUGAAACAAAA | chr11:5237877-5237897 | 1737 |
| CR003073 | HPFH | + | AAGCUGAAACAAAAAGGAAA | chr11:5237883-5237903 | 1738 |
| CR003074 | HPFH | + | UGAAACAAAAAGGAAAAGGU | chr11:5237887-5237907 | 1739 |
| CR003075 | HPFH | + | GAAACAAAAAGGAAAAGGUA | chr11:5237888-5237908 | 1740 |
| CR003076 | HPFH | + | GGAAAAGGUAGGGUGAAAGA | chr11:5237898-5237918 | 1741 |
| CR003077 | HPFH | + | GAAAAGGUAGGGUGAAAGAU | chr11:5237899-5237919 | 1742 |
| CR003078 | HPFH | + | AAAGAUGGGAAAUGUAUGUA | chr11:5237913-5237933 | 1743 |
| CR003079 | HPFH | + | GAUGGGAAAUGUAUGUAAGG | chr11:5237916-5237936 | 1744 |
| CR003080 | HPFH | + | UGUAAGGAGGAUGAGCCACA | chr11:5237929-5237949 | 1745 |
| CR003081 | HPFH | + | GGAGGAUGAGCCACAUGGUA | chr11:5237934-5237954 | 1746 |
| CR003082 | HPFH | + | GAGGAUGAGCCACAUGGUAU | chr11:5237935-5237955 | 1747 |
| CR003083 | HPFH | + | GAUGAGCCACAUGGUAUGGG | chr11:5237938-5237958 | 1748 |
| CR003084 | HPFH | - | AGUAUACCUCCCAUACCAUG | chr11:5237947-5237967 | 1749 |
| CR003085 | HPFH | + | AUGGUAUGGGAGGUAUACUA | chr11:5237948-5237968 | 1750 |
| CR003086 | HPFH | + | GGAGGUAUACUAAGGACUCU | chr11:5237956-5237976 | 1751 |
| CR003087 | HPFH | + | GAGGUAUACUAAGGACUCUA | chr11:5237957-5237977 | 1752 |
| CR003088 | HPFH | + | ACUCUAGGGUCAGAGAAAUA | chr11:5237971-5237991 | 1753 |
| CR003089 | HPFH | + | CUCUAGGGUCAGAGAAAUAU | chr11:5237972-5237992 | 1754 |
| CR003090 | HPFH | - | AAGAAUGUGAAUUUUGUAGA | chr11:5238004-5238024 | 1755 |
| CR003091 | HPFH | + | UUCUACAAAAUUCACAUUCU | chr11:5238003-5238023 | 1756 |
| CR003092 | HPFH | + | ACAAAAUUCACAUUCUUGGC | chr11:5238007-5238027 | 1757 |
| CR003093 | HPFH | + | CAAAAUUCACAUUCUUGGCU | chr11:5238008-5238028 | 1758 |
| CR003094 | HPFH | + | UUCACAUUCUUGGCUGGGUG | chr11:5238013-5238033 | 1759 |
| CR003095 | HPFH | + | AGGGUGGAUCACCUGAUGUU | chr11:5238071-5238093 | 1760 |
| CR003096 | HPFH | - | GAUCUCGAACUCCUAACAUC | chr11:5238090-5238110 | 1761 |

In some aspects of the invention, it is preferred that the gRNA molecule to an HPFH region comprise, e.g., consist of, a targeting domain of a gRNA capable of producing at least about a 20% increase in F cells relative to control, e.g., in an assay described in Example 4. In an aspect, the gRNA molecule includes a targeting domain comprising, e.g., consisting of, SEQ ID NO: 113. In an aspect, the gRNA molecule includes a targeting domain comprising, e.g., consisting of, SEQ ID NO: 99. In an aspect, the gRNA molecule includes a targeting domain comprising, e.g., consisting of, SEQ ID NO: 112. In an aspect, the gRNA molecule includes a targeting domain comprising, e.g., consisting of, SEQ ID NO: 98. In an aspect, the gRNA molecule includes a targeting domain comprising, e.g., consisting of, SEQ ID NO: 1580. In an aspect, the gRNA molecule includes a targeting domain comprising, e.g., consisting of, SEQ ID NO: 106. In an aspect, the gRNA molecule includes a targeting domain comprising, e.g., consisting of, SEQ ID NO: 1589. In an aspect, the gRNA molecule includes a targeting domain comprising, e.g., consisting of, SEQ ID NO: 1503. In an aspect, the gRNA molecule includes a targeting domain comprising, e.g., consisting of, SEQ ID NO: 160. In an aspect, the gRNA molecule includes a targeting domain comprising, e.g., consisting of, SEQ ID NO: 1537. In an aspect, the gRNA molecule includes a targeting domain comprising, e.g., consisting of, SEQ ID NO: 159. In an aspect, the gRNA molecule includes a targeting domain comprising, e.g., consisting of, SEQ ID NO: 101. In an aspect, the gRNA molecule includes a targeting domain comprising, e.g., consisting of, SEQ ID NO: 162. In an aspect, the gRNA molecule includes a targeting domain comprising, e.g., consisting of, SEQ ID NO: 104. In an aspect, the gRNA molecule includes a targeting domain comprising, e.g., consisting of, SEQ ID NO: 138. In an aspect, the gRNA molecule includes a targeting domain comprising, e.g., consisting of, SEQ ID NO: 1536. In an aspect, the gRNA molecule includes a targeting domain comprising, e.g., consisting of, SEQ ID NO: 1539. In an aspect, the gRNA molecule includes a targeting domain comprising, e.g., consisting of, SEQ ID NO: 1585.

In some aspects of the invention, e.g., as indicated herein, it may be beneficial to include gRNA molecules targeting more than one, e.g., two, target sites (e.g., a first gRNA molecule and a second gRNA molecule). In some embodiments, the two target sites are both located in an HPFH region. In such aspects, any combination of more than one, e.g., two, gRNA molecules (e.g., a first gRNA molecule and a second gRNA molecule) comprising targeting domains listed in Table 6 may be used. In an aspect, the first gRNA molecule and the second gRNA molecule include targeting domains comprising, e.g., consisting of, SEQ ID NO: 100 and SEQ ID NO: 165, respectively. In an aspect, the first gRNA molecule and the second gRNA molecule include targeting domains comprising, e.g., consisting of, SEQ ID NO: 100 and SEQ ID NO: 113, respectively. In an aspect, the first gRNA molecule and the second gRNA molecule include targeting domains comprising, e.g., consisting of, SEQ ID NO: 100 and SEQ ID NO: 99, respectively. In an aspect, the first gRNA molecule and the second gRNA molecule include targeting domains comprising, e.g., consisting of, SEQ ID NO: 100 and SEQ ID NO: 112, respectively. In an aspect, the first gRNA molecule and the second gRNA molecule include targeting domains comprising, e.g., consisting of, SEQ ID NO: 100 and SEQ ID NO: 98, respectively. In an aspect, the first gRNA molecule and the second gRNA molecule include targeting domains comprising, e.g., consisting of, SEQ ID NO: 100 and SEQ ID NO: 1580, respectively. In an aspect, the first gRNA molecule and the second gRNA molecule include targeting domains comprising, e.g., consisting of, SEQ ID NO: 100 and SEQ ID NO: 106, respectively. In an aspect, the first gRNA molecule and the second gRNA molecule include targeting domains comprising, e.g., consisting of, SEQ ID NO: 100 and SEQ ID NO: 1503, respectively. In an aspect, the first gRNA molecule and the second gRNA molecule include targeting domains comprising, e.g., consisting of, SEQ ID NO: 100 and SEQ ID NO: 1589, respectively. In an aspect, the first gRNA molecule and the second gRNA molecule include targeting domains comprising, e.g., consisting of, SEQ ID NO: 100 and SEQ ID NO: 160, respectively. In an aspect, the first gRNA molecule and the second gRNA molecule include targeting domains comprising, e.g., consisting of, SEQ ID NO: 100 and SEQ ID NO: 1537, respectively. In an aspect, the first gRNA molecule and the second gRNA molecule include targeting domains comprising, e.g., consisting of, SEQ ID NO: 100 and SEQ ID NO: 159, respectively. In an aspect, the first gRNA molecule and the second gRNA molecule include targeting domains comprising, e.g., consisting of, SEQ ID NO: 100 and SEQ ID NO: 101, respectively. In an aspect, the first gRNA molecule and the second gRNA molecule include targeting domains comprising, e.g., consisting of, SEQ ID NO: 100 and SEQ ID NO: 162, respectively. In an aspect, the first gRNA molecule and the second gRNA molecule include targeting domains comprising, e.g., consisting of, SEQ ID NO: 100 and SEQ ID NO: 104, respectively. In an aspect, the first gRNA molecule and the second gRNA molecule include targeting domains comprising, e.g., consisting of, SEQ ID NO: 100 and SEQ ID NO: 138, respectively. In an aspect, the first gRNA molecule and the second gRNA molecule include targeting domains comprising, e.g., consisting of, SEQ ID NO: 100 and SEQ ID NO: 1536, respectively. In an aspect, the first gRNA molecule and the second gRNA molecule include targeting domains comprising, e.g., consisting of, SEQ ID NO: 100 and SEQ ID NO: 1539, respectively. In an aspect, the first gRNA molecule and the second gRNA molecule include targeting domains comprising, e.g., consisting of, SEQ ID NO: 100 and SEQ ID NO: 1585, respectively. In an aspect, the first gRNA molecule and the second gRNA molecule include targeting domains comprising, e.g., consisting of, SEQ ID NO: 165 and SEQ ID NO: 113, respectively. In an aspect, the first gRNA molecule and the second gRNA molecule include targeting domains comprising, e.g., consisting of, SEQ ID NO: 165 and SEQ ID NO: 99, respectively. In an aspect, the first gRNA molecule and the second gRNA molecule include targeting domains comprising, e.g., consisting of, SEQ ID NO: 165 and SEQ ID NO: 112, respectively. In an aspect, the first gRNA molecule and the second gRNA molecule include targeting domains comprising, e.g., consisting of, SEQ ID NO: 165 and SEQ ID NO: 98, respectively. In an aspect, the first gRNA molecule and the second gRNA molecule include targeting domains comprising, e.g., consisting of, SEQ ID NO: 165 and SEQ ID NO: 1580, respectively. In an aspect, the first gRNA molecule and the second gRNA molecule include targeting domains comprising, e.g., consisting of, SEQ ID NO: 165 and SEQ ID NO: 106, respectively. In an aspect, the first gRNA molecule and the second gRNA molecule include targeting domains comprising, e.g., consisting of, SEQ ID NO: 165 and SEQ ID NO: 1503, respectively. In an aspect, the first gRNA molecule and the second gRNA molecule include targeting domains comprising, e.g., consisting of, SEQ ID NO: 165 and SEQ ID NO: 1589, respectively. In an aspect, the first gRNA molecule and the second gRNA molecule include targeting domains comprising, e.g., consisting of, SEQ ID NO: 165 and SEQ ID NO: 160, respectively. In an aspect, the first gRNA molecule and the second gRNA molecule include targeting domains comprising, e.g., consisting of, SEQ ID NO: 165 and SEQ ID NO: 1537, respectively. In an aspect, the first gRNA molecule and the second gRNA molecule include targeting domains comprising, e.g., consisting of, SEQ ID NO: 165 and SEQ ID NO: 159, respectively. In an aspect, the first gRNA molecule and the second gRNA molecule include targeting domains comprising, e.g., consisting of, SEQ ID NO: 165 and SEQ ID NO: 101, respectively. In an aspect, the first gRNA molecule and the second gRNA molecule include targeting domains comprising, e.g., consisting of, SEQ ID NO: 165 and SEQ ID NO: 162, respectively. In an aspect, the first gRNA molecule and the second gRNA molecule include targeting domains comprising, e.g., consisting of, SEQ ID NO: 165 and SEQ ID NO: 104, respectively. In an aspect, the first gRNA molecule and the second gRNA molecule include targeting domains comprising, e.g., consisting of, SEQ ID NO: 165 and SEQ ID NO: 138, respectively. In an aspect, the first gRNA molecule and the second gRNA molecule include targeting domains comprising, e.g., consisting of, SEQ ID NO: 165 and SEQ ID NO: 1536, respectively. In an aspect, the first gRNA molecule and the second gRNA molecule include targeting domains comprising, e.g., consisting of, SEQ ID NO: 165 and SEQ ID NO: 1539, respectively. In an aspect, the first gRNA molecule and the second gRNA molecule include targeting domains comprising, e.g., consisting of, SEQ ID NO: 165 and SEQ ID NO: 1585, respectively. In an aspect, the first gRNA molecule and the second gRNA molecule include targeting domains comprising, e.g., consisting of, SEQ ID NO: 113 and SEQ ID NO: 165, respectively. In an aspect, the first gRNA molecule and the second gRNA molecule include targeting domains comprising, e.g., consisting of, SEQ ID NO: 113 and SEQ ID NO: 99, respectively. In an aspect, the first gRNA molecule and the second gRNA molecule include targeting domains comprising, e.g., consisting of, SEQ ID NO: 113 and SEQ ID NO: 112, respectively. In an aspect, the first gRNA molecule and the second gRNA molecule include targeting domains comprising, e.g., consisting of, SEQ ID NO: 113 and SEQ ID NO: 98, respectively. In an aspect, the first gRNA molecule and the second gRNA molecule include targeting domains comprising, e.g., consisting of, SEQ ID NO: 113 and SEQ ID NO: 1580, respectively. In an aspect, the first gRNA molecule and the second gRNA molecule include targeting domains comprising, e.g., consisting of, SEQ ID NO: 113 and SEQ ID NO: 106, respectively. In an aspect, the first gRNA molecule and the second gRNA molecule include targeting domains comprising, e.g., consisting of, SEQ ID NO: 113 and SEQ ID NO: 1503, respectively. In an aspect, the first gRNA molecule and the second gRNA molecule include targeting domains comprising, e.g., consisting of, SEQ ID NO: 113 and SEQ ID NO: 1589, respectively. In an aspect, the first gRNA molecule and the second gRNA molecule include targeting domains comprising, e.g., consisting of, SEQ ID NO: 113 and SEQ ID NO: 160, respectively. In an aspect, the first gRNA molecule and the second gRNA molecule include targeting domains comprising, e.g., consisting of, SEQ ID NO: 113 and SEQ ID NO: 1537, respectively. In an aspect, the first gRNA molecule and the second gRNA molecule include targeting domains comprising, e.g., consisting of, SEQ ID NO: 113 and SEQ ID NO: 159, respectively. In an aspect, the first gRNA molecule and the second gRNA molecule include targeting domains comprising, e.g., consisting of, SEQ ID NO: 113 and SEQ ID NO: 101, respectively. In an aspect, the first gRNA molecule and the second gRNA molecule include targeting domains comprising, e.g., consisting of, SEQ ID NO: 113 and SEQ ID NO: 162, respectively. In an aspect, the first gRNA molecule and the second gRNA molecule include targeting domains comprising, e.g., consisting of, SEQ ID NO: 113 and SEQ ID NO: 104, respectively. In an aspect, the first gRNA molecule and the second gRNA molecule include targeting domains comprising, e.g., consisting of, SEQ ID NO: 113 and SEQ ID NO: 138, respectively. In an aspect, the first gRNA molecule and the second gRNA molecule include targeting domains comprising, e.g., consisting of, SEQ ID NO: 113 and SEQ ID NO: 1536, respectively. In an aspect, the first gRNA molecule and the second gRNA molecule include targeting domains comprising, e.g., consisting of, SEQ ID NO: 113 and SEQ ID NO: 1539, respectively. In an aspect, the first gRNA molecule and the second gRNA molecule include targeting domains comprising, e.g., consisting of, SEQ ID NO: 113 and SEQ ID NO: 1585, respectively. In an aspect, the first gRNA molecule and the second gRNA molecule include targeting domains comprising, e.g., consisting of, SEQ ID NO: 99 and SEQ ID NO: 165, respectively. In an aspect, the first gRNA molecule and the second gRNA molecule include targeting domains comprising, e.g., consisting of, SEQ ID NO: 99 and SEQ ID NO: 113, respectively. In an aspect, the first gRNA molecule and the second gRNA molecule include targeting domains comprising, e.g., consisting of, SEQ ID NO: 99 and SEQ ID NO: 112, respectively. In an aspect, the first gRNA molecule and the second gRNA molecule include targeting domains comprising, e.g., consisting of, SEQ ID NO: 99 and SEQ ID NO: 98, respectively. In an aspect, the first gRNA molecule and the second gRNA molecule include targeting domains comprising, e.g., consisting of, SEQ ID NO: 99 and SEQ ID NO: 1580, respectively. In an aspect, the first gRNA molecule and the second gRNA molecule include targeting domains comprising, e.g., consisting of, SEQ ID NO: 99 and SEQ ID NO: 106, respectively. In an aspect, the first gRNA molecule and the second gRNA molecule include targeting domains comprising, e.g., consisting of, SEQ ID NO: 99 and SEQ ID NO: 1503, respectively. In an aspect, the first gRNA molecule and the second gRNA molecule include targeting domains comprising, e.g., consisting of, SEQ ID NO: 99 and SEQ ID NO: 1589, respectively. In an aspect, the first gRNA molecule and the second gRNA molecule include targeting domains comprising, e.g., consisting of, SEQ ID NO: 99 and SEQ ID NO: 160, respectively. In an aspect, the first gRNA molecule and the second gRNA molecule include targeting domains comprising, e.g., consisting of, SEQ ID NO: 99 and SEQ ID NO: 1537, respectively. In an aspect, the first gRNA molecule and the second gRNA molecule include targeting domains comprising, e.g., consisting of, SEQ ID NO: 99 and SEQ ID NO: 159, respectively. In an aspect, the first gRNA molecule and the second gRNA molecule include targeting domains comprising, e.g., consisting of, SEQ ID NO: 99 and SEQ ID NO: 101, respectively. In an aspect, the first gRNA molecule and the second gRNA molecule include targeting domains comprising, e.g., consisting of, SEQ ID NO: 99 and SEQ ID NO: 162, respectively. In an aspect, the first gRNA molecule and the second gRNA molecule include targeting domains comprising, e.g., consisting of, SEQ ID NO: 99 and SEQ ID NO: 104, respectively. In an aspect, the first gRNA molecule and the second gRNA molecule include targeting domains comprising, e.g., consisting of, SEQ ID NO: 99 and SEQ ID NO: 138, respectively. In an aspect, the first gRNA molecule and the second gRNA molecule include targeting domains comprising, e.g., consisting of, SEQ ID NO: 99 and SEQ ID NO: 1536, respectively. In an aspect, the first gRNA molecule and the second gRNA molecule include targeting domains comprising, e.g., consisting of, SEQ ID NO: 99 and SEQ ID NO: 1539, respectively. In an aspect, the first gRNA molecule and the second gRNA molecule include targeting domains comprising, e.g., consisting of, SEQ ID NO: 99 and SEQ ID NO: 1585, respectively. In an aspect, the first gRNA molecule and the second gRNA molecule include targeting domains comprising, e.g., consisting of, SEQ ID NO: 112 and SEQ ID NO: 165, respectively. In an aspect, the first gRNA molecule and the second gRNA molecule include targeting domains comprising, e.g., consisting of, SEQ ID NO: 112 and SEQ ID NO: 113, respectively. In an aspect, the first gRNA molecule and the second gRNA molecule include targeting domains comprising, e.g., consisting of, SEQ ID NO: 112 and SEQ ID NO: 99, respectively. In an aspect, the first gRNA molecule and the second gRNA molecule include targeting domains comprising, e.g., consisting of, SEQ ID NO: 112 and SEQ ID NO: 98, respectively. In an aspect, the first gRNA molecule and the second gRNA molecule include targeting domains comprising, e.g., consisting of, SEQ ID NO: 112 and SEQ ID NO: 1580, respectively. In an aspect, the first gRNA molecule and the second gRNA molecule include targeting domains comprising, e.g., consisting of, SEQ ID NO: 112 and SEQ ID NO: 106, respectively. In an aspect, the first gRNA molecule and the second gRNA molecule include targeting domains comprising, e.g., consisting of, SEQ ID NO: 112 and SEQ ID NO: 1503, respectively. In an aspect, the first gRNA molecule and the second gRNA molecule include targeting domains comprising, e.g., consisting of, SEQ ID NO: 112 and SEQ ID NO: 1589, respectively. In an aspect, the first gRNA molecule and the second gRNA molecule include targeting domains comprising, e.g., consisting of, SEQ ID NO: 112 and SEQ ID NO: 160, respectively. In an aspect, the first gRNA molecule and the second gRNA molecule include targeting domains comprising, e.g., consisting of, SEQ ID NO: 112 and SEQ ID NO: 1537, respectively. In an aspect, the first gRNA molecule and the second gRNA molecule include targeting domains comprising, e.g., consisting of, SEQ ID NO: 112 and SEQ ID NO: 159, respectively. In an aspect, the first gRNA molecule and the second gRNA molecule include targeting domains comprising, e.g., consisting of, SEQ ID NO: 112 and SEQ ID NO: 101, respectively. In an aspect, the first gRNA molecule and the second gRNA molecule include targeting domains comprising, e.g., consisting of, SEQ ID NO: 112 and SEQ ID NO: 162, respectively. In an aspect, the first gRNA molecule and the second gRNA molecule include targeting domains comprising, e.g., consisting of, SEQ ID NO: 112 and SEQ ID NO: 104, respectively. In an aspect, the first gRNA molecule and the second gRNA molecule include targeting domains comprising, e.g., consisting of, SEQ ID NO: 112 and SEQ ID NO: 138, respectively. In an aspect, the first gRNA molecule and the second gRNA molecule include targeting domains comprising, e.g., consisting of, SEQ ID NO: 112 and SEQ ID NO: 1536, respectively. In an aspect, the first gRNA molecule and the second gRNA molecule include targeting domains comprising, e.g., consisting of, SEQ ID NO: 112 and SEQ ID NO: 1539, respectively. In an aspect, the first gRNA molecule and the second gRNA molecule include targeting domains comprising, e.g., consisting of, SEQ ID NO: 112 and SEQ ID NO: 1585, respectively. In an aspect, the first gRNA molecule and the second gRNA molecule include targeting domains comprising, e.g., consisting of, SEQ ID NO: 98 and SEQ ID NO: 165, respectively. In an aspect, the first gRNA molecule and the second gRNA molecule include targeting domains comprising, e.g., consisting of, SEQ ID NO: 98 and SEQ ID NO: 113, respectively. In an aspect, the first gRNA molecule and the second gRNA molecule include targeting domains comprising, e.g., consisting of, SEQ ID NO: 98 and SEQ ID NO: 99, respectively. In an aspect, the first gRNA molecule and the second gRNA molecule include targeting domains comprising, e.g., consisting of, SEQ ID NO: 98 and SEQ ID NO: 112, respectively. In an aspect, the first gRNA molecule and the second gRNA molecule include targeting domains comprising, e.g., consisting of, SEQ ID NO: 98 and SEQ ID NO: 1580, respectively. In an aspect, the first gRNA molecule and the second gRNA molecule include targeting domains comprising, e.g., consisting of, SEQ ID NO: 98 and SEQ ID NO: 106, respectively. In an aspect, the first gRNA molecule and the second gRNA molecule include targeting domains comprising, e.g., consisting of, SEQ ID NO: 98 and SEQ ID NO: 1503, respectively. In an aspect, the first gRNA molecule and the second gRNA molecule include targeting domains comprising, e.g., consisting of, SEQ ID NO: 98 and SEQ ID NO: 1589, respectively. In an aspect, the first gRNA molecule and the second gRNA molecule include targeting domains comprising, e.g., consisting of, SEQ ID NO: 98 and SEQ ID NO: 160, respectively. In an aspect, the first gRNA molecule and the second gRNA molecule include targeting domains comprising, e.g., consisting of, SEQ ID NO: 98 and SEQ ID NO: 1537, respectively. In an aspect, the first gRNA molecule and the second gRNA molecule include targeting domains comprising, e.g., consisting of, SEQ ID NO: 98 and SEQ ID NO: 159, respectively. In an aspect, the first gRNA molecule and the second gRNA molecule include targeting domains comprising, e.g., consisting of, SEQ ID NO: 98 and SEQ ID NO: 101, respectively. In an aspect, the first gRNA molecule and the second gRNA molecule include targeting domains comprising, e.g., consisting of, SEQ ID NO: 98 and SEQ ID NO: 162, respectively. In an aspect, the first gRNA molecule and the second gRNA molecule include targeting domains comprising, e.g., consisting of, SEQ ID NO: 98 and SEQ ID NO: 104, respectively. In an aspect, the first gRNA molecule and the second gRNA molecule include targeting domains comprising, e.g., consisting of, SEQ ID NO: 98 and SEQ ID NO: 138, respectively. In an aspect, the first gRNA molecule and the second gRNA molecule include targeting domains comprising, e.g., consisting of, SEQ ID NO: 98 and SEQ ID NO: 1536, respectively. In an aspect, the first gRNA molecule and the second gRNA molecule include targeting domains comprising, e.g., consisting of, SEQ ID NO: 98 and SEQ ID NO: 1539, respectively. In an aspect, the first gRNA molecule and the second gRNA molecule include targeting domains comprising, e.g., consisting of, SEQ ID NO: 98 and SEQ ID NO: 1585, respectively. In an aspect, the first gRNA molecule and the second gRNA molecule include targeting domains comprising, e.g., consisting of, SEQ ID NO: 1580 and SEQ ID NO: 165, respectively. In an aspect, the first gRNA molecule and the second gRNA molecule include targeting domains comprising, e.g., consisting of, SEQ ID NO: 1580 and SEQ ID NO: 113, respectively. In an aspect, the first gRNA molecule and the second gRNA molecule include targeting domains comprising, e.g., consisting of, SEQ ID NO: 1580 and SEQ ID NO: 99, respectively. In an aspect, the first gRNA molecule and the second gRNA molecule include targeting domains comprising, e.g., consisting of, SEQ ID NO: 1580 and SEQ ID NO: 112, respectively. In an aspect, the first gRNA molecule and the second gRNA molecule include targeting domains comprising, e.g., consisting of, SEQ ID NO: 1580 and SEQ ID NO: 98, respectively. In an aspect, the first gRNA molecule and the second gRNA molecule include targeting domains comprising, e.g., consisting of, SEQ ID NO: 1580 and SEQ ID NO: 106, respectively. In an aspect, the first gRNA molecule and the second gRNA molecule include targeting domains comprising, e.g., consisting of, SEQ ID NO: 1580 and SEQ ID NO: 1503, respectively. In an aspect, the first gRNA molecule and the second gRNA molecule include targeting domains comprising, e.g., consisting of, SEQ ID NO: 1580 and SEQ ID NO: 1589, respectively. In an aspect, the first gRNA molecule and the second gRNA molecule include targeting domains comprising, e.g., consisting of, SEQ ID NO: 1580 and SEQ ID NO: 160, respectively. In an aspect, the first gRNA molecule and the second gRNA molecule include targeting domains comprising, e.g., consisting of, SEQ ID NO: 1580 and SEQ ID NO: 1537, respectively. In an aspect, the first gRNA molecule and the second gRNA molecule include targeting domains comprising, e.g., consisting of, SEQ ID NO: 1580 and SEQ ID NO: 159, respectively. In an aspect, the first gRNA molecule and the second gRNA molecule include targeting domains comprising, e.g., consisting of, SEQ ID NO: 1580 and SEQ ID NO: 101, respectively. In an aspect, the first gRNA molecule and the second gRNA molecule include targeting domains comprising, e.g., consisting of, SEQ ID NO: 1580 and SEQ ID NO: 162, respectively. In an aspect, the first gRNA molecule and the second gRNA molecule include targeting domains comprising, e.g., consisting of, SEQ ID NO: 1580 and SEQ ID NO: 104, respectively. In an aspect, the first gRNA molecule and the second gRNA molecule include targeting domains comprising, e.g., consisting of, SEQ ID NO: 1580 and SEQ ID NO: 138, respectively. In an aspect, the first gRNA molecule and the second gRNA molecule include targeting domains comprising, e.g., consisting of, SEQ ID NO: 1580 and SEQ ID NO: 1536, respectively. In an aspect, the first gRNA molecule and the second gRNA molecule include targeting domains comprising, e.g., consisting of, SEQ ID NO: 1580 and SEQ ID NO: 1539, respectively. In an aspect, the first gRNA molecule and the second gRNA molecule include targeting domains comprising, e.g., consisting of, SEQ ID NO: 1580 and SEQ ID NO: 1585, respectively. In an aspect, the first gRNA molecule and the second gRNA molecule include targeting domains comprising, e.g., consisting of, SEQ ID NO: 106 and SEQ ID NO: 165, respectively. In an aspect, the first gRNA molecule and the second gRNA molecule include targeting domains comprising, e.g., consisting of, SEQ ID NO: 106 and SEQ ID NO: 113, respectively. In an aspect, the first gRNA molecule and the second gRNA molecule include targeting domains comprising, e.g., consisting of, SEQ ID NO: 106 and SEQ ID NO: 99, respectively. In an aspect, the first gRNA molecule and the second gRNA molecule include targeting domains comprising, e.g., consisting of, SEQ ID NO: 106 and SEQ ID NO: 112, respectively. In an aspect, the first gRNA molecule and the second gRNA molecule include targeting domains comprising, e.g., consisting of, SEQ ID NO: 106 and SEQ ID NO: 98, respectively. In an aspect, the first gRNA molecule and the second gRNA molecule include targeting domains comprising, e.g., consisting of, SEQ ID NO: 106 and SEQ ID NO: 1580, respectively. In an aspect, the first gRNA molecule and the second gRNA molecule include targeting domains comprising, e.g., consisting of, SEQ ID NO: 106 and SEQ ID NO: 1503, respectively. In an aspect, the first gRNA molecule and the second gRNA molecule include targeting domains comprising, e.g., consisting of, SEQ ID NO: 106 and SEQ ID NO: 1589, respectively. In an aspect, the first gRNA molecule and the second gRNA molecule include targeting domains comprising, e.g., consisting of, SEQ ID NO: 106 and SEQ ID NO: 160, respectively. In an aspect, the first gRNA molecule and the second gRNA molecule include targeting domains comprising, e.g., consisting of, SEQ ID NO: 106 and SEQ ID NO: 1537, respectively. In an aspect, the first gRNA molecule and the second gRNA molecule include targeting domains comprising, e.g., consisting of, SEQ ID NO: 106 and SEQ ID NO: 159, respectively. In an aspect, the first gRNA molecule and the second gRNA molecule include targeting domains comprising, e.g., consisting of, SEQ ID NO: 106 and SEQ ID NO: 101, respectively. In an aspect, the first gRNA molecule and the second gRNA molecule include targeting domains comprising, e.g., consisting of, SEQ ID NO: 106 and SEQ ID NO: 162, respectively. In an aspect, the first gRNA molecule and the second gRNA molecule include targeting domains comprising, e.g., consisting of, SEQ ID NO: 106 and SEQ ID NO: 104, respectively. In an aspect, the first gRNA molecule and the second gRNA molecule include targeting domains comprising, e.g., consisting of, SEQ ID NO: 106 and SEQ ID NO: 138, respectively. In an aspect, the first gRNA molecule and the second gRNA molecule include targeting domains comprising, e.g., consisting of, SEQ ID NO: 106 and SEQ ID NO: 1536, respectively. In an aspect, the first gRNA molecule and the second gRNA molecule include targeting domains comprising, e.g., consisting of, SEQ ID NO: 106 and SEQ ID NO: 1539, respectively. In an aspect, the first gRNA molecule and the second gRNA molecule include targeting domains comprising, e.g., consisting of, SEQ ID NO: 106 and SEQ ID NO: 1585, respectively. In an aspect, the first gRNA molecule and the second gRNA molecule include targeting domains comprising, e.g., consisting of, SEQ ID NO: 1503 and SEQ ID NO: 165, respectively. In an aspect, the first gRNA molecule and the second gRNA molecule include targeting domains comprising, e.g., consisting of, SEQ ID NO: 1503 and SEQ ID NO: 113, respectively. In an aspect, the first gRNA molecule and the second gRNA molecule include targeting domains comprising, e.g., consisting of, SEQ ID NO: 1503 and SEQ ID NO: 99, respectively. In an aspect, the first gRNA molecule and the second gRNA molecule include targeting domains comprising, e.g., consisting of, SEQ ID NO: 1503 and SEQ ID NO: 112, respectively. In an aspect, the first gRNA molecule and the second gRNA molecule include targeting domains comprising, e.g., consisting of, SEQ ID NO: 1503 and SEQ ID NO: 98, respectively. In an aspect, the first gRNA molecule and the second gRNA molecule include targeting domains comprising, e.g., consisting of, SEQ ID NO: 1503 and SEQ ID NO: 1580, respectively. In an aspect, the first gRNA molecule and the second gRNA molecule include targeting domains comprising, e.g., consisting of, SEQ ID NO: 1503 and SEQ ID NO: 106, respectively. In an aspect, the first gRNA molecule and the second gRNA molecule include targeting domains comprising, e.g., consisting of, SEQ ID NO: 1503 and SEQ ID NO: 1589, respectively. In an aspect, the first gRNA molecule and the second gRNA molecule include targeting domains comprising, e.g., consisting of, SEQ ID NO: 1503 and SEQ ID NO: 160, respectively. In an aspect, the first gRNA molecule and the second gRNA molecule include targeting domains comprising, e.g., consisting of, SEQ ID NO: 1503 and SEQ ID NO: 1537, respectively. In an aspect, the first gRNA molecule and the second gRNA molecule include targeting domains comprising, e.g., consisting of, SEQ ID NO: 1503 and SEQ ID NO: 159, respectively. In an aspect, the first gRNA molecule and the second gRNA molecule include targeting domains comprising, e.g., consisting of, SEQ ID NO: 1503 and SEQ ID NO: 101, respectively. In an aspect, the first gRNA molecule and the second gRNA molecule include targeting domains comprising, e.g., consisting of, SEQ ID NO: 1503 and SEQ ID NO: 162, respectively. In an aspect, the first gRNA molecule and the second gRNA molecule include targeting domains comprising, e.g., consisting of, SEQ ID NO: 1503 and SEQ ID NO: 104, respectively. In an aspect, the first gRNA molecule and the second gRNA molecule include targeting domains comprising, e.g., consisting of, SEQ ID NO: 1503 and SEQ ID NO: 138, respectively. In an aspect, the first gRNA molecule and the second gRNA molecule include targeting domains comprising, e.g., consisting of, SEQ ID NO: 1503 and SEQ ID NO: 1536, respectively. In an aspect, the first gRNA molecule and the second gRNA molecule include targeting domains comprising, e.g., consisting of, SEQ ID NO: 1503 and SEQ ID NO: 1539, respectively. In an aspect, the first gRNA molecule and the second gRNA molecule include targeting domains comprising, e.g., consisting of, SEQ ID NO: 1503 and SEQ ID NO: 1585, respectively. In an aspect, the first gRNA molecule and the second gRNA molecule include targeting domains comprising, e.g., consisting of, SEQ ID NO: 1589 and SEQ ID NO: 165, respectively. In an aspect, the first gRNA molecule and the second gRNA molecule include targeting domains comprising, e.g., consisting of, SEQ ID NO: 1589 and SEQ ID NO: 113, respectively. In an aspect, the first gRNA molecule and the second gRNA molecule include targeting domains comprising, e.g., consisting of, SEQ ID NO: 1589 and SEQ ID NO: 99, respectively. In an aspect, the first gRNA molecule and the second gRNA molecule include targeting domains comprising, e.g., consisting of, SEQ ID NO: 1589 and SEQ ID NO: 112, respectively. In an aspect, the first gRNA molecule and the second gRNA molecule include targeting domains comprising, e.g., consisting of, SEQ ID NO: 1589 and SEQ ID NO: 98, respectively. In an aspect, the first gRNA molecule and the second gRNA molecule include targeting domains comprising, e.g., consisting of, SEQ ID NO: 1589 and SEQ ID NO: 1580, respectively. In an aspect, the first gRNA molecule and the second gRNA molecule include targeting domains comprising, e.g., consisting of, SEQ ID NO: 1589 and SEQ ID NO: 106, respectively. In an aspect, the first gRNA molecule and the second gRNA molecule include targeting domains comprising, e.g., consisting of, SEQ ID NO: 1589 and SEQ ID NO: 1503, respectively. In an aspect, the first gRNA molecule and the second gRNA molecule include targeting domains comprising, e.g., consisting of, SEQ ID NO: 1589 and SEQ ID NO: 160, respectively. In an aspect, the first gRNA molecule and the second gRNA molecule include targeting domains comprising, e.g., consisting of, SEQ ID NO: 1589 and SEQ ID NO: 1537, respectively. In an aspect, the first gRNA molecule and the second gRNA molecule include targeting domains comprising, e.g., consisting of, SEQ ID NO: 1589 and SEQ ID NO: 159, respectively. In an aspect, the first gRNA molecule and the second gRNA molecule include targeting domains comprising, e.g., consisting of, SEQ ID NO: 1589 and SEQ ID NO: 101, respectively. In an aspect, the first gRNA molecule and the second gRNA molecule include targeting domains comprising, e.g., consisting of, SEQ ID NO: 1589 and SEQ ID NO: 162, respectively. In an aspect, the first gRNA molecule and the second gRNA molecule include targeting domains comprising, e.g., consisting of, SEQ ID NO: 1589 and SEQ ID NO: 104, respectively. In an aspect, the first gRNA molecule and the second gRNA molecule include targeting domains comprising, e.g., consisting of, SEQ ID NO: 1589 and SEQ ID NO: 138, respectively. In an aspect, the first gRNA molecule and the second gRNA molecule include targeting domains comprising, e.g., consisting of, SEQ ID NO: 1589 and SEQ ID NO: 1536, respectively. In an aspect, the first gRNA molecule and the second gRNA molecule include targeting domains comprising, e.g., consisting of, SEQ ID NO: 1589 and SEQ ID NO: 1539, respectively. In an aspect, the first gRNA molecule and the second gRNA molecule include targeting domains comprising, e.g., consisting of, SEQ ID NO: 1589 and SEQ ID NO: 1585, respectively. In an aspect, the first gRNA molecule and the second gRNA molecule include targeting domains comprising, e.g., consisting of, SEQ ID NO: 160 and SEQ ID NO: 165, respectively. In an aspect, the first gRNA molecule and the second gRNA molecule include targeting domains comprising, e.g., consisting of, SEQ ID NO: 160 and SEQ ID NO: 113, respectively. In an aspect, the first gRNA molecule and the second gRNA molecule include targeting domains comprising, e.g., consisting of, SEQ ID NO: 160 and SEQ ID NO: 99, respectively. In an aspect, the first gRNA molecule and the second gRNA molecule include targeting domains comprising, e.g., consisting of, SEQ ID NO: 160 and SEQ ID NO: 112, respectively. In an aspect, the first gRNA molecule and the second gRNA molecule include targeting domains comprising, e.g., consisting of, SEQ ID NO: 160 and SEQ ID NO: 98, respectively. In an aspect, the first gRNA molecule and the second gRNA molecule include targeting domains comprising, e.g., consisting of, SEQ ID NO: 160 and SEQ ID NO: 1580, respectively. In an aspect, the first gRNA molecule and the second gRNA molecule include targeting domains comprising, e.g., consisting of, SEQ ID NO: 160 and SEQ ID NO: 106, respectively. In an aspect, the first gRNA molecule and the second gRNA molecule include targeting domains comprising, e.g., consisting of, SEQ ID NO: 160 and SEQ ID NO: 1503, respectively. In an aspect, the first gRNA molecule and the second gRNA molecule include targeting domains comprising, e.g., consisting of, SEQ ID NO: 160 and SEQ ID NO: 1589, respectively. In an aspect, the first gRNA molecule and the second gRNA molecule include targeting domains comprising, e.g., consisting of, SEQ ID NO: 160 and SEQ ID NO: 1537, respectively. In an aspect, the first gRNA molecule and the second gRNA molecule include targeting domains comprising, e.g., consisting of, SEQ ID NO: 160 and SEQ ID NO: 159, respectively. In an aspect, the first gRNA molecule and the second gRNA molecule include targeting domains comprising, e.g., consisting of, SEQ ID NO: 160 and SEQ ID NO: 101, respectively. In an aspect, the first gRNA molecule and the second gRNA molecule include targeting domains comprising, e.g., consisting of, SEQ ID NO: 160 and SEQ ID NO: 162, respectively. In an aspect, the first gRNA molecule and the second gRNA molecule include targeting domains comprising, e.g., consisting of, SEQ ID NO: 160 and SEQ ID NO: 104, respectively. In an aspect, the first gRNA molecule and the second gRNA molecule include targeting domains comprising, e.g., consisting of, SEQ ID NO: 160 and SEQ ID NO: 138, respectively. In an aspect, the first gRNA molecule and the second gRNA molecule include targeting domains comprising, e.g., consisting of, SEQ ID NO: 160 and SEQ ID NO: 1536, respectively. In an aspect, the first gRNA molecule and the second gRNA molecule include targeting domains comprising, e.g., consisting of, SEQ ID NO: 160 and SEQ ID NO: 1539, respectively. In an aspect, the first gRNA molecule and the second gRNA molecule include targeting domains comprising, e.g., consisting of, SEQ ID NO: 160 and SEQ ID NO: 1585, respectively. In an aspect, the first gRNA molecule and the second gRNA molecule include targeting domains comprising, e.g., consisting of, SEQ ID NO: 1537 and SEQ ID NO: 165, respectively. In an aspect, the first gRNA molecule and the second gRNA molecule include targeting domains comprising, e.g., consisting of, SEQ ID NO: 1537 and SEQ ID NO: 113, respectively. In an aspect, the first gRNA molecule and the second gRNA molecule include targeting domains comprising, e.g., consisting of, SEQ ID NO: 1537 and SEQ ID NO: 99, respectively. In an aspect, the first gRNA molecule and the second gRNA molecule include targeting domains comprising, e.g., consisting of, SEQ ID NO: 1537 and SEQ ID NO: 112, respectively. In an aspect, the first gRNA molecule and the second gRNA molecule include targeting domains comprising, e.g., consisting of, SEQ ID NO: 1537 and SEQ ID NO: 98, respectively. In an aspect, the first gRNA molecule and the second gRNA molecule include targeting domains comprising, e.g., consisting of, SEQ ID NO: 1537 and SEQ ID NO: 1580, respectively. In an aspect, the first gRNA molecule and the second gRNA molecule include targeting domains comprising, e.g., consisting of, SEQ ID NO: 1537 and SEQ ID NO: 106, respectively. In an aspect, the first gRNA molecule and the second gRNA molecule include targeting domains comprising, e.g., consisting of, SEQ ID NO: 1537 and SEQ ID NO: 1503, respectively. In an aspect, the first gRNA molecule and the second gRNA molecule include targeting domains comprising, e.g., consisting of, SEQ ID NO: 1537 and SEQ ID NO: 1589, respectively. In an aspect, the first gRNA molecule and the second gRNA molecule include targeting domains comprising, e.g., consisting of, SEQ ID NO: 1537 and SEQ ID NO: 160, respectively. In an aspect, the first gRNA molecule and the second gRNA molecule include targeting domains comprising, e.g., consisting of, SEQ ID NO: 1537 and SEQ ID NO: 159, respectively. In an aspect, the first gRNA molecule and the second gRNA molecule include targeting domains comprising, e.g., consisting of, SEQ ID NO: 1537 and SEQ ID NO: 101, respectively. In an aspect, the first gRNA molecule and the second gRNA molecule include targeting domains comprising, e.g., consisting of, SEQ ID NO: 1537 and SEQ ID NO: 162, respectively. In an aspect, the first gRNA molecule and the second gRNA molecule include targeting domains comprising, e.g., consisting of, SEQ ID NO: 1537 and SEQ ID NO: 104, respectively. In an aspect, the first gRNA molecule and the second gRNA molecule include targeting domains comprising, e.g., consisting of, SEQ ID NO: 1537 and SEQ ID NO: 138, respectively. In an aspect, the first gRNA molecule and the second gRNA molecule include targeting domains comprising, e.g., consisting of, SEQ ID NO: 1537 and SEQ ID NO: 1536, respectively. In an aspect, the first gRNA molecule and the second gRNA molecule include targeting domains comprising, e.g., consisting of, SEQ ID NO: 1537 and SEQ ID NO: 1539, respectively. In an aspect, the first gRNA molecule and the second gRNA molecule include targeting domains comprising, e.g., consisting of, SEQ ID NO: 1537 and SEQ ID NO: 1585, respectively. In an aspect, the first gRNA molecule and the second gRNA molecule include targeting domains comprising, e.g., consisting of, SEQ ID NO: 159 and SEQ ID NO: 165, respectively. In an aspect, the first gRNA molecule and the second gRNA molecule include targeting domains comprising, e.g., consisting of, SEQ ID NO: 159 and SEQ ID NO: 113, respectively. In an aspect, the first gRNA molecule and the second gRNA molecule include targeting domains comprising, e.g., consisting of, SEQ ID NO: 159 and SEQ ID NO: 99, respectively. In an aspect, the first gRNA molecule and the second gRNA molecule include targeting domains comprising, e.g., consisting of, SEQ ID NO: 159 and SEQ ID NO: 112, respectively. In an aspect, the first gRNA molecule and the second gRNA molecule include targeting domains comprising, e.g., consisting of, SEQ ID NO: 159 and SEQ ID NO: 98, respectively. In an aspect, the first gRNA molecule and the second gRNA molecule include targeting domains comprising, e.g., consisting of, SEQ ID NO: 159 and SEQ ID NO: 1580, respectively. In an aspect, the first gRNA molecule and the second gRNA molecule include targeting domains comprising, e.g., consisting of, SEQ ID NO: 159 and SEQ ID NO: 106, respectively. In an aspect, the first gRNA molecule and the second gRNA molecule include targeting domains comprising, e.g., consisting of, SEQ ID NO: 159 and SEQ ID NO: 1503, respectively. In an aspect, the first gRNA molecule and the second gRNA molecule include targeting domains comprising, e.g., consisting of, SEQ ID NO: 159 and SEQ ID NO: 1589, respectively. In an aspect, the first gRNA molecule and the second gRNA molecule include targeting domains comprising, e.g., consisting of, SEQ ID NO: 159 and SEQ ID NO: 160, respectively. In an aspect, the first gRNA molecule and the second gRNA molecule include targeting domains comprising, e.g., consisting of, SEQ ID NO: 159 and SEQ ID NO: 1537, respectively. In an aspect, the first gRNA molecule and the second gRNA molecule include targeting domains comprising, e.g., consisting of, SEQ ID NO: 159 and SEQ ID NO: 101, respectively. In an aspect, the first gRNA molecule and the second gRNA molecule include targeting domains comprising, e.g., consisting of, SEQ ID NO: 159 and SEQ ID NO: 162, respectively. In an aspect, the first gRNA molecule and the second gRNA molecule include targeting domains comprising, e.g., consisting of, SEQ ID NO: 159 and SEQ ID NO: 104, respectively. In an aspect, the first gRNA molecule and the second gRNA molecule include targeting domains comprising, e.g., consisting of, SEQ ID NO: 159 and SEQ ID NO: 138, respectively. In an aspect, the first gRNA molecule and the second gRNA molecule include targeting domains comprising, e.g., consisting of, SEQ ID NO: 159 and SEQ ID NO: 1536, respectively. In an aspect, the first gRNA molecule and the second gRNA molecule include targeting domains comprising, e.g., consisting of, SEQ ID NO: 159 and SEQ ID NO: 1539, respectively. In an aspect, the first gRNA molecule and the second gRNA molecule include targeting domains comprising, e.g., consisting of, SEQ ID NO: 159 and SEQ ID NO: 1585, respectively. In an aspect, the first gRNA molecule and the second gRNA molecule include targeting domains comprising, e.g., consisting of, SEQ ID NO: 101 and SEQ ID NO: 165, respectively. In an aspect, the first gRNA molecule and the second gRNA molecule include targeting domains comprising, e.g., consisting of, SEQ ID NO: 101 and SEQ ID NO: 113, respectively. In an aspect, the first gRNA molecule and the second gRNA molecule include targeting domains comprising, e.g., consisting of, SEQ ID NO: 101 and SEQ ID NO: 99, respectively. In an aspect, the first gRNA molecule and the second gRNA molecule include targeting domains comprising, e.g., consisting of, SEQ ID NO: 101 and SEQ ID NO: 112, respectively. In an aspect, the first gRNA molecule and the second gRNA molecule include targeting domains comprising, e.g., consisting of, SEQ ID NO: 101 and SEQ ID NO: 98, respectively. In an aspect, the first gRNA molecule and the second gRNA molecule include targeting domains comprising, e.g., consisting of, SEQ ID NO: 101 and SEQ ID NO: 1580, respectively. In an aspect, the first gRNA molecule and the second gRNA molecule include targeting domains comprising, e.g., consisting of, SEQ ID NO: 101 and SEQ ID NO: 106, respectively. In an aspect, the first gRNA molecule and the second gRNA molecule include targeting domains comprising, e.g., consisting of, SEQ ID NO: 101 and SEQ ID NO: 1503, respectively. In an aspect, the first gRNA molecule and the second gRNA molecule include targeting domains comprising, e.g., consisting of, SEQ ID NO: 101 and SEQ ID NO: 1589, respectively. In an aspect, the first gRNA molecule and the second gRNA molecule include targeting domains comprising, e.g., consisting of, SEQ ID NO: 101 and SEQ ID NO: 160, respectively. In an aspect, the first gRNA molecule and the second gRNA molecule include targeting domains comprising, e.g., consisting of, SEQ ID NO: 101 and SEQ ID NO: 1537, respectively. In an aspect, the first gRNA molecule and the second gRNA molecule include targeting domains comprising, e.g., consisting of, SEQ ID NO: 101 and SEQ ID NO: 159, respectively. In an aspect, the first gRNA molecule and the second gRNA molecule include targeting domains comprising, e.g., consisting of, SEQ ID NO: 101 and SEQ ID NO: 162, respectively. In an aspect, the first gRNA molecule and the second gRNA molecule include targeting domains comprising, e.g., consisting of, SEQ ID NO: 101 and SEQ ID NO: 104, respectively. In an aspect, the first gRNA molecule and the second gRNA molecule include targeting domains comprising, e.g., consisting of, SEQ ID NO: 101 and SEQ ID NO: 138, respectively. In an aspect, the first gRNA molecule and the second gRNA molecule include targeting domains comprising, e.g., consisting of, SEQ ID NO: 101 and SEQ ID NO: 1536, respectively. In an aspect, the first gRNA molecule and the second gRNA molecule include targeting domains comprising, e.g., consisting of, SEQ ID NO: 101 and SEQ ID NO: 1539, respectively. In an aspect, the first gRNA molecule and the second gRNA molecule include targeting domains comprising, e.g., consisting of, SEQ ID NO: 101 and SEQ ID NO: 1585, respectively. In an aspect, the first gRNA molecule and the second gRNA molecule include targeting domains comprising, e.g., consisting of, SEQ ID NO: 162 and SEQ ID NO: 165, respectively. In an aspect, the first gRNA molecule and the second gRNA molecule include targeting domains comprising, e.g., consisting of, SEQ ID NO: 162 and SEQ ID NO: 113, respectively. In an aspect, the first gRNA molecule and the second gRNA molecule include targeting domains comprising, e.g., consisting of, SEQ ID NO: 162 and SEQ ID NO: 99, respectively. In an aspect, the first gRNA molecule and the second gRNA molecule include targeting domains comprising, e.g., consisting of, SEQ ID NO: 162 and SEQ ID NO: 112, respectively. In an aspect, the first gRNA molecule and the second gRNA molecule include targeting domains comprising, e.g., consisting of, SEQ ID NO: 162 and SEQ ID NO: 98, respectively. In an aspect, the first gRNA molecule and the second gRNA molecule include targeting domains comprising, e.g., consisting of, SEQ ID NO: 162 and SEQ ID NO: 1580, respectively. In an aspect, the first gRNA molecule and the second gRNA molecule include targeting domains comprising, e.g., consisting of, SEQ ID NO: 162 and SEQ ID NO: 106, respectively. In an aspect, the first gRNA molecule and the second gRNA molecule include targeting domains comprising, e.g., consisting of, SEQ ID NO: 162 and SEQ ID NO: 1503, respectively. In an aspect, the first gRNA molecule and the second gRNA molecule include targeting domains comprising, e.g., consisting of, SEQ ID NO: 162 and SEQ ID NO: 1589, respectively. In an aspect, the first gRNA molecule and the second gRNA molecule include targeting domains comprising, e.g., consisting of, SEQ ID NO: 162 and SEQ ID NO: 160, respectively. In an aspect, the first gRNA molecule and the second gRNA molecule include targeting domains comprising, e.g., consisting of, SEQ ID NO: 162 and SEQ ID NO: 1537, respectively. In an aspect, the first gRNA molecule and the second gRNA molecule include targeting domains comprising, e.g., consisting of, SEQ ID NO: 162 and SEQ ID NO: 159, respectively. In an aspect, the first gRNA molecule and the second gRNA molecule include targeting domains comprising, e.g., consisting of, SEQ ID NO: 162 and SEQ ID NO: 101, respectively. In an aspect, the first gRNA molecule and the second gRNA molecule include targeting domains comprising, e.g., consisting of, SEQ ID NO: 104 and SEQ ID NO: 165, respectively. In an aspect, the first gRNA molecule and the second gRNA molecule include targeting domains comprising, e.g., consisting of, SEQ ID NO: 104 and SEQ ID NO: 113, respectively. In an aspect, the first gRNA molecule and the second gRNA molecule include targeting domains comprising, e.g., consisting of, SEQ ID NO: 104 and SEQ ID NO: 99, respectively. In an aspect, the first gRNA molecule and the second gRNA molecule include targeting domains comprising, e.g., consisting of, SEQ ID NO: 104 and SEQ ID NO: 112, respectively. In an aspect, the first gRNA molecule and the second gRNA molecule include targeting domains comprising, e.g., consisting of, SEQ ID NO: 104 and SEQ ID NO: 98, respectively. In an aspect, the first gRNA molecule and the second gRNA molecule include targeting domains comprising, e.g., consisting of, SEQ ID NO: 104 and SEQ ID NO: 1580, respectively. In an aspect, the first gRNA molecule and the second gRNA molecule include targeting domains comprising, e.g., consisting of, SEQ ID NO: 104 and SEQ ID NO: 106, respectively. In an aspect, the first gRNA molecule and the second gRNA molecule include targeting domains comprising, e.g., consisting of, SEQ ID NO: 104 and SEQ ID NO: 1503, respectively. In an aspect, the first gRNA molecule and the second gRNA molecule include targeting domains comprising, e.g., consisting of, SEQ ID NO: 104 and SEQ ID NO: 1589, respectively. In an aspect, the first gRNA molecule and the second gRNA molecule include targeting domains comprising, e.g., consisting of, SEQ ID NO: 104 and SEQ ID NO: 160, respectively. In an aspect, the first gRNA molecule and the second gRNA molecule include targeting domains comprising, e.g., consisting of, SEQ ID NO: 104 and SEQ ID NO: 1537, respectively. In an aspect, the first gRNA molecule and the second gRNA molecule include targeting domains comprising, e.g., consisting of, SEQ ID NO: 104 and SEQ ID NO: 159, respectively. In an aspect, the first gRNA molecule and the second gRNA molecule include targeting domains comprising, e.g., consisting of, SEQ ID NO: 104 and SEQ ID NO: 101, respectively. In an aspect, the first gRNA molecule and the second gRNA molecule include targeting domains comprising, e.g., consisting of, SEQ ID NO: 104 and SEQ ID NO: 162, respectively. In an aspect, the first gRNA molecule and the second gRNA molecule include targeting domains comprising, e.g., consisting of, SEQ ID NO: 104 and SEQ ID NO: 138, respectively. In an aspect, the first gRNA molecule and the second gRNA molecule include targeting domains comprising, e.g., consisting of, SEQ ID NO: 104 and SEQ ID NO: 1536, respectively. In an aspect, the first gRNA molecule and the second gRNA molecule include targeting domains comprising, e.g., consisting of, SEQ ID NO: 104 and SEQ ID NO: 1539, respectively. In an aspect, the first gRNA molecule and the second gRNA molecule include targeting domains comprising, e.g., consisting of, SEQ ID NO: 104 and SEQ ID NO: 1585, respectively. In an aspect, the first gRNA molecule and the second gRNA molecule include targeting domains comprising, e.g., consisting of, SEQ ID NO: 138 and SEQ ID NO: 165, respectively. In an aspect, the first gRNA molecule and the second gRNA molecule include targeting domains comprising, e.g., consisting of, SEQ ID NO: 138 and SEQ ID NO: 113, respectively. In an aspect, the first gRNA molecule and the second gRNA molecule include targeting domains comprising, e.g., consisting of, SEQ ID NO: 138 and SEQ ID NO: 99, respectively. In an aspect, the first gRNA molecule and the second gRNA molecule include targeting domains comprising, e.g., consisting of, SEQ ID NO: 138 and SEQ ID NO: 112, respectively. In an aspect, the first gRNA molecule and the second gRNA molecule include targeting domains comprising, e.g., consisting of, SEQ ID NO: 138 and SEQ ID NO: 98, respectively. In an aspect, the first gRNA molecule and the second gRNA molecule include targeting domains comprising, e.g., consisting of, SEQ ID NO: 138 and SEQ ID NO: 1580, respectively. In an aspect, the first gRNA molecule and the second gRNA molecule include targeting domains comprising, e.g., consisting of, SEQ ID NO: 138 and SEQ ID NO: 106, respectively. In an aspect, the first gRNA molecule and the second gRNA molecule include targeting domains comprising, e.g., consisting of, SEQ ID NO: 138 and SEQ ID NO: 1503, respectively. In an aspect, the first gRNA molecule and the second gRNA molecule include targeting domains comprising, e.g., consisting of, SEQ ID NO: 138 and SEQ ID NO: 1589, respectively. In an aspect, the first gRNA molecule and the second gRNA molecule include targeting domains comprising, e.g., consisting of, SEQ ID NO: 138 and SEQ ID NO: 160, respectively. In an aspect, the first gRNA molecule and the second gRNA molecule include targeting domains comprising, e.g., consisting of, SEQ ID NO: 138 and SEQ ID NO: 1537, respectively. In an aspect, the first gRNA molecule and the second gRNA molecule include targeting domains comprising, e.g., consisting of, SEQ ID NO: 138 and SEQ ID NO: 159, respectively. In an aspect, the first gRNA molecule and the second gRNA molecule include targeting domains comprising, e.g., consisting of, SEQ ID NO: 138 and SEQ ID NO: 101, respectively. In an aspect, the first gRNA molecule and the second gRNA molecule include targeting domains comprising, e.g., consisting of, SEQ ID NO: 138 and SEQ ID NO: 162, respectively. In an aspect, the first gRNA molecule and the second gRNA molecule include targeting domains comprising, e.g., consisting of, SEQ ID NO: 138 and SEQ ID NO: 104, respectively. In an aspect, the first gRNA molecule and the second gRNA molecule include targeting domains comprising, e.g., consisting of, SEQ ID NO: 138 and SEQ ID NO: 1536, respectively. In an aspect, the first gRNA molecule and the second gRNA molecule include targeting domains comprising, e.g., consisting of, SEQ ID NO: 138 and SEQ ID NO: 1539, respectively. In an aspect, the first gRNA molecule and the second gRNA molecule include targeting domains comprising, e.g., consisting of, SEQ ID NO: 138 and SEQ ID NO: 1585, respectively. In an aspect, the first gRNA molecule and the second gRNA molecule include targeting domains comprising, e.g., consisting of, SEQ ID NO: 1536 and SEQ ID NO: 165, respectively. In an aspect, the first gRNA molecule and the second gRNA molecule include targeting domains comprising, e.g., consisting of, SEQ ID NO: 1536 and SEQ ID NO: 113, respectively. In an aspect, the first gRNA molecule and the second gRNA molecule include targeting domains comprising, e.g., consisting of, SEQ ID NO: 1536 and SEQ ID NO: 99, respectively. In an aspect, the first gRNA molecule and the second gRNA molecule include targeting domains comprising, e.g., consisting of, SEQ ID NO: 1536 and SEQ ID NO: 112, respectively. In an aspect, the first gRNA molecule and the second gRNA molecule include targeting domains comprising, e.g., consisting of, SEQ ID NO: 1536 and SEQ ID NO: 98, respectively. In an aspect, the first gRNA molecule and the second gRNA molecule include targeting domains comprising, e.g., consisting of, SEQ ID NO: 1536 and SEQ ID NO: 1580, respectively. In an aspect, the first gRNA molecule and the second gRNA molecule include targeting domains comprising, e.g., consisting of, SEQ ID NO: 1536 and SEQ ID NO: 106, respectively. In an aspect, the first gRNA molecule and the second gRNA molecule include targeting domains comprising, e.g., consisting of, SEQ ID NO: 1536 and SEQ ID NO: 1503, respectively. In an aspect, the first gRNA molecule and the second gRNA molecule include targeting domains comprising, e.g., consisting of, SEQ ID NO: 1536 and SEQ ID NO: 1589, respectively. In an aspect, the first gRNA molecule and the second gRNA molecule include targeting domains comprising, e.g., consisting of, SEQ ID NO: 1536 and SEQ ID NO: 160, respectively. In an aspect, the first gRNA molecule and the second gRNA molecule include targeting domains comprising, e.g., consisting of, SEQ ID NO: 1536 and SEQ ID NO: 1537, respectively. In an aspect, the first gRNA molecule and the second gRNA molecule include targeting domains comprising, e.g., consisting of, SEQ ID NO: 1536 and SEQ ID NO: 159, respectively. In an aspect, the first gRNA molecule and the second gRNA molecule include targeting domains comprising, e.g., consisting of, SEQ ID NO: 1536 and SEQ ID NO: 101, respectively. In an aspect, the first gRNA molecule and the second gRNA molecule include targeting domains comprising, e.g., consisting of, SEQ ID NO: 1536 and SEQ ID NO: 162, respectively. In an aspect, the first gRNA molecule and the second gRNA molecule include targeting domains comprising, e.g., consisting of, SEQ ID NO: 1536 and SEQ ID NO: 104, respectively. In an aspect, the first gRNA molecule and the second gRNA molecule include targeting domains comprising, e.g., consisting of, SEQ ID NO: 1536 and SEQ ID NO: 138, respectively. In an aspect, the first gRNA molecule and the second gRNA molecule include targeting domains comprising, e.g., consisting of, SEQ ID NO: 1536 and SEQ ID NO: 1539, respectively. In an aspect, the first gRNA molecule and the second gRNA molecule include targeting domains comprising, e.g., consisting of, SEQ ID NO: 1536 and SEQ ID NO: 1585, respectively. In an aspect, the first gRNA molecule and the second gRNA molecule include targeting domains comprising, e.g., consisting of, SEQ ID NO: 1539 and SEQ ID NO: 165, respectively. In an aspect, the first gRNA molecule and the second gRNA molecule include targeting domains comprising, e.g., consisting of, SEQ ID NO: 1539 and SEQ ID NO: 113, respectively. In an aspect, the first gRNA molecule and the second gRNA molecule include targeting domains comprising, e.g., consisting of, SEQ ID NO: 1539 and SEQ ID NO: 99, respectively. In an aspect, the first gRNA molecule and the second gRNA molecule include targeting domains comprising, e.g., consisting of, SEQ ID NO: 1539 and SEQ ID NO: 112, respectively. In an aspect, the first gRNA molecule and the second gRNA molecule include targeting domains comprising, e.g., consisting of, SEQ ID NO: 1539 and SEQ ID NO: 98, respectively. In an aspect, the first gRNA molecule and the second gRNA molecule include targeting domains comprising, e.g., consisting of, SEQ ID NO: 1539 and SEQ ID NO: 1580, respectively. In an aspect, the first gRNA molecule and the second gRNA molecule include targeting domains comprising, e.g., consisting of, SEQ ID NO: 1539 and SEQ ID NO: 106, respectively. In an aspect, the first gRNA molecule and the second gRNA molecule include targeting domains comprising, e.g., consisting of, SEQ ID NO: 1539 and SEQ ID NO: 1503, respectively. In an aspect, the first gRNA molecule and the second gRNA molecule include targeting domains comprising, e.g., consisting of, SEQ ID NO: 1539 and SEQ ID NO: 1589, respectively. In an aspect, the first gRNA molecule and the second gRNA molecule include targeting domains comprising, e.g., consisting of, SEQ ID NO: 1539 and SEQ ID NO: 160, respectively. In an aspect, the first gRNA molecule and the second gRNA molecule include targeting domains comprising, e.g., consisting of, SEQ ID NO: 1539 and SEQ ID NO: 1537, respectively. In an aspect, the first gRNA molecule and the second gRNA molecule include targeting domains comprising, e.g., consisting of, SEQ ID NO: 1539 and SEQ ID NO: 159, respectively. In an aspect, the first gRNA molecule and the second gRNA molecule include targeting domains comprising, e.g., consisting of, SEQ ID NO: 1539 and SEQ ID NO: 101, respectively. In an aspect, the first gRNA molecule and the second gRNA molecule include targeting domains comprising, e.g., consisting of, SEQ ID NO: 1539 and SEQ ID NO: 162, respectively. In an aspect, the first gRNA molecule and the second gRNA molecule include targeting domains comprising, e.g., consisting of, SEQ ID NO: 1539 and SEQ ID NO: 104, respectively. In an aspect, the first gRNA molecule and the second gRNA molecule include targeting domains comprising, e.g., consisting of, SEQ ID NO: 1539 and SEQ ID NO: 138, respectively. In an aspect, the first gRNA molecule and the second gRNA molecule include targeting domains comprising, e.g., consisting of, SEQ ID NO: 1539 and SEQ ID NO: 1536, respectively. In an aspect, the first gRNA molecule and the second gRNA molecule include targeting domains comprising, e.g., consisting of, SEQ ID NO: 1539 and SEQ ID NO: 1585, respectively. In an aspect, the first gRNA molecule and the second gRNA molecule include targeting domains comprising, e.g., consisting of, SEQ ID NO: 1585 and SEQ ID NO: 165, respectively. In an aspect, the first gRNA molecule and the second gRNA molecule include targeting domains comprising, e.g., consisting of, SEQ ID NO: 1585 and SEQ ID NO: 113, respectively. In an aspect, the first gRNA molecule and the second gRNA molecule include targeting domains comprising, e.g., consisting of, SEQ ID NO: 1585 and SEQ ID NO: 99, respectively. In an aspect, the first gRNA molecule and the second gRNA molecule include targeting domains comprising, e.g., consisting of, SEQ ID NO: 1585 and SEQ ID NO: 112, respectively. In an aspect, the first gRNA molecule and the second gRNA molecule include targeting domains comprising, e.g., consisting of, SEQ ID NO: 1585 and SEQ ID NO: 98, respectively. In an aspect, the first gRNA molecule and the second gRNA molecule include targeting domains comprising, e.g., consisting of, SEQ ID NO: 1585 and SEQ ID NO: 1580, respectively. In an aspect, the first gRNA molecule and the second gRNA molecule include targeting domains comprising, e.g., consisting of, SEQ ID NO: 1585 and SEQ ID NO: 106, respectively. In an aspect, the first gRNA molecule and the second gRNA molecule include targeting domains comprising, e.g., consisting of, SEQ ID NO: 1585 and SEQ ID NO: 1503, respectively. In an aspect, the first gRNA molecule and the second gRNA molecule include targeting domains comprising, e.g., consisting of, SEQ ID NO: 1585 and SEQ ID NO: 1589, respectively. In an aspect, the first gRNA molecule and the second gRNA molecule include targeting domains comprising, e.g., consisting of, SEQ ID NO: 1585 and SEQ ID NO: 160, respectively. In an aspect, the first gRNA molecule and the second gRNA molecule include targeting domains comprising, e.g., consisting of, SEQ ID NO: 1585 and SEQ ID NO: 1537, respectively. In an aspect, the first gRNA molecule and the second gRNA molecule include targeting domains comprising, e.g., consisting of, SEQ ID NO: 1585 and SEQ ID NO: 159, respectively. In an aspect, the first gRNA molecule and the second gRNA molecule include targeting domains comprising, e.g., consisting of, SEQ ID NO: 1585 and SEQ ID NO: 101, respectively. In an aspect, the first gRNA molecule and the second gRNA molecule include targeting domains comprising, e.g., consisting of, SEQ ID NO: 1585 and SEQ ID NO: 162, respectively. In an aspect, the first gRNA molecule and the second gRNA molecule include targeting domains comprising, e.g., consisting of, SEQ ID NO: 1585 and SEQ ID NO: 104, respectively. In an aspect, the first gRNA molecule and the second gRNA molecule include targeting domains comprising, e.g., consisting of, SEQ ID NO: 1585 and SEQ ID NO: 138, respectively. In an aspect, the first gRNA molecule and the second gRNA molecule include targeting domains comprising, e.g., consisting of, SEQ ID NO: 1585 and SEQ ID NO: 1536, respectively. In an aspect, the first gRNA molecule and the second gRNA molecule include targeting domains comprising, e.g., consisting of, SEQ ID NO: 1585 and SEQ ID NO: 1539, respectively.

**Table 7: Preferred Guide RNA Targeting Domains directed to the +58 Enhancer Region of the BCL11a Gene (i.e., to a BCL11a Enhancer)**

| **Id.** | **Exon/ Feature** | **Strand** | **Targeting domain** | **Locations** | **SEQ ID NO:** |
|---|---|---|---|---|---|
| CR00242 | 58 | + | UAUGCAAUUUUUGCCAAGAU | Chr2:60494419-60494441 | 182 |
| CR00243 | 58 | + | UUUUGCCAAGAUGGGAGUAU | Chr2:60494427-60494449 | 183 |
| CR00244 | 58 | + | UUUGCCAAGAUGGGAGUAUG | Chr2:60494428-60494450 | 184 |
| CR00245 | 58 | + | UCAGUGAGAUGAGAUAUCAA | Chr2:60494477-60494499 | 185 |
| CR00246 | 58 | + | CAGUGAGAUGAGAUAUCAAA | Chr2:60494478-60494500 | 186 |
| CR00247 | 58 | + | CCAUCUCCCUAAUCUCCAAU | Chr2:60494518-60494540 | 187 |
| CR00248 | 58 | - | CCAAUUGGAGAUUAGGGAGA | Chr2:60494518-60494540 | 188 |
| CR00249 | 58 | - | GCUUUGCCAAUUGGAGAUUA | Chr2:60494524-60494546 | 189 |
| CR00250 | 58 | - | GGCUUUGCCAAUUGGAGAUU | Chr2:60494525-60494547 | 190 |
| CR00251 | 58 | + | AAUUGGCAAAGCCAGACUUG | Chr2:60494535-60494557 | 191 |
| CR00252 | 58 | - | AGUCUGUAUUGCCCCAAGUC | Chr2:60494546-60494568 | 192 |
| CR00253 | 58 | + | AGACUUGGGGCAAUACAGAC | Chr2:60494548-60494570 | 193 |
| CR00255 | 58 | - | ACAUUUGGUGAUAAAUCAUU | Chr2:60494602-60494624 | 194 |
| CR00256 | 58 | + | CCAAAUGUUCUUUCUUCAGC | Chr2:60494617-60494639 | 195 |
| CR00257 | 58 | + | AUAAUAGUAUAUGCUUCAUA | Chr2:60494676-60494698 | 196 |
| CR00258 | 58 | - | CGGAGCACUUACUCUGCUCU | Chr2:60494737-60494759 | 197 |
| CR00259 | 58 | - | AGCAUUUUAGUUCACAAGCU | Chr2:60494757-60494779 | 198 |
| CR00260 | 58 | - | UGUAACUAAUAAAUACCAGG | Chr2:60494781-60494803 | 199 |
| CR00261 | 58 | - | AGGUGUAACUAAUAAAUACC | Chr2:60494784-60494806 | 200 |
| CR00264 | 58 | - | UCUGACCCAAACUAGGAAUU | Chr2:60494836-60494858 | 201 |
| CR00266 | 58 | + | AAGGAAAAGAAUAUGACGUC | Chr2:60494883-60494905 | 202 |
| CR00267 | 58 | + | AGGAAAAGAAUAUGACGUCA | Chr2:60494884-60494906 | 203 |
| CR00268 | 58 | + | GGAAAAGAAUAUGACGUCAG | Chr2:60494885-60494907 | 204 |
| CR00269 | 58 | + | GAAAAGAAUAUGACGUCAGG | Chr2:60494886-60494908 | 205 |
| CR00270 | 58 | + | UCAGGGGGAGGCAAGUCAGU | Chr2:60494901-60494923 | 206 |
| CR00271 | 58 | + | CAGGGGGAGGCAAGUCAGUU | Chr2:60494902-60494924 | 207 |
| CR00272 | 58 | | AUCACAUAUAGGCACCUAUC | Chr2:60494939-60494961 | 208 |
| CR00273 | 58 | | CAGGUACCAGCUACUGUGUU | Chr2:60494939-60494961 | 209 |
| CR00274 | 58 | | ACUAUCCACGGAUAUACACU | Chr2:60494939-60494961 | 210 |
| CR00275 | 58 | + | CACAGUAGCUGGUACCUGAU | Chr2:60494939-60494961 | 211 |
| CR00276 | 58 | - | AUCACAUAUAGGCACCUAUC | Chr2:60494953-60494975 | 212 |
| CR00277 | 58 | + | UGAUAGGUGCCUAUAUGUGA | Chr2:60494955-60494977 | 213 |
| CR00278 | 58 | + | AGGUGCCUAUAUGUGAUGGA | Chr2:60494959-60494981 | 214 |
| CR00279 | 58 | + | GGUGCCUAUAUGUGAUGGAU | Chr2:60494960-60494982 | 215 |
| CR00280 | 58 | - | UCCACCCAUCCAUCACAUAU | Chr2:60494964-60494986 | 216 |
| CR00281 | 58 | + | ACAGCCCGACAGAUGAAAAA | Chr2:60494986-60495008 | 217 |
| CR00282 | 58 | + | AUGAAAAAUGGACAAUUAUG | Chr2:60494998-60495020 | 218 |
| CR00283 | 58 | + | AAAAAUGGACAAUUAUGAGG | Chr2:60495001-60495023 | 219 |
| CR00284 | 58 | + | AAAAUGGACAAUUAUGAGGA | Chr2:60495002-60495024 | 220 |
| CR00285 | 58 | + | AAAUGGACAAUUAUGAGGAG | Chr2:60495003-60495025 | 221 |
| CR00286 | 58 | + | GAGGAGGGGAGAGUGCAGAC | Chr2:60495017-60495039 | 222 |
| CR00287 | 58 | + | AGGAGGGGAGAGUGCAGACA | Chr2:60495018-60495040 | 223 |
| CR00288 | 58 | + | CUUCACCUCCUUUACAAUUU | Chr2:60495045-60495067 | 224 |
| CR00289 | 58 | + | UUCACCUCCUUUACAAUUUU | Chr2:60495046-60495068 | 225 |
| CR00290 | 58 | - | GACUCCCAAAAUUGUAAAGG | Chr2:60495050-60495072 | 226 |
| CR00291 | 58 | - | GUGGACUCCCAAAAUUGUAA | Chr2:60495053-60495075 | 227 |
| CR00292 | 58 | + | UUUUGGGAGUCCACACGGCA | Chr2:60495062-60495084 | 228 |
| CR00293 | 58 | - | AAUUUGUAUGCCAUGCCGUG | Chr2:60495072-60495094 | 229 |
| CR00294 | 58 | + | CCAAGAGAGCCUUCCGAAAG | Chr2:60495135-60495157 | 230 |
| CR00295 | 58 | - | CCAGGGGGGCCUCUUUCGGA | Chr2:60495144-60495166 | 231 |
| CR00296 | 58 | + | CCUUCCGAAAGAGGCCCCCC | Chr2:60495144-60495166 | 232 |
| CR00297 | 58 | + | CUUCCGAAAGAGGCCCCCCU | Chr2:60495145-60495167 | 233 |
| CR00298 | 58 | + | AAGAGGCCCCCCUGGGCAAA | Chr2:60495152-60495174 | 234 |
| CR00299 | 58 | - | CGGUGGCCGUUUGCCCAGGG | Chr2:60495158-60495180 | 235 |
| CR00300 | 58 | - | UCGGUGGCCGUUUGCCCAGG | Chr2:60495159-60495181 | 236 |
| CR00301 | 58 | - | AUCGGUGGCCGUUUGCCCAG | Chr2:60495160-60495182 | 237 |
| CR00302 | 58 | - | CAUCGGUGGCCGUUUGCCCA | Chr2:60495161-60495183 | 238 |
| CR00303 | 58 | + | CCUGGGCAAACGGCCACCGA | Chr2:60495162-60495184 | 239 |
| CR00304 | 58 | - | CCAUCGGUGGCCGUUUGCCC | Chr2:60495162-60495184 | 240 |
| CR00305 | 58 | + | GCAAACGGCCACCGAUGGAG | Chr2:60495167-60495189 | 241 |
| CR00306 | 58 | - | CUGGCAGACCUCUCCAUCGG | Chr2:60495175-60495197 | 242 |
| CR00307 | 58 | - | GGACUGGCAGACCUCUCCAU | Chr2:60495178-60495200 | 243 |
| CR00308 | 58 | - | UCUGAUUAGGGUGGGGGCGU | Chr2:60495213-60495235 | 244 |
| CR00309 | 58 | + | CACGCCCCCACCCUAAUCAG | Chr2:60495215-60495237 | 245 |
| CR00310 | 58 | - | UUGGCCUCUGAUUAGGGUGG | Chr2:60495219-60495241 | 246 |
| CR00311 | 58 | - | UUUGGCCUCUGAUUAGGGUG | Chr2:60495220-60495242 | 247 |
| CR00312 | 58 | - | GUUUGGCCUCUGAUUAGGGU | Chr2:60495221-60495243 | 248 |
| CR00313 | 58 | - | GGUUUGGCCUCUGAUUAGGG | Chr2:60495222-60495244 | 249 |
| CR00314 | 58 | - | AAGGGUUUGGCCUCUGAUUA | Chr2:60495225-60495247 | 250 |
| CR00315 | 58 | - | GAAGGGUUUGGCCUCUGAUU | Chr2:60495226-60495248 | 251 |
| CR00316 | 58 | - | UUGCUUUUAUCACAGGCUCC | Chr2:60495248-60495270 | 252 |
| CR00317 | 58 | - | CUAACAGUUGCUUUUAUCAC | Chr2:60495255-60495277 | 253 |
| CR00318 | 58 | + | CUUCAAAGUUGUAUUGACCC | Chr2:60495293-60495315 | 254 |
| CR00319 | 58 | - | ACUCUUAGACAUAACACACC | Chr2:60495311-60495333 | 255 |
| CR00320 | 58 | + | UAGAUGCCAUAUCUCUUUUC | Chr2:60495333-60495355 | 256 |
| CR00321 | 58 | - | CAUAGGCCAGAAAAGAGAUA | Chr2:60495339-60495361 | 257 |
| CR00322 | 58 | + | GGCCUAUGUUAUUACCUGUA | Chr2:60495354-60495376 | 258 |
| CR00323 | 58 | - | GUCCAUACAGGUAAUAACAU | Chr2:60495356-60495378 | 259 |
| CR00324 | 58 | + | UACCUGUAUGGACUUUGCAC | Chr2:60495366-60495388 | 260 |
| CR00325 | 58 | - | UUCCAGUGCAAAGUCCAUAC | Chr2:60495368-60495390 | 261 |
| CR00326 | 58 | + | UGCUCUUACUUAUGCACACC | Chr2:60495400-60495422 | 262 |
| CR00327 | 58 | + | GCUCUUACUUAUGCACACCU | Chr2:60495401-60495423 | 263 |
| CR00328 | 58 | + | CUCUUACUUAUGCACACCUG | Chr2:60495402-60495424 | 264 |
| CR00329 | 58 | - | CAGGGCUGGCUCUAUGCCCC | Chr2:60495418-60495440 | 265 |
| CR00330 | 58 | - | GCUGAAAAGCGAUACAGGGC | Chr2:60495432-60495454 | 266 |
| CR00331 | 58 | - | GAUGGCUGAAAAGCGAUACA | Chr2:60495436-60495458 | 267 |
| CR00332 | 58 | - | AGAUGGCUGAAAAGCGAUAC | Chr2:60495437-60495459 | 268 |
| CR00333 | 58 | - | GGGAGUUAUCUGUAGUGAGA | Chr2:60495454-60495476 | 269 |
| CR00334 | 58 | - | AAGGCAGCUAGACAGGACUU | Chr2:60495474-60495496 | 270 |
| CR00335 | 58 | - | GAAGGCAGCUAGACAGGACU | Chr2:60495475-60495497 | 271 |
| CR00336 | 58 | - | GAUAAGGAAGGCAGCUAGAC | Chr2:60495481-60495503 | 272 |
| CR00337 | 58 | + | CUAGCUGCCUUCCUUAUCAC | Chr2:60495486-60495508 | 273 |
| CR00338 | 58 | - | UGGGUGCUAUUCCUGUGAUA | Chr2:60495497-60495519 | 274 |
| CR00339 | 58 | + | UAUCACAGGAAUAGCACCCA | Chr2:60495500-60495522 | 275 |
| CR00340 | 58 | - | CUGAGGUACUGAUGGACCUU | Chr2:60495516-60495538 | 276 |
| CR00341 | 58 | - | UCUGAGGUACUGAUGGACCU | Chr2:60495517-60495539 | 277 |
| CR001124 | 58 | - | UUAGGGUGGGGGCGUGGGUG | Chr2:60495214-60495236 | 334 |
| CR001125 | 58 | - | UUUUAUCACAGGCUCCAGGA | Chr2:60495215-60495237 | 335 |
| CR001126 | 58 | - | UUUAUCACAGGCUCCAGGAA | Chr2:60495216-60495238 | 336 |
| CR001127 | 58 | - | CACAGGCUCCAGGAAGGGUU | Chr2:60495220-60495242 | 337 |
| CR001128 | 58 | + | AUCAGAGGCCAAACCCUUCC | Chr2:60495236-60495258 | 338 |
| CR001129 | 58 | - | CUCUGAUUAGGGUGGGGGCG | Chr2:60495244-60495266 | 339 |
| CR001130 | 58 | - | GAUUAGGGUGGGGGCGUGGG | Chr2:60495249-60495271 | 340 |
| CR001131 | 58 | - | AUUAGGGUGGGGGCGUGGGU | Chr2:60495250-60495272 | 341 |

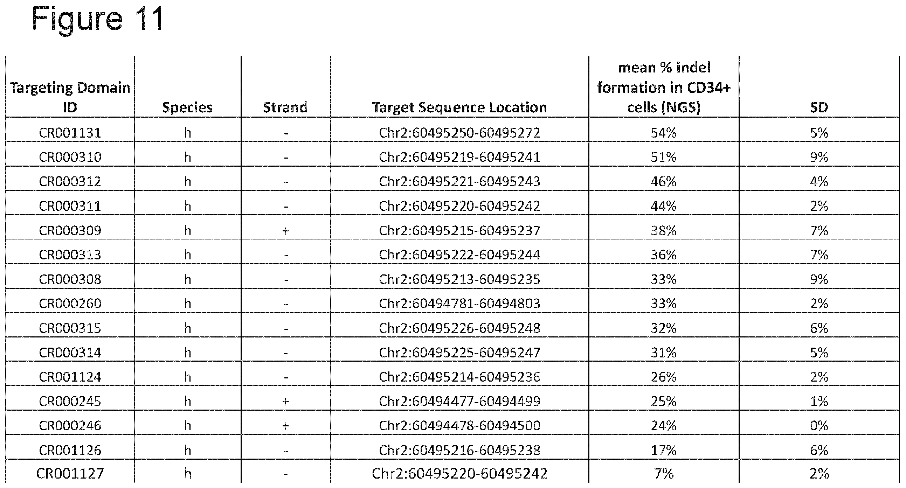

In some aspects of the invention, it is preferred that the gRNA molecule to the +58 Enhancer region of BCL11a comprise a targeting domain of a gRNA listed in Figure 11. In an aspect, the gRNA molecule includes a targeting domain comprising, e.g., consisting of, SEQ ID NO: 341. In an aspect, the gRNA molecule includes a targeting domain comprising, e.g., consisting of, SEQ ID NO: 246. In an aspect, the gRNA molecule includes a targeting domain comprising, e.g., consisting of, SEQ ID NO: 248. In an aspect, the gRNA molecule includes a targeting domain comprising, e.g., consisting of, SEQ ID NO: 247. In an aspect, the gRNA molecule includes a targeting domain comprising, e.g., consisting of, SEQ ID NO: 245. In an aspect, the gRNA molecule includes a targeting domain comprising, e.g., consisting of, SEQ ID NO: 249. In an aspect, the gRNA molecule includes a targeting domain comprising, e.g., consisting of, SEQ ID NO: 244. In an aspect, the gRNA molecule includes a targeting domain comprising, e.g., consisting of, SEQ ID NO: 199. In an aspect, the gRNA molecule includes a targeting domain comprising, e.g., consisting of, SEQ ID NO: 251. In an aspect, the gRNA molecule includes a targeting domain comprising, e.g., consisting of, SEQ ID NO: 250. In an aspect, the gRNA molecule includes a targeting domain comprising, e.g., consisting of, SEQ ID NO: 334. In an aspect, the gRNA molecule includes a targeting domain comprising, e.g., consisting of, SEQ ID NO: 185. In an aspect, the gRNA molecule includes a targeting domain comprising, e.g., consisting of, SEQ ID NO: 186. In an aspect, the gRNA molecule includes a targeting domain comprising, e.g., consisting of, SEQ ID NO: 336. In an aspect, the gRNA molecule includes a targeting domain comprising, e.g., consisting of, SEQ ID NO: 337.

In some aspects of the invention, e.g., as indicated herein, it may be beneficial to include gRNA moleucles targeting more than one, e.g., two, target sites (e.g., a first gRNA molecule and a second gRNA molecule). In some embodiments, the two target sites are both located in the +58 BCL11a enhancer region. In such aspects, any combination of more than one, e.g., two, gRNA molecules (e.g., a first gRNA molecule and a second gRNA molecule) comprising targeting domains listed in Table 7 may be used. In an aspect, the first gRNA molecule and the second gRNA molecule include targeting domains comprising, e.g., consisting of, SEQ ID NO: 341 and SEQ ID NO: 244, respectively. In an aspect, the first gRNA molecule and the second gRNA molecule include targeting domains comprising, e.g., consisting of, SEQ ID NO: 341 and SEQ ID NO: 199, respectively. In an aspect, the first gRNA molecule and the second gRNA molecule include targeting domains comprising, e.g., consisting of, SEQ ID NO: 341 and SEQ ID NO: 251, respectively. In an aspect, the first gRNA molecule and the second gRNA molecule include targeting domains comprising, e.g., consisting of, SEQ ID NO: 341 and SEQ ID NO: 250, respectively. In an aspect, the first gRNA molecule and the second gRNA molecule include targeting domains comprising, e.g., consisting of, SEQ ID NO: 341 and SEQ ID NO: 334, respectively. In an aspect, the first gRNA molecule and the second gRNA molecule include targeting domains comprising, e.g., consisting of, SEQ ID NO: 341 and SEQ ID NO: 185, respectively. In an aspect, the first gRNA molecule and the second gRNA molecule include targeting domains comprising, e.g., consisting of, SEQ ID NO: 341 and SEQ ID NO: 186, respectively. In an aspect, the first gRNA molecule and the second gRNA molecule include targeting domains comprising, e.g., consisting of, SEQ ID NO: 341 and SEQ ID NO: 336, respectively. In an aspect, the first gRNA molecule and the second gRNA molecule include targeting domains comprising, e.g., consisting of, SEQ ID NO: 341 and SEQ ID NO: 337, respectively. In an aspect, the first gRNA molecule and the second gRNA molecule include targeting domains comprising, e.g., consisting of, SEQ ID NO: 341 and SEQ ID NO: 246, respectively. In an aspect, the first gRNA molecule and the second gRNA molecule include targeting domains comprising, e.g., consisting of, SEQ ID NO: 341 and SEQ ID NO: 248, respectively. In an aspect, the first gRNA molecule and the second gRNA molecule include targeting domains comprising, e.g., consisting of, SEQ ID NO: 341 and SEQ ID NO: 247, respectively. In an aspect, the first gRNA molecule and the second gRNA molecule include targeting domains comprising, e.g., consisting of, SEQ ID NO: 341 and SEQ ID NO: 245, respectively. In an aspect, the first gRNA molecule and the second gRNA molecule include targeting domains comprising, e.g., consisting of, SEQ ID NO: 341 and SEQ ID NO: 249, respectively. In an aspect, the first gRNA molecule and the second gRNA molecule include targeting domains comprising, e.g., consisting of, SEQ ID NO: 341 and SEQ ID NO: 244, respectively. In an aspect, the first gRNA molecule and the second gRNA molecule include targeting domains comprising, e.g., consisting of, SEQ ID NO: 341 and SEQ ID NO: 199, respectively. In an aspect, the first gRNA molecule and the second gRNA molecule include targeting domains comprising, e.g., consisting of, SEQ ID NO: 341 and SEQ ID NO: 251, respectively. In an aspect, the first gRNA molecule and the second gRNA molecule include targeting domains comprising, e.g., consisting of, SEQ ID NO: 341 and SEQ ID NO: 250, respectively. In an aspect, the first gRNA molecule and the second gRNA molecule include targeting domains comprising, e.g., consisting of, SEQ ID NO: 341 and SEQ ID NO: 334, respectively. In an aspect, the first gRNA molecule and the second gRNA molecule include targeting domains comprising, e.g., consisting of, SEQ ID NO: 341 and SEQ ID NO: 185, respectively. In an aspect, the first gRNA molecule and the second gRNA molecule include targeting domains comprising, e.g., consisting of, SEQ ID NO: 341 and SEQ ID NO: 186, respectively. In an aspect, the first gRNA molecule and the second gRNA molecule include targeting domains comprising, e.g., consisting of, SEQ ID NO: 341 and SEQ ID NO: 336, respectively. In an aspect, the first gRNA molecule and the second gRNA molecule include targeting domains comprising, e.g., consisting of, SEQ ID NO: 341 and SEQ ID NO: 337, respectively. In an aspect, the first gRNA molecule and the second gRNA molecule include targeting domains comprising, e.g., consisting of, SEQ ID NO: 246 and SEQ ID NO: 244, respectively. In an aspect, the first gRNA molecule and the second gRNA molecule include targeting domains comprising, e.g., consisting of, SEQ ID NO: 246 and SEQ ID NO: 199, respectively. In an aspect, the first gRNA molecule and the second gRNA molecule include targeting domains comprising, e.g., consisting of, SEQ ID NO: 246 and SEQ ID NO: 251, respectively. In an aspect, the first gRNA molecule and the second gRNA molecule include targeting domains comprising, e.g., consisting of, SEQ ID NO: 246 and SEQ ID NO: 250, respectively. In an aspect, the first gRNA molecule and the second gRNA molecule include targeting domains comprising, e.g., consisting of, SEQ ID NO: 246 and SEQ ID NO: 334, respectively. In an aspect, the first gRNA molecule and the second gRNA molecule include targeting domains comprising, e.g., consisting of, SEQ ID NO: 246 and SEQ ID NO: 185, respectively. In an aspect, the first gRNA molecule and the second gRNA molecule include targeting domains comprising, e.g., consisting of, SEQ ID NO: 246 and SEQ ID NO: 186, respectively. In an aspect, the first gRNA molecule and the second gRNA molecule include targeting domains comprising, e.g., consisting of, SEQ ID NO: 246 and SEQ ID NO: 336, respectively. In an aspect, the first gRNA molecule and the second gRNA molecule include targeting domains comprising, e.g., consisting of, SEQ ID NO: 246 and SEQ ID NO: 337, respectively. In an aspect, the first gRNA molecule and the second gRNA molecule include targeting domains comprising, e.g., consisting of, SEQ ID NO: 246 and SEQ ID NO: 248, respectively. In an aspect, the first gRNA molecule and the second gRNA molecule include targeting domains comprising, e.g., consisting of, SEQ ID NO: 246 and SEQ ID NO: 247, respectively. In an aspect, the first gRNA molecule and the second gRNA molecule include targeting domains comprising, e.g., consisting of, SEQ ID NO: 246 and SEQ ID NO: 245, respectively. In an aspect, the first gRNA molecule and the second gRNA molecule include targeting domains comprising, e.g., consisting of, SEQ ID NO: 246 and SEQ ID NO: 249, respectively. In an aspect, the first gRNA molecule and the second gRNA molecule include targeting domains comprising, e.g., consisting of, SEQ ID NO: 246 and SEQ ID NO: 244, respectively. In an aspect, the first gRNA molecule and the second gRNA molecule include targeting domains comprising, e.g., consisting of, SEQ ID NO: 246 and SEQ ID NO: 199, respectively. In an aspect, the first gRNA molecule and the second gRNA molecule include targeting domains comprising, e.g., consisting of, SEQ ID NO: 246 and SEQ ID NO: 251, respectively. In an aspect, the first gRNA molecule and the second gRNA molecule include targeting domains comprising, e.g., consisting of, SEQ ID NO: 246 and SEQ ID NO: 250, respectively. In an aspect, the first gRNA molecule and the second gRNA molecule include targeting domains comprising, e.g., consisting of, SEQ ID NO: 246 and SEQ ID NO: 334, respectively. In an aspect, the first gRNA molecule and the second gRNA molecule include targeting domains comprising, e.g., consisting of, SEQ ID NO: 246 and SEQ ID NO: 185, respectively. In an aspect, the first gRNA molecule and the second gRNA molecule include targeting domains comprising, e.g., consisting of, SEQ ID NO: 246 and SEQ ID NO: 186, respectively. In an aspect, the first gRNA molecule and the second gRNA molecule include targeting domains comprising, e.g., consisting of, SEQ ID NO: 246 and SEQ ID NO: 336, respectively. In an aspect, the first gRNA molecule and the second gRNA molecule include targeting domains comprising, e.g., consisting of, SEQ ID NO: 246 and SEQ ID NO: 337, respectively. In an aspect, the first gRNA molecule and the second gRNA molecule include targeting domains comprising, e.g., consisting of, SEQ ID NO: 248 and SEQ ID NO: 244, respectively. In an aspect, the first gRNA molecule and the second gRNA molecule include targeting domains comprising, e.g., consisting of, SEQ ID NO: 248 and SEQ ID NO: 199, respectively. In an aspect, the first gRNA molecule and the second gRNA molecule include targeting domains comprising, e.g., consisting of, SEQ ID NO: 248 and SEQ ID NO: 251, respectively. In an aspect, the first gRNA molecule and the second gRNA molecule include targeting domains comprising, e.g., consisting of, SEQ ID NO: 248 and SEQ ID NO: 250, respectively. In an aspect, the first gRNA molecule and the second gRNA molecule include targeting domains comprising, e.g., consisting of, SEQ ID NO: 248 and SEQ ID NO: 334, respectively. In an aspect, the first gRNA molecule and the second gRNA molecule include targeting domains comprising, e.g., consisting of, SEQ ID NO: 248 and SEQ ID NO: 185, respectively. In an aspect, the first gRNA molecule and the second gRNA molecule include targeting domains comprising, e.g., consisting of, SEQ ID NO: 248 and SEQ ID NO: 186, respectively. In an aspect, the first gRNA molecule and the second gRNA molecule include targeting domains comprising, e.g., consisting of, SEQ ID NO: 248 and SEQ ID NO: 336, respectively. In an aspect, the first gRNA molecule and the second gRNA molecule include targeting domains comprising, e.g., consisting of, SEQ ID NO: 248 and SEQ ID NO: 337, respectively. In an aspect, the first gRNA molecule and the second gRNA molecule include targeting domains comprising, e.g., consisting of, SEQ ID NO: 248 and SEQ ID NO: 246, respectively. In an aspect, the first gRNA molecule and the second gRNA molecule include targeting domains comprising, e.g., consisting of, SEQ ID NO: 248 and SEQ ID NO: 247, respectively. In an aspect, the first gRNA molecule and the second gRNA molecule include targeting domains comprising, e.g., consisting of, SEQ ID NO: 248 and SEQ ID NO: 245, respectively. In an aspect, the first gRNA molecule and the second gRNA molecule include targeting domains comprising, e.g., consisting of, SEQ ID NO: 248 and SEQ ID NO: 249, respectively. In an aspect, the first gRNA molecule and the second gRNA molecule include targeting domains comprising, e.g., consisting of, SEQ ID NO: 248 and SEQ ID NO: 244, respectively. In an aspect, the first gRNA molecule and the second gRNA molecule include targeting domains comprising, e.g., consisting of, SEQ ID NO: 248 and SEQ ID NO: 199, respectively. In an aspect, the first gRNA molecule and the second gRNA molecule include targeting domains comprising, e.g., consisting of, SEQ ID NO: 248 and SEQ ID NO: 251, respectively. In an aspect, the first gRNA molecule and the second gRNA molecule include targeting domains comprising, e.g., consisting of, SEQ ID NO: 248 and SEQ ID NO: 250, respectively. In an aspect, the first gRNA molecule and the second gRNA molecule include targeting domains comprising, e.g., consisting of, SEQ ID NO: 248 and SEQ ID NO: 334, respectively. In an aspect, the first gRNA molecule and the second gRNA molecule include targeting domains comprising, e.g., consisting of, SEQ ID NO: 248 and SEQ ID NO: 185, respectively. In an aspect, the first gRNA molecule and the second gRNA molecule include targeting domains comprising, e.g., consisting of, SEQ ID NO: 248 and SEQ ID NO: 186, respectively. In an aspect, the first gRNA molecule and the second gRNA molecule include targeting domains comprising, e.g., consisting of, SEQ ID NO: 248 and SEQ ID NO: 336, respectively. In an aspect, the first gRNA molecule and the second gRNA molecule include targeting domains comprising, e.g., consisting of, SEQ ID NO: 248 and SEQ ID NO: 337, respectively. In an aspect, the first gRNA molecule and the second gRNA molecule include targeting domains comprising, e.g., consisting of, SEQ ID NO: 247 and SEQ ID NO: 244, respectively. In an aspect, the first gRNA molecule and the second gRNA molecule include targeting domains comprising, e.g., consisting of, SEQ ID NO: 247 and SEQ ID NO: 199, respectively. In an aspect, the first gRNA molecule and the second gRNA molecule include targeting domains comprising, e.g., consisting of, SEQ ID NO: 247 and SEQ ID NO: 251, respectively. In an aspect, the first gRNA molecule and the second gRNA molecule include targeting domains comprising, e.g., consisting of, SEQ ID NO: 247 and SEQ ID NO: 250, respectively. In an aspect, the first gRNA molecule and the second gRNA molecule include targeting domains comprising, e.g., consisting of, SEQ ID NO: 247 and SEQ ID NO: 334, respectively. In an aspect, the first gRNA molecule and the second gRNA molecule include targeting domains comprising, e.g., consisting of, SEQ ID NO: 247 and SEQ ID NO: 185, respectively. In an aspect, the first gRNA molecule and the second gRNA molecule include targeting domains comprising, e.g., consisting of, SEQ ID NO: 247 and SEQ ID NO: 186, respectively. In an aspect, the first gRNA molecule and the second gRNA molecule include targeting domains comprising, e.g., consisting of, SEQ ID NO: 247 and SEQ ID NO: 336, respectively. In an aspect, the first gRNA molecule and the second gRNA molecule include targeting domains comprising, e.g., consisting of, SEQ ID NO: 247 and SEQ ID NO: 337, respectively. In an aspect, the first gRNA molecule and the second gRNA molecule include targeting domains comprising, e.g., consisting of, SEQ ID NO: 247 and SEQ ID NO: 246, respectively. In an aspect, the first gRNA molecule and the second gRNA molecule include targeting domains comprising, e.g., consisting of, SEQ ID NO: 247 and SEQ ID NO: 248, respectively. In an aspect, the first gRNA molecule and the second gRNA molecule include targeting domains comprising, e.g., consisting of, SEQ ID NO: 247 and SEQ ID NO: 245, respectively. In an aspect, the first gRNA molecule and the second gRNA molecule include targeting domains comprising, e.g., consisting of, SEQ ID NO: 247 and SEQ ID NO: 249, respectively. In an aspect, the first gRNA molecule and the second gRNA molecule include targeting domains comprising, e.g., consisting of, SEQ ID NO: 247 and SEQ ID NO: 244, respectively. In an aspect, the first gRNA molecule and the second gRNA molecule include targeting domains comprising, e.g., consisting of, SEQ ID NO: 247 and SEQ ID NO: 199, respectively. In an aspect, the first gRNA molecule and the second gRNA molecule include targeting domains comprising, e.g., consisting of, SEQ ID NO: 247 and SEQ ID NO: 251, respectively. In an aspect, the first gRNA molecule and the second gRNA molecule include targeting domains comprising, e.g., consisting of, SEQ ID NO: 247 and SEQ ID NO: 250, respectively. In an aspect, the first gRNA molecule and the second gRNA molecule include targeting domains comprising, e.g., consisting of, SEQ ID NO: 247 and SEQ ID NO: 334, respectively. In an aspect, the first gRNA molecule and the second gRNA molecule include targeting domains comprising, e.g., consisting of, SEQ ID NO: 247 and SEQ ID NO: 185, respectively. In an aspect, the first gRNA molecule and the second gRNA molecule include targeting domains comprising, e.g., consisting of, SEQ ID NO: 247 and SEQ ID NO: 186, respectively. In an aspect, the first gRNA molecule and the second gRNA molecule include targeting domains comprising, e.g., consisting of, SEQ ID NO: 247 and SEQ ID NO: 336, respectively. In an aspect, the first gRNA molecule and the second gRNA molecule include targeting domains comprising, e.g., consisting of, SEQ ID NO: 247 and SEQ ID NO: 337, respectively. In an aspect, the first gRNA molecule and the second gRNA molecule include targeting domains comprising, e.g., consisting of, SEQ ID NO: 245 and SEQ ID NO: 244, respectively. In an aspect, the first gRNA molecule and the second gRNA molecule include targeting domains comprising, e.g., consisting of, SEQ ID NO: 245 and SEQ ID NO: 199, respectively. In an aspect, the first gRNA molecule and the second gRNA molecule include targeting domains comprising, e.g., consisting of, SEQ ID NO: 245 and SEQ ID NO: 251, respectively. In an aspect, the first gRNA molecule and the second gRNA molecule include targeting domains comprising, e.g., consisting of, SEQ ID NO: 245 and SEQ ID NO: 250, respectively. In an aspect, the first gRNA molecule and the second gRNA molecule include targeting domains comprising, e.g., consisting of, SEQ ID NO: 245 and SEQ ID NO: 334, respectively. In an aspect, the first gRNA molecule and the second gRNA molecule include targeting domains comprising, e.g., consisting of, SEQ ID NO: 245 and SEQ ID NO: 185, respectively. In an aspect, the first gRNA molecule and the second gRNA molecule include targeting domains comprising, e.g., consisting of, SEQ ID NO: 245 and SEQ ID NO: 186, respectively. In an aspect, the first gRNA molecule and the second gRNA molecule include targeting domains comprising, e.g., consisting of, SEQ ID NO: 245 and SEQ ID NO: 336, respectively. In an aspect, the first gRNA molecule and the second gRNA molecule include targeting domains comprising, e.g., consisting of, SEQ ID NO: 245 and SEQ ID NO: 337, respectively. In an aspect, the first gRNA molecule and the second gRNA molecule include targeting domains comprising, e.g., consisting of, SEQ ID NO: 245 and SEQ ID NO: 246, respectively. In an aspect, the first gRNA molecule and the second gRNA molecule include targeting domains comprising, e.g., consisting of, SEQ ID NO: 245 and SEQ ID NO: 248, respectively. In an aspect, the first gRNA molecule and the second gRNA molecule include targeting domains comprising, e.g., consisting of, SEQ ID NO: 245 and SEQ ID NO: 247, respectively. In an aspect, the first gRNA molecule and the second gRNA molecule include targeting domains comprising, e.g., consisting of, SEQ ID NO: 245 and SEQ ID NO: 249, respectively. In an aspect, the first gRNA molecule and the second gRNA molecule include targeting domains comprising, e.g., consisting of, SEQ ID NO: 245 and SEQ ID NO: 244, respectively. In an aspect, the first gRNA molecule and the second gRNA molecule include targeting domains comprising, e.g., consisting of, SEQ ID NO: 245 and SEQ ID NO: 199, respectively. In an aspect, the first gRNA molecule and the second gRNA molecule include targeting domains comprising, e.g., consisting of, SEQ ID NO: 245 and SEQ ID NO: 251, respectively. In an aspect, the first gRNA molecule and the second gRNA molecule include targeting domains comprising, e.g., consisting of, SEQ ID NO: 245 and SEQ ID NO: 250, respectively. In an aspect, the first gRNA molecule and the second gRNA molecule include targeting domains comprising, e.g., consisting of, SEQ ID NO: 245 and SEQ ID NO: 334, respectively. In an aspect, the first gRNA molecule and the second gRNA molecule include targeting domains comprising, e.g., consisting of, SEQ ID NO: 245 and SEQ ID NO: 185, respectively. In an aspect, the first gRNA molecule and the second gRNA molecule include targeting domains comprising, e.g., consisting of, SEQ ID NO: 245 and SEQ ID NO: 186, respectively. In an aspect, the first gRNA molecule and the second gRNA molecule include targeting domains comprising, e.g., consisting of, SEQ ID NO: 245 and SEQ ID NO: 336, respectively. In an aspect, the first gRNA molecule and the second gRNA molecule include targeting domains comprising, e.g., consisting of, SEQ ID NO: 245 and SEQ ID NO: 337, respectively. In an aspect, the first gRNA molecule and the second gRNA molecule include targeting domains comprising, e.g., consisting of, SEQ ID NO: 249 and SEQ ID NO: 244, respectively. In an aspect, the first gRNA molecule and the second gRNA molecule include targeting domains comprising, e.g., consisting of, SEQ ID NO: 249 and SEQ ID NO: 199, respectively. In an aspect, the first gRNA molecule and the second gRNA molecule include targeting domains comprising, e.g., consisting of, SEQ ID NO: 249 and SEQ ID NO: 251, respectively. In an aspect, the first gRNA molecule and the second gRNA molecule include targeting domains comprising, e.g., consisting of, SEQ ID NO: 249 and SEQ ID NO: 250, respectively. In an aspect, the first gRNA molecule and the second gRNA molecule include targeting domains comprising, e.g., consisting of, SEQ ID NO: 249 and SEQ ID NO: 334, respectively. In an aspect, the first gRNA molecule and the second gRNA molecule include targeting domains comprising, e.g., consisting of, SEQ ID NO: 249 and SEQ ID NO: 185, respectively. In an aspect, the first gRNA molecule and the second gRNA molecule include targeting domains comprising, e.g., consisting of, SEQ ID NO: 249 and SEQ ID NO: 186, respectively. In an aspect, the first gRNA molecule and the second gRNA molecule include targeting domains comprising, e.g., consisting of, SEQ ID NO: 249 and SEQ ID NO: 336, respectively. In an aspect, the first gRNA molecule and the second gRNA molecule include targeting domains comprising, e.g., consisting of, SEQ ID NO: 249 and SEQ ID NO: 337, respectively. In an aspect, the first gRNA molecule and the second gRNA molecule include targeting domains comprising, e.g., consisting of, SEQ ID NO: 249 and SEQ ID NO: 246, respectively. In an aspect, the first gRNA molecule and the second gRNA molecule include targeting domains comprising, e.g., consisting of, SEQ ID NO: 249 and SEQ ID NO: 248, respectively. In an aspect, the first gRNA molecule and the second gRNA molecule include targeting domains comprising, e.g., consisting of, SEQ ID NO: 249 and SEQ ID NO: 247, respectively. In an aspect, the first gRNA molecule and the second gRNA molecule include targeting domains comprising, e.g., consisting of, SEQ ID NO: 249 and SEQ ID NO: 245, respectively. In an aspect, the first gRNA molecule and the second gRNA molecule include targeting domains comprising, e.g., consisting of, SEQ ID NO: 249 and SEQ ID NO: 244, respectively. In an aspect, the first gRNA molecule and the second gRNA molecule include targeting domains comprising, e.g., consisting of, SEQ ID NO: 249 and SEQ ID NO: 199, respectively. In an aspect, the first gRNA molecule and the second gRNA molecule include targeting domains comprising, e.g., consisting of, SEQ ID NO: 249 and SEQ ID NO: 251, respectively. In an aspect, the first gRNA molecule and the second gRNA molecule include targeting domains comprising, e.g., consisting of, SEQ ID NO: 249 and SEQ ID NO: 250, respectively. In an aspect, the first gRNA molecule and the second gRNA molecule include targeting domains comprising, e.g., consisting of, SEQ ID NO: 249 and SEQ ID NO: 334, respectively. In an aspect, the first gRNA molecule and the second gRNA molecule include targeting domains comprising, e.g., consisting of, SEQ ID NO: 249 and SEQ ID NO: 185, respectively. In an aspect, the first gRNA molecule and the second gRNA molecule include targeting domains comprising, e.g., consisting of, SEQ ID NO: 249 and SEQ ID NO: 186, respectively. In an aspect, the first gRNA molecule and the second gRNA molecule include targeting domains comprising, e.g., consisting of, SEQ ID NO: 249 and SEQ ID NO: 336, respectively. In an aspect, the first gRNA molecule and the second gRNA molecule include targeting domains comprising, e.g., consisting of, SEQ ID NO: 249 and SEQ ID NO: 337, respectively. In an aspect, the first gRNA molecule and the second gRNA molecule include targeting domains comprising, e.g., consisting of, SEQ ID NO: 244 and SEQ ID NO: 199, respectively. In an aspect, the first gRNA molecule and the second gRNA molecule include targeting domains comprising, e.g., consisting of, SEQ ID NO: 244 and SEQ ID NO: 251, respectively. In an aspect, the first gRNA molecule and the second gRNA molecule include targeting domains comprising, e.g., consisting of, SEQ ID NO: 244 and SEQ ID NO: 250, respectively. In an aspect, the first gRNA molecule and the second gRNA molecule include targeting domains comprising, e.g., consisting of, SEQ ID NO: 244 and SEQ ID NO: 334, respectively. In an aspect, the first gRNA molecule and the second gRNA molecule include targeting domains comprising, e.g., consisting of, SEQ ID NO: 244 and SEQ ID NO: 185, respectively. In an aspect, the first gRNA molecule and the second gRNA molecule include targeting domains comprising, e.g., consisting of, SEQ ID NO: 244 and SEQ ID NO: 186, respectively. In an aspect, the first gRNA molecule and the second gRNA molecule include targeting domains comprising, e.g., consisting of, SEQ ID NO: 244 and SEQ ID NO: 336, respectively. In an aspect, the first gRNA molecule and the second gRNA molecule include targeting domains comprising, e.g., consisting of, SEQ ID NO: 244 and SEQ ID NO: 337, respectively. In an aspect, the first gRNA molecule and the second gRNA molecule include targeting domains comprising, e.g., consisting of, SEQ ID NO: 244 and SEQ ID NO: 246, respectively. In an aspect, the first gRNA molecule and the second gRNA molecule include targeting domains comprising, e.g., consisting of, SEQ ID NO: 244 and SEQ ID NO: 248, respectively. In an aspect, the first gRNA molecule and the second gRNA molecule include targeting domains comprising, e.g., consisting of, SEQ ID NO: 244 and SEQ ID NO: 247, respectively. In an aspect, the first gRNA molecule and the second gRNA molecule include targeting domains comprising, e.g., consisting of, SEQ ID NO: 244 and SEQ ID NO: 245, respectively. In an aspect, the first gRNA molecule and the second gRNA molecule include targeting domains comprising, e.g., consisting of, SEQ ID NO: 244 and SEQ ID NO: 249, respectively. In an aspect, the first gRNA molecule and the second gRNA molecule include targeting domains comprising, e.g., consisting of, SEQ ID NO: 244 and SEQ ID NO: 199, respectively. In an aspect, the first gRNA molecule and the second gRNA molecule include targeting domains comprising, e.g., consisting of, SEQ ID NO: 244 and SEQ ID NO: 251, respectively. In an aspect, the first gRNA molecule and the second gRNA molecule include targeting domains comprising, e.g., consisting of, SEQ ID NO: 244 and SEQ ID NO: 250, respectively. In an aspect, the first gRNA molecule and the second gRNA molecule include targeting domains comprising, e.g., consisting of, SEQ ID NO: 244 and SEQ ID NO: 334, respectively. In an aspect, the first gRNA molecule and the second gRNA molecule include targeting domains comprising, e.g., consisting of, SEQ ID NO: 244 and SEQ ID NO: 185, respectively. In an aspect, the first gRNA molecule and the second gRNA molecule include targeting domains comprising, e.g., consisting of, SEQ ID NO: 244 and SEQ ID NO: 186, respectively. In an aspect, the first gRNA molecule and the second gRNA molecule include targeting domains comprising, e.g., consisting of, SEQ ID NO: 244 and SEQ ID NO: 336, respectively. In an aspect, the first gRNA molecule and the second gRNA molecule include targeting domains comprising, e.g., consisting of, SEQ ID NO: 244 and SEQ ID NO: 337, respectively. In an aspect, the first gRNA molecule and the second gRNA molecule include targeting domains comprising, e.g., consisting of, SEQ ID NO: 199 and SEQ ID NO: 244, respectively. In an aspect, the first gRNA molecule and the second gRNA molecule include targeting domains comprising, e.g., consisting of, SEQ ID NO: 199 and SEQ ID NO: 251, respectively. In an aspect, the first gRNA molecule and the second gRNA molecule include targeting domains comprising, e.g., consisting of, SEQ ID NO: 199 and SEQ ID NO: 250, respectively. In an aspect, the first gRNA molecule and the second gRNA molecule include targeting domains comprising, e.g., consisting of, SEQ ID NO: 199 and SEQ ID NO: 334, respectively. In an aspect, the first gRNA molecule and the second gRNA molecule include targeting domains comprising, e.g., consisting of, SEQ ID NO: 199 and SEQ ID NO: 185, respectively. In an aspect, the first gRNA molecule and the second gRNA molecule include targeting domains comprising, e.g., consisting of, SEQ ID NO: 199 and SEQ ID NO: 186, respectively. In an aspect, the first gRNA molecule and the second gRNA molecule include targeting domains comprising, e.g., consisting of, SEQ ID NO: 199 and SEQ ID NO: 336, respectively. In an aspect, the first gRNA molecule and the second gRNA molecule include targeting domains comprising, e.g., consisting of, SEQ ID NO: 199 and SEQ ID NO: 337, respectively. In an aspect, the first gRNA molecule and the second gRNA molecule include targeting domains comprising, e.g., consisting of, SEQ ID NO: 199 and SEQ ID NO: 246, respectively. In an aspect, the first gRNA molecule and the second gRNA molecule include targeting domains comprising, e.g., consisting of, SEQ ID NO: 199 and SEQ ID NO: 248, respectively. In an aspect, the first gRNA molecule and the second gRNA molecule include targeting domains comprising, e.g., consisting of, SEQ ID NO: 199 and SEQ ID NO: 247, respectively. In an aspect, the first gRNA molecule and the second gRNA molecule include targeting domains comprising, e.g., consisting of, SEQ ID NO: 199 and SEQ ID NO: 245, respectively. In an aspect, the first gRNA molecule and the second gRNA molecule include targeting domains comprising, e.g., consisting of, SEQ ID NO: 199 and SEQ ID NO: 249, respectively. In an aspect, the first gRNA molecule and the second gRNA molecule include targeting domains comprising, e.g., consisting of, SEQ ID NO: 199 and SEQ ID NO: 244, respectively. In an aspect, the first gRNA molecule and the second gRNA molecule include targeting domains comprising, e.g., consisting of, SEQ ID NO: 199 and SEQ ID NO: 251, respectively. In an aspect, the first gRNA molecule and the second gRNA molecule include targeting domains comprising, e.g., consisting of, SEQ ID NO: 199 and SEQ ID NO: 250, respectively. In an aspect, the first gRNA molecule and the second gRNA molecule include targeting domains comprising, e.g., consisting of, SEQ ID NO: 199 and SEQ ID NO: 334, respectively. In an aspect, the first gRNA molecule and the second gRNA molecule include targeting domains comprising, e.g., consisting of, SEQ ID NO: 199 and SEQ ID NO: 185, respectively. In an aspect, the first gRNA molecule and the second gRNA molecule include targeting domains comprising, e.g., consisting of, SEQ ID NO: 199 and SEQ ID NO: 186, respectively. In an aspect, the first gRNA molecule and the second gRNA molecule include targeting domains comprising, e.g., consisting of, SEQ ID NO: 199 and SEQ ID NO: 336, respectively. In an aspect, the first gRNA molecule and the second gRNA molecule include targeting domains comprising, e.g., consisting of, SEQ ID NO: 199 and SEQ ID NO: 337, respectively. In an aspect, the first gRNA molecule and the second gRNA molecule include targeting domains comprising, e.g., consisting of, SEQ ID NO: 251 and SEQ ID NO: 244, respectively. In an aspect, the first gRNA molecule and the second gRNA molecule include targeting domains comprising, e.g., consisting of, SEQ ID NO: 251 and SEQ ID NO: 199, respectively. In an aspect, the first gRNA molecule and the second gRNA molecule include targeting domains comprising, e.g., consisting of, SEQ ID NO: 251 and SEQ ID NO: 250, respectively. In an aspect, the first gRNA molecule and the second gRNA molecule include targeting domains comprising, e.g., consisting of, SEQ ID NO: 251 and SEQ ID NO: 334, respectively. In an aspect, the first gRNA molecule and the second gRNA molecule include targeting domains comprising, e.g., consisting of, SEQ ID NO: 251 and SEQ ID NO: 185, respectively. In an aspect, the first gRNA molecule and the second gRNA molecule include targeting domains comprising, e.g., consisting of, SEQ ID NO: 251 and SEQ ID NO: 186, respectively. In an aspect, the first gRNA molecule and the second gRNA molecule include targeting domains comprising, e.g., consisting of, SEQ ID NO: 251 and SEQ ID NO: 336, respectively. In an aspect, the first gRNA molecule and the second gRNA molecule include targeting domains comprising, e.g., consisting of, SEQ ID NO: 251 and SEQ ID NO: 337, respectively. In an aspect, the first gRNA molecule and the second gRNA molecule include targeting domains comprising, e.g., consisting of, SEQ ID NO: 251 and SEQ ID NO: 246, respectively. In an aspect, the first gRNA molecule and the second gRNA molecule include targeting domains comprising, e.g., consisting of, SEQ ID NO: 251 and SEQ ID NO: 248, respectively. In an aspect, the first gRNA molecule and the second gRNA molecule include targeting domains comprising, e.g., consisting of, SEQ ID NO: 251 and SEQ ID NO: 247, respectively. In an aspect, the first gRNA molecule and the second gRNA molecule include targeting domains comprising, e.g., consisting of, SEQ ID NO: 251 and SEQ ID NO: 245, respectively. In an aspect, the first gRNA molecule and the second gRNA molecule include targeting domains comprising, e.g., consisting of, SEQ ID NO: 251 and SEQ ID NO: 249, respectively. In an aspect, the first gRNA molecule and the second gRNA molecule include targeting domains comprising, e.g., consisting of, SEQ ID NO: 251 and SEQ ID NO: 244, respectively. In an aspect, the first gRNA molecule and the second gRNA molecule include targeting domains comprising, e.g., consisting of, SEQ ID NO: 251 and SEQ ID NO: 199, respectively. In an aspect, the first gRNA molecule and the second gRNA molecule include targeting domains comprising, e.g., consisting of, SEQ ID NO: 251 and SEQ ID NO: 250, respectively. In an aspect, the first gRNA molecule and the second gRNA molecule include targeting domains comprising, e.g., consisting of, SEQ ID NO: 251 and SEQ ID NO: 334, respectively. In an aspect, the first gRNA molecule and the second gRNA molecule include targeting domains comprising, e.g., consisting of, SEQ ID NO: 251 and SEQ ID NO: 185, respectively. In an aspect, the first gRNA molecule and the second gRNA molecule include targeting domains comprising, e.g., consisting of, SEQ ID NO: 251 and SEQ ID NO: 186, respectively. In an aspect, the first gRNA molecule and the second gRNA molecule include targeting domains comprising, e.g., consisting of, SEQ ID NO: 251 and SEQ ID NO: 336, respectively. In an aspect, the first gRNA molecule and the second gRNA molecule include targeting domains comprising, e.g., consisting of, SEQ ID NO: 251 and SEQ ID NO: 337, respectively. In an aspect, the first gRNA molecule and the second gRNA molecule include targeting domains comprising, e.g., consisting of, SEQ ID NO: 250 and SEQ ID NO: 244, respectively. In an aspect, the first gRNA molecule and the second gRNA molecule include targeting domains comprising, e.g., consisting of, SEQ ID NO: 250 and SEQ ID NO: 199, respectively. In an aspect, the first gRNA molecule and the second gRNA molecule include targeting domains comprising, e.g., consisting of, SEQ ID NO: 250 and SEQ ID NO: 251, respectively. In an aspect, the first gRNA molecule and the second gRNA molecule include targeting domains comprising, e.g., consisting of, SEQ ID NO: 250 and SEQ ID NO: 334, respectively. In an aspect, the first gRNA molecule and the second gRNA molecule include targeting domains comprising, e.g., consisting of, SEQ ID NO: 250 and SEQ ID NO: 185, respectively. In an aspect, the first gRNA molecule and the second gRNA molecule include targeting domains comprising, e.g., consisting of, SEQ ID NO: 250 and SEQ ID NO: 186, respectively. In an aspect, the first gRNA molecule and the second gRNA molecule include targeting domains comprising, e.g., consisting of, SEQ ID NO: 250 and SEQ ID NO: 336, respectively. In an aspect, the first gRNA molecule and the second gRNA molecule include targeting domains comprising, e.g., consisting of, SEQ ID NO: 250 and SEQ ID NO: 337, respectively. In an aspect, the first gRNA molecule and the second gRNA molecule include targeting domains comprising, e.g., consisting of, SEQ ID NO: 250 and SEQ ID NO: 246, respectively. In an aspect, the first gRNA molecule and the second gRNA molecule include targeting domains comprising, e.g., consisting of, SEQ ID NO: 250 and SEQ ID NO: 248, respectively. In an aspect, the first gRNA molecule and the second gRNA molecule include targeting domains comprising, e.g., consisting of, SEQ ID NO: 250 and SEQ ID NO: 247, respectively. In an aspect, the first gRNA molecule and the second gRNA molecule include targeting domains comprising, e.g., consisting of, SEQ ID NO: 250 and SEQ ID NO: 245, respectively. In an aspect, the first gRNA molecule and the second gRNA molecule include targeting domains comprising, e.g., consisting of, SEQ ID NO: 250 and SEQ ID NO: 249, respectively. In an aspect, the first gRNA molecule and the second gRNA molecule include targeting domains comprising, e.g., consisting of, SEQ ID NO: 250 and SEQ ID NO: 244, respectively. In an aspect, the first gRNA molecule and the second gRNA molecule include targeting domains comprising, e.g., consisting of, SEQ ID NO: 250 and SEQ ID NO: 199, respectively. In an aspect, the first gRNA molecule and the second gRNA molecule include targeting domains comprising, e.g., consisting of, SEQ ID NO: 250 and SEQ ID NO: 251, respectively. In an aspect, the first gRNA molecule and the second gRNA molecule include targeting domains comprising, e.g., consisting of, SEQ ID NO: 250 and SEQ ID NO: 334, respectively. In an aspect, the first gRNA molecule and the second gRNA molecule include targeting domains comprising, e.g., consisting of, SEQ ID NO: 250 and SEQ ID NO: 185, respectively. In an aspect, the first gRNA molecule and the second gRNA molecule include targeting domains comprising, e.g., consisting of, SEQ ID NO: 250 and SEQ ID NO: 186, respectively. In an aspect, the first gRNA molecule and the second gRNA molecule include targeting domains comprising, e.g., consisting of, SEQ ID NO: 250 and SEQ ID NO: 336, respectively. In an aspect, the first gRNA molecule and the second gRNA molecule include targeting domains comprising, e.g., consisting of, SEQ ID NO: 250 and SEQ ID NO: 337, respectively. In an aspect, the first gRNA molecule and the second gRNA molecule include targeting domains comprising, e.g., consisting of, SEQ ID NO: 334 and SEQ ID NO: 244, respectively. In an aspect, the first gRNA molecule and the second gRNA molecule include targeting domains comprising, e.g., consisting of, SEQ ID NO: 334 and SEQ ID NO: 199, respectively. In an aspect, the first gRNA molecule and the second gRNA molecule include targeting domains comprising, e.g., consisting of, SEQ ID NO: 334 and SEQ ID NO: 251, respectively. In an aspect, the first gRNA molecule and the second gRNA molecule include targeting domains comprising, e.g., consisting of, SEQ ID NO: 334 and SEQ ID NO: 250, respectively. In an aspect, the first gRNA molecule and the second gRNA molecule include targeting domains comprising, e.g., consisting of, SEQ ID NO: 334 and SEQ ID NO: 185, respectively. In an aspect, the first gRNA molecule and the second gRNA molecule include targeting domains comprising, e.g., consisting of, SEQ ID NO: 334 and SEQ ID NO: 186, respectively. In an aspect, the first gRNA molecule and the second gRNA molecule include targeting domains comprising, e.g., consisting of, SEQ ID NO: 334 and SEQ ID NO: 336, respectively. In an aspect, the first gRNA molecule and the second gRNA molecule include targeting domains comprising, e.g., consisting of, SEQ ID NO: 334 and SEQ ID NO: 337, respectively. In an aspect, the first gRNA molecule and the second gRNA molecule include targeting domains comprising, e.g., consisting of, SEQ ID NO: 334 and SEQ ID NO: 246, respectively. In an aspect, the first gRNA molecule and the second gRNA molecule include targeting domains comprising, e.g., consisting of, SEQ ID NO: 334 and SEQ ID NO: 248, respectively. In an aspect, the first gRNA molecule and the second gRNA molecule include targeting domains comprising, e.g., consisting of, SEQ ID NO: 334 and SEQ ID NO: 247, respectively. In an aspect, the first gRNA molecule and the second gRNA molecule include targeting domains comprising, e.g., consisting of, SEQ ID NO: 334 and SEQ ID NO: 245, respectively. In an aspect, the first gRNA molecule and the second gRNA molecule include targeting domains comprising, e.g., consisting of, SEQ ID NO: 334 and SEQ ID NO: 249, respectively. In an aspect, the first gRNA molecule and the second gRNA molecule include targeting domains comprising, e.g., consisting of, SEQ ID NO: 334 and SEQ ID NO: 244, respectively. In an aspect, the first gRNA molecule and the second gRNA molecule include targeting domains comprising, e.g., consisting of, SEQ ID NO: 334 and SEQ ID NO: 199, respectively. In an aspect, the first gRNA molecule and the second gRNA molecule include targeting domains comprising, e.g., consisting of, SEQ ID NO: 334 and SEQ ID NO: 251, respectively. In an aspect, the first gRNA molecule and the second gRNA molecule include targeting domains comprising, e.g., consisting of, SEQ ID NO: 334 and SEQ ID NO: 250, respectively. In an aspect, the first gRNA molecule and the second gRNA molecule include targeting domains comprising, e.g., consisting of, SEQ ID NO: 334 and SEQ ID NO: 185, respectively. In an aspect, the first gRNA molecule and the second gRNA molecule include targeting domains comprising, e.g., consisting of, SEQ ID NO: 334 and SEQ ID NO: 186, respectively. In an aspect, the first gRNA molecule and the second gRNA molecule include targeting domains comprising, e.g., consisting of, SEQ ID NO: 334 and SEQ ID NO: 336, respectively. In an aspect, the first gRNA molecule and the second gRNA molecule include targeting domains comprising, e.g., consisting of, SEQ ID NO: 334 and SEQ ID NO: 337, respectively. In an aspect, the first gRNA molecule and the second gRNA molecule include targeting domains comprising, e.g., consisting of, SEQ ID NO: 185 and SEQ ID NO: 244, respectively. In an aspect, the first gRNA molecule and the second gRNA molecule include targeting domains comprising, e.g., consisting of, SEQ ID NO: 185 and SEQ ID NO: 199, respectively. In an aspect, the first gRNA molecule and the second gRNA molecule include targeting domains comprising, e.g., consisting of, SEQ ID NO: 185 and SEQ ID NO: 251, respectively. In an aspect, the first gRNA molecule and the second gRNA molecule include targeting domains comprising, e.g., consisting of, SEQ ID NO: 185 and SEQ ID NO: 250, respectively. In an aspect, the first gRNA molecule and the second gRNA molecule include targeting domains comprising, e.g., consisting of, SEQ ID NO: 185 and SEQ ID NO: 334, respectively. In an aspect, the first gRNA molecule and the second gRNA molecule include targeting domains comprising, e.g., consisting of, SEQ ID NO: 185 and SEQ ID NO: 186, respectively. In an aspect, the first gRNA molecule and the second gRNA molecule include targeting domains comprising, e.g., consisting of, SEQ ID NO: 185 and SEQ ID NO: 336, respectively. In an aspect, the first gRNA molecule and the second gRNA molecule include targeting domains comprising, e.g., consisting of, SEQ ID NO: 185 and SEQ ID NO: 337, respectively. In an aspect, the first gRNA molecule and the second gRNA molecule include targeting domains comprising, e.g., consisting of, SEQ ID NO: 185 and SEQ ID NO: 246, respectively. In an aspect, the first gRNA molecule and the second gRNA molecule include targeting domains comprising, e.g., consisting of, SEQ ID NO: 185 and SEQ ID NO: 248, respectively. In an aspect, the first gRNA molecule and the second gRNA molecule include targeting domains comprising, e.g., consisting of, SEQ ID NO: 185 and SEQ ID NO: 247, respectively. In an aspect, the first gRNA molecule and the second gRNA molecule include targeting domains comprising, e.g., consisting of, SEQ ID NO: 185 and SEQ ID NO: 245, respectively. In an aspect, the first gRNA molecule and the second gRNA molecule include targeting domains comprising, e.g., consisting of, SEQ ID NO: 185 and SEQ ID NO: 249, respectively. In an aspect, the first gRNA molecule and the second gRNA molecule include targeting domains comprising, e.g., consisting of, SEQ ID NO: 185 and SEQ ID NO: 244, respectively. In an aspect, the first gRNA molecule and the second gRNA molecule include targeting domains comprising, e.g., consisting of, SEQ ID NO: 185 and SEQ ID NO: 199, respectively. In an aspect, the first gRNA molecule and the second gRNA molecule include targeting domains comprising, e.g., consisting of, SEQ ID NO: 185 and SEQ ID NO: 251, respectively. In an aspect, the first gRNA molecule and the second gRNA molecule include targeting domains comprising, e.g., consisting of, SEQ ID NO: 185 and SEQ ID NO: 250, respectively. In an aspect, the first gRNA molecule and the second gRNA molecule include targeting domains comprising, e.g., consisting of, SEQ ID NO: 185 and SEQ ID NO: 334, respectively. In an aspect, the first gRNA molecule and the second gRNA molecule include targeting domains comprising, e.g., consisting of, SEQ ID NO: 185 and SEQ ID NO: 186, respectively. In an aspect, the first gRNA molecule and the second gRNA molecule include targeting domains comprising, e.g., consisting of, SEQ ID NO: 185 and SEQ ID NO: 336, respectively. In an aspect, the first gRNA molecule and the second gRNA molecule include targeting domains comprising, e.g., consisting of, SEQ ID NO: 185 and SEQ ID NO: 337, respectively. In an aspect, the first gRNA molecule and the second gRNA molecule include targeting domains comprising, e.g., consisting of, SEQ ID NO: 186 and SEQ ID NO: 244, respectively. In an aspect, the first gRNA molecule and the second gRNA molecule include targeting domains comprising, e.g., consisting of, SEQ ID NO: 186 and SEQ ID NO: 199, respectively. In an aspect, the first gRNA molecule and the second gRNA molecule include targeting domains comprising, e.g., consisting of, SEQ ID NO: 186 and SEQ ID NO: 251, respectively. In an aspect, the first gRNA molecule and the second gRNA molecule include targeting domains comprising, e.g., consisting of, SEQ ID NO: 186 and SEQ ID NO: 250, respectively. In an aspect, the first gRNA molecule and the second gRNA molecule include targeting domains comprising, e.g., consisting of, SEQ ID NO: 186 and SEQ ID NO: 334, respectively. In an aspect, the first gRNA molecule and the second gRNA molecule include targeting domains comprising, e.g., consisting of, SEQ ID NO: 186 and SEQ ID NO: 185, respectively. In an aspect, the first gRNA molecule and the second gRNA molecule include targeting domains comprising, e.g., consisting of, SEQ ID NO: 186 and SEQ ID NO: 336, respectively. In an aspect, the first gRNA molecule and the second gRNA molecule include targeting domains comprising, e.g., consisting of, SEQ ID NO: 186 and SEQ ID NO: 337, respectively. In an aspect, the first gRNA molecule and the second gRNA molecule include targeting domains comprising, e.g., consisting of, SEQ ID NO: 186 and SEQ ID NO: 246, respectively. In an aspect, the first gRNA molecule and the second gRNA molecule include targeting domains comprising, e.g., consisting of, SEQ ID NO: 186 and SEQ ID NO: 248, respectively. In an aspect, the first gRNA molecule and the second gRNA molecule include targeting domains comprising, e.g., consisting of, SEQ ID NO: 186 and SEQ ID NO: 247, respectively. In an aspect, the first gRNA molecule and the second gRNA molecule include targeting domains comprising, e.g., consisting of, SEQ ID NO: 186 and SEQ ID NO: 245, respectively. In an aspect, the first gRNA molecule and the second gRNA molecule include targeting domains comprising, e.g., consisting of, SEQ ID NO: 186 and SEQ ID NO: 249, respectively. In an aspect, the first gRNA molecule and the second gRNA molecule include targeting domains comprising, e.g., consisting of, SEQ ID NO: 186 and SEQ ID NO: 244, respectively. In an aspect, the first gRNA molecule and the second gRNA molecule include targeting domains comprising, e.g., consisting of, SEQ ID NO: 186 and SEQ ID NO: 199, respectively. In an aspect, the first gRNA molecule and the second gRNA molecule include targeting domains comprising, e.g., consisting of, SEQ ID NO: 186 and SEQ ID NO: 251, respectively. In an aspect, the first gRNA molecule and the second gRNA molecule include targeting domains comprising, e.g., consisting of, SEQ ID NO: 186 and SEQ ID NO: 250, respectively. In an aspect, the first gRNA molecule and the second gRNA molecule include targeting domains comprising, e.g., consisting of, SEQ ID NO: 186 and SEQ ID NO: 334, respectively. In an aspect, the first gRNA molecule and the second gRNA molecule include targeting domains comprising, e.g., consisting of, SEQ ID NO: 186 and SEQ ID NO: 185, respectively. In an aspect, the first gRNA molecule and the second gRNA molecule include targeting domains comprising, e.g., consisting of, SEQ ID NO: 186 and SEQ ID NO: 336, respectively. In an aspect, the first gRNA molecule and the second gRNA molecule include targeting domains comprising, e.g., consisting of, SEQ ID NO: 186 and SEQ ID NO: 337, respectively. In an aspect, the first gRNA molecule and the second gRNA molecule include targeting domains comprising, e.g., consisting of, SEQ ID NO: 336 and SEQ ID NO: 244, respectively. In an aspect, the first gRNA molecule and the second gRNA molecule include targeting domains comprising, e.g., consisting of, SEQ ID NO: 336 and SEQ ID NO: 199, respectively. In an aspect, the first gRNA molecule and the second gRNA molecule include targeting domains comprising, e.g., consisting of, SEQ ID NO: 336 and SEQ ID NO: 251, respectively. In an aspect, the first gRNA molecule and the second gRNA molecule include targeting domains comprising, e.g., consisting of, SEQ ID NO: 336 and SEQ ID NO: 250, respectively. In an aspect, the first gRNA molecule and the second gRNA molecule include targeting domains comprising, e.g., consisting of, SEQ ID NO: 336 and SEQ ID NO: 334, respectively. In an aspect, the first gRNA molecule and the second gRNA molecule include targeting domains comprising, e.g., consisting of, SEQ ID NO: 336 and SEQ ID NO: 185, respectively. In an aspect, the first gRNA molecule and the second gRNA molecule include targeting domains comprising, e.g., consisting of, SEQ ID NO: 336 and SEQ ID NO: 186, respectively. In an aspect, the first gRNA molecule and the second gRNA molecule include targeting domains comprising, e.g., consisting of, SEQ ID NO: 336 and SEQ ID NO: 337, respectively. In an aspect, the first gRNA molecule and the second gRNA molecule include targeting domains comprising, e.g., consisting of, SEQ ID NO: 336 and SEQ ID NO: 246, respectively. In an aspect, the first gRNA molecule and the second gRNA molecule include targeting domains comprising, e.g., consisting of, SEQ ID NO: 336 and SEQ ID NO: 248, respectively. In an aspect, the first gRNA molecule and the second gRNA molecule include targeting domains comprising, e.g., consisting of, SEQ ID NO: 336 and SEQ ID NO: 247, respectively. In an aspect, the first gRNA molecule and the second gRNA molecule include targeting domains comprising, e.g., consisting of, SEQ ID NO: 336 and SEQ ID NO: 245, respectively. In an aspect, the first gRNA molecule and the second gRNA molecule include targeting domains comprising, e.g., consisting of, SEQ ID NO: 336 and SEQ ID NO: 249, respectively. In an aspect, the first gRNA molecule and the second gRNA molecule include targeting domains comprising, e.g., consisting of, SEQ ID NO: 336 and SEQ ID NO: 244, respectively. In an aspect, the first gRNA molecule and the second gRNA molecule include targeting domains comprising, e.g., consisting of, SEQ ID NO: 336 and SEQ ID NO: 199, respectively. In an aspect, the first gRNA molecule and the second gRNA molecule include targeting domains comprising, e.g., consisting of, SEQ ID NO: 336 and SEQ ID NO: 251, respectively. In an aspect, the first gRNA molecule and the second gRNA molecule include targeting domains comprising, e.g., consisting of, SEQ ID NO: 336 and SEQ ID NO: 250, respectively. In an aspect, the first gRNA molecule and the second gRNA molecule include targeting domains comprising, e.g., consisting of, SEQ ID NO: 336 and SEQ ID NO: 334, respectively. In an aspect, the first gRNA molecule and the second gRNA molecule include targeting domains comprising, e.g., consisting of, SEQ ID NO: 336 and SEQ ID NO: 185, respectively. In an aspect, the first gRNA molecule and the second gRNA molecule include targeting domains comprising, e.g., consisting of, SEQ ID NO: 336 and SEQ ID NO: 186, respectively. In an aspect, the first gRNA molecule and the second gRNA molecule include targeting domains comprising, e.g., consisting of, SEQ ID NO: 336 and SEQ ID NO: 337, respectively. In an aspect, the first gRNA molecule and the second gRNA molecule include targeting domains comprising, e.g., consisting of, SEQ ID NO: 337 and SEQ ID NO: 244, respectively. In an aspect, the first gRNA molecule and the second gRNA molecule include targeting domains comprising, e.g., consisting of, SEQ ID NO: 337 and SEQ ID NO: 199, respectively. In an aspect, the first gRNA molecule and the second gRNA molecule include targeting domains comprising, e.g., consisting of, SEQ ID NO: 337 and SEQ ID NO: 251, respectively. In an aspect, the first gRNA molecule and the second gRNA molecule include targeting domains comprising, e.g., consisting of, SEQ ID NO: 337 and SEQ ID NO: 250, respectively. In an aspect, the first gRNA molecule and the second gRNA molecule include targeting domains comprising, e.g., consisting of, SEQ ID NO: 337 and SEQ ID NO: 334, respectively. In an aspect, the first gRNA molecule and the second gRNA molecule include targeting domains comprising, e.g., consisting of, SEQ ID NO: 337 and SEQ ID NO: 185, respectively. In an aspect, the first gRNA molecule and the second gRNA molecule include targeting domains comprising, e.g., consisting of, SEQ ID NO: 337 and SEQ ID NO: 186, respectively. In an aspect, the first gRNA molecule and the second gRNA molecule include targeting domains comprising, e.g., consisting of, SEQ ID NO: 337 and SEQ ID NO: 336, respectively. In an aspect, the first gRNA molecule and the second gRNA molecule include targeting domains comprising, e.g., consisting of, SEQ ID NO: 337 and SEQ ID NO: 246, respectively. In an aspect, the first gRNA molecule and the second gRNA molecule include targeting domains comprising, e.g., consisting of, SEQ ID NO: 337 and SEQ ID NO: 248, respectively. In an aspect, the first gRNA molecule and the second gRNA molecule include targeting domains comprising, e.g., consisting of, SEQ ID NO: 337 and SEQ ID NO: 247, respectively. In an aspect, the first gRNA molecule and the second gRNA molecule include targeting domains comprising, e.g., consisting of, SEQ ID NO: 337 and SEQ ID NO: 245, respectively. In an aspect, the first gRNA molecule and the second gRNA molecule include targeting domains comprising, e.g., consisting of, SEQ ID NO: 337 and SEQ ID NO: 249, respectively. In an aspect, the first gRNA molecule and the second gRNA molecule include targeting domains comprising, e.g., consisting of, SEQ ID NO: 337 and SEQ ID NO: 244, respectively. In an aspect, the first gRNA molecule and the second gRNA molecule include targeting domains comprising, e.g., consisting of, SEQ ID NO: 337 and SEQ ID NO: 199, respectively. In an aspect, the first gRNA molecule and the second gRNA molecule include targeting domains comprising, e.g., consisting of, SEQ ID NO: 337 and SEQ ID NO: 251, respectively. In an aspect, the first gRNA molecule and the second gRNA molecule include targeting domains comprising, e.g., consisting of, SEQ ID NO: 337 and SEQ ID NO: 250, respectively. In an aspect, the first gRNA molecule and the second gRNA molecule include targeting domains comprising, e.g., consisting of, SEQ ID NO: 337 and SEQ ID NO: 334, respectively. In an aspect, the first gRNA molecule and the second gRNA molecule include targeting domains comprising, e.g., consisting of, SEQ ID NO: 337 and SEQ ID NO: 185, respectively. In an aspect, the first gRNA molecule and the second gRNA molecule include targeting domains comprising, e.g., consisting of, SEQ ID NO: 337 and SEQ ID NO: 186, respectively. In an aspect, the first gRNA molecule and the second gRNA molecule include targeting domains comprising, e.g., consisting of, SEQ ID NO: 337 and SEQ ID NO: 336, respectively.

In embodiments, the aspects of the invention relate to or incorporate a gRNA molecule that is complementary to a target sequence within the +58 enhancer region of the BCL11a gene which is disposed 3' to the GATA-1 binding site, e.g., 3' to the GATA1 binding site and TAL-1 binding site. In embodiments, the CRISPR system comprising a gRNA molecule described herein induces one or more indels at or near the target site. In embodiments, the indel does not comprise a nucleotide of a GATA-1 binding site and/or does not comprise a nucleotide of a TAL-1 binding site. In embodiments, the CRISPR system induces one or more frameshift indels at or near the target site, e.g., at an overall frameshift indel rate of at least about 10%, at least about 20%, at least about 30%, at least about 40%, or at least about 50%, e.g., as measured by NGS. In embodiments, the overall indel rate is at least about 70%, at least about 80%, at least about 90%, or at least about 95%, e.g., as measured by NGS.

**Table 8: Preferred Guide RNA Targeting Domains directed to the +62 Enhancer Region of the BCL11a Gene (i.e., to a BCL11a Enhancer)**

| **Id.** | **Exon/ Feature** | **Strand** | **Targeting domain** | **Locations** | **SEQ ID NO:** |
|---|---|---|---|---|---|
| CR00171 | 62 | - | AGCUCUGGAAUGAUGGCUUA | Chr2:60490354-60490376 | 278 |
| CR00172 | 62 | - | AUUGUGGAGCUCUGGAAUGA | Chr2:60490361-60490383 | 279 |
| CR00173 | 62 | - | CUGGAAUAGAAAAUUGGAGU | Chr2:60490384-60490406 | 280 |
| CR00174 | 62 | - | UGGGUACGGGGAACUAAGAC | Chr2:60490403-60490425 | 281 |
| CR00175 | 62 | + | UUAGUUCCCCGUACCCAUCA | Chr2:60490409-60490431 | 282 |
| CR00176 | 62 | - | UAUUUUCCUUGAUGGGUACG | Chr2:60490415-60490437 | 283 |
| CR00177 | 62 | - | AUAUUUUCCUUGAUGGGUAC | Chr2:60490416-60490438 | 284 |
| CR00178 | 62 | - | AAUAUUUUCCUUGAUGGGUA | Chr2:60490417-60490439 | 285 |
| CR00179 | 62 | - | GGAAGGAAAUGAGAACGGAA | Chr2:60490456-60490478 | 286 |
| CR00180 | 62 | - | AGGAAGGAAAUGAGAACGGA | Chr2:60490457-60490479 | 287 |
| CR00181 | 62 | + | AUACUUCAAGGCCUCAAUGA | Chr2:60490520-60490542 | 288 |
| CR00182 | 62 | - | GACUAGGUAGACCUUCAUUG | Chr2:60490531-60490553 | 289 |
| CR00183 | 62 | - | UUCUUCUUGCUAAGGUGACU | Chr2:60490547-60490569 | 290 |
| CR00184 | 62 | - | GAGAUUGAUUCUUCUUGCUA | Chr2:60490555-60490577 | 291 |
| CR00185 | 62 | - | GUGUUCUAUGAGGUUGGAGA | Chr2:60490580-60490602 | 292 |
| CR00186 | 62 | - | GGAUGAGUGUUCUAUGAGGU | Chr2:60490586-60490608 | 293 |
| CR00187 | 62 | - | CAUGGGAUGAGUGUUCUAUG | Chr2:60490590-60490612 | 294 |
| CR00188 | 62 | - | UGAGUCAGGGAGUGGUGCAU | Chr2:60490607-60490629 | 295 |
| CR00189 | 62 | - | AUGAGUCAGGGAGUGGUGCA | Chr2:60490608-60490630 | 296 |
| CR00190 | 62 | + | CCACUCCCUGACUCAUAUCU | Chr2:60490615-60490637 | 297 |
| CR00191 | 62 | - | UAAGGCCUAGAUAUGAGUCA | Chr2:60490620-60490642 | 298 |
| CR00192 | 62 | - | GUAAGGCCUAGAUAUGAGUC | Chr2:60490621-60490643 | 299 |
| CR00193 | 62 | + | AUAUCUAGGCCUUACAUUGC | Chr2:60490629-60490651 | 300 |
| CR00194 | 62 | - | AAAUUAAUUAGAGGCAUAGA | Chr2:60490656-60490678 | 301 |
| CR00195 | 62 | - | GAAAUUAAUUAGAGGCAUAG | Chr2:60490657-60490679 | 302 |
| CR00196 | 62 | - | GAACACAUGAAAUUAAUUAG | Chr2:60490665-60490687 | 303 |
| CR00197 | 62 | + | CCAAUGAGUUUCUUCAAUAC | Chr2:60490688-60490710 | 304 |
| CR00198 | 62 | - | CAAAUAUAAUAGAAGCAAGU | Chr2:60490721-60490743 | 305 |
| CR00199 | 62 | - | AAAUAACUUCCCUUUUAGGA | Chr2:60490821-60490843 | 306 |
| CR00200 | 62 | - | GGAAAAAUAACUUCCCUUUU | Chr2:60490825-60490847 | 307 |
| CR00201 | 62 | - | UUUUGAACAGAAAUGAUAUU | Chr2:60490846-60490868 | 308 |
| CR00202 | 62 | - | AGUUCAAGUAGAUAUCAGAA | Chr2:60490905-60490927 | 309 |
| CR00203 | 62 | - | AAGUUCAAGUAGAUAUCAGA | Chr2:60490906-60490928 | 310 |
| CR00204 | 62 | - | GGGUGGCUGUUUAAAGAGGG | Chr2:60490994-60491016 | 311 |
| CR00205 | 62 | - | GUGGGGUGGCUGUUUAAAGA | Chr2:60490997-60491019 | 312 |
| CR00206 | 62 | - | UGUGGGGUGGCUGUUUAAAG | Chr2:60490998-60491020 | 313 |
| CR00207 | 62 | + | UGCCAACCAGACUGUGCGCC | Chr2:60491051-60491073 | 314 |
| CR00208 | 62 | - | AACCUGGCGCACAGUCUGGU | Chr2:60491053-60491075 | 315 |
| CR00209 | 62 | + | AACCAGACUGUGCGCCAGGU | Chr2:60491055-60491077 | 316 |
| CR00210 | 62 | - | UACCAACCUGGCGCACAGUC | Chr2:60491057-60491079 | 317 |
| CR00211 | 62 | - | UCUGUCAGACUUUACCAACC | Chr2:60491069-60491091 | 318 |
| CR00212 | 62 | + | AUAUGUGAAGCCCAACUACG | Chr2:60491118-60491140 | 319 |
| CR00213 | 62 | - | AGUUGCACAACCACGUAGUU | Chr2:60491128-60491150 | 320 |
| CR00214 | 62 | - | GAGUUGCACAACCACGUAGU | Chr2:60491129-60491151 | 321 |
| CR00215 | 62 | + | CUAUAGCUGACUUUCAACCA | Chr2:60491151-60491173 | 322 |
| CR00216 | 62 | - | AACUUCUUUGCAGAUGACCA | Chr2:60491168-60491190 | 323 |
| CR00217 | 62 | - | UUGCAUUGAGGAUGCGCAGG | Chr2:60491199-60491221 | 324 |
| CR00218 | 62 | - | UUUUUGCAUUGAGGAUGCGC | Chr2:60491202-60491224 | 325 |
| CR00221 | 62 | + | GACCUCAUUUUGAUGCCAGA | Chr2:60491281-60491303 | 326 |
| CR00222 | 62 | - | UGCCCUCUGGCAUCAAAAUG | Chr2:60491283-60491305 | 327 |
| CR00223 | 62 | + | AUGCCAGAGGGCAGCAAACA | Chr2:60491293-60491315 | 328 |
| CR00224 | 62 | - | CUGUACUUAAUAGCUGAAGG | Chr2:60491326-60491348 | 329 |
| CR00225 | 62 | - | ACUGUACUUAAUAGCUGAAG | Chr2:60491327-60491349 | 330 |
| CR00227 | 62 | + | CAGCUAUUAAGUACAGUAAA | Chr2:60491333-60491355 | 331 |
| CR00228 | 62 | - | GAGCAUUUCAAAUGAUAGUU | Chr2:60491360-60491382 | 332 |
| CR00229 | 62 | + | CAUUUGAAAUGCUCCCGGCC | Chr2:60491369-60491391 | 333 |

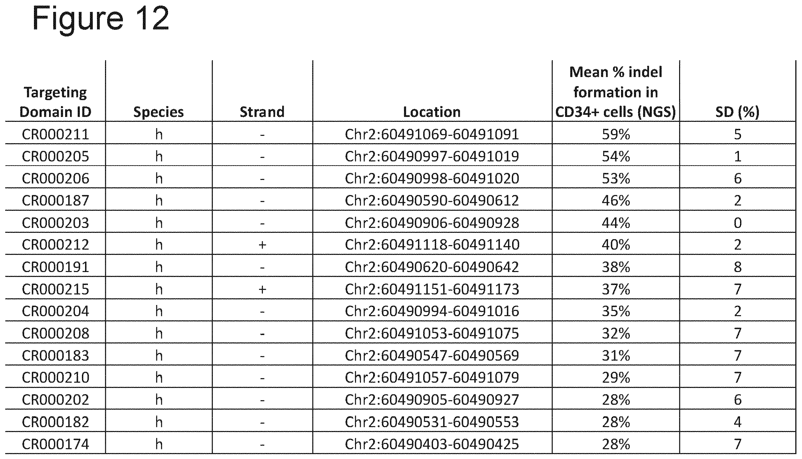

In some aspects of the invention, it is preferred that the gRNA molecule to the +62 Enhancer region of BCL11a comprise a targeting domain of a gRNA listed in Figure 12. In an aspect, the gRNA molecule includes a targeting domain comprising, e.g., consisting of, SEQ ID NO: 318. In an aspect, the gRNA molecule includes a targeting domain comprising, e.g., consisting of, SEQ ID NO: 312. In an aspect, the gRNA molecule includes a targeting domain comprising, e.g., consisting of, SEQ ID NO: 313. In an aspect, the gRNA molecule includes a targeting domain comprising, e.g., consisting of, SEQ ID NO: 294. In an aspect, the gRNA molecule includes a targeting domain comprising, e.g., consisting of, SEQ ID NO: 310. In an aspect, the gRNA molecule includes a targeting domain comprising, e.g., consisting of, SEQ ID NO: 319. In an aspect, the gRNA molecule includes a targeting domain comprising, e.g., consisting of, SEQ ID NO: 298. In an aspect, the gRNA molecule includes a targeting domain comprising, e.g., consisting of, SEQ ID NO: 322. In an aspect, the gRNA molecule includes a targeting domain comprising, e.g., consisting of, SEQ ID NO: 311. In an aspect, the gRNA molecule includes a targeting domain comprising, e.g., consisting of, SEQ ID NO: 315. In an aspect, the gRNA molecule includes a targeting domain comprising, e.g., consisting of, SEQ ID NO: 290. In an aspect, the gRNA molecule includes a targeting domain comprising, e.g., consisting of, SEQ ID NO: 317. In an aspect, the gRNA molecule includes a targeting domain comprising, e.g., consisting of, SEQ ID NO: 309. In an aspect, the gRNA molecule includes a targeting domain comprising, e.g., consisting of, SEQ ID NO: 289. In an aspect, the gRNA molecule includes a targeting domain comprising, e.g., consisting of, SEQ ID NO: 281.

In some aspects of the invention, e.g., as indicated herein, it may be beneficial to include gRNA moleucles targeting more than one, e.g., two, target sites (e.g., a first gRNA molecule and a second gRNA molecule). In some embodiments, the two target sites are both located in the +62 BCL11a enhancer region. In such aspects, any combination of more than one, e.g., two, gRNA molecules (e.g., a first gRNA molecule and a second gRNA molecule) comprising targeting domains listed in Table 8 may be used. In an aspect, the first gRNA molecule and the second gRNA molecule include targeting domains comprising, e.g., consisting of, SEQ ID NO: 318 and SEQ ID NO: 312, respectively. In an aspect, the first gRNA molecule and the second gRNA molecule include targeting domains comprising, e.g., consisting of, SEQ ID NO: 318 and SEQ ID NO: 313, respectively. In an aspect, the first gRNA molecule and the second gRNA molecule include targeting domains comprising, e.g., consisting of, SEQ ID NO: 318 and SEQ ID NO: 294, respectively. In an aspect, the first gRNA molecule and the second gRNA molecule include targeting domains comprising, e.g., consisting of, SEQ ID NO: 318 and SEQ ID NO: 310, respectively. In an aspect, the first gRNA molecule and the second gRNA molecule include targeting domains comprising, e.g., consisting of, SEQ ID NO: 318 and SEQ ID NO: 319, respectively. In an aspect, the first gRNA molecule and the second gRNA molecule include targeting domains comprising, e.g., consisting of, SEQ ID NO: 318 and SEQ ID NO: 298, respectively. In an aspect, the first gRNA molecule and the second gRNA molecule include targeting domains comprising, e.g., consisting of, SEQ ID NO: 318 and SEQ ID NO: 322, respectively. In an aspect, the first gRNA molecule and the second gRNA molecule include targeting domains comprising, e.g., consisting of, SEQ ID NO: 318 and SEQ ID NO: 311, respectively. In an aspect, the first gRNA molecule and the second gRNA molecule include targeting domains comprising, e.g., consisting of, SEQ ID NO: 318 and SEQ ID NO: 315, respectively. In an aspect, the first gRNA molecule and the second gRNA molecule include targeting domains comprising, e.g., consisting of, SEQ ID NO: 318 and SEQ ID NO: 290, respectively. In an aspect, the first gRNA molecule and the second gRNA molecule include targeting domains comprising, e.g., consisting of, SEQ ID NO: 318 and SEQ ID NO: 317, respectively. In an aspect, the first gRNA molecule and the second gRNA molecule include targeting domains comprising, e.g., consisting of, SEQ ID NO: 318 and SEQ ID NO: 309, respectively. In an aspect, the first gRNA molecule and the second gRNA molecule include targeting domains comprising, e.g., consisting of, SEQ ID NO: 318 and SEQ ID NO: 289, respectively. In an aspect, the first gRNA molecule and the second gRNA molecule include targeting domains comprising, e.g., consisting of, SEQ ID NO: 318 and SEQ ID NO: 281, respectively. In an aspect, the first gRNA molecule and the second gRNA molecule include targeting domains comprising, e.g., consisting of, SEQ ID NO: 312 and SEQ ID NO: 313, respectively. In an aspect, the first gRNA molecule and the second gRNA molecule include targeting domains comprising, e.g., consisting of, SEQ ID NO: 312 and SEQ ID NO: 294, respectively. In an aspect, the first gRNA molecule and the second gRNA molecule include targeting domains comprising, e.g., consisting of, SEQ ID NO: 312 and SEQ ID NO: 310, respectively. In an aspect, the first gRNA molecule and the second gRNA molecule include targeting domains comprising, e.g., consisting of, SEQ ID NO: 312 and SEQ ID NO: 319, respectively. In an aspect, the first gRNA molecule and the second gRNA molecule include targeting domains comprising, e.g., consisting of, SEQ ID NO: 312 and SEQ ID NO: 298, respectively. In an aspect, the first gRNA molecule and the second gRNA molecule include targeting domains comprising, e.g., consisting of, SEQ ID NO: 312 and SEQ ID NO: 322, respectively. In an aspect, the first gRNA molecule and the second gRNA molecule include targeting domains comprising, e.g., consisting of, SEQ ID NO: 312 and SEQ ID NO: 311, respectively. In an aspect, the first gRNA molecule and the second gRNA molecule include targeting domains comprising, e.g., consisting of, SEQ ID NO: 312 and SEQ ID NO: 315, respectively. In an aspect, the first gRNA molecule and the second gRNA molecule include targeting domains comprising, e.g., consisting of, SEQ ID NO: 312 and SEQ ID NO: 290, respectively. In an aspect, the first gRNA molecule and the second gRNA molecule include targeting domains comprising, e.g., consisting of, SEQ ID NO: 312 and SEQ ID NO: 317, respectively. In an aspect, the first gRNA molecule and the second gRNA molecule include targeting domains comprising, e.g., consisting of, SEQ ID NO: 312 and SEQ ID NO: 309, respectively. In an aspect, the first gRNA molecule and the second gRNA molecule include targeting domains comprising, e.g., consisting of, SEQ ID NO: 312 and SEQ ID NO: 289, respectively. In an aspect, the first gRNA molecule and the second gRNA molecule include targeting domains comprising, e.g., consisting of, SEQ ID NO: 312 and SEQ ID NO: 281, respectively. In an aspect, the first gRNA molecule and the second gRNA molecule include targeting domains comprising, e.g., consisting of, SEQ ID NO: 313 and SEQ ID NO: 312, respectively. In an aspect, the first gRNA molecule and the second gRNA molecule include targeting domains comprising, e.g., consisting of, SEQ ID NO: 313 and SEQ ID NO: 294, respectively. In an aspect, the first gRNA molecule and the second gRNA molecule include targeting domains comprising, e.g., consisting of, SEQ ID NO: 313 and SEQ ID NO: 310, respectively. In an aspect, the first gRNA molecule and the second gRNA molecule include targeting domains comprising, e.g., consisting of, SEQ ID NO: 313 and SEQ ID NO: 319, respectively. In an aspect, the first gRNA molecule and the second gRNA molecule include targeting domains comprising, e.g., consisting of, SEQ ID NO: 313 and SEQ ID NO: 298, respectively. In an aspect, the first gRNA molecule and the second gRNA molecule include targeting domains comprising, e.g., consisting of, SEQ ID NO: 313 and SEQ ID NO: 322, respectively. In an aspect, the first gRNA molecule and the second gRNA molecule include targeting domains comprising, e.g., consisting of, SEQ ID NO: 313 and SEQ ID NO: 311, respectively. In an aspect, the first gRNA molecule and the second gRNA molecule include targeting domains comprising, e.g., consisting of, SEQ ID NO: 313 and SEQ ID NO: 315, respectively. In an aspect, the first gRNA molecule and the second gRNA molecule include targeting domains comprising, e.g., consisting of, SEQ ID NO: 313 and SEQ ID NO: 290, respectively. In an aspect, the first gRNA molecule and the second gRNA molecule include targeting domains comprising, e.g., consisting of, SEQ ID NO: 313 and SEQ ID NO: 317, respectively. In an aspect, the first gRNA molecule and the second gRNA molecule include targeting domains comprising, e.g., consisting of, SEQ ID NO: 313 and SEQ ID NO: 309, respectively. In an aspect, the first gRNA molecule and the second gRNA molecule include targeting domains comprising, e.g., consisting of, SEQ ID NO: 313 and SEQ ID NO: 289, respectively. In an aspect, the first gRNA molecule and the second gRNA molecule include targeting domains comprising, e.g., consisting of, SEQ ID NO: 313 and SEQ ID NO: 281, respectively. In an aspect, the first gRNA molecule and the second gRNA molecule include targeting domains comprising, e.g., consisting of, SEQ ID NO: 294 and SEQ ID NO: 312, respectively. In an aspect, the first gRNA molecule and the second gRNA molecule include targeting domains comprising, e.g., consisting of, SEQ ID NO: 294 and SEQ ID NO: 313, respectively. In an aspect, the first gRNA molecule and the second gRNA molecule include targeting domains comprising, e.g., consisting of, SEQ ID NO: 294 and SEQ ID NO: 310, respectively. In an aspect, the first gRNA molecule and the second gRNA molecule include targeting domains comprising, e.g., consisting of, SEQ ID NO: 294 and SEQ ID NO: 319, respectively. In an aspect, the first gRNA molecule and the second gRNA molecule include targeting domains comprising, e.g., consisting of, SEQ ID NO: 294 and SEQ ID NO: 298, respectively. In an aspect, the first gRNA molecule and the second gRNA molecule include targeting domains comprising, e.g., consisting of, SEQ ID NO: 294 and SEQ ID NO: 322, respectively. In an aspect, the first gRNA molecule and the second gRNA molecule include targeting domains comprising, e.g., consisting of, SEQ ID NO: 294 and SEQ ID NO: 311, respectively. In an aspect, the first gRNA molecule and the second gRNA molecule include targeting domains comprising, e.g., consisting of, SEQ ID NO: 294 and SEQ ID NO: 315, respectively. In an aspect, the first gRNA molecule and the second gRNA molecule include targeting domains comprising, e.g., consisting of, SEQ ID NO: 294 and SEQ ID NO: 290, respectively. In an aspect, the first gRNA molecule and the second gRNA molecule include targeting domains comprising, e.g., consisting of, SEQ ID NO: 294 and SEQ ID NO: 317, respectively. In an aspect, the first gRNA molecule and the second gRNA molecule include targeting domains comprising, e.g., consisting of, SEQ ID NO: 294 and SEQ ID NO: 309, respectively. In an aspect, the first gRNA molecule and the second gRNA molecule include targeting domains comprising, e.g., consisting of, SEQ ID NO: 294 and SEQ ID NO: 289, respectively. In an aspect, the first gRNA molecule and the second gRNA molecule include targeting domains comprising, e.g., consisting of, SEQ ID NO: 294 and SEQ ID NO: 281, respectively. In an aspect, the first gRNA molecule and the second gRNA molecule include targeting domains comprising, e.g., consisting of, SEQ ID NO: 310 and SEQ ID NO: 312, respectively. In an aspect, the first gRNA molecule and the second gRNA molecule include targeting domains comprising, e.g., consisting of, SEQ ID NO: 310 and SEQ ID NO: 313, respectively. In an aspect, the first gRNA molecule and the second gRNA molecule include targeting domains comprising, e.g., consisting of, SEQ ID NO: 310 and SEQ ID NO: 294, respectively. In an aspect, the first gRNA molecule and the second gRNA molecule include targeting domains comprising, e.g., consisting of, SEQ ID NO: 310 and SEQ ID NO: 319, respectively. In an aspect, the first gRNA molecule and the second gRNA molecule include targeting domains comprising, e.g., consisting of, SEQ ID NO: 310 and SEQ ID NO: 298, respectively. In an aspect, the first gRNA molecule and the second gRNA molecule include targeting domains comprising, e.g., consisting of, SEQ ID NO: 310 and SEQ ID NO: 322, respectively. In an aspect, the first gRNA molecule and the second gRNA molecule include targeting domains comprising, e.g., consisting of, SEQ ID NO: 310 and SEQ ID NO: 311, respectively. In an aspect, the first gRNA molecule and the second gRNA molecule include targeting domains comprising, e.g., consisting of, SEQ ID NO: 310 and SEQ ID NO: 315, respectively. In an aspect, the first gRNA molecule and the second gRNA molecule include targeting domains comprising, e.g., consisting of, SEQ ID NO: 310 and SEQ ID NO: 290, respectively. In an aspect, the first gRNA molecule and the second gRNA molecule include targeting domains comprising, e.g., consisting of, SEQ ID NO: 310 and SEQ ID NO: 317, respectively. In an aspect, the first gRNA molecule and the second gRNA molecule include targeting domains comprising, e.g., consisting of, SEQ ID NO: 310 and SEQ ID NO: 309, respectively. In an aspect, the first gRNA molecule and the second gRNA molecule include targeting domains comprising, e.g., consisting of, SEQ ID NO: 310 and SEQ ID NO: 289, respectively. In an aspect, the first gRNA molecule and the second gRNA molecule include targeting domains comprising, e.g., consisting of, SEQ ID NO: 310 and SEQ ID NO: 281, respectively. In an aspect, the first gRNA molecule and the second gRNA molecule include targeting domains comprising, e.g., consisting of, SEQ ID NO: 319 and SEQ ID NO: 312, respectively. In an aspect, the first gRNA molecule and the second gRNA molecule include targeting domains comprising, e.g., consisting of, SEQ ID NO: 319 and SEQ ID NO: 313, respectively. In an aspect, the first gRNA molecule and the second gRNA molecule include targeting domains comprising, e.g., consisting of, SEQ ID NO: 319 and SEQ ID NO: 294, respectively. In an aspect, the first gRNA molecule and the second gRNA molecule include targeting domains comprising, e.g., consisting of, SEQ ID NO: 319 and SEQ ID NO: 310, respectively. In an aspect, the first gRNA molecule and the second gRNA molecule include targeting domains comprising, e.g., consisting of, SEQ ID NO: 319 and SEQ ID NO: 298, respectively. In an aspect, the first gRNA molecule and the second gRNA molecule include targeting domains comprising, e.g., consisting of, SEQ ID NO: 319 and SEQ ID NO: 322, respectively. In an aspect, the first gRNA molecule and the second gRNA molecule include targeting domains comprising, e.g., consisting of, SEQ ID NO: 319 and SEQ ID NO: 311, respectively. In an aspect, the first gRNA molecule and the second gRNA molecule include targeting domains comprising, e.g., consisting of, SEQ ID NO: 319 and SEQ ID NO: 315, respectively. In an aspect, the first gRNA molecule and the second gRNA molecule include targeting domains comprising, e.g., consisting of, SEQ ID NO: 319 and SEQ ID NO: 290, respectively. In an aspect, the first gRNA molecule and the second gRNA molecule include targeting domains comprising, e.g., consisting of, SEQ ID NO: 319 and SEQ ID NO: 317, respectively. In an aspect, the first gRNA molecule and the second gRNA molecule include targeting domains comprising, e.g., consisting of, SEQ ID NO: 319 and SEQ ID NO: 309, respectively. In an aspect, the first gRNA molecule and the second gRNA molecule include targeting domains comprising, e.g., consisting of, SEQ ID NO: 319 and SEQ ID NO: 289, respectively. In an aspect, the first gRNA molecule and the second gRNA molecule include targeting domains comprising, e.g., consisting of, SEQ ID NO: 319 and SEQ ID NO: 281, respectively. In an aspect, the first gRNA molecule and the second gRNA molecule include targeting domains comprising, e.g., consisting of, SEQ ID NO: 298 and SEQ ID NO: 312, respectively. In an aspect, the first gRNA molecule and the second gRNA molecule include targeting domains comprising, e.g., consisting of, SEQ ID NO: 298 and SEQ ID NO: 313, respectively. In an aspect, the first gRNA molecule and the second gRNA molecule include targeting domains comprising, e.g., consisting of, SEQ ID NO: 298 and SEQ ID NO: 294, respectively. In an aspect, the first gRNA molecule and the second gRNA molecule include targeting domains comprising, e.g., consisting of, SEQ ID NO: 298 and SEQ ID NO: 310, respectively. In an aspect, the first gRNA molecule and the second gRNA molecule include targeting domains comprising, e.g., consisting of, SEQ ID NO: 298 and SEQ ID NO: 319, respectively. In an aspect, the first gRNA molecule and the second gRNA molecule include targeting domains comprising, e.g., consisting of, SEQ ID NO: 298 and SEQ ID NO: 322, respectively. In an aspect, the first gRNA molecule and the second gRNA molecule include targeting domains comprising, e.g., consisting of, SEQ ID NO: 298 and SEQ ID NO: 311, respectively. In an aspect, the first gRNA molecule and the second gRNA molecule include targeting domains comprising, e.g., consisting of, SEQ ID NO: 298 and SEQ ID NO: 315, respectively. In an aspect, the first gRNA molecule and the second gRNA molecule include targeting domains comprising, e.g., consisting of, SEQ ID NO: 298 and SEQ ID NO: 290, respectively. In an aspect, the first gRNA molecule and the second gRNA molecule include targeting domains comprising, e.g., consisting of, SEQ ID NO: 298 and SEQ ID NO: 317, respectively. In an aspect, the first gRNA molecule and the second gRNA molecule include targeting domains comprising, e.g., consisting of, SEQ ID NO: 298 and SEQ ID NO: 309, respectively. In an aspect, the first gRNA molecule and the second gRNA molecule include targeting domains comprising, e.g., consisting of, SEQ ID NO: 298 and SEQ ID NO: 289, respectively. In an aspect, the first gRNA molecule and the second gRNA molecule include targeting domains comprising, e.g., consisting of, SEQ ID NO: 298 and SEQ ID NO: 281, respectively. In an aspect, the first gRNA molecule and the second gRNA molecule include targeting domains comprising, e.g., consisting of, SEQ ID NO: 322 and SEQ ID NO: 312, respectively. In an aspect, the first gRNA molecule and the second gRNA molecule include targeting domains comprising, e.g., consisting of, SEQ ID NO: 322 and SEQ ID NO: 313, respectively. In an aspect, the first gRNA molecule and the second gRNA molecule include targeting domains comprising, e.g., consisting of, SEQ ID NO: 322 and SEQ ID NO: 294, respectively. In an aspect, the first gRNA molecule and the second gRNA molecule include targeting domains comprising, e.g., consisting of, SEQ ID NO: 322 and SEQ ID NO: 310, respectively. In an aspect, the first gRNA molecule and the second gRNA molecule include targeting domains comprising, e.g., consisting of, SEQ ID NO: 322 and SEQ ID NO: 319, respectively. In an aspect, the first gRNA molecule and the second gRNA molecule include targeting domains comprising, e.g., consisting of, SEQ ID NO: 322 and SEQ ID NO: 298, respectively. In an aspect, the first gRNA molecule and the second gRNA molecule include targeting domains comprising, e.g., consisting of, SEQ ID NO: 322 and SEQ ID NO: 311, respectively. In an aspect, the first gRNA molecule and the second gRNA molecule include targeting domains comprising, e.g., consisting of, SEQ ID NO: 322 and SEQ ID NO: 315, respectively. In an aspect, the first gRNA molecule and the second gRNA molecule include targeting domains comprising, e.g., consisting of, SEQ ID NO: 322 and SEQ ID NO: 290, respectively. In an aspect, the first gRNA molecule and the second gRNA molecule include targeting domains comprising, e.g., consisting of, SEQ ID NO: 322 and SEQ ID NO: 317, respectively. In an aspect, the first gRNA molecule and the second gRNA molecule include targeting domains comprising, e.g., consisting of, SEQ ID NO: 322 and SEQ ID NO: 309, respectively. In an aspect, the first gRNA molecule and the second gRNA molecule include targeting domains comprising, e.g., consisting of, SEQ ID NO: 322 and SEQ ID NO: 289, respectively. In an aspect, the first gRNA molecule and the second gRNA molecule include targeting domains comprising, e.g., consisting of, SEQ ID NO: 322 and SEQ ID NO: 281, respectively. In an aspect, the first gRNA molecule and the second gRNA molecule include targeting domains comprising, e.g., consisting of, SEQ ID NO: 311 and SEQ ID NO: 312, respectively. In an aspect, the first gRNA molecule and the second gRNA molecule include targeting domains comprising, e.g., consisting of, SEQ ID NO: 311 and SEQ ID NO: 313, respectively. In an aspect, the first gRNA molecule and the second gRNA molecule include targeting domains comprising, e.g., consisting of, SEQ ID NO: 311 and SEQ ID NO: 294, respectively. In an aspect, the first gRNA molecule and the second gRNA molecule include targeting domains comprising, e.g., consisting of, SEQ ID NO: 311 and SEQ ID NO: 310, respectively. In an aspect, the first gRNA molecule and the second gRNA molecule include targeting domains comprising, e.g., consisting of, SEQ ID NO: 311 and SEQ ID NO: 319, respectively. In an aspect, the first gRNA molecule and the second gRNA molecule include targeting domains comprising, e.g., consisting of, SEQ ID NO: 311 and SEQ ID NO: 298, respectively. In an aspect, the first gRNA molecule and the second gRNA molecule include targeting domains comprising, e.g., consisting of, SEQ ID NO: 311 and SEQ ID NO: 322, respectively. In an aspect, the first gRNA molecule and the second gRNA molecule include targeting domains comprising, e.g., consisting of, SEQ ID NO: 311 and SEQ ID NO: 315, respectively. In an aspect, the first gRNA molecule and the second gRNA molecule include targeting domains comprising, e.g., consisting of, SEQ ID NO: 311 and SEQ ID NO: 290, respectively. In an aspect, the first gRNA molecule and the second gRNA molecule include targeting domains comprising, e.g., consisting of, SEQ ID NO: 311 and SEQ ID NO: 317, respectively. In an aspect, the first gRNA molecule and the second gRNA molecule include targeting domains comprising, e.g., consisting of, SEQ ID NO: 311 and SEQ ID NO: 309, respectively. In an aspect, the first gRNA molecule and the second gRNA molecule include targeting domains comprising, e.g., consisting of, SEQ ID NO: 311 and SEQ ID NO: 289, respectively. In an aspect, the first gRNA molecule and the second gRNA molecule include targeting domains comprising, e.g., consisting of, SEQ ID NO: 311 and SEQ ID NO: 281, respectively. In an aspect, the first gRNA molecule and the second gRNA molecule include targeting domains comprising, e.g., consisting of, SEQ ID NO: 315 and SEQ ID NO: 312, respectively. In an aspect, the first gRNA molecule and the second gRNA molecule include targeting domains comprising, e.g., consisting of, SEQ ID NO: 315 and SEQ ID NO: 313, respectively. In an aspect, the first gRNA molecule and the second gRNA molecule include targeting domains comprising, e.g., consisting of, SEQ ID NO: 315 and SEQ ID NO: 294, respectively. In an aspect, the first gRNA molecule and the second gRNA molecule include targeting domains comprising, e.g., consisting of, SEQ ID NO: 315 and SEQ ID NO: 310, respectively. In an aspect, the first gRNA molecule and the second gRNA molecule include targeting domains comprising, e.g., consisting of, SEQ ID NO: 315 and SEQ ID NO: 319, respectively. In an aspect, the first gRNA molecule and the second gRNA molecule include targeting domains comprising, e.g., consisting of, SEQ ID NO: 315 and SEQ ID NO: 298, respectively. In an aspect, the first gRNA molecule and the second gRNA molecule include targeting domains comprising, e.g., consisting of, SEQ ID NO: 315 and SEQ ID NO: 322, respectively. In an aspect, the first gRNA molecule and the second gRNA molecule include targeting domains comprising, e.g., consisting of, SEQ ID NO: 315 and SEQ ID NO: 311, respectively. In an aspect, the first gRNA molecule and the second gRNA molecule include targeting domains comprising, e.g., consisting of, SEQ ID NO: 315 and SEQ ID NO: 290, respectively. In an aspect, the first gRNA molecule and the second gRNA molecule include targeting domains comprising, e.g., consisting of, SEQ ID NO: 315 and SEQ ID NO: 317, respectively. In an aspect, the first gRNA molecule and the second gRNA molecule include targeting domains comprising, e.g., consisting of, SEQ ID NO: 315 and SEQ ID NO: 309, respectively. In an aspect, the first gRNA molecule and the second gRNA molecule include targeting domains comprising, e.g., consisting of, SEQ ID NO: 315 and SEQ ID NO: 289, respectively. In an aspect, the first gRNA molecule and the second gRNA molecule include targeting domains comprising, e.g., consisting of, SEQ ID NO: 315 and SEQ ID NO: 281, respectively. In an aspect, the first gRNA molecule and the second gRNA molecule include targeting domains comprising, e.g., consisting of, SEQ ID NO: 290 and SEQ ID NO: 312, respectively. In an aspect, the first gRNA molecule and the second gRNA molecule include targeting domains comprising, e.g., consisting of, SEQ ID NO: 290 and SEQ ID NO: 313, respectively. In an aspect, the first gRNA molecule and the second gRNA molecule include targeting domains comprising, e.g., consisting of, SEQ ID NO: 290 and SEQ ID NO: 294, respectively. In an aspect, the first gRNA molecule and the second gRNA molecule include targeting domains comprising, e.g., consisting of, SEQ ID NO: 290 and SEQ ID NO: 310, respectively. In an aspect, the first gRNA molecule and the second gRNA molecule include targeting domains comprising, e.g., consisting of, SEQ ID NO: 290 and SEQ ID NO: 319, respectively. In an aspect, the first gRNA molecule and the second gRNA molecule include targeting domains comprising, e.g., consisting of, SEQ ID NO: 290 and SEQ ID NO: 298, respectively. In an aspect, the first gRNA molecule and the second gRNA molecule include targeting domains comprising, e.g., consisting of, SEQ ID NO: 290 and SEQ ID NO: 322, respectively. In an aspect, the first gRNA molecule and the second gRNA molecule include targeting domains comprising, e.g., consisting of, SEQ ID NO: 290 and SEQ ID NO: 311, respectively. In an aspect, the first gRNA molecule and the second gRNA molecule include targeting domains comprising, e.g., consisting of, SEQ ID NO: 290 and SEQ ID NO: 315, respectively. In an aspect, the first gRNA molecule and the second gRNA molecule include targeting domains comprising, e.g., consisting of, SEQ ID NO: 290 and SEQ ID NO: 317, respectively. In an aspect, the first gRNA molecule and the second gRNA molecule include targeting domains comprising, e.g., consisting of, SEQ ID NO: 290 and SEQ ID NO: 309, respectively. In an aspect, the first gRNA molecule and the second gRNA molecule include targeting domains comprising, e.g., consisting of, SEQ ID NO: 290 and SEQ ID NO: 289, respectively. In an aspect, the first gRNA molecule and the second gRNA molecule include targeting domains comprising, e.g., consisting of, SEQ ID NO: 290 and SEQ ID NO: 281, respectively. In an aspect, the first gRNA molecule and the second gRNA molecule include targeting domains comprising, e.g., consisting of, SEQ ID NO: 317 and SEQ ID NO: 312, respectively. In an aspect, the first gRNA molecule and the second gRNA molecule include targeting domains comprising, e.g., consisting of, SEQ ID NO: 317 and SEQ ID NO: 313, respectively. In an aspect, the first gRNA molecule and the second gRNA molecule include targeting domains comprising, e.g., consisting of, SEQ ID NO: 317 and SEQ ID NO: 294, respectively. In an aspect, the first gRNA molecule and the second gRNA molecule include targeting domains comprising, e.g., consisting of, SEQ ID NO: 317 and SEQ ID NO: 310, respectively. In an aspect, the first gRNA molecule and the second gRNA molecule include targeting domains comprising, e.g., consisting of, SEQ ID NO: 317 and SEQ ID NO: 319, respectively. In an aspect, the first gRNA molecule and the second gRNA molecule include targeting domains comprising, e.g., consisting of, SEQ ID NO: 317 and SEQ ID NO: 298, respectively. In an aspect, the first gRNA molecule and the second gRNA molecule include targeting domains comprising, e.g., consisting of, SEQ ID NO: 317 and SEQ ID NO: 322, respectively. In an aspect, the first gRNA molecule and the second gRNA molecule include targeting domains comprising, e.g., consisting of, SEQ ID NO: 317 and SEQ ID NO: 311, respectively. In an aspect, the first gRNA molecule and the second gRNA molecule include targeting domains comprising, e.g., consisting of, SEQ ID NO: 317 and SEQ ID NO: 315, respectively. In an aspect, the first gRNA molecule and the second gRNA molecule include targeting domains comprising, e.g., consisting of, SEQ ID NO: 317 and SEQ ID NO: 290, respectively. In an aspect, the first gRNA molecule and the second gRNA molecule include targeting domains comprising, e.g., consisting of, SEQ ID NO: 317 and SEQ ID NO: 309, respectively. In an aspect, the first gRNA molecule and the second gRNA molecule include targeting domains comprising, e.g., consisting of, SEQ ID NO: 317 and SEQ ID NO: 289, respectively. In an aspect, the first gRNA molecule and the second gRNA molecule include targeting domains comprising, e.g., consisting of, SEQ ID NO: 317 and SEQ ID NO: 281, respectively. In an aspect, the first gRNA molecule and the second gRNA molecule include targeting domains comprising, e.g., consisting of, SEQ ID NO: 309 and SEQ ID NO: 312, respectively. In an aspect, the first gRNA molecule and the second gRNA molecule include targeting domains comprising, e.g., consisting of, SEQ ID NO: 309 and SEQ ID NO: 313, respectively. In an aspect, the first gRNA molecule and the second gRNA molecule include targeting domains comprising, e.g., consisting of, SEQ ID NO: 309 and SEQ ID NO: 294, respectively. In an aspect, the first gRNA molecule and the second gRNA molecule include targeting domains comprising, e.g., consisting of, SEQ ID NO: 309 and SEQ ID NO: 310, respectively. In an aspect, the first gRNA molecule and the second gRNA molecule include targeting domains comprising, e.g., consisting of, SEQ ID NO: 309 and SEQ ID NO: 319, respectively. In an aspect, the first gRNA molecule and the second gRNA molecule include targeting domains comprising, e.g., consisting of, SEQ ID NO: 309 and SEQ ID NO: 298, respectively. In an aspect, the first gRNA molecule and the second gRNA molecule include targeting domains comprising, e.g., consisting of, SEQ ID NO: 309 and SEQ ID NO: 322, respectively. In an aspect, the first gRNA molecule and the second gRNA molecule include targeting domains comprising, e.g., consisting of, SEQ ID NO: 309 and SEQ ID NO: 311, respectively. In an aspect, the first gRNA molecule and the second gRNA molecule include targeting domains comprising, e.g., consisting of, SEQ ID NO: 309 and SEQ ID NO: 315, respectively. In an aspect, the first gRNA molecule and the second gRNA molecule include targeting domains comprising, e.g., consisting of, SEQ ID NO: 309 and SEQ ID NO: 290, respectively. In an aspect, the first gRNA molecule and the second gRNA molecule include targeting domains comprising, e.g., consisting of, SEQ ID NO: 309 and SEQ ID NO: 317, respectively. In an aspect, the first gRNA molecule and the second gRNA molecule include targeting domains comprising, e.g., consisting of, SEQ ID NO: 309 and SEQ ID NO: 289, respectively. In an aspect, the first gRNA molecule and the second gRNA molecule include targeting domains comprising, e.g., consisting of, SEQ ID NO: 309 and SEQ ID NO: 281, respectively. In an aspect, the first gRNA molecule and the second gRNA molecule include targeting domains comprising, e.g., consisting of, SEQ ID NO: 289 and SEQ ID NO: 312, respectively. In an aspect, the first gRNA molecule and the second gRNA molecule include targeting domains comprising, e.g., consisting of, SEQ ID NO: 289 and SEQ ID NO: 313, respectively. In an aspect, the first gRNA molecule and the second gRNA molecule include targeting domains comprising, e.g., consisting of, SEQ ID NO: 289 and SEQ ID NO: 294, respectively. In an aspect, the first gRNA molecule and the second gRNA molecule include targeting domains comprising, e.g., consisting of, SEQ ID NO: 289 and SEQ ID NO: 310, respectively. In an aspect, the first gRNA molecule and the second gRNA molecule include targeting domains comprising, e.g., consisting of, SEQ ID NO: 289 and SEQ ID NO: 319, respectively. In an aspect, the first gRNA molecule and the second gRNA molecule include targeting domains comprising, e.g., consisting of, SEQ ID NO: 289 and SEQ ID NO: 298, respectively. In an aspect, the first gRNA molecule and the second gRNA molecule include targeting domains comprising, e.g., consisting of, SEQ ID NO: 289 and SEQ ID NO: 322, respectively. In an aspect, the first gRNA molecule and the second gRNA molecule include targeting domains comprising, e.g., consisting of, SEQ ID NO: 289 and SEQ ID NO: 311, respectively. In an aspect, the first gRNA molecule and the second gRNA molecule include targeting domains comprising, e.g., consisting of, SEQ ID NO: 289 and SEQ ID NO: 315, respectively. In an aspect, the first gRNA molecule and the second gRNA molecule include targeting domains comprising, e.g., consisting of, SEQ ID NO: 289 and SEQ ID NO: 290, respectively. In an aspect, the first gRNA molecule and the second gRNA molecule include targeting domains comprising, e.g., consisting of, SEQ ID NO: 289 and SEQ ID NO: 317, respectively. In an aspect, the first gRNA molecule and the second gRNA molecule include targeting domains comprising, e.g., consisting of, SEQ ID NO: 289 and SEQ ID NO: 309, respectively. In an aspect, the first gRNA molecule and the second gRNA molecule include targeting domains comprising, e.g., consisting of, SEQ ID NO: 289 and SEQ ID NO: 281, respectively. In an aspect, the first gRNA molecule and the second gRNA molecule include targeting domains comprising, e.g., consisting of, SEQ ID NO: 281 and SEQ ID NO: 312, respectively. In an aspect, the first gRNA molecule and the second gRNA molecule include targeting domains comprising, e.g., consisting of, SEQ ID NO: 281 and SEQ ID NO: 313, respectively. In an aspect, the first gRNA molecule and the second gRNA molecule include targeting domains comprising, e.g., consisting of, SEQ ID NO: 281 and SEQ ID NO: 294, respectively. In an aspect, the first gRNA molecule and the second gRNA molecule include targeting domains comprising, e.g., consisting of, SEQ ID NO: 281 and SEQ ID NO: 310, respectively. In an aspect, the first gRNA molecule and the second gRNA molecule include targeting domains comprising, e.g., consisting of, SEQ ID NO: 281 and SEQ ID NO: 319, respectively. In an aspect, the first gRNA molecule and the second gRNA molecule include targeting domains comprising, e.g., consisting of, SEQ ID NO: 281 and SEQ ID NO: 298, respectively. In an aspect, the first gRNA molecule and the second gRNA molecule include targeting domains comprising, e.g., consisting of, SEQ ID NO: 281 and SEQ ID NO: 322, respectively. In an aspect, the first gRNA molecule and the second gRNA molecule include targeting domains comprising, e.g., consisting of, SEQ ID NO: 281 and SEQ ID NO: 311, respectively. In an aspect, the first gRNA molecule and the second gRNA molecule include targeting domains comprising, e.g., consisting of, SEQ ID NO: 281 and SEQ ID NO: 315, respectively. In an aspect, the first gRNA molecule and the second gRNA molecule include targeting domains comprising, e.g., consisting of, SEQ ID NO: 281 and SEQ ID NO: 290, respectively. In an aspect, the first gRNA molecule and the second gRNA molecule include targeting domains comprising, e.g., consisting of, SEQ ID NO: 281 and SEQ ID NO: 317, respectively. In an aspect, the first gRNA molecule and the second gRNA molecule include targeting domains comprising, e.g., consisting of, SEQ ID NO: 281 and SEQ ID NO: 309, respectively. In an aspect, the first gRNA molecule and the second gRNA molecule include targeting domains comprising, e.g., consisting of, SEQ ID NO: 281 and SEQ ID NO: 289, respectively.

**Table 9. Preferred Guide RNA Targeting Domains directed to the +55 Enhancer Region of the BCL11a Gene (i.e., to a BCL11a Enhancer)**

| **ID** | **Species** | **Strand** | **Location** | **Targeting Domain Sequence** | **SEQ ID NO:** |
|---|---|---|---|---|---|
| CR002142 | h | + | Chr2:60498031 -60498053 | CCUGGCAGACCCUCAAGAGC | 1596 |
| CR002143 | h | - | Chr2:60498031 -60498053 | CCUGGCAGACCCUCAAGAGC | 1597 |
| CR002144 | h | + | Chr2:60498032 -60498054 | CUGGCAGACCCUCAAGAGCA | 1598 |
| CR002145 | h | + | Chr2:60498033 -60498055 | UGGCAGACCCUCAAGAGCAG | 1599 |
| CR002146 | h | - | Chr2:60498040 -60498062 | CCCUCAAGAGCAGGGGUCUU | 1600 |
| CR002147 | h | - | Chr2:60498041 -60498063 | CCUCAAGAGCAGGGGUCUUC | 1601 |
| CR001262 | h | + | Chr1:55039155 -55039177 | UAAGGCCAGUGGAAAGAAUU | 1602 |
| CR002148 | h | + | Chr2:60498045 -60498067 | AAGAGCAGGGGUCUUCUCUU | 1603 |
| CR002149 | h | + | Chr2:60498046 -60498068 | AGAGCAGGGGUCUUCUCUUU | 1604 |
| CR002150 | h | + | Chr2:60498047 -60498069 | GAGCAGGGGUCUUCUCUUUG | 1605 |
| CR002151 | h | + | Chr2:60498048 -60498070 | AGCAGGGGUCUUCUCUUUGG | 1606 |
| CR002152 | h | + | Chr2:60498051 -60498073 | AGGGGUCUUCUCUUUGGGGG | 1607 |
| CR002153 | h | + | Chr2:60498068 -60498090 | GGGAGGACAUCACUCUUAGC | 1608 |
| CR002154 | h | + | Chr2:60498069 -60498091 | GGAGGACAUCACUCUUAGCA | 1609 |
| CR001261 | h | - | Chr1:55039269 -55039291 | GCCAGACUCCAAGUUCUGCC | 1610 |
| CR002155 | h | + | Chr2:60498073 | GACAUCACUCUUAGCAGGGC | 1611 |
| | | | -60498095 | | |
| CR002156 | h | + | Chr2:60498074 -60498096 | ACAUCACUCUUAGCAGGGCU | 1612 |
| CR002157 | h | + | Chr2:60498075 -60498097 | CAUCACUCUUAGCAGGGCUG | 1613 |
| CR002158 | h | + | Chr2:60498091 -60498113 | GCUGGGGUGAGUCAAAAGUC | 1614 |
| CR002159 | h | + | Chr2:60498092 -60498114 | CUGGGGUGAGUCAAAAGUCU | 1615 |
| CR002160 | h | + | Chr2:60498099 -60498121 | GAGUCAAAAGUCUGGGAGAA | 1616 |
| CR002161 | h | + | Chr2:60498102 -60498124 | UCAAAAGUCUGGGAGAAUGG | 1617 |
| CR002162 | h | + | Chr2:60498107 -60498129 | AGUCUGGGAGAAUGGAGGUG | 1618 |
| CR002163 | h | + | Chr2:60498110 -60498132 | CUGGGAGAAUGGAGGUGUGG | 1619 |
| CR002164 | h | + | Chr2:60498111 -60498133 | UGGGAGAAUGGAGGUGUGGA | 1620 |
| CR002165 | h | + | Chr2:60498112 -60498134 | GGGAGAAUGGAGGUGUGGAG | 1621 |
| CR002166 | h | + | Chr2:60498120 -60498142 | GGAGGUGUGGAGGGGAUAAC | 1622 |
| CR001263 | h | + | Chr1:55039180 -55039202 | GGCAGCGAGGAGUCCACAGU | 1623 |
| CR002167 | h | + | Chr2:60498121 -60498143 | GAGGUGUGGAGGGGAUAACU | 1624 |
| CR002168 | h | + | Chr2:60498137 -60498159 | AACUGGGUCAGACCCCAAGC | 1625 |
| CR002169 | h | + | Chr2:60498141 -60498163 | GGGUCAGACCCCAAGCAGGA | 1626 |
| CR002170 | h | + | Chr2:60498142 -60498164 | GGUCAGACCCCAAGCAGGAA | 1627 |
| CR002171 | h | - | Chr2:60498149 -60498171 | CCCCAAGCAGGAAGGGCCUC | 1628 |
| CR002172 | h | - | Chr2:60498150 -60498172 | CCCAAGCAGGAAGGGCCUCU | 1629 |
| CR002173 | h | - | Chr2:60498151 -60498173 | CCAAGCAGGAAGGGCCUCUA | 1630 |
| CR002174 | h | + | Chr2:60498157 -60498179 | AGGAAGGGCCUCUAUGUAGA | 1631 |
| CR002175 | h | + | Chr2:60498158 -60498180 | GGAAGGGCCUCUAUGUAGAC | 1632 |
| CR002176 | h | + | Chr2:60498165 -60498187 | CCUCUAUGUAGACGGGUGUG | 1633 |
| CR002177 | h | - | Chr2:60498165 -60498187 | CCUCUAUGUAGACGGGUGUG | 1634 |
| CR002178 | h | + | Chr2:60498175 -60498197 | GACGGGUGUGUGGCUCCUUA | 1635 |
| CR002179 | h | - | Chr2:60498190 -60498212 | CCUUAAGGUGACCCAGCAGC | 1636 |
| CR002180 | h | + | Chr2:60498192 -60498214 | UUAAGGUGACCCAGCAGCCC | 1637 |
| CR002181 | h | + | Chr2:60498193 -60498215 | UAAGGUGACCCAGCAGCCCU | 1638 |
| CR002182 | h | - | Chr2:60498201 -60498223 | CCCAGCAGCCCUGGGCACAG | 1639 |
| CR002183 | h | - | Chr2:60498202 -60498224 | CCAGCAGCCCUGGGCACAGA | 1640 |
| CR001261 | h | - | Chr1:55039269 -55039291 | GCCAGACUCCAAGUUCUGCC | 1641 |
| CR002184 | h | + | Chr2:60498204 -60498226 | AGCAGCCCUGGGCACAGAAG | 1642 |
| CR002185 | h | - | Chr2:60498209 -60498231 | CCCUGGGCACAGAAGUGGUG | 1643 |
| CR002186 | h | - | Chr2:60498210 -60498232 | CCUGGGCACAGAAGUGGUGC | 1644 |
| CR002187 | h | + | Chr2:60498211 -60498233 | CUGGGCACAGAAGUGGUGCG | 1645 |
| CR002188 | h | + | Chr2:60498240 -60498262 | UGCCAACAGUGAUAACCAGC | 1646 |
| CR002189 | h | + | Chr2:60498241 -60498263 | GCCAACAGUGAUAACCAGCA | 1647 |
| CR001262 | h | + | Chr1:55039155 -55039177 | UAAGGCCAGUGGAAAGAAUU | 1648 |
| CR002190 | h | - | Chr2:60498242 -60498264 | CCAACAGUGAUAACCAGCAG | 1649 |
| CR002191 | h | + | Chr2:60498255 -60498277 | CCAGCAGGGCCUGUCAGAAG | 1650 |
| CR002192 | h | - | Chr2:60498255 -60498277 | CCAGCAGGGCCUGUCAGAAG | 1651 |
| CR002193 | h | + | Chr2:60498261 -60498283 | GGGCCUGUCAGAAGAGGCCC | 1652 |
| CR002194 | h | - | Chr2:60498264 -60498286 | CCUGUCAGAAGAGGCCCUGG | 1653 |
| CR002195 | h | + | Chr2:60498271 -60498293 | GAAGAGGCCCUGGACACUGA | 1654 |
| CR002196 | h | + | Chr2:60498275 -60498297 | AGGCCCUGGACACUGAAGGC | 1655 |
| CR002197 | h | + | Chr2:60498276 -60498298 | GGCCCUGGACACUGAAGGCU | 1656 |
| CR001264 | h | - | Chr1:55039149 -55039171 | UCUUUCCACUGGCCUUAACC | 1657 |
| CR002198 | h | - | Chr2:60498278 -60498300 | CCCUGGACACUGAAGGCUGG | 1658 |
| CR002199 | h | - | Chr2:60498279 -60498301 | CCUGGACACUGAAGGCUGGG | 1659 |
| CR002200 | h | + | Chr2:60498287 -60498309 | CUGAAGGCUGGGCACAGCCU | 1660 |
| CR002201 | h | + | Chr2:60498288 -60498310 | UGAAGGCUGGGCACAGCCUU | 1661 |
| CR002202 | h | + | Chr2:60498289 -60498311 | GAAGGCUGGGCACAGCCUUG | 1662 |
| CR002203 | h | + | Chr2:60498301 -60498323 | CAGCCUUGGGGACCGCUCAC | 1663 |
| CR002204 | h | - | Chr2:60498304 -60498326 | CCUUGGGGACCGCUCACAGG | 1664 |
| CR002205 | h | - | Chr2:60498313 -60498335 | CCGCUCACAGGACAUGCAGC | 1665 |
| CR002206 | h | - | Chr2:60498341 -60498363 | CCGACAACUCCCUACCGCGA | 1666 |
| CR002207 | h | - | Chr2:60498350 -60498372 | CCCUACCGCGACCCCUAUCA | 1667 |
| CR002208 | h | - | Chr2:60498351 -60498373 | CCUACCGCGACCCCUAUCAG | 1668 |
| CR002209 | h | - | Chr2:60498355 -60498377 | CCGCGACCCCUAUCAGUGCC | 1669 |
| CR002210 | h | - | Chr2:60498361 -60498383 | CCCCUAUCAGUGCCGACCAA | 1670 |
| CR002211 | h | - | Chr2:60498362 -60498384 | CCCUAUCAGUGCCGACCAAG | 1671 |
| CR002212 | h | - | Chr2:60498363 -60498385 | CCUAUCAGUGCCGACCAAGC | 1672 |
| CR002213 | h | - | Chr2:60498373 -60498395 | CCGACCAAGCACACAAGAUG | 1673 |
| CR002214 | h | - | Chr2:60498377 -60498399 | CCAAGCACACAAGAUGCACA | 1674 |
| CR002215 | h | + | Chr2:60498380 -60498402 | AGCACACAAGAUGCACACCC | 1675 |
| CR002216 | h | + | Chr2:60498384 -60498406 | CACAAGAUGCACACCCAGGC | 1676 |
| CR002217 | h | + | Chr2:60498385 -60498407 | ACAAGAUGCACACCCAGGCU | 1677 |
| CR001264 | h | - | Chr1:55039149 -55039171 | UCUUUCCACUGGCCUUAACC | 1678 |
| CR002218 | h | + | Chr2:60498389 -60498411 | GAUGCACACCCAGGCUGGGC | 1679 |
| CR002219 | h | + | Chr2:60498396 -60498418 | ACCCAGGCUGGGCUGGACAG | 1680 |
| CR002220 | h | + | Chr2:60498397 -60498419 | CCCAGGCUGGGCUGGACAGA | 1681 |
| CR002221 | h | - | Chr2:60498397 -60498419 | CCCAGGCUGGGCUGGACAGA | 1682 |
| CR002222 | h | + | Chr2:60498398 -60498420 | CCAGGCUGGGCUGGACAGAG | 1683 |
| CR002223 | h | - | Chr2:60498398 -60498420 | CCAGGCUGGGCUGGACAGAG | 1684 |
| CR002224 | h | + | Chr2:60498415 -60498437 | GAGGGGUCCCACAAGAUCAC | 1685 |
| CR002225 | h | + | Chr2:60498416 -60498438 | AGGGGUCCCACAAGAUCACA | 1686 |
| CR002226 | h | - | Chr2:60498422 -60498444 | CCCACAAGAUCACAGGGUGU | 1687 |
| CR001263 | h | + | Chr1:55039180 -55039202 | GGCAGCGAGGAGUCCACAGU | 1688 |
| CR002227 | h | - | Chr2:60498423 -60498445 | CCACAAGAUCACAGGGUGUG | 1689 |
| CR002228 | h | + | Chr2:60498431 -60498453 | UCACAGGGUGUGCCCUGAGA | 1690 |
| CR002229 | h | + | Chr2:60498434 -60498456 | CAGGGUGUGCCCUGAGAAGG | 1691 |

In some aspects of the invention, it is preferred that the gRNA molecule to the +55 Enhancer region of BCL11a comprise a targeting domain comprising, e.g., consisting of a targeting domain sequence selected from SEQ ID NO: 1683, SEQ ID NO: 1638, SEQ ID NO: 1647, SEQ ID NO: 1609, SEQ ID NO: 1621, SEQ ID NO: 1617, SEQ ID NO: 1654, SEQ ID NO: 1631, SEQ ID NO: 1620, SEQ ID NO: 1637, SEQ ID NO: 1612, SEQ ID NO: 1656, SEQ ID NO: 1619, SEQ ID NO: 1675, SEQ ID NO: 1645, SEQ ID NO: 1598, SEQ ID NO: 1599, SEQ ID NO: 1663, SEQ ID NO: 1677, and SEQ ID NO: 1626.

In some aspects of the invention, e.g., as indicated herein, it may be beneficial to include gRNA moleucles targeting more than one, e.g., two, target sites (e.g., a first gRNA molecule and a second gRNA molecule). In some embodiments, the two target sites are both located in the +55 BCL11a enhancer region. In such aspects, any combination of more than one, e.g., two, gRNA molecules (e.g., a first gRNA molecule and a second gRNA molecule) comprising, e.g., consisting of, targeting domains listed in Table 9 may be used, that target different target sites of the +55 BCL11a enhancer region. In an aspect, the first gRNA molecule and the second gRNA molecule include targeting domains comprising, e.g., consisting of, SEQ ID NO: 1683 and SEQ ID NO: 1638, respectively. In an aspect, the first gRNA molecule and the second gRNA molecule include targeting domains comprising, e.g., consisting of, SEQ ID NO: 1683 and SEQ ID NO: 1647, respectively. In an aspect, the first gRNA molecule and the second gRNA molecule include targeting domains comprising, e.g., consisting of, SEQ ID NO: 1683 and SEQ ID NO: 1609, respectively. In an aspect, the first gRNA molecule and the second gRNA molecule include targeting domains comprising, e.g., consisting of, SEQ ID NO: 1683 and SEQ ID NO: 1621, respectively. In an aspect, the first gRNA molecule and the second gRNA molecule include targeting domains comprising, e.g., consisting of, SEQ ID NO: 1683 and SEQ ID NO: 1617, respectively. In an aspect, the first gRNA molecule and the second gRNA molecule include targeting domains comprising, e.g., consisting of, SEQ ID NO: 1683 and SEQ ID NO: 1654, respectively. In an aspect, the first gRNA molecule and the second gRNA molecule include targeting domains comprising, e.g., consisting of, SEQ ID NO: 1683 and SEQ ID NO: 1631, respectively. In an aspect, the first gRNA molecule and the second gRNA molecule include targeting domains comprising, e.g., consisting of, SEQ ID NO: 1683 and SEQ ID NO: 1620, respectively. In an aspect, the first gRNA molecule and the second gRNA molecule include targeting domains comprising, e.g., consisting of, SEQ ID NO: 1683 and SEQ ID NO: 1637, respectively. In an aspect, the first gRNA molecule and the second gRNA molecule include targeting domains comprising, e.g., consisting of, SEQ ID NO: 1683 and SEQ ID NO: 1612, respectively. In an aspect, the first gRNA molecule and the second gRNA molecule include targeting domains comprising, e.g., consisting of, SEQ ID NO: 1683 and SEQ ID NO: 1656, respectively. In an aspect, the first gRNA molecule and the second gRNA molecule include targeting domains comprising, e.g., consisting of, SEQ ID NO: 1683 and SEQ ID NO: 1619, respectively. In an aspect, the first gRNA molecule and the second gRNA molecule include targeting domains comprising, e.g., consisting of, SEQ ID NO: 1683 and SEQ ID NO: 1675, respectively. In an aspect, the first gRNA molecule and the second gRNA molecule include targeting domains comprising, e.g., consisting of, SEQ ID NO: 1683 and SEQ ID NO: 1645, respectively. In an aspect, the first gRNA molecule and the second gRNA molecule include targeting domains comprising, e.g., consisting of, SEQ ID NO: 1683 and SEQ ID NO: 1598, respectively. In an aspect, the first gRNA molecule and the second gRNA molecule include targeting domains comprising, e.g., consisting of, SEQ ID NO: 1683 and SEQ ID NO: 1599, respectively. In an aspect, the first gRNA molecule and the second gRNA molecule include targeting domains comprising, e.g., consisting of, SEQ ID NO: 1683 and SEQ ID NO: 1663, respectively. In an aspect, the first gRNA molecule and the second gRNA molecule include targeting domains comprising, e.g., consisting of, SEQ ID NO: 1683 and SEQ ID NO: 1677, respectively. In an aspect, the first gRNA molecule and the second gRNA molecule include targeting domains comprising, e.g., consisting of, SEQ ID NO: 1683 and SEQ ID NO: 1626, respectively. In an aspect, the first gRNA molecule and the second gRNA molecule include targeting domains comprising, e.g., consisting of, SEQ ID NO: 1638 and SEQ ID NO: 1647, respectively. In an aspect, the first gRNA molecule and the second gRNA molecule include targeting domains comprising, e.g., consisting of, SEQ ID NO: 1638 and SEQ ID NO: 1609, respectively. In an aspect, the first gRNA molecule and the second gRNA molecule include targeting domains comprising, e.g., consisting of, SEQ ID NO: 1638 and SEQ ID NO: 1621, respectively. In an aspect, the first gRNA molecule and the second gRNA molecule include targeting domains comprising, e.g., consisting of, SEQ ID NO: 1638 and SEQ ID NO: 1617, respectively. In an aspect, the first gRNA molecule and the second gRNA molecule include targeting domains comprising, e.g., consisting of, SEQ ID NO: 1638 and SEQ ID NO: 1654, respectively. In an aspect, the first gRNA molecule and the second gRNA molecule include targeting domains comprising, e.g., consisting of, SEQ ID NO: 1638 and SEQ ID NO: 1631, respectively. In an aspect, the first gRNA molecule and the second gRNA molecule include targeting domains comprising, e.g., consisting of, SEQ ID NO: 1638 and SEQ ID NO: 1620, respectively. In an aspect, the first gRNA molecule and the second gRNA molecule include targeting domains comprising, e.g., consisting of, SEQ ID NO: 1638 and SEQ ID NO: 1637, respectively. In an aspect, the first gRNA molecule and the second gRNA molecule include targeting domains comprising, e.g., consisting of, SEQ ID NO: 1638 and SEQ ID NO: 1612, respectively. In an aspect, the first gRNA molecule and the second gRNA molecule include targeting domains comprising, e.g., consisting of, SEQ ID NO: 1638 and SEQ ID NO: 1656, respectively. In an aspect, the first gRNA molecule and the second gRNA molecule include targeting domains comprising, e.g., consisting of, SEQ ID NO: 1638 and SEQ ID NO: 1619, respectively. In an aspect, the first gRNA molecule and the second gRNA molecule include targeting domains comprising, e.g., consisting of, SEQ ID NO: 1638 and SEQ ID NO: 1675, respectively. In an aspect, the first gRNA molecule and the second gRNA molecule include targeting domains comprising, e.g., consisting of, SEQ ID NO: 1638 and SEQ ID NO: 1645, respectively. In an aspect, the first gRNA molecule and the second gRNA molecule include targeting domains comprising, e.g., consisting of, SEQ ID NO: 1638 and SEQ ID NO: 1598, respectively. In an aspect, the first gRNA molecule and the second gRNA molecule include targeting domains comprising, e.g., consisting of, SEQ ID NO: 1638 and SEQ ID NO: 1599, respectively. In an aspect, the first gRNA molecule and the second gRNA molecule include targeting domains comprising, e.g., consisting of, SEQ ID NO: 1638 and SEQ ID NO: 1663, respectively. In an aspect, the first gRNA molecule and the second gRNA molecule include targeting domains comprising, e.g., consisting of, SEQ ID NO: 1638 and SEQ ID NO: 1677, respectively. In an aspect, the first gRNA molecule and the second gRNA molecule include targeting domains comprising, e.g., consisting of, SEQ ID NO: 1638 and SEQ ID NO: 1626, respectively. In an aspect, the first gRNA molecule and the second gRNA molecule include targeting domains comprising, e.g., consisting of, SEQ ID NO: 1647 and SEQ ID NO: 1638, respectively. In an aspect, the first gRNA molecule and the second gRNA molecule include targeting domains comprising, e.g., consisting of, SEQ ID NO: 1647 and SEQ ID NO: 1609, respectively. In an aspect, the first gRNA molecule and the second gRNA molecule include targeting domains comprising, e.g., consisting of, SEQ ID NO: 1647 and SEQ ID NO: 1621, respectively. In an aspect, the first gRNA molecule and the second gRNA molecule include targeting domains comprising, e.g., consisting of, SEQ ID NO: 1647 and SEQ ID NO: 1617, respectively. In an aspect, the first gRNA molecule and the second gRNA molecule include targeting domains comprising, e.g., consisting of, SEQ ID NO: 1647 and SEQ ID NO: 1654, respectively. In an aspect, the first gRNA molecule and the second gRNA molecule include targeting domains comprising, e.g., consisting of, SEQ ID NO: 1647 and SEQ ID NO: 1631, respectively. In an aspect, the first gRNA molecule and the second gRNA molecule include targeting domains comprising, e.g., consisting of, SEQ ID NO: 1647 and SEQ ID NO: 1620, respectively. In an aspect, the first gRNA molecule and the second gRNA molecule include targeting domains comprising, e.g., consisting of, SEQ ID NO: 1647 and SEQ ID NO: 1637, respectively. In an aspect, the first gRNA molecule and the second gRNA molecule include targeting domains comprising, e.g., consisting of, SEQ ID NO: 1647 and SEQ ID NO: 1612, respectively. In an aspect, the first gRNA molecule and the second gRNA molecule include targeting domains comprising, e.g., consisting of, SEQ ID NO: 1647 and SEQ ID NO: 1656, respectively. In an aspect, the first gRNA molecule and the second gRNA molecule include targeting domains comprising, e.g., consisting of, SEQ ID NO: 1647 and SEQ ID NO: 1619, respectively. In an aspect, the first gRNA molecule and the second gRNA molecule include targeting domains comprising, e.g., consisting of, SEQ ID NO: 1647 and SEQ ID NO: 1675, respectively. In an aspect, the first gRNA molecule and the second gRNA molecule include targeting domains comprising, e.g., consisting of, SEQ ID NO: 1647 and SEQ ID NO: 1645, respectively. In an aspect, the first gRNA molecule and the second gRNA molecule include targeting domains comprising, e.g., consisting of, SEQ ID NO: 1647 and SEQ ID NO: 1598, respectively. In an aspect, the first gRNA molecule and the second gRNA molecule include targeting domains comprising, e.g., consisting of, SEQ ID NO: 1647 and SEQ ID NO: 1599, respectively. In an aspect, the first gRNA molecule and the second gRNA molecule include targeting domains comprising, e.g., consisting of, SEQ ID NO: 1647 and SEQ ID NO: 1663, respectively. In an aspect, the first gRNA molecule and the second gRNA molecule include targeting domains comprising, e.g., consisting of, SEQ ID NO: 1647 and SEQ ID NO: 1677, respectively. In an aspect, the first gRNA molecule and the second gRNA molecule include targeting domains comprising, e.g., consisting of, SEQ ID NO: 1647 and SEQ ID NO: 1626, respectively. In an aspect, the first gRNA molecule and the second gRNA molecule include targeting domains comprising, e.g., consisting of, SEQ ID NO: 1609 and SEQ ID NO: 1638, respectively. In an aspect, the first gRNA molecule and the second gRNA molecule include targeting domains comprising, e.g., consisting of, SEQ ID NO: 1609 and SEQ ID NO: 1647, respectively. In an aspect, the first gRNA molecule and the second gRNA molecule include targeting domains comprising, e.g., consisting of, SEQ ID NO: 1609 and SEQ ID NO: 1621, respectively. In an aspect, the first gRNA molecule and the second gRNA molecule include targeting domains comprising, e.g., consisting of, SEQ ID NO: 1609 and SEQ ID NO: 1617, respectively. In an aspect, the first gRNA molecule and the second gRNA molecule include targeting domains comprising, e.g., consisting of, SEQ ID NO: 1609 and SEQ ID NO: 1654, respectively. In an aspect, the first gRNA molecule and the second gRNA molecule include targeting domains comprising, e.g., consisting of, SEQ ID NO: 1609 and SEQ ID NO: 1631, respectively. In an aspect, the first gRNA molecule and the second gRNA molecule include targeting domains comprising, e.g., consisting of, SEQ ID NO: 1609 and SEQ ID NO: 1620, respectively. In an aspect, the first gRNA molecule and the second gRNA molecule include targeting domains comprising, e.g., consisting of, SEQ ID NO: 1609 and SEQ ID NO: 1637, respectively. In an aspect, the first gRNA molecule and the second gRNA molecule include targeting domains comprising, e.g., consisting of, SEQ ID NO: 1609 and SEQ ID NO: 1612, respectively. In an aspect, the first gRNA molecule and the second gRNA molecule include targeting domains comprising, e.g., consisting of, SEQ ID NO: 1609 and SEQ ID NO: 1656, respectively. In an aspect, the first gRNA molecule and the second gRNA molecule include targeting domains comprising, e.g., consisting of, SEQ ID NO: 1609 and SEQ ID NO: 1619, respectively. In an aspect, the first gRNA molecule and the second gRNA molecule include targeting domains comprising, e.g., consisting of, SEQ ID NO: 1609 and SEQ ID NO: 1675, respectively. In an aspect, the first gRNA molecule and the second gRNA molecule include targeting domains comprising, e.g., consisting of, SEQ ID NO: 1609 and SEQ ID NO: 1645, respectively. In an aspect, the first gRNA molecule and the second gRNA molecule include targeting domains comprising, e.g., consisting of, SEQ ID NO: 1609 and SEQ ID NO: 1598, respectively. In an aspect, the first gRNA molecule and the second gRNA molecule include targeting domains comprising, e.g., consisting of, SEQ ID NO: 1609 and SEQ ID NO: 1599, respectively. In an aspect, the first gRNA molecule and the second gRNA molecule include targeting domains comprising, e.g., consisting of, SEQ ID NO: 1609 and SEQ ID NO: 1663, respectively. In an aspect, the first gRNA molecule and the second gRNA molecule include targeting domains comprising, e.g., consisting of, SEQ ID NO: 1609 and SEQ ID NO: 1677, respectively. In an aspect, the first gRNA molecule and the second gRNA molecule include targeting domains comprising, e.g., consisting of, SEQ ID NO: 1609 and SEQ ID NO: 1626, respectively. In an aspect, the first gRNA molecule and the second gRNA molecule include targeting domains comprising, e.g., consisting of, SEQ ID NO: 1621 and SEQ ID NO: 1638, respectively. In an aspect, the first gRNA molecule and the second gRNA molecule include targeting domains comprising, e.g., consisting of, SEQ ID NO: 1621 and SEQ ID NO: 1647, respectively. In an aspect, the first gRNA molecule and the second gRNA molecule include targeting domains comprising, e.g., consisting of, SEQ ID NO: 1621 and SEQ ID NO: 1609, respectively. In an aspect, the first gRNA molecule and the second gRNA molecule include targeting domains comprising, e.g., consisting of, SEQ ID NO: 1621 and SEQ ID NO: 1617, respectively. In an aspect, the first gRNA molecule and the second gRNA molecule include targeting domains comprising, e.g., consisting of, SEQ ID NO: 1621 and SEQ ID NO: 1654, respectively. In an aspect, the first gRNA molecule and the second gRNA molecule include targeting domains comprising, e.g., consisting of, SEQ ID NO: 1621 and SEQ ID NO: 1631, respectively. In an aspect, the first gRNA molecule and the second gRNA molecule include targeting domains comprising, e.g., consisting of, SEQ ID NO: 1621 and SEQ ID NO: 1620, respectively. In an aspect, the first gRNA molecule and the second gRNA molecule include targeting domains comprising, e.g., consisting of, SEQ ID NO: 1621 and SEQ ID NO: 1637, respectively. In an aspect, the first gRNA molecule and the second gRNA molecule include targeting domains comprising, e.g., consisting of, SEQ ID NO: 1621 and SEQ ID NO: 1612, respectively. In an aspect, the first gRNA molecule and the second gRNA molecule include targeting domains comprising, e.g., consisting of, SEQ ID NO: 1621 and SEQ ID NO: 1656, respectively. In an aspect, the first gRNA molecule and the second gRNA molecule include targeting domains comprising, e.g., consisting of, SEQ ID NO: 1621 and SEQ ID NO: 1619, respectively. In an aspect, the first gRNA molecule and the second gRNA molecule include targeting domains comprising, e.g., consisting of, SEQ ID NO: 1621 and SEQ ID NO: 1675, respectively. In an aspect, the first gRNA molecule and the second gRNA molecule include targeting domains comprising, e.g., consisting of, SEQ ID NO: 1621 and SEQ ID NO: 1645, respectively. In an aspect, the first gRNA molecule and the second gRNA molecule include targeting domains comprising, e.g., consisting of, SEQ ID NO: 1621 and SEQ ID NO: 1598, respectively. In an aspect, the first gRNA molecule and the second gRNA molecule include targeting domains comprising, e.g., consisting of, SEQ ID NO: 1621 and SEQ ID NO: 1599, respectively. In an aspect, the first gRNA molecule and the second gRNA molecule include targeting domains comprising, e.g., consisting of, SEQ ID NO: 1621 and SEQ ID NO: 1663, respectively. In an aspect, the first gRNA molecule and the second gRNA molecule include targeting domains comprising, e.g., consisting of, SEQ ID NO: 1621 and SEQ ID NO: 1677, respectively. In an aspect, the first gRNA molecule and the second gRNA molecule include targeting domains comprising, e.g., consisting of, SEQ ID NO: 1621 and SEQ ID NO: 1626, respectively. In an aspect, the first gRNA molecule and the second gRNA molecule include targeting domains comprising, e.g., consisting of, SEQ ID NO: 1617 and SEQ ID NO: 1638, respectively. In an aspect, the first gRNA molecule and the second gRNA molecule include targeting domains comprising, e.g., consisting of, SEQ ID NO: 1617 and SEQ ID NO: 1647, respectively. In an aspect, the first gRNA molecule and the second gRNA molecule include targeting domains comprising, e.g., consisting of, SEQ ID NO: 1617 and SEQ ID NO: 1609, respectively. In an aspect, the first gRNA molecule and the second gRNA molecule include targeting domains comprising, e.g., consisting of, SEQ ID NO: 1617 and SEQ ID NO: 1621, respectively. In an aspect, the first gRNA molecule and the second gRNA molecule include targeting domains comprising, e.g., consisting of, SEQ ID NO: 1617 and SEQ ID NO: 1654, respectively. In an aspect, the first gRNA molecule and the second gRNA molecule include targeting domains comprising, e.g., consisting of, SEQ ID NO: 1617 and SEQ ID NO: 1631, respectively. In an aspect, the first gRNA molecule and the second gRNA molecule include targeting domains comprising, e.g., consisting of, SEQ ID NO: 1617 and SEQ ID NO: 1620, respectively. In an aspect, the first gRNA molecule and the second gRNA molecule include targeting domains comprising, e.g., consisting of, SEQ ID NO: 1617 and SEQ ID NO: 1637, respectively. In an aspect, the first gRNA molecule and the second gRNA molecule include targeting domains comprising, e.g., consisting of, SEQ ID NO: 1617 and SEQ ID NO: 1612, respectively. In an aspect, the first gRNA molecule and the second gRNA molecule include targeting domains comprising, e.g., consisting of, SEQ ID NO: 1617 and SEQ ID NO: 1656, respectively. In an aspect, the first gRNA molecule and the second gRNA molecule include targeting domains comprising, e.g., consisting of, SEQ ID NO: 1617 and SEQ ID NO: 1619, respectively. In an aspect, the first gRNA molecule and the second gRNA molecule include targeting domains comprising, e.g., consisting of, SEQ ID NO: 1617 and SEQ ID NO: 1675, respectively. In an aspect, the first gRNA molecule and the second gRNA molecule include targeting domains comprising, e.g., consisting of, SEQ ID NO: 1617 and SEQ ID NO: 1645, respectively. In an aspect, the first gRNA molecule and the second gRNA molecule include targeting domains comprising, e.g., consisting of, SEQ ID NO: 1617 and SEQ ID NO: 1598, respectively. In an aspect, the first gRNA molecule and the second gRNA molecule include targeting domains comprising, e.g., consisting of, SEQ ID NO: 1617 and SEQ ID NO: 1599, respectively. In an aspect, the first gRNA molecule and the second gRNA molecule include targeting domains comprising, e.g., consisting of, SEQ ID NO: 1617 and SEQ ID NO: 1663, respectively. In an aspect, the first gRNA molecule and the second gRNA molecule include targeting domains comprising, e.g., consisting of, SEQ ID NO: 1617 and SEQ ID NO: 1677, respectively. In an aspect, the first gRNA molecule and the second gRNA molecule include targeting domains comprising, e.g., consisting of, SEQ ID NO: 1617 and SEQ ID NO: 1626, respectively. In an aspect, the first gRNA molecule and the second gRNA molecule include targeting domains comprising, e.g., consisting of, SEQ ID NO: 1654 and SEQ ID NO: 1638, respectively. In an aspect, the first gRNA molecule and the second gRNA molecule include targeting domains comprising, e.g., consisting of, SEQ ID NO: 1654 and SEQ ID NO: 1647, respectively. In an aspect, the first gRNA molecule and the second gRNA molecule include targeting domains comprising, e.g., consisting of, SEQ ID NO: 1654 and SEQ ID NO: 1609, respectively. In an aspect, the first gRNA molecule and the second gRNA molecule include targeting domains comprising, e.g., consisting of, SEQ ID NO: 1654 and SEQ ID NO: 1621, respectively. In an aspect, the first gRNA molecule and the second gRNA molecule include targeting domains comprising, e.g., consisting of, SEQ ID NO: 1654 and SEQ ID NO: 1617, respectively. In an aspect, the first gRNA molecule and the second gRNA molecule include targeting domains comprising, e.g., consisting of, SEQ ID NO: 1654 and SEQ ID NO: 1631, respectively. In an aspect, the first gRNA molecule and the second gRNA molecule include targeting domains comprising, e.g., consisting of, SEQ ID NO: 1654 and SEQ ID NO: 1620, respectively. In an aspect, the first gRNA molecule and the second gRNA molecule include targeting domains comprising, e.g., consisting of, SEQ ID NO: 1654 and SEQ ID NO: 1637, respectively. In an aspect, the first gRNA molecule and the second gRNA molecule include targeting domains comprising, e.g., consisting of, SEQ ID NO: 1654 and SEQ ID NO: 1612, respectively. In an aspect, the first gRNA molecule and the second gRNA molecule include targeting domains comprising, e.g., consisting of, SEQ ID NO: 1654 and SEQ ID NO: 1656, respectively. In an aspect, the first gRNA molecule and the second gRNA molecule include targeting domains comprising, e.g., consisting of, SEQ ID NO: 1654 and SEQ ID NO: 1619, respectively. In an aspect, the first gRNA molecule and the second gRNA molecule include targeting domains comprising, e.g., consisting of, SEQ ID NO: 1654 and SEQ ID NO: 1675, respectively. In an aspect, the first gRNA molecule and the second gRNA molecule include targeting domains comprising, e.g., consisting of, SEQ ID NO: 1654 and SEQ ID NO: 1645, respectively. In an aspect, the first gRNA molecule and the second gRNA molecule include targeting domains comprising, e.g., consisting of, SEQ ID NO: 1654 and SEQ ID NO: 1598, respectively. In an aspect, the first gRNA molecule and the second gRNA molecule include targeting domains comprising, e.g., consisting of, SEQ ID NO: 1654 and SEQ ID NO: 1599, respectively. In an aspect, the first gRNA molecule and the second gRNA molecule include targeting domains comprising, e.g., consisting of, SEQ ID NO: 1654 and SEQ ID NO: 1663, respectively. In an aspect, the first gRNA molecule and the second gRNA molecule include targeting domains comprising, e.g., consisting of, SEQ ID NO: 1654 and SEQ ID NO: 1677, respectively. In an aspect, the first gRNA molecule and the second gRNA molecule include targeting domains comprising, e.g., consisting of, SEQ ID NO: 1654 and SEQ ID NO: 1626, respectively. In an aspect, the first gRNA molecule and the second gRNA molecule include targeting domains comprising, e.g., consisting of, SEQ ID NO: 1631 and SEQ ID NO: 1638, respectively. In an aspect, the first gRNA molecule and the second gRNA molecule include targeting domains comprising, e.g., consisting of, SEQ ID NO: 1631 and SEQ ID NO: 1647, respectively. In an aspect, the first gRNA molecule and the second gRNA molecule include targeting domains comprising, e.g., consisting of, SEQ ID NO: 1631 and SEQ ID NO: 1609, respectively. In an aspect, the first gRNA molecule and the second gRNA molecule include targeting domains comprising, e.g., consisting of, SEQ ID NO: 1631 and SEQ ID NO: 1621, respectively. In an aspect, the first gRNA molecule and the second gRNA molecule include targeting domains comprising, e.g., consisting of, SEQ ID NO: 1631 and SEQ ID NO: 1617, respectively. In an aspect, the first gRNA molecule and the second gRNA molecule include targeting domains comprising, e.g., consisting of, SEQ ID NO: 1631 and SEQ ID NO: 1654, respectively. In an aspect, the first gRNA molecule and the second gRNA molecule include targeting domains comprising, e.g., consisting of, SEQ ID NO: 1631 and SEQ ID NO: 1620, respectively. In an aspect, the first gRNA molecule and the second gRNA molecule include targeting domains comprising, e.g., consisting of, SEQ ID NO: 1631 and SEQ ID NO: 1637, respectively. In an aspect, the first gRNA molecule and the second gRNA molecule include targeting domains comprising, e.g., consisting of, SEQ ID NO: 1631 and SEQ ID NO: 1612, respectively. In an aspect, the first gRNA molecule and the second gRNA molecule include targeting domains comprising, e.g., consisting of, SEQ ID NO: 1631 and SEQ ID NO: 1656, respectively. In an aspect, the first gRNA molecule and the second gRNA molecule include targeting domains comprising, e.g., consisting of, SEQ ID NO: 1631 and SEQ ID NO: 1619, respectively. In an aspect, the first gRNA molecule and the second gRNA molecule include targeting domains comprising, e.g., consisting of, SEQ ID NO: 1631 and SEQ ID NO: 1675, respectively. In an aspect, the first gRNA molecule and the second gRNA molecule include targeting domains comprising, e.g., consisting of, SEQ ID NO: 1631 and SEQ ID NO: 1645, respectively. In an aspect, the first gRNA molecule and the second gRNA molecule include targeting domains comprising, e.g., consisting of, SEQ ID NO: 1631 and SEQ ID NO: 1598, respectively. In an aspect, the first gRNA molecule and the second gRNA molecule include targeting domains comprising, e.g., consisting of, SEQ ID NO: 1631 and SEQ ID NO: 1599, respectively. In an aspect, the first gRNA molecule and the second gRNA molecule include targeting domains comprising, e.g., consisting of, SEQ ID NO: 1631 and SEQ ID NO: 1663, respectively. In an aspect, the first gRNA molecule and the second gRNA molecule include targeting domains comprising, e.g., consisting of, SEQ ID NO: 1631 and SEQ ID NO: 1677, respectively. In an aspect, the first gRNA molecule and the second gRNA molecule include targeting domains comprising, e.g., consisting of, SEQ ID NO: 1631 and SEQ ID NO: 1626, respectively. In an aspect, the first gRNA molecule and the second gRNA molecule include targeting domains comprising, e.g., consisting of, SEQ ID NO: 1620 and SEQ ID NO: 1638, respectively. In an aspect, the first gRNA molecule and the second gRNA molecule include targeting domains comprising, e.g., consisting of, SEQ ID NO: 1620 and SEQ ID NO: 1647, respectively. In an aspect, the first gRNA molecule and the second gRNA molecule include targeting domains comprising, e.g., consisting of, SEQ ID NO: 1620 and SEQ ID NO: 1609, respectively. In an aspect, the first gRNA molecule and the second gRNA molecule include targeting domains comprising, e.g., consisting of, SEQ ID NO: 1620 and SEQ ID NO: 1621, respectively. In an aspect, the first gRNA molecule and the second gRNA molecule include targeting domains comprising, e.g., consisting of, SEQ ID NO: 1620 and SEQ ID NO: 1617, respectively. In an aspect, the first gRNA molecule and the second gRNA molecule include targeting domains comprising, e.g., consisting of, SEQ ID NO: 1620 and SEQ ID NO: 1654, respectively. In an aspect, the first gRNA molecule and the second gRNA molecule include targeting domains comprising, e.g., consisting of, SEQ ID NO: 1620 and SEQ ID NO: 1631, respectively. In an aspect, the first gRNA molecule and the second gRNA molecule include targeting domains comprising, e.g., consisting of, SEQ ID NO: 1620 and SEQ ID NO: 1637, respectively. In an aspect, the first gRNA molecule and the second gRNA molecule include targeting domains comprising, e.g., consisting of, SEQ ID NO: 1620 and SEQ ID NO: 1612, respectively. In an aspect, the first gRNA molecule and the second gRNA molecule include targeting domains comprising, e.g., consisting of, SEQ ID NO: 1620 and SEQ ID NO: 1656, respectively. In an aspect, the first gRNA molecule and the second gRNA molecule include targeting domains comprising, e.g., consisting of, SEQ ID NO: 1620 and SEQ ID NO: 1619, respectively. In an aspect, the first gRNA molecule and the second gRNA molecule include targeting domains comprising, e.g., consisting of, SEQ ID NO: 1620 and SEQ ID NO: 1675, respectively. In an aspect, the first gRNA molecule and the second gRNA molecule include targeting domains comprising, e.g., consisting of, SEQ ID NO: 1620 and SEQ ID NO: 1645, respectively. In an aspect, the first gRNA molecule and the second gRNA molecule include targeting domains comprising, e.g., consisting of, SEQ ID NO: 1620 and SEQ ID NO: 1598, respectively. In an aspect, the first gRNA molecule and the second gRNA molecule include targeting domains comprising, e.g., consisting of, SEQ ID NO: 1620 and SEQ ID NO: 1599, respectively. In an aspect, the first gRNA molecule and the second gRNA molecule include targeting domains comprising, e.g., consisting of, SEQ ID NO: 1620 and SEQ ID NO: 1663, respectively. In an aspect, the first gRNA molecule and the second gRNA molecule include targeting domains comprising, e.g., consisting of, SEQ ID NO: 1620 and SEQ ID NO: 1677, respectively. In an aspect, the first gRNA molecule and the second gRNA molecule include targeting domains comprising, e.g., consisting of, SEQ ID NO: 1620 and SEQ ID NO: 1626, respectively. In an aspect, the first gRNA molecule and the second gRNA molecule include targeting domains comprising, e.g., consisting of, SEQ ID NO: 1637 and SEQ ID NO: 1638, respectively. In an aspect, the first gRNA molecule and the second gRNA molecule include targeting domains comprising, e.g., consisting of, SEQ ID NO: 1637 and SEQ ID NO: 1647, respectively. In an aspect, the first gRNA molecule and the second gRNA molecule include targeting domains comprising, e.g., consisting of, SEQ ID NO: 1637 and SEQ ID NO: 1609, respectively. In an aspect, the first gRNA molecule and the second gRNA molecule include targeting domains comprising, e.g., consisting of, SEQ ID NO: 1637 and SEQ ID NO: 1621, respectively. In an aspect, the first gRNA molecule and the second gRNA molecule include targeting domains comprising, e.g., consisting of, SEQ ID NO: 1637 and SEQ ID NO: 1617, respectively. In an aspect, the first gRNA molecule and the second gRNA molecule include targeting domains comprising, e.g., consisting of, SEQ ID NO: 1637 and SEQ ID NO: 1654, respectively. In an aspect, the first gRNA molecule and the second gRNA molecule include targeting domains comprising, e.g., consisting of, SEQ ID NO: 1637 and SEQ ID NO: 1631, respectively. In an aspect, the first gRNA molecule and the second gRNA molecule include targeting domains comprising, e.g., consisting of, SEQ ID NO: 1637 and SEQ ID NO: 1620, respectively. In an aspect, the first gRNA molecule and the second gRNA molecule include targeting domains comprising, e.g., consisting of, SEQ ID NO: 1637 and SEQ ID NO: 1612, respectively. In an aspect, the first gRNA molecule and the second gRNA molecule include targeting domains comprising, e.g., consisting of, SEQ ID NO: 1637 and SEQ ID NO: 1656, respectively. In an aspect, the first gRNA molecule and the second gRNA molecule include targeting domains comprising, e.g., consisting of, SEQ ID NO: 1637 and SEQ ID NO: 1619, respectively. In an aspect, the first gRNA molecule and the second gRNA molecule include targeting domains comprising, e.g., consisting of, SEQ ID NO: 1637 and SEQ ID NO: 1675, respectively. In an aspect, the first gRNA molecule and the second gRNA molecule include targeting domains comprising, e.g., consisting of, SEQ ID NO: 1637 and SEQ ID NO: 1645, respectively. In an aspect, the first gRNA molecule and the second gRNA molecule include targeting domains comprising, e.g., consisting of, SEQ ID NO: 1637 and SEQ ID NO: 1598, respectively. In an aspect, the first gRNA molecule and the second gRNA molecule include targeting domains comprising, e.g., consisting of, SEQ ID NO: 1637 and SEQ ID NO: 1599, respectively. In an aspect, the first gRNA molecule and the second gRNA molecule include targeting domains comprising, e.g., consisting of, SEQ ID NO: 1637 and SEQ ID NO: 1663, respectively. In an aspect, the first gRNA molecule and the second gRNA molecule include targeting domains comprising, e.g., consisting of, SEQ ID NO: 1637 and SEQ ID NO: 1677, respectively. In an aspect, the first gRNA molecule and the second gRNA molecule include targeting domains comprising, e.g., consisting of, SEQ ID NO: 1637 and SEQ ID NO: 1626, respectively. In an aspect, the first gRNA molecule and the second gRNA molecule include targeting domains comprising, e.g., consisting of, SEQ ID NO: 1612 and SEQ ID NO: 1638, respectively. In an aspect, the first gRNA molecule and the second gRNA molecule include targeting domains comprising, e.g., consisting of, SEQ ID NO: 1612 and SEQ ID NO: 1647, respectively. In an aspect, the first gRNA molecule and the second gRNA molecule include targeting domains comprising, e.g., consisting of, SEQ ID NO: 1612 and SEQ ID NO: 1609, respectively. In an aspect, the first gRNA molecule and the second gRNA molecule include targeting domains comprising, e.g., consisting of, SEQ ID NO: 1612 and SEQ ID NO: 1621, respectively. In an aspect, the first gRNA molecule and the second gRNA molecule include targeting domains comprising, e.g., consisting of, SEQ ID NO: 1612 and SEQ ID NO: 1617, respectively. In an aspect, the first gRNA molecule and the second gRNA molecule include targeting domains comprising, e.g., consisting of, SEQ ID NO: 1612 and SEQ ID NO: 1654, respectively. In an aspect, the first gRNA molecule and the second gRNA molecule include targeting domains comprising, e.g., consisting of, SEQ ID NO: 1612 and SEQ ID NO: 1631, respectively. In an aspect, the first gRNA molecule and the second gRNA molecule include targeting domains comprising, e.g., consisting of, SEQ ID NO: 1612 and SEQ ID NO: 1620, respectively. In an aspect, the first gRNA molecule and the second gRNA molecule include targeting domains comprising, e.g., consisting of, SEQ ID NO: 1612 and SEQ ID NO: 1637, respectively. In an aspect, the first gRNA molecule and the second gRNA molecule include targeting domains comprising, e.g., consisting of, SEQ ID NO: 1612 and SEQ ID NO: 1656, respectively. In an aspect, the first gRNA molecule and the second gRNA molecule include targeting domains comprising, e.g., consisting of, SEQ ID NO: 1612 and SEQ ID NO: 1619, respectively. In an aspect, the first gRNA molecule and the second gRNA molecule include targeting domains comprising, e.g., consisting of, SEQ ID NO: 1612 and SEQ ID NO: 1675, respectively. In an aspect, the first gRNA molecule and the second gRNA molecule include targeting domains comprising, e.g., consisting of, SEQ ID NO: 1612 and SEQ ID NO: 1645, respectively. In an aspect, the first gRNA molecule and the second gRNA molecule include targeting domains comprising, e.g., consisting of, SEQ ID NO: 1612 and SEQ ID NO: 1598, respectively. In an aspect, the first gRNA molecule and the second gRNA molecule include targeting domains comprising, e.g., consisting of, SEQ ID NO: 1612 and SEQ ID NO: 1599, respectively. In an aspect, the first gRNA molecule and the second gRNA molecule include targeting domains comprising, e.g., consisting of, SEQ ID NO: 1612 and SEQ ID NO: 1663, respectively. In an aspect, the first gRNA molecule and the second gRNA molecule include targeting domains comprising, e.g., consisting of, SEQ ID NO: 1612 and SEQ ID NO: 1677, respectively. In an aspect, the first gRNA molecule and the second gRNA molecule include targeting domains comprising, e.g., consisting of, SEQ ID NO: 1612 and SEQ ID NO: 1626, respectively. In an aspect, the first gRNA molecule and the second gRNA molecule include targeting domains comprising, e.g., consisting of, SEQ ID NO: 1656 and SEQ ID NO: 1638, respectively. In an aspect, the first gRNA molecule and the second gRNA molecule include targeting domains comprising, e.g., consisting of, SEQ ID NO: 1656 and SEQ ID NO: 1647, respectively. In an aspect, the first gRNA molecule and the second gRNA molecule include targeting domains comprising, e.g., consisting of, SEQ ID NO: 1656 and SEQ ID NO: 1609, respectively. In an aspect, the first gRNA molecule and the second gRNA molecule include targeting domains comprising, e.g., consisting of, SEQ ID NO: 1656 and SEQ ID NO: 1621, respectively. In an aspect, the first gRNA molecule and the second gRNA molecule include targeting domains comprising, e.g., consisting of, SEQ ID NO: 1656 and SEQ ID NO: 1617, respectively. In an aspect, the first gRNA molecule and the second gRNA molecule include targeting domains comprising, e.g., consisting of, SEQ ID NO: 1656 and SEQ ID NO: 1654, respectively. In an aspect, the first gRNA molecule and the second gRNA molecule include targeting domains comprising, e.g., consisting of, SEQ ID NO: 1656 and SEQ ID NO: 1631, respectively. In an aspect, the first gRNA molecule and the second gRNA molecule include targeting domains comprising, e.g., consisting of, SEQ ID NO: 1656 and SEQ ID NO: 1620, respectively. In an aspect, the first gRNA molecule and the second gRNA molecule include targeting domains comprising, e.g., consisting of, SEQ ID NO: 1656 and SEQ ID NO: 1637, respectively. In an aspect, the first gRNA molecule and the second gRNA molecule include targeting domains comprising, e.g., consisting of, SEQ ID NO: 1656 and SEQ ID NO: 1612, respectively. In an aspect, the first gRNA molecule and the second gRNA molecule include targeting domains comprising, e.g., consisting of, SEQ ID NO: 1656 and SEQ ID NO: 1619, respectively. In an aspect, the first gRNA molecule and the second gRNA molecule include targeting domains comprising, e.g., consisting of, SEQ ID NO: 1656 and SEQ ID NO: 1675, respectively. In an aspect, the first gRNA molecule and the second gRNA molecule include targeting domains comprising, e.g., consisting of, SEQ ID NO: 1656 and SEQ ID NO: 1645, respectively. In an aspect, the first gRNA molecule and the second gRNA molecule include targeting domains comprising, e.g., consisting of, SEQ ID NO: 1656 and SEQ ID NO: 1598, respectively. In an aspect, the first gRNA molecule and the second gRNA molecule include targeting domains comprising, e.g., consisting of, SEQ ID NO: 1656 and SEQ ID NO: 1599, respectively. In an aspect, the first gRNA molecule and the second gRNA molecule include targeting domains comprising, e.g., consisting of, SEQ ID NO: 1656 and SEQ ID NO: 1663, respectively. In an aspect, the first gRNA molecule and the second gRNA molecule include targeting domains comprising, e.g., consisting of, SEQ ID NO: 1656 and SEQ ID NO: 1677, respectively. In an aspect, the first gRNA molecule and the second gRNA molecule include targeting domains comprising, e.g., consisting of, SEQ ID NO: 1656 and SEQ ID NO: 1626, respectively. In an aspect, the first gRNA molecule and the second gRNA molecule include targeting domains comprising, e.g., consisting of, SEQ ID NO: 1619 and SEQ ID NO: 1638, respectively. In an aspect, the first gRNA molecule and the second gRNA molecule include targeting domains comprising, e.g., consisting of, SEQ ID NO: 1619 and SEQ ID NO: 1647, respectively. In an aspect, the first gRNA molecule and the second gRNA molecule include targeting domains comprising, e.g., consisting of, SEQ ID NO: 1619 and SEQ ID NO: 1609, respectively. In an aspect, the first gRNA molecule and the second gRNA molecule include targeting domains comprising, e.g., consisting of, SEQ ID NO: 1619 and SEQ ID NO: 1621, respectively. In an aspect, the first gRNA molecule and the second gRNA molecule include targeting domains comprising, e.g., consisting of, SEQ ID NO: 1619 and SEQ ID NO: 1617, respectively. In an aspect, the first gRNA molecule and the second gRNA molecule include targeting domains comprising, e.g., consisting of, SEQ ID NO: 1619 and SEQ ID NO: 1654, respectively. In an aspect, the first gRNA molecule and the second gRNA molecule include targeting domains comprising, e.g., consisting of, SEQ ID NO: 1619 and SEQ ID NO: 1631, respectively. In an aspect, the first gRNA molecule and the second gRNA molecule include targeting domains comprising, e.g., consisting of, SEQ ID NO: 1619 and SEQ ID NO: 1620, respectively. In an aspect, the first gRNA molecule and the second gRNA molecule include targeting domains comprising, e.g., consisting of, SEQ ID NO: 1619 and SEQ ID NO: 1637, respectively. In an aspect, the first gRNA molecule and the second gRNA molecule include targeting domains comprising, e.g., consisting of, SEQ ID NO: 1619 and SEQ ID NO: 1612, respectively. In an aspect, the first gRNA molecule and the second gRNA molecule include targeting domains comprising, e.g., consisting of, SEQ ID NO: 1619 and SEQ ID NO: 1656, respectively. In an aspect, the first gRNA molecule and the second gRNA molecule include targeting domains comprising, e.g., consisting of, SEQ ID NO: 1619 and SEQ ID NO: 1675, respectively. In an aspect, the first gRNA molecule and the second gRNA molecule include targeting domains comprising, e.g., consisting of, SEQ ID NO: 1619 and SEQ ID NO: 1645, respectively. In an aspect, the first gRNA molecule and the second gRNA molecule include targeting domains comprising, e.g., consisting of, SEQ ID NO: 1619 and SEQ ID NO: 1598, respectively. In an aspect, the first gRNA molecule and the second gRNA molecule include targeting domains comprising, e.g., consisting of, SEQ ID NO: 1619 and SEQ ID NO: 1599, respectively. In an aspect, the first gRNA molecule and the second gRNA molecule include targeting domains comprising, e.g., consisting of, SEQ ID NO: 1619 and SEQ ID NO: 1663, respectively. In an aspect, the first gRNA molecule and the second gRNA molecule include targeting domains comprising, e.g., consisting of, SEQ ID NO: 1619 and SEQ ID NO: 1677, respectively. In an aspect, the first gRNA molecule and the second gRNA molecule include targeting domains comprising, e.g., consisting of, SEQ ID NO: 1619 and SEQ ID NO: 1626, respectively. In an aspect, the first gRNA molecule and the second gRNA molecule include targeting domains comprising, e.g., consisting of, SEQ ID NO: 1675 and SEQ ID NO: 1638, respectively. In an aspect, the first gRNA molecule and the second gRNA molecule include targeting domains comprising, e.g., consisting of, SEQ ID NO: 1675 and SEQ ID NO: 1647, respectively. In an aspect, the first gRNA molecule and the second gRNA molecule include targeting domains comprising, e.g., consisting of, SEQ ID NO: 1675 and SEQ ID NO: 1609, respectively. In an aspect, the first gRNA molecule and the second gRNA molecule include targeting domains comprising, e.g., consisting of, SEQ ID NO: 1675 and SEQ ID NO: 1621, respectively. In an aspect, the first gRNA molecule and the second gRNA molecule include targeting domains comprising, e.g., consisting of, SEQ ID NO: 1675 and SEQ ID NO: 1617, respectively. In an aspect, the first gRNA molecule and the second gRNA molecule include targeting domains comprising, e.g., consisting of, SEQ ID NO: 1675 and SEQ ID NO: 1654, respectively. In an aspect, the first gRNA molecule and the second gRNA molecule include targeting domains comprising, e.g., consisting of, SEQ ID NO: 1675 and SEQ ID NO: 1631, respectively. In an aspect, the first gRNA molecule and the second gRNA molecule include targeting domains comprising, e.g., consisting of, SEQ ID NO: 1675 and SEQ ID NO: 1620, respectively. In an aspect, the first gRNA molecule and the second gRNA molecule include targeting domains comprising, e.g., consisting of, SEQ ID NO: 1675 and SEQ ID NO: 1637, respectively. In an aspect, the first gRNA molecule and the second gRNA molecule include targeting domains comprising, e.g., consisting of, SEQ ID NO: 1675 and SEQ ID NO: 1612, respectively. In an aspect, the first gRNA molecule and the second gRNA molecule include targeting domains comprising, e.g., consisting of, SEQ ID NO: 1675 and SEQ ID NO: 1656, respectively. In an aspect, the first gRNA molecule and the second gRNA molecule include targeting domains comprising, e.g., consisting of, SEQ ID NO: 1675 and SEQ ID NO: 1619, respectively. In an aspect, the first gRNA molecule and the second gRNA molecule include targeting domains comprising, e.g., consisting of, SEQ ID NO: 1675 and SEQ ID NO: 1645, respectively. In an aspect, the first gRNA molecule and the second gRNA molecule include targeting domains comprising, e.g., consisting of, SEQ ID NO: 1675 and SEQ ID NO: 1598, respectively. In an aspect, the first gRNA molecule and the second gRNA molecule include targeting domains comprising, e.g., consisting of, SEQ ID NO: 1675 and SEQ ID NO: 1599, respectively. In an aspect, the first gRNA molecule and the second gRNA molecule include targeting domains comprising, e.g., consisting of, SEQ ID NO: 1675 and SEQ ID NO: 1663, respectively. In an aspect, the first gRNA molecule and the second gRNA molecule include targeting domains comprising, e.g., consisting of, SEQ ID NO: 1675 and SEQ ID NO: 1677, respectively. In an aspect, the first gRNA molecule and the second gRNA molecule include targeting domains comprising, e.g., consisting of, SEQ ID NO: 1675 and SEQ ID NO: 1626, respectively. In an aspect, the first gRNA molecule and the second gRNA molecule include targeting domains comprising, e.g., consisting of, SEQ ID NO: 1645 and SEQ ID NO: 1638, respectively. In an aspect, the first gRNA molecule and the second gRNA molecule include targeting domains comprising, e.g., consisting of, SEQ ID NO: 1645 and SEQ ID NO: 1647, respectively. In an aspect, the first gRNA molecule and the second gRNA molecule include targeting domains comprising, e.g., consisting of, SEQ ID NO: 1645 and SEQ ID NO: 1609, respectively. In an aspect, the first gRNA molecule and the second gRNA molecule include targeting domains comprising, e.g., consisting of, SEQ ID NO: 1645 and SEQ ID NO: 1621, respectively. In an aspect, the first gRNA molecule and the second gRNA molecule include targeting domains comprising, e.g., consisting of, SEQ ID NO: 1645 and SEQ ID NO: 1617, respectively. In an aspect, the first gRNA molecule and the second gRNA molecule include targeting domains comprising, e.g., consisting of, SEQ ID NO: 1645 and SEQ ID NO: 1654, respectively. In an aspect, the first gRNA molecule and the second gRNA molecule include targeting domains comprising, e.g., consisting of, SEQ ID NO: 1645 and SEQ ID NO: 1631, respectively. In an aspect, the first gRNA molecule and the second gRNA molecule include targeting domains comprising, e.g., consisting of, SEQ ID NO: 1645 and SEQ ID NO: 1620, respectively. In an aspect, the first gRNA molecule and the second gRNA molecule include targeting domains comprising, e.g., consisting of, SEQ ID NO: 1645 and SEQ ID NO: 1637, respectively. In an aspect, the first gRNA molecule and the second gRNA molecule include targeting domains comprising, e.g., consisting of, SEQ ID NO: 1645 and SEQ ID NO: 1612, respectively. In an aspect, the first gRNA molecule and the second gRNA molecule include targeting domains comprising, e.g., consisting of, SEQ ID NO: 1645 and SEQ ID NO: 1656, respectively. In an aspect, the first gRNA molecule and the second gRNA molecule include targeting domains comprising, e.g., consisting of, SEQ ID NO: 1645 and SEQ ID NO: 1619, respectively. In an aspect, the first gRNA molecule and the second gRNA molecule include targeting domains comprising, e.g., consisting of, SEQ ID NO: 1645 and SEQ ID NO: 1675, respectively. In an aspect, the first gRNA molecule and the second gRNA molecule include targeting domains comprising, e.g., consisting of, SEQ ID NO: 1598 and SEQ ID NO: 1638, respectively. In an aspect, the first gRNA molecule and the second gRNA molecule include targeting domains comprising, e.g., consisting of, SEQ ID NO: 1598 and SEQ ID NO: 1647, respectively. In an aspect, the first gRNA molecule and the second gRNA molecule include targeting domains comprising, e.g., consisting of, SEQ ID NO: 1598 and SEQ ID NO: 1609, respectively. In an aspect, the first gRNA molecule and the second gRNA molecule include targeting domains comprising, e.g., consisting of, SEQ ID NO: 1598 and SEQ ID NO: 1621, respectively. In an aspect, the first gRNA molecule and the second gRNA molecule include targeting domains comprising, e.g., consisting of, SEQ ID NO: 1598 and SEQ ID NO: 1617, respectively. In an aspect, the first gRNA molecule and the second gRNA molecule include targeting domains comprising, e.g., consisting of, SEQ ID NO: 1598 and SEQ ID NO: 1654, respectively. In an aspect, the first gRNA molecule and the second gRNA molecule include targeting domains comprising, e.g., consisting of, SEQ ID NO: 1598 and SEQ ID NO: 1631, respectively. In an aspect, the first gRNA molecule and the second gRNA molecule include targeting domains comprising, e.g., consisting of, SEQ ID NO: 1598 and SEQ ID NO: 1620, respectively. In an aspect, the first gRNA molecule and the second gRNA molecule include targeting domains comprising, e.g., consisting of, SEQ ID NO: 1598 and SEQ ID NO: 1637, respectively. In an aspect, the first gRNA molecule and the second gRNA molecule include targeting domains comprising, e.g., consisting of, SEQ ID NO: 1598 and SEQ ID NO: 1612, respectively. In an aspect, the first gRNA molecule and the second gRNA molecule include targeting domains comprising, e.g., consisting of, SEQ ID NO: 1598 and SEQ ID NO: 1656, respectively. In an aspect, the first gRNA molecule and the second gRNA molecule include targeting domains comprising, e.g., consisting of, SEQ ID NO: 1598 and SEQ ID NO: 1619, respectively. In an aspect, the first gRNA molecule and the second gRNA molecule include targeting domains comprising, e.g., consisting of, SEQ ID NO: 1598 and SEQ ID NO: 1675, respectively. In an aspect, the first gRNA molecule and the second gRNA molecule include targeting domains comprising, e.g., consisting of, SEQ ID NO: 1598 and SEQ ID NO: 1645, respectively. In an aspect, the first gRNA molecule and the second gRNA molecule include targeting domains comprising, e.g., consisting of, SEQ ID NO: 1598 and SEQ ID NO: 1599, respectively. In an aspect, the first gRNA molecule and the second gRNA molecule include targeting domains comprising, e.g., consisting of, SEQ ID NO: 1598 and SEQ ID NO: 1663, respectively. In an aspect, the first gRNA molecule and the second gRNA molecule include targeting domains comprising, e.g., consisting of, SEQ ID NO: 1598 and SEQ ID NO: 1677, respectively. In an aspect, the first gRNA molecule and the second gRNA molecule include targeting domains comprising, e.g., consisting of, SEQ ID NO: 1598 and SEQ ID NO: 1626, respectively. In an aspect, the first gRNA molecule and the second gRNA molecule include targeting domains comprising, e.g., consisting of, SEQ ID NO: 1599 and SEQ ID NO: 1638, respectively. In an aspect, the first gRNA molecule and the second gRNA molecule include targeting domains comprising, e.g., consisting of, SEQ ID NO: 1599 and SEQ ID NO: 1647, respectively. In an aspect, the first gRNA molecule and the second gRNA molecule include targeting domains comprising, e.g., consisting of, SEQ ID NO: 1599 and SEQ ID NO: 1609, respectively. In an aspect, the first gRNA molecule and the second gRNA molecule include targeting domains comprising, e.g., consisting of, SEQ ID NO: 1599 and SEQ ID NO: 1621, respectively. In an aspect, the first gRNA molecule and the second gRNA molecule include targeting domains comprising, e.g., consisting of, SEQ ID NO: 1599 and SEQ ID NO: 1617, respectively. In an aspect, the first gRNA molecule and the second gRNA molecule include targeting domains comprising, e.g., consisting of, SEQ ID NO: 1599 and SEQ ID NO: 1654, respectively. In an aspect, the first gRNA molecule and the second gRNA molecule include targeting domains comprising, e.g., consisting of, SEQ ID NO: 1599 and SEQ ID NO: 1631, respectively. In an aspect, the first gRNA molecule and the second gRNA molecule include targeting domains comprising, e.g., consisting of, SEQ ID NO: 1599 and SEQ ID NO: 1620, respectively. In an aspect, the first gRNA molecule and the second gRNA molecule include targeting domains comprising, e.g., consisting of, SEQ ID NO: 1599 and SEQ ID NO: 1637, respectively. In an aspect, the first gRNA molecule and the second gRNA molecule include targeting domains comprising, e.g., consisting of, SEQ ID NO: 1599 and SEQ ID NO: 1612, respectively. In an aspect, the first gRNA molecule and the second gRNA molecule include targeting domains comprising, e.g., consisting of, SEQ ID NO: 1599 and SEQ ID NO: 1656, respectively. In an aspect, the first gRNA molecule and the second gRNA molecule include targeting domains comprising, e.g., consisting of, SEQ ID NO: 1599 and SEQ ID NO: 1619, respectively. In an aspect, the first gRNA molecule and the second gRNA molecule include targeting domains comprising, e.g., consisting of, SEQ ID NO: 1599 and SEQ ID NO: 1675, respectively. In an aspect, the first gRNA molecule and the second gRNA molecule include targeting domains comprising, e.g., consisting of, SEQ ID NO: 1599 and SEQ ID NO: 1645, respectively. In an aspect, the first gRNA molecule and the second gRNA molecule include targeting domains comprising, e.g., consisting of, SEQ ID NO: 1599 and SEQ ID NO: 1598, respectively. In an aspect, the first gRNA molecule and the second gRNA molecule include targeting domains comprising, e.g., consisting of, SEQ ID NO: 1599 and SEQ ID NO: 1663, respectively. In an aspect, the first gRNA molecule and the second gRNA molecule include targeting domains comprising, e.g., consisting of, SEQ ID NO: 1599 and SEQ ID NO: 1677, respectively. In an aspect, the first gRNA molecule and the second gRNA molecule include targeting domains comprising, e.g., consisting of, SEQ ID NO: 1599 and SEQ ID NO: 1626, respectively. In an aspect, the first gRNA molecule and the second gRNA molecule include targeting domains comprising, e.g., consisting of, SEQ ID NO: 1663 and SEQ ID NO: 1638, respectively. In an aspect, the first gRNA molecule and the second gRNA molecule include targeting domains comprising, e.g., consisting of, SEQ ID NO: 1663 and SEQ ID NO: 1647, respectively. In an aspect, the first gRNA molecule and the second gRNA molecule include targeting domains comprising, e.g., consisting of, SEQ ID NO: 1663 and SEQ ID NO: 1609, respectively. In an aspect, the first gRNA molecule and the second gRNA molecule include targeting domains comprising, e.g., consisting of, SEQ ID NO: 1663 and SEQ ID NO: 1621, respectively. In an aspect, the first gRNA molecule and the second gRNA molecule include targeting domains comprising, e.g., consisting of, SEQ ID NO: 1663 and SEQ ID NO: 1617, respectively. In an aspect, the first gRNA molecule and the second gRNA molecule include targeting domains comprising, e.g., consisting of, SEQ ID NO: 1663 and SEQ ID NO: 1654, respectively. In an aspect, the first gRNA molecule and the second gRNA molecule include targeting domains comprising, e.g., consisting of, SEQ ID NO: 1663 and SEQ ID NO: 1631, respectively. In an aspect, the first gRNA molecule and the second gRNA molecule include targeting domains comprising, e.g., consisting of, SEQ ID NO: 1663 and SEQ ID NO: 1620, respectively. In an aspect, the first gRNA molecule and the second gRNA molecule include targeting domains comprising, e.g., consisting of, SEQ ID NO: 1663 and SEQ ID NO: 1637, respectively. In an aspect, the first gRNA molecule and the second gRNA molecule include targeting domains comprising, e.g., consisting of, SEQ ID NO: 1663 and SEQ ID NO: 1612, respectively. In an aspect, the first gRNA molecule and the second gRNA molecule include targeting domains comprising, e.g., consisting of, SEQ ID NO: 1663 and SEQ ID NO: 1656, respectively. In an aspect, the first gRNA molecule and the second gRNA molecule include targeting domains comprising, e.g., consisting of, SEQ ID NO: 1663 and SEQ ID NO: 1619, respectively. In an aspect, the first gRNA molecule and the second gRNA molecule include targeting domains comprising, e.g., consisting of, SEQ ID NO: 1663 and SEQ ID NO: 1675, respectively. In an aspect, the first gRNA molecule and the second gRNA molecule include targeting domains comprising, e.g., consisting of, SEQ ID NO: 1663 and SEQ ID NO: 1645, respectively. In an aspect, the first gRNA molecule and the second gRNA molecule include targeting domains comprising, e.g., consisting of, SEQ ID NO: 1663 and SEQ ID NO: 1598, respectively. In an aspect, the first gRNA molecule and the second gRNA molecule include targeting domains comprising, e.g., consisting of, SEQ ID NO: 1663 and SEQ ID NO: 1599, respectively. In an aspect, the first gRNA molecule and the second gRNA molecule include targeting domains comprising, e.g., consisting of, SEQ ID NO: 1663 and SEQ ID NO: 1677, respectively. In an aspect, the first gRNA molecule and the second gRNA molecule include targeting domains comprising, e.g., consisting of, SEQ ID NO: 1663 and SEQ ID NO: 1626, respectively. In an aspect, the first gRNA molecule and the second gRNA molecule include targeting domains comprising, e.g., consisting of, SEQ ID NO: 1677 and SEQ ID NO: 1638, respectively. In an aspect, the first gRNA molecule and the second gRNA molecule include targeting domains comprising, e.g., consisting of, SEQ ID NO: 1677 and SEQ ID NO: 1647, respectively. In an aspect, the first gRNA molecule and the second gRNA molecule include targeting domains comprising, e.g., consisting of, SEQ ID NO: 1677 and SEQ ID NO: 1609, respectively. In an aspect, the first gRNA molecule and the second gRNA molecule include targeting domains comprising, e.g., consisting of, SEQ ID NO: 1677 and SEQ ID NO: 1621, respectively. In an aspect, the first gRNA molecule and the second gRNA molecule include targeting domains comprising, e.g., consisting of, SEQ ID NO: 1677 and SEQ ID NO: 1617, respectively. In an aspect, the first gRNA molecule and the second gRNA molecule include targeting domains comprising, e.g., consisting of, SEQ ID NO: 1677 and SEQ ID NO: 1654, respectively. In an aspect, the first gRNA molecule and the second gRNA molecule include targeting domains comprising, e.g., consisting of, SEQ ID NO: 1677 and SEQ ID NO: 1631, respectively. In an aspect, the first gRNA molecule and the second gRNA molecule include targeting domains comprising, e.g., consisting of, SEQ ID NO: 1677 and SEQ ID NO: 1620, respectively. In an aspect, the first gRNA molecule and the second gRNA molecule include targeting domains comprising, e.g., consisting of, SEQ ID NO: 1677 and SEQ ID NO: 1637, respectively. In an aspect, the first gRNA molecule and the second gRNA molecule include targeting domains comprising, e.g., consisting of, SEQ ID NO: 1677 and SEQ ID NO: 1612, respectively. In an aspect, the first gRNA molecule and the second gRNA molecule include targeting domains comprising, e.g., consisting of, SEQ ID NO: 1677 and SEQ ID NO: 1656, respectively. In an aspect, the first gRNA molecule and the second gRNA molecule include targeting domains comprising, e.g., consisting of, SEQ ID NO: 1677 and SEQ ID NO: 1619, respectively. In an aspect, the first gRNA molecule and the second gRNA molecule include targeting domains comprising, e.g., consisting of, SEQ ID NO: 1677 and SEQ ID NO: 1675, respectively. In an aspect, the first gRNA molecule and the second gRNA molecule include targeting domains comprising, e.g., consisting of, SEQ ID NO: 1677 and SEQ ID NO: 1645, respectively. In an aspect, the first gRNA molecule and the second gRNA molecule include targeting domains comprising, e.g., consisting of, SEQ ID NO: 1677 and SEQ ID NO: 1598, respectively. In an aspect, the first gRNA molecule and the second gRNA molecule include targeting domains comprising, e.g., consisting of, SEQ ID NO: 1677 and SEQ ID NO: 1599, respectively. In an aspect, the first gRNA molecule and the second gRNA molecule include targeting domains comprising, e.g., consisting of, SEQ ID NO: 1677 and SEQ ID NO: 1663, respectively. In an aspect, the first gRNA molecule and the second gRNA molecule include targeting domains comprising, e.g., consisting of, SEQ ID NO: 1677 and SEQ ID NO: 1626, respectively. In an aspect, the first gRNA molecule and the second gRNA molecule include targeting domains comprising, e.g., consisting of, SEQ ID NO: 1626 and SEQ ID NO: 1638, respectively. In an aspect, the first gRNA molecule and the second gRNA molecule include targeting domains comprising, e.g., consisting of, SEQ ID NO: 1626 and SEQ ID NO: 1647, respectively. In an aspect, the first gRNA molecule and the second gRNA molecule include targeting domains comprising, e.g., consisting of, SEQ ID NO: 1626 and SEQ ID NO: 1609, respectively. In an aspect, the first gRNA molecule and the second gRNA molecule include targeting domains comprising, e.g., consisting of, SEQ ID NO: 1626 and SEQ ID NO: 1621, respectively. In an aspect, the first gRNA molecule and the second gRNA molecule include targeting domains comprising, e.g., consisting of, SEQ ID NO: 1626 and SEQ ID NO: 1617, respectively. In an aspect, the first gRNA molecule and the second gRNA molecule include targeting domains comprising, e.g., consisting of, SEQ ID NO: 1626 and SEQ ID NO: 1654, respectively. In an aspect, the first gRNA molecule and the second gRNA molecule include targeting domains comprising, e.g., consisting of, SEQ ID NO: 1626 and SEQ ID NO: 1631, respectively. In an aspect, the first gRNA molecule and the second gRNA molecule include targeting domains comprising, e.g., consisting of, SEQ ID NO: 1626 and SEQ ID NO: 1620, respectively. In an aspect, the first gRNA molecule and the second gRNA molecule include targeting domains comprising, e.g., consisting of, SEQ ID NO: 1626 and SEQ ID NO: 1637, respectively. In an aspect, the first gRNA molecule and the second gRNA molecule include targeting domains comprising, e.g., consisting of, SEQ ID NO: 1626 and SEQ ID NO: 1612, respectively. In an aspect, the first gRNA molecule and the second gRNA molecule include targeting domains comprising, e.g., consisting of, SEQ ID NO: 1626 and SEQ ID NO: 1656, respectively. In an aspect, the first gRNA molecule and the second gRNA molecule include targeting domains comprising, e.g., consisting of, SEQ ID NO: 1626 and SEQ ID NO: 1619, respectively. In an aspect, the first gRNA molecule and the second gRNA molecule include targeting domains comprising, e.g., consisting of, SEQ ID NO: 1626 and SEQ ID NO: 1675, respectively. In an aspect, the first gRNA molecule and the second gRNA molecule include targeting domains comprising, e.g., consisting of, SEQ ID NO: 1626 and SEQ ID NO: 1645, respectively. In an aspect, the first gRNA molecule and the second gRNA molecule include targeting domains comprising, e.g., consisting of, SEQ ID NO: 1626 and SEQ ID NO: 1598, respectively. In an aspect, the first gRNA molecule and the second gRNA molecule include targeting domains comprising, e.g., consisting of, SEQ ID NO: 1626 and SEQ ID NO: 1599, respectively. In an aspect, the first gRNA molecule and the second gRNA molecule include targeting domains comprising, e.g., consisting of, SEQ ID NO: 1626 and SEQ ID NO: 1663, respectively. In an aspect, the first gRNA molecule and the second gRNA molecule include targeting domains comprising, e.g., consisting of, SEQ ID NO: 1626 and SEQ ID NO: 1677, respectively.

### III. Methods for Designing gRNAs

Methods for designing gRNAs are described herein, including methods for selecting, designing and validating target sequences. Exemplary targeting domains are also provided herein. Targeting Domains discussed herein can be incorporated into the gRNAs described herein.

Methods for selection and validation of target sequences as well as off-target analyses are described, e.g., in. Mali el al., 2013 SCIENCE 339(6121): 823-826; Hsu et al, 2013 NAT BIOTECHNOL, 31 (9): 827-32; Fu et al, 2014 NAT BIOTECHNOL, doi: 10.1038/nbt.2808. PubMed PM ID: 24463574; Heigwer et al, 2014 NAT METHODS 11 (2): 122-3. doi: 10.1038/nmeth.2812. PubMed PMID: 24481216; Bae el al, 2014 BIOINFORMATICS PubMed PMID: 24463181 ; Xiao A el al, 2014 BIOINFORMATICS PubMed PMID: 24389662.

For example, a software tool can be used to optimize the choice of gRNA within a user's target sequence, e.g., to minimize total off-target activity across the genome. Off target activity may be other than cleavage. For each possible gRNA choice e.g., using S. pyogenes Cas9, the tool can identify all off-target sequences (e.g., preceding either NAG or NGG PAMs) across the genome that contain up to certain number (e.g., 1 , 2, 3, 4, 5, 6, 7, 8, 9, or 10) of mismatched base-pairs. The cleavage efficiency at each off-target sequence can be predicted, e.g., using an experimentally-derived weighting scheme. Each possible gRNA is then ranked according to its total predicted off-target cleavage; the top-ranked gRNAs represent those that are likely to have the greatest on-target and the least off-target cleavage. Other functions, e.g., automated reagent design for CRISPR construction, primer design for the on-target Surveyor assay, and primer design for high-throughput detection and quantification of off-target cleavage via next-gen sequencing, can also be included in the tool. Candidate gRNA molecules can be evaluated by art-known methods or as described herein.

Although software algorithms may be used to generate an initial list of potential gRNA molecules, cutting efficiency and specificity will not necessarily reflect the predicted values, and gRNA molecules typically require screening in specific cell lines, e.g., primary human cell lines, e.g., human HSPCs, e.g., human CD34+ cells, to determine, for example, cutting efficiency, indel formation, cutting specificity and change in desired phenotype. These properties may be assayed by the methods described herein.

In aspects of the invention, a gRNA comprising the targeting domain of CR00312 (SEQ ID NO: 248, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR00312 #1: 
GUUUGGCCUCUGAUUAGGGUGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 342)
sgRNA CR00312 #2: 
mG*mU*mU*UGGCCUCUGAUUAGGGUGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 343)
sgRNA CR00312 #3: 
mG*mU*mU*UGGCCUCUGAUUAGGGUGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1762))
dgRNA CR00312 #1: 
crRNA: GUUUGGCCUCUGAUUAGGGUGUUUUAGAGCUAUGCUGUUUUG (SEQ ID NO:
344)
tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR00312 #2:
   crRNA: 
mG*mU*mU*UGGCCUCUGAUUAGGGUGUUUUAGAGCUAUGCUGUU*mU*mU*mG (SEQ
ID NO: 345)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR00312 #3:
   crRNA: 
mG*mU*mU*UGGCCUCUGAUUAGGGUGUUUUAGAGCUAUGCUGUU*mU*mU*mG (SEQ
ID NO: 345)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001128 (SEQ ID NO: 338, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001128 #1: 
AUCAGAGGCCAAACCCUUCCGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 347)
sgRNA CR001128 #2: 
mA*mU*mC*AGAGGCCAAACCCUUCCGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 348)
sgRNA CR001128 #3: 
mA*mU*mC*AGAGGCCAAACCCUUCCGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1763)
dgRNA CR001128 #1:
   crRNA: AUCAGAGGCCAAACCCUUCCGUUUUAGAGCUAUGCUGUUUUG (SEQ ID NO: 349)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001128 #2:
   crRNA: mA^{∗}mU^{∗}mC^{∗}AGAGGCCAAACCCUUCCGUUUUAGAGCUAUGCUGUU^{∗}mU^{∗}mU^{∗}mG (SEQ ID NO: 350)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001128 #3:
   crRNA: mA^{*}mU^{*}mC^{*}AGAGGCCAAACCCUUCCGUUUUAGAGCUAUGCUGUU^{*}mU^{*}mU^{*}mG (SEQ ID NO: 350)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001126 (SEQ ID NO: 336, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001126 #1: 
UUUAUCACAGGCUCCAGGAAGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 351)
sgRNA CR001126 #2: 
mU*mU*mU*AUCACAGGCUCCAGGAAGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 352)
sgRNA CR001126 #3: 
mU*mU*mU*AUCACAGGCUCCAGGAAGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1764)
dgRNA CR001126 #1:
   crRNA: UUUAUCACAGGCUCCAGGAAGUUUUAGAGCUAUGCUGUUUUG (SEQ ID NO: 353)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001126 #2:
   crRNA: mU^{∗}mU^{∗}mU^{∗}AUCACAGGCUCCAGGAAGUUUUAGAGCUAUGCUGUU^{∗}mU^{∗}mU^{∗}mG (SEQ ID NO: 354)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001126 #3:
   crRNA: mU^{∗}mU^{∗}mU^{∗}AUCACAGGCUCCAGGAAGUUUUAGAGCUAUGCUGUU^{∗}mU^{∗}mU^{∗}mG (SEQ ID NO: 354)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR00311 (SEQ ID NO: 247, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR00311 #1: 
UUUGGCCUCUGAUUAGGGUGGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 355)
sgRNA CR00311 #2: 
mU*mU*mU*GGCCUCUGAUUAGGGUGGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 356)
sgRNA CR00311 #3: 
mU*mU*mU*GGCCUCUGAUUAGGGUGGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1765)
dgRNA CR00311 #1:
   crRNA: UUUGGCCUCUGAUUAGGGUGGUUUUAGAGCUAUGCUGUUUUG (SEQ ID NO: 357)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR00311 #2:
   crRNA:
      mU^{∗}mU^{∗}mU^{∗}GGCCUCUGAUUAGGGUGGUUUUAGAGCUAUGCUGUU^{∗}mU^{∗}mU^{∗}mG (SEQ ID NO: 358)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR00311 #3:
   crRNA:
      mU^{∗}mU^{∗}mU^{∗}GGCCUCUGAUUAGGGUGGUUUUAGAGCUAUGCUGUU^{∗}mU^{∗}mU^{∗}mG (SEQ ID NO: 358)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR00309 (SEQ ID NO: 245, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR00309 #1: 
CACGCCCCCACCCUAAUCAGGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 359)
sgRNA CR00309 #2: 
mC*mA*mC*GCCCCCACCCUAAUCAGGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 360)
sgRNA CR00309 #3: 
mC*mA*mC*GCCCCCACCCUAAUCAGGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1766)
dgRNA CR00309 #1:
   crRNA: CACGCCCCCACCCUAAUCAGGUUUUAGAGCUAUGCUGUUUUG (SEQ ID NO: 361)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR00309 #2:
   crRNA: mC^{∗}mA^{∗}mC^{∗}GCCCCCACCCUAAUCAGGUUUUAGAGCUAUGCUGUU^{*}mU^{*}mU^{*}mG (SEQ ID NO: 362)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR00309 #3:
   crRNA: mC^{*}mA^{*}mC^{*}GCCCCCACCCUAAUCAGGUUUUAGAGCUAUGCUGUU^{∗}mU^{∗}mU^{∗}mG (SEQ ID NO: 362)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001127 (SEQ ID NO: 337, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001127 #1: 
CACAGGCUCCAGGAAGGGUUGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 363)
sgRNA CR001127 #2: 
mC*mA*mC*AGGCUCCAGGAAGGGUUGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 364)
sgRNA CR001127 #3: 
mC*mA*mC*AGGCUCCAGGAAGGGUUGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1767)
dgRNA CR001127 #1:
   crRNA: CACAGGCUCCAGGAAGGGUUGUUUUAGAGCUAUGCUGUUUUG (SEQ ID NO: 365)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001127 #2:
   crRNA: mC^{∗}mA^{∗}mC^{∗}AGGCUCCAGGAAGGGUUGUUUUAGAGCUAUGCUGUU^{∗}mU^{∗}mU^{∗}mG (SEQ ID NO: 366)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001127 #3:
   crRNA: mC^{∗}mA^{∗}mC^{∗}AGGCUCCAGGAAGGGUUGUUUUAGAGCUAUGCUGUU^{∗}mU^{∗}mU^{∗}mG (SEQ ID NO: 366)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR00316 (SEQ ID NO: 252, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR00316 #1: 
UUGCUUUUAUCACAGGCUCCGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 367)
sgRNA CR00316 #2: 
mU*mU*mG*CUUUUAUCACAGGCUCCGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 368)
sgRNA CR00316 #3: 
mU*mU*mG*CUUUUAUCACAGGCUCCGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1768)
dgRNA CR00316 #1:
   crRNA: UUGCUUUUAUCACAGGCUCCGUUUUAGAGCUAUGCUGUUUUG (SEQ ID NO: 369)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR00316 #2:
   crRNA: mU^{∗}mU^{∗}mG^{∗}CUUUUAUCACAGGCUCCGUUUUAGAGCUAUGCUGUU^{∗}mU^{∗}mU^{∗}mG (SEQ ID NO: 370)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR00316 #3:
   crRNA: mU^{∗}mU^{∗}mG^{∗}CUUUUAUCACAGGCUCCGUUUUAGAGCUAUGCUGUU^{∗}mU^{∗}mU^{∗}mG (SEQ ID NO: 370)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001125 (SEQ ID NO: 335, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001125 #1: 
UUUUAUCACAGGCUCCAGGAGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 371)
sgRNA CR001125 #2: 
mU*mU*mU*UAUCACAGGCUCCAGGAGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 372)
sgRNA CR001125 #3: 
mU*mU*mU*UAUCACAGGCUCCAGGAGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1769)
dgRNA CR001125 #1:
   crRNA: UUUUAUCACAGGCUCCAGGAGUUUUAGAGCUAUGCUGUUUUG (SEQ ID NO: 373)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001125 #2:
   crRNA: mU^{∗}mU^{∗}mU^{∗}UAUCACAGGCUCCAGGAGUUUUAGAGCUAUGCUGUU^{∗}mU^{∗}mU^{∗}mG (SEQ ID NO: 374)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001125 #3:
   crRNA: mU^{∗}mU^{∗}mU^{∗}UAUCACAGGCUCCAGGAGUUUUAGAGCUAUGCUGUU^{∗}mU^{∗}mU^{∗}mG (SEQ ID NO: 374)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001030 (SEQ ID NO: 100, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001030 #1: 
ACUGCUGAAAGAGAUGCGGUGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 375)
sgRNA CR001030 #2: 
mA*mC*mU*GCUGAAAGAGAUGCGGUGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 376)
sgRNA CR001030 #3: 
mA*mC*mU*GCUGAAAGAGAUGCGGUGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1770)
dgRNA CR001030 #1:
   crRNA: ACUGCUGAAAGAGAUGCGGUGUUUUAGAGCUAUGCUGUUUUG (SEQ ID NO: 377)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001030 #2:
   crRNA:
      mA^{∗}mC^{∗}mU^{∗}GCUGAAAGAGAUGCGGUGUUUUAGAGCUAUGCUGUU^{∗}mU^{∗}mU^{∗}mG (SEQ ID NO: 378)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001030 #3:
   crRNA:
      mA^{∗}mC^{∗}mU^{∗}GCUGAAAGAGAUGCGGUGUUUUAGAGCUAUGCUGUU^{∗}mU^{∗}mU^{∗}mG (SEQ ID NO: 378)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001028 (SEQ ID NO: 98, underlined below), e.g., one of the gRNA molecules described below, is useful in the CRISPR systems, methods, cells and other aspects and embodiments of the invention , including in aspects involving more than one gRNA molecule, e.g., described herein:
sgRNA CR001028 #1: 
UGCGGUGGGAGAUAUGUAGGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 379)
sgRNA CR001028 #2: 
mU*mG*mC*GGUGGGAGAUAUGUAGGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 380)
sgRNA CR001028 #3: 
mU*mG*mC*GGUGGGAGAUAUGUAGGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1771)
dgRNA CR001028 #1: 
crRNA: UGCGGUGGGAGAUAUGUAGGUUUUAGAGCUAUGCUGUUUUG (SEQ ID NO:
381)
tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001028 #2:
   crRNA:
      mU^{∗}mG^{∗}mC^{∗}GGUGGGGAGAUAUGUAGGUUUUAGAGCUAUGCUGUU^{∗}mU^{∗}mU^{∗}mG (SEQ ID NO: 382)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001028 #3:
   crRNA:
      mU^{∗}mG^{∗}mC^{∗}GGUGGGGAGAUAUGUAGGUUUUAGAGCUAUGCUGUU^{∗}mU^{∗}mU^{∗}mG (SEQ ID NO: 382)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001221 (SEQ ID NO: 1589, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001221 #1: 
GAAACAAUGAGGACCUGACUGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 383)
sgRNA CR001221 #2: 
mG*mA*mA*ACAAUGAGGACCUGACUGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 384)
sgRNA CR001221 #3: 
mG*mA*mA*ACAAUGAGGACCUGACUGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1772)
dgRNA CR001221 #1:
   crRNA: GAAACAAUGAGGACCUGACUGUUUUAGAGCUAUGCUGUUUUG (SEQ ID NO: 385)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001221 #2:
   crRNA:
      mG^{∗}mA^{∗}mA^{∗}ACAAUGAGGACCUGACUGUUUUAGAGCUAUGCUGUU^{∗}mU^{∗}mU^{∗}mG (SEQ ID NO: 386)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001221 #3:
   crRNA:
      mG^{∗}mA^{∗}mA^{∗}ACAAUGAGGACCUGACUGUUUUAGAGCUAUGCUGUU^{∗}mU^{∗}mU^{∗}mG (SEQ ID NO: 386)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR001137 (SEQ ID NO: 1505, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR001137 #1: 
GUAAGCAUUUAAGUGGCUACGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 387)
sgRNA CR001137 #2: 
mG*mU*mA*AGCAUUUAAGUGGCUACGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 388)
sgRNA CR001137 #3: 
mG*mU*mA*AGCAUUUAAGUGGCUACGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1773)
dgRNA CR001137 #1:
   crRNA: GUAAGCAUUUAAGUGGCUACGUUUUAGAGCUAUGCUGUUUUG (SEQ ID NO: 389)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR001137 #2:
   crRNA:
      mG^{∗}mU^{∗}mA^{∗}AGCAUUUAAGUGGCUACGUUUUAGAGCUAUGCUGUU^{∗}mU^{∗}mU^{∗}mG (SEQ ID NO: 390)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR001137 #3:
   crRNA:
      mG^{∗}mU^{∗}mA^{∗}AGCAUUUAAGUGGCUACGUUUUAGAGCUAUGCUGUU^{∗}mU^{∗}mU^{∗}mG (SEQ ID NO: 390)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR003035 (SEQ ID NO: 1505, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR003035 #1: 
AGGCACCUCAGACUCAGCAUGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 391)
sgRNA CR003035 #2: 
mA*mG*mG*CACCUCAGACUCAGCAUGUUUUAGAGCUAGAAAUAGCAAGUUAAAAUAA
GGCUAGUCCGUUAUCAACUUGAAAAAGUGGCACCGAGUCGGUGCU*mU*mU*mU (SEQ
ID NO: 392)
sgRNA CR003035 #3: 
mA*mG*mG*CACCUCAGACUCAGCAUGUUUUAGAGCUAGAAAUAGCAAGUUAAAAUAA
GGCUAGUCCGUUAUCAACUUGAAAAAGUGGCACCGAGUCGGUGCmU*mU*mU*U (SEQ
ID NO: 1774)
dgRNA CR003035 #1:
   crRNA: AGGCACCUCAGACUCAGCAUGUUUUAGAGCUAUGCUGUUUUG (SEQ ID NO: 393)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR003035 #2:
   crRNA: mA^{∗}mG^{∗}mG^{∗}CACCUCAGACUCAGCAUGUUUUAGAGCUAUGCUGUU^{∗}mU^{∗}mU^{∗}mG (SEQ ID NO: 394)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR003035 #3:
   crRNA: mA^{∗}mG^{∗}mG^{∗}CACCUCAGACUCAGCAUGUUUUAGAGCUAUGCUGUU^{∗}mU^{∗}mU^{∗}mG (SEQ ID NO: 394)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In aspects of the invention, a gRNA comprising the targeting domain of CR003085 (SEQ ID NO: 1750, underlined below), e.g., one of the gRNA molecules described below, is useful in the CRISPR systems, methods, cells and other aspects and embodiments of the invention, including in aspects involving more than one gRNA molecule, e.g., described herein:
sgRNA CR003085 #1: 
AUGGUAUGGAGGUAUACUAGUUUUAGAGCUAGAAAUAGCAAGUUAAAAUAAGGCUA
GUCCGUUAUCAACUUGAAAAAGUGGCACCGAGUCGGUGCUUUU (SEQ ID NO: 395)
sgRNA CR003085 #2: 
mA*mU*mG*GUAUGGAGGUAUACUAGUUUUAGAGCUAGAAAUAGCAAGUUAAAAUA
AGGCUAGUCCGUUAUCAACUUGAAAAAGUGGCACCGAGUCGGUGCU*mU*mU*mU
(SEQ ID NO: 396)
sgRNA CR003085 #3: 
mA*mU*mG*GUAUGGAGGUAUACUAGUUUUAGAGCUAGAAAUAGCAAGUUAAAAUA
AGGCUAGUCCGUUAUCAACUUGAAAAAGUGGCACCGAGUCGGUGCmU*mU*mU*U
(SEQ ID NO: 1775)
dgRNA CR003085 #1:
   crRNA: AUGGUAUGGGAGGUAUACUAGUUUUAGAGCUAUGCUGUUUUG (SEQ ID NO: 397)
   tracr: 
AACAGCAUAGCAAGUUAAAAUAAGGCUAGUCCGUUAUCAACUUGAAAAAGUGGCACC
GAGUCGGUGCUUUUUUU (SEQ ID NO: 6660)
dgRNA CR003085 #2:
   crRNA:
      mA^{∗}mU^{∗}mG^{∗}GUAUGGGAGGUAUACUAGUUUUAGAGCUAUGCUGUU^{∗}mU^{∗}mU^{∗}mG (SEQ ID NO: 398)
   tracr: 
mA*mA*mC*AGCAUAGCAAGUUAAAAUAAGGCUAGUCCGUUAUCAACUUGAAAAAGUG
GCACCGAGUCGGUGCUUUU*mU*mU*mU (SEQ ID NO: 346)
dgRNA CR003085 #3:
   crRNA:
      mA^{∗}mU^{∗}mG^{∗}GUAUGGGAGGUAUACUAGUUUUAGAGCUAUGCUGUU^{∗}mU^{∗}mU^{∗}mG (SEQ ID NO: 398)
   tracr:

AACAGCAUAGCAAGUUAAAAUAAGGCUAGUCCGUUAUCAACUUGAAAAAGUGGCACC
GAGUCGGUGCUUUUUUU (SEQ ID NO: 6660).

In each of the gRNA molecules described above, a "^{∗}" denotes a phosphorothioate bond between the adjacent nucleotides, and "mN" (where N = A, G, C or U) denotes a 2'-OMe modified nucleotide. In embodiments, any of the gRNA molecules described herein, e.g., described above, is complexed with a Cas9 molecule, e.g., as described herein, to form a ribonuclear protein complex (RNP). Such RNPs are particularly useful in the methods, cells, and other aspects and embodiments of the invention, e.g., described herein.

### IV. Cas Molecules

### Cas9 Molecules

In preferred embodiments, the Cas molecule is a Cas9 molecule. Cas9 molecules of a variety of species can be used in the methods and compositions described herein. While the S. pyogenes Cas9 molecule are the subject of much of the disclosure herein, Cas9 molecules of, derived from, or based on the Cas9 proteins of other species listed herein can be used as well. In other words, other Cas9 molecules, e.g., S. thermophilus, Staphylococcus aureus and/or Neisseria meningitidis Cas9 molecules, may be used in the systems, methods and compositions described herein. Additional Cas9 species include: Acidovorax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhiz' obium sp., Brevibacillus latemsporus, Campylobacter coli, Campylobacter jejuni, Campylobacter lad, Candidatus Puniceispirillum, Clostridiu cellulolyticum, Clostridium perfringens, Corynebacterium accolens, Corynebacterium diphtheria, Corynebacterium matruchotii, Dinoroseobacter sliibae, Eubacterium dolichum, gamma proteobacterium, Gluconacetobacler diazotrophicus, Haemophilus parainfluenzae, Haemophilus sputorum, Helicobacter canadensis, Helicobacter cinaedi, Helicobacter mustelae, Ilyobacler polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica. Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tislrella mobilis, Treponema sp., or Verminephrobacter eiseniae.

A Cas9 molecule, as that term is used herein, refers to a molecule that can interact with a gRNA molecule (e.g., sequence of a domain of a tracr) and, in concert with the gRNA molecule, localize (e.g., target or home) to a site which comprises a target sequence and PAM sequence.

In an embodiment, the Cas9 molecule is capable of cleaving a target nucleic acid molecule, which may be referred to herein as an active Cas9 molecule. In an embodiment, an active Cas9 molecule, comprises one or more of the following activities: a nickase activity, i.e., .the ability to cleave a single strand, e.g., the non-complementary strand or the complementary strand, of a nucleic acid molecule; a double stranded nuclease activity, i.e., the ability to cleave both strands of a double stranded nucleic acid and create a double stranded break, which in an embodiment is the presence of two nickase activities; an endonuclease activity; an exonuclease activity; and a helicase activity, i.e., the ability to unwind the helical structure of a double stranded nucleic acid.

In an embodiment, an enzymatically active Cas9 molecule cleaves both DNA strands and results in a double stranded break. In an embodiment, a Cas9 molecule cleaves only one strand, e.g., the strand to which the gRNA hybridizes to, or the strand complementary to the strand the gRNA hybridizes with. In an embodiment, an active Cas9 molecule comprises cleavage activity associated with an HNH-like domain. In an embodiment, an active Cas9 molecule comprises cleavage activity associated with an N-terminal RuvC-like domain. In an embodiment, an active Cas9 molecule comprises cleavage activity associated with an HNH-like domain and cleavage activity associated with an N-terminal RuvC-like domain. In an embodiment, an active Cas9 molecule comprises an active, or cleavage competent, HNH-like domain and an inactive, or cleavage incompetent, N-terminal RuvC-like domain. In an embodiment, an active Cas9 molecule comprises an inactive, or cleavage incompetent, HNH-like domain and an active, or cleavage competent, N-terminal RuvC-like domain.

In an embodiment, the ability of an active Cas9 molecule to interact with and cleave a target nucleic acid is PAM sequence dependent. A PAM sequence is a sequence in the target nucleic acid. In an embodiment, cleavage of the target nucleic acid occurs upstream from the PAM sequence. Active Cas9 molecules from different bacterial species can recognize different sequence motifs (e.g., PAM sequences). In an embodiment, an active Cas9 molecule of S. pyogenes recognizes the sequence motif NGG and directs cleavage of a target nucleic acid sequence 1 to 10, e.g., 3 to 5, base pairs upstream from that sequence. See, e.g., Mali el ai, SCIENCE 2013; 339(6121): 823- 826. In an embodiment, an active Cas9 molecule of S. thermophilus recognizes the sequence motif NGGNG and NNAG AAW (W = A or T) and directs cleavage of a core target nucleic acid sequence 1 to 10, e.g., 3 to 5, base pairs upstream from these sequences. See, e.g., Horvath et al., SCIENCE 2010; 327(5962): 167- 170, and Deveau et al, J BACTERIOL 2008; 190(4): 1390- 1400. In an embodiment, an active Cas9 molecule of S. mulans recognizes the sequence motif NGG or NAAR (R - A or G) and directs cleavage of a core target nucleic acid sequence 1 to 10, e.g., 3 to 5 base pairs, upstream from this sequence. See, e.g., Deveau et al. , J BACTERIOL 2008; 190(4): 1 390- 1400.

In an embodiment, an active Cas9 molecule of S. aureus recognizes the sequence motif NNGRR (R = A or G) and directs cleavage of a target nucleic acid sequence 1 to 10, e.g., 3 to 5, base pairs upstream from that sequence. See, e.g., Ran F. et al., NATURE, vol. 520, 2015, pp. 186-191. In an embodiment, an active Cas9 molecule of N. meningitidis recognizes the sequence motif NNNNGATT and directs cleavage of a target nucleic acid sequence 1 to 10, e.g., 3 to 5, base pairs upstream from that sequence. See, e.g., Hou et al., PNAS EARLY EDITION 2013, 1 -6. The ability of a Cas9 molecule to recognize a PAM sequence can be determined, e.g., using a transformation assay described in Jinek et al, SCIENCE 2012, 337:816.

Some Cas9 molecules have the ability to interact with a gRNA molecule, and in conjunction with the gRNA molecule home (e.g., targeted or localized) to a core target domain, but are incapable of cleaving the target nucleic acid, or incapable of cleaving at efficient rates. Cas9 molecules having no, or no substantial, cleavage activity may be referred to herein as an inactive Cas9 (an enzymatically inactive Cas9), a dead Cas9, or a dCas9 molecule. For example, an inactive Cas9 molecule can lack cleavage activity or have substantially less, e.g., less than 20, 10, 5, 1 or 0.1 % of the cleavage activity of a reference Cas9 molecule, as measured by an assay described herein.

Exemplary naturally occurring Cas9 molecules are described in Chylinski et al, RNA Biology 2013; 10:5, 727-737. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 1 1 bacterial family, a cluster 12 bacterial family, a cluster 13 bacterial family, a cluster 14 bacterial family, a cluster 1 bacterial family, a cluster 16 bacterial family, a cluster 17 bacterial family, a cluster 1 8 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family,. a cluster 49 bacterial family, a cluster 50 bacterial family, a cluster 5 1 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: S. pyogenes (e.g., strain SF370, MGAS 10270, MGAS 10750, MGAS2096, MGAS315, MGAS5005, MGAS6180, MGAS9429, NZ131 and SSI- 1), S. thermophilus (e.g., strain LMD-9), S. pseudoporcinus (e.g., strain SPIN 20026), S. mutans (e.g., strain UA 159, NN2025), S. macacae (e.g., strain NCTC1 1558), S. gallolylicus (e.g., strain UCN34, ATCC BAA-2069), S. equines (e.g., strain ATCC 9812, MGCS 124), S. dysdalactiae (e.g., strain GGS 124), S. bovis (e.g., strain ATCC 700338), S. cmginosus (e.g.; strain F021 1 ), S. agalactia^{∗} (e.g., strain NEM316, A909), Listeria monocytogenes (e.g., strain F6854), Listeria innocua (L. innocua, e.g., strain Clip 1 1262), EtUerococcus italicus (e.g., strain DSM 15952), or Enterococcus faecium (e.g., strain 1,231 ,408). Additional exemplary Cas9 molecules are a Cas9 molecule of Neisseria meningitidis (Hou et al. PNAS Early Edition 2013, 1 -6) and a S. aureus Cas9 molecule.

In an embodiment, a Cas9 molecule, e.g., an active Cas9 molecule or inactive Cas9 molecule, comprises an amino acid sequence: having 60%, 65%, 70%, 75%, 80%, 85%, 90%, 95%, 96%, 97%, 98%, or 99% homology with; differs at no more than 1%, 2%, 5%, 10%, 15%, 20%, 30%, or 40% of the amino acid residues when compared with; differs by at least 1, 2, 5, 10 or 20 amino acids but by no more than 100, 80, 70, 60, 50, 40 or 30 amino acids from; or is identical to; any Cas9 molecule sequence described herein or a naturally occurring Cas9 molecule sequence, e.g., a Cas9 molecule from a species listed herein or described in Chylinski et al. , RNA Biology 2013, 10:5, 'I2'I-T,1 Hou et al. PNAS Early Edition 2013, 1-6.

In an embodiment, a Cas9 molecule comprises an amino acid sequence having 60%, 65%, 70%, 75%, 80%, 85%, 90%, 95%, 96%, 97%, 98%, or 99% homology with; differs at no more than 1%, 2%, 5%, 10%, 15%, 20%, 30%, or 40% of the amino acid residues when compared with; differs by at least 1, 2, 5, 10 or 20 amino acids but by no more than 100, 80, 70, 60, 50, 40 or 30 amino acids from; or is identical to; *S. pyogenes* Cas9:


1               5                   10                  15

            20                  25                  30

        35                  40                  45

    50                  55                  60

65                  70                  75                  80

                85                  90                  95

            100                 105                 110

        115                 120                 125


    130                 135                 140

145                 150                 155                 160

                165                 170                 175

            180                 185                 190

        195                 200                 205

    210                 215                 220

225                 230                 235                 240

                245                 250                 255

            260                 265                 270

        275                 280                 285

    290                 295                 300

305                 310                 315                 320

                325                 330                 335

            340                 345                 350

        355                 360                 365

    370                 375                 380

385                 390                 395                 400

                405                 410                 415


            420                 425                 430

        435                 440                 445

    450                 455                 460

465                 470                 475                 480

                485                 490                 495

            500                 505                 510

        515                 520                 525

    530                 535                 540

545                 550                 555                 560

                565                 570                 575

            580                 585                 590

        595                 600                 605

    610                 615                 620

625                 630                 635                 640

                645                 650                 655

            660                 665                 670

        675                 680                 685

    690                 695                 700

705                 710                 715                 720


                725                 730                 735

            740                 745                 750

        755                 760                 765

    770                 775                 780

785                 790                 795                 800

                805                 810                 815

            820                 825                 830

        835                 840                 845

    850                 855                 860

865                 870                 875                 880

                885                 890                 895

            900                 905                 910

        915                 920                 925

    930                 935                 940

945                 950                 955                 960

                965                 970                 975

            980                 985                 990

        995                 1000                1005


1010                1015                1020

1025                1030                1035                1040

                1045                1050                1055

            1060                1065                1070

        1075                1080                1085

    1090                1095                1100

1105                1110                1115                1120

                1125                1130                1135

            1140                1145                1150

        1155                1160                1165

    1170                1175                1180

1185                1190                1195                1200

                1205                1210                1215

            1220                1225                1230

        1235                1240                1245

    1250                1255                1260

1265                1270                1275                1280

                1285                1290                1295

            1300                1305                1310


        1315                1320                1325

    1330                1335                1340

1345                1350                1355                1360

                1365
(SEQ ID NO: 6611)

In embodiments, the Cas9 molecule is a *S. pyogenes* Cas9 variant of SEQ ID NO: 6611 that includes one or more mutations to positively charged amino acids (e.g., lysine, arginine or histidine) that introduce an uncharged or nonpolar amino acid, e.g., alanine, at said position. In embodiments, the mutation is to one or more positively charged amino acids in the nt-groove of Cas9. In embodiments, the Cas9 molecule is a *S. pyogenes* Cas9 variant of SEQ ID NO: 6611 that includes a mutatation at position 855 of SEQ ID NO: 6611, for example a mutation to an uncharged amino acid, e.g., alanine, at position 855 of SEQ ID NO: 6611. In embodiments, the Cas9 molecule has a mutation only at position 855 of SEQ ID NO: 6611, relative to SEQ ID NO: 6611, e.g., to an uncharged amino acid, e.g., alanine. In embodiments, the Cas9 molecule is a *S. pyogenes* Cas9 variant of SEQ ID NO: 6611 that includes a mutatation at position 810, a mutation at position 1003, and/or a mutation at position 1060 of SEQ ID NO: 6611, for example a mutation to alanine at position 810, position 1003, and/or position 1060 of SEQ ID NO: 6611. In embodiments, the Cas9 molecule has a mutation only at position 810, position 1003, and position 1060 of SEQ ID NO: 6611, relative to SEQ ID NO: 6611, e.g., where each mutation is to an uncharged amino acid, for example, alanine. In embodiments, the Cas9 molecule is a *S. pyogenes* Cas9 variant of SEQ ID NO: 6611 that includes a mutatation at position 848, a mutation at position 1003, and/or a mutation at position 1060 of SEQ ID NO: 6611, for example a mutation to alanine at position 848, position 1003, and/or position 1060 of SEQ ID NO: 6611. In embodiments, the Cas9 molecule has a mutation only at position 848, position 1003, and position 1060 of SEQ ID NO: 6611, relative to SEQ ID NO: 6611, e.g., where each mutation is to an uncharged amino acid, for example, alanine. In embodiments, the Cas9 molecule is a Cas9 molecule as described in Slaymaker et al., *Science Express*, available online December 1, 2015 at Science DOI: 10.1126/science.aad5227.

In embodiments, the Cas9 molecule is a *S. pyogenes* Cas9 variant of SEQ ID NO: 6611 that includes one or more mutations. In embodiments, the Cas9 variant comprises a mutation at position 80 of SEQ ID NO: 6611, e.g., includes a leucine at position 80 of SEQ ID NO: 6611 (i.e., comprises, e.g., consists of, SEQ ID NO: 6611 with a C80L mutation). In embodiments, the Cas9 variant comprises a mutation at position 574 of SEQ ID NO: 6611, e.g., includes a glutamic acid at position 574 of SEQ ID NO: 6611 (i.e., comprises, e.g., consists of, SEQ ID NO: 6611 with a C574E mutation). In embodiments, the Cas9 variant comprises a mutation at position 80 and a mutation at position 574 of SEQ ID NO: 6611, e.g., includes a leucine at position 80 of SEQ ID NO: 6611, and a glutamic acid at position 574 of SEQ ID NO: 6611 (i.e., comprises, e.g., consists of, SEQ ID NO: 6611 with a C80L mutation and a C574E mutation). Without being bound by theory, it is believed that such mutations improve the solution properties of the Cas9 molecule.

In embodiments, the Cas9 molecule is a *S. pyogenes* Cas9 variant of SEQ ID NO: 6611 that includes one or more mutations. In embodiments, the Cas9 variant comprises a mutation at position 147 of SEQ ID NO: 6611, e.g., includes a tyrosine at position 147 of SEQ ID NO: 6611 (i.e., comprises, e.g., consists of, SEQ ID NO: 6611 with a D147Y mutation). In embodiments, the Cas9 variant comprises a mutation at position 411 of SEQ ID NO: 6611, e.g., includes a threonine at position 411 of SEQ ID NO: 6611 (i.e., comprises, e.g., consists of, SEQ ID NO: 6611 with a P411T mutation). In embodiments, the Cas9 variant comprises a mutation at position 147 and a mutation at position 411 of SEQ ID NO: 6611, e.g., includes a tyrosine at position 147 of SEQ ID NO: 6611, and a threonine at position 411 of SEQ ID NO: 6611 (i.e., comprises, e.g., consists of, SEQ ID NO: 6611 with a D147Y mutation and a P411T mutation). Without being bound by theory, it is believed that such mutations improve the targeting efficiency of the Cas9 molecule, e.g., in yeast.

In embodiments, the Cas9 molecule is a *S. pyogenes* Cas9 variant of SEQ ID NO: 6611 that includes one or more mutations. In embodiments, the Cas9 variant comprises a mutation at position 1135 of SEQ ID NO: 6611, e.g., includes a glutamic acid at position 1135 of SEQ ID NO: 6611 (i.e., comprises, e.g., consists of, SEQ ID NO: 6611 with a D1135E mutation). Without being bound by theory, it is believed that such mutations improve the selectivity of the Cas9 molecule for the NGG PAM sequence versus the NAG PAM sequence.

In embodiments, the Cas9 molecule is a *S. pyogenes* Cas9 variant of SEQ ID NO: 6611 that includes one or more mutations that introduce an uncharged or nonpolar amino acid, e.g., alanine, at certain positions. In embodiments, the Cas9 molecule is a *S. pyogenes* Cas9 variant of SEQ ID NO: 6611 that includes a mutatation at position 497, a mutation at position 661, a mutation at position 695 and/or a mutation at position 926 of SEQ ID NO: 6611, for example a mutation to alanine at position 497, position 661, position 695 and/or position 926 of SEQ ID NO: 6611. In embodiments, the Cas9 molecule has a mutation only at position 497, position 661, position 695, and position 926 of SEQ ID NO: 6611, relative to SEQ ID NO: 6611, e.g., where each mutation is to an uncharged amino acid, for example, alanine. Without being bound by theory, it is believed that such mutations reduce the cutting by the Cas9 molecule at off-target sites

It will be understood that the mutations described herein to the Cas9 molecule may be combined, and may be combined with any of the fusions or other modifications described herein, and the Cas9 molecule tested in the assays described herein.

Various types of Cas molecules can be used to practice the inventions disclosed herein. In some embodiments, Cas molecules of Type II Cas systems are used. In other embodiments, Cas molecules of other Cas systems are used. For example, Type I or Type III Cas molecules may be used.

Exemplary Cas molecules (and Cas systems) are described, e.g., in Haft et ai, PLoS COMPUTATIONAL BIOLOGY 2005, 1(6): e60 and Makarova et al, NATURE REVIEW MICROBIOLOGY 201 1 , 9:467-477, the contents of both references are incorporated herein by reference in their entirety.

In an embodiment, the Cas9 molecule comprises one or more of the following activities: a nickase activity; a double stranded cleavage activity (e.g., an endonuclease and/or exonuclease activity); a helicase activity; or the ability, together with a gRNA molecule, to localize to a target nucleic acid.

### Altered Cas9 Molecules

Naturally occurring Cas9 molecules possess a number of properties, including: nickase activity, nuclease activity (e.g., endonuclease and/or exonuclease activity); helicase activity; the ability to associate functionally with a gRNA molecule; and the ability to target (or localize to) a site on a nucleic acid (e.g., PAM recognition and specificity). In an embodiment, a Cas9 molecules can include all or a subset of these properties. In typical embodiments, Cas9 molecules have the ability to interact with a gRNA molecule and, in concert with the gRNA molecule, localize to a site in a nucleic acid. Other activities, e.g., PAM specificity, cleavage activity, or helicase activity can vary more widely in Cas9 molecules.

Cas9 molecules with desired properties can be made in a number of ways, e.g., by alteration of a parental, e.g., naturally occurring Cas9 molecules to provide an altered Cas9 molecule having a desired property. For example, one or more mutations or differences relative to a parental Cas9 molecule can be introduced. Such mutations and differences comprise: substitutions (e.g., conservative substitutions or substitutions of non-essential amino acids); insertions; or deletions. In an embodiment, a Cas9 molecule can comprises one or more mutations or differences, e.g., at least 1 , 2, 3, 4, 5, 10, 15, 20, 30, 40 or 50 mutations but less than 200, 100, or 80 mutations relative to a reference Cas9 molecule.

In an embodiment, a mutation or mutations do not have a substantial effect on a Cas9 activity, e.g. a Cas9 activity described herein. In an embodiment, a mutation or mutations have a substantial effect on a Cas9 activity, e.g. a Cas9 activity described herein. In an embodiment, exemplary activities comprise one or more of PAM specificity, cleavage activity, and helicase activity. A mutation(s) can be present, e.g., in: one or more RuvC-like domain, e.g., an N- terminal RuvC-like domain; an HNH-like domain; a region outside the RuvC-like domains and the HNH-like domain. In some embodiments, a mutation(s) is present in an N-terminal RuvC- like domain. In some embodiments, a mutation(s) is present in an HNH-like domain. In some embodiments, mutations are present in both an N-terminal RuvC-like domain and an HNH-like domain.

Whether or not a particular sequence, e.g., a substitution, may affect one or more activity, such as targeting activity, cleavage activity, etc, can be evaluated or predicted, e.g., by evaluating whether the mutation is conservative or by the method described in Section ΠI. In an embodiment, a "non-essential" amino acid residue, as used in the context of a Cas9 molecule, is a residue that can be altered from the wild-type sequence of a Cas9 molecule, e.g., a naturally occurring Cas9 molecule, e.g., an active Cas9 molecule, without abolishing or more preferably, without substantially altering a Cas9 activity (e.g., cleavage activity), whereas changing an "essential" amino acid residue results in a substantial loss of activity (e.g., cleavage activity).

### Cas9 Molecules with altered PAM recognition or no PAM recognition

Naturally occurring Cas9 molecules can recognize specific PAM sequences, for example the PAM recognition sequences described above for S. pyogenes, S. thermophilus, S. mutans, S. aureus and N. meningitidis.

In an embodiment, a Cas9 molecule has the same PAM specificities as a naturally occurring Cas9 molecule. In other embodiments, a Cas9 molecule has a PAM specificity not associated with a naturally occurring Cas9 molecule, or a PAM specificity not associated with the naturally occurring Cas9 molecule to which it has the closest sequence homology. For example, a naturally occurring Cas9 molecule can be altered, e.g., to alter PAM recognition, e.g., to alter the PAM sequence that the Cas9 molecule recognizes to decrease off target sites and/or improve specificity; or eliminate a PAM recognition requirement. In an embodiment, a Cas9 molecule can be altered, e.g., to increase length of PAM recognition sequence and/or improve Cas9 specificity to high level of identity to decrease off target sites and increase specificity. In an embodiment, the length of the PAM recognition sequence is at least 4, 5, 6, 7, 8, 9, 10 or 15 amino acids in length. Cas9 molecules that recognize different PAM sequences and/or have reduced off- target activity can be generated using directed evolution. Exemplary methods and systems that can be used for directed evolution of Cas9 molecules are described, e.g., in Esvelt el al , Nature 2011, 472(7344): 499-503. Candidate Cas9 molecules can be evaluated, e.g., by methods described herein.

### Non-Cleaving and Modified-Cleavage Cas9 Molecules

In an embodiment, a Cas9 molecule comprises a cleavage property that differs from naturally occurring Cas9 molecules, e.g., that differs from the naturally occurring Cas9 molecule having the closest homology. For example, a Cas9 molecule can differ from naturally occurring Cas9 molecules, e.g., a Cas9 molecule of S. pyogenes, as follows: its ability to modulate, e.g., decreased or increased, cleavage of a double stranded break (endonuclease and/or exonuclease activity), e.g., as compared to a naturally occurring Cas9 molecule (e.g., a Cas9 molecule of S. pyogenes); its ability to modulate, e.g., decreased or increased, cleavage of a single strand of a nucleic acid, e.g., a non-complimentary strand of a nucleic acid molecule or a complementary strand of a nucleic acid molecule (nickase activity), e.g., as compared to a naturally occurring Cas9 molecule (e.g., a Cas9 molecule of S. pyogenes); or the ability to cleave a nucleic acid molecule, e.g., a double stranded or single stranded nucleic acid molecule, can be eliminated.

### Modified Cleavage active Cas9 Molecules

In an embodiment, an active Cas9 molecule comprises one or more of the following activities: cleavage activity associated with an N-terminal RuvC-like domain; cleavage activity associated with an HNH-like domain; cleavage activity associated with an HNH domain and cleavage activity associated with an N-terminal RuvC-like domain.

In an embodiment, the Cas9 molecule is a Cas9 nickase, e.g., cleaves only a single strand of DNA. In an embodiment, the Cas9 nickase includes a mutation at position 10 and/or a mutation at position 840 of SEQ ID NO: 6611, e.g., comprises a D10A and/or H840A mutation to SEQ ID NO: 6611.

### Non-Cleaving inactive Cas9 Molecules

In an embodiment, the altered Cas9 molecule is an inactive Cas9 molecule which does not cleave a nucleic acid molecule (either double stranded or single stranded nucleic acid molecules) or cleaves a nucleic acid molecule with significantly less efficiency, e.g., less than 20, 10, 5, 1 or 0.1 % of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of S. pyogenes, S. thermophilus, S. aureus or N. meningitidis. In an embodiment, the reference Cas9 molecule is the naturally occurring Cas9 molecule having the closest sequence identity or homology. In an embodiment, the inactive Cas9 molecule lacks substantial cleavage activity associated with an N- terminal RuvC-like domain and cleavage activity associated with an HNH-like domain.

In an embodiment, the Cas9 molecule is dCas9. Tsai et al. (2014), Nat. Biotech. 32:569-577.

A catalytically inactive Cas9 molecule may be fused with a transcription repressor. An inactive Cas9 fusion protein complexes with a gRNA and localizes to a DNA sequence specified by gRNA's targeting domain, but, unlike an active Cas9, it will not cleave the target DNA. Fusion of an effector domain, such as a transcriptional repression domain, to an inactive Cas9 enables recruitment of the effector to any DNA site specified by the gRNA. Site specific targeting of a Cas9 fusion protein to a promoter region of a gene can block or affect polymerase binding to the promoter region, for example, a Cas9 fusion with a transcription factor (e.g., a transcription activator) and/or a transcriptional enhancer binding to the nucleic acid to increase or inhibit transcription activation. Alternatively, site specific targeting of a a Cas9- fusion to a transcription repressor to a promoter region of a gene can be used to decrease transcription activation.

Transcription repressors or transcription repressor domains that may be fused to an inactive Cas9 molecule can include ruppel associated box (KRAB or SKD), the Mad mSIN3 interaction domain (SID) or the ERF repressor domain (ERD).

In another embodiment, an inactive Cas9 molecule may be fused with a protein that modifies chromatin. For example, an inactive Cas9 molecule may be fused to heterochromatin protein 1 (HPl ), a histone lysine methyltransferase (e.g., SUV39H 1 , SUV39H2, G9A, ESET/SETDB 1, Pr-SET7/8, SUV4-20H 1,RIZ1), a histone lysine demethylates (e.g., LSD1/BHC1 10, SpLsdl/Sw, 1/Safl 10, Su(var)3-3, JMJD2A/JHDM3A, JMJD2B, JMJD2C/GASC1 , JMJD2D, Rph 1, JARID 1 A/RBP2, JARI DIB/PLU-I, JAR1D 1C/SMCX, JARID1 D/SMCY, Lid, Jhn2, Jmj2), a histone lysine deacetylases (e.g., HDAC1, HDAC2, HDAC3, HDAC8, Rpd3, Hos 1, Cir6, HDAC4, HDAC5, HDAC7, HDAC9, Hdal , Cir3, SIRT 1 , SIRT2, Sir2, Hst 1, Hst2, Hst3, Hst4, HDAC 1 1 ) and a DNA methylases (DNMT1,DNMT2a/DMNT3b, MET1). An inactive Cas9-chomatin modifying molecule fusion protein can be used to alter chromatin status to reduce expression a target gene.

The heterologous sequence (e.g., the transcription repressor domain) may be fused to the N- or C-terminus of the inactive Cas9 protein. In an alternative embodiment, the heterologous sequence (e.g., the transcription repressor domain) may be fused to an internal portion (i.e., a portion other than the N-terminus or C-terminus) of the inactive Cas9 protein.

The ability of a Cas9 molecule/gRNA molecule complex to bind to and cleave a target nucleic acid can be evaluated, e.g., by the methods described herein in Section ΠI. The activity of a Cas9 molecule, e.g., either an active Cas9 or a inactive Cas9, alone or in a complex with a gRNA molecule may also be evaluated by methods well-known in the art, including, gene expression assays and chromatin-based assays, e.g., chromatin immunoprecipitation (ChiP) and chromatin in vivo assay (CiA).

### Other Cas9 Molecule Fusions

In embodiments, the Cas9 molecule, e.g, a Cas9 of *S. pyogenes,* may additionally comprise one or more amino acid sequences that confer additional activity.

In some aspects, the Cas9 molecule may comprise one or more nuclear localization sequences (NLSs), such as at least 1, 2, 3, 4, 5, 6, 7, 8, 9, 10, or more NLSs. In some embodiments, the Cas9 molecule comprises at least 1, 2, 3, 4, 5, 6, 7, 8, 9, 10, or more NLSs at or near the amino-terminus, at least 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Typically, an NLS consists of one or more short sequences of positively charged lysines or arginines exposed on the protein surface, but other types of NLS are known. Non-limiting examples of NLSs include an NLS sequence comprising or derived from: the NLS of the SV40 virus large T-antigen, having the amino acid sequence PKKKRKV (SEQ ID NO: 6612); the NLS from nucleoplasmin (e.g. the nucleoplasmin bipartite NLS with the sequence KRPAATKKAGQAKKKK (SEQ ID NO: 6613); the c-myc NLS having the amino acid sequence PAAKRVKLD (SEQ ID NO: 6614) or RQRRNELKRSP (SEQ ID NO: 6615); the hRNPA1 M9 NLS having the sequence NQSSNFGPMKGGNFGGRSSGPYGGGGQYFAKPRNQGGY (SEQ ID NO: 6616); the sequence RMRIZFKNKGKDTAELRRRRVEVSVELRKAKKDEQILKRRNV (SEQ ID NO: 6617) of the IBB domain from importin-alpha; the sequences VSRKRPRP (SEQ ID NO: 6618) and PPKKARED (SEQ ID NO: 6619) of the myoma T protein; the sequence PQPKKKPL (SEQ ID NO: 6620) of human p53; the sequence SALIKKKKKMAP (SEQ ID NO: 6621) of mouse c-ab1 IV; the sequences DRLRR (SEQ ID NO: 6622) and PKQKKRK (SEQ ID NO: 6623) of the influenza virus NS1; the sequence RKLKKKIKKL (SEQ ID NO: 6624) of the Hepatitis virus delta antigen; the sequence REKKKFLKRR (SEQ ID NO: 6625) of the mouse Mx1 protein; the sequence KRKGDEVDGVDEVAKKKSKK (SEQ ID NO: 6626) of the human poly(ADP-ribose) polymerase; and the sequence RKCLQAGMNLEARKTKK (SEQ ID NO: 6627) of the steroid hormone receptors (human) glucocorticoid. Other suitable NLS sequences are known in the art (e.g., Sorokin, Biochemistry (Moscow) (2007) 72:13, 1439-1457; Lange J Biol Chem. (2007) 282:8, 5101-5).

In an embodiment, the Cas9 molecule, e.g., *S. pyogenes* Cas9 molecule, comprises a NLS sequence of SV40, e.g., disposed N terminal to the Cas9 molecule. In an embodiment, the Cas9 molecule, e.g., *S. pyogenes* Cas9 molecule, comprises a NLS sequence of SV40 disposed N-terminal to the Cas9 molecule and a NLS sequence of SV40 disposed C terminal to the Cas9 molecule. In an embodiment, the Cas9 molecule, e.g., *S. pyogenes* Cas9 molecule, comprises a NLS sequence of SV40 disposed N-terminal to the Cas9 molecule and a NLS sequence of nucleoplasmin disposed C-terminal to the Cas9 molecule. In any of the aforementioned embodiments, the molecule may additionally comprise a tag, e.g., a His tag, e.g., a His(6) tag or His(8) tag, e.g., at the N terminus or the C terminus.

In some aspects, the Cas9 molecule may comprise one or more amino acid sequences that allow the Cas9 molecule to be specifically recognized, for example a tag. In one embodiment, the tag is a Histidine tag, e.g., a histidine tag comprising at least 3, 4, 5, 6, 7, 8, 9, 10 or more histidine amino acids. In embodiments, the histidine tag is a His6 tag (six histidines). In other embodiments, the histidine tag is a His8 tag (eight histidines). In embodiments, the histidine tag may be separated from one or more other portions of the Cas9 molecule by a linker. In embodiments, the linker is GGS. An example of such a fusion is the Cas9 molecule iProt106520.

In some aspects, the Cas9 molecule may comprise one or more amino acid sequences that are recognized by a protease (e.g., comprise a protease cleavage site). In embodiments, the cleavage site is the tobacco etch virus (TEV) cleavage site, e.g., comprises the sequence ENLYFQG (SEQ ID NO: 7810). In some aspects the protease cleavage site, e.g., the TEV cleavage site is disposed between a tag, e.g., a His tag, e.g., a His6 or His8 tag, and the remainder of the Cas9 molecule. Without being bound by theory it is believed that such introduction will allow for the use of the tag for, e.g., purification of the Cas9 molecule, and then subsequent cleavage so the tag does not interfere with the Cas9 molecule function.

In embodiments, the Cas9 molecule (e.g., a Cas9 molecule as described herein) comprises an N-terminal NLS, and a C-terminal NLS (e.g., comprises, from N- to C-terminal NLS-Cas9-NLS), e.g., wherein each NLS is an SV40 NLS (PKKKRKV (SEQ ID NO: 6612)). In embodiments, the Cas9 molecule (e.g., a Cas9 molecule as described herein) comprises an N-terminal NLS, a C-terminal NLS, and a C-terminal His6 tag (e.g., comprises, from N- to C-terminal NLS-Cas9-NLS-His tag), e.g., wherein each NLS is an SV40 NLS (PKKKRKV (SEQ ID NO: 6612)). In embodiments, the Cas9 molecule (e.g., a Cas9 molecule as described herein) comprises an N-terminal His tag (e.g., His6 tag), an N-terminal NLS, and a C-terminal NLS (e.g., comprises, from N- to C-terminal His tag-NLS-Cas9-NLS), e.g., wherein each NLS is an SV40 NLS (PKKKRKV (SEQ ID NO: 6612)). In embodiments, the Cas9 molecule (e.g., a Cas9 molecule as described herein) comprises an N-terminal NLS and a C-terminal His tag (e.g., His6 tag) (e.g., comprises from N- to C- terminal His tag-Cas9-NLS), e.g., wherein the NLS is an SV40 NLS (PKKKRKV (SEQ ID NO: 6612)). In embodiments, the Cas9 molecule (e.g., a Cas9 molecule as described herein) comprises an N-terminal NLS and a C-terminal His tag (e.g., His6 tag) (e.g., comprises from N- to C- terminal NLS-Cas9-His tag), e.g., wherein the NLS is an SV40 NLS (PKKKRKV (SEQ ID NO: 6612)). In embodiments, the Cas9 molecule (e.g., a Cas9 molecule as described herein) comprises an N-terminal His tag (e.g., His8 tag), an N-terminal cleavage domain (e.g., a tobacco etch virus (TEV) cleavage domain (e.g., comprises the sequence ENLYFQG (SEQ ID NO: 7810))), an N-terminal NLS (e.g., an SV40 NLS; SEQ ID NO: 6612), and a C-terminal NLS (e.g., an SV40 NLS; SEQ ID NO: 6612) (e.g., comprises from N- to C-terminal His tag-TEV-NLS-Cas9-NLS). In any of the aforementioned embodiments the Cas9 has the sequence of SEQ ID NO: 6611. Alternatively, in any of the aforementioned embodiments, the Cas9 has a sequence of a Cas9 variant of SEQ ID NO: 6611, e.g., as described herein. In any of the aforementioned embodiments, the Cas9 molecule comprises a linker between the His tag and another portion of the molecule, e.g., a GGS linker. Amino acid sequences of exemplary Cas9 molecules described above are provided below. "iProt" identifiers match those in Figure 60.
iProt105026 (also referred to as iProt106154, iProt106331, iProt106545, and PID426303, depending on the preparation of the protein) (SEQ ID NO: 7821): 
MAPKKKRKVD KKYSIGLDIG TNSVGWAVIT DEYKVPSKKF KVLGNTDRHS IKKNLIGALL
FDSGETAEAT RLKRTARRRY TRRKNRICYL QEIFSNEMAK VDDSFFHRLE ESFLVEEDKK
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE
KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTNFDKNL PNEKVLPKHS LLYEYFTVYN ELTKVKYVTE
GMRKPAFLSG EQKKAIVDLL FKTNRKVTVK QLKEDYFKKI ECFDSVEISG VEDRFNASLG
TYHDLLKIIK DKDFLDNEEN EDILEDIVLT LTLFEDREMI EERLKTYAHL FDDKVMKQLK
RRRYTGWGRL SRKLINGIRD KQSGKTILDF LKSDGFANRN FMQLIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEKLY LYYLQNGRDM YVDQELDINR
LSDYDVDHIV PQSFLKDDSI DNKVLTRSDK NRGKSDNVPS EEVVKKMKNY
WRQLLNAKLI TQRKFDNLTK AERGGLSELD KAGFIKRQLV ETRQITKHVA QILDSRMNTK
YDENDKLIRE VKVITLKSKL VSDFRKDFQF YKVREINNYH HAHDAYLNAV VGTALIKKYP
KLESEFVYGD YKVYDVRKMI AKSEQEIGKA TAKYFFYSNI MNFFKTEITL ANGEIRKRPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
REQAENIIHL FTLTNLGAPA AFKYFDTTID RKRYTSTKEV LDATLIHQSI TGLYETRIDL
SQLGGDSRAD PKKKRKVHHH HHH
iProt106518 (SEQ ID NO: 7822): 
MAPKKKRKVD KKYSIGLDIG TNSVGWAVIT DEYKVPSKKF KVLGNTDRHS IKKNLIGALL
FDSGETAEAT RLKRTARRRY TRRKNRILYL QEIFSNEMAK VDDSFFHRLE ESFLVEEDKK
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE
KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTNFDKNL PNEKVLPKHS LLYEYFTVYN ELTKVKYVTE
GMRKPAFLSG EQKKAIVDLL FKTNRKVTVK QLKEDYFKKI EEFDSVEISG VEDRFNASLG
TYHDLLKIIK DKDFLDNEEN EDILEDIVLT LTLFEDREMI EERLKTYAHL FDDKVMKQLK
RRRYTGWGRL SRKLINGIRD KQSGKTILDF LKSDGFANRN FMQLIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEKLY LYYLQNGRDM YVDQELDINR
LSDYDVDHIV PQSFLKDDSI DNKVLTRSDK NRGKSDNVPS EEVVKKMKNY
WRQLLNAKLI TQRKFDNLTK AERGGLSELD KAGFIKRQLV ETRQITKHVA QILDSRMNTK
YDENDKLIRE VKVITLKSKL VSDFRKDFQF YKVREINNYH HAHDAYLNAV VGTALIKKYP
KLESEFVYGD YKVYDVRKMI AKSEQEIGKA TAKYFFYSNI MNFFKTEITL ANGEIRKRPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
 
REQAENIIHL FTLTNLGAPA AFKYFDTTID RKRYTSTKEV LDATLIHQSI TGLYETRIDL
SQLGGDSRAD PKKKRKVHHH HHH
iProt106519 (SEQ ID NO: 7823): 
MGSSHHHHHH HHENLYFQGS MDKKYSIGLD IGTNSVGWAV ITDEYKVPSK
KFKVLGNTDR HSIKKNLIGA LLFDSGETAE ATRLKRTARR RYTRRKNRIC YLQEIFSNEM
AKVDDSFFHR LEESFLVEED KKHERHPIFG NIVDEVAYHE KYPTIYHLRK KLVDSTDKAD
LRLIYLALAH MIKFRGHFLI EGDLNPDNSD VDKLFIQLVQ TYNQLFEENP INASGVDAKA
ILSARLSKSR RLENLIAQLP GEKKNGLFGN LIALSLGLTP NFKSNFDLAE DAKLQLSKDT
YDDDLDNLLA QIGDQYADLF LAAKNLSDAI LLSDILRVNT EITKAPLSAS MIKRYDEHHQ
DLTLLKALVR QQLPEKYKEI FFDQSKNGYA GYIDGGASQE EFYKFIKPIL EKMDGTEELL
VKLNREDLLR KQRTFDNGSI PHQIHLGELH AILRRQEDFY PFLKDNREKI EKILTFRIPY
YVGPLARGNS RFAWMTRKSE ETITPWNFEE VVDKGASAQS FIERMTNFDK NLPNEKVLPK
HSLLYEYFTV YNELTKVKYV TEGMRKPAFL SGEQKKAIVD LLFKTNRKVT
VKQLKEDYFK KIECFDSVEI SGVEDRFNAS LGTYHDLLKI IKDKDFLDNE ENEDILEDIV
LTLTLFEDRE MIEERLKTYA HLFDDKVMKQ LKRRRYTGWG RLSRKLINGI RDKQSGKTIL
DFLKSDGFAN RNFMQLIHDD SLTFKEDIQK AQVSGQGDSL HEHIANLAGS PAIKKGILQT
VKVVDELVKV MGRHKPENIV IEMARENQTT QKGQKNSRER MKRIEEGIKE LGSQILKEHP
VENTQLQNEK LYLYYLQNGR DMYVDQELDI NRLSDYDVDH IVPQSFLKDD SIDNKVLTRS
DKNRGKSDNV PSEEVVKKMK NYWRQLLNAK LITQRKFDNL TKAERGGLSE
LDKAGFIKRQ LVETRQITKH VAQILDSRMN TKYDENDKLI REVKVITLKS KLVSDFRKDF
QFYKVREINN YHHAHDAYLN AVVGTALIKK YPKLESEFVY GDYKVYDVRK
MIAKSEQEIG KATAKYFFYS NIMNFFKTEI TLANGEIRKR PLIETNGETG EIVWDKGRDF
ATVRKVLSMP QVNIVKKTEV QTGGFSKESI LPKRNSDKLI ARKKDWDPKK YGGFDSPTVA
YSVLVVAKVE KGKSKKLKSV KELLGITIME RSSFEKNPID FLEAKGYKEV KKDLIIKLPK
YSLFELENGR KRMLASAGEL QKGNELALPS KYVNFLYLAS HYEKLKGSPE DNEQKQLFVE
QHKHYLDEII EQISEFSKRV ILADANLDKV LSAYNKHRDK PIREQAENII HLFTLTNLGA
PAAFKYFDTT IDRKRYTSTK EVLDATLIHQ SITGLYETRI DLSQLGGDGG GSPKKKRKV
iProt106520 (SEQ ID NO: 7824): 
MAHHHHHHGG SPKKKRKVDK KYSIGLDIGT NSVGWAVITD EYKVPSKKFK
VLGNTDRHSI KKNLIGALLF DSGETAEATR LKRTARRRYT RRKNRICYLQ EIFSNEMAKV
DDSFFHRLEE SFLVEEDKKH ERHPIFGNIV DEVAYHEKYP TIYHLRKKLV DSTDKADLRL
IYLALAHMIK FRGHFLIEGD LNPDNSDVDK LFIQLVQTYN QLFEENPINA SGVDAKAILS
ARLSKSRRLE NLIAQLPGEK KNGLFGNLIA LSLGLTPNFK SNFDLAEDAK LQLSKDTYDD
DLDNLLAQIG DQYADLFLAA KNLSDAILLS DILRVNTEIT KAPLSASMIK RYDEHHQDLT
LLKALVRQQL PEKYKEIFFD QSKNGYAGYI DGGASQEEFY KFIKPILEKM DGTEELLVKL
NREDLLRKQR TFDNGSIPHQ IHLGELHAIL RRQEDFYPFL KDNREKIEKI LTFRIPYYVG
PLARGNSRFA WMTRKSEETI TPWNFEEVVD KGASAQSFIE RMTNFDKNLP NEKVLPKHSL
LYEYFTVYNE LTKVKYVTEG MRKPAFLSGE QKKAIVDLLF KTNRKVTVKQ LKEDYFKKIE
CFDSVEISGV EDRFNASLGT YHDLLKIIKD KDFLDNEENE DILEDIVLTL TLFEDREMIE
ERLKTYAHLF DDKVMKQLKR RRYTGWGRLS RKLINGIRDK QSGKTILDFL KSDGFANRNF
MQLIHDDSLT FKEDIQKAQV SGQGDSLHEH IANLAGSPAI KKGILQTVKV VDELVKVMGR
HKPENIVIEM ARENQTTQKG QKNSRERMKR IEEGIKELGS QILKEHPVEN TQLQNEKLYL
YYLQNGRDMY VDQELDINRL SDYDVDHIVP QSFLKDDSID NKVLTRSDKN RGKSDNVPSE
EVVKKMKNYW RQLLNAKLIT QRKFDNLTKA ERGGLSELDK AGFIKRQLVE
TRQITKHVAQ ILDSRMNTKY DENDKLIREV KVITLKSKLV SDFRKDFQFY KVREINNYHH
AHDAYLNAVV GTALIKKYPK LESEFVYGDY KVYDVRKMIA KSEQEIGKAT
AKYFFYSNIM NFFKTEITLA NGEIRKRPLI ETNGETGEIV WDKGRDFATV RKVLSMPQVN
IVKKTEVQTG GFSKESILPK RNSDKLIARK KDWDPKKYGG FDSPTVAYSV LVVAKVEKGK
SKKLKSVKEL LGITIMERSS FEKNPIDFLE AKGYKEVKKD LIIKLPKYSL FELENGRKRM
LASAGELQKG NELALPSKYV NFLYLASHYE KLKGSPEDNE QKQLFVEQHK HYLDEIIEQI
SEFSKRVILA DANLDKVLSA YNKHRDKPIR EQAENIIHLF TLTNLGAPAA FKYFDTTIDR
KRYTSTKEVL DATLIHQSIT GLYETRIDLS QLGGDSRADP KKKRKV
iProt106521 (SEQ ID NO: 7825): 
MAPKKKRKVD KKYSIGLDIG TNSVGWAVIT DEYKVPSKKF KVLGNTDRHS IKKNLIGALL
FDSGETAEAT RLKRTARRRY TRRKNRICYL QEIFSNEMAK VDDSFFHRLE ESFLVEEDKK
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE
KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTNFDKNL PNEKVLPKHS LLYEYFTVYN ELTKVKYVTE
GMRKPAFLSG EQKKAIVDLL FKTNRKVTVK QLKEDYFKKI ECFDSVEISG VEDRFNASLG
TYHDLLKIIK DKDFLDNEEN EDILEDIVLT LTLFEDREMI EERLKTYAHL FDDKVMKQLK
RRRYTGWGRL SRKLINGIRD KQSGKTILDF LKSDGFANRN FMQLIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEKLY LYYLQNGRDM YVDQELDINR
LSDYDVDHIV PQSFLKDDSI DNKVLTRSDK NRGKSDNVPS EEVVKKMKNY
WRQLLNAKLI TQRKFDNLTK AERGGLSELD KAGFIKRQLV ETRQITKHVA QILDSRMNTK
YDENDKLIRE VKVITLKSKL VSDFRKDFQF YKVREINNYH HAHDAYLNAV VGTALIKKYP
KLESEFVYGD YKVYDVRKMI AKSEQEIGKA TAKYFFYSNI MNFFKTEITL ANGEIRKRPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
REQAENIIHL FTLTNLGAPA AFKYFDTTID RKRYTSTKEV LDATLIHQSI TGLYETRIDL
SQLGGDSRAD HHHHHH
iProt106522 (SEQ ID NO: 7826): 
MAHHHHHHGG SDKKYSIGLD IGTNSVGWAV ITDEYKVPSK KFKVLGNTDR HSIKKNLIGA
LLFDSGETAE ATRLKRTARR RYTRRKNRIC YLQEIFSNEM AKVDDSFFHR LEESFLVEED
KKHERHPIFG NIVDEVAYHE KYPTIYHLRK KLVDSTDKAD LRLIYLALAH MIKFRGHFLI
EGDLNPDNSD VDKLFIQLVQ TYNQLFEENP INASGVDAKA ILSARLSKSR RLENLIAQLP
GEKKNGLFGN LIALSLGLTP NFKSNFDLAE DAKLQLSKDT YDDDLDNLLA QIGDQYADLF
LAAKNLSDAI LLSDILRVNT EITKAPLSAS MIKRYDEHHQ DLTLLKALVR QQLPEKYKEI
FFDQSKNGYA GYIDGGASQE EFYKFIKPIL EKMDGTEELL VKLNREDLLR KQRTFDNGSI
PHQIHLGELH AILRRQEDFY PFLKDNREKI EKILTFRIPY YVGPLARGNS RFAWMTRKSE
ETITPWNFEE VVDKGASAQS FIERMTNFDK NLPNEKVLPK HSLLYEYFTV YNELTKVKYV
TEGMRKPAFL SGEQKKAIVD LLFKTNRKVT VKQLKEDYFK KIECFDSVEI SGVEDRFNAS
LGTYHDLLKI IKDKDFLDNE ENEDILEDIV LTLTLFEDRE MIEERLKTYA HLFDDKVMKQ
LKRRRYTGWG RLSRKLINGI RDKQSGKTIL DFLKSDGFAN RNFMQLIHDD SLTFKEDIQK
AQVSGQGDSL HEHIANLAGS PAIKKGILQT VKVVDELVKV MGRHKPENIV IEMARENQTT
QKGQKNSRER MKRIEEGIKE LGSQILKEHP VENTQLQNEK LYLYYLQNGR DMYVDQELDI
NRLSDYDVDH IVPQSFLKDD SIDNKVLTRS DKNRGKSDNV PSEEVVKKMK
NYWRQLLNAK LITQRKFDNL TKAERGGLSE LDKAGFIKRQ LVETRQITKH VAQILDSRMN
TKYDENDKLI REVKVITLKS KLVSDFRKDF QFYKVREINN YHHAHDAYLN AVVGTALIKK
YPKLESEFVY GDYKVYDVRK MIAKSEQEIG KATAKYFFYS NIMNFFKTEI TLANGEIRKR
PLIETNGETG EIVWDKGRDF ATVRKVLSMP QVNIVKKTEV QTGGFSKESI LPKRNSDKLI
ARKKDWDPKK YGGFDSPTVA YSVLVVAKVE KGKSKKLKSV KELLGITIME RSSFEKNPID
FLEAKGYKEV KKDLIIKLPK YSLFELENGR KRMLASAGEL QKGNELALPS KYVNFLYLAS
HYEKLKGSPE DNEQKQLFVE QHKHYLDEII EQISEFSKRV ILADANLDKV LSAYNKHRDK
PIREQAENII HLFTLTNLGA PAAFKYFDTT IDRKRYTSTK EVLDATLIHQ SITGLYETRI
DLSQLGGDSR ADPKKKRKV
iProt106658 (SEQ ID NO: 7827): 
MGSSHHHHHH HHENLYFQGS MDKKYSIGLD IGTNSVGWAV ITDEYKVPSK
KFKVLGNTDR HSIKKNLIGA LLFDSGETAE ATRLKRTARR RYTRRKNRIC YLQEIFSNEM
AKVDDSFFHR LEESFLVEED KKHERHPIFG NIVDEVAYHE KYPTIYHLRK KLVDSTDKAD
LRLIYLALAH MIKFRGHFLI EGDLNPDNSD VDKLFIQLVQ TYNQLFEENP INASGVDAKA
ILSARLSKSR RLENLIAQLP GEKKNGLFGN LIALSLGLTP NFKSNFDLAE DAKLQLSKDT
YDDDLDNLLA QIGDQYADLF LAAKNLSDAI LLSDILRVNT EITKAPLSAS MIKRYDEHHQ
DLTLLKALVR QQLPEKYKEI FFDQSKNGYA GYIDGGASQE EFYKFIKPIL EKMDGTEELL
VKLNREDLLR KQRTFDNGSI PHQIHLGELH AILRRQEDFY PFLKDNREKI EKILTFRIPY
YVGPLARGNS RFAWMTRKSE ETITPWNFEE VVDKGASAQS FIERMTNFDK NLPNEKVLPK
HSLLYEYFTV YNELTKVKYV TEGMRKPAFL SGEQKKAIVD LLFKTNRKVT
VKQLKEDYFK KIECFDSVEI SGVEDRFNAS LGTYHDLLKI IKDKDFLDNE ENEDILEDIV
LTLTLFEDRE MIEERLKTYA HLFDDKVMKQ LKRRRYTGWG RLSRKLINGI RDKQSGKTIL
DFLKSDGFAN RNFMQLIHDD SLTFKEDIQK AQVSGQGDSL HEHIANLAGS PAIKKGILQT
VKVVDELVKV MGRHKPENIV IEMARENQTT QKGQKNSRER MKRIEEGIKE LGSQILKEHP
VENTQLQNEK LYLYYLQNGR DMYVDQELDI NRLSDYDVDH IVPQSFLKDD SIDNKVLTRS
DKNRGKSDNV PSEEVVKKMK NYWRQLLNAK LITQRKFDNL TKAERGGLSE
LDKAGFIKRQ LVETRQITKH VAQILDSRMN TKYDENDKLI REVKVITLKS KLVSDFRKDF
QFYKVREINN YHHAHDAYLN AVVGTALIKK YPKLESEFVY GDYKVYDVRK
MIAKSEQEIG KATAKYFFYS NIMNFFKTEI TLANGEIRKR PLIETNGETG EIVWDKGRDF
ATVRKVLSMP QVNIVKKTEV QTGGFSKESI LPKRNSDKLI ARKKDWDPKK YGGFDSPTVA
YSVLVVAKVE KGKSKKLKSV KELLGITIME RSSFEKNPID FLEAKGYKEV KKDLIIKLPK
YSLFELENGR KRMLASAGEL QKGNELALPS KYVNFLYLAS HYEKLKGSPE DNEQKQLFVE
QHKHYLDEII EQISEFSKRV ILADANLDKV LSAYNKHRDK PIREQAENII HLFTLTNLGA
PAAFKYFDTT IDRKRYTSTK EVLDATLIHQ SITGLYETRI DLSQLGGDGG GSPKKKRKV
iProt106745 (SEQ ID NO: 7828): 
MAPKKKRKVD KKYSIGLDIG TNSVGWAVIT DEYKVPSKKF KVLGNTDRHS IKKNLIGALL
FDSGETAEAT RLKRTARRRY TRRKNRICYL QEIFSNEMAK VDDSFFHRLE ESFLVEEDKK
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE
KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTNFDKNL PNEKVLPKHS LLYEYFTVYN ELTKVKYVTE
GMRKPAFLSG EQKKAIVDLL FKTNRKVTVK QLKEDYFKKI ECFDSVEISG VEDRFNASLG
TYHDLLKIIK DKDFLDNEEN EDILEDIVLT LTLFEDREMI EERLKTYAHL FDDKVMKQLK
RRRYTGWGRL SRKLINGIRD KQSGKTILDF LKSDGFANRN FMQLIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEKLY LYYLQNGRDM YVDQELDINR
LSDYDVDHIV PQSFLKDDSI DNAVLTRSDK NRGKSDNVPS EEVVKKMKNY
WRQLLNAKLI TQRKFDNLTK AERGGLSELD KAGFIKRQLV ETRQITKHVA QILDSRMNTK
YDENDKLIRE VKVITLKSKL VSDFRKDFQF YKVREINNYH HAHDAYLNAV VGTALIKKYP
KLESEFVYGD YKVYDVRKMI AKSEQEIGKA TAKYFFYSNI MNFFKTEITL ANGEIRKRPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
REQAENIIHL FTLTNLGAPA AFKYFDTTID RKRYTSTKEV LDATLIHQSI TGLYETRIDL
SQLGGDSRAD PKKKRKVHHH HHH
iProt106746 (SEQ ID NO: 7829): 
MAPKKKRKVD KKYSIGLDIG TNSVGWAVIT DEYKVPSKKF KVLGNTDRHS IKKNLIGALL
FDSGETAEAT RLKRTARRRY TRRKNRICYL QEIFSNEMAK VDDSFFHRLE ESFLVEEDKK
 
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE
KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTNFDKNL PNEKVLPKHS LLYEYFTVYN ELTKVKYVTE
GMRKPAFLSG EQKKAIVDLL FKTNRKVTVK QLKEDYFKKI ECFDSVEISG VEDRFNASLG
TYHDLLKIIK DKDFLDNEEN EDILEDIVLT LTLFEDREMI EERLKTYAHL FDDKVMKQLK
RRRYTGWGRL SRKLINGIRD KQSGKTILDF LKSDGFANRN FMQLIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEALY LYYLQNGRDM YVDQELDINR
LSDYDVDHIV PQSFLKDDSI DNKVLTRSDK NRGKSDNVPS EEVVKKMKNY
WRQLLNAKLI TQRKFDNLTK AERGGLSELD KAGFIKRQLV ETRQITKHVA QILDSRMNTK
YDENDKLIRE VKVITLKSKL VSDFRKDFQF YKVREINNYH HAHDAYLNAV VGTALIKKYP
ALESEFVYGD YKVYDVRKMI AKSEQEIGKA TAKYFFYSNI MNFFKTEITL ANGEIRKAPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
REQAENIIHL FTLTNLGAPA AFKYFDTTID RKRYTSTKEV LDATLIHQSI TGLYETRIDL
SQLGGDSRAD PKKKRKVHHH HHH
iProt106747 (SEQ ID NO: 7830): 
MAPKKKRKVD KKYSIGLDIG TNSVGWAVIT DEYKVPSKKF KVLGNTDRHS IKKNLIGALL
FDSGETAEAT RLKRTARRRY TRRKNRICYL QEIFSNEMAK VDDSFFHRLE ESFLVEEDKK
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE
KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTNFDKNL PNEKVLPKHS LLYEYFTVYN ELTKVKYVTE
GMRKPAFLSG EQKKAIVDLL FKTNRKVTVK QLKEDYFKKI ECFDSVEISG VEDRFNASLG
TYHDLLKIIK DKDFLDNEEN EDILEDIVLT LTLFEDREMI EERLKTYAHL FDDKVMKQLK
RRRYTGWGRL SRKLINGIRD KQSGKTILDF LKSDGFANRN FMQLIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEKLY LYYLQNGRDM YVDQELDINR
LSDYDVDHIV PQSFLADDSI DNKVLTRSDK NRGKSDNVPS EEVVKKMKNY
WRQLLNAKLI TQRKFDNLTK AERGGLSELD KAGFIKRQLV ETRQITKHVA QILDSRMNTK
YDENDKLIRE VKVITLKSKL VSDFRKDFQF YKVREINNYH HAHDAYLNAV VGTALIKKYP
ALESEFVYGD YKVYDVRKMI AKSEQEIGKA TAKYFFYSNI MNFFKTEITL ANGEIRKAPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
REQAENIIHL FTLTNLGAPA AFKYFDTTID RKRYTSTKEV LDATLIHQSI TGLYETRIDL
SQLGGDSRAD PKKKRKVHHH HHH
iProt106884 (SEQ ID NO: 7831):

MAPKKKRKVD KKYSIGLDIG TNSVGWAVIT DEYKVPSKKF KVLGNTDRHS IKKNLIGALL
FDSGETAEAT RLKRTARRRY TRRKNRICYL QEIFSNEMAK VDDSFFHRLE ESFLVEEDKK
HERHPIFGNI VDEVAYHEKY PTIYHLRKKL VDSTDKADLR LIYLALAHMI KFRGHFLIEG
DLNPDNSDVD KLFIQLVQTY NQLFEENPIN ASGVDAKAIL SARLSKSRRL ENLIAQLPGE

KKNGLFGNLI ALSLGLTPNF KSNFDLAEDA KLQLSKDTYD DDLDNLLAQI GDQYADLFLA
AKNLSDAILL SDILRVNTEI TKAPLSASMI KRYDEHHQDL TLLKALVRQQ LPEKYKEIFF
DQSKNGYAGY IDGGASQEEF YKFIKPILEK MDGTEELLVK LNREDLLRKQ RTFDNGSIPH
QIHLGELHAI LRRQEDFYPF LKDNREKIEK ILTFRIPYYV GPLARGNSRF AWMTRKSEET
ITPWNFEEVV DKGASAQSFI ERMTAFDKNL PNEKVLPKHS LLYEYFTVYN ELTKVKYVTE
GMRKPAFLSG EQKKAIVDLL FKTNRKVTVK QLKEDYFKKI ECFDSVEISG VEDRFNASLG
TYHDLLKIIK DKDFLDNEEN EDILEDIVLT LTLFEDREMI EERLKTYAHL FDDKVMKQLK
RRRYTGWGAL SRKLINGIRD KQSGKTILDF LKSDGFANRN FMALIHDDSL TFKEDIQKAQ
VSGQGDSLHE HIANLAGSPA IKKGILQTVK VVDELVKVMG RHKPENIVIE MARENQTTQK
GQKNSRERMK RIEEGIKELG SQILKEHPVE NTQLQNEKLY LYYLQNGRDM YVDQELDINR
LSDYDVDHIV PQSFLKDDSI DNKVLTRSDK NRGKSDNVPS EEVVKKMKNY
WRQLLNAKLI TQRKFDNLTK AERGGLSELD KAGFIKRQLV ETRAITKHVA QILDSRMNTK
YDENDKLIRE VKVITLKSKL VSDFRKDFQF YKVREINNYH HAHDAYLNAV VGTALIKKYP
KLESEFVYGD YKVYDVRKMI AKSEQEIGKA TAKYFFYSNI MNFFKTEITL ANGEIRKRPL
IETNGETGEI VWDKGRDFAT VRKVLSMPQV NIVKKTEVQT GGFSKESILP KRNSDKLIAR
KKDWDPKKYG GFDSPTVAYS VLVVAKVEKG KSKKLKSVKE LLGITIMERS SFEKNPIDFL
EAKGYKEVKK DLIIKLPKYS LFELENGRKR MLASAGELQK GNELALPSKY VNFLYLASHY
EKLKGSPEDN EQKQLFVEQH KHYLDEIIEQ ISEFSKRVIL ADANLDKVLS AYNKHRDKPI
REQAENIIHL FTLTNLGAPA AFKYFDTTID RKRYTSTKEV LDATLIHQSI TGLYETRIDL
SQLGGDSRAD PKKKRKVHHH HHH

### Nucleic Acids Encoding Cas9 Molecules

Nucleic acids encoding the Cas9 molecules, e.g., an active Cas9 molecule or an inactive Cas9 molecule are provided herein.

Exemplary nucleic acids encoding Cas9 molecules are described in Cong et al, SCIENCE 2013, 399(6121):819-823; Wang et al, CELL 2013, 153(4):910-918; Mali et al. , SCIENCE 2013, 399(6121):823-826; Jinek et al, SCIENCE 2012, 337(6096):816-821.

In an embodiment, a nucleic acid encoding a Cas9 molecule can be a synthetic nucleic acid sequence. For example, the synthetic nucleic acid molecule can be chemically modified, e.g., as described in Section XIII. In an embodiment, the Cas9 mRNA has one or more of, e.g., all of the following properties: it is capped, polyadenylated, substituted with 5-methylcytidine and/or pseudouridine.

In addition or alternatively, the synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein.

Provided below is an exemplary codon optimized nucleic acid sequence encoding a Cas9 molecule of S. pyogenes.

atggataaaa agtacagcat cgggctggac atcggtacaa actcagtggg gtgggccgtg 60

attacggacg agtacaaggt accctccaaa aaatttaaag tgctgggtaa cacggacaga 120

cactctataa agaaaaatct tattggagcc ttgctgttcg actcaggcga gacagccgaa 180

gccacaaggt tgaagcggac cgccaggagg cggtatacca ggagaaagaa ccgcatatgc 240
tacctgcaag aaatcttcag taacgagatg gcaaaggttg acgatagctt tttccatcgc 300
ctggaagaat cctttcttgt tgaggaagac aagaagcacg aacggcaccc catctttggc 360
aatattgtcg acgaagtggc atatcacgaa aagtacccga ctatctacca cctcaggaag 420
aagctggtgg actctaccga taaggcggac ctcagactta tttatttggc actcgcccac 480
atgattaaat ttagaggaca tttcttgatc gagggcgacc tgaacccgga caacagtgac 540
gtcgataagc tgttcatcca acttgtgcag acctacaatc aactgttcga agaaaaccct 600
ataaatgctt caggagtcga cgctaaagca atcctgtccg cgcgcctctc aaaatctaga 660
agacttgaga atctgattgc tcagttgccc ggggaaaaga aaaatggatt gtttggcaac 720
ctgatcgccc tcagtctcgg actgacccca aatttcaaaa gtaacttcga cctggccgaa 780
gacgctaagc tccagctgtc caaggacaca tacgatgacg acctcgacaa tctgctggcc 840
cagattgggg atcagtacgc cgatctcttt ttggcagcaa agaacctgtc cgacgccatc 900
ctgttgagcg atatcttgag agtgaacacc gaaattacta aagcacccct tagcgcatct 960
atgatcaagc ggtacgacga gcatcatcag gatctgaccc tgctgaaggc tcttgtgagg 1020
caacagctcc ccgaaaaata caaggaaatc ttctttgacc agagcaaaaa cggctacgct 1080
ggctatatag atggtggggc cagtcaggag gaattctata aattcatcaa gcccattctc 1140
gagaaaatgg acggcacaga ggagttgctg gtcaaactta acagggagga cctgctgcgg 1200
aagcagcgga cctttgacaa cgggtctatc ccccaccaga ttcatctggg cgaactgcac 1260
gcaatcctga ggaggcagga ggatttttat ccttttctta aagataaccg cgagaaaata 1320
gaaaagattc ttacattcag gatcccgtac tacgtgggac ctctcgcccg gggcaattca 1380
cggtttgcct ggatgacaag gaagtcagag gagactatta caccttggaa cttcgaagaa 1440
gtggtggaca agggtgcatc tgcccagtct ttcatcgagc ggatgacaaa ttttgacaag 1500
aacctcccta atgagaaggt gctgcccaaa cattctctgc tctacgagta ctttaccgtc 1560
tacaatgaac tgactaaagt caagtacgtc accgagggaa tgaggaagcc ggcattcctt 1620
agtggagaac agaagaaggc gattgtagac ctgttgttca agaccaacag gaaggtgact 1680
gtgaagcaac ttaaagaaga ctactttaag aagatcgaat gttttgacag tgtggaaatt 1740

tcaggggttg aagaccgctt caatgcgtca ttggggactt accatgatct tctcaagatc 1800

ataaaggaca aagacttcct ggacaacgaa gaaaatgagg atattctcga agacatcgtc 1860

ctcaccctga ccctgttcga agacagggaa atgatagaag agcgcttgaa aacctatgcc 1920

cacctcttcg acgataaagt tatgaagcag ctgaagcgca ggagatacac aggatgggga 1980

agattgtcaa ggaagctgat caatggaatt agggataaac agagtggcaa gaccatactg 2040

gatttcctca aatctgatgg cttcgccaat aggaacttca tgcaactgat tcacgatgac 2100

tctcttacct tcaaggagga cattcaaaag gctcaggtga gcgggcaggg agactccctt 2160

catgaacaca tcgcgaattt ggcaggttcc cccgctatta aaaagggcat ccttcaaact 2220

gtcaaggtgg tggatgaatt ggtcaaggta atgggcagac ataagccaga aaatattgtg 2280

atcgagatgg cccgcgaaaa ccagaccaca cagaagggcc agaaaaatag tagagagcgg 2340

atgaagagga tcgaggaggg catcaaagag ctgggatctc agattctcaa agaacacccc 2400

gtagaaaaca cacagctgca gaacgaaaaa ttgtacttgt actatctgca gaacggcaga 2460

gacatgtacg tcgaccaaga acttgatatt aatagactgt ccgactatga cgtagaccat 2520

atcgtgcccc agtccttcct gaaggacgac tccattgata acaaagtctt gacaagaagc 2580

gacaagaaca ggggtaaaag tgataatgtg cctagcgagg aggtggtgaa aaaaatgaag 2640

aactactggc gacagctgct taatgcaaag ctcattacac aacggaagtt cgataatctg 2700

acgaaagcag agagaggtgg cttgtctgag ttggacaagg cagggtttat taagcggcag 2760

ctggtggaaa ctaggcagat cacaaagcac gtggcgcaga ttttggacag ccggatgaac 2820

acaaaatacg acgaaaatga taaactgata cgagaggtca aagttatcac gctgaaaagc 2880

aagctggtgt ccgattttcg gaaagacttc cagttctaca aagttcgcga gattaataac 2940

taccatcatg ctcacgatgc gtacctgaac gctgttgtcg ggaccgcctt gataaagaag 3000

tacccaaagc tggaatccga gttcgtatac ggggattaca aagtgtacga tgtgaggaaa 3060

atgatagcca agtccgagca ggagattgga aaggccacag ctaagtactt cttttattct 3120

aacatcatga atttttttaa gacggaaatt accctggcca acggagagat cagaaagcgg 3180

ccccttatag agacaaatgg tgaaacaggt gaaatcgtct gggataaggg cagggatttc 3240

gctactgtga ggaaggtgct gagtatgcca caggtaaata tcgtgaaaaa aaccgaagta 3300

cagaccggag gattttccaa ggaaagcatt ttgcctaaaa gaaactcaga caagctcatc 3360

gcccgcaaga aagattggga ccctaagaaa tacgggggat ttgactcacc caccgtagcc 3420

tattctgtgc tggtggtagc taaggtggaa aaaggaaagt ctaagaagct gaagtccgtg 3480

aaggaactct tgggaatcac tatcatggaa agatcatcct ttgaaaagaa ccctatcgat 3540

ttcctggagg ctaagggtta caaggaggtc aagaaagacc tcatcattaa actgccaaaa 3600

tactctctct tcgagctgga aaatggcagg aagagaatgt tggccagcgc cggagagctg 3660

caaaagggaa acgagcttgc tctgccctcc aaatatgtta attttctcta tctcgcttcc 3720

cactatgaaa agctgaaagg gtctcccgaa gataacgagc agaagcagct gttcgtcgaa 3780

cagcacaagc actatctgga tgaaataatc gaacaaataa gcgagttcag caaaagggtt 3840

atcctggcgg atgctaattt ggacaaagta ctgtctgctt ataacaagca ccgggataag 3900

cctattaggg aacaagccga gaatataatt cacctcttta cactcacgaa tctcggagcc 3960

cccgccgcct tcaaatactt tgatacgact atcgaccgga aacggtatac cagtaccaaa 4020

gaggtcctcg atgccaccct catccaccag tcaattactg gcctgtacga aacacggatc 4080

gacctctctc aactgggcgg cgactag 4107

(SEQ ID NO: 6628)

If the above Cas9 sequences are fused with a peptide or polypeptide at the C-terminus (e.g., an inactive Cas9 fused with a transcription repressor at the C-terminus), it is understood that the stop codon will be removed.

### VI. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule are described, e.g., in Jinek el al., SCIENCE 2012; 337(6096):8 16-821.

### VII. Template Nucleic Acids (For Introduction of Nucleic Acids)

The term "template nucleic acid" or "donor template" as used herein refers to a nucleic acid to be inserted at or near a target sequence that has been modified, e.g., cleaved, by a CRISPR system of the present invention. In an embodiment, nucleic acid sequence at or near the target sequence is modified to have some or all of the sequence of the template nucleic acid, typically at or near cleavage site(s). In an embodiment, the template nucleic acid is single stranded. In an alternate embodiment, the template nucleic acid is double stranded. In an embodiment, the template nucleic acid is DNA, e.g., double stranded DNA. In an alternate embodiment, the template nucleic acid is single stranded DNA.

In embodiments, the template nucleic acid comprises sequence encoding a globin protein, e.g., a beta globin, e.g., comprises a beta globin gene. In an embodiment, the beta globin encoded by the nucleic acid comprises one or more mutations, e.g., anti-sickling mutations. In an embodiment, the beta globin encoded by the nucleic acid comprises the mutation T87Q. In an embodiment, the beta globin encoded by the nucleic acid comprises the mutation G16D. In an embodiment, the beta globin encoded by the nucleic acid comprises the mutation E22A. In an embodiment, the beta globin gene comprises the mutations G16D, E22A and T87Q. In embodiments, the template nucleic acid further comprises one or more regulatory elements, e.g., a promoter (e.g., a human beta globin promoter), a 3' enhancer, and/or at least a portion of a globin locus control regoin (e.g., one or more DNAseI hypersensitivity sites (e.g., HS2, HS3 and/or HS4 of the human globin locus)).

In other embodiments, the template nucleic acid comprises sequence encoding a gamma globin, e.g., comprises a gamma globin gene. In embodiments, the template nucleic acid comprises sequence encoding more than one copy of a gamma globin protein, e.g., comprises two or more, e.g., two, gamma globin gene sequences. In embodiments, the template nucleic acid further comprises one or more regulatory elements, e.g., a promotor and/or enhancer.

In an embodiment, the template nucleic acid alters the structure of the target position by participating in a homology directed repair event. In an embodiment, the template nucleic acid alters the sequence of the target position. In an embodiment, the template nucleic acid results in the incorporation of a modified, or non-naturally occurring base into the target nucleic acid.

Mutations in a gene or pathway described herein may be corrected using one of the approaches discussed herein. In an embodiment, a mutation in a gene or pathway described herein is corrected by homology directed repair (HDR) using a template nucleic acid. In an embodiment, a mutation in a gene or pathway described herein is corrected by homologous recombination (HR) using a template nucleic acid. In an embodiment, a mutation in a gene or pathway described herein is corrected by Non-Homologous End Joining (NHEJ) repair using a template nucleic acid. In other embodiments, nucleic acid encoding molecules of interest may be inserted at or near a site modified by a CRISPR system of the present invention. In embodiments, the template nucleic acid comprises regulatory elements, e.g., one or more promotors and/or enhancers, operably linked to the nucleic acid sequence encoding a molecule of interest, e.g., as described herein.

### HDR or HR Repair and Template Nucleic Acids

As described herein, nuclease-induced homology directed repair (HDR) or homologous recombination (HR) can be used to alter a target sequence and correct (e.g., repair or edit) a mutation in the genome. While not wishing to be bound by theory, it is believed that alteration of the target sequence occurs by repair based on a donor template or template nucleic acid. For example, the donor template or the template nucleic acid provides for alteration of the target sequence. It is contemplated that a plasmid donor or linear double stranded template can be used as a template for homologous recombination. It is further contemplated that a single stranded donor template can be used as a template for alteration of the target sequence by alternate methods of homology directed repair (e.g., single strand annealing) between the target sequence and the donor template. Donor template-effected alteration of a target sequence may depend on cleavage by a Cas9 molecule. Cleavage by Cas9 can comprise a double strand break, one single strand break, or two single strand breaks.

In an embodiment, a mutation can be corrected by either a single double-strand break or two single strand breaks. In an embodiment, a mutation can be corrected by providing a template and a CRISPR/Cas9 system that creates (1) one double strand break, (2) two single strand breaks, (3) two double stranded breaks with a break occurring on each side of the target sequence, (4) one double stranded break and two single strand breaks with the double strand break and two single strand breaks occurring on each side of the target sequence, (5) four single stranded breaks with a pair of single stranded breaks occurring on each side of the target sequence, or (6) one single strand break.

### Double strand break mediated correction

In an embodiment, double strand cleavage is effected by a Cas9 molecule having cleavage activity associated with an HNH-like domain and cleavage activity associated with a RuvC-like domain, e.g., an N-terminal RuvC-like domain, e.g., a wild type Cas9. Such embodiments require only a single gRNA.

### Single strand break mediated correction

In other embodiments, two single strand breaks, or nicks, are effected by a Cas9 molecule having nickase activity, e.g., cleavage activity associated with an HNH-like domain or cleavage activity associated with an N-terminal RuvC-like domain. Such embodiments require two gRNAs, one for placement of each single strand break. In an embodiment, the Cas9 molecule having nickase activity cleaves the strand to which the gRNA hybridizes, but not the strand that is complementary to the strand to which the gRNA hybridizes. In an embodiment, the Cas9 molecule having nickase activity does not cleave the strand to which the gRNA hybridizes, but rather cleaves the strand that is complementary to the strand to which the gRNA hybridizes.

In an embodiment, the nickase has HNH activity, e.g., a Cas9 molecule having the RuvC activity inactivated, e.g., a Cas9 molecule having a mutation at D10, e.g., the D10A mutation. D10A inactivates RuvC; therefore, the Cas9 nickase has (only) HN H activity and will cut on the strand to which the gRNA hybridizes (e.g., the complementary strand, which does not have the NGG PAM on it). In other embodiments, a Cas9 molecule having an H840, e.g., an H840A, mutation can be used as a nickase. H840A inactivates HNH; therefore, the Cas9 nickase has (only) RuvC activity and cuts on the non-complementary strand (e.g., the strand that has the NGG PAM and whose sequence is identical to the gRNA).

In an embodiment, in which a nickase and two gRNAs are used to position two single strand nicks, one nick is on the + strand and one nick is on the - strand of the target nucleic acid. The PAMs are outwardly facing. The gRNAs can be selected such that the gRNAs are separated by, from about 0-50, 0- 100, or 0-200 nucleotides. In an embodiment, there is no overlap between the target sequence that is complementary to the targeting domains of the two gRNAs. In an embodiment, the gRNAs do not overlap and are separated by as much as 50, 100, or 200 nucleotides. In an embodiment, the use of two gRNAs can increase specificity, e.g., by decreasing off-target binding (Ran el cil., CELL 2013).

In an embodiment, a single nick can be used to induce HDR. It is contemplated herein that a single nick can be used to increase the ratio of HDR, HR or NHEJ at a given cleavage site.

### Placement of the double strand break or a single strand break relative to target position

The double strand break or single strand break in one of the strands should be sufficiently close to target position such that correction occurs. In an embodiment, the distance is not more than 50, 100, 200, 300, 350 or 400 nucleotides. While not wishing to be bound by theory, it is believed that the break should be sufficiently close to target position such that the break is within the region that is subject to exonuclease-mediated removal during end resection. If the distance between the target position and a break is too great, the mutation may not be included in the end resection and, therefore, may not be corrected, as donor sequence may only be used to correct sequence within the end resection region.

In an embodiment, in which a gRNA (unimolecular (or chimeric) or modular gRNA) and Cas9 nuclease induce a double strand break for the purpose of inducing HDR- or HR-mediated correction, the cleavage site is between 0-200 bp (e.g., 0 to 175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 1 25, 75 to 100 bp) away from the target position. In an embodiment, the cleavage site is between 0- 100 bp (e.g., 0 to 75, 0 to 50, 0 to 25, 25 to 100, 25 to 75, 25 to 50, 50 to 100, 50 to 75 or 75 to 100 bp) away from the target position.

In an embodiment, in which two gRNAs (independently, unimolecular (or chimeric) or modular gRNA) complexing with Cas9 nickases induce two single strand breaks for the purpose of inducing HDR-mediated correction, the closer nick is between 0-200 bp (e.g., 0 to 175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position and the two nicks will ideally be within 25-55 bp of each other (e.g., 25 to 50, 25 to 45, 25 to 40, 25 to 35, 25 to 30, 30 to 55, 30 to 50, 30 to 45, 30 to 40, 30 to 35, 35 to 55, 35 to 50, 35 to 45, 35 to 40, 40 to 55, 40 to 50, 40 to 45 bp) and no more than 100 bp away from each other (e.g., no more than 90, 80, 70, 60, 50, 40, 30, 20, 10 or 5 bp away from each other). In an embodiment, the cleavage site is between 0- 100 bp (e.g., 0 to 75, 0 to 50, 0 to 25, 25 to 100, 25 to 75, 25 to 50, 50 to 100, 50 to 75 or 75 to 100 bp) away from the target position.

In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a Cas9 nuclease) and two single strand breaks or paired single stranded breaks (i.e., two gRNAs complex with Cas9 nickases) on either side of the target position (e.g., the first gRNA is used to target upstream (i.e., 5') of the target positionand the second gRNA is used to target downstream (i.e., 3') of the target position). In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position (e.g., the first gRNA is used to target upstream (i.e., 5') of the target position and the second gRNA is used to target downstream (i.e., 3') of the target position). The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35. to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a Cas9 nuclease) and two single strand breaks or paired single stranded breaks (i.e., two gRNAs complex with Cas9 nickases) on two target sequences (e.g., the first gRNA is used to target an upstream (i.e., 5') target sequence and the second gRNA is used to target a downstream (i.e., 3') target sequence of an insertion site. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of an insertion site (e.g., the first gRNA is used to target an upstream (i.e., 5') target sequence described herein, and the second gRNA is used to target a downstream (i.e., 3') target sequence described herein). The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

### Length of the homology arms

The homology arm should extend at least as far as the region in which end resection may occur, e.g., in order to allow the resected single stranded overhang to find a complementary region within the donor template. The overall length could be limited by parameters such as plasmid size or viral packaging limits. In an embodiment, a homology arm does not extend into repeated elements, e.g., ALU repeats, LINE repeats. A template may have two homology arms of the same or different lengths.

Exemplary homology arm lengths include at least 25, 50, 100, 250, 500, 750 or 1000 nucleotides.

Target position, as used herein, refers to a site on a target nucleic acid (e.g., the chromosome) that is modified by a Cas9 molecule-dependent process. For example, the target position can be a modified Cas9 molecule cleavage of the target nucleic acid and template nucleic acid directed modification, e.g., correction, of the target position. In an embodiment, a target position can be a site between two nucleotides, e.g., adjacent nucleotides, on the target nucleic acid into which one or more nucleotides is added. The target position may comprise one or more nucleotides that are altered, e.g., corrected, by a template nucleic acid. In an embodiment, the target position is within a target sequence (e.g., the sequence to which the gRN A binds). In an embodiment, a target position is upstream or downstream of a target sequence (e.g., the sequence to which the gRNA binds).

Typically, the template sequence undergoes a breakage mediated or catalyzed recombination with the target sequence. In an embodiment, the template nucleic acid includes sequence that corresponds to a site on the target sequence that is cleaved by a Cas9 mediated cleavage event. In an embodiment, the template nucleic acid includes sequence that corresponds to both, a first site on the target sequence that is cleaved in a first Cas9 mediated event, and a second site on the target sequence that is cleaved in a second Cas9 mediated event.

In an embodiment, the template nucleic acid can include sequence which results in an alteration in the coding sequence of a translated sequence, e.g., one which results in the substitution of one amino acid for another in a protein product, e.g., transforming a mutant allele into a wild type allele, transforming a wild type allele into a mutant allele, and/or introducing a stop codon, insertion of an amino acid residue, deletion of an amino acid residue, or a nonsense mutation.

In other embodiments, the template nucleic acid can include sequence which results in an alteration in a non-coding sequence, e.g., an alteration in an exon or in a 5' or 3' non-translated or non-transcribed region. Such alterations include an alteration in a control element, e.g., a promoter, enhancer, and an alteration in a cis-acting or trans-acting control element.

The template nucleic acid can include sequence which, when integrated, results in:
decreasing the activity of a positive control element;
increasing the activity of a positive control element;
decreasing the activity of a negative control element;
increasing the activityof a negative control element;
decreasing the expression of a gene;
increasing the expression of a gene;
increasing resistance to a disorder or disease;
increasing resistance to viral entry;
correcting a mutation or altering an unwanted amino acid residue;
conferring, increasing, abolishing or decreasing a biological property of a gene product, e.g., increasing the enzymatic activity of an enzyme, or increasing the ability of a gene product to interact with another molecule.

The template nucleic acid can include sequence which results in:
a change in sequence of 1 , 2, 3, 4, 5, 6, 7, 8, 9, 10, 1 1 , 12 or more nucleotides of the target sequence.

In an embodiment, the template nucleic acid is 20+/- 10, 30+/- 10, 40+/- 10, 50+/- 10, 60+/- 10, 70+/- 10, 80+/- 10, 90+/- 10, 100+/- 10, 1 10+/- 10, 120+/- 10, 130+/- 10, 140+/- 10, 150+/- 10, 160+/- 10, 170+/- 10, 1 80+/- 10, 190+/- 10, 200+/- 10, 210+/-10, 220+/- 10, 200-300, 300-400, 400-500, 500-600, 600-700, 700-800, 800-900, 900-1000, 1000-2000, 2000-3000 or more than 3000 nucleotides in length.

A template nucleic acid comprises the following components:
[5' homology arm]-[insertion sequence]-[3' homology arm].

The homology arms provide for recombination into the chromosome, which can replace the undesired element, e.g., a mutation or signature, with the replacement sequence. In an embodiment, the homology arms flank the most distal cleavage sites.

In an embodiment, the 3' end of the 5' homology arm is the position next to the 5' end of the replacement sequence. In an embodiment, the 5' homology arm can extend at least 10, 20, 30, 40, 50, 60, 70, 80, 90, 100, 120, 150, 180, 200, 300, 400, 500, 600, 700, 800, 900, 1000, 1500, or 2000 nucleotides 5' from the 5' end of the replacement sequence.

In an embodiment, the 5' end of the 3' homology arm is the position next to the 3' end of the replacement sequence. In an embodiment, the 3' homology arm can extend at least 10, 20, 30, 40, 50, 60, 70, 80, 90, 100, 120, 150, 180, 200, 300, 400, 500, 600, 700, 800, 900, 1000, 1500, or 2000 nucleotides 3' from the 3' end of the replacement sequence.

It is contemplated herein that one or both homology arms may be shortened to avoid including certain sequence repeat elements, e.g., Alu repeats, LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

It is contemplated herein that template nucleic acids for correcting a mutation may designed for use as a single-stranded oligonucleotide (ssODN). When using a ssODN, 5' and 3' homology arms may range up to about 200 base pairs (bp) in length, e.g., at least 25, 50, 75, 100, 125, 150, 175, or 200 bp in length. Longer homology arms are also contemplated for ssODNs as improvements in oligonucleotide synthesis continue to be made.

### NHEJ Approaches for Gene Targeting

As described herein, nuclease-induced non-homologous end-joining (NHEJ) can be used to target gene-specific knockouts. Nuclease-induced NHEJ can also be used to remove (e.g., delete) sequence in a gene of interest.

While not wishing to be bound by theory, it is believed that, in an embodiment, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, at one or both strands, prior to rejoining of the ends. This results in the presence of insertion and/or deletion (indel) mutations in the DNA sequence at the site of the NHEJ repair. Two-thirds of these mutations may alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in noncritical regions of the protein.

The indel mutations generated by NHEJ are unpredictable in nature; however, at a given break site certain indel sequences are favored and are over represented in the population. The lengths of deletions can vary widely; most commonly in the 1 -50 bp range, but they can easily reach greater than 100-200 bp. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double strand cleaving Cas9 molecules and single strand, or nickase, Cas9 molecules can be used in the methods and compositions described herein to generate NHEJ- mediated indels. NHEJ-mediated indels targeted to the gene, e.g., a coding region, e.g., an early coding region of a gene of interest can be used to knockout (i.e., eliminate expression of) a gene of interest. For example, early coding region of a gene of interest includes sequence immediately following a transcription start site, within a first exon of the coding sequence, or within 500 bp of the transcription start site (e.g., less than 500, 450, 400, 350, 300, 250, 200, 150, 100 or 50 bp).

### Placement of double strand or single strand breaks relative to the target position

In an embodiment, in which a gRNA and Cas9 nuclease generate a double strand break for the purpose of inducing NHEJ-mediated indels, a gRNA, e.g., a unimolecular (or chimeric) or modular gRNA molecule, is configured to position one double-strand break in close proximity to a nucleotide of the target position. In an embodiment, the cleavage site is between 0-500 bp away from the target position (e.g., less than 500, 400, 300, 200, 100, 50, 40, 30, 25, 20, 15, 10, 9, 8, 7, 6, 5, 4, 3, 2 or 1 bp from the target position).

In an embodiment, in which two gRNAs complexing with Cas9 nickases induce two single strand breaks for the puipose of inducing NHEJ-mediated indels, two gRNAs, e.g., independently, unimolecular (or chimeric) or modular gRNA, are configured to position two single-strand breaks to provide for NHEJ repair a nucleotide of the target position. In an embodiment, the gRNAs are configured to position cuts at the same position, or within a few nucleotides of one another, on different strands, essentially mimicking a double strand break. In an embodiment, the closer nick is between 0-30 bp away from the target position (e.g., less than 30, 25, 20, 1 , 10, 9, 8, 7, 6, 5, 4, 3, 2 or 1 bp from the target position), and the two nicks are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In an embodiment, the gRNAs are configured to place a single strand break on either side of a nucleotide of the target position.

Both double strand cleaving Cas9 molecules and single strand, or nickase, Cas9 molecules can be used in the methods and compositions described herein to generate breaks both sides of a target position. Double strand or paired single strand breaks may be generated on both sides of a target position to remove the nucleic acid sequence between the two cuts (e.g., the region between the two breaks is deleted). In one embodiment, two gRNAs, e.g., independently, unimolecular (or chimeric) or modular gRNA, are configured to position a double-strand break on both sides of a target position (e.g., the first gRNA is used to target upstream (i.e., 5') of the mutation in a gene or pathway described herein, and the second gRNA is used to target downstream (i.e., 3') of the mutation in a gene or pathway described herein). In an alternate embodiment, three gRNAs, e.g., independently, unimolecular (or chimeric) or modular gRNA, are configured to position a double strand break (i.e., one gRNA complexes with a Cas9 nuclease) and two single strand breaks or paired single stranded breaks (i.e., two gRNAs complex with Cas9 nickases) on either side of a target position (e.g., the fu st gRNA is used to target upstream (i.e., 5') of the mutation in a gene or pathway described herein, and the second gRNA is used to target downstream (i.e., 3') of the mutation in a gene or pathway described herein). In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position (e.g., the first gRNA is used to target upstream (i.e., 5') of the mutation in a gene or pathway described herein, and the second gRNA is used to target downstream (i.e., 3') of the mutation in a gene or pathway described herein). The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

In other embodiments, the insertion of template nucleic acid may be mediated by microhomology end joining (MMEJ). See, e.g., Saksuma et al., "MMEJ-assisted gene knock-in using TALENs and CRISPR-Cas9 with the PITCh systems." Nature Protocols 11, 118-133 (2016) doi:10.1038/nprot.2015.140 Published online 17 December 2015, the contents of which are incorporated by reference in their entirety.

### VIII. Systems Comprising More Than One gRNA Molecule

While not intending to be bound by theory, it has been surprisingly shown herein that the targeting of two target sequences (e.g., by two gRNA molecule/Cas9 molecule complexes which each induce a single- or double-strand break at or near their respective target sequences) located in close proximity on a continuous nucleic acid induces excision (e.g., deletion) of the nucleic acid sequence (or at least 80%, 85%, 90%, 95%, 96%, 97%, 98%, 99% of the nucleic acid sequence) located between the two target sequences. In some aspects, the present disclosure provides for the use of two or more gRNA molecules that comprise targeting domains targeting target sequences in close proximity on a continuous nucleic acid, e.g., a chromosome, e.g., a gene or gene locus, including its introns, exons and regulatory elements. The use may be, for example, by introduction of the two or more gRNA molecules, together with one or more Cas9 molecules (or nucleic acid encoding the two or more gRNA molecules and/or the one or more Cas9 molecules) into a cell.

In some aspects, the target sequences of the two or more gRNA molecules are located at least 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10,000, 11,000, 12,000, 13,000, 14,000, or 15,000 nucleotides apart on a continuous nucleic acid, but not more than 25,000 nucleotides apart on a continuous nucleic acid. In an embodiment, the target sequences are located about 4000 nucleotides apart. In an embodiment, the target sequences are located about 6000 nucleotides apart.

In some aspects, the plurality of gRNA molecules each target sequences within the same gene or gene locus. In another aspect, the plurality of gRNA molecules each target sequences within 2 or more different genes.

In some aspects, the invention provides compositions and cells comprising a plurality, for example, 2 or more, for example, 2, gRNA molecules of the invention, wherein the plurality of gRNA molecules target sequences less than 15,000, less than 14,000, less than 13,000, less than 12,000, less than 11,000, less than 10,000, less than 9,000, less than 8,000, less than 7,000, less than 6,000, less than 5,000, less than 4,000, less than 3,000, less than 2,000, less than 1,000, less than 900, less than 800, less than 700, less than 600, less than 500, less than 400, less than 300, less than 200, less than 100, less than 90, less than 80, less than 70, less than 60, less than 50, less than 40, or less than 30 nucleotides apart. In an embodiment, the target sequences are on the same strand of duplex nulceic acid. In an embodiment, the target sequences are on different strands of duplex nucleic acid.

In one embodiment, the invention provides a method for excising (e.g., deleting) nucleic acid disposed between two gRNA binding sites disposed less than 25,000, less than 20,000, less than 15,000, less than 14,000, less than 13,000, less than 12,000, less than 11,000, less than 10,000, less than 9,000, less than 8,000, less than 7,000, less than 6,000, less than 5,000, less than 4,000, less than 3,000, less than 2,000, less than 1,000, less than 900, less than 800, less than 700, less than 600, less than 500, less than 400, less than 300, less than 200, less than 100, less than 90, less than 80, less than 70, less than 60, less than 50, less than 40, or less than 30 nucleotides apart on the same or different strands of duplex nucleic acid. In an embodiment, the method provides for deletion of more than 50%, more than 60%, more than 70%, more than 80%, more than 85%, more than 86%, more than 87%, more than 88%, more than 89%, more than 90%, more than 91%, more than 92%, more than 93%, more than 94%, more than 95%, more than 96%, more than 97%, more than 98%, more than 99%, or 100% of the nucleotides disposed between the PAM sites associated with each gRNA binding site. In embodiments, the deletion further comprises of one or more nucleotides within one or more of the PAM sites associated with each gRNA binding site. In embodiments, the deletion also comprises one or more nucleotides outside of the region between the PAM sites associated with each gRNA binding site.

In one aspect, the two or more gRNA molecules comprise targeting domains targeting target sequences flanking a gene regulatory element, e.g., a promotor binding site, an enhancer region, or a repressor region, such that excision of the intervening sequence (or a portion of the intervening sequence) causes up- or down-regulation of a gene of interest.

In an embodiment, the two or more gRNA molecules include targeting domains comprising, e.g., consisting of, a targeting domain sequence of Table 1 or Table 5. In aspects, the two or more gRNA molecules comprise targeting domains that are complementary with sequences in the same gene. In aspects, the two or more gRNA molecules comprise targeting domains that are complementary with sequences of different genes. In an embodiment, the two or more gRNA molecules include targeting domains comprising, e.g., consisting of, a targeting domain sequence of Table 6. In an embodiment, the two or more gRNA molecules include targeting domains comprising, e.g., consisting of, a targeting domain sequence of Table 2, Table 7, Table 8 and/or Table 9. In aspects, the two or more gRNA molecules comprise targeting domains that are complementary with sequences in the same gene. In aspects, the two or more gRNA molecules comprise targeting domains that are complementary with sequences of different genes. In an embodiment, the two or more gRNA molecules are selected from the gRNA molecules of Table 7, Table 8 and/or Table 9. In an embodiment, the first and second gRNA molecules comprise targeting domains comprising, e.g., consisting of, targeting domain sequences selected from Tables 1-9, and are selected from different tables, e.g., and comprise targeting domains that are complementary with sequences of different genes.

In one aspect, the two or more gRNA molecules comprise targeting domains targeting target sequences flanking a gene regulatory element, e.g., a promotor binding site, an enhancer region, or a repressor region, such that excision of the intervening sequence (or a portion of the intervening sequence) causes up- or down-regulation of a gene of interest. By way of example, the two or more gRNA molecules comprise targeting domains targeting target sequences flanking a GATA1 binding site (or portion thereof) or TAL1 binding site (or portion thereof) of the erythroid enhancer region of the BCL11a gene (e.g., within the +55, +58 or +62 region). In other embodiments, the gRNA molecule or molecules do not result in a disruption of the GATA1 binding site or TAL1 binding site within the BCL11a Enhancer.

In an embodiment, the two or more gRNA molecules comprise targeting domains that comprise, e.g., consist of, targeting domains selected from Table 1. In an embodiment, the two or more gRNA molecules comprise targeting domains that comprise, e.g., consist of, targeting domains selected from Table 2. In an embodiment, the two or more gRNA molecules comprise targeting domains that comprise, e.g., consist of, targeting domains selected from Table 3. In an embodiment, the two or more gRNA molecules comprise targeting domains that comprise, e.g., consist of, targeting domains selected from Table 4. In an embodiment, the two or more gRNA molecules comprise targeting domains that comprise, e.g., consist of, targeting domains selected from Table 5. In an embodiment, the two or more gRNA molecules comprise targeting domains that comprise, e.g., consist of, targeting domains selected from Table 6. In an embodiment, the two or more gRNA molecules comprise targeting domains that comprise, e.g., consist of, targeting domains selected from Table 7. In an embodiment, the two or more gRNA molecules comprise targeting domains that comprise, e.g., consist of, targeting domains selected from Table 8. In an embodiment, the two or more gRNA molecules comprise targeting domains that comprise, e.g., consist of, targeting domains selected from Table 9.

In aspects, the two or more gRNA molecules comprise targeting domains comprising, e.g., consisting of the targeting domain sequence pairs listed in section II, above.

Without being bound by theory, it is believed that increasing the expression of fetal hemoglobin (e.g., gamma globin) while simultaneously reducing or eliminating expression of beta globin (e.g., beta globin comprising a sickling mutation), will result in increased efficacy in treating a hemoglobinopathy, e.g., sickle cell disease. Thus, in one aspect, the two or more gRNA molecules comprise a first gRNA molecule that causes an increase in gamma globin expression and a second gRNA molecule that reduces or eliminates expression of beta globin, e.g., beta globin carrying a sickle cell mutation. In embodiments, the two or more gRNA molecules comprise a first gRNA molecule that targets a sequence within a BCL11a enhancer region, e.g., as described herein, e.g., a gRNA molecule comprising a targeting domain comprising, e.g., consisting of, a targeting domain of Table 7, Table 8 or Table 9, and a second gRNA molecule that targets a sequence of a sickle globin gene (e.g., a beta globin gene comprising a sickling mutation).

### IX. Properties of the gRNA

It has further been surprisingly shown herein that gRNA molecules and CRISPR systems comprising said gRNA molecules produce similar or identical indel patterns across multiple experiments using the same cell type, method of delivery and crRNA/tracr components. Without being bound by theory, it is believed that some indel patterns may be more advantageous than others. For example, indels which predominantly include insertions and/or deletions wich result in a "frameshift mutation" (e.g., 1- or 2- base pair insertion or deletions, or any insertion or deletion where n/3 is not a whole number (where n=the number of nucleotides in the insertion or deletion)) may be beneficial in reducing or eliminating expression of a functional protein. Likewise, indels which predominantly include "large deletions" (deletions of more than 10, 11, 12, 13, 14, 15, 20, 25, or 30 nucleotides) may also be beneficial in, for example, removing critical regulatory sequences such as promoter binding sites, which may similarly have an impoved effect on expression of functional protein. While the indel patterns induced by a given gRNA/CRISPR system have surprisingly been found to be consistently reproduced across cell types, as described herein, not any single indel structure will inevtiably be produced in a given cell upon introduction of a gRNA/CRISPR system.

The invention thus provides for gRNA molecules which create a beneficial indel pattern or structure, for example, which have indel patterns or structures predominantly composed of frameshif mutation(s) and/or large deletions. Such gRNA molecules may be selected by assessing the indel pattern or structure created by a candidate gRNA molecule in a test cell (for example, a HEK293 cell) or in the cell of interest, e.g., a HSPC cell by NGS, as described herein. As shown in the Examples, gRNA molecules have been discovered, which, when introduced into the desired cell population, result in a population of cells comprising a significant fraction of the cells having a frameshift mutation in the targeted gene. In some cases, the rate of frameshift mutation is as high as 75%, 80%, 85%, 90% or more. The invention thus provides for populations of cells which comprise at least about 40% of cells (e.g., at least about 45%, at least about 50%, at least about 55%, at least about 60%, at least about 65%, at least about 70%, at least about 75%, at least about 80%, at least about 85%, at least about 90%, at least about 95%, or at least about 99%) having a frameshift mutation, e.g., as described herein, at or near the target site of a gRNA moleucle described herein. The invention also provides for populations of cells which comprise at least about 50% of cells (e.g., at least about 55%, at least about 60%, at least about 65%, at least about 70%, at least about 75%, at least about 80%, at least about 85%, at least about 90%, at least about 95%, or at least about 99%) having a frameshift mutation, e.g., as described herein, at or near the target site of a gRNA moleucle described herein.

The invention thus provides methods of selecting gRNA molecules for use in the therapeutic methods of the invention comprising: 1) providing a plurality of gRNA molecules to a target of interest, 2) assessing the indel pattern or structure created by use of said gRNA molecules, 3) selecting a gRNA molecule that forms an indel pattern or structure composed predominantly of frameshift mutations, large deletions or a combination thereof, and 4) using said selected gRNA in a methods of the invention.

The invention further provides methods of altering cells, and altered cells, wherein a particular indel pattern is constently produced with a given gRNA/CRISPR system in that cell type. The indel patterns, including the top 5 most frequently occuring indels observed with the gRNA/CRISPR systems described herein are disclosed, for example, in the Examples. As shown in the examples, populations of cells are generated, wherein a signficant fraction of the cells comprises one of the top 5 indels (for example, populations of cells wherein one of the top 5 indels is present in more than 30%, more than 40%, more than 50%, more than 60% or more of the cells of the population. Thus, the invention provides cells, e.g., HSPCs (as described herein), which comprise an indel of any one of the top 5 indels observed with a given gRNA/CRISPR system. Further, the invention provides populations of cells, e.g., HSPCs (as described herein), which when assessed by, for example, NGS, comprise a high percentage of cells comprising one of the top 5 indels described herein for a given gRNA/CRISPR system. When used in connection with indel pattern analysis, a "high percentage" refers to at least about 50% (e.g., at least about 55%, at least about 60%, at least about 65%, at least about 70%, at least about 75%, at least about 80%, at least about 85%, at least about 90%, at least about 95%, or at least about 99%) of the cells of the population comprising one of the top 5 indels described herein for a given gRNA/CRISPR system. In other embodiments, the population of cells comprises at least about 25% (e.g., from about 25% to about 60%, e.g., from about 25% to about 50%, e.g., from about 25% to about 40%, e.g., from about 25% to about 35%) of cells which have one of the top 5 indels described herein for a given gRNA/CRISPR system. In embodiments, the top indels, e.g., top 5 indels for a given gRNA/CRISPR system which targets a +58 region of the BCL11a enhancer are provided in Table 15, Figure 25, and Table 37. In embodiments, the top indels, e.g., top 5 indels for a given gRNA/CRISPR system which targets a HPFH region are provided in Table 26, Table 27 and Table 37.

It has also been discovered that certain gRNA molecules do not create indels at off-target sequences within the genome of the target cell type, or produce indels at off target sites at very low frequencies (e.g., <5% of cells within a population) relative to the frequency of indel creation at the target site. Thus, the invention provides for gRNA molecules and CRISPR systems which do not exhibit off-target indel formation in the target cell type, or which produce a frequency of off-target indel formation of <5%. In embodiments, the invention provides gRNA molecules and CRISPR systems which do not exhibit any off target indel formation in the target cell type. Thus, the invention further provides a cell, e.g., a population of cells, e.g., HSPCs, e.g., as described herein, which comprise an indel at or near a target site of a gRNA molecule described herein (e.g., a frameshift indel, or any one of the top 5 indels produced by a given gRNA/CRISPR system, e.g., as described herein), but does not comprise an indel at any off-target site of the gRNA molecule. In other embodiments, the invention further provides a a population of cells, e.g., HSPCs, e.g., as described herein, which comprises > 50% of cells which have an indel at or near a target site of a gRNA molecule described herein (e.g., a frameshift indel, or any one of the top 5 indels produced by a given gRNA/CRISPR system, e.g., as described herein), but which comprises less than 5%, e.g., less than 4%, less than 3%, less than 2% or less than 1%, of cells comprising an indel at any off-target site of the gRNA molecule.

In embodiments, the indel produced by a CRISPR system described herein (e.g., a CRISPR system comprising a gRNA molecule described herein), does not comprise a nucelotide of a GATA-1 binding site and/or does not comprise a nucelotide of a TAL-1 binding site (e.g., does not comprise a nucleotide of a GATA-1 binding site and/or TAL-1 binding site described in Figure 25).

### X. Delivery/Constructs

The components, e.g., a Cas9 molecule or gRNA molecule, or both, can be delivered, formulated, or administered in a variety of forms. As a non-limiting example, the gRNA molecule and Cas9 molecule can be formulated (in one or more compositions), directly delivered or administered to a cell in which a genome editing event is desired. Alternatively, nucleic acid encoding one or more components, e.g., a Cas9 molecule or gRNA molecule, or both, can be formulated (in one or more compositions), delivered or administered. In one aspect, the gRNA molecule is provided as DNA encoding the gRNA molecule and the Cas9 molecule is provided as DNA encoding the Cas9 molecule. In one embodiment, the gRNA molecule and Cas9 molecule are encoded on separate nucleic acid molecules. In one embodiment, the gRNA molecule and Cas9 molecule are encoded on the same nucleic acid molecule. In one aspect, the gRNA molecule is provided as RNA and the Cas9 molecule is provided as DNA encoding the Cas9 molecule. In one embodiment, the gRNA molecule is provided with one or more modifications, e.g., as described herein. In one aspect, the gRNA molecule is provided as RNA and the Cas9 molecule is provided as mRNA encoding the Cas9 molecule. In one aspect, the gRNA molecule is provided as RNA and the Cas9 molecule is provided as a protein. In one embodiment, the gRNA and Cas9 molecule are provided as a ribonuclear protein complex (RNP). In one aspect, the gRNA molecule is provided as DNA encoding the gRNA molecule and the Cas9 molecule is provided as a protein.

Delivery, e.g., delivery of the RNP, (e.g., to HSPC cells as described herein) may be accomplished by, for example, electroporation (e.g., as known in the art) or other method that renders the cell membrane permeable to nucleic acid and/or polypeptide molecules. In embodiments, the CRISPR system, e.g., the RNP as described herein, is delivered by electroporation using a 4D-Nucleofector (Lonza), for example, using program CM-137 on the 4D-Nucleofector (Lonza). In embodiments, the CRISPR system, e.g., the RNP as described herein, is delivered by electroporation using a voltage from about 800 volts to about 2000 volts, e.g., from about 1000 volts to about 1800 volts, e.g., from about 1200 volts to about 1800 volts, e.g., from about 1400 volts to about 1800 volts, e.g., from about 1600 volts to about 1800 volts, e.g., about 1700 volts, e.g., at a voltage of 1700 volts. In embodiments, the pulse width/lenth is from about 10 ms to about 50 ms, e.g., from about 10 ms to about 40 ms, e.g., from about 10 ms to about 30 ms, e.g., from about 15 ms to about 25 ms, e.g., about 20 ms, e.g., 20 ms. In embodiments, 1, 2, 3, 4, 5, or more, e.g., 2, e.g., 1 pulses are used. In an embodiment, the CRISPR system, e.g., the RNP as described herein, is delivered by electroporation using a voltage of about 1700 volts (e.g., 1700 volts), a pulse width of about 20 ms (e.g., 20 ms), using a single (1) pulse. In embodiments, electroporation is accomplished using a Neon electroporator. Additional techniques for rendering the membrane permeable are known in the art and include, for example, cell squeezing (e.g., as described in WO2015/023982 and WO2013/059343, the contents of which are hereby incorporated by reference in their entirety), nanoneedles (e.g., as described in Chiappini et al., Nat. Mat., 14; 532-39, or US2014/0295558, the contents of which are hereby incorporated by reference in their entirety) and nanostraws (e.g., as described in Xie, ACS Nano, 7(5); 4351-58, the contents of which are hereby incorporated by reference in their entirety).

When a component is delivered encoded in DNA the DNA will typically include a control region, e.g., comprising a promoter, to effect expression. Useful promoters for Cas9 molecule sequences include CMV, EF- lalpha, MSCV, PGK, CAG control promoters. Useful promoters for gRNAs include HI, EF- 1a and U6 promoters. Promoters with similar or dissimilar strengths can be selected to tune the expression of components. Sequences encoding a Cas9 molecule can comprise a nuclear localization signal (NLS), e.g., an SV40 NLS. In an embodiment, a promoter for a Cas9 molecule or a gRNA molecule can be, independently, inducible, tissue specific, or cell specific.

### DNA-based Delivery of a Cas9 molecule and or a gRNA molecule

DNA encoding Cas9 molecules and/or gRNA molecules, can be administered to subjects or delivered into cells by art-known methods or as described herein. For example, Cas9-encoding and/or gRNA-encoding DNA can be delivered, e.g., by vectors (e.g., viral or non-viral vectors), non-vector based methods (e.g., using naked DNA or DNA complexes), or a combination thereof.

In some embodiments, the Cas9- and/or gRNA-encoding DNA is delivered by a vector (e.g., viral vector/virus, plasmid, minicircle or nanoplasmid).

A vector can comprise a sequence that encodes a Cas9 molecule and/or a gRNA molecule. A vector can also comprise a sequence encoding a signal peptide (e.g., for nuclear localization, nucleolar localization, mitochondrial localization), fused, e.g., to a Cas9 molecule sequence. For example, a vector can comprise one or more nuclear localization sequence (e.g., from SV40) fused to the sequence encoding the Cas9 molecule.

One or more regulatory/control elements, e.g., a promoter, an enhancer, an intron, a polyadenylation signal, a Kozak consensus sequence, internal ribosome entry sites (IRES), a 2A sequence, and a splice acceptor or donor can be included in the vectors. In some embodiments, the promoter is recognized by RNA polymerase II (e.g., a CMV promoter). In other embodiments, the promoter is recognized by RNA polymerase III (e.g., a U6 promoter). In some embodiments, the promoter is a regulated promoter (e.g., inducible promoter). In other embodiments, the promoter is a constitutive promoter. In some embodiments, the promoter is a tissue specific promoter. In some embodiments, the promoter is a viral promoter. In other embodiments, the promoter is a non-viral promoter.

In some embodiments, the vector or delivery vehicle is a minicircle. In some embodiments, the vector or delivery vehicle is a nanoplasmid.

In some embodiments, the vector or delivery vehicle is a viral vector (e.g., for generation of recombinant viruses). In some embodiments, the virus is a DNA virus (e.g., dsDNA or ssDNA virus). In other embodiments, the virus is an RNA virus (e.g., an ssRNA virus).

Exemplary viral vectors/viruses include, e.g., retroviruses, lentiviruses, adenovirus, adeno- associated virus (AAV), vaccinia viruses, poxviruses, and herpes simplex viruses. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals.

In some embodiments, the virus infects dividing cells. In other embodiments, the virus infects non-dividing cells. In some embodiments, the virus infects both dividing and non-dividing cells. In some embodiments, the virus can integrate into the host genome. In some embodiments, the virus is engineered to have reduced immunity, e.g., in human. In some embodiments, the virus is replication-competent. In other embodiments, the virus is replication- defective, e.g., having one or more coding regions for the genes necessary for additional rounds of virion replication and/or packaging replaced with other genes or deleted. In some embodiments, the virus causes transient expression of the Cas9 molecule and/or the gRNA molecule. In other embodiments, the viurs causes long-lasting, e.g., at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, e.g., from at least about 4 kb to at least about 30 kb, e.g., at least about 5 kb, 10 kb, 15 kb, 20 kb, 25 kb, 30 kb, 35 kb, 40 kb, 45 kb, or 50 kb.

In some embodiments, the Cas9- and/or gRNA-encoding DNA is delivered by a recombinant retrovirus. In some embodiments, the retrovirus (e.g., Moloney murine leukemia vims) comprises a reverse transcriptase, e.g., that allows integration into the host genome. In some embodiments, the retrovirus is replication-competent. In other embodiments, the retrovirus is replication-defective, e.g., having one of more coding regions for the genes necessary for additional rounds of virion replication and packaging replaced with other genes, or deleted.

In some embodiments, the Cas9- and/or gRNA-encoding DNA is delivered by a recombinant lentivirus. For example, the lentivirus is replication-defective, e.g., does not comprise one or more genes required for viral replication.

In some embodiments, the Cas9- and/or gRNA-encoding DNA is delivered by a recombinant adenovirus. In some embodiments, the adenovirus is engineered to have reduced immunity in human.

In some embodiments, the Cas9- and/or gRNA-encoding DNA is delivered by a recombinant AAV. In some embodiments, the AAV can incorporate its genome into that of a host cell, e.g., a target cell as described herein. In some embodiments, the AAV is a self- complementary adeno-associated virus (scAAV), e.g., a scAAV that packages both strands which anneal together to form double stranded DNA. AAV serotypes that may be used in the disclosed methods include, e.g., AAV 1, AAV2, modified AAV2 (e.g., modifications at Y444F, Y500F, Y730F and/or S662V), AAV3, modified AAV3 (e.g., modifications at Y705F, Y73 1 F and/or. T492V), AAV4, AAV5, AAV6, modified AAV6 (e.g., modifications at S663V and/or T492V), AAV8. AAV 8.2, AAV9, AAV rh 10, and pseudotyped AAV, such as AAV2/8, AAV2/5 and AAV2/6 can also be used in the disclosed methods.

In some embodiments, the Cas9- and/or gRNA-encoding DNA is delivered by a hybrid virus, e.g., a hybrid of one or more of the viruses described herein.

A Packaging cell is used to form a virus particle that is capable of infecting a host or target cell. Such a cell includes a 293 cell, which can package adenovirus, and a ψ2 cell or a PA317 cell, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed. For example, an AAV vector used in gene therapy typically only possesses inverted terminal repeat (ITR) sequences from the AAV genome which are required for packaging and gene expression in the host or target cell. The missing viral functions are supplied in trans by the packaging cell line. Henceforth, the viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

In an embodiment, the viral vector has the ability of cell type and/or tissue type recognition. For example, the viral vector can be pseudotyped with a different/alternative viral envelope glycoprotein; engineered with a cell type-specific receptor (e.g., genetic modification of the viral envelope glycoproteins to incorporate targeting ligands such as a peptide ligand, a single chain antibodie, a growth factor); and/or engineered to have a molecular bridge with dual specificities with one end recognizing a viral glycoprotein and the other end recognizing a moiety of the target cell surface (e.g., ligand-receptor, monoclonal antibody, avidin-biotin and chemical conjugation).

In an embodiment, the viral vector achieves cell type specific expression. For example, a tissue-specific promoter can be constructed to restrict expression of the transgene (Cas 9 and gRNA) in only the target cell. The specificity of the vector can also be mediated by microRNA- dependent control of transgene expression. In an embodiment, the viral vector has increased efficiency of fusion of the viral vector and a target cell membrane. For example, a fusion protein such as fusion-competent hemagglutin (HA) can be incorporated to increase viral uptake into cells. In an embodiment, the viral vector has the ability of nuclear localization. For example, aviruse that requires the breakdown of the cell wall (during cell division) and therefore will not infect a non-diving cell can be altered to incorporate a nuclear localization peptide in the matrix protein of the virus thereby enabling the transduction of non-proliferating cells.

In some embodiments, the Cas9- and/or gRNA-encoding DNA is delivered by a non- vector based method (e.g., using naked DNA or DNA complexes). For example, the DNA can be delivered, e.g., by organically modified silica or silicate (Ormosil), electroporation, gene gun, sonoporation, magnetofection, lipid-mediated transfection, dendrimers, inorganic nanoparticles, calcium phosphates, or a combination thereof.

In some embodiments, the Cas9- and/or gRNA-encoding DNA is delivered by a combination of a vector and a non-vector based method. For example, a virosome comprises a liposome combined with an inactivated virus (e.g., HIV or influenza virus), which can result in more efficient gene transfer, e.g., in a respiratory epithelial cell than either a viral or a liposomal method alone.

In an embodiment, the delivery vehicle is a non-viral vector. In an embodiment, the non- viral vector is an inorganic nanoparticle (e.g., attached to the payload to the surface of the nanoparticle).

Exemplary inorganic nanoparticles include, e.g., magnetic nanoparticles (e.g., Fe lvln0₂), or silica. The outer surface of the nanoparticle can be conjugated with a positively charged polymer (e.g., polyethylenimine, polylysine, polyserine) which allows for attachment (e.g., conjugation or entrapment) of payload. In an embodiment, the non-viral vector is an organic nanoparticle (e.g., entrapment of the payload inside the nanoparticle). Exemplary organic nanoparticles include, e.g., SNALP liposomes that contain cationic lipids together with neutral helper lipids which are coated with polyethylene glycol (PEG) and protamine and nucleic acid complex coated with lipid coating.

Exemplary lipids and/or polymers for for transfer of CRISPR systems or nucleic acid, e.g., vectors, encoding CRISPR systems or components thereof include, for example, those described in WO2011/076807, WO2014/136086, WO2005/060697, WO2014/140211, WO2012/031046, WO2013/103467, WO2013/006825, WO2012/006378, WO2015/095340, and WO2015/095346, the contents of each of the foregoing are hereby incorported by reference in their entirety. In an embodiment, the vehicle has targeting modifications to increase target cell update of nanoparticles and liposomes, e.g., cell specific antigens, monoclonal antibodies, single chain antibodies, aptamers, polymers, sugars, and cell penetrating peptides. In an embodiment, the vehicle uses fusogenic and endosome-destabilizing peptides/polymers. In an embodiment, the vehicle undergoes acid-triggered conformational changes (e.g., to accelerate endosomal escape of the cargo). In an embodiment, a stimuli-cleavable polymer is used, e.g., for release in a cellular compartment. For example, disulfide-based cationic polymers that are cleaved in the reducing cellular environment can be used.

In an embodiment, the delivery vehicle is a biological non-viral delivery vehicle. In an embodiment, the vehicle is an attenuated bacterium (e.g., naturally or artificially engineered to be invasive but attenuated to prevent pathogenesis and expressing the transgene (e.g., Listeria monocytogenes, certain Salmonella strains, Bifidobacterium longum, and modified Escherichia coli), bacteria having nutritional and tissue-specific tropism to target specific tissues, bacteria having modified surface proteins to alter target tissue specificity). In an embodiment, the vehicle is a genetically modified bacteriophage (e.g., engineered phages having large packaging capacity, less immunogenic, containing mammalian plasmid maintenance sequences and having incorporated targeting ligands). In an embodiment, the vehicle is a mammalian virus-like particle. For example, modified viral particles can be generated (e.g., by purification of the "empty" particles followed by ex vivo assembly of the virus with the desired cargo). The vehicle can also be engineered to incorporate targeting ligands to alter target tissue specificity. In an embodiment, the vehicle is a biological liposome. For example, the biological liposome is a phospholipid-based particle derived from human cells (e.g., erythrocyte ghosts, which are red blood cells broken down into spherical structures derived from the subject (e.g., tissue targeting can be achieved by attachment of various tissue or cell-specific ligands), or secretory exosomes - subject (i.e., patient) derived membrane-bound nanovescicle (30 - 100 nm) of endocytic origin (e.g., can be produced from various cell types and can therefore be taken up by cells without the need of for targeting ligands).

In an embodiment, one or more nucleic acid molecules (e.g., DNA molecules) other than the components of a Cas system, e.g., the Cas9 molecule component and/or the gRNA molecule component described herein, are delivered. In an embodiment, the nucleic acid molecule is delivered at the same time as one or more of the components of the Cas system are delivered. In an embodiment, the nucleic acid molecule is delivered before or after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas9 system are delivered. In an embodiment, the nucleic acid molecule is delivered by a different means than one or more of the components of the Cas9 system, e.g., the Cas9 molecule component and/or the gRNA molecule component, are delivered. The nucleic acid molecule can be delivered by any of the delivery methods described herein. For example, the nucleic acid molecule can be delivered by a viral vector, e.g., an integration-deficient lentivirus, and the Cas9 molecule component and/or the gRNA molecule component can be delivered by electroporation, e.g., such that the toxicity caused by nucleic acids (e.g., DNAs) can be reduced. In an embodiment, the nucleic acid molecule encodes a therapeutic protein, e.g., a protein described herein. In an embodiment, the nucleic acid molecule encodes an RNA molecule, e.g., an RNA molecule described herein. Delivery of RNA encoding a Cas9 molecule

RNA encoding Cas9 molecules (e.g., active Cas9 molecules, inactive Cas9 molecules or inactive Cas9 fusion proteins) and/or gRNA molecules, can be delivered into cells, e.g., target cells described herein, by art-known methods or as described herein. For example, Cas9-encoding and/or gRNA-encoding RNA can be delivered, e.g., by microinjection, electroporation, lipid-mediated transfection, peptide-mediated delivery, or a combination thereof.

### Delivery of Cas9 molecule as protein

Cas9 molecules (e.g., active Cas9 molecules, inactive Cas9 molecules or inactive Cas9 fusion proteins) can be delivered into cells by art-known methods or as described herein. For example, Cas9 protein molecules can be delivered, e.g., by microinjection, electroporation, lipid-mediated transfection, peptide-mediated delivery, cell squeezing or abrasion (e.g., by nanoneedles) or a combination thereof. Delivery can be accompanied by DNA encoding a gRNA or by a gRNA, e.g., by precomplexing the gRNA and the Cas9 protein in a ribonuclear protein complex (RNP).

In an aspect the Cas9 molecule, e.g., as described herein, is delivered as a protein and the gRNA molecule is delivered as one or more RNAs (e.g., as a dgRNA or sgRNA, as described herein). In embodiments, the Cas9 protein is complexed with the gRNA molecule prior to delivery to a cell, e.g., as described herein, as a ribonuclear protein complex ("RNP"). In embodiments, the RNP can be delivered into cells, e.g., described herein, by any art-known method, e.g., electroporation. As described herein, and without being bound by theory, it can be preferrable to use a gRNA moleucle and Cas9 molecule which result in high % editing at the target sequence (e.g., >85%, >90%, >95%, >98%, or >99%) in the target cell, e.g., described herein, even when the concentration of RNP delivered to the cell is reduced. Again, without being bound by theory, delivering a reduced or low concentration of RNP comprising a gRNA moleucle that produces a high % editing at the target sequence in the target cell (including at the low RNP concentration), can be beneficial because it may reduce the frequency and number of off-target editing events. In one aspect, where a low or reduced concentration of RNP is to be used, the following exemplary procedure can be used to generate the RNP with a dgRNA molecule:
1. Provide the Cas9 molecule and the tracr in solution at a high concentration (e.g., a concentration higher than the final RNP concentration to be delivered to the cell), and allow the two components to equilibrate;
2. Provide the crRNA molecule, and allow the components to equilibrate (thereby forming a high-concentration solution of the RNP);
3. Dilute the RNP solution to the desired concentration;
4. Deliver said RNP at said desired concentration to the target cells, e.g., by electroporation.

The above procedure may be modified for use with sgRNA molecules by omitting step 2, above, and in step 1, providing the Cas9 molecule and the sgRNA molecule in solution at high concentraiton, and allowing the components to equilibrate. In embodiments, the Cas9 moleucle and each gRNA component are provided in solution at a 1:2 ratio (Cas9:gRNA), e.g., a 1:2 molar ratio of Cas9:gRNA molecule. Where dgRNA molecules are used, the ratio, e.g., molar rato, is 1:2:2 (Cas9:tracr:crRNA). In embodiments, the RNP is formed at a concentration of 20uM or higher, e.g., a concentration from about 20uM to about 50 uM. In embodiments, the RNP is formed at a concentration of 10 uM or higher, e.g., a concentration from about 10 uM to about 30 uM. In embodiments, the RNP is diluted to a final concentration of 10uM or less (e.g., a concentration from about 0.01 uM to about 10uM) in a solution comprising the target cell (e.g., described herein) for delivery to said target cell. In embodiments, the RNP is diluted to a final concentration of 3uM or less (e.g., a concentration from about 0.01 uM to about 3uM) in a solution comprising the target cell (e.g., described herein) for delivery to said target cell. In embodiments, the RNP is diluted to a final concentration of 1uM or less (e.g., a concentration from about 0.01 uM to about 1uM) in a solution comprising the target cell (e.g., described herein) for delivery to said target cell. In embodiments, the RNP is diluted to a final concentration of 0.3uM or less (e.g., a concentration from about 0.01 uM to about 0.3uM) in a solution comprising the target cell (e.g., described herein) for delivery to said target cell. In embodiments, the RNP is provided at a final concentration of about 3uM in a solution comprising the target cell (e.g., described herein) for delivery to said target cell. In embodiments, the RNP is provided at a final concentration of about 1uM in a solution comprising the target cell (e.g., described herein) for delivery to said target cell. In embodiments, the RNP is provided at a final concentration of about 0.3uM in a solution comprising the target cell (e.g., described herein) for delivery to said target cell. In embodiments, the RNP is provided at a final concentration of about 0.1uM in a solution comprising the target cell (e.g., described herein) for delivery to said target cell. In embodiments, the RNP is provided at a final concentration of about 0.05uM in a solution comprising the target cell (e.g., described herein) for delivery to said target cell. In embodiments, the RNP is provided at a final concentration of about 0.03uM in a solution comprising the target cell (e.g., described herein) for delivery to said target cell. In embodiments, the RNP is provided at a final concentration of about 0.01uM in a solution comprising the target cell (e.g., described herein) for delivery to said target cell. In embodiments, the RNP is formulated in a medium suitable for electroporation. In embodiments, the RNP is delivered to cells, e.g., HSPC cells, e.g., as described herein, by electroporation, e.g., using electroporation conditions described herein.

### Bi-Modal or Differential Delivery of Components

Separate delivery of the components of a Cas system, e.g., the Cas9 molecule component and the gRNA molecule component, and more particularly, delivery of the components by differing modes, can enhance performance, e.g., by improving tissue specificity and safety.

In an embodiment, the Cas9 molecule and the gRNA molecule are delivered by different modes, or as sometimes referred to herein as differential modes. Different or differential modes, as used herein, refer modes of delivery that confer different pharmacodynamic or pharmacokinetic properties on the subject component molecule, e.g., a Cas9 molecule, gRNA molecule, or template nucleic acid. For example, the modes of delivery can result in different tissue distribution, different half-life, or different temporal distribution, e.g., in a selected compartment, tissue, or organ.

Some modes of delivery, e.g., delivery by a nucleic acid vector that persists in a cell, or in progeny of a cell, e.g., by autonomous replication or insertion into cellular nucleic acid, result- in more persistent expression of and presence of a component.

### XI. Methods of Treatment

The Cas9 systems, e.g., one or more gRNA molecules and one or more Cas9 molecules, described herein are useful for the treatment of disease in a mammal, e.g., in a human. The terms "treat," "treated," "treating," and "treatment," include the administration of cas9 systems, e.g., one or more gRNA molecules and one or more cas9 molecules, to cells to prevent or delay the onset of the symptoms, complications, or biochemical indicia of a disease, alleviating the symptoms or arresting or inhibiting further development of the disease, condition, or disorder. Treatment may also include the administration of one or more (e.g., a population of) cells, e.g., HSPCs, that have been modified by the introduction of a gRNA molecule (or more than one gRNA molecule) of the present invention, or by the introduction of a CRISPR system as described herein, or by any of the methods of preparing said cells described herein, to prevent or delay the onset of the symptoms, complications, or biochemical indicia of a disease, alleviating the symptoms or arresting or inhibiting further development of the disease, condition, or disorder. Treatment may be prophylactic (to prevent or delay the onset of the disease, or to prevent the manifestation of clinical or subclinical symptoms thereof) or therapeutic suppression or alleviation of symptoms after the manifestation of the disease. Treatment can be measured by the therapeutic measures described hererin. Thus, the methods of "treatment" of the present invention also include administration of cells altered by the introduction of a cas9 system (e.g., one or more gRNA molecules and one or more Cas9 molecules) into said cells to a subject in order to cure, reduce the severity of, or ameliorate one or more symptoms of a disease or condition, in order to prolong the health or survival of a subject beyond that expected in the absence of such treatment. For example, "treatment" includes the alleviation of a disease symptom in a subject by at least 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95% or more.

Cas9 systems comprising gRNA molecules comprising the targeting domains described herein, e.g., in Tables 1, 2, 3, 4, 5, 6, 7, 8, and/or 9, are useful for the treatment of hemoglobinopathies.

### Hemoglobinopathies

Hemoglobinopathies encompass a number of anemias of genetic origin in which there is a decreased production and/or increased destruction (hemolysis) of red blood cells (RBCs). These also include genetic defects that result in the production of abnormal hemoglobins with a concomitant impaired ability to maintain oxygen concentration. Some such disorders involve the failure to produce normal β -globin in sufficient amounts, while others involve the failure to produce normal β-globin entirely. These disorders associated with the β-globin protein are referred to generally as β-hemoglobinopathies. For example, β-thalassemias result from a partial or complete defect in the expression of the β -globin gene, leading to deficient or absent HbA. Sickle cell anemia results from a point mutation in the β -globin structural gene, leading to the production of an abnormal (sickle) hemoglobin (HbS). HbS is prone to polymerization, particularly under deoxygenated conditions. HbS RBCs are more fragile than normal RBCs and undergo hemolysis more readily, leading eventually to anemia.

In an embodiment, a genetic defect in alpha globin or beta globin is corrected, e.g., by homologous recombination, using the Cas9 molecules and gRNA molecules, e.g., CRISPR systems, described herein.

In an embodiment, a gene encoding a wild type (e.g., non-mutated) copy of alpha globin or beta globin is inserted into the genome of the cell, e.g., at a safe harbor site, e.g., at an AAVS1 safe harbor site, by homologous recombination, using a CRISPR system and methods described herein.

In an embodiment, a hemoglobinopathies-associated gene is targeted, using the Cas9 molecule and gRNA molecule described herein. Exemplary targets include, e.g., genes associated with control of the gamma-globin genes. In an embodiment, the target is BCL11A. In an embodiment, the target is a BCL11a enhancer. In an embodiment, the target is a HPFH region.

Fetal hemoglobin (also hemoglobin F or HbF or α2γ2) is a tetramer of two adult alpha- globin polypeptides and two fetal beta-like gamma-globin polypeptides. HbF is the main oxygen transport protein in the human fetus during the last seven months of development in the uterus and in the newborn until roughly 6 months old. Functionally, fetal hemoglobin differs most from adult hemoglobin in that it is able to bind oxygen with greater affinity than the adult form, giving the developing fetus better access to oxygen from the mother's bloodstream.

In newborns, fetal hemoglobin is nearly completely replaced by adult hemoglobin by approximately 6 months postnatally. In adults, fetal hemoglobin production can be reactivated pharmacologically, which is useful in the treatment of diseases such as hemoglobinopathies. For example, in certain patients with hemoglobinopathies, higher levels of gamma-globin expression can partially compensate for defective or impaired beta-globin gene production, which can ameliorate the clinical severity in these diseases. Increased HbF levels or F-cell (HbF containing erythrocyte) numbers can ameliorate the disease severity of hemoglobinopathies, e.g., beta- thalassemia major and sickle cell anemia.

Increased HbF levels or F-cell can be associated reduced BCL11A expression in cells. The BCL11A gene encodes a multi-zinc finger transcription factor. In an embodiment, the expression of BCL11A is modulated, e.g., down-regulated. In an embodiment, the BCL11A gene is edited. In an embodiment, the function of BCL11a, e.g., in an enrythroid lineage cell, is impaired or down-regulated. In an embodiment, the cell is a hemopoietic stem cell or progenitor cell.

### Sickle cell diseases

Sickle cell disease is a group of disorders that affects hemoglobin. People with this disorder have atypical hemoglobin molecules (hemoglobin S), which can distort red blood cells into a sickle, or crescent, shape. Characteristic features of this disorder include a low number of red blood cells (anemia), repeated infections, and periodic episodes of pain.

Mutations in the HBB gene cause sickle cell disease. T e HBB gene provides instructions for making beta-globin. Various versions of beta-globin result from different mutations in the HBB gene. One particular HBB gene mutation produces an abnormal version of beta-globin known as hemoglobin S (HbS). Other mutations in the HBB gene lead to additional abnormal versions of beta-globin such as hemoglobin C (HbC) and hemoglobin E (HbE). HBB gene mutations can also result in an unusually low level of beta-globin, i.e., beta thalassemia.

In people with sickle cell disease, at least one of the beta-globin subunits in hemoglobin is replaced with hemoglobin S. In sickle cell anemia, which is a common form of sickle cell disease, hemoglobin S replaces both beta-globin subunits in hemoglobin. In other types of sickle cell disease, just one beta-globin subunit in hemoglobin is replaced with hemoglobin S. The other beta-globin subunit is replaced with a different abnormal variant, such as hemoglobin C. For example, people with sickle-hemoglobin C (HbSC) disease have hemoglobin molecules with hemoglobin S and hemoglobin C instead of beta-globin. If mutations that produce hemoglobin S and beta thalassemia occur together, individuals have hemoglobin S-beta thalassemia (HbSBetaThal) disease.

### Beta thalassemia

Beta thalassemia is a blood disorder that reduces the production of hemoglobin. In people with beta thalassemia, low levels of hemoglobin lead to a lack of oxygen in many parts of the body. Affected individuals also have a shortage of red blood cells (anemia), which can cause pale skin, weakness, fatigue, and more serious complications. People with beta thalassemia are at an increased risk of developing abnormal blood clots.

Beta thalassemia is classified into two types depending on the severity of symptoms: thalassemia major (also known as Cooley's anemia) and thalassemia intermedia. Of the two types, thalassemia major is more severe.

Mutations in the HBB gene cause beta thalassemia. The HBB gene provides instructions for making beta-globin. Some mutations in the HBB gene prevent the production of any beta- globin. The absence of beta-globin is referred to as beta-zero (B°) thalassemia. Other HBB gene mutations allow some beta-globin to be produced but in reduced amounts, i.e., beta-plus (B⁺) thalassemia. People with both types have been diagnosed with thalassemia major and thalassemia intermedia.

In an embodiment, a Cas9 molecule/gRNA molecule complex targeting a first gene is used to treat a disorder characterized by second gene, e.g., a mutation in a second gene. By way of example, targeting of the first gene, e.g., by editing or payload delivery, can compensate for, or inhibit further damage from, the affect of a second gene, e.g., a mutant second gene. In an embodiment the allele(s) of the first gene carried by the subject is not causative of the disorder.

In one aspect, the invention relates to the treatment of a mammal, e.g., a human, in need of increased fetal hemoglobin (HbF).

In one aspect, the invention relates to the treatment of a mammal, e.g., a human, that has been diagnosed with, or is at risk of developing, a hemoglobinopathy.

In one aspect, the hemoglobinopathy is a β -hemoglobinopathy. In one aspect, the hemoglobinopathy is sickle cell disease. In one aspect, the hemoglobinopathy is beta thalassemia.

### Methods of Treatment of Hemoglobinopathies

In another aspect the invention provides methdos of treatment. In aspects, the gRNA molecules, CRISPR systems and/or cells of the invention are used to treat a patient in need thereof. In aspects, the patient is a mammal, e.g., a human. In aspects, the patient has a hemoglobinopathy. In embodiments, the patient has sickle cell disease. In embodiments, the patient has beta thalassemia.

In one aspect, the method of treatment comprises administering to a mammal, e.g., a human, one or more gRNA molecules, e.g., one or more gRNA molecules comprising a targeting domain described in Table 1, 2, 3, 4, 5, 6, 7, 8, or 9, and one or more cas9 molecules described herein.

In one aspect, the method of treatment comprises administering to a mammal a cell population, wherein the cell population is a cell population from a mammal, e.g., a human, that has been administered one or more gRNA molecules, e.g., one or more gRNA molecules comprising a targeting domain described in Table 1, 2, 3, 4, 5, 6, 7, 8, or 9, and one or more cas9 molecules described herein, e.g., a CRISPR system as described herein. In one embodiment, the administration of the one or more gRNA molecules or CRISPR systems to the cell is accomplished in vivo. In one embodiment the administration of the one or more gRNA molecules or CRISPR systems to the cell is accomplished ex vivo.

In one aspect, the method of treatment comprises administering to the mammal, e.g., the human, an effective amount of a cell population comprising cells which comprise or at one time comprised one or more gRNA molecules, e.g., one or more gRNA molecules comprising a targeting domain described in Table 1, 2, 3, 4, 5, 6, 7, 8, or 9, and one or more cas9 molecules described herein, or the progeny of said cells. In one embodiment, the cells are allogeneic to the mammal. In one embodiment, the cells are autologous to the mammal. In one embodiment the cells are harvested from the mammal, manipulated ex vivo, and returned to the mammal.

In aspects, the cells comprising or which at one time comprised one or more gRNA molecules, e.g., one or more gRNA molecules comprising a targeting domain described in Table 1, 2, 3, 4, 5, 6, 7, 8, or 9, and one or more cas9 molecules described herein, or the progeny of said cells, comprise stem cells or progenitor cells. In one aspect, the stem cells are hematopoietic stem cells. In one aspect, the progenitor cells are hematopoietic progenitor cells. In one aspect, the cells comprise both hematopoietic stem cells and hematopoietic progenitor cells, e.g., are HSPCs. In one aspect, the cells comprise, e.g., consist of, CD34+ cells. In one aspect the cells are substantially free of CD34- cells. In one aspect, the cells comprise, e.g., consist of, CD34+/CD90+ stem cells. In one aspect, the cells comprise, e.g., consist of, CD34+/CD90- cells. In an aspect, the cells are a population comprising one or more of the cell types described above or described herein.

In one embodiment, the disclosure provides a method for treating a hemoglobinopathy, e.g., sickle cell disease or beta-thalassemia, or a method for increasing fetal hemoglobin expression in a mammal, e.g., a human, in need thereof, the method comprising:
a) providing, e.g., harvesting or isolating, a population of HSPCs (e.g., CD34+ cells) from a mammal;
b) providing said cells ex vivo, e.g., in a cell culture medium, optionally in the presence of an effective amount of a composition comprising at least one stem cell expander, whereby said population of HSPCs (e.g., CD34+ cells) expands to a greater degree than an untreated population;
c) contacting the population of HSPCs (e.g., CD34+ cells) with an effective amount of: a composition comprising at least one gRNA molecule comprising a targeting domain described herein, e.g., a targeting domain described in Tables 1, 2, 3, 4, 5, 6, 7, 8, or 9, or a nucleic acid encoding said gRNA molecule, and at least one cas9 molecule, e.g., described herein, or a nucleic acid encoding said cas9 molecule, e.g., one or more RNPs as described herein, e.g., with a CRISPR system described herein;
d) causing at least one modification in at least a portion of the cells of the population (e.g., at least a portion of the HSPCs, e.g., CD34+ cells, of the population), whereby, e.g., when said HSPCs are differentiated into cells of an erythroid lineage, e.g., red blood cells, fetal hemoglobin expression is increased, e.g., relative to cells not contacted according to step c); and
f) returning a population of cells comprising said modified HSPCs (e.g., CD34+ cells) to the mammal.

In an aspect, the HSPCs are allogeneic to the mammal to which they are returned. In an aspect, the HSPCs are autologous to the mammal to which they are returned. In aspects, the HSPCs are isolated from bone marrow. In aspects, the HSPCs are isolated from peripheral blood, e.g., mobilized peripheral blood. In aspects, the moblized peripheral blood is isolated from a subject who has been administered a G-CSF. In aspects, the moblized peripheral blood is isolated from a subject who has been administered a moblization agent other than G-CSF, for example, Plerixafor^{®} (AMD3100). In aspects, the HSPCs are isolated from umbilical cord blood.

In further embodiments of the method, the method furhter comprises, after providing a population of HSPCs (e.g., CD34+ cells), e.g., from a source described above, the step of enriching the population of cells for HSPCs (e.g., CD34+ cells). In embodiments of the method, after said enriching, the population of cells, e.g., HSPCs, is substantially free of CD34- cells.

In embodiments, the population of cells which is returned to the mammal includes at least 70% viable cells. In embodiments, the population of cells which is returned to the mammal includes at least 75% viable cells. In embodiments, the population of cells which is returned to the mammal includes at least 80% viable cells. In embodiments, the population of cells which is returned to the mammal includes at least 85% viable cells. In embodiments, the population of cells which is returned to the mammal includes at least 90% viable cells. In embodiments, the population of cells which is returned to the mammal includes at least 95% viable cells. In embodiments, the population of cells which is returned to the mammal includes at least 99% viable cells. Viability can be determined by staining a representative portion of the population of cells for a cell viability marker, e.g., as known in the art.

In another embodiment, the disclosure provides a method for treating a hemoglobinopathy, e.g., sickle cell disease or beta-thalassemia, or a method for increasing fetal hemoglobin expression in a mammal, e.g., a human, in need thereof, the method comprising the steps of:
a) providing, e.g., harvesting or isolating, a population of HSPCs (e.g., CD34+ cells) of a mammal, e.g., from the bone marrow of a mammal;
b) isolating the CD34+ cells from the population of cells of step a);
c) providing said CD34+ cells ex vivo, and culturing said cells, e.g., in a cell culture medium, in the presence of an effective amount of a composition comprising at least one stem cell expander, e.g., Compound 4, e.g., Compound 4 at a concentration of about 0.5 to about 0.75 micromolar, whereby said population of CD34+ cells expands to a greater degree than an untreated population;
d) introducing into the cells of the population CD34+ cells an effective amount of: a composition comprising a Cas9 molecule, e.g., as described herein, and a gRNA molecule, e.g., as described herein, e.g., optionally where the Cas9 molecule and the gRNA molecule are in the form of an RNP, e.g., as dsecribed herein, and optionally where said introduction is by electroporation, e.g., as desecribed herein, of said RNP into said cells;
e) causing at least one genetic modification in at least a portion of the cells of the population (e.g., at least a portion of the HSPCs, e.g., CD34+ cells, of the population), whereby an indel, e.g., as described herein, is created at or near the genomic site complementary to the targeting domain of the gRNA introduced in step d);
f) optionally, additionallly culturing said cells after said introducing, e.g., in a cell culture medium, in the presence of an effective amount of a composition comprising at least one stem cell expander, e.g., Compound 4, e.g., Compound 4 at a concentration of about 0.5 to about 0.75 micromolar, such that the cells expand at least 2-fold, e.g., at least 4-fold, e.g., at least 5-fold;
g) cryopreserving said cells; and
h) returning the cells to the mammal, wherein,
   the cells returned to the mammal comprise cells that 1) maintain the abilitiy to differentiate into cells of the erythroid lineage, e.g., red blood cells; 2) when differentiated into red blood cells, produce an increased level of fetal hemoglobin, e.g., relative to cells unmodified by the gRNA of step e), e.g., produce at least 6 picograms fetal hemoglobin per cell.

In an aspect, the HSPCs are allogeneic to the mammal to which they are returned. In an aspect, the HSPCs are autologous to the mammal to which they are returned. In aspects, the HSPCs are isolated from bone marrow. In aspects, the HSPCs are isolated from peripheral blood, e.g., mobilized peripheral blood. In aspects, the moblized peripheral blood is isolated from a subject who has been administered a G-CSF. In aspects, the moblized peripheral blood is isolated from a subject who has been administered a moblization agent other than G-CSF, for example, Plerixafor^{®} (AMD3100). In aspects, the HSPCs are isolated from umbilical cord blood.

In embodiments of the method above, the recited step b) results in a population of cells which is substantially free of CD34- cells.

In further embodiments of the method, the method furhter comprises, after providing a population of HSPCs (e.g., CD34+ cells), e.g., from a source described above, the population of cells is enriched for HSPCs (e.g., CD34+ cells).

In a further embodiments of these methods, the population of modified HSPCs (e.g., CD34+ stem cells) having the ability to differentiate increased fetal hemoglobin expression is cryopreserved and stored prior to being reintroduced into the mammal. In embodiments, the cryopreserved population of HSPCs having the ability to differentiate into cells of the erythroid lineage, e.g., red blood cells, and/or when differentiated into cells of the erythroid lineage, e.g., red blood cells, produce an increased level of fetal hemoglobin is thawed and then reintroduced into the mammal. In a further embodiment of these methods, the method comprises chemotherapy and/or radiation therapy to remove or reduce the endogenous hematopoietic progenitor or stem cells in the mammal. In a further embodiment of these methods, the method does not comprise a step of chemotherapy and/or radiation therapy to remove or reduce the endogenous hematopoietic progenitor or stem cells in the mammal. In a further embodiment of these methods, the method comprises a chemotherapy and/or radiation therapy to reduce partially (e.g., partial lymphodepletion) the endogenous hematopoietic progenitor or stem cells in the mammal. In embodiments the patient is treated with a fully lymphodepleting dose of busulfan prior to reintroduction of the modifiedHSPCs to the mammal. In embodiments, the patient is treated with a partially lymphodepleting dose of busulfan prior to reintroduction of the modified HSPCs to the mammal.

In embodiments, the cells are contacted with RNP comprising a Cas9 molecule, e.g., as described herein, complexed with a gRNA to the +58 BCL11a enhancer region. In embodiments, the gRNA comprises the targeting domain of CR000312. In embodiments, the gRNA comprises the targeting domain of CR000311. In embodiments, the gRNA comprises the targeting domain of CR001128. In embodiments, the gRNA comprises the targeting domain of CR001125. In embodiments, the gRNA comprises the targeting domain of CR001126. In embodiments, the gRNA comprises the targeting domain of CR001127. In embodiments the gRNA is a dual guide RNA comprising a crRNA comprising, e.g., consisting of, [SEQ ID NO: 248]-[SEQ ID NO: 6607], and a tracr comprising, e.g., consisting of, [SEQ ID NO: 7812], optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 248]-[SEQ ID NO: 6601, optionally with 1, 2, 3, 4, 5, 6, or 7 additional 3' uracil nucleotides, e.g., [SEQ ID NO: 248]-[SEQ ID NO: 7811]. In embodiments the gRNA is a dual guide RNA comprising a crRNA comprising, e.g., consisting of, [SEQ ID NO: 247]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 247]-[SEQ ID NO: 6601, optionally with 1, 2, 3, 4, 5, 6, or 7 additional 3' uracil nucleotides, e.g., [SEQ ID NO: 247]-[SEQ ID NO: 7811]. In embodiments the gRNA is a dual guide RNA comprising a crRNA comprising, e.g., consisting of, [SEQ ID NO: 338]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 338]-[SEQ ID NO: 6601], optionally with 1, 2, 3, 4, 5, 6, or 7 additional 3' uracil nucleotides, e.g., [SEQ ID NO: 338]-[SEQ ID NO: 7811]. In embodiments the gRNA is a dual guide RNA comprising a crRNA comprising, e.g., consisting of, [SEQ ID NO: 335]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 335]-[SEQ ID NO: 6601], optionally with 1, 2, 3, 4, 5, 6, or 7 additional 3' uracil nucleotides, e.g., [SEQ ID NO: 335]-[SEQ ID NO: 7811]. In embodiments the gRNA is a dual guide RNA comprising a crRNA comprising, e.g., consisting of, [SEQ ID NO: 336]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 336]-[SEQ ID NO: 6601], optionally with 1, 2, 3, 4, 5, 6, or 7 additional 3' uracil nucleotides, e.g., [SEQ ID NO: 336]-[SEQ ID NO: 7811]. In embodiments the gRNA is a dual guide RNA comprising a crRNA comprising, e.g., consisting of, [SEQ ID NO: 337]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 337]-[SEQ ID NO: 6601], optionally with 1, 2, 3, 4, 5, 6, or 7 additional 3' uracil nucleotides, e.g., [SEQ ID NO: 337]-[SEQ ID NO: 7811]. In any of the aforementioned embodiments, any or all of the RNA componments of the gRNA may be modified at one or more nucleotides, e.g., as described herein. In embodiments, the gRNA molecule is SEQ ID NO: 342. In embodiments, the gRNA molecule is SEQ ID NO: 343. In embodiments, the gRNA molecule is SEQ ID NO: 1762. In embodiments the gRNA molecule is a dgRNA molecule that consists of SEQ ID NO: 344 and SEQ ID NO: 6660. In embodiments the gRNA molecule is a dgRNA molecule that consists of SEQ ID NO: 344 and SEQ ID NO: 346. In embodiments the gRNA molecule is a dgRNA molecule that consists of SEQ ID NO: 345 and SEQ ID NO: 6660. In embodiments the gRNA molecule is a dgRNA molecule that consists of SEQ ID NO: 345 and SEQ ID NO: 346. In embodiments, the gRNA molecule is SEQ ID NO: 347. In embodiments, the gRNA molecule is SEQ ID NO: 348. In embodiments, the gRNA molecule is SEQ ID NO: 1763. In embodiments the gRNA molecule is a dgRNA molecule that consists of SEQ ID NO: 349 and SEQ ID NO: 6660. In embodiments the gRNA molecule is a dgRNA molecule that consists of SEQ ID NO: 349 and SEQ ID NO: 346. In embodiments the gRNA molecule is a dgRNA molecule that consists of SEQ ID NO: 350 and SEQ ID NO: 6660. In embodiments the gRNA molecule is a dgRNA molecule that consists of SEQ ID NO: 350 and SEQ ID NO: 346. In embodiments, the gRNA molecule is SEQ ID NO: 351. In embodiments, the gRNA molecule is SEQ ID NO: 352. In embodiments, the gRNA molecule is SEQ ID NO: 1764. In embodiments the gRNA molecule is a dgRNA molecule that consists of SEQ ID NO: 353 and SEQ ID NO: 6660. In embodiments the gRNA molecule is a dgRNA molecule that consists of SEQ ID NO: 353 and SEQ ID NO: 346. In embodiments the gRNA molecule is a dgRNA molecule that consists of SEQ ID NO: 354 and SEQ ID NO: 6660. In embodiments the gRNA molecule is a dgRNA molecule that consists of SEQ ID NO: 354 and SEQ ID NO: 346. In embodiments, the gRNA molecule is SEQ ID NO: 355. In embodiments, the gRNA molecule is SEQ ID NO: 356. In embodiments, the gRNA molecule is SEQ ID NO: 1765. In embodiments the gRNA molecule is a dgRNA molecule that consists of SEQ ID NO: 357 and SEQ ID NO: 6660. In embodiments the gRNA molecule is a dgRNA molecule that consists of SEQ ID NO: 357 and SEQ ID NO: 346. In embodiments the gRNA molecule is a dgRNA molecule that consists of SEQ ID NO: 358 and SEQ ID NO: 6660. In embodiments the gRNA molecule is a dgRNA molecule that consists of SEQ ID NO: 358 and SEQ ID NO: 346. In embodiments, the gRNA molecule is SEQ ID NO: 359. In embodiments, the gRNA molecule is SEQ ID NO: 360. In embodiments, the gRNA molecule is SEQ ID NO: 1766. In embodiments the gRNA molecule is a dgRNA molecule that consists of SEQ ID NO: 361 and SEQ ID NO: 6660. In embodiments the gRNA molecule is a dgRNA molecule that consists of SEQ ID NO: 361 and SEQ ID NO: 346. In embodiments the gRNA molecule is a dgRNA molecule that consists of SEQ ID NO: 362 and SEQ ID NO: 6660. In embodiments the gRNA molecule is a dgRNA molecule that consists of SEQ ID NO: 362 and SEQ ID NO: 346. In embodiments, the gRNA molecule is SEQ ID NO: 363. In embodiments, the gRNA molecule is SEQ ID NO: 364. In embodiments, the gRNA molecule is SEQ ID NO: 1767. In embodiments the gRNA molecule is a dgRNA molecule that consists of SEQ ID NO: 365 and SEQ ID NO: 6660. In embodiments the gRNA molecule is a dgRNA molecule that consists of SEQ ID NO: 365 and SEQ ID NO: 346. In embodiments the gRNA molecule is a dgRNA molecule that consists of SEQ ID NO: 366 and SEQ ID NO: 6660. In embodiments the gRNA molecule is a dgRNA molecule that consists of SEQ ID NO: 366 and SEQ ID NO: 346. In embodiments, the gRNA molecule is SEQ ID NO: 367. In embodiments, the gRNA molecule is SEQ ID NO: 368. In embodiments, the gRNA molecule is SEQ ID NO: 1768. In embodiments the gRNA molecule is a dgRNA molecule that consists of SEQ ID NO: 369 and SEQ ID NO: 6660. In embodiments the gRNA molecule is a dgRNA molecule that consists of SEQ ID NO: 369 and SEQ ID NO: 346. In embodiments the gRNA molecule is a dgRNA molecule that consists of SEQ ID NO: 370 and SEQ ID NO: 6660. In embodiments the gRNA molecule is a dgRNA molecule that consists of SEQ ID NO: 370 and SEQ ID NO: 346. In embodiments, the gRNA molecule is SEQ ID NO: 371. In embodiments, the gRNA molecule is SEQ ID NO: 372. In embodiments, the gRNA molecule is SEQ ID NO: 1769. In embodiments the gRNA molecule is a dgRNA molecule that consists of SEQ ID NO: 373 and SEQ ID NO: 6660. In embodiments the gRNA molecule is a dgRNA molecule that consists of SEQ ID NO: 373 and SEQ ID NO: 346. In embodiments the gRNA molecule is a dgRNA molecule that consists of SEQ ID NO: 374 and SEQ ID NO: 6660. In embodiments the gRNA molecule is a dgRNA molecule that consists of SEQ ID NO: 374 and SEQ ID NO: 346.

In embodiments, the cells are contacted with RNP comprising a Cas9 molecule, e.g., as described herein, complexed with a gRNA to the HPFH region. In embodiments, the gRNA comprises the targeting domain of CR001030. In embodiments, the gRNA comprises the targeting domain of CR001028. In embodiments, the gRNA comprises the targeting domain of CR001221. In embodiments, the gRNA comprises the targeting domain of CR001137. In embodiments, the gRNA comprises the targeting domain of CR003035. In embodiments, the gRNA comprises the targeting domain of CR003085. In embodiments the gRNA is a dual guide RNA comprising a crRNA comprising, e.g., consisting of, [SEQ ID NO: 98]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 98]-[SEQ ID NO: 6601], optionally with 1, 2, 3, 4, 5, 6, or 7 additional 3' uracil nucleotides, e.g., [SEQ ID NO: 98]-[SEQ ID NO: 7811]. In embodiments the gRNA is a dual guide RNA comprising a crRNA comprising, e.g., consisting of, [SEQ ID NO: 100]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 100]-[SEQ ID NO: 6601], optionally with 1, 2, 3, 4, 5, 6, or 7 additional 3' uracil nucleotides, e.g., [SEQ ID NO: 100]-[SEQ ID NO: 7811]. In embodiments the gRNA is a dual guide RNA comprising a crRNA comprising, e.g., consisting of, [SEQ ID NO: 1589]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 1589]-[SEQ ID NO: 6601], optionally with 1, 2, 3, 4, 5, 6, or 7 additional 3' uracil nucleotides, e.g., [SEQ ID NO: 1589]-[SEQ ID NO: 7811]. In embodiments the gRNA is a dual guide RNA comprising a crRNA comprising, e.g., consisting of, [SEQ ID NO: 1505]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 1505]-[SEQ ID NO: 6601], optionally with 1, 2, 3, 4, 5, 6, or 7 additional 3' uracil nucleotides, e.g., [SEQ ID NO: 1505]-[SEQ ID NO: 7811]. In embodiments the gRNA is a dual guide RNA comprising a crRNA comprising, e.g., consisting of, [SEQ ID NO: 1700]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 1700]-[SEQ ID NO: 6601], optionally with 1, 2, 3, 4, 5, 6, or 7 additional 3' uracil nucleotides, e.g., [SEQ ID NO: 1700]-[SEQ ID NO: 7811]. In embodiments the gRNA is a dual guide RNA comprising a crRNA comprising, e.g., consisting of, [SEQ ID NO: 1750]-[SEQ ID NO: 6607], and a tracr comprising, e.g., consisting of, SEQ ID NO: 7812, optionally with 1, 2, 3, 4, 5, 6, or 7 additional 3' uracil nucleotides, e.g., SEQ ID NO: 6660. In embodiments, the gRNA is a sgRNA comprising, e.g., consisting of, [SEQ ID NO: 1750]-[SEQ ID NO: 6601], optionally with 1, 2, 3, 4, 5, 6, or 7 additional 3' uracil nucleotides, e.g., [SEQ ID NO: 1750]-[SEQ ID NO: 7811]. In embodiments, the gRNA molecule is SEQ ID NO: 375. In embodiments, the gRNA molecule is SEQ ID NO: 376. In embodiments, the gRNA molecule is SEQ ID NO: 1770. In embodiments the gRNA molecule is a dgRNA molecule that consists of SEQ ID NO: 377 and SEQ ID NO: 6660. In embodiments the gRNA molecule is a dgRNA molecule that consists of SEQ ID NO: 377 and SEQ ID NO: 346. In embodiments the gRNA molecule is a dgRNA molecule that consists of SEQ ID NO: 378 and SEQ ID NO: 6660. In embodiments the gRNA molecule is a dgRNA molecule that consists of SEQ ID NO: 378 and SEQ ID NO: 346. In embodiments, the gRNA molecule is SEQ ID NO: 379. In embodiments, the gRNA molecule is SEQ ID NO: 380. In embodiments, the gRNA molecule is SEQ ID NO: 1771. In embodiments the gRNA molecule is a dgRNA molecule that consists of SEQ ID NO: 381 and SEQ ID NO: 6660. In embodiments the gRNA molecule is a dgRNA molecule that consists of SEQ ID NO: 381 and SEQ ID NO: 346. In embodiments the gRNA molecule is a dgRNA molecule that consists of SEQ ID NO: 382 and SEQ ID NO: 6660. In embodiments the gRNA molecule is a dgRNA molecule that consists of SEQ ID NO: 382 and SEQ ID NO: 346. In embodiments, the gRNA molecule is SEQ ID NO: 383. In embodiments, the gRNA molecule is SEQ ID NO: 384. In embodiments, the gRNA molecule is SEQ ID NO: 1772. In embodiments the gRNA molecule is a dgRNA molecule that consists of SEQ ID NO: 385 and SEQ ID NO: 6660. In embodiments the gRNA molecule is a dgRNA molecule that consists of SEQ ID NO: 385 and SEQ ID NO: 346. In embodiments the gRNA molecule is a dgRNA molecule that consists of SEQ ID NO: 386 and SEQ ID NO: 6660. In embodiments the gRNA molecule is a dgRNA molecule that consists of SEQ ID NO: 386 and SEQ ID NO: 346. In embodiments, the gRNA molecule is SEQ ID NO: 387. In embodiments, the gRNA molecule is SEQ ID NO: 388. In embodiments, the gRNA molecule is SEQ ID NO: 1773. In embodiments the gRNA molecule is a dgRNA molecule that consists of SEQ ID NO: 389 and SEQ ID NO: 6660. In embodiments the gRNA molecule is a dgRNA molecule that consists of SEQ ID NO: 389 and SEQ ID NO: 346. In embodiments the gRNA molecule is a dgRNA molecule that consists of SEQ ID NO: 390 and SEQ ID NO: 6660. In embodiments the gRNA molecule is a dgRNA molecule that consists of SEQ ID NO: 390 and SEQ ID NO: 346. In embodiments, the gRNA molecule is SEQ ID NO: 391. In embodiments, the gRNA molecule is SEQ ID NO: 392. In embodiments, the gRNA molecule is SEQ ID NO: 1774. In embodiments the gRNA molecule is a dgRNA molecule that consists of SEQ ID NO: 393 and SEQ ID NO: 6660. In embodiments the gRNA molecule is a dgRNA molecule that consists of SEQ ID NO: 393 and SEQ ID NO: 346. In embodiments the gRNA molecule is a dgRNA molecule that consists of SEQ ID NO: 394 and SEQ ID NO: 6660. In embodiments the gRNA molecule is a dgRNA molecule that consists of SEQ ID NO: 394 and SEQ ID NO: 346. In embodiments, the gRNA molecule is SEQ ID NO: 395. In embodiments, the gRNA molecule is SEQ ID NO: 396. In embodiments, the gRNA molecule is SEQ ID NO: 1775. In embodiments the gRNA molecule is a dgRNA molecule that consists of SEQ ID NO: 397 and SEQ ID NO: 6660. In embodiments the gRNA molecule is a dgRNA molecule that consists of SEQ ID NO: 397 and SEQ ID NO: 346. In embodiments the gRNA molecule is a dgRNA molecule that consists of SEQ ID NO: 398 and SEQ ID NO: 6660. In embodiments the gRNA molecule is a dgRNA molecule that consists of SEQ ID NO: 398 and SEQ ID NO: 346.

In embodiments, the stem cell expander is Compound 1. In embodiments, the stem cell expander is Compound 2. In embodiments, the stem cell expander is Compound 3. In embodiments, the stem cell expander is Compound 4. In embodiments, the stem cell expander is Compound 4 and is present at a concentration of 2-0.1 micromolar, e.g., 1-0.25 micromolar, e.g., 0.75-0.5 micromolar. In embodiments, the stem cell expander is a molecule described in WO2010/059401 (e.g., the molecule described in Example 1 of WO2010/059401).

In embodiments, the cells, e.g., HSPCs, e.g., as described herein, are cultured ex vivo for a period of about 1 hour to about 15 days, e.g., a period of about 12 hours to about 12 days, e.g., a period of about 12 hours to 4 days, e.g., a period of about 1 day to about 4 days, e.g., a period of about 1 day to about 2 days, e.g., a period of about 1 day or a period of about 2 days, prior to the step of contacting the cells with a CRISPR system, e.g., described herein. In embodiments, said culturing prior to said contacting step is in a composition (e.g., a cell culture medium) comprising a stem cell expander, e.g., described herein, e.g., compound 4, e.g., compound 4 at a concentration of about 0.25 uM to about 1 uM, e.g., compound 4 at a concentration of about 0.75-0.5 micromolar. In embodiments, the cells are cultured ex vivo for a period of no more than about about 1 day, e.g., no more than about 18, 17, 16, 15, 14, 13, 12, 11, 10, 9, 8, 7, 6, 5, 4, 3, 2, or 1 hour(s) after the step of contacting the cells with a CRISPR system, e.g., described herein, e.g., in a cell culture medium which comprises a stem cell expander, e.g., described herein, e.g., compound 4, e.g., compound 4 at a concentration of about 0.25 uM to about 1 uM, e.g., compound 4 at a concentration of about 0.75-0.5 micromolar. In other embodiments, the cells are cultured ex vivo for a period of about 1 hour to about 15 days, e.g., a period of about 12 hours to about 10 days, e.g., a period of about 1 day to about 10 days, e.g., a period of about 1 day to about 5 days, e.g., a period of about 1 day to about 4 days, e.g., a period of about 2 days to about 4 days, e.g., a period of about 2 days, about 3 days or about 4 days, after the step of contacting the cells with a CRISPR system, e.g., described herein, in a cell culture medium, e.g., which comprises a stem cell expander, e.g., described herein, e.g., compound 4, e.g., compound 4 at a concentration of about 0.25 uM to about 1 uM, e.g., compound 4 at a concentration of about 0.75-0.5 micromolar. In embodiments, the cells are cultured ex vivo (e.g., cultured prior to said contacting step and/or cultured after said contacting step) for a period of about 1 hour to about 20 days, e.g., a period of about 6-12 days, e.g., a period of about 6, about 7, about 8, about 9, about 10, about 11, or about 12 days.

In embodiments, the population of cells comprising the modified HSPCs returned to the mammal comprises at least about 1 million cells (e.g., at least about 1 million CD34+ cells) per kg. In embodiments, the population of cells comprising the modified HSPCs returned to the mammal comprises at least about 2 million cells (e.g., at least about 2 million CD34+ cells) per kg. In embodiments, the population of cells comprising the modified HSPCs returned to the mammal comprises at least about 3 million cells (e.g., at least about 3 million CD34+ cells) per kg. In embodiments, the population of cells comprising the modified HSPCs returned to the mammal comprises at least about 4 million cells (e.g., at least about 4 million CD34+ cells) per kg. In embodiments, the population of cells comprising the modified HSPCs returned to the mammal comprises at least about 5 million cells (e.g., at least about 5 million CD34+ cells) per kg. In embodiments, the population of cells comprising the modified HSPCs returned to the mammal comprises at least about 6 million cells (e.g., at least about 6 million CD34+ cells) per kg. In embodiments, the population of cells comprising the modified HSPCs returned to the mammal comprises at least 1 million cells (e.g., at least 1 million CD34+ cells) per kg. In embodiments, the population of cells comprising the modified HSPCs returned to the mammal comprises at least 2 million cells (e.g., at least 2 million CD34+ cells) per kg. In embodiments, the population of cells comprising the modified HSPCs returned to the mammal comprises at least 3 million cells (e.g., at least 3 million CD34+ cells) per kg. In embodiments, the population of cells comprising the modified HSPCs returned to the mammal comprises at least 4 million cells (e.g., at least 4 million CD34+ cells) per kg. In embodiments, the population of cells comprising the modified HSPCs returned to the mammal comprises at least 5 million cells (e.g., at least 5 million CD34+ cells) per kg. In embodiments, the population of cells comprising the modified HSPCs returned to the mammal comprises at least 6 million cells (e.g., at least 6 million CD34+ cells) per kg. In embodiments, the population of cells comprising the modified HSPCs returned to the mammal comprises about 1 million cells (e.g., about 1 million CD34+ cells) per kg. In embodiments, the population of cells comprising the modified HSPCs returned to the mammal comprises about 2 million cells (e.g., about 2 million CD34+ cells) per kg. In embodiments, the population of cells comprising the modified HSPCs returned to the mammal comprises about 3 million cells (e.g., about 3 million CD34+ cells) per kg. In embodiments, the population of cells comprising the modified HSPCs returned to the mammal comprises about 4 million cells (e.g., about 4 million CD34+ cells) per kg. In embodiments, the population of cells comprising the modified HSPCs returned to the mammal comprises about 5 million cells (e.g., about 5 million CD34+ cells) per kg. In embodiments, the population of cells comprising the modified HSPCs returned to the mammal comprises about 6 million cells (e.g., about 6 million CD34+ cells) per kg. In embodiments, the population of cells comprising the modified HSPCs returned to the mammal comprises about 2 x 10⁶ cells per kg body weight of the patient. In embodiments, the population of cells comprising the modified HSPCs returned to the mammal comprises at least 2 x 10⁶ cells per kg body weight of the patient.

In embodiments, any of the methods described above results in the patient having at least 80% of its circulating CD34+ cells comprising an indel at or near the genomic site complementary to the targeting domain of the gRNA molecule used in the method, e.g., as measured at least 15 days, e.g., at least 20, at least 30, at least 40 at least 50 or at least 60 days after reintroduction of the cells into the mammal.

In embodiments, any of the methods described above results in the patient having at least 20% of its bone marrow CD34+ cells comprising an indel at or near the genomic site complementary to the targeting domain of the gRNA molecule used in the method, e.g., as measured at least 15 days, e.g., at least 20, at least 30, at least 40 at least 50 or at least 60 days after reintroduction of the cells into the mammal.

In embodiments, the HSPCs that are reintroduced into the mammal are able to differentiate in vivo into cells of the erythroid lineage, e.g., red blood cells, and said differentiated cells exhibit increased fetal hemoglobin levels, e.g., produce at least 6 picograms fetal hemoglobin per cell, e.g., at least 7 picograms fetal hemoglobin per cell, at least 8 picograms fetal hemoglobin per cell, at least 9 picograms fetal hemoglobin per cell, at least 10 picograms fetal hemoglobin per cell, e.g., between about 9 and about 10 picograms fetal hemoglobin per cell, e.g., such that the hemoglobinopathy is treated the mammal.

It will be understood that when a cell is characterized as having increased fetal hemoglobin, that includes embodiments in which a progeny, e.g., a differentiated progeny, of that cell exhibits increased fetal hemoglobin. For example, in the methods described herein, the altered or modified CD34+ cell (or cell population) may not express increased fetal hemoglobin, but when differentiated into cells of erythroid lineage, e.g., red blood cells, the cells express increased fetal hemoglobin, e.g., increased fetal hemoglobin relative to an unmodified or unaltered cell under similar conditions.

### XII. Culture Methods and Methods of Manufacturing Cells

The disclosure provides methods of culturing cells, e.g., HSPCs, e.g., hematopoietic stem cells, e.g., CD34+ cells modified, or to be modified, with the gRNA molecules described herein.

### DNA Repair Pathway Inhibitors

Without being bound by theory, it is believed that the pattern of indels produced by a given gRNA molecule at a particular target sequence is a product of each of the active DNA repair mechamisms within the cell (e.g., non-homologous end joining, microhomology-mediated end joining, etc.). Wihtout being bound by theory, it is believed that a particularlyfavorable indel may be selected for or enriched for by contacting the cells to be edited with an inhibitor of a DNA repair pathway that does not produce the desired indel. Thus, the gRNA molecules, CRISPR systems, methods and other aspects of the invention may be perfomred in combination with such inhibitors. Examples of such inhibitors include those described in, e.g., WO2014/130955, the contents of which are hereby incorproated by reference in their entirety. In embodiment, the inhibitor is a DNAPKc inhibitor, e.g., NU7441.

### Stem Cell Expanders

In one aspect the invention relates to culturing the cells, e.g., HSPCs, e.g., CD34+ cells modified, or to be modified, with the gRNA molecules described herein, with one or more agents that result in an increased expansion rate, increased expansion level, or increased engraftment relative to cells not treated with the agent. Such agents are referred to herein as stem cell expanders.

In an aspect, the one or more agents that result in an increased expansion rate or increased expansion level, relative to cells not treated with the agent, e.g., the stem cell expander, comprises an agent that is an inhibitor of the aryl hydrocarbon receptor (AHR) pathway. In aspects, the stem cell expander is a compound disclosed in WO2013/110198 or a compound disclosed in WO2010/059401, the contents of which are incorporated by reference in their entirety.

In one aspect, the one or more agents that result in an increased expansion rate or increased expansion level, relative to cells not treated with the agent, is a pyrimido[4,5-b]indole derivative, e.g., as disclosed in WO2013/110198, the contents of which are hereby incorporated by reference in their entirety. In one embodiment the agent is compound 1 ((1r,4r)-N¹-(2-benzyl-7-(2-methyl-2H-tetrazol-5 -yl)-9H-pyrimido [4,5-b]indol-4-yl)cyclohexane-1,4-diamine):

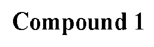

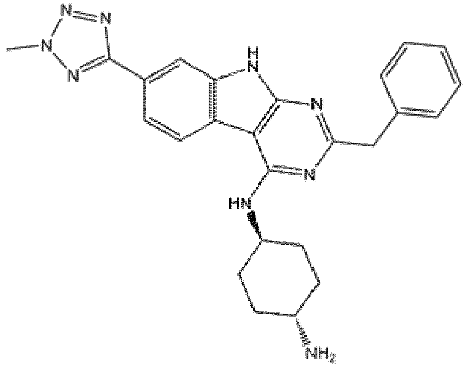

In another aspect, the agent is Compound 2 (methyl 4-(3-piperidin-1-ylpropylamino)-9H-pyrimido[4,5-b]indole-7-carboxylate):

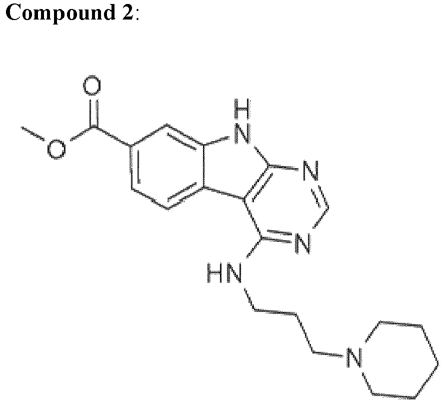

In another aspect, the one or more agents that result in an increased expansion rate or increased expansion level, relative to cells not treated with the agent, is an agent disclosed in WO2010/059401, the contents of which are hereby incorporated by reference in their entirety.

In one embodiment, the stem cell expander is 4-(2-(2-(benzo[b]thiophen-3-yl)-9-isopropyl-9H-purin-6-ylamino)ethyl)phenol, i.e., is the compound from example 1 of WO2010/059401, having the following structure:

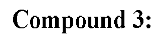

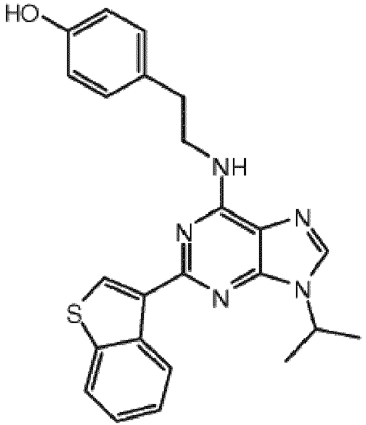

In another aspect, the stem cell expander is compound 4 ((S)-2-(6-(2-(lH-indol-3-yl)ethylamino)-2-(5-fluoropyridin-3-yl)-9H-purin-9-yl)propan-1-ol, i.e., is the compound 157S according to WO2010/059401), having the following structure:

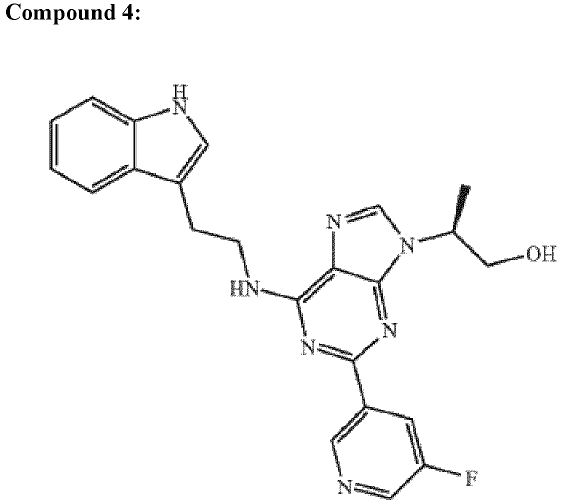

In embodiments the population of HSPCs is contacted with the stem cell expander, e.g., compound 1, compound 2, compound 3, compound 4, or combinations thereof (e.g., a combination of compound 1 and compound 4) before introduction of the CRISPR system (e.g., gRNA molecule and/or Cas9 molecule of the invention) to said HSPCs. In embodiements, the population of HSPCs is contacted with the stem cell expander, e.g., compound 1, compound 2, compound 3, compound 4, or combinations thereof (e.g., a combination of compound 1 and compound 4), after introduction of the CRISPR system (e.g., gRNA molecule and/or Cas9 molecule of the invention) to said HSPCs. In embodiments, the population of HSPCs is contacted with the stem cell expander, e.g., compound 1, compound 2, compound 3, compound 4, or combinations thereof (e.g., a combination of compound 1 and compound 4), both before and after introduction of the CRISPR system (e.g., gRNA molecule and/or Cas9 molecule of the invention) to said HSPCs.

In embodiments, the stem cell expander is present in an effective amount to increase the expansion level of the HSPCs, relative to HSPCs in the same media but for the absence of the stem cell expander. In embodimetns, the stem cell expander is present at a concentration ranging from about 0.01 to about 10 uM, e.g., from about 0.1 uM to about 1 uM. In embodiments, the stem cell expander is present in the cell culture medium at a concentration of about 1 uM, about 950 nM, about 900 nM, about 850 nM, about 800 nM, about 750 nM, about 700nM, about 650 nM, about 600 nM, about 550 nM, about 500 nM, about 450 nM, about 400 nM, about 350 nM, about 300 nM, about 250 nM, about 200 nM, about 150 nM, about 100 nM, about 50 nM, about 25 nM, or about 10 nM. In embodiments, the stem cell expander is present at a concentration ranging from about 500 nM to about 750 nM.

In embodiments, the stem cell expander is compound 4, which is present in the cell culture medium at a concentration ranging from about 0.01 to about 10 micromolar (uM). In embodiments, the stem cell expander is compound 4, which is present in the cell culture medium at a concentration ranging from about 0.1 to about 1 micromolar (uM). In embodiments, the stem cell expander is compound 4, which is present in the cell culture medium at a concentration of about 0.75 micromolar (uM). In embodiments, the stem cell expander is compound 4, which is present in the cell culture medium at a concentration of about 0. 5 micromolar (uM). In embodiments of any of the foregoing, the cell culture medium additionally comprises compound 1.

In embodiments, the stem cell expander is a mixture of compound 1 and compound 4.

In embodiments, the cells of the invention are contacted with one or more stem cell expander molecules for a sufficient time and in a sufficient amount to cause a 2 to 10,000-fold expansion of CD34+ cells, e.g., a 2-1000-fold expansion of CD34+ cells, e.g., a 2-100-fold expansion of CD34+ cells, e.g., a 20-200-fold expansion of CD34+ cells. As described herein, the contacting with the one or more stem cell expanders may be before the cells are contacted with a CRISPR system, e.g., as described herein, after the cells are contacted with a CRISPR system, e.g., as described herein, or a combination thereof. In an embodiment, the cells are contacted with one or more stem cell expander molecules, e.g., Compound 4, for a sufficient time and in a sufficient amount to cause at least a 2-fold expansion of CD34+ cells, e.g., CD34+ cells comprising an indel at or near the target site having complementarity to the targeting domain of the gRNA of the CRISPR/Cas9 system introduced into said cell. In an embodiment, the cells are contacted with one or more stem cell expander molecules, e.g., Compound 4, for a sufficient time and in a sufficient amount to cause at least a 4-fold expansion of CD34+ cells, e.g., CD34+ cells comprising an indel at or near the target site having complementarity to the targeting domain of the gRNA of the CRISPR/Cas9 system introduced into said cell. In an embodiment, the cells are contacted with one or more stem cell expander molecules, e.g., Compound 4, for a sufficient time and in a sufficient amount to cause at least a 5-fold expansion of CD34+ cells, e.g., CD34+ cells comprising an indel at or near the target site having complementarity to the targeting domain of the gRNA of the CRISPR/Cas9 system introduced into said cell. In an embodiment, the cells are contacted with one or more stem cell expander molecules for a sufficient time and in a sufficient amount to cause at least a 10-fold expansion of CD34+ cells. In an embodiment, the cells are contacted with one or more stem cell expander molecules for a sufficient time and in a sufficient amount to cause at least a 20-fold expansion of CD34+ cells. In an embodiment, the cells are contacted with one or more stem cell expander molecules for a sufficient time and in a sufficient amount to cause at least a 30-fold expansion of CD34+ cells. In an embodiment, the cells are contacted with one or more stem cell expander molecules for a sufficient time and in a sufficient amount to cause at least a 40-fold expansion of CD34+ cells. In an embodiment, the cells are contacted with one or more stem cell expander molecules for a sufficient time and in a sufficient amount to cause at least a 50-fold expansion of CD34+ cells. In an embodiment, the cells are contacted with one or more stem cell expander molecules for a sufficient time and in a sufficient amount to cause at least a 60-fold expansion of CD34+ cells. In embodiments, the cells are contacted with the one or more stem cell expanders for a period of about 1-60 days, e.g., about 1-50 days, e.g., about 1-40 days, e.g., about 1-30 days, e.g., 1-20 days, e.g., about 1-10 days, e.g., about 7 days, e.g., about 1-5 days, e.g., about 2-5 days, e.g., about 2-4 days, e.g., about 2 days or, e.g., about 4 days.

In embodiments, the cells, e.g., HSPCs, e.g., as described herein, are cultured ex vivo for a period of about 1 hour to about 10 days, e.g., a period of about 12 hours to about 5 days, e.g., a period of about 12 hours to 4 days, e.g., a period of about 1 day to about 4 days, e.g., a period of about 1 day to about 2 days, e.g., a period of about 1 day or a period of about 2 days, prior to the step of contacting the cells with a CRISPR system, e.g., described herein. In embodiments, said culturing prior to said contacting step is in a composition (e.g., a cell culture medium) comprising a stem cell expander, e.g., described herein, e.g., compound 4, e.g., compound 4 at a concentration of about 0.25 uM to about 1 uM, e.g., compound 4 at a concentration of about 0.75-0.5 micromolar. In embodiments, the cells are cultured ex vivo for a period of no more than about about 1 day, e.g., no more than about 18, 17, 16, 15, 14, 13, 12, 11, 10, 9, 8, 7, 6, 5, 4, 3, 2, or 1 hour(s) after the step of contacting the cells with a CRISPR system, e.g., described herein, e.g., in a cell culture medium which comprises a stem cell expander, e.g., described herein, e.g., compound 4, e.g., compound 4 at a concentration of about 0.25 uM to about 1 uM, e.g., compound 4 at a concentration of about 0.75-0.5 micromolar. In other embodiments, the cells are cultured ex vivo for a period of about 1 hour to about 14 days, e.g., a period of about 12 hours to about 10 days, e.g., a period of about 1 day to about 10 days, e.g., a period of about 1 day to about 5 days, e.g., a period of about 1 day to about 4 days, e.g., a period of about 2 days to about 4 days, e.g., a period of about 2 days, about 3 days or about 4 days, after the step of contacting the cells with a CRISPR system, e.g., described herein, in a cell culture medium, e.g., which comprises a stem cell expander, e.g., described herein, e.g., compound 4, e.g., compound 4 at a concentration of about 0.25 uM to about 1 uM, e.g., compound 4 at a concentration of about 0.75-0.5 micromolar.

In embodiments, the cell culture medium is a chemically defined medium. In embodiments, the cell culture medium may additionally contain, for example, StemSpan SFEM (StemCell Technologies; Cat no. 09650). In embodiments, the cell culture medium may alternatively or additionally contain, for example, HSC Brew, GMP (Miltenyi). In embodiments, the media may be supplemented with thrombopoietin (TPO), human Flt3 ligand (Flt-3L), human stem cell factor (SCF), human interleukin-6, L-glutamine, and/or penicillin/streptomycin. In embodiments, the media is supplemented with thrombopoietin (TPO), human Flt3 ligand (Flt-3L), human stem cell factor (SCF), human interleukin-6, and L-glutamine. In other embodiments, the media is supplemented with thrombopoietin (TPO), human Flt3 ligand (Flt-3L), human stem cell factor (SCF), and human interleukin-6. In other embodiments the media is supplemented with thrombopoietin (TPO), human Flt3 ligand (Flt-3L), and human stem cell factor (SCF), but not human interleukin-6. When present in the medium, the thrombopoietin (TPO), human Flt3 ligand (Flt-3L), human stem cell factor (SCF), human interleukin-6, and/or L-glutamine are each present in a concentration ranging from about 1 ng/mL to about 1000 ng/mL, e.g., a concentration ranging from about 10 ng/mL to about 500 ng/mL, e.g., a concentration ranging from about 10 ng/mL to about 100 ng/mL, e.g., a concentration ranging from about 25 ng/mL to about 75 ng/mL, e.g., a concentration of about 50 ng/mL. In embodiments, each of the supplemented components is at the same concentration. In other embodiments, each of the supplemented components is at a different concentration. In an embodiment, the medium comprises StemSpan SFEM (StemCell Technologies; Cat no. 09650), 50 ng/mL of thrombopoietin (Tpo), 50 ng/mL of human Flt3 ligand (Flt-3L), 50ng/mL of human stem cell factor (SCF), and 50ng/mL of human interleukin-6 (IL-6). In an embodiment, the medium comprises StemSpan SFEM (StemCell Technologies; Cat no. 09650), 50 ng/mL of thrombopoietin (Tpo), 50 ng/mL of human Flt3 ligand (Flt-3L), and 50ng/mL of human stem cell factor (SCF), and does not comprise IL-6. In embodiments, the media further comprises a stem cell expander, e.g., compound 4, e.g., compound 4 at a concentration of 0.75 µM. In embodiments, the media further comprises a stem cell expander, e.g., compound 4, e.g., compound 4 at a concentration of 0.5 µM. In embodiments, the media further comprises 1% L-glutamine and 2% penicillin/streptomycin. In embodiments, the cell culture medium is serum free.

### XII. Combination Therapy

The present disclosure contemplates the use of the gRNA molecules described herein, or cells (e.g., hematopoietic stem cells, e.g., CD34+ cells) modified with the gRNA molecules described herein, in combination with one or more other therapeutic modalities and/or agents agents. Thus, in addition to the use of the gRNA molecules or cells modified with the gRNA molecules described herein, one may also administer to the subject one or more "standard" therapies for treating hemoglobinopathies.

The one or more additional therapies for treating hemoglobinopathies may include, for example, additional stem cell transplantation, e.g., hematopoietic stem cell transplantation. The stem cell transplantation may be allogeneic or autologous.

The one or more additional therapies for treating hemoglobinopathies may include, for example, blood transfusion and/or iorn chealation (e.g., removal) therapy. Known iron chealation agents include, for example, deferoxamine and deferasirox.

The one or more additional therapies for treating hemoglobinopathies may include, for example, folic acid supplements, or hydroxyurea (e.g., 5-hydroxyurea). The one or more additional therapies for treating hemoglobinopathies may be hydroxyurea. In embodiments, the hydroxyurea may be administered at a dose of, for example, 10-35mg/kg per day, e.g., 10-20 mg/kg per day. In embodiments, the hydroxyurea is adminstered at a dose of 10 mg/kg per day. In embodiments, the hydroxyurea is adminstered at a dose of 10 mg/kg per day. In embodiments, the hydroxyurea is adminstered at a dose of 20 mg/kg per day. In embodiments, the hydroxyurea is administered before and/or after the cell (or population of cells), e.g., CD34+ cell (or population of cells) of the invention, e.g., as described herein.

The one or more additional therapeutic agents may include, for example, an anti-p-selectin antibody, e.g., SelG1 (Selexys). P-selectin antibodies are described in, for example, PCT publication WO1993/021956, PCT publication WO1995/034324, PCT publication WO2005/100402, PCT publication WO2008/069999, US patent applicatation publication US2011/0293617, US Patent No. 5800815, US Patent No. 6667036, US Patent No. 8945565, US Patent No. 8377440 and US Patent No. 9068001, the contents of each of which are incorporated herein in their entirety.

The one or more additional agents may include, for example, a small molecule which upregulates fetal hemoglobin. Examples of such molecules include TN1 (e.g., as described in Nam, T. et al., ChemMedChem 2011, 6, 777 - 780, DOI: 10.1002/cmdc.201000505, herein incorporated by reference).

The one or more additional therapies may also include irradiation or other bone marrow ablation therapeis known in the art. An example of such a therapy is busulfan. Such additional therapy may be performed prior to introduction of the cells of the invention into the subject. In an embodiment the methods of treatment described herein (e.g., the methods of treatment that include administration of cells (e.g., HSPCs) modified by the methods described herein (e.g., modified with a CRISPR system described herein, e.g., to increase HbF production)), the method does not include the step of bone marrow ablation. In embodiments, the methods include a partial bone marrow ablation step.

The therapies described herein (e.g., comprising administering a population of HSPCs, e.g., HSPCs modified using a CRISPR system described herein) may also be combined with an additional therapeutic agent. In an embodiment, the additional therapeutic agent is an HDAC inhibitor, e.g., panobinostat. In an embodiment, the additional therapeutic is a compound described in PCT Publication No. WO2014/150256, e.g., a compound described in Table 1 of WO2014/150256, e.g., GBT440. Other examples of HDAC inhibitors include, for example, suberoylanilide hydroxamic acid (SAHA). The one or more additional agents may include, for example, a DNA methylation inhibitor. Such agents have been shown to increase the HbF induction in cells having reduced BCL11a activity (e.g., Jian Xu et al, Science 334, 993 (2011); DOI: 0.1126/science.1211053, herein incorporated by reference). Other HDAC inhibitors include any HDAC inhibitor known in the art, for example, trichostatin A, HC toxin, DACI-2, FK228, DACI-14, depudicin, DACI-16, tubacin, NK57, MAZ1536, NK125, Scriptaid, Pyroxamide, MS-275, ITF-2357, MCG-D0103, CRA-024781, CI-994, and LBH589 (see, e.g., Bradner JE, et al., PNAS, 2010 (vol. 107:28), 12617-12622, herein incorporated by reference in its entirety).

The gRNA molecules described herein, or cells (e.g., hematopoietic stem cells, e.g., CD34+ cells) modified with the gRNA molecules described herein, and the co-therapeutic agent or co-therapy can be administered in the same formulation or separately. In the case of separate administration, the gRNA molecules described herein, or cells modified with the gRNA molecules described herein, can be administered before, after or concurrently with the co-therapeutic or co-therapy. One agent may precede or follow administration of the other agent by intervals ranging from minutes to weeks. In embodiments where two or more different kinds of therapeutic agents are applied separately to a subject, one would generally ensure that a significant period of time did not expire between the time of each delivery, such that these different kinds of agents would still be able to exert an advantageously combined effect on the target tissues or cells.

### XIII. Modified Nucleosides, Nucleotides, and Nucleic Acids

Modified nucleosides and modified nucleotides can be present in nucleic acids, e.g., particularly gRNA, but also other forms of RNA, e.g., mRNA, RNAi, or siRNA. As described herein "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, e.g., replacement, of one or both of the non-linking phosphate oxygens and/or of one or more of the linking phosphate oxygens in the phosphodiester backbone linkage;
(ii) alteration, e.g., replacement, of a constituent of the ribose sugar, e.g., of the 2' hydroxyl on the ribose sugar;
(iii) wholesale replacement of the phosphate moiety with "dephospho" linkers;
(iv) modification or replacement of a naturally occurring nucleobase, including with a non-canonical nucleobase;
(v) replacement or modification of the ribose-phosphate backbone;
(vi) modification of the 3' end or 5' end of the oligonucleotide, e.g., removal, modification or replacement of a terminal phosphate group or conjugation of a moiety, cap or linker; and
(vii) modification or replacement of the sugar.

The modifications listed above can be combined to provide modified nucleosides and nucleotides that can have two, three, four, or more modifications. For example, a modified nucleoside or nucleotide can have a modified sugar and a modified nucleobase. In an embodiment, every base of a gRNA is modified, e.g., all bases have a modified phosphate group, e.g., all are phosphorothioate groups. In an embodiment, all, or substantially all, of the phosphate groups of a unimolecular or modular gRNA molecule are replaced with phosphorothioate groups. In embodiments, one or more of the five 3'-terminal bases and/or one or more of the fige 5'-terminal bases of the gRNA are modified with a phosphorothioate group.

In an embodiment, modified nucleotides, e.g., nucleotides having modifications as described herein, can be incorporated into a nucleic acid, e.g., a "modified nucleic acid." In some embodiments, the modified nucleic acids comprise one, two, three or more modified nucleotides. In some embodiments, at least 5% (e.g., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, e.g., cellular nucleases. For example, nucleases can hydrolyze nucleic acid phosphodiester bonds. Accordingly, in one aspect the modified nucleic acids described herein can contain one or more modified nucleosides or nucleotides, e.g., to introduce stability toward nucleases.

In some embodiments, the modified nucleosides, modified nucleotides, and modified nucleic acids described herein can exhibit a reduced innate immune response when introduced into a population of cells, both in vivo and ex vivo. The term "innate immune response" includes a cellular response to exogenous nucleic acids, including single stranded nucleic acids, generally of viral or bacterial origin, which involves the induction of cytokine expression and release, particularly the interferons, and cell death. In some embodiments, the modified nucleosides, modified nucleotides, and modified nucleic acids described herein can disrupt binding of a major groove interacting partner with the nucleic acid. In some embodiments, the modified nucleosides, modified nucleotides, and modified nucleic acids described herein can exhibit a reduced innate immune response when introduced into a population of cells, both in vivo and ex vivo, and also disrupt binding of a major groove interacting partner with the nucleic acid.

### Definitions of Chemical Groups

As used herein, "alkyl" is meant to refer to a saturated hydrocarbon group which is straight-chained or branched. Example alkyl groups include methyl (Me), ethyl (Et), propyl (e.g. , n-propyl and isopropyl), butyl (e.g. , n-butyl, isobutyl, t-butyl), pentyl (e.g. , n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (e.g. , having 2, 3 or 4 fused rings) aromatic hydrocarbons such as, for example, phenyl, naphthyl, anthracenyl,
phenanthrenyl, indanyl, indenyl, and the like. In some embodiments, aryl groups have from 6 to about 20 carbon atoms.

As used herein, "alkenyl" refers to an aliphatic group containing at least one double bond. As used herein, "alkynyl" refers to a straight or branched hydrocarbon chain containing 2- 12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic non- aromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### Phosphate Backbone Modifications

### The Phosphate Group

In some embodiments, the phosphate group of a modified nucleotide can be modified by replacing one or more of the oxygens with a different substituent. Further, the modified nucleotide, e.g., modified nucleotide present in a modified nucleic acid, can include the wholesale replacement of an unmodified phosphate moiety with a modified phosphate as described herein. In some embodiments, the modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. In some embodiments, one of the non-bridging phosphate oxygen atoms in the phosphate backbone moiety can be replaced by any of the following groups: sulfur (S), selenium (Se), BR₃ (wherein R can be, e.g., hydrogen, alkyl, or aryl), C (e.g., an alkyl group, an aryl group, and the like), H, NR₂ (wherein R can be, e.g., hydrogen, alkyl, or aryl), or OR (wherein R can be, e.g., alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In some embodiments, modifications to one or both non-bridging oxygens can also include the replacement of the non-bridging oxygens with a group independently selected from S, Se, B, C, H, N, and OR (R can be, e.g., alkyl or aryl).

The phosphate linker can also be modified by replacement of a bridging oxygen, (i.e., the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In some embodiments, the charge phosphate group can be replaced by a neutral moiety.

Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In some embodiments, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. In some embodiments, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, e.g., alkyl, cycloalkyl, aryl, aralkyl, heteroaryl or a sugar); polyethyleneglycols (PEG), 0(CH₂CH₂0)ₙCH2CH₂OR wherein R can be, e.g., H or optionally substituted alkyl, and n can be an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In some embodiments, the "oxy"-2' hydroxyl group modification can include "locked" nucleic acids (LNA) in which the 2' hydroxyl can be connected, e.g., by a Ci₋₆ alkylene or Cj-6 heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy, 0(CH₂)ₙ-amino, (wherein amino can be, e.g., NH₂; alkylamino, dialkylamino, heterocyclyl, arylamino, diarylamino, heteroarylamino, or diheteroarylamino, ethylenediamine, or polyamino). In some embodiments, the "oxy"-2' hydroxyl group modification can include the methoxyethyl group (MOE), (OCH₂CH₂OCH₃, e.g., a PEG derivative).

"Deoxy" modifications can include hydrogen (i.e. deoxyribose sugars, e.g., at the overhang portions of partially ds RNA); halo (e.g., bromo, chloro, fluoro, or iodo); amino (wherein amino can be, e.g., NH₂; alkylamino, dialkylamino, heterocyclyl, arylamino, diarylamino, heteroarylamino, diheteroarylamino, or amino acid); NH(CH₂CH₂NH)ₙCH2CH₂- amino (wherein amino can be, e.g., as described herein), -NHC(0)R (wherein R can be, e.g., alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino as described herein.

The sugar group can also contain one or more carbons that possess the opposite stereochemical configuration than that of the corresponding carbon in ribose. Thus, a modified nucleic acid can include nucleotides containing e.g., arabinose, as the sugar. The nucleotide "monomer" can have an alpha linkage at the Γ position on the sugar, e.g., alpha-nucleosides. The modified nucleic acids can also include "abasic" sugars, which lack a nucleobase at C-. These abasic sugars can also be further modified at one or more of the constituent sugar atoms. The modified nucleic acids can also include one or more sugars that are in the L form, e.g. L- nucleosides.

Generally, RNA includes the sugar group ribose, which is a 5-membered ring having an oxygen. Exemplary modified nucleosides and modified nucleotides can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In some embodiments, the modified nucleotides can include multicyclic forms (e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replaced with a-L-threofuranosyl-(3'-→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In some embodiments, the nucleobase can include, for example, naturally-occurring and synthetic derivatives of a base.

### Uracil

In some embodiments, the modified nucleobase is a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio- uridine (s2U), 4-thio-uridine (s4U), 4-thio-pseudouridine, 2-thio-pseudouridine, 5-hydroxy- u,ridine (ho⁵U), 5-aminoallyl-uridine, 5-halo-uridine (e.g., 5-iodo-uridine or 5-bromo-uridine), 3- methyl-uridine (m³U), 5-methoxy-uridine (mo⁵U), uridine 5-oxyacetic acid (cmo⁵U), uridine 5- oxyacetic acid methyl ester (mcmo^U), 5-carboxymethyl-uridine (cm^{s}U), 1 -carboxymethyl- pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5- methoxycarbonylmethyl-2-thio-uridine (mcm⁵s2U), 5-aminomethyl-2-thio-uridine (nm⁵s2U), 5- methylaminomethyl-uridine (mnm⁵U), 5-methylaminomethyl-2-thio-uridine (mnm⁵s2U), 5- methylaminomethyl-2-seleno-uridine (mnm⁵se²U), 5-carbamoylmethyl-uridine (ncm⁵U), 5- carboxymethylaminomethyl-uridine (cmnm⁵U), 5-carboxymethylaminomethyl-2-thio-uridine (cmnm \s2U), 5-propynyl-uridine, 1 -propynyl-pseudouridine, 5-taurinomethyl-uridine (xcm⁵U), 1 -taurinomethyl-pseudouridine, 5-taurinomethyl-2-thio-uridine(Trn⁵s2U), 1-taurinomethyl-4- thio-pseudouridine, 5-methyl-uridine (m⁵U, i.e., having the nucleobase deoxythymine), 1- methyl-pseudouridine (ιτι'ψ). 5-methyl-2-thio-uridine (m⁵s2U), 1-methyl-4-thio-pseudouridine (m' s \|/), 4-thio- 1-methyl-pseudouridine, 3-methyl-pseudouridine (m'V), 2-thio-1 -methyl- pseudouridine, 1 - methyl- 1 -deaza-pseudouridine, 2-thio- 1 -methyl- 1 -deaza-pseudouridine, dihydroundine (D), dihydropseudoundine, 5,6-dihydrouridine, 5-methyl-dihydrouridine (m⁵D), 2- thio-dihydrouridine, 2-thio-dihydropseudouridine, 2-methoxy-uridine, 2-methoxy-4-thio- uridine, 4-methoxy-pseudouridine, 4-methoxy-2-thio-pseudouridine, N 1 -methyl-pseudouridine, 3- (3-amino-3-carboxypropyl)uridine (acp³U), 1-methyl-3-(3-amino-3- carboxypropy pseudouridine 5-(isopentenylaminomethyl)uridine (inm⁵U), 5- (isopentenylaminomethy])-2-thio-uridine (inm⁵s2U), a-thio-uridine, 2'-0-methyl-uridine (Urn), 5,2'-0-dimethyl-uridine (m⁵Um), 2'-0-methyl-pseudouridine (ψπι), 2-thio-2'-0-methyl-uridine (s2Um), 5-methoxycarbonylmethyl-2'-0-methyl-uridine (mcm ⁵Um), 5-carbamoylmethyl-2'-0-methyl-uridine (ncm ⁵Um), 5-carboxymethylaminomethyl-2'-0-methyl-uridine (cmnm ⁵Um), 3,2'-0-dimethyl-uridine (m³Um), 5-(isopentenylaminomethyl)-2'-0-methyl-uridine (inm ⁵Um), 1 -thio-uridine, deoxythymidine, 2'-F-ara-uridine, 2'-F-uridine, 2'-OH-ara-uridine, 5-(2- carbomethoxyvinyl) uridine, 5-[3-( l-E-propenylamino)uridine, pyrazolo[3,4-d]pyrimidines, xanthine, and hypoxanthine.

### Cytosine

In some embodiments, the modified nucleobase is a modified cytosine. Exemplary nucleobases and nucleosides having a modified cytosine include without limitation 5-aza- cytidine, 6-aza-cytidine, pseudoisocytidine, 3-methyl-cytidine (m³C), N4-acetyl-cytidine (act), 5- formyl-cytidine (f⁵C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo- cytidine, pyrrolo-pseudoisocytidine, 2-thio-cytidine (s2C), 2-thio-5-methyl-cytidine, 4-thio- pseudoisocytidine, 4-thio-1 -methyl-pseudoisocytidine, 4-thio-1 -methyl- 1-deaza- pseudoisocytidine, 1-methyl- 1-deaza-pseudoisocytidine, zebularine, 5-aza-zebularine, 5-methyl- zebularine, 5-aza-2-thio-zebularine, 2-thio-zebularine, 2-methoxy-cytidine, 2-methoxy-5-methyl- cytidine, 4-methoxy-pseudoisocytidine, 4-methoxy- 1 -methyl-pseudoisocytidine, lysidine (k²C), a-thio-cytidine, 2'-0-methyl-cytidine (Cm), 5,2'-0-dimethyl-cytidine (m⁵Cm), N4-acetyl-2'-0- methyl-cytidine (ac⁴Cm), N4,2'-0-dimethyl-cytidine (m⁴Cm), 5-formyl-2'-0-methyl-cytidine (f ⁵Cm), N4,N4,2'-0-trimethyl-cytidine (m⁴₂Cm), 1 -thio-cytidine, 2'-F-ara-cytidine, 2'-F-cytidine, and 2'-OH-ara-cytidine.

### Adenine

In some embodiments, the modified nucleobase is a modified adenine. Exemplary nucleobases and nucleosides having a modified adenine include without limitation 2-amino- purine, 2,6-diaminopurine, 2-amino-6-halo-purine (e.g., 2-amino-6-chloro-purine), 6-halo-purine (e.g., 6-chloi - purine), 2-amino-6-methyl-purine, 8-azido-adenosine, 7-deaza-adenine, 7-deaza- 8-aza-adenine, 7-deaza-2-amino-purine, 7-deaza-8-aza-2-amino-purine, 7-deaza-2,6- diaminopurine, 7-deaza-8-aza-2,6-diaminopurine, 1-methyl-adenosine (m'A), 2-methyl-adenine (m A), N6-methyl-adenosine (m⁶A), 2-methylthio-N6-methyl-adenosine (ms2m⁶A), N6- isopentenyl-adenosine (i⁶A), 2-methylthio-N6-isopentenyl-adenosine (ms²i⁶A), N6-(cis- hydroxyisopentenyl)adenosine (io⁶A), 2-methylthio-N6-(cis-hydroxyisopentenyl)adenosine (ms2io⁶A), N6-glycinylcarbamoyl-adenosine (g⁶A), N6-threonylcarbamoyl-adenosine (t⁶A), N6- methyl-N6-threonylcarbamoyl-adenosine (m⁶t⁶A), 2-methylthio-N6-threonylcarbamoyl- adenosine (ms²g⁶A), N6,N6-dimethyl-adenosine (m⁶₂A), N6-hydroxynorvalylcarbamoyl- adenosine (hn⁶A), 2-methylthio-N6-hydroxynorvalylcarbamoyl-adenosine (ms2hn⁶A), N6- acetyl-adenosine (ac⁶A), 7-methyl-adenine, 2-methylthio-adenine, 2-methoxy-adenine, a-thio- adenosine, 2'-0-methyl-adenosine (Am), N⁶,2'-0-dimethyl-adenosine (m⁵Am), N⁶-Methyl-2'- deoxyadenosine, N6,N6,2'-0-trimethyl-adenosine (m⁶₂Am), 1 ,2'-0-dimethyl-adenosine (m' Am), 2'-0-ribosyladenosine (phosphate) (Ar(p)), 2-amino-N6-methyl-purine, 1 -thio-adenosine, 8- azido-adenosine, 2'-F-ara-adenosine, 2'-F-adenosine, 2'-OH-ara-adenosine, and N6-( 19-amino- pentaoxanonadecyl)-adenosine.

### Guanine

In some embodiments, the modified nucleobase is a modified guanine. Exemplary nucleobases and nucleosides having a modified guanine include without limitation inosine (I), 1 - methyl-inosine (m 'l), wyosine (imG), methylwyosine (mimG), 4-demethyl-wyo "sine (imG- 14), isowyosine (imG2), wybutosine (yW), peroxywybutosine (o₂yW), hydroxywybutosine (OHyW), undemriodified hydroxywybutosine (OHyW^{∗}), 7-deaza-guanosine, queuosine (Q), epoxyqueuosine (oQ), galactosyl-queuosine (galQ), mannosyl-queuosine (manQ), 7-cyano-7- deaza-guanosine (preQ₀), 7-aminomethyI-7-deaza-guanosine (preQi), archaeosine (G⁺), 7-deaza- 8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1 -methyl-guanosine (m'G), N2-methyl-guanosine (m²G), N2,N2-dimethyl-guanosine (m² ₂G), N2,7-dimethyl-guanosine (m²,7G), N2, N2,7-dimethyl-guanosine (m²,2,7G), 8-oxo-guanosine, 7-methyl-8-oxo-guanosine, 1 -meth thio-guanosine, N2-methyl-6-thio-guanosine, N2,N2- dimethyl-6-thio-guanosine, a-thio-guanosine, 2'-0-methyl-guanosine (Gm), N2-methyl-2'-0- methyl-guanosine (m¾m), N2,N2-dimethyl-2'-0-methyl-guanosine (m² ₂Gm), 1 -methyl-2'-0- methyl-guanosine (m'Gm), N2,7-dimethyl-2'-0-methyl-guanosine (m²,7Gm), 2'-0-methyl- inosine (Im), 1 ,2'-0-dimethyl-inosine (m'lm), 0⁶-phenyl-2'-deoxyinosine, 2'-0-ribosylguanosine (phosphate) (Gr(p)), 1 -thio-guanosine, 0⁶-methy]-guanosine, 0⁶-Methyl-2'-deoxyguanosine, 2'- F-ara-guanosine, and 2'-F-guanosine.

### Modified gRNAs

In some embodiments, the modified nucleic acids can be modified gRNAs. In some embodiments, gRNAs can be modified at the 3' end. In this embodiment, the gRNAs can be modified at the 3' terminal U ribose. For example, the two terminal hydroxyl groups of the U ribose can be oxidized to aldehyde groups and a concomitant opening of the ribose ring to afford a modified nucleoside, wherein U can be an unmodified or modified uridine.

In another embodiment, the 3' terminal U can be modified with a 2' 3' cyclic phosphate, wherein U can be an unmodified or modified uridine. In some embodiments, the gRNA molecules may contain 3' nucleotides which can be stabilized against degradation, e.g., by incorporating one or more of the modified nucleotides described herein. In this embodiment, e.g., uridines can be replaced with modified uridines, e.g., 5-(2-amino)propyl uridine, and 5-bromo uridine, or with any of the modified uridines described herein; adenosines and guanosines can be replaced with modified adenosines and guanosines, e.g., with modifications at the 8-position, e.g., 8-bromo guanosine, or with any of the modified adenosines or guanosines described herein. In some embodiments, deaza nucleotides, e.g., 7- deaza-adenosine, can be incoiporated into the gRNA. In some embodiments, O- and N-alkylated nucleotides, e.g., N6-methyl andenosine, can be incorporated into the gRNA. In some embodiments, sugar-modified ribonucleotides can be incorporated, e.g., wherein the 2' OH- group is replaced by a group selected from H, -OR, -R (wherein R can be, e.g., methyl, alkyl, cycloalkyl, aryl, aralkyl, heteroaryl or sugar), halo, -SH, -SR (wherein R can be, e.g., alkyl, cycloalkyl, aryl, aralkyl, heteroaryl or sugar), amino (wherein amino can be, e.g., NH₂; alkylamino, dialkylamino, heterocyclyl, arylamino, diarylamino, heteroarylamino, diheteroarylamino, or amino acid); or cyano (-CN). In some embodiments, the phosphate backbone can be modified as described herein, e.g., with a phosphothioate group. In some embodiments, the nucleotides in the overhang region of the gRNA can each independently be a modified or unmodified nucleotide including, but not limited to 2'-sugar modified, such as, 2-F 2'-0-methyl, thymidine (T), 2'-O-methoxyethyl-5-methyluridine (Teo), 2'-O-methoxyethyladenosine (Aeo), 2'-O-methoxyethyl- 5-methylcytidine (m5Ceo ), and any combinations thereof.

In an embodiment, a one or more or all of the nucleotides in single stranded overhang of an RNA molecule, e.g., a gRNA molecule, are deoxynucleotides.

### Pharmaceutical Compositions

Pharmaceutical compositions of the present invention may comprise a gRNA molecule described herein, e.g., a plurality of gRNA molecules as described herein, or a cell (e.g., a population of cells, e.g., a population of hematopoietic stem cells, e.g., of CD34+ cells) comprising one or more cells modified with one or more gRNA molecules described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one embodiment, the pharmaceutical composition is substantially free of, e.g., there are no detectable levels of a contaminant, e.g., selected from the group consisting of endotoxin, mycoplasma, mouse antibodies, pooled human serum, bovine serum albumin, bovine serum, culture media components, unwanted CRISPR system components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

The administration of the subject compositions may be carried out in any convenient manner, including by aerosol inhalation, injection, ingestion, transfusion, implantation or transplantation. The compositions described herein may be administered to a patient transarterially, subcutaneously, intradermally, intratumorally, intranodally, intramedullary, intramuscularly, by intravenous (i.v.) injection, or intraperitoneally. In one aspect, the compositions of the present invention are administered to a patient by intradermal or subcutaneous injection. In one aspect, the cell compositions of the present invention are administered by i.v. injection.

The dosage of the above treatments to be administered to a patient will vary with the precise nature of the condition being treated and the recipient of the treatment. The scaling of dosages for human administration can be performed according to art-accepted practices.

### Cells

The invention also relates to cells comprising a gRNA molecule of the invention, or nucleic acid encoding said gRNA molecules.

In an aspect the cells are cells made by a process described herein.

In embodiments, the cells are hematopoietic stem cells (e.g., hematopoietic stem and progenitor cells; HSPCs), for example, CD34+ stem cells. In embodiments, the cells are CD34+/CD90+ stem cells. In embodiments, the cells are CD34+/CD90- stem cells. In embodiments, the cells are human hematopoietic stem cells. In embodiments, the cells are autologous. In embodiments, the cells are allogeneic.

In embodiments, the cells are derived from bone marrow, e.g., autologous bone marrow. In embodiments, the cells are derived from peripheral blood, e.g., mobilized peripheral blood, e.g., autologous mobilized peripheral blood. In embodiments employing moblized peripheral blood, the cells are isoalted from patients who have been administered a mobilization agent. In embodiments, the mobilization agent is G-CSF. In embodiments, the mobilization agent is Plerixafor^{®} (AMD3100). In embodiments, the cells are derived from umbilical cord blood, e.g., allogeneic umbilical cord blood.

In embodiments, the cells are mammallian. In embodiments, the cells are human.

In an aspect, the invention provides a cell comprising a modification or alteration, e.g., an indel, at or near (e.g., within 20, 19, 18, 17, 16, 15, 14, 13, 12, 11, 10, 9, 8, 7, 6, 5, 4, 3, 2 or 1 nucleotides of) a nucleic acid sequence having complementarity to a gRNA molecule or gRNA molecules, e.g., as described herein, introduced into said cells, e.g., as part of a CRISPR system as described herein. In embodiments, the cell is a CD34+ cell. In embodiments, the altered or modified cell, e.g., CD34+ cell, maintains the ability to differentiate into cells of multiple lineages, e.g., maintains the ability to differentiate into cells of the erythroid lineage. In embodiments, the altered or modified cell, e.g., CD34+ cell, has undergone or is able to undergo at least 2, at least 3, at least 4, at least 5, at least 6, at least 7, at least 8, at least 9 or at least 10 or more doublings in culture, e.g., in culture comprising a stem cell expander, e.g., Compound 4. In embodiments, the altered or modified cell, e.g., CD34+ cell, has undergone or is able to undergo at least 5, e.g., about 5, doublings in culture, e.g., in culture comprising a stem cell expander molecule, e.g., as described herein, e.g., Compound 4. In embodiments the altered or modified cell, e.g., CD34+ cell, exhibits and/or is able to differentiate into a cell, e.g., into a cell of the erythroid lineage, e.g., into a red blood cell, that exhibits increased fetal hemoglobin level (e.g., expression level and/or protein level), e.g., at least a 20% increase in fetal hemoglobin protein level, relative to a similar unmodified or unaltered cell. In embodiments the altered or modified cell, e.g., CD34+ cell, exhibits and/or is able to differentiate into a cell, e.g., into a cell of the erythroid lineage, e.g., into a red blood cell, that exhibits increased fetal hemoglobin level (e.g., expression level and/or protein level), relative to a similar unmodified or unaltered cell, e.g., produces at least 6 picograms, e.g., at least 7 picograms, at least 8 picograms, at least 9 picograms, or at least 10 picograms of fetal hemoglobin. In embodiments the altered or modified cell, e.g., CD34+ cell, exhibits and/or is able to differentiate into a cell, e.g., into a cell of the erythroid lineage, e.g., into a red blood cell, that exhibits increased fetal hemoglobin level (e.g., expression level and/or protein level), relative to a similar unmodified or unaltered cell, e.g., produces about 6 to about 12, about about 6 to about 7, about 7 to about 8, about 8 to about 9, about 9 to about 10, about 10 to about 11 or about 11 to about 12 picograms of fetal hemoglobin.

In an aspect, the invention provides a population of cells comprising cells having a modification or alteration, e.g., an indel, at or near (e.g., within 20, 19, 18, 17, 16, 15, 14, 13, 12, 11, 10, 9, 8, 7, 6, 5, 4, 3, 2 or 1 nucleotides of) a nucleic acid sequence having complementarity to a gRNA molecule or gRNA molecules, e.g., as described herein, introduced into said cells, e.g., as part of a CRISPR system as described herein. In embodiments, at least 50%, e.g., at least 60%, at least 70%, at least 80% or at least 90% of the cells of the population have the modification or alteration (e.g., have at least one modification or alteration) , e.g., as measured by NGS, e.g., as described herein, e.g., at day two following introduction of the gRNA and/or CRISPR system of the invention. In embodiments, at least 90%, e.g., at least 91%, at least 92%, at least 93%, at least 94%, at least 95%, at least 96%, at least 97%, at least 98% or at least 99% of the cells of the population have the modification or alteration (e.g., have at least one modification or alteration), e.g., as measured by NGS, e.g., as described herein, e.g., at day two following introduction of the gRNA and/or CRISPR system of the invention. In embodiments, the population of cells comprise CD34+ cells, e.g., comprise at least about 50%, at least about 60%, at least about 70%, at least about 80%, at least about 90%, at least about 95% or at least about 98% CD34+ cells. In embodiments, the population of cells comprising the altered or modified cells, e.g., CD34+ cells, maintain the ability to produce, e.g., differentiate into, cells of multiple lineages, e.g., maintains the ability to produce, e.g., differentiate into, cells of the erythroid lineage. In embodiments, the population of cells, e.g., population of CD34+ cells, has undergone or is able to undergo at least 2, at least 3, at least 4, at least 5, at least 6, at least 7, at least 8, at least 9 or at least 10 or more population doublings in culture, e.g., in culture comprising a stem cell expander, e.g., Compound 4. In embodiments, the population of altered or modified cells, e.g., population of CD34+ cells, has undergone or is capable of undergoing at least 5, e.g., about 5, population doublings in culture, e.g., in culture comprising a stem cell expander molecule, e.g., as described herein, e.g., Compound 4. In embodiments the population of cells comprising altered or modified cells, e.g., CD34+ cells, exhibits and/or is able to differentiate into a population of cells, e.g., into a population of cells of the erythroid lineage, e.g., into a population of red blood cells, that exhibits increased fetal hemoglobin level (e.g., expression level and/or protein level), e.g., at least a 20% increase in fetal hemoglobin protein level, relative to a similar unmodified or unaltered cells. In embodiments the population of cells comprising altered or modified cells, e.g., CD34+ cells, exhibits and/or is able to differentiate into a population of cells, e.g., into a population of cells of the erythroid lineage, e.g., into a population of red blood cells, that exhibits increased fetal hemoglobin level (e.g., expression level and/or protein level), relative to a similar unmodified or unaltered cells, e.g., comprises cells that produce at least 6 picograms, e.g., at least 7 picograms, at least 8 picograms, at least 9 picograms, or at least 10 picograms of fetal hemoglobin per cell. In embodiments the population of altered or modified cells, e.g., CD34+ cells, exhibits and/or is able to differentiate into a population of cells, e.g., into a population of cells of the erythroid lineage, e.g., into a population of red blood cells, that exhibits increased fetal hemoglobin level (e.g., expression level and/or protein level), relative to a similar unmodified or unaltered cell, e.g., comprises cells that produce about 6 to about 12, about about 6 to about 7, about 7 to about 8, about 8 to about 9, about 9 to about 10, about 10 to about 11 or about 11 to about 12 picograms of fetal hemoglobin per cell.

In embodiments, the population of cells, e.g., as described herein, comprises at least about 1e3 cells. In embodiments, the population of cells, e.g., as described herein, comprises at least about 1e4 cells. In embodiments, the population of cells, e.g., as described herein, comprises at least about 1e5 cells. In embodiments, the population of cells, e.g., as described herein, comprises at least about 1e6 cells. In embodiments, the population of cells, e.g., as described herein, comprises at least about 1e7 cells. In embodiments, the population of cells, e.g., as described herein, comprises at least about 1e8 cells. In embodiments, the population of cells, e.g., as described herein, comprises at least about 1e9 cells. In embodiments, the population of cells, e.g., as described herein, comprises at least about 1e10 cells. In embodiments, the population of cells, e.g., as described herein, comprises at least about 1e11 cells. In embodiments, the population of cells, e.g., as described herein, comprises at least about 1e12 cells. In embodiments, the population of cells, e.g., as described herein, comprises at least about 1e13 cells per kilogram body weight of the patient to which they are to be administered. In embodiments, the population of cells, e.g., as described herein, comprises at least about 1e6 cells per kilogram body weight of the patient to which they are to be administered. In embodiments, the population of cells, e.g., as described herein, comprises at least about 2e6 cells per kilogram body weight of the patient to which they are to be administered. In embodiments, the population of cells, e.g., as described herein, comprises at least about 3e6 cells per kilogram body weight of the patient to which they are to be administered. In embodiments, the population of cells, e.g., as described herein, comprises at least about 4e6 cells per kilogram body weight of the patient to which they are to be administered. In embodiments, the population of cells, e.g., as described herein, comprises at least about 5e6 cells per kilogram body weight of the patient to which they are to be administered. In embodiments, the population of cells, e.g., as described herein, comprises at least about 6e6 cells per kilogram body weight of the patient to which they are to be administered. In embodiments, the population of cells, e.g., as described herein, comprises at least about 7e6 cells per kilogram body weight of the patient to which they are to be administered. In embodiments, the population of cells, e.g., as described herein, comprises at least about 8e6 cells per kilogram body weight of the patient to which they are to be administered. In embodiments, the population of cells, e.g., as described herein, comprises at least about 9e6 cells per kilogram body weight of the patient to which they are to be administered. In embodiments, the population of cells, e.g., as described herein, comprises at least about 1e7 cells per kilogram body weight of the patient to which they are to be administered. In embodiments, the population of cells, e.g., as described herein, comprises at least about 2e7 cells per kilogram body weight of the patient to which they are to be administered. In embodiments, the population of cells, e.g., as described herein, comprises at least about 3e7 cells per kilogram body weight of the patient to which they are to be administered. In embodiments, the population of cells, e.g., as described herein, comprises at least about 4e7 cells per kilogram body weight of the patient to which they are to be administered. In embodiments, the population of cells, e.g., as described herein, comprises at least about 5e7 cells per kilogram body weight of the patient to which they are to be administered. In embodiments, the population of cells, e.g., as described herein, comprises at least about 6e7 cells per kilogram body weight of the patient to which they are to be administered. In embodiments, the population of cells, e.g., as described herein, comprises at least about 7e7 cells per kilogram body weight of the patient to which they are to be administered. In embodiments, the population of cells, e.g., as described herein, comprises at least about 8e7 cells per kilogram body weight of the patient to which they are to be administered. In embodiments, the population of cells, e.g., as described herein, comprises at least about 9e7 cells per kilogram body weight of the patient to which they are to be administered. In embodiments, the population of cells, e.g., as described herein, comprises at least about 1e8 cells per kilogram body weight of the patient to which they are to be administered. In any of the aforementioned embodiments, the population of cells may comprise at least about 50% (for example, at least about 60%, at least about 70%, at least about 80%, at least about 90%, at least about 95% or at least about 99%) HSPCs, e.g., CD34+ cells. In any of the aforementioned embodiments, the population of cells may comprise about 60% HSPCs, e.g., CD34+ cells. In an embodiment, the population of cells, e.g., as described herein, comprises about 3e7 cells and comprises about 2e7 HSPCs, e.g., CD34+ cells.

In embodiments, the population of cells, e.g., as described herein, comprises at least about 1e6 HSPCs, e.g., CD34+ cells, per kilogram body weight of the patient to which they are to be administered. In embodiments, the population of cells, e.g., as described herein, comprises at least about 1.5e6 HSPCs, e.g., CD34+ cells, per kilogram body weight of the patient to which they are to be administered. In embodiments, the population of cells, e.g., as described herein, comprises at least about 2e6 HSPCs, e.g., CD34+ cells, per kilogram body weight of the patient to which they are to be administered. In embodiments, the population of cells, e.g., as described herein, comprises at least about 3e6 HSPCs, e.g., CD34+ cells, per kilogram body weight of the patient to which they are to be administered. In embodiments, the population of cells, e.g., as described herein, comprises at least about 4e6 HSPCs, e.g., CD34+ cells, per kilogram body weight of the patient to which they are to be administered. In embodiments, the population of cells, e.g., as described herein, comprises at least about 5e6 HSPCs, e.g., CD34+ cells, per kilogram body weight of the patient to which they are to be administered. In embodiments, the population of cells, e.g., as described herein, comprises at least about 6e6 HSPCs, e.g., CD34+ cells, per kilogram body weight of the patient to which they are to be administered. In embodiments, the population of cells, e.g., as described herein, comprises at least about 7e6 HSPCs, e.g., CD34+ cells, per kilogram body weight of the patient to which they are to be administered. In embodiments, the population of cells, e.g., as described herein, comprises at least about 8e6 HSPCs, e.g., CD34+ cells, per kilogram body weight of the patient to which they are to be administered. In embodiments, the population of cells, e.g., as described herein, comprises at least about 9e6 HSPCs, e.g., CD34+ cells, per kilogram body weight of the patient to which they are to be administered. In embodiments, the population of cells, e.g., as described herein, comprises at least about 1e7 HSPCs, e.g., CD34+ cells, per kilogram body weight of the patient to which they are to be administered. In embodiments, the population of cells, e.g., as described herein, comprises at least about 2e7 HSPCs, e.g., CD34+ cells, per kilogram body weight of the patient to which they are to be administered. In embodiments, the population of cells, e.g., as described herein, comprises at least about 3e7 HSPCs, e.g., CD34+ cells, per kilogram body weight of the patient to which they are to be administered. In embodiments, the population of cells, e.g., as described herein, comprises at least about 4e7 HSPCs, e.g., CD34+ cells, per kilogram body weight of the patient to which they are to be administered. In embodiments, the population of cells, e.g., as described herein, comprises at least about 5e7 HSPCs, e.g., CD34+ cells, per kilogram body weight of the patient to which they are to be administered. In embodiments, the population of cells, e.g., as described herein, comprises at least about 6e7 HSPCs, e.g., CD34+ cells, per kilogram body weight of the patient to which they are to be administered. In embodiments, the population of cells, e.g., as described herein, comprises at least about 7e7 HSPCs, e.g., CD34+ cells, per kilogram body weight of the patient to which they are to be administered. In embodiments, the population of cells, e.g., as described herein, comprises at least about 8e7 HSPCs, e.g., CD34+ cells, per kilogram body weight of the patient to which they are to be administered. In embodiments, the population of cells, e.g., as described herein, comprises at least about 9e7 HSPCs, e.g., CD34+ cells, per kilogram body weight of the patient to which they are to be administered. In embodiments, the population of cells, e.g., as described herein, comprises at least about 1e8 HSPCs, e.g., CD34+ cells, per kilogram body weight of the patient to which they are to be administered. In embodiments, the population of cells, e.g., as described herein, comprises at least about 2e8 HSPCs, e.g., CD34+ cells, per kilogram body weight of the patient to which they are to be administered. In embodiments, the population of cells, e.g., as described herein, comprises at least about 3e8 HSPCs, e.g., CD34+ cells, per kilogram body weight of the patient to which they are to be administered. In embodiments, the population of cells, e.g., as described herein, comprises at least about 4e8 HSPCs, e.g., CD34+ cells, per kilogram body weight of the patient to which they are to be administered. In embodiments, the population of cells, e.g., as described herein, comprises at least about 5e8 HSPCs, e.g., CD34+ cells, per kilogram body weight of the patient to which they are to be administered.

In embodiments, the population of cells, e.g., as described herein, comprises about 1e6 HSPCs, e.g., CD34+ cells, per kilogram body weight of the patient to which they are to be administered. In embodiments, the population of cells, e.g., as described herein, comprises about 1.5e6 HSPCs, e.g., CD34+ cells, per kilogram body weight of the patient to which they are to be administered. In embodiments, the population of cells, e.g., as described herein, comprises about 2e6 HSPCs, e.g., CD34+ cells, per kilogram body weight of the patient to which they are to be administered. In embodiments, the population of cells, e.g., as described herein, comprises about 3e6 HSPCs, e.g., CD34+ cells, per kilogram body weight of the patient to which they are to be administered. In embodiments, the population of cells, e.g., as described herein, comprises about 4e6 HSPCs, e.g., CD34+ cells, per kilogram body weight of the patient to which they are to be administered. In embodiments, the population of cells, e.g., as described herein, comprises about 5e6 HSPCs, e.g., CD34+ cells, per kilogram body weight of the patient to which they are to be administered. In embodiments, the population of cells, e.g., as described herein, comprises about 6e6 HSPCs, e.g., CD34+ cells, per kilogram body weight of the patient to which they are to be administered. In embodiments, the population of cells, e.g., as described herein, comprises about 7e6 HSPCs, e.g., CD34+ cells, per kilogram body weight of the patient to which they are to be administered. In embodiments, the population of cells, e.g., as described herein, comprises about 8e6 HSPCs, e.g., CD34+ cells, per kilogram body weight of the patient to which they are to be administered. In embodiments, the population of cells, e.g., as described herein, comprises about 9e6 HSPCs, e.g., CD34+ cells, per kilogram body weight of the patient to which they are to be administered. In embodiments, the population of cells, e.g., as described herein, comprises about 1e7 HSPCs, e.g., CD34+ cells, per kilogram body weight of the patient to which they are to be administered. In embodiments, the population of cells, e.g., as described herein, comprises about 2e7 HSPCs, e.g., CD34+ cells, per kilogram body weight of the patient to which they are to be administered. In embodiments, the population of cells, e.g., as described herein, comprises about 3e7 HSPCs, e.g., CD34+ cells, per kilogram body weight of the patient to which they are to be administered. In embodiments, the population of cells, e.g., as described herein, comprises about 4e7 HSPCs, e.g., CD34+ cells, per kilogram body weight of the patient to which they are to be administered. In embodiments, the population of cells, e.g., as described herein, comprises about 5e7 HSPCs, e.g., CD34+ cells, per kilogram body weight of the patient to which they are to be administered. In embodiments, the population of cells, e.g., as described herein, comprises about 6e7 HSPCs, e.g., CD34+ cells, per kilogram body weight of the patient to which they are to be administered. In embodiments, the population of cells, e.g., as described herein, comprises about 7e7 HSPCs, e.g., CD34+ cells, per kilogram body weight of the patient to which they are to be administered. In embodiments, the population of cells, e.g., as described herein, comprises about 8e7 HSPCs, e.g., CD34+ cells, per kilogram body weight of the patient to which they are to be administered. In embodiments, the population of cells, e.g., as described herein, comprises about 9e7 HSPCs, e.g., CD34+ cells, per kilogram body weight of the patient to which they are to be administered. In embodiments, the population of cells, e.g., as described herein, comprises about 1e8 HSPCs, e.g., CD34+ cells, per kilogram body weight of the patient to which they are to be administered. In embodiments, the population of cells, e.g., as described herein, comprises about 2e8 HSPCs, e.g., CD34+ cells, per kilogram body weight of the patient to which they are to be administered. In embodiments, the population of cells, e.g., as described herein, comprises about 3e8 HSPCs, e.g., CD34+ cells, per kilogram body weight of the patient to which they are to be administered. In embodiments, the population of cells, e.g., as described herein, comprises about 4e8 HSPCs, e.g., CD34+ cells, per kilogram body weight of the patient to which they are to be administered. In embodiments, the population of cells, e.g., as described herein, comprises about 5e8 HSPCs, e.g., CD34+ cells, per kilogram body weight of the patient to which they are to be administered.

In embodiments, the population of cells, e.g., as described herein, comprises from about 2e6 to about 10e6 HSPCs, e.g., CD34+ cells, per kilogram body weight of the patient to which they are to be administered. In embodiments, the population of cells, e.g., as described herein, comprises from 2e6 to 10e6 HSPCs, e.g., CD34+ cells, per kilogram body weight of the patient to which they are to be administered.

The cells of the invention may comprise a gRNA molecule of the present invention, or nucleic acid encoding said gRNA molecule, and a Cas9 molecule of the present invention, or nucleic acid encoding said Cas9 molecule. In an embodiment, the cells of the invention may comprise a ribonuclear protein (RNP) complex which comprises a gRNA molecule of the invention and a Cas9 molecule of the invention.

The cells of the invention are preferrably modified to comprise a gRNA molecule of the invention ex vivo, for example by a method described herein, e.g., by electroporation.

The cells of the invention include cells in which expression of one or more genes has been altered, for example, reduced or inhibited, by introduction of a CRISPR system comprising a gRNA of the invention. For example, the cells of the present invention may have a reduced level of BCL11a expression relative to unmodified cells. As another example, the cells of the present invention may have an increased level of fetal hemoglobin expression relative to unmodified cells. As another example, the cells of the present invention may have reduced level of beta globin exprsesion relative to unmodified cells. Alternatively, or in addition, a cell of the invention may give rise, e.g., differentiate into, another type of cell, e.g., an erythrocyte, that has a reduced level of BCL11a expression relative to cells differentiated from unmodified cells. Alternatively, or in addition, a cell of the invention may give rise, e.g., differentiate into, another type of cell, e.g., an erythrocyte, that has an increased level of fetal hemoglobin expression relative to cells differentiated from unmodified cells. Alternatively, or in addition, a cell of the invention may give rise, e.g., differentiate into, another type of cell, e.g., an erythrocyte, that has a reduced level of beta globin expression relative to cells differentiated from unmodified cells.

The cells of the invention include cells in which expression of one or more genes has been altered, for example, reduced or inhibited, by introduction of a CRISPR system comprising a gRNA of the invention. For example, the cells of the present invention may have a reduced level of hemeoglobin beta, for example a mutated or wild-type hemoglobin beta, expression relative to unmodified cells. In another aspect, the invention provides cells which are derived from, e.g., differentiated from, cells in which a CRISPR system comprising a gRNA of the invention has been introduced. In such aspects, the cells in which the CRISPR system comprising the gRNA of the invention has been introduced may not exhibit the reduced level of hemoglobin beta, for example a mutated or wild-type hemoglobin beta, but the cells derived from, e.g., differentiated from, said cells exhibit the reduced level of hemoglobin beta, for example a mutated or wild-type hemoglobin beta. In embodiments, the derivation, e.g., differentation, is accomplished in vivo (e.g., in a patient). In embodiments the cells in which the CRISPR system comprising the gRNA of the invention has been introduced are CD34+ cells and the cells derived, e.g., differentiated, therefrom are of the erythroid lineage, e.g., red blood cells.

The cells of the invention include cells in which expression of one or more genes has been altered, for example, increased or promoted, by introduction of a CRISPR system comprising a gRNA of the invention. For example, the cells of the present invention may have an increased level of fetal hemoglobin expression relative to unmodified cells. In another aspect, the invention provides cells which are derived from, e.g., differentiated from, cells in which a CRISPR system comprising a gRNA of the invention has been introduced. In such aspects, the cells in which the CRISPR system comprising the gRNA of the invention has been introduced may not exhibit the increased level of fetal hemoglobin but the cells derived from, e.g., differentiated from, said cells exhibit the increased level of fetal hemoglobin. In embodiments, the derivation, e.g., differentation, is accomplished in vivo (e.g., in a patient). In embodiments the cells in which the CRISPR system comprising the gRNA of the invention has been introduced are CD34+ cells and the cells derived, e.g., differentiated, therefrom are of the erythroid lineage, e.g., red blood cells.

In another aspect, the invention relates to cells which include an indel at (e.g., within) or near (e.g., within 20, 19, 18, 17, 16, 15, 14, 13, 12, 11, 10, 9, 8, 7, 6, 5, 4, 3, 2 or 1 nucleotides of) a nucleic acid sequence having complementarity to the gRNA molecule or gRNA molecules introduced into said cells. In embodiments, the indel is a frameshift indel. In embodiments, the indel is an indel listed in Table 15. In embodiments, the indel is an indel listed in Table 26, Table 27 or Table 37. In embodiments the invention pertains to a population of cells, e.g., as described herein, wherein the population of cells comprises cells having an indel listed in Table 15. In embodiments the invention pertains to a population of cells, e.g., as described herein, wherein the population of cells comprises cells having an indel listed in Table 26, Table 27 or Table 37.

In an aspect, the invention relates to a population of cells, e.g., a population of HSPCs, which comprises cells which include an indel, e.g., as described herein, e.g., an indel or pattern of indels described in Figure 25, Table 15, Table 26, Table 27 or Table 37, at or near (e.g., within 20, 19, 18, 17, 16, 15, 14, 13, 12, 11, 10, 9, 8, 7, 6, 5, 4, 3, 2 or 1 nucleotides of) a nucleic acid sequence having complementarity to a gRNA molecule or gRNA molecules, e.g., as described herein, introduced into said cells, e.g., as described herein. In embodiments, the indel is a frameshift indel. In embodiments, 20%-100% of the cells of the population include said indel or indels. In embodiments, 30%-100% of the cells of the population include said indel or indels. In embodiments, 40%-100% of the cells of the population include said indel or indels. In embodiments, 50%-100% of the cells of the population include said indel or indels. In embodiments, 60%-100% of the cells of the population include said indel or indels. In embodiments, 70%-100% of the cells of the population include said indel or indels. In embodiments, 80%-100% of the cells of the population include said indel or indels. In embodiments, 90%-100% of the cells of the population include said indel or indels. In embodiments, the population of cells retains the ability to differentiate into multiple cell types, e.g., maintains the ability to differentiate into cells of erythroid lineage, e.g., red blood cells. In embodiments, the edited and differentiated cells (e.g., red blood cells) maintain the ability to proliferate, e.g., proliferate at least 5-fold, at least 10-fold, at least 15-fold, at least 20-fold, at least 25-fold, at least 30-fold, at least 35-fold, at least 40-fold, at least 45-fold, at least 50-fold or more after 7 days in erythroid differentiation medium (EDM), e.g., as described in the Examples, and/or, proliferate at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, at least 95-fold, at least 100-fold, at least 110-fold, at least 120-fold, at least 130-fold, at least 140-fold, at least 150-fold, at least 200-fold, at least 300-fold, at least 400-fold, at least 500-fold, at least 600-fold, at least 700-fold, at least 800-fold, at least 900-fold, at least 1000-fold, at least 1100-fold, at least 1200-fold, at least 1300-fold, at least 1400-fold, at least 1500-fold or more after 21 days in erythroid differentiation medium (EDM), e.g., as described in the Examples,

In an embodiment, the invention provides a population of cells, e.g., CD34+ cells, of which at least 90%, e.g., at least 95%, e.g., at least 98%, of the cells of the population comprise an indel, e.g., as described herein (without being bound by theory, it is believed that introduction of a gRNA molecule or CRISPR system as described herein into a populaiton of cells produces a pattern of indels in said population, and thus, each cell of the population which comprises an indel may not exhibit the same indel), at or near a site complementary to the targeting domain of a gRNA molecule described herein; whrein said cells maintain the ability to differentiate into cells of an erythroid lineage, e.g., red blood cells; and/or wherein said cells differentiated from the population of cells have an increased level of fetal hemoglobin (e.g., the population has a higher % F cells) relative to cells differentiated from a similar population of unmodified cells. In embodiments, the population of cells has undergone at least a 5-fold expansion ex vivo, e.g., in the media comprising Compound 4.

In embodiments, the indel is less than about 50 nucleotides, e.g., less than about 45, less than about 40, less than about 35, less than about 30 or less than about 25 nucelotides. In embodiments, the indel is less than about 25 nucleotides. In embodiments, the indel is less than about 20 nucleotides. In embodiments, the indel is less than about 15 nucelotides. In embodiments, the indel is less than about 10 nucleotides. In embodiments, the indel is less than about 9 nucleotides. In embodiments, the indel is less than about 9 nucleotides. In embodiments, the indel is less than about 7 nucleotides. In embodiments, the indel is less than about 6 nucleotides. In embodiments, the indel is less than about 5 nucleotides. In embodiments, the indel is less than about 4 nucleotides. In embodiments, the indel is less than about 3 nucleotides. In embodiments, the indel is less than about 2 nucleotides. In any of the aforementioned embodiments, the indel is at least 1 nucleotide. In embodiments, the indel is 1 nucleotide.

In an aspect, the invention provides a population of modified HSPCs or erythroid cells differentiated from said HSPCs (e.g., differentiated ex vivo or in a patient), e.g., as described herein, wherein at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of the cells are F cells. In embodiments, the population of cells contains (or is capable of differentiating, e.g., in vivo, into a population of erythrocytes that contains) a higher percent of F cells than a similar population of cells which have not had a gRNA molecule or gRNA molecules, e.g., as described herein, introduced into said cells. In embodiments, the population of cells has (or is capable of differentiating, e.g., in vivo, into a population of erythrocytes that has) at least a 20% increase, e.g, at least 21% increase, at least 22% increase, at least 23% increase, at least 24% increase, at least 25% increase, at least 26% increase, at least 27% increase, at least 28% increase, or at least 29% increase, in F cells relative to the similar population of cells which have not had a gRNA molecule or gRNA molecules, e.g., as described herein, introduced into said cells. In embodiments, the population of cells has (or is capable of differentiating, e.g., in vivo, into a population of erythrocytes that has) at least a 30% increase, e.g., at least a 35% increase, at least a 40% increase, at least a 45% increase, at least a 50% increase, at least a 55% increase, at least a 60% increase, at least a 65% increase, at least a 70% increase, at least a 75% increase, at least a 80% increase, at least a 85% increase, at least a 90% increase or at least a 95% increase, in F cells relative to the similar population of cells which have not had a gRNA molecule or gRNA molecules, e.g., as described herein, introduced into said cells. In embodiments, the population of cells has (or is capable of differentiating, e.g., in vivo, into a population of erythrocytes that has) at a 10-90%, a 20%-80%, a 20%-70%, a 20%-60%, a 20%-50%, a 20%-40%, a 20%-30%, a 25%-80%, a 25%-70%, a 25%-60%, a 25%-50%, a 25%-40%, a 25%-35%, a 25%-30%, a 30%-80%, a 30%-70%, a 30%-60%, a 30%-50%, a 30%-40%, or a 30%-35% increase in F cells relative to the similar population of cells which have not had a gRNA molecule or gRNA molecules, e.g., as described herein, introduced into said cells.In embodiments, the population of cells, e.g., as produced by a method described herein, comprises a sufficient number or cells and/or a sufficient increase in % F cells to treat a hemoglobinopathy, e.g., as described herein, e.g., sickle cell disease and/or beta thalassemia, in a patient in need thereof when introduced into said patient, e.g., in a therapeutically effective amount. In embodiments, the increase in F cells is as measured in an erythroid differentiation assay, e.g., as described herein.

In embodiments, including in any of the embodiments and aspects described herein, the invention relates to a cell, e.g., a population of cells, e.g., as modified by any of the gRNA, methods and/or CRISPR systems described herein, comprising F cells that produce at least 6 picograms fetal hemoglobin per cell. In embodiments, the F cells produce at least 7 picograms fetal hemoglobin per cell. In embodiments, the F cells produce at least 8 picograms fetal hemoglobin per cell. In embodiments, the F cells produce at least 9 picograms fetal hemoglobin per cell. In embodiments, the F cells produce at least 10 picograms fetal hemoglobin per cell. In embodiments, the F cells produce an average of between 6.0 and 7.0 picograms, between 7.0 and 8.0, between 8.0 and 9.0, between 9.0 and 10.0, between 10.0 and 11.0, or between 11.0 and 12.0 picograms of fetal hemoglobin per cell.

In embodiments, including in any of the aforementioned embodiments, the cell and/or population of cells of the invention includes, e.g., consists of, cells which do not comprise nucleic acid encoding a Cas9 molecule.

### Methods of Treatment

### Delivery Timing

In an embodiment, one or more nucleic acid molecules (e.g., DNA molecules) other than the components of a Cas system, e.g., the Cas9 molecule component and/or the gRNA molecule component described herein, are delivered. In an embodiment, the nucleic acid molecule is delivered at the same time as one or more of the compoments of the Cas system are delivered. In an embodiment, the nucleic acid molecule is delivered before or after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In an embodiment, the nucleic acid molecule is delivered by a different means than one or more of the components of the Cas system, e.g., the Cas9 molecule component and/or the gRNA molecule compoment, are delivered. The nucleic acid molecule can be delivered by any of the delivery methods described herein. For example, the nucleic acid molecule can be delivered by a viral vector, e.g., an integration-deficient lentivirus, and the Cas9 molecule component and/or the gRNA molecule component can be delivered by electroporation, e.g., such that the toxicity caused by nucleic acids (e.g., DNAs) can be reduced. In an embodiment, the nucleic acid molecule encodes a therapeutic protein, e.g., a protein described herein. In an embodiment, the nucleic acid molecule encodes an RNA molecule, e.g, an RNA molecule described herein.

### Bi-modal or Differential Delivery of Components

Separate delivery of the components of a Cas system, e.g., the Cas9 molecule component and the gRNA molecule component, and more particularly, delivery of the components by differing modes, can enhance performance, e.g., by improving tissue specificity and safety. In an embodiment, the Cas9 molecule and the gRNA molecule are delivered by different modes, or as sometimes referred to herein as differential modes. Different or differential modes, as used herein, refer modes of delivery, that confer different pharmacodynamic or pharmacokinetic properties on the subject component molecule, e.g., a Cas9 molecule, gRNA molecule, template nucleic acid, or payload. E.g., the modes of delivery can result in different tissue distribution, different half-life, or different temporal distribution, e.g., in a selected compartment, tissue, or organ.

Some modes of delivery, e.g., delivery by a nucleic acid vector that persists in a cell, or in progeny of a cell, e.g., by autonomous replication or insertion into cellular nucleic acid, result in more persistent expression of and presence of a component. Examples include viral, e.g., adeno associated virus or lentivirus, delivery.

By way of example, the components, e.g., a Cas9 molecule and a gRNA molecule, can be delivered by modes that differ in terms of resulting half life or persistent of the delivered component the body, or in a particular compartment, tissue or organ. In an embodiment, a gRNA molecule can be delivered by such modes. The Cas9 molecule component can be delivered by a mode which results in less persistence or less exposure of its to the body or a particular compartment or tissue or organ.

More generally, in an embodiment, a first mode of delivery is used to deliver a first component and a second mode of delivery is used to deliver a second component. The first mode of delivery confers a first pharmacodynamic or pharmacokinetic property. The first pharmacodynamic property can be, e.g., distribution, persistence, or exposure, of the component, or of a nucleic acid that encodes the component, in the body, a compartment, tissue or organ. The second mode of delivery confers a second pharmacodynamic or pharmacokinetic property. The second pharmacodynamic property can be, e.g., distribution, persistence, or exposure, of the component, or of a nucleic acid that encodes the component, in the body, a compartment, tissue or organ.

In an embodiment, the first pharmacodynamic or pharmacokinetic property, e.g., distribution, persistence or exposure, is more limited than the second pharmacodynamic or pharmacokinetic property.

In an embodiment, the first mode of delivery is selected to optimize, e.g., minimize, a pharmacodynamic or pharmacokinetic property, e.g., distribution, persistence or exposure.

In an embodiment, the second mode of delivery is selected to optimize, e.g., maximize, a pharmacodynamic or pharmcokinetic property, e.g., distribution, persistence or exposure.

In an embodiment, the first mode of delivery comprises the use of a relatively persistent element, e.g., a nucleic acid, e.g., a plasmid or viral vector, e.g., an AAV or lentivirus. As such vectors are relatively persistent product transcribed from them would be relatively persistent.

In an embodiment, the second mode of delivery comprises a relatively transient element, e.g., an RNA or protein.

In an embodiment, the first component comprises gRNA, and the delivery mode is relatively persistent, e.g., the gRNA is transcribed from a plasmid or viral vector, e.g., an AAV or lentivirus. Transcription of these genes would be of little physiological consequence because the genes do not encode for a protein product, and the gR As are incapable of acting in isolation. The second component, a Cas9 molecule, is delivered in a transient manner, for example as mRNA or as protein, ensuring that the full Cas9 molecule/gRNA molecule complex is only present and active for a short period of time.

Furthermore, the components can be delivered in different molecular form or with different delivery vectors that complement one another to enhance safety and tissue specificity.

Use of differential delivery modes can enhance performance,' safety and efficacy. For example, the likelihood of an eventual off-target modification can be reduced. Delivery of immunogenic components, e.g., Cas9 molecules, by less persistent modes can reduce immunogenicity, as peptides from the bacterially-derived Cas enzyme are displayed on the surface of the cell by MHC molecules. A two-part deliveiy system can alleviate these drawbacks.

Differential delivery modes can be used to deliver components to different, but overlapping target regions. The formation active complex is minimized outside the overlap of the target regions. Thus, in an embodiment, a first component, e.g., a gRNA molecule is delivered by a first delivery mode that results in a first spatial, e.g., tissue, distribution. A second component, e.g., a Cas9 molecule is delivered by a second delivery mode that results in a second spatial, e.g., tissue, distribution. In an embodiment, the first mode comprises a first element selected from a liposome, nanoparticle, e.g., polymeric nanoparticle, and a nucleic acid, e.g., viral vector. The second mode comprises a second element selected from the group. In an embodiment, the first mode of delivery comprises a first targeting element, e.g., a cell specific receptor or an antibody, and the second mode of delivery does not include that element. In an embodiment, the second mode of delivery comprises a second targeting element, e.g., a second cell specific receptor or second antibody.

When the Cas9 molecule is delivered in a virus delivery vector, a liposome, or polymeric nanoparticle, there is the potential for delivery to and therapeutic activity in multiple tissues, when it may be desirable to only target a single tissue. A two-part delivery system can resolve this challenge and enhance tissue specificity. If the gRNA molecule and the Cas9 molecule are packaged in separated delivery vehicles with distinct but overlapping tissue tropism, the fully functional complex is only be formed in the tissue that is targeted by both vectors.

Candidate Cas molecules, e.g., Cas9 molecules, candidate gRNA molecules, candidate Cas9 molecule/gRNA molecule complexes, and candidate CRISPR systems, can be evaluated by art-known methods or as described herein. For example, exemplary methods for evaluating the endonuclease activity of Cas9 molecule are described, e.g., in Jinek el al., SCIENCE 2012; 337(6096):8 16-821.

### Enumerated embodiments of the invention

1. A gRNA molecule comprising a tracr and crRNA, wherein the crRNA comprises a targeting domain that is complementary with a target sequence of a BCL11A gene (e.g., a human BCL11a gene), a BCL11a enhancer (e.g., a human BCL11a enhancer), or a HFPH region (e.g., a human HPFH region).
2. A gRNA molecule of embodiment 1, wherein the target sequence is of the BCL11A gene, and the targeting domain comprises, e.g., consists of, any one of SEQ ID NO: 1 to SEQ ID NO: 85 or SEQ ID NO: 400 to SEQ ID NO: 1231.
3. A gRNA molecule of embodiment 1, wherein the target sequence is of a BCL11a enhancer, and the targeting domain comprises, e.g., consists of, any one of SEQ ID NO: 1232 to SEQ ID NO: 1499.
4. A gRNA molecule of embodiment 1, wherein the target sequence is of a BCL11a enhancer, and the targeting domain comprises, e.g., consists of, any one of SEQ ID NO: 182 to SEQ ID NO: 277 or SEQ ID NO: 334 to SEQ ID NO: 341.
5. A gRNA molecule of embodiment 4, wherein the targeting domain comprises, e.g., consists of, any one of SEQ ID NO: 341, SEQ ID NO: 246, SEQ ID NO: 248, SEQ ID NO: 247, SEQ ID NO: 245, SEQ ID NO: 249, SEQ ID NO: 244, SEQ ID NO: 199, SEQ ID NO: 251, SEQ ID NO: 250, SEQ ID NO: 334, SEQ ID NO: 185, SEQ ID NO: 186, SEQ ID NO: 336, or SEQ ID NO: 337.
6. A gRNA molecule of embodiment 4, wherein the targeting domain comprises, e.g., consists of, any one of SEQ ID NO: 247, SEQ ID NO: 248, SEQ ID NO: 335, SEQ ID NO: 336, SEQ ID NO: 337, or SEQ ID NO: 338.
7. A gRNA molecule of embodiment 4, wherein the targeting domain comprises, e.g., consists of, any one of SEQ ID NO: 248 or SEQ ID NO: 338.
8. A gRNA molecule of embodiment 4, wherein the targeting domain comprises, e.g., consists of, SEQ ID NO: 248.
9. A gRNA molecule of embodiment 4, wherein the targeting domain comprises, e.g., consists of, SEQ ID NO: 338.
10. A gRNA molecule of embodiment 1, wherein the target sequence is of a BCL11a enhancer, and the targeting domain comprises, e.g., consists of, any one of SEQ ID NO: 278 to SEQ ID NO: 333.
11. A gRNA molecule of embodiment 10, wherein the targeting domain comprises, e.g., consists of, any one of SEQ ID NO: 318, SEQ ID NO: 312, SEQ ID NO: 313, SEQ ID NO: 294, SEQ ID NO: 310, SEQ ID NO: 319, SEQ ID NO: 298, SEQ ID NO: 322, SEQ ID NO: 311, SEQ ID NO: 315, SEQ ID NO: 290, SEQ ID NO: 317, SEQ ID NO: 309, SEQ ID NO: 289, or SEQ ID NO: 281.
12. A gRNA molecule of embodiment 10, wherein the targeting domain comprises, e.g., consists of, SEQ ID NO: 318.
13. A gRNA molecule of embodiment 1, wherein the target sequence is of a BCL11a enhancer, and the targeting domain comprises, e.g., consists of, any one of SEQ ID NO: 1596 to SEQ ID NO: 1691.
14. A gRNA molecule of embodiment 13, wherein the targeting domain comprises, e.g., consists of, any one of SEQ ID NO: 1683, SEQ ID NO: 1638, SEQ ID NO: 1647, SEQ ID NO: 1609, SEQ ID NO: 1621, SEQ ID NO: 1617, SEQ ID NO: 1654, SEQ ID NO: 1631, SEQ ID NO: 1620, SEQ ID NO: 1637, SEQ ID NO: 1612, SEQ ID NO: 1656, SEQ ID NO: 1619, SEQ ID NO: 1675, SEQ ID NO: 1645, SEQ ID NO: 1598, SEQ ID NO: 1599, SEQ ID NO: 1663, SEQ ID NO: 1677, or SEQ ID NO: 1626.
15. A gRNA molecule of embodiment 1, wherein the target sequence is of a HFPH region (e.g., a French HPFH region), and the targeting domain comprises, e.g., consists of, any one of SEQ ID NO: 86 to SEQ ID NO: 181, SEQ ID NO: 1500 to SEQ ID NO: 1595, or SEQ ID NO: 1692 to SEQ ID NO: 1761.
16. A gRNA molecule of embodiment 15, wherein the targeting domain comprises, e.g., consists of, any one of SEQ ID NO: 100, SEQ ID NO: 165, SEQ ID NO: 113, SEQ ID NO: 99, SEQ ID NO: 112, SEQ ID NO: 98, SEQ ID NO: 1580, SEQ ID NO: 106, SEQ ID NO: 1503, SEQ ID NO: 1589, SEQ ID NO: 160, SEQ ID NO: 1537, SEQ ID NO: 159, SEQ ID NO: 101, SEQ ID NO: 162, SEQ ID NO: 104, SEQ ID NO: 138, SEQ ID NO: 1536, SEQ ID NO: 1539, SEQ ID NO: 1585.
17. A gRNA molecule of embodiment 15, wherein the targeting domain comprises, e.g., consists of, any one of SEQ ID NO: 98, SEQ ID NO: 100, SEQ ID NO: 1505, SEQ ID NO: 1589, SEQ ID NO: 1700, or SEQ ID NO: 1750.
18. A gRNA molecule of embodiment 15, wherein the targeting domain comprises, e.g., consists of, any one of SEQ ID NO: 100, SEQ ID NO: 165, or SEQ ID NO: 113.
19. The gRNA molecule of any of embodiments 2-18, wherein the targeting domain comprises, e.g., consists of, 17, 18, 19, 20, 21, 22, 23, or 24 consecutive nucleic acids of any one of the recited targeting domain sequences.
20. The gRNA molecule of embodiment 16, wherein the 17, 18, 19, 20, 21, 22, 23, or 24 consecutive nucleic acids of any one of the recited targeting domain sequences are the 17, 18, 19, 20, 21, 22, 23, or 24 consecutive nucleic acids disposed at the 3' end of the recited targeting domain sequence.
21. The gRNA molecule of embodiment 16, wherein the 17, 18, 19, 20, 21, 22, 23, or 24 consecutive nucleic acids of any one of the recited targeting domain sequences are the 17, 18, 19, 20, 21, 22, 23, or 24 consecutive nucleic acids disposed at the 5' end of the recited targeting domain sequence.
22. The gRNA molecule of embodiment 16, wherein the 17, 18, 19, 20, 21, 22, 23, or 24 consecutive nucleic acids of any one of the recited targeting domain sequences do not comprise either the 5' or 3' nucleic acid of the recited targeting domain sequence.
23. The gRNA molecule of any of embodiments 2-22, wherein the targeting domain consists of the recited targeting domain sequence.
24. The gRNA molecule of any of the previous embodiments, wherein a portion of the crRNA and a portion of the tracr hybridize to form a flagpole comprising SEQ ID NO: 6584 or 6585.
25. The gRNA molecule of embodiment 24, wherein the flagpole further comprises a first flagpole extension, located 3' to the crRNA portion of the flagpole, wherein said first flagpole extension comprises SEQ ID NO: 6586.
26. The gRNA molecule of embodiment 24 or 25, wherein the flagpole further comprises a second flagpole extension located 3' to the crRNA portion of the flagpole and, if present, the first flagpole extension, wherein said second flagpole extension comprises SEQ ID NO: 6587.
27. The gRNA molecule of any of embodiments 1-26, wherein the tracr comprises SEQ ID NO: 6660 or SEQ ID NO: 6661.
28. The gRNA molecule of any of embodiments 1-26, wherein the tracr comprises SEQ ID NO: 7812, optionally further comprising, at the 3' end, an additional 1, 2, 3, 4, 5, 6, or 7 uracil (U) nucleotides.
29. The gRNA molecule of any of embodiments 1-28, wherein the crRNA comprises, from 5' to 3', [targeting domain]-:
   a) SEQ ID NO: 6584;
   b) SEQ ID NO: 6585;
   c) SEQ ID NO: 6605;
   d) SEQ ID NO: 6606;
   e) SEQ ID NO: 6607;
   f) SEQ ID NO: 6608; or
   g) SEQ ID NO: 7806.
30. The gRNA molecule of any of embodiments 1-23 or 29, wherein the tracr comprises, from 5' to 3':
   a) SEQ ID NO: 6589;
   b) SEQ ID NO: 6590;
   c) SEQ ID NO: 6609;
   d) SEQ ID NO: 6610;
   e) SEQ ID NO: 6660;
   f) SEQ ID NO: 6661;
   g) SEQ ID NO: 7812;
   h) SEQ ID NO: 7807;
   i) (SEQ ID NO: 7808;
   j) SEQ ID NO: 7809;
   k) any of a) to j), above, further comprising, at the 3' end, at least 1, 2, 3, 4, 5, 6 or 7 uracil (U) nucleotides, e.g., 1, 2, 3, 4, 5, 6, or 7 uracil (U) nucleotides;
   l) any of a) to k), above, further comprising, at the 3' end, at least 1, 2, 3, 4, 5, 6 or 7 adenine (A) nucleotides, e.g., 1, 2, 3, 4, 5, 6, or 7 adenine (A) nucleotides; or
   m) any of a) to 1), above, further comprising, at the 5' end (e.g., at the 5' terminus), at least 1, 2, 3, 4, 5, 6 or 7 adenine (A) nucleotides, e.g., 1, 2, 3, 4, 5, 6, or 7 adenine (A) nucleotides.
31. The gRNA molecule of any of embodiments 1-23, wherein the targeting domain and the tracr are disposed on separate nucleic acid molecules, and wherein the nucleic acid molecule comprising the targeting domain comprises SEQ ID NO: 6607, optionally disposed immediately 3' to the targeting domain, and the nucleic acid molecule comprising the tracr comprises, e.g., consists of, SEQ ID NO: 6660.
32. The gRNA molecule of any of embodiments 27-28, wherein the crRNA portion of the flagpole comprises SEQ ID NO: 6607 or SEQ ID NO: 6608.
33. The gRNA molecule of any of embodiments 1-26, wherein the tracr comprises SEQ ID NO: 6589 or 6590, and optionally, if a first flagpole extension is present, a first tracr extension, disposed 5' to SEQ ID NO: 6589 or 6590, said first tracr extension comprising SEQ ID NO: 6591.
34. The gRNA molecule of any of embodiments 1-33, wherein the targeting domain and the tracr are disposed on separate nucleic acid molecules.
35. The gRNA molecule of any of embodiments 1-33, wherein the targeting domain and the tracr are disposed on a single nucleic acid molecule, and wherein the tracr is disposed 3' to the targeting domain.
36. The gRNA molecule of embodiment 35, further comprising a loop, disposed 3' to the targeting domain and 5' to the tracr.
37. The gRNA molecule of embodiment 36, wherein the loop comprises SEQ ID NO: 6588.
38. The gRNA molecule of any of embodiments 1-23, comprising, from 5' to 3', [targeting domain]-:
   (a) SEQ ID NO: 6601;
   (b) SEQ ID NO: 6602;
   (c) SEQ ID NO: 6603;
   (d) SEQ ID NO: 6604;
   (e) SEQ ID NO: 7811; or
   (f) any of (a) to (e), above, further comprising, at the 3' end, 1, 2, 3, 4, 5, 6 or 7 uracil (U) nucleotides.
39. The gRNA molecule of any of embodiments 1-40, wherein the targeting domain and the tracr are disposed on a single nucleic acid molecule, and wherein said nucleic acid molecule comprises, e.g., consists of, said targeting domain and SEQ ID NO: 7811, optionally disposed immediately 3' to said targeting domain.
40. The gRNA molecule of any of embodiments 1-39 wherein one, or optionally more than one, of the nucleic acid molecules comprising the gRNA molecule comprises:
   a) one or more, e.g., three, phosphorothioate modifications at the 3' end of said nucleic acid molecule or molecules;
   b) one or more, e.g., three, phosphorothioate modifications at the 5' end of said nucleic acid molecule or molecules;
   c) one or more, e.g., three, 2'-O-methyl modifications at the 3' end of said nucleic acid molecule or molecules;
   d) one or more, e.g., three, 2'-O-methyl modifications at the 5' end of said nucleic acid molecule or molecules;
   e) a 2' O-methyl modification at each of the 4^{th}-to-terminal, 3^{rd}-to-tertninal, and 2^{nd}-to-terminal 3' residues of said nucleic acid molecule or molecules;
   f) a 2' O-methyl modification at each of the 4^{th}-to-terminal, 3^{rd}-to-tertninal, and 2^{nd}-to-terminal 5' residues of said nucleic acid molecule or molecules; or
   f) any combination thereof.
41. A gRNA molecule of embodiment 1, comprising, e.g., consisting of, the sequence:
   (a) SEQ ID NO: 342;
   (b) SEQ ID NO: 343; or
   (c) SEQ ID NO: 1762.
42. A gRNA molecule of embodiment 1, comprising, e.g., consisting of:
   (a) a crRNA comprising, e.g., consisting of, SEQ ID NO: 344, and a tracr comprising, e.g., consisting of, SEQ ID NO: 6660;
   (b) a crRNA comprising, e.g., consisting of, SEQ ID NO: 344, and a tracr comprising, e.g., consisting of, SEQ ID NO: 346;
   (c) a crRNA comprising, e.g., consisting of, SEQ ID NO: 345, and a tracr comprising, e.g., consisting of, SEQ ID NO: 6660; or
   (d) a crRNA comprising, e.g., consisting of, SEQ ID NO: 345, and a tracr comprising, e.g., consisting of, SEQ ID NO: 346.
43. A gRNA molecule of embodiment 1, comprising, e.g., consisting of, the sequence:
   (a) SEQ ID NO: 347;
   (b) SEQ ID NO: 348; or
   (c) SEQ ID NO: 1763.
44. A gRNA molecule of embodiment 1, comprising, e.g., consisting of:
   (a) a crRNA comprising, e.g., consisting of, SEQ ID NO: 349, and a tracr comprising, e.g., consisting of, SEQ ID NO: 6660;
   (b) a crRNA comprising, e.g., consisting of, SEQ ID NO: 349, and a tracr comprising, e.g., consisting of, SEQ ID NO: 346;
   (c) a crRNA comprising, e.g., consisting of, SEQ ID NO: 350, and a tracr comprising, e.g., consisting of, SEQ ID NO: 6660; or
   (d) a crRNA comprising, e.g., consisting of, SEQ ID NO: 350, and a tracr comprising, e.g., consisting of, SEQ ID NO: 346.
45. A gRNA molecule of embodiment 1, comprising, e.g., consisting of, the sequence:
   (a) SEQ ID NO: 351;
   (b) SEQ ID NO: 352; or
   (c) SEQ ID NO: 1764.
46. A gRNA molecule of embodiment 1, comprising, e.g., consisting of:
   (a) a crRNA comprising, e.g., consisting of, SEQ ID NO: 353, and a tracr comprising, e.g., consisting of, SEQ ID NO: 6660;
   (b) a crRNA comprising, e.g., consisting of, SEQ ID NO: 353, and a tracr comprising, e.g., consisting of, SEQ ID NO: 346;
   (c) a crRNA comprising, e.g., consisting of, SEQ ID NO: 354, and a tracr comprising, e.g., consisting of, SEQ ID NO: 6660; or
   (d) a crRNA comprising, e.g., consisting of, SEQ ID NO: 354, and a tracr comprising, e.g., consisting of, SEQ ID NO: 346.
47. A gRNA molecule of embodiment 1, comprising, e.g., consisting of, the sequence:
   (a) SEQ ID NO: 355;
   (b) SEQ ID NO: 356; or
   (c) SEQ ID NO: 1765.
48. A gRNA molecule of embodiment 1, comprising, e.g., consisting of:
   (a) a crRNA comprising, e.g., consisting of, SEQ ID NO: 357, and a tracr comprising, e.g., consisting of, SEQ ID NO: 6660;
   (b) a crRNA comprising, e.g., consisting of, SEQ ID NO: 357, and a tracr comprising, e.g., consisting of, SEQ ID NO: 346;
   (c) a crRNA comprising, e.g., consisting of, SEQ ID NO: 358, and a tracr comprising, e.g., consisting of, SEQ ID NO: 6660; or
   (d) a crRNA comprising, e.g., consisting of, SEQ ID NO: 358, and a tracr comprising, e.g., consisting of, SEQ ID NO: 346.
49. A gRNA molecule of embodiment 1, comprising, e.g., consisting of, the sequence:
   (a) SEQ ID NO: 359;
   (b) SEQ ID NO: 360; or
   (c) SEQ ID NO: 1766.
50. A gRNA molecule of embodiment 1, comprising, e.g., consisting of:
   (a) a crRNA comprising, e.g., consisting of, SEQ ID NO: 361, and a tracr comprising, e.g., consisting of, SEQ ID NO: 6660;
   (b) a crRNA comprising, e.g., consisting of, SEQ ID NO: 361, and a tracr comprising, e.g., consisting of, SEQ ID NO: 346;
   (c) a crRNA comprising, e.g., consisting of, SEQ ID NO: 362, and a tracr comprising, e.g., consisting of, SEQ ID NO: 6660; or
   (d) a crRNA comprising, e.g., consisting of, SEQ ID NO: 362, and a tracr comprising, e.g., consisting of, SEQ ID NO: 346.
51. A gRNA molecule of embodiment 1, comprising, e.g., consisting of, the sequence:
   (a) SEQ ID NO: 363;
   (b) SEQ ID NO: 364; or
   (c) SEQ ID NO: 1767.
52. A gRNA molecule of embodiment 1, comprising, e.g., consisting of:
   (a) a crRNA comprising, e.g., consisting of, SEQ ID NO: 365, and a tracr comprising, e.g., consisting of, SEQ ID NO: 6660;
   (b) a crRNA comprising, e.g., consisting of, SEQ ID NO: 365, and a tracr comprising, e.g., consisting of, SEQ ID NO: 346;
   (c) a crRNA comprising, e.g., consisting of, SEQ ID NO: 366, and a tracr comprising, e.g., consisting of, SEQ ID NO: 6660; or
   (d) a crRNA comprising, e.g., consisting of, SEQ ID NO: 366, and a tracr comprising, e.g., consisting of, SEQ ID NO: 346.
53. A gRNA molecule of embodiment 1, comprising, e.g., consisting of, the sequence:
   (a) SEQ ID NO: 367;
   (b) SEQ ID NO: 368; or
   (c) SEQ ID NO: 1768.
54. A gRNA molecule of embodiment 1, comprising, e.g., consisting of:
   (a) a crRNA comprising, e.g., consisting of, SEQ ID NO: 369, and a tracr comprising, e.g., consisting of, SEQ ID NO: 6660;
   (b) a crRNA comprising, e.g., consisting of, SEQ ID NO: 369, and a tracr comprising, e.g., consisting of, SEQ ID NO: 346;
   (c) a crRNA comprising, e.g., consisting of, SEQ ID NO: 370, and a tracr comprising, e.g., consisting of, SEQ ID NO: 6660; or
   (d) a crRNA comprising, e.g., consisting of, SEQ ID NO: 370, and a tracr comprising, e.g., consisting of, SEQ ID NO: 346.
55. A gRNA molecule of embodiment 1, comprising, e.g., consisting of, the sequence:
   (a) SEQ ID NO: 371;
   (b) SEQ ID NO: 372; or
   (c) SEQ ID NO: 1769.
56. A gRNA molecule of embodiment 1, comprising, e.g., consisting of:
   (a) a crRNA comprising, e.g., consisting of, SEQ ID NO: 373, and a tracr comprising, e.g., consisting of, SEQ ID NO: 6660;
   (b) a crRNA comprising, e.g., consisting of, SEQ ID NO: 373, and a tracr comprising, e.g., consisting of, SEQ ID NO: 346;
   (c) a crRNA comprising, e.g., consisting of, SEQ ID NO: 374, and a tracr comprising, e.g., consisting of, SEQ ID NO: 6660; or
   (d) a crRNA comprising, e.g., consisting of, SEQ ID NO: 374, and a tracr comprising, e.g., consisting of, SEQ ID NO: 346.
57. A gRNA molecule of embodiment 1, comprising, e.g., consisting of, the sequence:
   (a) SEQ ID NO: 375;
   (b) SEQ ID NO: 376; or
   (c) SEQ ID NO: 1770.
58. A gRNA molecule of embodiment 1, comprising, e.g., consisting of:
   (a) a crRNA comprising, e.g., consisting of, SEQ ID NO: 377, and a tracr comprising, e.g., consisting of, SEQ ID NO: 6660;
   (b) a crRNA comprising, e.g., consisting of, SEQ ID NO: 377, and a tracr comprising, e.g., consisting of, SEQ ID NO: 346;
   (c) a crRNA comprising, e.g., consisting of, SEQ ID NO: 378, and a tracr comprising, e.g., consisting of, SEQ ID NO: 6660; or
   (d) a crRNA comprising, e.g., consisting of, SEQ ID NO: 378, and a tracr comprising, e.g., consisting of, SEQ ID NO: 346.
59. A gRNA molecule of embodiment 1, comprising, e.g., consisting of, the sequence:
   (a) SEQ ID NO: 379;
   (b) SEQ ID NO: 380; or
   (c) SEQ ID NO: 1771.
60. A gRNA molecule of embodiment 1, comprising, e.g., consisting of:
   (a) a crRNA comprising, e.g., consisting of, SEQ ID NO: 381, and a tracr comprising, e.g., consisting of, SEQ ID NO: 6660;
   (b) a crRNA comprising, e.g., consisting of, SEQ ID NO: 381, and a tracr comprising, e.g., consisting of, SEQ ID NO: 346;
   (c) a crRNA comprising, e.g., consisting of, SEQ ID NO: 382, and a tracr comprising, e.g., consisting of, SEQ ID NO: 6660; or
   (d) a crRNA comprising, e.g., consisting of, SEQ ID NO: 382, and a tracr comprising, e.g., consisting of, SEQ ID NO: 346.
61. A gRNA molecule of embodiment 1, comprising, e.g., consisting of, the sequence:
   (a) SEQ ID NO: 383;
   (b) SEQ ID NO: 384; or
   (c) SEQ ID NO: 1772.
62. A gRNA molecule of embodiment 1, comprising, e.g., consisting of:
   (a) a crRNA comprising, e.g., consisting of, SEQ ID NO: 385, and a tracr comprising, e.g., consisting of, SEQ ID NO: 6660;
   (b) a crRNA comprising, e.g., consisting of, SEQ ID NO: 385, and a tracr comprising, e.g., consisting of, SEQ ID NO: 346;
   (c) a crRNA comprising, e.g., consisting of, SEQ ID NO: 386, and a tracr comprising, e.g., consisting of, SEQ ID NO: 6660; or
   (d) a crRNA comprising, e.g., consisting of, SEQ ID NO: 386, and a tracr comprising, e.g., consisting of, SEQ ID NO: 346.
63. A gRNA molecule of embodiment 1, comprising, e.g., consisting of, the sequence:
   (a) SEQ ID NO: 387;
   (b) SEQ ID NO: 388; or
   (c) SEQ ID NO: 1773.
64. A gRNA molecule of embodiment 1, comprising, e.g., consisting of:
   (a) a crRNA comprising, e.g., consisting of, SEQ ID NO: 389, and a tracr comprising, e.g., consisting of, SEQ ID NO: 6660;
   (b) a crRNA comprising, e.g., consisting of, SEQ ID NO: 389, and a tracr comprising, e.g., consisting of, SEQ ID NO: 346;
   (c) a crRNA comprising, e.g., consisting of, SEQ ID NO: 390, and a tracr comprising, e.g., consisting of, SEQ ID NO: 6660; or
   (d) a crRNA comprising, e.g., consisting of, SEQ ID NO: 390, and a tracr comprising, e.g., consisting of, SEQ ID NO: 346.
65. A gRNA molecule of embodiment 1, comprising, e.g., consisting of, the sequence:
   (a) SEQ ID NO: 391;
   (b) SEQ ID NO: 392; or
   (c) SEQ ID NO: 1774.
66. A gRNA molecule of embodiment 1, comprising, e.g., consisting of:
   (a) a crRNA comprising, e.g., consisting of, SEQ ID NO: 393, and a tracr comprising, e.g., consisting of, SEQ ID NO: 6660;
   (b) a crRNA comprising, e.g., consisting of, SEQ ID NO: 393, and a tracr comprising, e.g., consisting of, SEQ ID NO: 346;
   (c) a crRNA comprising, e.g., consisting of, SEQ ID NO: 394, and a tracr comprising, e.g., consisting of, SEQ ID NO: 6660; or
   (d) a crRNA comprising, e.g., consisting of, SEQ ID NO: 394, and a tracr comprising, e.g., consisting of, SEQ ID NO: 346.
67. A gRNA molecule of embodiment 1, comprising, e.g., consisting of, the sequence:
   (a) SEQ ID NO: 395;
   (b) SEQ ID NO: 396; or
   (c) SEQ ID NO: 1775.
68. A gRNA molecule of embodiment 1, comprising, e.g., consisting of:
   (a) a crRNA comprising, e.g., consisting of, SEQ ID NO: 397, and a tracr comprising, e.g., consisting of, SEQ ID NO: 6660;
   (b) a crRNA comprising, e.g., consisting of, SEQ ID NO: 397, and a tracr comprising, e.g., consisting of, SEQ ID NO: 346;
   (c) a crRNA comprising, e.g., consisting of, SEQ ID NO: 398, and a tracr comprising, e.g., consisting of, SEQ ID NO: 6660; or
   (d) a crRNA comprising, e.g., consisting of, SEQ ID NO: 398, and a tracr comprising, e.g., consisting of, SEQ ID NO: 346.
69. A gRNA molecule of any of embodiments 1-68, wherein when a CRISPR system (e.g., an RNP as described herein) comprising the gRNA molecule is introduced into a cell, an indel is formed at or near the target sequence complementary to the targeting domain of the gRNA molecule.
70. The gRNA molecule of embodiment 69, wherein the indel does not comprise a nucleotide of a GATA-1 or TAL-1 binding site.
71. A gRNA molecule of any of embodiments 1-70, wherein when a CRISPR system (e.g., an RNP as described herein) comprising the gRNA molecule is introduced into a population of cells, an indel is formed at or near the target sequence complementary to the targeting domain of the gRNA molecule in at least about 40%, e.g., at least about 50%, e.g., at least about 60%, e.g., at least about 70%, e.g., at least about 80%, e.g., at least about 90%, e.g., at least about 95%, e.g., at least about 96%, e.g., at least about 97%, e.g., at least about 98%, e.g., at least about 99%, of the cells of the population.
72. A gRNA molecule of any of embodiments 1-68, wherein when a CRISPR system (e.g., an RNP as described herein) comprising the gRNA molecule is introduced into a population of cells, an indel that does not comprise a nucleotide of a GATA-1 or TAL-1 binding site is formed at or near the target sequence complementary to the targeting domain of the gRNA molecule in at least about 20%, e.g., at least about 30%, e.g., at least about 35%, e.g., at least about 40%, e.g., at least about 45%, e.g., at least about 50%, e.g., at least about 55%, e.g., at least about 60%, e.g., at least about 65%, e.g., at least about 70%, e.g., at least about 75%, e.g., at least about 80%, e.g., at least about 85%, e.g., at least about 90%, e.g., at least about 95%, e.g., at least about 99%, of the cells of the population.
73. The gRNA molecule of any of embodiments 71-72, wherein in at least about 30%, e.g., least about 40%, e.g., at least about 50%, e.g., at least about 60%, e.g., at least about 70%, e.g., at least about 80%, e.g., at least about 90%, e.g., at least about 95%, e.g., at least about 96%, e.g., at least about 97%, e.g., at least about 98%, e.g., at least about 99%, of the cells of the population, the indel is an indel listed in any of Figure 25, Table 15, Table 26, Table 27 or Table 37.
74. The gRNA molecule of any of embodiments 71-73, wherein the three most frequently detected indels in said population of cells comprise the indels associated with any gRNA molecule listed in any of Figure 25, Table 15, Table 26, Table 27 or Table 37.
75. The gRNA molecule of any of embodiments 69-74, wherein the indel is as measured by next generation sequencing (NGS).
76. A gRNA molecule of any of embodiments 1-75, wherein when a CRISPR system (e.g., an RNP as described herein) comprising the gRNA molecule is introduced into a cell, expression of fetal hemoglobin is increased in said cell or its progeny, e.g., its erythroid progeny, e.g., its red blood cell progeny.
77. A gRNA molecule of embodiment 76, wherein expression of fetal hemoglobin is increased in said cell or its progeny, e.g., its erythroid progeny, e.g., its red blood cell progeny, by at least about 20%, e.g., at least about 30%, e.g., at least about 40%, e.g., at least about 50%, e.g., at least about 60%, e.g., at least about 70%, e.g., at least about 80%, e.g., at least about 90%, e.g., at least about 95%, e.g., at least about 96%, e.g., at least about 97%, e.g., at least about 98%, e.g., at least about 99%.
78. A gRNA molecule of any of embodiments 76-77, wherein said cell or its progeny, e.g., its erythroid progeny, e.g., its red blood cell progeny, produces at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin per cell.
79. The gRNA molecule of any of embodiments 1-78, wherein when a CRISPR system (e.g., an RNP as described herein) comprising the gRNA molecule is introduced into a cell, no off-target indels are formed in said cell, e.g., as detectible by next generation sequencing and/or a nucleotide insertional assay.
80. The gRNA molecule of any of embodiments 1-78, wherein when a CRISPR system (e.g., an RNP as described herein) comprising the gRNA molecule is introduced into a population of cells, no off-target indel is detected in more than about 5%, e.g., more than about 1%, e.g., more than about 0.1%, e.g., more than about 0.01%, of the cells of the population of cells, e.g., as detectible by next generation sequencing and/or a nucleotide insertional assay.
81. The gRNA molecule of any of embodiments 69-80, wherein the cell is (or population of cells comprises) a mammalian, primate, or human cell, e.g., is a human cell.
82. The gRNA molecule of embodiment 81, wherein the cell is (or population of cells comprises) an HSPC.
83. The gRNA molecule of embodiment 82, wherein the HSPC is CD34+.
84. The gRNA molecule of embodiment 83, wherein the HSPC is CD34+CD90+.
85. The gRNA molecule of any of embodiments 69-84, wherein the cell is autologous with respect to a patient to be administered said cell.
86. The gRNA molecule of any of embodiments 69-84, wherein the cell is allogeneic with respect to a patient to be administered said cell.
87. A composition comprising:
   1) one or more gRNA molecules (including a first gRNA molecule) of any of embodiments 1-86 and a Cas9 molecule;
   2) one or more gRNA molecules (including a first gRNA molecule) of any of embodiments 1-86 and nucleic acid encoding a Cas9 molecule;
   3) nucleic acid encoding one or more gRNA molecules (including a first gRNA molecule) of any of embodiments 1-86 and a Cas9 molecule;
   4) nucleic acid encoding one or more gRNA molecules (including a first gRNA molecule) of any of embodiments 1-86 and nucleic acid encoding a Cas9 molecule; or
   5) any of 1) to 4), above, and a template nucleic acid; or
   6) any of 1) to 4) above, and nucleic acid comprising sequence encoding a template nucleic acid.
88. A composition comprising a first gRNA molecule of any of embodiments 1-86, further comprising a Cas9 molecule.
89. The composition of embodiment 87 or 88, wherein the Cas9 molecule is an active or inactive *s*. *pyogenes* Cas9.
90. The composition of embodiment 87-89, wherein the Cas9 molecule comprises SEQ ID NO: 6611.
91. The composition of embodiment 87-89, wherein the Cas9 molecule comprises, e.g., consists of:
   (a) SEQ ID NO: 7821;
   (b) SEQ ID NO: 7822;
   (c) SEQ ID NO: 7823;
   (d) SEQ ID NO: 7824;
   (e) SEQ ID NO: 7825;
   (f) SEQ ID NO: 7826;
   (g) SEQ ID NO: 7827;
   (h) SEQ ID NO: 7828;
   (i) SEQ ID NO: 7829;
   (j) SEQ ID NO: 7830; or
   (k) SEQ ID NO: 7831.
92. The composition of any of embodiments 88-91, wherein the first gRNA molecule and Cas9 molecule are present in a ribonuclear protein complex (RNP).
93. The composition of any of embodiments 87-92, further comprising a second gRNA molecule; a second gRNA molecule and a third gRNA molecule; or a second gRNA molecule, optionally, a third gRNA molecule, and, optionally, a fourth gRNA molecule, wherein the second gRNA molecule, the optional third gRNA molecule, and the optional fourth gRNA molecule are a gRNA molecule of any of embodiments 1-68, and wherein each gRNA molecule of the composition is complementary to a different target sequence.
94. The composition of embodiment 93, wherein two or more of the first gRNA molecule, the second gRNA molecule, the optional third gRNA molecule, and the optional fourth gRNA molecule are complementary to target sequences within the same gene or region.
95. The composition of embodiment 93 or 94, wherein the first gRNA molecule, the second gRNA molecule, the optional third gRNA molecule, and the optional fourth gRNA molecule are complementary to target sequences not more than 20000 nucleotides, not more than 10000 nucleotides, not more than 6000, not more than 5000 nucleotides, not more than 4000, not more than 1000 nucleotides, not more than 500 nucleotides, not more than 400 nucleotides, not more than 300 nucleotides, not more than 200 nucleotides, not more than 100 nucleotides, not more than 90 nucleotides, not more than 80 nucleotides, not more than 70 nucleotides, not more than 60 nucleotides, not more than 50 nucleotides, not more than 40 nucleotides, not more than 30 nucleotides, not more than 20 nucleotides or not more than 10 nucleotides apart.
96. The composition of embodiment 93, wherein two or more of the first gRNA molecule, the second gRNA molecule, the optional third gRNA molecule, and the optional fourth gRNA molecule are complementary to target sequence within different genes or regions.
97. The composition of any of embodiments 94-95, comprising a first gRNA molecule and a second gRNA molecule, wherein the first gRNA molecule and second gRNA molecule are:
   (a) independently selected from the gRNA molecules of embodiment 4, and are complementary to different target sequences;
   (b) independently selected from the gRNA molecules of embodiment 5, and are complementary to different target sequences;
   c) independently selected from the gRNA molecules of embodiment 6, and are complementary to different target sequences; or
   (d) independently selected from the gRNA molecules of embodiment 7, and are complementary to different target sequences; or
   (e) independently selected from the gRNA molecules of any of embodiments 41-56, and are complementary to different target sequences.
98. The composition of any of embodiments 94-95, comprising a first gRNA molecule and a second gRNA molecule, wherein the first gRNA molecule and second gRNA molecule are:
   (a) independently selected from the gRNA molecules of embodiment 10, and are complementary to different target sequences; or
   (b) independently selected from the gRNA molecules of embodiment 11, and are complementary to different target sequences.
99. The composition of any of embodiments 94-95, comprising a first gRNA molecule and a second gRNA molecule, wherein the first gRNA molecule and second gRNA molecule are:
   (a) independently selected from the gRNA molecules of embodiment 13, and are complementary to different target sequences; or
   (b) independently selected from the gRNA molecules of embodiment 14, and are complementary to different target sequences.
100. The composition of any of embodiments 94-96, comprising a first gRNA molecule and a second gRNA molecule, wherein the first gRNA molecule and second gRNA molecule are:
   (a) independently selected from the gRNA molecules of embodiment 16, and are complementary to different target sequences;
   (b) independently selected from the gRNA molecules of embodiment 17, and are complementary to different target sequences;
   (c) independently selected from the gRNA molecules of embodiment 18, and are complementary to different target sequences; or
   (d) independently selected from the gRNA molecules of any of embodiments 57-68, and are complementary to different target sequences.
101. The composition of any of embodiments 94-96, comprising a first gRNA molecule and a second gRNA molecule, wherein:
   (1) the first gRNA molecule is: (a) selected from the gRNA molecules of embodiment 4, (b) selected from the gRNA molecules of embodiment 5, (c) selected from the gRNA molecules of embodiment 6, (d) selected from the gRNA molecules of embodiment 7, or (e) selected from the gRNA molecules of any of embodiments 41-56; and
   (2) the second gRNA molecule is: (a) selected from the gRNA molecules of embodiment 10, or (b) selected from the gRNA molecules of embodiment 11.
102. The composition of any of embodiments 94-96, comprising a first gRNA molecule and a second gRNA molecule, wherein:
   (1) the first gRNA molecule is: (a) selected from the gRNA molecules of embodiment 4, (b) selected from the gRNA molecules of embodiment 5, (c) selected from the gRNA molecules of embodiment 6, (d) selected from the gRNA molecules of embodiment 7, or (e) selected from the gRNA molecules of any of embodiments 41-56; and
   (2) the second gRNA molecule is: (a) selected from the gRNA molecules of embodiment 13, (b) selected from the gRNA molecules of embodiment 14.
103. The composition of any of embodiments 94-96, comprising a first gRNA molecule and a second gRNA molecule, wherein:
   (1) the first gRNA molecule is: (a) selected from the gRNA molecules of embodiment 4, (b) selected from the gRNA molecules of embodiment 5, (c) selected from the gRNA molecules of embodiment 6, (d) selected from the gRNA molecules of embodiment 7, or (e) selected from the gRNA molecules of any of embodiments 41-56; and
   (2) the second gRNA molecule is: (a) selected from the gRNA molecules of embodiment 16, (b) selected from the gRNA molecules of embodiment 17, (c) selected from the gRNA molecules of embodiment 18, or (d) selected from the gRNA molecules of any of embodiments 57-68.
104. The composition of any of embodiments 94-96, comprising a first gRNA molecule and a second gRNA molecule, wherein:
   (1) the first gRNA molecule is: (a) selected from the gRNA molecules of embodiment 10, or (b) selected from the gRNA molecules of embodiment 11; and
   (2) the second gRNA molecule is: (a) selected from the gRNA molecules of embodiment 13, (b) selected from the gRNA molecules of embodiment 14.
105. The composition of any of embodiments 94-96, comprising a first gRNA molecule and a second gRNA molecule, wherein:
   (1) the first gRNA molecule is: (a) selected from the gRNA molecules of embodiment 10, or (b) selected from the gRNA molecules of embodiment 11; and
   (2) the second gRNA molecule is: (a) selected from the gRNA molecules of embodiment 16, (b) selected from the gRNA molecules of embodiment 17, (c) selected from the gRNA molecules of embodiment 18, or (d) selected from the gRNA molecules of any of embodiments 57-68.
106. The composition of any of embodiments 94-96, comprising a first gRNA molecule and a second gRNA molecule, wherein:
   (1) the first gRNA molecule is: (a) selected from the gRNA molecules of embodiment 13, (b) selected from the gRNA molecules of embodiment 14; and
   (2) the second gRNA molecule is: (a) selected from the gRNA molecules of embodiment 16, (b) selected from the gRNA molecules of embodiment 17, (c) selected from the gRNA molecules of embodiment 18, or (d) selected from the gRNA molecules of any of embodiments 57-68.
107. The composition of embodiment 96, comprising a first gRNA molecule and a second gRNA molecule, wherein:
   (1) the first gRNA molecule is: (a) selected from the gRNA molecules of embodiment 16, (b) selected from the gRNA molecules of embodiment 17, (c) selected from the gRNA molecules of embodiment 18, or (d) selected from the gRNA molecules of any of embodiments 57-68; and (2) the second gRNA molecule comprises a targeting domain that is complementary to a target sequence of the beta globin gene; or
   (1) the first gRNA molecule is: (a) selected from the gRNA molecules of embodiment 4, (b) selected from the gRNA molecules of embodiment 5, (c) selected from the gRNA molecules of embodiment 6, (d) selected from the gRNA molecules of embodiment 7, (e) selected from the gRNA molecules of any of embodiments 41-56, (f) selected from the gRNA molecules of embodiment 10, (g) selected from the gRNA molecules of embodiment 11, (h) selected from the gRNA molecules of embodiment 13, or (i) selected from the gRNA molecules of embodiment 14; and (2) the second gRNA molecule comprises a targeting domain that is complementary to a target sequence of the beta globin gene.
108. The composition of embodiment 94-96, wherein the first gRNA molecule and the second gRNA molecule are independently selected from the gRNA molecules of any of embodiments 41-68.
109. The composition of any of embodiments 87-108, wherein with respect to the gRNA molecule components of the composition, the composition consists of a first gRNA molecule and a second gRNA molecule.
110. The composition of any one of embodiments 87-109, wherein each of said gRNA molecules is in a ribonuclear protein complex (RNP) with a Cas9 molecule described herein, e.g., a Cas9 molecule of any of embodiments 90 or 91.
111. The composition of any of embodiments 87-110, comprising a template nucleic acid, wherein the template nucleic acid comprises a nucleotide that corresponds to a nucleotide at or near the target sequence of the first gRNA molecule.
112. The composition of any of embodiments 111, wherein the template nucleic acid comprises nucleic acid encoding:
   (a) human beta globin, e.g., human beta globin comprising one or more of the mutations G16D, E22A and T87Q, or fragment thereof; or
   (b) human gamma globin, or fragment thereof.
113. The composition of any of embodiments 87-112, formulated in a medium suitable for electroporation.
114. The composition of any of embodiments 87-113, wherein each of said gRNA molecules is in a RNP with a Cas9 molecule described herein, and wherein each of said RNP is at a concentration of less than about 10uM, e.g., less than about 3uM, e.g., less than about 1uM, e.g., less than about 0.5uM, e.g., less than about 0.3uM, e.g., less than about 0.1uM.
115. A nucleic acid sequence that encodes one or more gRNA molecules of any of embodiments 1-68.
116. The nucleic acid sequence of embodiment 115, wherein the nucleic acid comprises a promoter operably linked to the sequence that encodes the one or more gRNA molecules.
117. The nucleic acid sequence of embodiment 116, wherein the promoter is a promoter recognized by an RNA polymerase II or RNA polymerase III.
118. The nucleic acid sequence of embodiment 117, wherein the promoter is a U6 promoter or an HI promoter.
119. The nucleic acid sequence of any of embodiments 115-118, wherein the nucleic acid further encodes a Cas9 molecule.
120. The nucleic acid sequence of embodiment 119, wherien the Cas9 molecule comprises any of SEQ ID NO: 6611, SEQ ID NO: 7821, SEQ ID NO: 7822, SEQ ID NO: 7823, SEQ ID NO: 7824, SEQ ID NO: 7825, SEQ ID NO: 7826, SEQ ID NO: 7827, SEQ ID NO: 7828, SEQ ID NO: 7829, SEQ ID NO: 7830, or SEQ ID NO: 7831.
121. The nucleic acid sequence of any of embodiments 119-120, wherein said nucleic acid comprises a promoter operably linked to the sequence that encodes a Cas9 molecule.
122. The nucleic acid sequence of embodiment 121, wherein the promoter is an EF-1 promoter, a CMV IE gene promoter, an EF-1α promoter, an ubiquitin C promoter, or a phosphoglycerate kinase (PGK) promoter.
123. A vector comprising the nucleic acid of any of embodiments 115-122.
124. The vector of embodiment 123, wherein in the vector is selected from the group consisting of a lentiviral vector, an adenoviral vector, an adeno-associated viral (AAV) vector, a herpes simplex virus (HSV) vector, a plasmid, a minicircle, a nanoplasmid, and an RNA vector.
125. A method of altering a cell (e.g., a population of cells), (e.g., altering the structure (e.g., sequence) of nucleic acid) at or near a target sequence within said cell, comprising contacting (e.g., introducing into) said cell (e.g., population of cells) with:
   1) one or more gRNA molecules of any of embodiments 1-68 and a Cas9 molecule;
   2) one or more gRNA molecules of any of embodiments 1-68 and nucleic acid encoding a Cas9 molecule;
   3) nucleic acid encoding one or more gRNA molecules of any of embodiments 1-68 and a Cas9 molecule;
   4) nucleic acid encoding one or more gRNA molecules of any of embodiments 1-68 and nucleic acid encoding a Cas9 molecule;
   5) any of 1) to 4), above, and a template nucleic acid;
   6) any of 1) to 4) above, and nucleic acid comprising sequence encoding a template nucleic acid;
   7) the composition of any of embodiments 87-114; or
   8) the vector of any of embodiments 123-124.
126. The method of embodiment 125, wherein the gRNA molecule or nucleic acid encoding the gRNA molecule, and the Cas9 molecule or nucleic acid encoding the Cas9 molecule, are formulated in a single composition.
127. The method of embodiment 125, wherein the gRNA molecule or nucleic acid encoding the gRNA molecule, and the Cas9 molecule or nucleic acid encoding the Cas9 molecule, are formulated in more than one composition.
128. The method of embodiment 127, wherein the more than one composition are delivered simultaneously or sequentially.
129. The method of any of embodiments 125-128, wherein the cell is an animal cell.
130. The method of any of embodiments 125-128, wherein the cell is a mammalian, primate, or human cell.
131. The method of embodiment 130, wherein the cell is a hematopoietic stem or progenitor cell (HSPC) (e.g., a population of HSPCs).
132. The method of any of embodiments 125-131, wherein the cell is a CD34+ cell.
133. The method of any of embodiments 125-132, wherein the cell is a CD34+CD90+ cell.
134. The method of any of embodiments 125-133, wherein the cell is disposed in a composition comprising a population of cells that has been enriched for CD34+ cells.
135. The method of any of embodiments 125-134, wherein the cell (e.g. population of cells) has been isolated from bone marrow, mobilized peripheral blood, or umbilical cord blood.
136. The method of any of embodiments 125-135, wherein the cell is autologous or allogeneic with respect to a patient to be administered said cell.
137. The method of any of embodiments 125-136, wherein the altering results in an indel at or near a genomic DNA sequence complementary to the targeting domain of the one or more gRNA molecules.
138. The method of embodiment 137, wherein the indel is an indel shown on Figure 25, Table 15, Table 26, Table 27 or Table 37.
139. The method of embodiment 137-138, wherein the indel is an insertion or deletion of less than about 40 nucleotides, e.g., less than 30 nucleotides, e.g., less than 20 nucleotides, e.g., less than 10 nucleotides.
140. The method of embodiment 139, wherein the indel is a single nucleotide deletion.
141. The method of any of embodiments 137-140, wherein the method results in a population of cells wherein at least about 50%, e.g., at least about 60%, e.g., at least about 70%, e.g., at least about 80%, e.g., at least about 90% (e.g., at least about 95%, at least about 96%, at least about 97%, at least about 98%, or at least about 99%) of the cells of the population have been altered, e.g., comprise an indel.
142. The method of any of embodiments 125-141, wherein the altering results in a cell (e.g., population of cells) that is capable of differentiating into a differentiated cell of an erythroid lineage (e.g., a red blood cell), and wherein said differentiated cell exhibits an increased level of fetal hemoglobin, e.g., relative to an unaltered cell (e.g., population of cells).
143. The method of any of embodiments 125-142, wherein the altering results in a population of cells that is capable of differentiating into a population of differentiated cells, e.g., a population of cells of an erythroid lineage (e.g., a population of red blood cells), and wherein said population of differentiated cells has an increased fraction of F cells (e.g., at least about 15%, at least about 20%, at least about 25%, at least about 30%, or at least about 40% higher) e.g., relative to a population of unaltered cells.
144. The method of any of embodiments 125-142, wherein the altering results in a cell that is capable of differentiating into a differentiated cell, e.g., a cell of an erythroid lineage (e.g., a red blood cell), and wherein said differentiated cell produces at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin per cell.
145. A cell, altered by the method of any of embodiments 125-144.
146. A cell, obtainable by the method of any of embodiments 125-144.
147. A cell, comprising a first gRNA molecule of any of embodiments 1-68, or a composition of any of embodiments 87-114, a nucleic acid of any of embodiments 115-122, or a vector of any of embodiments 123-124.
148. The cell of embodiment 147, comprising a Cas9 molecule.
149. The cell of embodiment 148, wherein the Cas9 molecule comprises any of SEQ ID NO: 6611, SEQ ID NO: 7821, SEQ ID NO: 7822, SEQ ID NO: 7823, SEQ ID NO: 7824, SEQ ID NO: 7825, SEQ ID NO: 7826, SEQ ID NO: 7827, SEQ ID NO: 7828, SEQ ID NO: 7829, SEQ ID NO: 7830, or SEQ ID NO: 7831.
150. The cell of any of embodiments 145-149, wherein the cell comprises, has comprised, or will comprise a second gRNA molecule of any of embodiments 1-68, or a nucleic acid encoding a second gRNA molecule of any of embodiments 1-68, wherein the first gRNA molecule and second gRNA molecule comprise nonidentical targeting domains.
151. The cell of any of embodiments 145-150, wherein expression of fetal hemoglobin is increased in said cell or its progeny (e.g., its erythroid progeny, e.g., its red blood cell progeny) relative to a cell or its progeny of the same cell type that has not been modified to comprise a gRNA molecule.
152. The cell of any of embodiments 145-150, wherein the cell is capable of differentiating into a differentiated cell, e.g., a cell of an erythroid lineage (e.g., a red blood cell), and wherein said differentiated cell exhibits an increased level of fetal hemoglobin, e.g., relative to a cell of the same type that has not been modified to comprise a gRNA molecule.
153. The cell of embodiment 152, wherein the differentiated cell (e.g., cell of an erythroid lineage, e.g., red blood cell) produces at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin, e.g., relative to a cell of the same type that has not been modified to comprise a gRNA molecule.
154. The cell of any of embodiments 145-153, that has been contacted with a stem cell expander.
155. The cell of embodiment 154, wherein the stem cell expander is compound 1, compound 2, compound 3, compound 4, or a combination thereof (e.g., compound 1 and compound 4).
156. The cell of embodiment 155, wherein the stem cell expander is compound 4.
157. A cell, e.g., a cell of any of embodiments 145-156, comprising an indel at or near a genomic DNA sequence complementary to the targeting domain of a gRNA molecule of any of embodiments 1-68.
158. The cell of embodiment 157, wherein the indel is an indel shown on Figure 25, Table 15, Table 26, Table 27 or Table 37.
159. The cell of any of embodiments 157-158, wherein the indel is an insertion or deletion of less than about 40 nucleotides, e.g., less than 30 nucleotides, e.g., less than 20 nucleotides, e.g., less than 10 nucleotides..
160. The cell of any of embodiments 157-159, wherein the indel is a single nucleotide deletion.
161. The cell of any of embodiments 145-160, wherein the cell is an animal cell.
162. The cell of embodiment 161, wherein the cell is a mammalian, a primate, or a human cell.
163. The cell of any of embodiments 145-162, wherein the cell is a hematopoietic stem or progenitor cell (HSPC) (e.g., a population of HSPCs).
164. The cell of any of embodiments 145-163, wherein the cell is a CD34+ cell.
165. The cell of embodiment 164, wherein the cell is a CD34+CD90+ cell.
166. The cell of any of embodiments 145-165, wherein the cell (e.g. population of cells) has been isolated from bone marrow, mobilized peripheral blood, or umbilical cord blood.
167. The cell of any of embodiments 145-166, wherein the cell is autologous with respect to a patient to be administered said cell.
168. The cell of any of embodiments 145-166, wherein the cell is allogeneic with respect to a patient to be administered said cell.
169. A population of cells comprising the cell of any of embodiments 145-168.
170. The population of cells of embodiment 169, wherein at least about 50%, e.g., at least about 60%, e.g., at least about 70%, e.g., at least about 80%, e.g., at least about 90% (e.g., at least about 95%, at least about 96%, at least about 97%, at least about 98%, or at least about 99%) of the cells of the population are a cell according to any one of embodiments 145-168.
171. The population of cells of any of embodiments 169-170, wherein the population of cells is capable of differentiating into a population of differentiated cells, e.g., a population of cells of an erythroid lineage (e.g., a population of red blood cells), and wherein said population of differentiated cells has an increased fraction of F cells (e.g., at least about 15%, at least about 20%, at least about 25%, at least about 30%, or at least about 40% higher) e.g., relative to a population of unmodified cells of the same type.
172. The population of cells of embodiment 171, wherein the F cells of the population of differentiated cells produce an average of at least about 6 picograms (e.g., at least about 7 picograms, at least about 8 picograms, at least about 9 picograms, at least about 10 picograms, or from about 8 to about 9 picograms, or from about 9 to about 10 picograms) fetal hemoglobin per cell.
173. The population of cells of any of embodiments 169-172, comprising:
   1) at least 1e6 CD34+ cells/kg body weight of the patient to whom the cells are to be administered;
   2) at least 2e6 CD34+ cells/kg body weight of the patient to whom the cells are to be administered;
   3) at least 3e6 CD34+ cells/kg body weight of the patient to whom the cells are to be administered;
   4) at least 4e6 CD34+ cells/kg body weight of the patient to whom the cells are to be administered; or
   5) from 2e6 to 10e6 CD34+ cells/kg body weight of the patient to whom the cells are to be administered.
174. The population of cells of any of embodiments 169-173, wherein at least about 40%, e.g., at least about 50%, (e.g., at least about 60%, at least about 70%, at least about 80%, or at least about 90%) of the cells of the population are CD34+ cells.
175. The population of cells of embodiment 174, wherein at least about 10%, e.g., at least about 15%, e.g., at least about 20%, e.g., at least about 30% of the cells of the population are CD34+CD90+ cells.
176. The population of cells of any of embodiments 169-175, wherein the population of cells is derived from bone marrow.
177. The population of cells of any of embodiments 169-176, wherein the population of cells comprises, e.g., consists of, mammalian cells, e.g., human cells.
178. The population of cells of any of embodiments 169-177, wherein the population of cells is autologous relative to a patient to which it is to be administered.
179. The population of cells of any of embodiments 169-177, wherein the population of cells is allogeneic relative to a patient to which it is to be administered.
180. A composition comprising a cell of any of embodiments 145-168, or the population of cells of any of embodiments 169-179.
181. The composition of embodiment 180, comprising a pharmaceutically acceptable medium, e.g., a pharmaceutically acceptable medium suitable for cryopreservation.
182. A method of treating a hemoglobinopathy, comprising administering to a patient a cell of any of embodiments 145-168, a population of cells of any of embodiments 169-179, or a composition of any of embodiments 180-181.
183. A method of increasing fetal hemoglobin expression in a mammal, comprising administering to a patient a cell of any of embodiments 145-168, a population of cells of any of embodiments 169-179, or a composition of any of embodiments 180-181.
184. The method of embodiment 182, wherein the hemoglobinopathy is beta-thalassemia or sickle cell disease.
185. A method of preparing a cell (e.g., a population of cells) comprising:
   (a) providing a cell (e.g., a population of cells) (e.g., a HSPC (e.g., a population of HSPCs));
   (b) culturing said cell (e.g., said population of cells) ex vivo in a cell culture medium comprising a stem cell expander; and
   (c) introducing into said cell a first gRNA molecule of any of embodiments 1-68, a nucleic acid molecule encoding a first gRNA molecule of any of embodiments 1-68, a composition of any of embodiments 87-114, a nucleic acid of any of embodiments 115-122, or a vector of any of embodiments 123-124.
186. The method of embodiment 185, wherein after said introducing of step (c), said cell (e.g., population of cells) is capable of differentiating into a differentiated cell (e.g., population of differentiated cells), e.g., a cell of an erythroid lineage (e.g., population of cells of an erythroid lineage), e.g., a red blood cell (e.g., a population of red blood cells), and wherein said differentiated cell (e.g., population of differentiated cells) produces increased fetal hemoglobin, e.g., relative to the same cells which have not been subjected to step (c).
187. The method of any of embodiments 185-186, wherein the stem cell expander is compound 1, compound 2, compound 3, compound 4 or a combination thereof (e.g., compound 1 and compound 4).
188. The method of embodiment 187, wherein the stem cell expander is compound 4.
189. The method of any of embodiments 185-188, wherein the cell culture medium comprises thrombopoietin (Tpo), Flt3 ligand (Flt-3L), and human stem cell factor (SCF).
190. The method of embodiment 189, wherein the cell culture medium further comprises human interleukin-6 (IL-6).
191. The method of embodiment 189-190, wherein the cell culture medium comprises thrombopoietin (Tpo), Flt3 ligand (Flt-3L), and human stem cell factor (SCF) each at a concentration ranging from about 10 ng/mL to about 1000 ng/mL.
192. The method of embodiment 191, wherein the cell culture medium comprises thrombopoietin (Tpo), Flt3 ligand (Flt-3L), and human stem cell factor (SCF) each at a concentration of about 50 ng/mL, e.g, at a concentration of 50 ng/mL.
193. The method of any of embodiments 190-192, wherein the cell culture medium comprises human interleukin-6 (IL-6) at a concentration ranging from about 10 ng/mL to about 1000 ng/mL.
194. The method of embodiment 193, wherein the cell culture medium comprises human interleukin-6 (IL-6) at a concentration of about 50 ng/mL, e.g, at a concentration of 50 ng/mL.
195. The method of any of embodiments 185-194, wherein the cell culture medium comprises a stem cell expander at a concentration ranging from about 1 nM to about 1 mM.
196. The method of embodiment 195, wherein the cell culture medium comprises a stem cell expander at a concentration ranging from about 1 uM to about 100 uM.
197. The method of embodiment 196, wherein the cell culture medium comprises a stem cell expander at a concentration ranging from about 50 uM to about 75 uM.
198. The method of embodiment 197, wherein the cell culture medium comprises a stem cell expander at a concentration of about 50 uM, e.g., at a concentration of 50 uM.
199. The method of embodiment 197, wherein the cell culture medium comprises a stem cell expander at a concentration of about 75 uM, e.g., at a concentration of 75 uM.
200. The method of any of embodiments 185-199, wherein the culturing of steb (b) comprises a period of culturing before the introducing of step (c).
201. The method of embodiment 200, wherein the period of culturing before the introducing of step (c) is at least 12 hours, e.g., is for a period of about 1 day to about 3 days, e.g., is for a period of about 1 day to about 2 days, e.g., is for a period of about 2 days.
202. The method of any of embodiments 185-201, wherein the culturing of step (b) comprises a period of culturing after the introducing of step (c).
203. The method of embodiment 202, wherein the period of culturing after the introducing of step (c) is at least 12 hours, e.g., is for a period of about 1 day to about 10 days, e.g., is for a period of about 1 day to about 5 days, e.g., is for a period of about 2 days to about 4 days, e.g., is for a period of about 2 days or is for a period of about 3 days or is for a period of about 4 days.
204. The method of any of embodiments 185-203, wherein the population of cells is expanded at least 4-fold, e.g., at least 5-fold, e.g, at least 10-fold, e.g., relative to cells which are not cultured according to step (b).
205. The method of any of embodiments 185-204, wherein the introducing of step (c) comprises an electroporation.
206. The method of embodiment 205, wherein the electroporation comprises 1 to 5 pulses, e.g., 1 pulse, and wherein each pulse is at a pulse voltage ranging from 700 volts to 2000 volts and has a pulse duration ranging from 10 ms to 100 ms.
207. The method of embodiment 206, wherein the electroporation comprises 1 pulse.
208. The method of any of embodiments 206-207, wherein the pulse voltage ranges from 1500 to 1900 volts, e.g., is 1700 volts.
209. The method of any of embodiments 206-208, wherein the pulse duration ranges from 10 ms to 40 ms, e.g., is 20 ms.
210. The method of any of embodiments 185-209, wherein the cell (e.g., population of cells) provided in step (a) is a human cell (e.g., a population of human cells).
211. The method of embodiment 210, wherein the cell (e.g., population of cells) provided in step (a) is isolated from bone marrow, peripheral blood (e.g., mobilized peripheral blood) or umbilical cord blood.
212. The method of embodiment 211, wherein the cell (e.g., population of cells) provided in step (a) is isolated from bone marrow, e.g., is isolated from bone marrow of a patient suffering from a hemoglobinopathy.
213. The method of any of embodiments 185-212, wherein the population of cells provided in step (a) is enriched for CD34+ cells.
214. The method of any of embodiments 185-213, wherein subsequent to the introducing of step (c), the cell (e.g., population of cells) is cryopreserved.
215. The method of any of embodiments 185-214, wherein subsequent to the introducing of step (c), the cell (e.g., population of cells) comprises an indel at or near a genomic DNA sequence complementary to the targeting domain of the first gRNA molecule.
216. The method of any of embodiments 132, wherein the indel is an indel shown on Figure 25, Table 15, Table 26, Table 27 or Table 37.
217. The method of any of embodiments 185-216, wherein after the introducing of step (c), at least about 50%, at least about 60%, at least about 70%, at least about 80%, at least about 90%, at least about 95%, at least about 96%, at least about 97%, at least about 98% or at least about 99% of the cells of the population of cells comprise an indel at or near a genomic DNA sequence complementary to the targeting domain of the first gRNA molecule.
218. The method of embodiment 217, wherein the indel in each of said cells of the population of cells is an indel shown in Figure 25, Table 15, Table 26, Table 27 or Table 37.
219. A cell (e.g., population of cells), obtainable by the method of any of embodiments 185-218.
220. A method of treating a hemoglobinopathy, comprising administering to a human patient a composition comprising a cell (e.g., a population of cells) of embodiment 219.
221. A method of increasing fetal hemoglobin expression in a human patient, comprising administering to said human patient a composition comprising a cell (e.g., a population of cells) of embodiment 219.
222. The method of embodiment 220, wherein the hemoglobinopathy is beta-thalassemia or sickle cell disease.
223. The method of any of embodiments 220-222, wherein the human patient is administered a composition comprising at least about 1e6 cells of embodiment 219 per kg body weight of the human patient, e.g., at least about 1e6 CD34+ cells of embodiment 219 per kg body weight of the human patient.
224. The method embodiment 223, wherein the human patient is administered a composition comprising at least about 2e6 cells of embodiment 219 per kg body weight of the human patient, e.g., at least about 2e6 CD34+ cells of embodiment 219 per kg body weight of the human patient.
225. The method embodiment 223, wherein the human patient is administered a composition comprising from about 2e6 to about 10e6 cells of embodiment 219 per kg body weight of the human patient, e.g., at least about 2e6 to about 10e6 CD34+ cells of embodiment 219 per kg body weight of the human patient.
226. A gRNA molecule of any of embodiments 1-86, a composition of any of embodiments 87-114 or 180-181, a nucleic acid of any of embodiments 115-122, a vector of any of embodiments 123-124, a cell of any of embodiments 145-168 or 219, or a population of cells of any of embodiments 169-179, for use as a medicament.
227. A gRNA molecule of any of embodiments 1-86, a composition of any of embodiments 87-114 or 180-181, a nucleic acid of any of embodiments 115-122, a vector of any of embodiments 123-124, a cell of any of embodiments 145-168 or 219, or a population of cells of any of embodiments 169-179, for use in the manufacture of a medicament.
228. A gRNA molecule of any of embodiments 1-86, a composition of any of embodiments 87-114 or 180-181, a nucleic acid of any of embodiments 115-122, a vector of any of embodiments 123-124, a cell of any of embodiments 145-168 or 219, or a population of cells of any of embodiments 169-179, for use in the treatment of a disease.
229. A gRNA molecule of any of embodiments 1-86, a composition of any of embodiments 87-114 or 180-181, a nucleic acid of any of embodiments 115-122, a vector of any of embodiments 123-124, a cell of any of embodiments 145-168 or 219, or a population of cells of any of embodiments 169-179, for use in the treatment of a disease, wherein the disease is a hemoglobinopathy.
230. A gRNA molecule of any of embodiments 1-86, a composition of any of embodiments 87-114 or 180-181, a nucleic acid of any of embodiments 115-122, a vector of any of embodiments 123-124, a cell of any of embodiments 145-168 or 219, or a population of cells of any of embodiments 169-179, for use in the treatment of a disease, wherein the hemoglobinopathy is beta-thalassemia or sickle cell disease.

### EXAMPLES

### Example 1

### Guide Selection and Design

Initial guide selection was performed *in silico* using a human reference genome and user defined genomic regions of interest (e.g., a gene, an exon of a gene, non-coding regulatory region, etc), for identifying PAMs in the regions of interest. For each identified PAM, analyses were performed and statistics reported. gRNA molecules were further selected and rank-ordered based on a number of methods for determining efficiency and efficacy, e.g., as described herein.

Throughout the Examples, in the experiments below, either sgRNA molecules or dgRNA molecules were used. Unless indicated otherwise, where dgRNA molecules were used, the gRNA includes the following:
crRNA: [targeting domain]-[SEQ ID NO: 6607]
tracr (trRNA): SEQ ID NO: 6660

Unless indicated otherwise, in experiments employing a sgRNA molecule, the following sequence was used:
[targeting domain]-[SEQ ID NO: 6601]-UUUU

Unless inidicated otherwise, in experiments employing a sgRNA molecule labeled "BC", the following sequence was used:
[targeting domain]-[SEQ ID NO: 6604]-UUUU

### Transfection of HEK-293 Cas9GFP Cells for Primary Guide Screening

Transfection of Cas9GFP-expressing HEK293 cells (HEK-293_Cas9GFP) was used for primary screening of target specific crRNAs. In this example, target specific crRNAs were designed and selected for primary screening using defined criteria including *in silico* off-target detection, e.g., as described herein. Selected crRNAs were chemically synthesized and delivered in a 96 well format. HEK-293-Cas9GFP cells were transfected with target crRNAs in a 1:1 ratio with stock trRNA. The transfection was mediated using lipofection technology according to manufacturer's protocol (Lipofectamine 2000, Life Technologies). Transfected cells were lysed 24h following lipofection and editing (e.g., cleavage) was detected within lysates with the T7E1 assay and/or next generation sequencing (NGS; below).

### T7E1 Assay

The T7E1 assay was used to detect mutation events in genomic DNA such as insertions, deletions and substitutions created through non-homologous end joining (NHEJ) following DNA cleavage by Cas9 (See Cho et al., Targeted genome engineering in human cells with the Cas9 RNA-guided endonuclease. Nature Biotechnology. 2013; 31, 230-232).

Genomic DNA regions that have been targeted for cutting by CRISPR/Cas9 were amplified by PCR, denatured at 95°C for 10 minutes, and then re-annealed by ramping down from 95°C to 25°C at 0.5°C per second. If mutations were present within the amplified region, the DNA combined to form heteroduplexes. The re-annealed heteroduplexes were then digested with T7E1 (New England Biolabs) at 37°C for 25 minutes or longer. T7E1 endonuclease recognizes DNA mismatches, heteroduplexes and nicked double stranded DNA and generates a double stranded break at these sites. The resulting DNA fragments were analyzed using a Fragment Analyzer and quantified to determine cleavage efficiency.

### Next-Generation Sequencing (NGS) and Analysis for On-Target Cleavage Efficiency and Indel Formation

To determine the efficiency of editing (e.g., cleaving) the target location in the genome, deep sequencing was utilized to identify the presence of insertions and deletions introduced by non-homologous end joining.

PCR primers were first designed around the target site, and the genomic area of interest PCR amplified. Additional PCR was performed according to manufacturer's protocols (Illumina) to add the necessary chemistry for sequencing. The amplicons were then sequenced on an Illumina MiSeq instrument. The reads were then aligned to the human reference genome (e.g., hg38) after eliminating those having low quality scores. From the resulting files containing the reads mapped to the reference genome (BAM files), reads which overlap the target region of interest were selected and the number of wild type reads versus the number of reads which contain an insertion or deletion was calculated. The editing percentage was then defined as the total number of reads with insertions or deletions over the total number of reads, including wild type. To determine the pattern of insertions and/or deletions that resulted from the edit, the aligned reads with indels were selected and the number of a reads with a given indel were summed. This information was then displayed as a list as well as visualized in the form on histograms which represent the frequency of each indel.

### RNP generation

The addition of crRNA and trRNA to Cas9 protein results in the formation of the active Cas9 ribonucleoprotein complex (RNP), which mediates binding to the target region specified by the crRNA and specific cleavage of the targeted genomic DNA. This complex was formed by loading trRNA and crRNA into Cas9, which is believed to cause conformational changes to Cas9 allowing it to bind and cleave dsDNA.

The crRNA and trRNA were separately denatured at 95°C for 2 minutes, and allowed to come to room temperature. Cas9 protein (10mg/ml) was added to 5X CCE buffer (20mM HEPES, 100mM KCl, 5mM MgCl₂, 1mM DTT, 5% glycerol), to which trRNA and the various crRNAs were then added (in separate reactions) and incubated at 37°C for 10 minutes, thereby forming the active RNP complex. The complex was delivered by electroportation and other methods into a wide variety of cells, including HEK-293 and CD34+ hematopoietic cells.

### Delivery of RNPs to CD34+ HSCs

Cas9 RNPs were delivered into CD34+ HSCs.

CD34+ HSCs were thawed and cultured (at ~500,000 cells/ml) overnight in StemSpan SFEM (StemCell Technologies) media with IL12, SCF, TPO, Flt3L and Pen/Strep added. Roughly 90,000 cells were aliquoted and pelleted per each RNP delivery reaction. The cells were then resuspended in 60ul P3 nucleofection buffer (Lonza), to which active RNP was subsequently added. The HSCs were then electroporated (e.g., nucleofected using program CA-137 on a Lonza Nucleofector) in triplicate (20 uL/electroporation). Immediately following electroporation, StemSpan SFEM media (with IL12, SCF, TPO, Flt3L and Pen/Strep) was added to the HSCs, which were cultured for at least 24 hours. HSCs were then harvested and subjected to T7E1, NGS, and/or surface marker expression analyses.

### HSC Functional Assay

CD34+ HSCs may be assayed for stem cell phenotype using known techniques such as flow cytometry or the in vitro colony forming assay. By way of example, cells were assayed by the in vitro colony forming assay (CFC) using the Methocult H4034 Optimum kit (StemCell Technologies) using the manufacturer's protocol. Briefly, 500-2000 CD34+ cells in <=100ul volume are added to 1-1.25ml methocult. The mixture was vortexed vigorously for 4-5 seconds to mix thoroughly, then allowed to rest at room temperature for at least 5 minutes. Using a syringe, 1-1.25ml of MethoCult + cells was transferred to a 35mm dish or well of a 6-well plate. Colony number and morphology was assessed after 12-14 days as per the manufacturer's protocol.

### In vivo xeno-transplantation

HSCs are functionally defined by their ability to self-renew and for multi-lineage differentiation. This functionality can only be assessed in vivo. The gold-standard for determining human HSC function is through xeno-transplantation into the NOD-SCID gamma mouse (NSG) that through a series of mutations is severely immunocompromised and thus can act as a recipient for human cells. HSCs following editing will be transplanted into NSG mice to validate that the induced edit does not impact HSC function. Monthly peripheral blood analysis will be used to assess human chimerism and lineage development and secondary transplantation following 20 weeks will be used to establish the presence of functional HSCs.

### Results

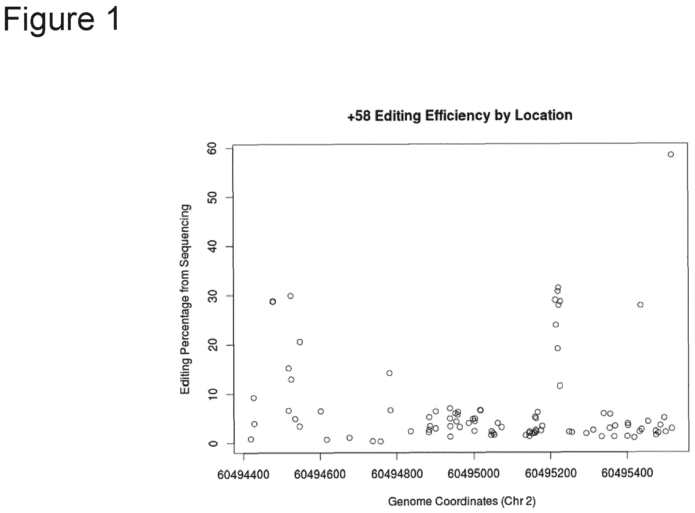

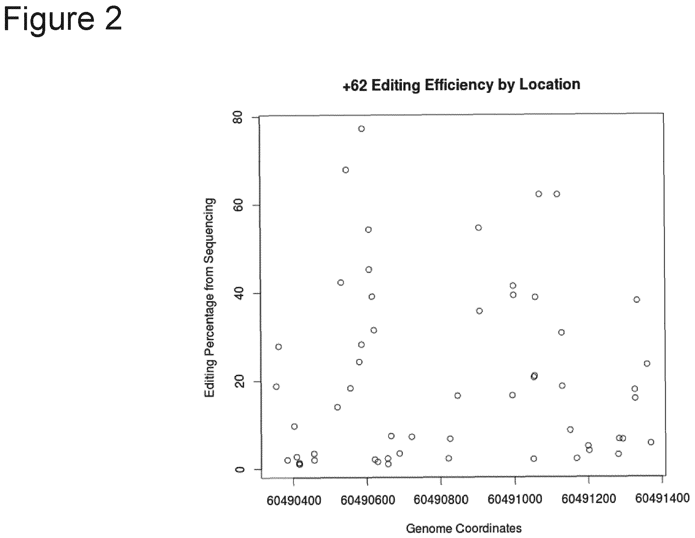

The results of editing using gRNA molecules (e.g., dgRNA molecules as describe above) described herein, in HEK293_Cas9GFP cells, as assayed by T7E1 ("T7") and/or NGS are summarized in Figure 1 (gRNAs molecules to +58 BCL11a enhancer region) and Figure 2 (gRNA moleucles to +62 BCL11a enhancer region), and in the tables below, as indicated. Average editing in CD34+ HSPCs are reported in, for example, Table 10 (gRNAs molecules to +58 BCL11a enhancer region), Table 11 (gRNAs molecules to +62 BCL11a enhancer region), Table 12B (gRNAs molecules to +55 BCL11a enhancer region), and Table 13 (gRNAs molecules to the French HPFH region), below. Where T7E1 assay results are reported, "2" indicates high efficiency cutting; "1" indicates low efficiency cutting, and "0" indicates no cutting. In general, T7E1 results correlate with the quantitative cutting assayed by NGS. Top 15 gRNAs (as ranked by highest % editing in CD34+ cells) are shown in Figure 11 (+58 BCL11a enhancer region) and Figure 12 (+62 BCL11a enhancer region).

**Table 10: Bcl11a +58 enhancer region crRNA 1° Screen results in CD34+ HSPCs (by NGS, n=3).**

| **Targeting Domain ID** | **Average % Editing (n=3)** | **StDev** |
|---|---|---|
| CR000242 | 1.4% | 0.3% |
| CR000243 | n/a | n/a |
| CR000244 | 3.7% | 1.0% |
| CR000245 | 24.7% | 1.4% |
| CR000246 | 24.2% | 0.3% |
| CR000247 | 3.6% | 0.6% |
| CR000248 | 6.4% | 0.5% |
| CR000249 | 13.3% | 0.6% |
| CR000250 | 4.8% | 0.5% |
| CR000251 | 4.7% | 0.5% |
| CR000252 | 2.7% | 0.5% |
| CR000253 | 9.0% | 0.6% |
| CR000255 | n/a | n/a |
| CR000256 | 1.3% | 0.2% |
| CR000257 | 2.4% | 0.4% |
| CR000258 | 2.0% | 0.0% |
| CR000259 | 3.9% | 0.4% |
| CR000260 | 32.7% | 2.3% |
| CR000261 | 17.9% | 0.9% |
| CR000264 | 2.1% | 0.2% |
| CR000266 | 2.9% | 0.1% |
| CR000267 | 3.9% | 0.1% |
| CR000268 | 6.3% | 0.6% |
| CR000269 | 4.4% | 0.2% |
| CR000270 | 2.7% | 0.9% |
| CR000271 | 7.2% | 1.4% |
| CR000272 | 11.9% | 2.9% |
| CR000273 | 3.1% | 0.2% |
| CR000274 | 1.4% | 0.1% |
| CR000275 | 9.6% | 0.6% |
| CR000276 | 14.5% | 0.5% |
| CR000277 | 8.6% | 0.5% |
| CR000278 | 4.8% | 0.6% |
| CR000279 | 6.5% | 0.9% |
| CR000280 | 7.9% | 0.8% |
| CR000281 | 3.3% | 0.7% |
| CR000282 | 10.1% | 1.2% |
| CR000283 | 4.7% | 0.2% |
| CR000284 | 5.8% | 0.1% |
| CR000285 | 8.9% | 1.2% |
| CR000286 | 20.2% | 1.1% |
| CR000287 | 10.1% | 1.0% |
| CR000288 | 1.6% | 0.1% |
| CR000289 | 3.0% | 0.4% |
| CR000290 | 2.3% | 0.4% |
| CR000291 | 2.8% | 0.1% |
| CR000292 | 9.9% | 0.9% |
| CR000293 | n/a | n/a |
| CR000294 | 2.7% | 0.5% |
| CR000295 | 1.9% | 0.4% |
| CR000296 | 2.8% | 0.4% |
| CR000297 | 5.7% | 0.4% |
| CR000298 | 2.2% | 0.2% |
| CR000299 | 2.9% | 0.2% |
| CR000300 | 2.3% | 0.2% |
| CR000301 | 10.2% | 0.8% |
| CR000302 | 5.0% | 0.6% |
| CR000303 | 3.7% | 0.3% |
| CR000304 | 6.2% | 0.8% |
| CR000305 | 7.7% | 2.2% |
| CR000306 | 4.0% | 2.2% |
| CR000307 | 8.8% | 3.6% |
| CR000308 | 39.9% | 1.7% |
| CR000309 | 38.3% | 8.2% |
| CR000310 | 56.8% | 1.0% |
| CR000311 | 46.0% | 1.2% |
| CR000312 | 48.5% | 1.7% |
| CR000313 | 41.2% | 4.4% |
| CR000314 | 29.9% | 0.9% |
| CR000315 | 32.0% | 2.9% |
| CR000316 | 3.9% | 1.0% |
| CR000317 | 2.8% | 0.3% |
| CR000318 | 2.6% | 0.2% |
| CR000319 | 7.9% | 1.8% |
| CR000320 | 1.9% | 0.1% |
| CR000321 | 13.2% | 1.7% |
| CR000322 | 5.2% | 0.8% |
| CR000323 | 20.3% | 2.1% |
| CR000324 | 2.2% | 0.4% |
| CR000325 | 8.4% | 0.9% |
| CR000326 | 1.4% | 0.2% |
| CR000327 | 6.9% | 1.7% |
| CR000328 | 2.6% | 0.4% |
| CR000329 | 1.1% | 0.1% |
| CR000330 | 1.7% | 0.2% |
| CR000331 | 22.0% | 0.9% |
| CR000332 | 3.2% | 1.0% |
| CR000333 | 3.8% | 0.7% |
| CR000334 | 3.2% | 0.1% |
| CR000335 | 2.7% | 0.3% |
| CR000336 | 2.2% | 0.2% |
| CR000337 | 7.2% | 1.0% |
| CR000338 | 10.3% | 1.8% |
| CR000339 | 4.2% | 0.2% |
| CR000340 | 4.6% | 0.6% |
| CR000341 | 7.5% | 1.4% |
| CR001124 | 26.1% | 2.0% |
| CR001125 | 8.4% | 1.6% |
| CR001126 | 17.2% | 5.8% |
| CR001127 | 7.4% | 2.3% |
| CR001128 | 9.0% | 0.9% |
| CR001129 | 16.5% | 6.0% |
| CR001130 | 11.1% | 8.3% |
| CR001131 | 54.1% | 4.9% |

**Table 11: Bcl11a +62 enhancer region crRNA 1° Screen results in CD34+ HSPCs (by NGS, n=3).**

| **Targeting Domain ID** | **Average % Edit (n=3)** | **StDev** |
|---|---|---|
| CR000171 | 4.4 | 0.7 |
| CR000172 | 4.6 | 2.4 |
| CR000173 | 3.1 | 0.7 |
| CR000174 | 27.6 | 6.5 |
| CR000175 | 14.7 | 1.0 |
| CR000176 | 2.1 | 0.6 |
| CR000177 | 7.6 | 2.3 |
| CR000178 | 7.1 | 1.7 |
| CR000179 | 7.7 | 1.8 |
| CR000180 | 2.8 | 0.5 |
| CR000181 | 25.6 | 3.8 |
| CR000182 | 27.6 | 3.5 |
| CR000183 | 30.5 | 6.9 |
| CR000184 | 4.2 | 1.9 |
| CR000185 | 11.4 | 0.7 |
| CR000186 | 7.2 | 3.7 |
| CR000187 | 46.3 | 2.2 |
| CR000188 | 21.0 | 6.0 |
| CR000189 | 22.3 | 2.1 |
| CR000190 | 20.2 | 5.7 |
| CR000191 | 38.2 | 7.9 |
| CR000192 | 5.4 | 1.8 |
| CR000193 | 14.7 | 2.0 |
| CR000194 | 13.5 | 0.7 |
| CR000195 | 8.2 | 1.2 |
| CR000196 | 27.0 | 6.4 |
| CR000197 | 6.6 | 0.6 |
| CR000198 | 8.7 | 3.2 |
| CR000199 | 3.3 | 1.3 |
| CR000200 | 6.9 | 0.5 |
| CR000201 | 1.6 | 0.5 |
| CR000202 | 28.1 | 5.6 |
| CR000203 | 43.7 | 0.5 |
| CR000204 | 35.1 | 2.2 |
| CR000205 | 53.6 | 0.8 |
| CR000206 | 52.8 | 6.0 |
| CR000207 | 2.5 | 0.3 |
| CR000208 | 31.5 | 7.5 |
| CR000209 | 13.5 | 2.9 |
| CR000210 | 28.7 | 6.6 |
| CR000211 | 59.3 | 5.5 |
| CR000212 | 40.0 | 1.6 |
| CR000213 | 13.6 | 7.1 |
| CR000214 | 9.4 | 0.7 |
| CR000215 | 37.0 | 6.9 |
| CR000216 | 25.3 | 2.6 |
| CR000217 | 7.6 | 1.6 |
| CR000218 | 5.1 | 1.3 |
| CR000221 | 12.4 | 3.5 |
| CR000222 | 7.2 | 2.6 |
| CR000223 | 8.4 | 2.1 |
| CR000224 | 13.9 | 5.3 |
| CR000225 | 10.3 | 4.4 |
| CR000227 | 12.6 | 1.8 |
| CR000228 | 13.5 | 4.8 |
| CR000229 | 3.2 | 0.8 |

**Table 12A: Bcl11a +55 enhancer region crRNA 1° Screen results in HEK-293-Cas9 Cells**

| **Targeting Domain ID** | **Average % Edit (n=3)** | **StDev** |
|---|---|---|
| CR002142 | 25.0 | 2.0 |
| CR002143 | n/a | n/a |
| CR002144 | 51.2 | 8.3 |
| CR002145 | 51.0 | 3.3 |
| CR002146 | n/a | n/a |
| CR002147 | n/a | n/a |
| CR002148 | 13.7 | 4.3 |
| CR002149 | 22.3 | 4.5 |
| CR002150 | 43.8 | 9.0 |
| CR002151 | 31.1 | 0.6 |
| CR002152 | 33.2 | 8.7 |
| CR002153 | 24.5 | 7.8 |
| CR002154 | 62.1 | 3.9 |
| CR002155 | 48.2 | 10.9 |
| CR002156 | 57.4 | 8.5 |
| CR002157 | 46.6 | 4.3 |
| CR002158 | 11.0 | 1.7 |
| CR002159 | 13.7 | 2.3 |
| CR002160 | 37.8 | 0.8 |
| CR002161 | 61.5 | 4.6 |
| CR002162 | 47.0 | 14.0 |
| CR002163 | 52.2 | 9.6 |
| CR002164 | 58.5 | 4.4 |
| CR002165 | 61.5 | 6.8 |
| CR002166 | 42.6 | 11.3 |
| CR002167 | 37.8 | 4.7 |
| CR002168 | 32.0 | 12.9 |
| CR002169 | 48.5 | 6.3 |
| CR002170 | 44.8 | 2.9 |
| CR002171 | n/a | n/a |
| CR002172 | n/a | n/a |
| CR002173 | n/a | n/a |
| CR002174 | 59.8 | 3.8 |
| CR002175 | 22.1 | 1.3 |
| CR002176 | 35.4 | 5.9 |
| CR002177 | n/a | n/a |
| CR002178 | 25.9 | 3.2 |
| CR002179 | n/a | n/a |
| CR002180 | 57.6 | 6.4 |
| CR002181 | 63.8 | 3.9 |
| CR002182 | n/a | n/a |
| CR002183 | n/a | n/a |
| CR002184 | 30.0 | 4.0 |
| CR002185 | n/a | n/a |
| CR002186 | n/a | n/a |
| CR002187 | 51.6 | 2.9 |
| CR002188 | 35.8 | 8.7 |
| CR002189 | 62.7 | 6.0 |
| CR002190 | n/a | n/a |
| CR002191 | 37.4 | 7.4 |
| CR002192 | n/a | n/a |
| CR002193 | 6.1 | 1.7 |
| CR002194 | n/a | n/a |
| CR002195 | 60.0 | 9.4 |
| CR002196 | 42.9 | 13.7 |
| CR002197 | 52.2 | 2.8 |
| CR002198 | n/a | n/a |
| CR002199 | n/a | n/a |
| CR002200 | 4.3 | 1.0 |
| CR002201 | 1.8 | 0.3 |
| CR002202 | 2.6 | 0.3 |
| CR002203 | 50.7 | 5.3 |
| CR002204 | n/a | n/a |
| CR002205 | n/a | n/a |
| CR002206 | n/a | n/a |
| CR002207 | n/a | n/a |
| CR002208 | n/a | n/a |
| CR002209 | n/a | n/a |
| CR002210 | n/a | n/a |
| CR002211 | n/a | n/a |
| CR002212 | n/a | n/a |
| CR002213 | n/a | n/a |
| CR002214 | n/a | n/a |
| CR002215 | 51.7 | 8.9 |
| CR002216 | 14.1 | 8.3 |
| CR002217 | 50.1 | 10.5 |
| CR002218 | 45.0 | 19.4 |
| CR002219 | 30.7 | 14.7 |
| CR002220 | 44.7 | 13.3 |
| CR002221 | n/a | n/a |
| CR002222 | 64.0 | 3.9 |
| CR002223 | n/a | n/a |
| CR002224 | n/a | n/a |
| CR002225 | n/a | n/a |
| CR002226 | n/a | n/a |
| CR002227 | n/a | n/a |
| CR002228 | 10.4 | 5.6 |
| CR002229 | n/a | n/a |

| | | |
|---|---|---|
| n/a = not assessed | | |

Aftter the experiment above, the same guide RNA molecules were tested again in triplicate in both HEK-293-Cas9 cells and in CD34+ cells using a newly designed set of primers for the NGS analysis. The results are reported below in Table 12B.

**Table 12B: Bcl11a +55 enhancer region crRNA screen results in HEK-293-Cas9 Cells and in CD34+ HSPCs (by NGS, n=3).**

| **ID** | **Editing % HEK-293-Cas9 Cells (n=3)** | **Editing % CD34+ HSPCs (n=3)** |
|---|---|---|
| CR002142 | 2.1 | 2.07 |
| CR002143 | 39.6 | 2.31 |
| CR002144 | 74.3 | 4.70 |
| CR002145 | 72.1 | 11.46 |
| CR002146 | 77.2 | 5.18 |
| CR002147 | 37.3 | 3.13 |
| CR002148 | 34.6 | 2.02 |
| CR002149 | 40.7 | 3.16 |
| CR002150 | 64.4 | 4.01 |
| CR002151 | 70.5 | 5.05 |
| CR002152 | 63.6 | 6.59 |
| CR002153 | 42.2 | 2.82 |
| CR002154 | 71.0 | 17.89 |
| CR002155 | 60.3 | 6.01 |
| CR002156 | 62.3 | 9.43 |
| CR002157 | 38.5 | 4.72 |
| CR002158 | 12.4 | 2.16 |
| CR002159 | 14.8 | 3.64 |
| CR002160 | 72.8 | 7.97 |
| CR002161 | 74.5 | 18.12 |
| CR002162 | 64.8 | 4.53 |
| CR002163 | 86.7 | 13.92 |
| CR002164 | 3.2 | 10.23 |
| CR002165 | 78.3 | 9.74 |
| CR002166 | 67.2 | 7.78 |
| CR002167 | 51.6 | 29.26 |
| CR002168 | 29.9 | 4.15 |
| CR002169 | 80.2 | 9.11 |
| CR002170 | 83.0 | 16.17 |
| CR002171 | 67.8 | 4.83 |
| CR002172 | 64.3 | 7.59 |
| CR002173 | 80.7 | 6.00 |
| CR002174 | 78.2 | 26.89 |
| CR002175 | 79.4 | 27.08 |
| CR002176 | 39.5 | 5.14 |
| CR002177 | 66.7 | 21.45 |
| CR002178 | 52.5 | 12.74 |
| CR002179 | 83.1 | 38.88 |
| CR002180 | 51.3 | 5.46 |
| CR002181 | 54.0 | 12.54 |
| CR002182 | 67.9 | 28.01 |
| CR002183 | 30.3 | 3.52 |
| CR002184 | 67.1 | 21.31 |
| CR002185 | 65.6 | 8.18 |
| CR002186 | 58.9 | 8.16 |
| CR002187 | 70.2 | 10.05 |
| CR002188 | 56.6 | 9.92 |
| CR002189 | 71.9 | 31.14 |
| CR002190 | 27.4 | 6.84 |
| CR002191 | 70.0 | 17.54 |
| CR002192 | 62.6 | 6.92 |
| CR002193 | 1.6 | 0.67 |
| CR002194 | 82.5 | 24.52 |
| CR002195 | 64.4 | 36.32 |
| CR002196 | 2.1 | 0.96 |
| CR002197 | 63.8 | 37.29 |
| CR002198 | 67.8 | 16.45 |
| CR002199 | 61.3 | 9.78 |
| CR002200 | 3.1 | 1.10 |
| CR002201 | 1.2 | 0.84 |
| CR002202 | 2.8 | 0.82 |
| CR002203 | 49.9 | 0.84 |
| CR002204 | 43.2 | 17.48 |
| CR002205 | 60.6 | 11.06 |
| CR002206 | 48.5 | 9.02 |
| CR002207 | 49.4 | 12.29 |
| CR002208 | 61.7 | 16.56 |
| CR002209 | 61.2 | 9.36 |
| CR002210 | 57.6 | 27.77 |
| CR002211 | 45.4 | 30.93 |
| CR002212 | 56.0 | 31.55 |
| CR002213 | 34.4 | 10.40 |
| CR002214 | 11.1 | 1.77 |
| CR002215 | 49.3 | 40.23 |
| CR002216 | 22.1 | 1.80 |
| CR002217 | 52.3 | 11.29 |
| CR002218 | 73.7 | 11.17 |
| CR002219 | 34.2 | 7.94 |
| CR002220 | 64.8 | 8.63 |
| CR002221 | 20.7 | 5.66 |
| CR002222 | 78.9 | 30.01 |
| CR002223 | 8.2 | 1.21 |
| CR002224 | 7.8 | n.d |
| CR002225 | n.d | n.d |
| CR002226 | 2.6 | n.d |
| CR002227 | n.d | n.d |
| CR002228 | 23.2 | 3.65 |
| CR002229 | 39.0 | n.d |

| | | |
|---|---|---|
| n.d. = not determined | | |

**Table 13: French HPFH region crRNA 1° Screen results in HEK-293-Cas9 Cells and in CD34+ HSPCs (by NGS, n=3).**

| **Targeting Domain ID** | **Average Edit % HEK-293-Cas9 (n=3)** | **Average Edit % CD34+ HSPCs (n=3)** | **StDev (%) for % Edit in CD34+ HSPCs** |
|---|---|---|---|
| CR001016 | 33.1 | 24.0 | 1.2 |
| CR001017 | 30.4 | 19.5 | 1.5 |
| CR001018 | 46.5 | 53.0 | 3.4 |
| CR001019 | 45.9 | 57.6 | 2.3 |
| CR001020 | 62.1 | 47.7 | 9.3 |
| CR001021 | 29.1 | 34.2 | 1.8 |
| CR001022 | 66.1 | 66.2 | 2.3 |
| CR001023 | 27.3 | 46.0 | 6.1 |
| CR001024 | 62.5 | 73.4 | 6.9 |
| CR001025 | 25.5 | 24.5 | 1.6 |
| CR001026 | 61.4 | 72.1 | 2.9 |
| CR001027 | 23.3 | 33.2 | 2.2 |
| CR001028 | 47.7 | 72.1 | 4.9 |
| CR001029 | 53.9 | 61.2 | 5.7 |
| CR001030 | 49.2 | 49.9 | 29.0 |
| CR001031 | 42.7 | 65.0 | 9.3 |
| CR001032 | 67.6 | 50.1 | 9.7 |
| CR001033 | 15.3 | 44.5 | 3.2 |
| CR001034 | 28.5 | 52.9 | 2.7 |
| CR001035 | 16.9 | 28.5 | 16.4 |
| CR001036 | 28.0 | 68.3 | 3.7 |
| CR001037 | 52.7 | 53.2 | 4.2 |
| CR001038 | 1.9 | 23.7 | 20.6 |
| CR001039 | 2.2 | 35.8 | 2.8 |
| CR001040 | nd | n/a | 0.0 |
| CR001041 | nd | n/a | 0.0 |
| CR001042 | 2.7 | n/a | 0.0 |
| CR001043 | nd | n/a | 0.0 |
| CR001044 | 4.9 | 1.5 | 0.4 |
| CR001045 | 38.1 | 31.2 | 2.0 |
| CR001046 | 18.4 | 28.4 | 3.9 |
| CR001047 | nd | n/a | 0.0 |
| CR001048 | 1.6 | 0.2 | 0.3 |
| CR001049 | nd | n/a | 0.0 |
| CR001050 | nd | n/a | 0.0 |
| CR001051 | nd | n/a | 0.0 |
| CR001052 | nd | n/a | 0.0 |
| CR001053 | 5.8 | 4.4 | 0.7 |
| CR001054 | 9.0 | 4.1 | 0.4 |
| CR001055 | 5.2 | 0.1 | 0.1 |
| CR001056 | 7.7 | 5.0 | 0.3 |
| CR001057 | 25.2 | 7.8 | 0.8 |
| CR001058 | 41.6 | 29.5 | 6.7 |
| CR001059 | 5.2 | 4.9 | 0.1 |
| CR001060 | 43.5 | 18.7 | 5.2 |
| CR001061 | 21.7 | 4.7 | 0.8 |
| CR001062 | 12.2 | 5.2 | 1.1 |
| CR001063 | 16.6 | 21.5 | 1.6 |
| CR001064 | 6.0 | 4.2 | 0.7 |
| CR001065 | 43.0 | 20.0 | 1.4 |
| CR001066 | 11.1 | 4.2 | 0.4 |
| CR001067 | 25.7 | 7.9 | 3.5 |
| CR001068 | 11.3 | 5.9 | 1.6 |
| CR001069 | 15.1 | 2.9 | 0.2 |
| CR001070 | 38.2 | 11.1 | 3.1 |
| CR001071 | 34.6 | 8.5 | 0.2 |
| CR001072 | 22.8 | 3.5 | 0.4 |
| CR001073 | 45.2 | 18.1 | 4.1 |
| CR001074 | 40.1 | 38.1 | 2.5 |
| CR001075 | 53.0 | 29.2 | 1.8 |
| CR001076 | 25.6 | 1.8 | 0.1 |
| CR001077 | 8.9 | 12.2 | 0.4 |
| CR001078 | 10.9 | 17.7 | 3.3 |
| CR001079 | 18.3 | 7.8 | 0.9 |
| CR001080 | 21.3 | 17.4 | 5.6 |
| CR001081 | 22.0 | 21.4 | 0.5 |
| CR001082 | 5.4 | 8.5 | 0.6 |
| CR001083 | 12.3 | 11.6 | 0.5 |
| CR001084 | 25.4 | 9.5 | 4.5 |
| CR001085 | 18.3 | 12.4 | 1.7 |
| CR001086 | 25.3 | 4.4 | 2.0 |
| CR001087 | 16.4 | 6.9 | 0.9 |
| CR001088 | 9.6 | 18.4 | 1.9 |
| CR001089 | nd | 15.8 | 12.9 |
| CR001090 | 16.9 | 30.1 | 3.5 |
| CR001091 | 11.4 | 0.8 | 0.5 |
| CR001092 | 17.4 | 17.0 | 9.8 |
| CR001093 | 23.0 | 13.0 | 4.5 |
| CR001094 | 10.0 | 24.9 | 5.4 |
| CR001095 | 22.1 | 34.8 | 3.6 |
| CR001096 | 29.8 | 20.4 | 2.0 |
| CR001097 | 17.9 | 20.5 | 0.5 |
| CR001098 | 49.8 | 34.7 | 5.1 |
| CR001099 | 24.7 | 39.9 | 1.8 |
| CR001100 | nd | 33.2 | 2.8 |
| CR001101 | 16.9 | 24.7 | 2.1 |
| CR001102 | 29.4 | 13.9 | 0.7 |
| CR001103 | 9.9 | 8.6 | 2.2 |
| CR001104 | 11.3 | 3.3 | 0.7 |
| CR001105 | 5.0 | 3.6 | 0.9 |
| CR001106 | 15.7 | 13.1 | 1.6 |
| CR001107 | 31.2 | 8.5 | 4.5 |
| CR001108 | 16.1 | 3.2 | 0.5 |
| CR001109 | 12.1 | 4.6 | 2.1 |
| CR001110 | 23.5 | 6.0 | 3.0 |
| CR001111 | 12.3 | 2.6 | 1.3 |
| CR001132 | 18.7 | 3.4 | 0.4 |
| CR001133 | 35.4 | 27.0 | 4.2 |
| CR001134 | 12.3 | 0.3 | 0.2 |
| CR001135 | 57.8 | 16.2 | 2.7 |
| CR001136 | 7.1 | 1.7 | 0.5 |
| CR001137 | 31.2 | 6.2 | 2.7 |
| CR001138 | 45.4 | 9.6 | 1.2 |
| CR001139 | nd | 3.3 | 0.3 |
| CR001140 | 28.7 | 11.2 | 1.2 |
| CR001141 | 3.6 | 1.5 | 0.5 |
| CR001142 | 56.7 | 9.7 | 1.2 |
| CR001143 | 58.4 | 4.6 | 1.3 |
| CR001144 | 23.2 | 4.3 | 2.5 |
| CR001145 | 4.9 | 0.1 | 0.0 |
| CR001146 | 38.2 | 0.2 | 0.0 |
| CR001147 | 16.0 | 0.1 | 0.1 |
| CR001148 | 22.3 | 3.0 | 1.6 |
| CR001149 | 19.4 | 6.0 | 3.9 |
| CR001150 | 30.8 | 30.4 | 17.9 |
| CR001151 | 35.8 | 0.8 | 0.2 |
| CR001152 | 24.6 | 0.3 | 0.1 |
| CR001153 | 5.4 | 0.3 | 0.4 |
| CR001154 | 6.3 | 3.6 | 2.1 |
| CR001155 | nd | 2.1 | 0.6 |
| CR001156 | 19.6 | 4.8 | 0.8 |
| CR001157 | 16.4 | 2.7 | 0.6 |
| CR001158 | 36.5 | 5.1 | 3.2 |
| CR001159 | 42.9 | 7.7 | 1.0 |
| CR001160 | 32.5 | 8.5 | 1.9 |
| CR001161 | 20.5 | 10.3 | 1.9 |
| CR001162 | 35.0 | 30.5 | 4.5 |
| CR001163 | 9.9 | 3.3 | 0.5 |
| CR001164 | 23.3 | 14.4 | 8.8 |
| CR001165 | 5.5 | 1.5 | 0.4 |
| CR001166 | 49.8 | 27.1 | 4.4 |
| CR001167 | 13.8 | 6.0 | 0.8 |
| CR001168 | nd | 6.4 | 4.7 |
| CR001169 | 43.9 | n/a | 0.0 |
| CR001170 | 28.5 | 10.7 | 6.4 |
| CR001171 | 37.5 | 2.7 | 2.6 |
| CR001172 | 41.5 | 14.8 | 3.5 |
| CR001173 | 49.5 | 16.7 | 3.9 |
| CR001174 | 36.6 | 4.4 | 0.7 |
| CR001175 | 17.0 | 5.3 | 1.7 |
| CR001176 | 7.7 | 3.6 | 2.8 |
| CR001177 | 4.0 | 4.1 | 2.3 |
| CR001178 | nd | 27.0 | 15.6 |
| CR001179 | 59.0 | 27.9 | 1.5 |
| CR001180 | nd | 8.5 | 5.3 |
| CR001181 | 30.9 | 5.7 | 0.8 |
| CR001182 | 34.5 | 13.6 | 7.8 |
| CR001183 | 6.9 | 0.4 | 0.3 |
| CR001184 | 15.7 | 5.2 | 2.7 |
| CR001185 | 18.2 | 7.6 | 1.2 |
| CR001186 | 12.0 | 5.0 | 0.6 |
| CR001187 | 8.3 | 4.1 | 0.3 |
| CR001188 | 16.8 | 7.1 | 2.0 |
| CR001189 | 13.3 | 3.7 | 1.3 |
| CR001190 | nd | 5.6 | 1.1 |
| CR001191 | 39.5 | 4.8 | 1.0 |
| CR001192 | 27.5 | 3.9 | 1.3 |
| CR001193 | 17.1 | 1.5 | 1.9 |
| CR001194 | 17.8 | 5.9 | 4.1 |
| CR001195 | nd | 16.4 | 9.5 |
| CR001196 | 5.8 | 2.2 | 0.7 |
| CR001197 | 32.4 | 8.1 | 3.1 |
| CR001198 | 28.4 | 18.2 | 5.8 |
| CR001199 | 40.8 | 7.9 | 4.0 |
| CR001200 | 24.4 | 1.2 | 0.7 |
| CR001201 | 1.7 | 0.3 | 0.1 |
| CR001202 | 46.0 | 39.3 | 22.7 |
| CR001203 | 11.9 | 3.1 | 1.6 |
| CR001204 | 1.2 | 0.8 | 0.1 |
| CR001205 | 3.0 | 0.2 | 0.1 |
| CR001206 | 3.9 | 0.5 | 0.2 |
| CR001207 | 7.6 | 2.4 | 1.2 |
| CR001208 | 5.5 | 1.5 | 0.7 |
| CR001209 | 4.4 | 1.7 | 0.2 |
| CR001210 | 10.8 | 3.9 | 2.5 |
| CR001211 | 6.8 | 3.4 | 2.0 |
| CR001212 | 32.7 | 11.1 | 6.5 |
| CR001213 | 21.6 | 6.2 | 1.5 |
| CR001214 | 28.4 | 5.7 | 0.8 |
| CR001215 | 9.0 | 1.5 | 0.5 |
| CR001216 | nd | 2.5 | 1.4 |
| CR001217 | nd | n/a | 0.0 |
| CR001218 | 7.7 | 4.1 | 1.0 |
| CR001219 | 40.0 | 20.3 | 0.6 |
| CR001220 | 33.8 | n/a | 0.0 |
| CR001221 | 41.3 | n/a | 0.0 |
| CR001222 | 39.1 | 18.9 | 10.9 |
| CR001223 | 35.9 | n/a | 0.0 |
| CR001224 | 34.9 | 16.5 | 7.1 |
| CR001225 | 55.0 | 24.6 | 1.1 |
| CR001226 | 34.1 | 4.7 | 4.1 |
| CR001227 | 46.4 | 6.4 | 0.6 |
| CR003027 | 50.2 | 4.5 | nd |
| CR003028 | 3.0 | 2.2 | nd |
| CR003029 | 14.4 | 3.4 | nd |
| CR003030 | 20.2 | 5.8 | nd |
| CR003031 | 31.3 | 9.7 | nd |
| CR003032 | 15.1 | 4.4 | nd |
| CR003033 | 46.5 | 25.2 | nd |
| CR003034 | 55.2 | 11.8 | nd |
| CR003035 | 42.4 | 15.9 | nd |
| CR003036 | 17.4 | 6.1 | nd |
| CR003037 | 38.6 | 19.6 | nd |
| CR003038 | 47.1 | 24.6 | nd |
| CR003039 | 53.8 | 21.1 | nd |
| CR003040 | 35.9 | 6.1 | nd |
| CR003041 | 23.7 | 9.9 | nd |
| CR003042 | 35.5 | 8.7 | nd |
| CR003043 | 24.4 | 4.3 | nd |
| CR003044 | 54.1 | 16.2 | nd |
| CR003045 | 33.7 | 6.6 | nd |
| CR003046 | 34.8 | 3.6 | nd |
| CR003047 | 46.9 | 9.8 | nd |
| CR003048 | 12.6 | 1.7 | nd |
| CR003049 | 24.4 | 4.9 | nd |
| CR003050 | 19.6 | 2.9 | nd |
| CR003051 | 15.4 | 1.8 | nd |
| CR003052 | 15.7 | 7.8 | nd |
| CR003053 | 10.8 | 0.9 | nd |
| CR003054 | 16.7 | 2.9 | nd |
| CR003055 | 17.3 | 4.4 | nd |
| CR003056 | 46.3 | 19.3 | nd |
| CR003057 | 40.9 | 11.3 | nd |
| CR003058 | 40.0 | 8.9 | nd |
| CR003059 | 23.7 | <2% | nd |
| CR003060 | 16.8 | <2% | nd |
| CR003061 | 27.6 | <2% | nd |
| CR003062 | 3.9 | <2% | nd |
| CR003063 | 19.8 | 2.5 | nd |
| CR003064 | 19.6 | <2% | nd |
| CR003065 | 42.3 | 0.1 | nd |
| CR003066 | 36.5 | 0.0 | nd |
| CR003067 | 53.1 | 0.1 | nd |
| CR003068 | 11.5 | <2% | nd |
| CR003069 | 42.7 | 0.0 | nd |
| CR003070 | 49.0 | 0.1 | nd |
| CR003071 | 41.8 | <2% | nd |
| CR003072 | 27.2 | <2% | nd |
| CR003073 | 45.1 | 0.1 | nd |
| CR003074 | 40.2 | 0.1 | nd |
| CR003075 | 58.5 | 0.1 | nd |
| CR003076 | 28.5 | <2% | nd |
| CR003077 | 44.9 | 0.0 | nd |
| CR003078 | 43.9 | <2% | nd |
| CR003079 | 31.1 | <2% | nd |
| CR003080 | 41.1 | <2% | nd |
| CR003081 | 29.4 | <2% | nd |
| CR003082 | 30.8 | <2% | nd |
| CR003083 | 26.5 | 0.0 | nd |
| CR003084 | 32.3 | <2% | nd |
| CR003085 | 24.0 | 0.1 | nd |
| CR003086 | 40.8 | 10.8 | nd |
| CR003087 | 51.0 | 45.0 | nd |
| CR003088 | 25.5 | 9.7 | nd |
| CR003089 | 26.0 | 11.5 | nd |
| CR003090 | 15.2 | <2% | nd |
| CR003091 | 26.4 | 5.5 | nd |
| CR003092 | 4.9 | 2.1 | nd |
| CR003093 | 16.7 | 2.9 | nd |
| CR003094 | 39.5 | 4.7 | nd |
| CR003095 | 12.0 | 1.7 | nd |
| CR003096 | 22.9 | 4.0 | nd |

| | | | |
|---|---|---|---|
| n/a = not assayed; nd = not determined | | | |

### Genome Editing at the BCL11a Enhancer Site

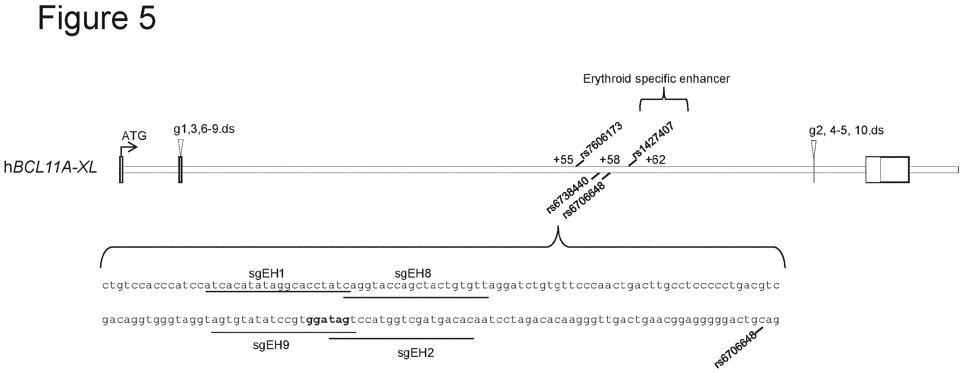

Synthetic sgRNA molecules containing targeting domains specific for genomic DNA within the +58 or +62 erythroid enhancer region of BCL11a were ordered. Figure 5 shows the genomic DNA targeted by the sgRNAs, which were named sgEH1 through sgEH9.
sgEH1 Targeting Domain (CR00276): AUCACAUAUAGGCACCUAUC (SEQ ID NO: 212)
sgEH2 Targeting Domain (CR00275): CACAGUAGCUGGUACCUGAU (SEQ ID NO: 211)
sgEH8 Targeting Domain (CR00273): CAGGUACCAGCUACUGUGUU (SEQ ID NO: 209)
sgEH9 (CR00277) Targeting Domain: UGAUAGGUGCCUAUAUGUGA (SEQ ID NO: 213)

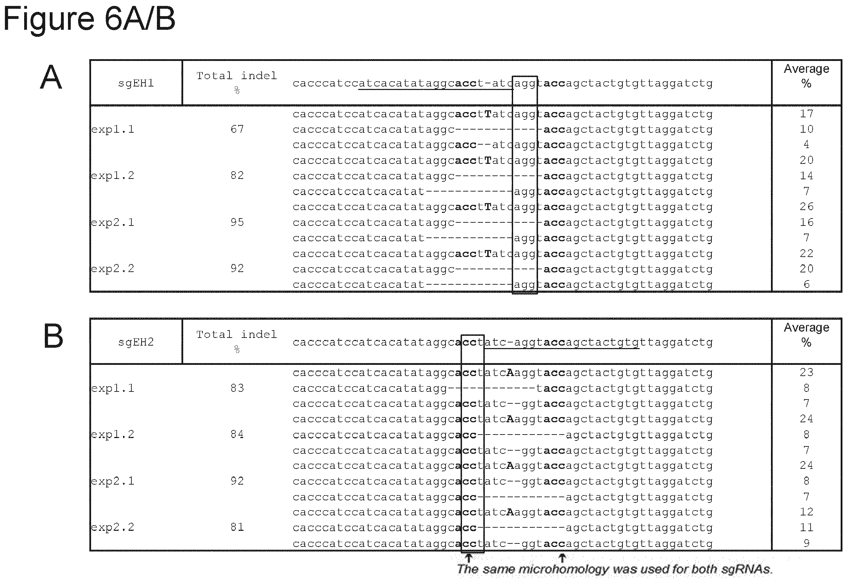

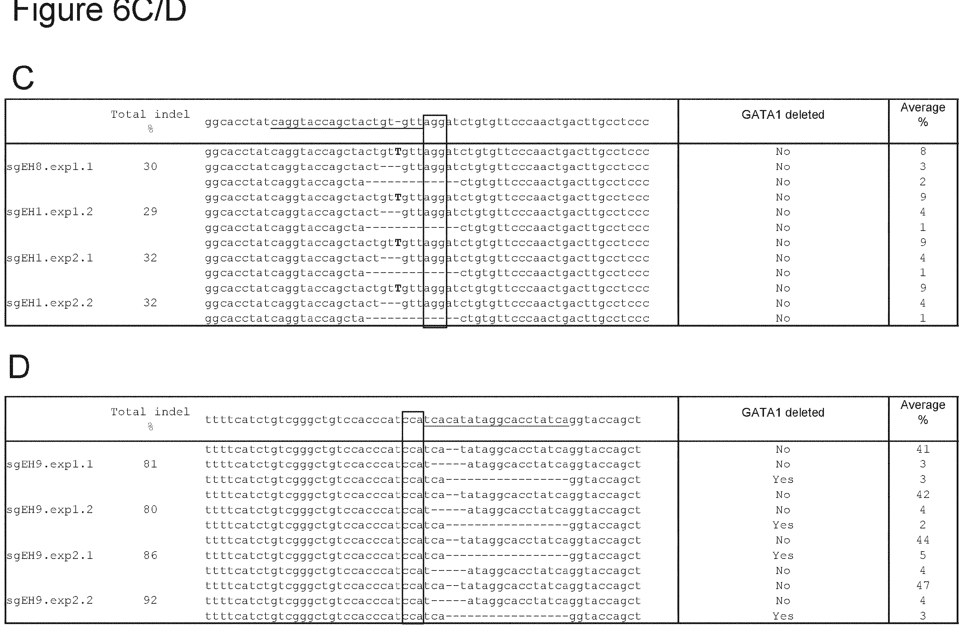

RNPs containing sgEH[X] precomplexed with Cas9 were introduced into CD34+ bone marrow cells (Bone Marrow CD34+ cells ordered from Lonza, Cat # 2M-101C , Lot# 466977 , 30y, F, B (96.8%)) using electroporation (NEON electroporator). Cutting was determined in CD34+ stem cells using T7E1 and NGS. The results are summarized in Figure 6A-D, which shows overall indel formation across four experiments (two experiments from two different donors), as well as the top indel patterns. sgEH1, 2 and 9 were able to direct cutting with high efficiency.

It was surprisingly found that across multiple experiments, including those using cells from different donors, the indel pattern remained constant (See Figure 6A-D). That is, each gRNA gave a consistent pattern of indel formation. As well, deletions appeared to correlate with regions of microhomology near the cutting site.

To further investigate this phenomenon, gRNAs to the BCL11a locus were designed at tested using different biological samples as well as different delivery methods, and gRNA scaffolds. For this experiment, gRNAs with the following targeting domains were used:
G7 (also referred to as g7) Targeting domain: GUGCCAGAUGAACUUCCCAU (e.g., the 3' 20 nt of SEQ ID NO: 1191)
G8 (also referred to as g8) Targeting domain: CACAAACGGAAACAAUGCAA (e.g., the 3' 20 nt of SEQ ID NO: 1186)

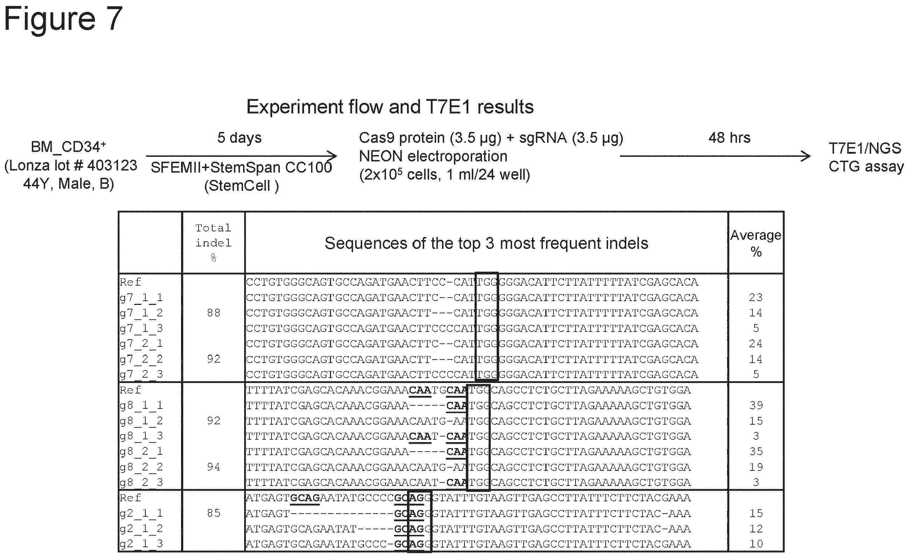

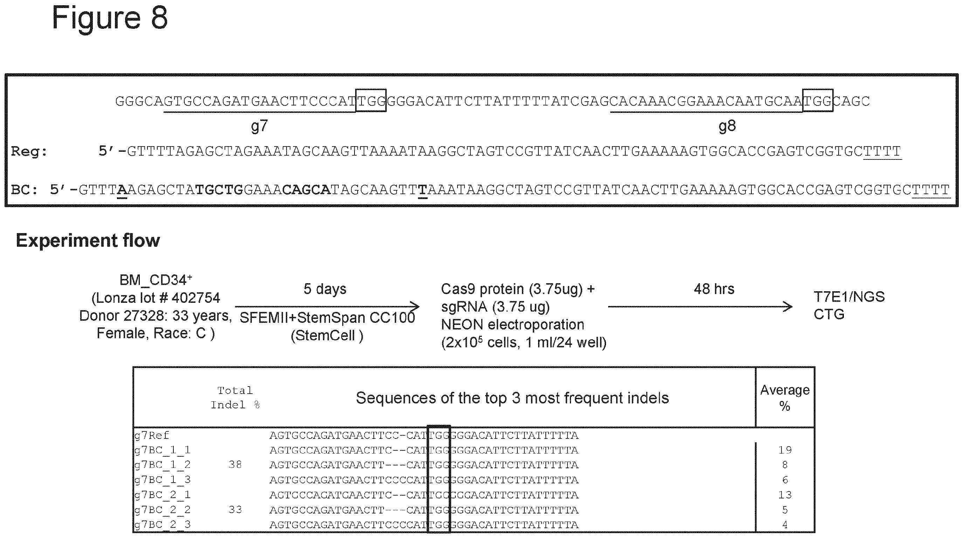

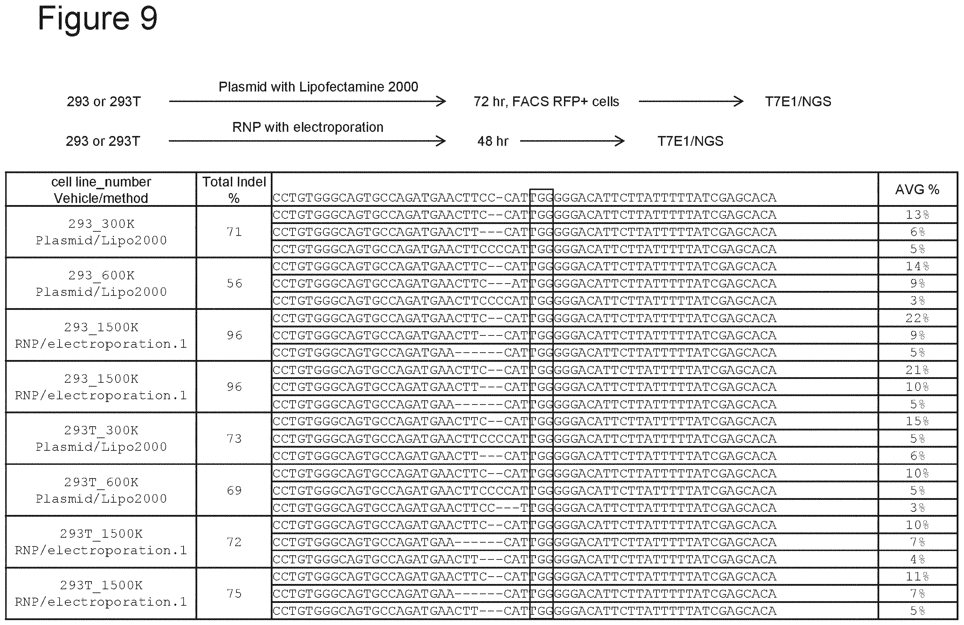

In a first experiment, g7 and g8 were tested across two different biological samples. As shown in Figure 7, the pattern of top 3 most prevalent indels for each gRNA was identical. Next, gRNA molecules comprising the G7 and G8 targeting domains were tested with a different tracrRNA component. In the previous experiments, SEQ ID NO: 6601, followed by -UUUU, was directly appended to the gRNA targeting domain to form the sgRNA. In the next set of experiments, SEQ ID NO: 6604, followed by -UUUU, was directly appended to the gRNA targeting domains of g7 and g8 to create sgRNA molecules with a different scaffold (gRNAs called g7BC or g8BC). As shown in Figure 8, the indel pattern remained constant even when different sgRNA scaffolds are used. Finally, the indel pattern was compared using different delivery methods. Delivery of g7 to 293 cells via either RNP electroporation or by delivery of a g7-encoding plasmid via lipofectamine 2000 was investigated and the results reported in Figure 9. As shown, the indel pattern remained the same. Together, these results strongly suggest that indel pattern formation is sequence-dependent. gRNA molecules that target sequences where cutting results in large deletions and/or frameshift deletions may be beneficial for loss of function.

### Excision with Multiple Guides

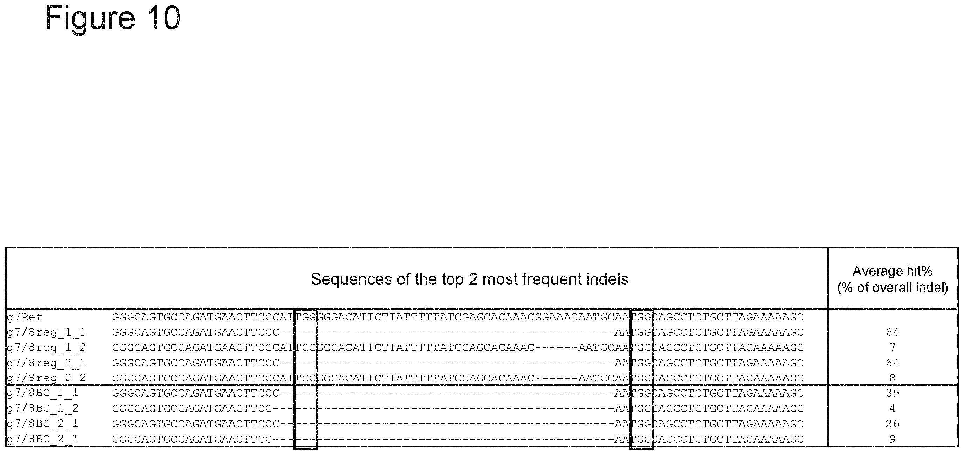

G7 and g8, above, target genomic DNA at proximal sites (PAM sequences are within 50 nucleotides of each other within the BCL11a gene). To investigate the effect of simultanesously targeting two proximal sites, CD34+ cells were transfected with RNPs comprising gRNAs with the targeting domain of G7 as well as RNPs comprising gRNAs with the targeting domain of G8. Cutting efficiency and indel pattern was assessed by NGS. As shown in Figure 10, the primary indel is a delection of the nucleotides between the two gRNA binding sites. These results demonstrate that a plurality, e.g., 2, guides that bind in proximity may be used to effect an excision of the DNA located between the two binding sites.

### Genome Editing in CD34+ Stem Cells

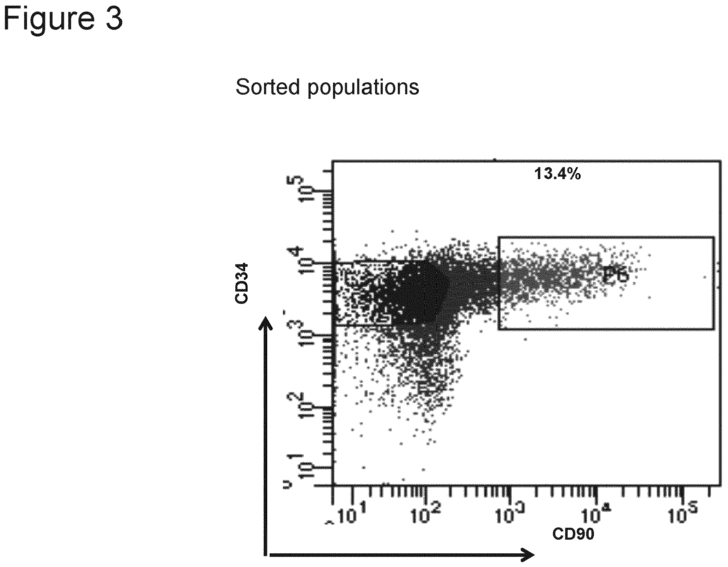

Genome editing at the BCL11a locus was demonstrated in CD34+ primary hematopoietic stem cells. Briefly, CD34+ cells were isolated from G-CSF mobilized peripheral blood from adult donors (AllCells) using immunoselection (Miltenyi) according to the manufacturer's instructions. Cell gating is shown in Figure 3. Cells were aliquotted and cryopreserved. Thawed cells were seeded at 2×10⁵/ml in SFEM (StemCell Technologies) + IX antibiotic/antimycotic + 50 ng/ml each cytokine (TPO, FLT3L, IL6 and SCF) + 500 nM compound 4 and cultured for 5 days at 37C, 5% CO2 in a humidified incubator. Cell concentration after 5 days was 1×10⁶/ml.

Preincubated RNP complex consisting of 150 ng each Cas9 (Genscript #265425-7) and sgRNA (TriLink, comprising the sgBCL11A_7 Targeting Domain (i.e., the targeting domain of g7): GTGCCAGATGAACTTCCCATGTTTAAGAGCTATGCTGGAAACAGCATAGCAAGTTTAAAT AAGGCTAGTCCGTTATCAACTTGAAAAAGTGGCACCGAGTCGGTGCTTTT) (SEQ ID NO: 2000) was added to 2×10⁵ cells and electroporated on the Neon system (Lifetechnologies) with pulse parameters: 1600 V, 20ms, 1 pulse, according to manufacturer's protocols. Duplicate electroporations were performed. Mock electroporations were also performed in the absence of RNP complex. After electroporation, cells were seeded into 1 ml media to recover overnight.

The day following electroporation, 1/20 of the unsorted culture was seeded in SFEM (StemCell Technologies) + IX antibiotic/antimycotic + 50 ng/ml each TPO, FLT3L, IL6, SCF, IL3 and GCSF for 6 days. The remaining cells were stained with antibodies to human CD34 and human CD90 and sorted by flow cytometry for CD34+CD90+ and CD34+CD90- populations. CD34+CD90+ cells are known to contain hematopoietic stem cells, as defined by long-term multi-lineage engraftment in immunodeficient mice (see Majeti et al. 2007 Cell Stem Cell 1, 635-645). Cell populations selected for sorting are indicated by the gates in the representative dot plot of Figure 3. Sorted cells were cultured in SFEM (StemCell Technologies) + IX antibiotic/antimycotic + 50 ng/ml each TPO, FLT3L, IL6, SCF, IL3 and GCSF for 6 days, at which point all cultures were harvested for analysis of genome editing.

Genomic DNA was purified from harvested cells and the targeted region was amplified with the following primers: BCL11A-7 F, gcttggctacagcacctctga; BCL11A-7 R, ggcatggggttgagatgtgct. PCR product was denatured and reannealed, followed by incubation with T7E1 (New England Biolabs) as per the manufacturer's recommendations. The reaction was then analyzed by agarose gel electrophoresis (Figure 4). The upper band represents uncleaved homoduplex DNA and the lower bands indicate cleavage products resulting from heteroduplex DNA. The far left lane is a DNA ladder. Band intensity was calculated by peak integration of unprocessed images by ImageJ software. % gene modification (indel) was calculated as follows: % gene modification = 100 x (1 - (1- fraction cleaved)^{1/2}) , and is indicated below each corresponding lane of the gel.

The results are shown in Figure 4. Gene editing efficiency in the hematopoietic stem cell-containing CD34+CD90+ population was equivalent to CD34+CD90- cells or unsorted. These experiments demonstrate that gene editing can be accomplished in CD34+ HSPCs and CD34+CD90+ cells that are further enriched in HSCs.

### Example 3. BCL11A erythroid enhancer editing in Hematopoietic Stem and Progenitor Cells (HSPCs) using CRISPR-Cas9 for derepression of fetal globin expression in adult erythroid cells

### Methods:

Human CD34⁺ cell culture. Human bone marrow CD34⁺ cells were purchased from either AllCells (Cat#: ABM017F) or Lonza (Cat#: 2M-101C) and expanded for 2 to 3 days using StemSpan SFEM (StemCell Technologies; Cat no. 09650) supplemented with 50 ng/mL of thrombopoietin (Tpo, Peprotech; Cat# 300-18 ), 50 ng/mL of human Flt3 ligand ( Flt-3L, Peprotech; Cat# 300-19), 50ng/mL of human stem cell factor (SCF, Peprotech; Cat# 300-07), human interleukin-6 (IL-6, Peprotech; Cat# 200-06), 1% L-glutamine; 2% penicillin/streptomycin, and Compound 4 (0.75 µM). After 2 to 3 days of expansion, the culture had expanded 2- to 3-fold relative to starting cell numbers purchased from AllCells and 1- to 1.5-fold from Lonza. These culture conditions were used for all CD34+ expansion steps, including both before and after introduction of the CRISPR/Cas systems as described herein.

Assembly of Cas9 and guide RNA ribonucleoprotein (RNP) complexes, preparation of HSC, and electroporation of RNP into HSC. Cas9-guide RNA ribonucleoprotein complexes (RNPs) were prepared immediately prior to electroporation. For formation of RNP using dual guide RNAs (dgRNAs), 3 µg of each of crRNA (in 2.24 µL) and tracr (in 1.25 µL) are first denatured at 95°C for 2 min in separate tubes and then cooled to room temperature. For preparation of Cas9 protein, 7.3 µg of CAS9 protein (in 1.21 µL) was mixed with 0.52 µL of 5 x CCE buffer (20 mM HEPES, 100 mM KCL, 5 mM MgCL2, 5% Glycerol and freshly added 1 mM DTT). Tracr was first mixed with the Cas9 preparation and incubated at 37°C for 5 min. The crRNA was then added to Tracr/CAS9 complexes and incubated for 5 min at 37°C. The HSC were collected by centrifugation at 200 x g for 15 min and resuspended in T buffer that comes with Neon electroporation kit (Invitrogen; Cat#: MPK1096) at a cell density of 2 x 10⁷/mL. The RNP mixed with 12 µL of cells by pipetting up and down 3 times gently. To prepare single guide RNA (sgRNA) and Cas9 complexes, 2.25 µg sgRNA (in 1.5 µL) was mixed with 2.25 µg Cas9 protein (in 3 µL) and incubated at RT for 5 min. The sgRNA/Cas9 complex was then mixed with 10.5 µL of 2 × 10⁷ /mL cells by pipetting up and down several times gently and incubated at RT for 2 min. The RNP/cell mixture (10 µL) was transferred into Neon electroporation probe. The electroporation was performed with Neon transfection system (Invitrogen; MPK5000S) using 1700 volts/20 milliseconds and 1 pulse. After electroporation, the cells were transferred into 0.5 mL pre-warmed StemSpan SFEM medium supplemented with growth factors and cytokines as described above and cultured at 37°C for 2 days.

Genomic DNA preparation. Genomic DNA was prepared from edited and unedited HSC at 48 hours post-electroporation. The cells were lysed in 10 mM Tris-HCL, pH 8.0; 0.05% SDS and freshly added Protease K of 25 µg/mL and incubated at 37°C for 1 hour followed by additional incubation at 85°C for 15 min to inactivate protease K.

T7E1 assay. To determine editing efficiency of HSCs, T7E1 and assay was performed. PCR was performed using Phusion Hot Start II High-Fidelity kit (Thermo Scientific; Cat#: F-549L) with the following cycling condition: 98°C for 30"; 35 cycles of 98°C for 5", 68°C for 20', 72°C for 30"; 72°C for 5 min. The following primers were used: forward primer: 5'-AGCTCCAAACTCTCAAACCACAGGG-3' (SEQ ID NO: 2001) and reverse primer: 5'-TACAATTTTGGGAGTCCACACGGCA-3' (SEQ ID NO: 2002). The PCR products were denatured and re-annealed using the following condition: 95°C for 5 min, 95-85°C at -2°C/s, 85-25°C at - 0.1°C/s, 4°C hold. After annealing, 1 µL of mismatch-sensitive T7 E1 nuclease (NEB, Cat# M0302L) was added into 10 µL of PCR product above and further incubated at 37°C for 15 min for digestion of hetero duplexes, and the resulting DNA fragments were analyzed by agarose gel (2%) electrophoresis. The editing efficiency was quantified using Image**J** software (http://rsb.info.nih.gov/ij/).

Next generation sequencing (NGS). To determine editing efficiency more precisely and patterns of insertions and deletions (indels), the PCR products were subjected to next generation sequencing (NGS). The PCR were performed in duplicate using Titanium Taq PCR kit (Clontech Laboratories; Cat#: 639210) with the following cycling condition: 98°C for 5 min; 30 cycles of 95°C for 15 seconds, 68°C for 15 seconds, 72°C 1 min; 72°C for 7 min. The following primers were used: forward primer: 5'-AGCTCCAAACTCTCAAACCACAGGG-3' (SEQ ID NO: 2001) and reverse primer: 5'-TACAATTTTGGGAGTCCACACGGCA-3' (SEQ ID NO: 2002). The PCR products were analyzed by 2% agarose gel electrophoresis and submitted for deep sequencing.

Flow cytometry analysis of HSPC culture. The HSPC were subjected to flow cytometry to characterize stem and progenitor cell populations. The percentage of CD34⁺, CD34⁺CD90⁺ cell subsets, prior to and after genome editing was determined on aliquots of the cell cultures. The cells were incubated with anti-CD34 (BD Biosciences, Cat# 555824), anti-CD90 (Biolegend, Cat# 328109) in staining buffer, containing PBS supplemented with 0.5% BSA, at 4°C, in dark for 30 min. The cell viability is determined by 7AAD. The cells were washed with staining buffer and Multicolor FACS analysis was performed on a FACSCanto (Becton Dickinson). Flow cytometry results analyzed using Flowjo and the data were presented as percent CD34⁺, CD34⁺CD90⁺ of the total cell population. Absolute numbers of each cell type population in the culture were calculated from the total number of cells multiplied by the percentage of each population.

*In vitro* erythropoiesis and FACS analysis for HbF containing erythroid cells. The genome edited HSC were subjected to *in vitro* erythroid differentiation 48 hours post-electroporation. Briefly, the edited HSC were cultured in erythroid differentiation medium (EDM) consisting of IMDM (Invitrogen, Cat# 31980-097) supplemented with 330 µg /mL human holo-transferrin (Sigma, Cat# T0665), 10 µg /mL recombinant human insulin (Sigma, Cat# 13536), 2 IU/mL heparin (Sigma, Cat # H3149), 5% human plasma (Sigma, Cat# P9523 ), 3 IU/mL human erythropoietin (R&D, Cat# 287-TC), 1% L-glutamine, and 2% penicillin/streptomycin. During days 0-7 of culture, EDM was further supplemented with 10⁻⁶M hydrocortisone (Sigma, Cat# H0888), 100 ng/mL human SCF (Peprotech, Cat# 300-07), and 5 ng/mL human IL-3 (Peprotech, Cat# 200-03) for 7-days. During days 7-11 of culture, EDM was supplemented with 100 ng/mL of human SCF only. During days 11-21 of culture, EDM has no additional supplements. On days 7, 14 and 21 of the culture, the cells (2-10 x 10⁵) were analyzed by intracellular staining for HbF expression. Briefly, the cells were collected by centrifugation at 350 x g for 5 min and washed once with staining buffer (Biolegend, Cat# 420201). The cells were then fixed with 0.5 mL of fixation buffer (Biolegend, Cat# 420801) and washed three times with 2 mL of 1x intracellular staining permeabilization wash buffer (Biolegend, Cat# 421002) by centrifugation at 350 x g for 5 min. The cells were then incubated with 5 µL of anti-HbF antibody (Life technologies, Cat# MHFH01) in 100 µL of 1x intracellular staining perm wash buffer for 20 min at room temperature. The cells were washed twice with 2 mL of 1x intracellular staining Perm wash buffer and resuspend in 200 µL staining buffer and analyzed on FACS Canto (Becton Dickinson) for HbF expression. The results analyzed using Flowjo and data were presented as % of HbF positive cells (F-cells) in total cell population.

Gene expression assays. ~1 x 10⁶ cells were used to purify RNA using RNeasy mini kit (Qiagen. Cat# 74104). 200 to 400 ng RNA was used for 1^{st} strand synthesize using qScript cDNA synthesis kit (Quanta, Cat# 95047-500). Taq-man PCR was performed using Taq-Man Fast Advance PR Mix (Life technologies, Cat# 4444963) and GAPDH (Life technologies, ID# Hs02758991_g1), HbB (Life technologies, ID#: Hs00747223_g1), HbG2/HbG1 (Life technologies, ID# Hs00361131_g1) and BCL11A (Life technologies, ID#: Hs01093197_m1) according to suppliers protocol. The relative expression of each gene was normalized to GAPDH expression.

Colony forming unit cell assay. For colony forming unit (CFU) assay, 300 cells per 1.1 mL Methocult/35 mm dish in duplicate were plated in Methocult H4434 methylcellulose medium containing SCF, GM-CSF, IL-3, and erythropoietin (StemCell Technologies). Pen/Strep was added into the Methocult. The culture dishes were incubated in a humidified incubator at 37°C. Colonies containing at least 30 cells were counted at day 14 post-plating using StemVision (StemCell Technologies) to take the image of entire plate. The total number of colonies, number of CFU-GEMM (Granulocyte, Erythrocyte, Macrophage, Megakaryocyte), CFU-GM (Granulocyte, Macrophage), CFU-M (Macrophage), CFU-E (erythroid) and BFU-E (Burst-forming unit-erythroid) were then counted StemVision image analyzer software and confirmed by manually using StemVision Colony Marker software.

*In vivo* engraftment study. In vivo engraftment studies are conducted under protocol approved by IACUC. A fraction of final culture of genome edited HSC equivalent to 30,000 starting cells are injected intravenously via the tail vein into sub-lethally irradiated (200 cGY) 6- to 8-week old NSG mice. Engraftment is performed within 24 h after irradiation. Engraftment is monitored by flow cytometric analysis of blood collected at 4, 8, and 12 weeks post-infusion using anti-human CD45 and anti-mouse CD45 antibodies. The mice are sacrificed 13 weeks post-transplantation and bone marrow, spleen and thymus collected for analysis by flow cytometry and colony forming cell unit assay. For secondary engraftment, 50% of the bone marrow from each recipient mouse is transplanted into one secondary sub-lethally irradiated NSG mouse. Engraftment is monitored by flow cytometric analysis of blood collected at 4, 8, and 12 weeks post-infusion using panleukocyte marker, CD45 using anti-human CD45 and anti-mouse CD45 antibodies. Fifteen weeks after transplantation, bone marrow and spleen were harvested from the secondary mice and analyzed by flow cytometry and colony forming cell unit assay.

### Results:

Without being bound by theory, it is believed that targeted genetic disruption of BCL11A erythroid enhancer in HSC will down regulate the expression of BCL11A selectively in erythroid cell lineage and relieve repression of γ-globin expression, allowing production of the red blood cells containing elevated HbF protein, F-cells. The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

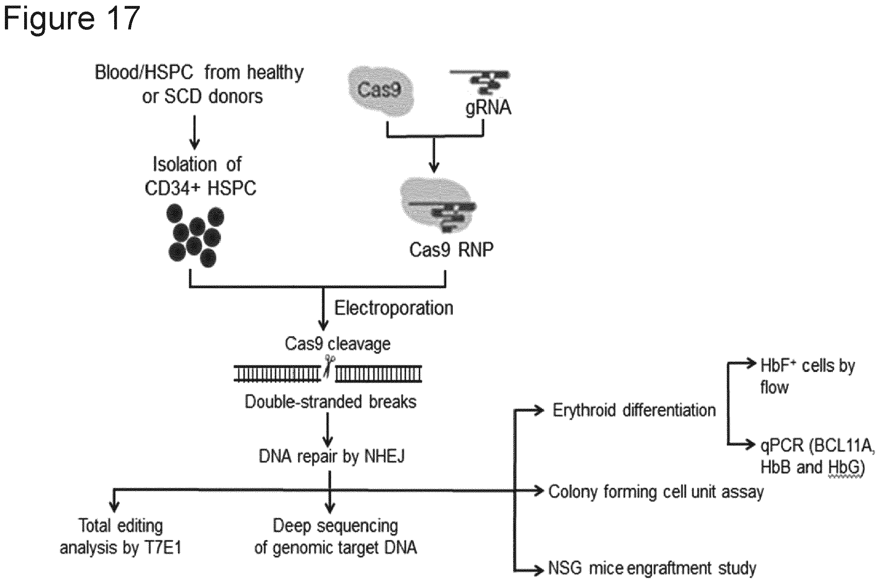

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

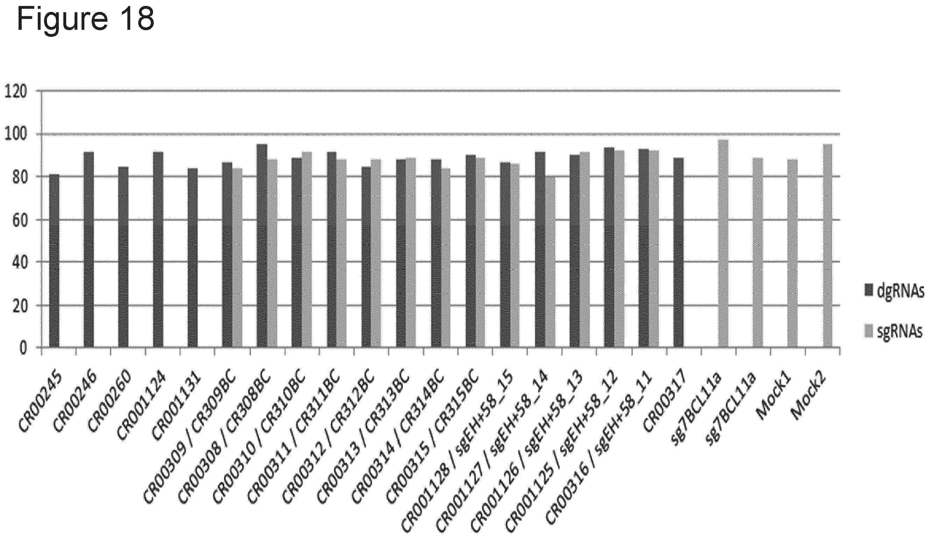

Recombinant Cas9 protein was purified from Escherichia coli and complexed with synthetic dual gRNAs (dgRNA) that consist of crRNA and tracr or full-length single guide RNA (sgRNA) to generate ribonucleoprotein (RNP) complexes. The list and sequences of gRNAs used in the study are shown in Table 14. The RNP complexes were electroporated into healthy or SCD patient derived bone marrow CD34+ HSPC via electroporation as described under materials and methods. The cells were expanded for 2-days in HSC expansion medium containing Compound 4 prior to the delivery of RNP complexes. Actively dividing cells may facilitate uptake of RNP complexes delivered by electroporation. To facilitate recovery from transfection process, the cells were cultured for two additional days in the modified expansion medium containing compound 4. Viability of the cells 48 hours after electroporation was determined by flow cytometry using 7AAD. The viability was relatively high with >80% of the cells viable (Figure 18) suggesting that the HSPC tolerated electroporation of the RNP complexes relatively well compared to other methods of Cas9 and gRNA delivery systems reported in the literature.

**Table 14. List of gRNAs targeting BCL11A erythroid specific enhancer used in the current study.**

| Guide RNA targeting domain ID | Region/ strand | dgRNA tested | sgRNA tested | Target Sequence | Coordinates (hg38) |
|---|---|---|---|---|---|
| CR000245 | +58/+ | x | | tcagtgagatgagatatcaa | chr2:60494483-60494502 |
| CR000246 | +58/+ | x | | cagtgagatgagatatcaaa | chr2:60494484-60494503 |
| CR000260 | +58/- | x | | tgtaactaataaataccagg | chr2:60494790-60494809 |
| CR001124 | +58/- | x | | ttagggtgggggcgtgggtg | chr2:60495217-60495236 |
| CR001131 | +58/- | x | | attagggtgggggcgtgggt | chr2:60495218-60495237 |
| CR000309 | +58/+ | x | X | cacgcccccaccctaatcag | chr2:60495221-60495240 |
| CR000308 | +58/- | x | X | tctgattagggtgggggcgt | chr2:60495222-60495241 |
| CR000310 | +58/- | x | X | ttggcctctgattagggtgg | chr2:60495228-60495247 |
| CR000311 | +58/- | x | X | tttggcctctgattagggtg | chr2:60495229-60495248 |
| CR000312 | +58/- | x | X | gtttggcctctgattagggt | chr2:60495230-60495249 |
| CR000313 | +58/- | x | X | ggtttggcctctgattaggg | chr2:60495231-60495250 |
| CR000314 | +58/- | x | X | aagggtttggcctctgatta | chr2:60495234-60495253 |
| CR000315 | +58/- | x | X | gaagggtttggcctctgatt | chr2:60495235-60495254 |
| CR001128 / sgEH15 | +58/+ | | X | atcagaggccaaacccttcc | chr2:60495236-60495255 |
| CR001127 / sgEH14 | +58/- | | X | cacaggctccaggaagggtt | chr2:60495247-60495266 |
| CR001126 / sgEH13 | +58/- | x | X | tttatcacaggctccaggaa | chr2:60495252-60495271 |
| CR001125 / sgEH12 | +58/- | | X | ttttatcacaggctccagga | chr2:60495253-60495272 |
| CR000316 / sgEH11 | +58/- | | X | ttgcttttatcacaggctcc | chr2:60495257-60495276 |
| CR000317 / sgEH4 | +58/- | | | ctaacagttgcttttatcac | chr2:60495264-60495283 |

The T7E1 assay is a convenient method to assess editing at specific sites in the genome. The genomic DNA was extracted from the HSPC 2-days post-electroporation. The targeted BCL11A erythroid enhancer region was amplified by polymerase chain reaction (PCR). The PCR products were subjected to T7E1 assay. The end products of T7E1 assay were subjected to 2% agarose gel electrophoresis and the percent edited alleles quantified by image**J** (http://rsb.info.nih.gov/ij/). Expected PCR product sizes and approximate expected sizes of T7E1-digested fragments are shown in Figure 19. The total editing frequencies are indicated as percentage of total edits below the agarose gel image. The genome editing at the BCL11A locus was observed in the cells treated with BCL11A Cas9 RNPs but not in mock electroporated control cells (Figure 19).

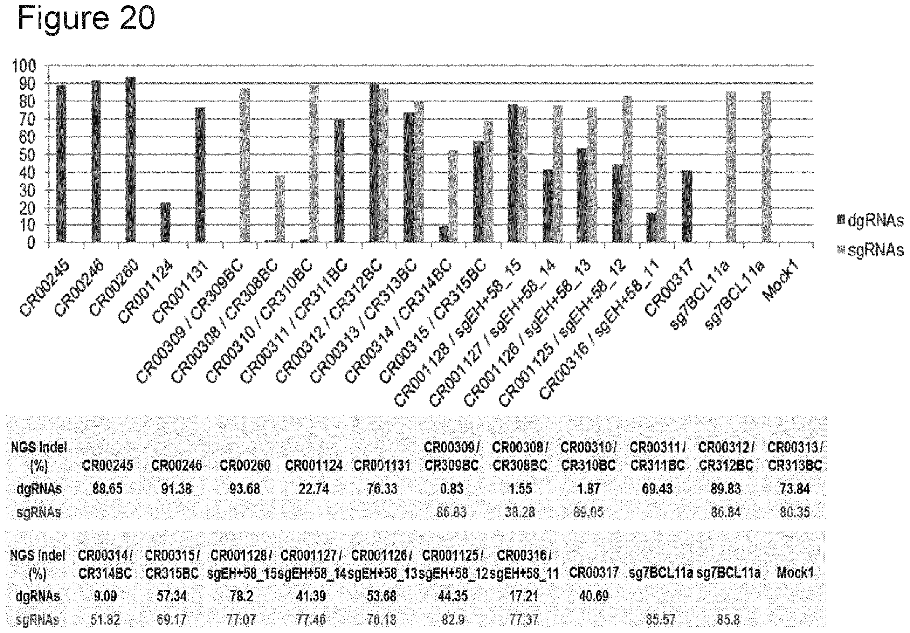

The genomic PCR products of the +58 erythroid enhancer region of BCL11A were also subjected to next generation sequencing (NGS). The NGS facilitates simultaneous monitoring of a large number of samples giving a global view of the alleles. Moreover, it provides information on the heterogeneity of insertions and deletions (indels). The sequences were compared with the sequences from control (mock) treated cells. The results of sequence analysis showed that CRISPR-Cas9 induced double stranded breaks were repaired by NHEJ resulting in variable frequencies of small deletions and insertions (indels) near Cas9 cleavage site located in +58 BCL11A erythroid enhancer region (Figure 20 and Table 15). Overall, the observed editing efficiencies was higher for sgRNAs compared to dgRNAs targeting the same sequence, with 12 out 14 sgRNA producing editing at more than 50% alleles (Figure 20 and Table 15), though (without being bound by theory) the differences in efficiency between sgRNA and dgRNA may be affected by the source of the materials.

**Table 15. Genotypes identified by NGS in HSPCs edited with Cas9-RNP. The nucleotide sequence for each population is given. The target sequence targeted by each gRNA is indicated above each indel population, with the PAM sequences indicated in lower case. Dashes denote deleted bases and lowercase letters denote insertions. In the description of the gRNA, dgRNA refers to dual gRNA molecules comprising the indicated targeting domain. sgRNA indicates single gRNA molecules comprising the indicated targeting domain. Where multiple experiments were run with the same dgRNA or sgRNA, successive experiments are labeled "dgRNA," "dlgRNA", "d2gRNA," etc.**

| **gRNA** | **Variant** | **% Allele** | **Indel length** |
|---|---|---|---|
| | CTAACAGTTGCTTTTATCAC**agg** | | |
| CR00317-dgRNA | TAGTGCAAGCTAACAG---------------GCTCCAGGAAGGGTTTGGCCTCTGATTAG | 5.96% | -15 |
| CR00317-dgRNA | TAGTGCAAGCTAACAGTTGCTTTTATtCACAGGCTCCAGGAAGGGTTTGGCCTCTGATTAG | 5.77% | 1 |
| CR00317-dgRNA | TAGTGCAAGCTAACAGTTGCT-------------CCAGGAAGGGTTTGGCCTCTGATTAG | 4.69% | -13 |
| CR00317-dgRNA | TAGTGCAAGCTAACAGTTGCTTTTATCA--GGCTCCAGGAAGGGTTTGGCCTCTGATTAG | 0.90% | -2 |
| CR00317-dgRNA | TAGTGCAAGCTAACAGTTGCTTTTA-CACAGGCTCCAGGAAGGGTTTGGCCTCTGATTAG | 0.88% | -1 |
| CR00317-dgRNA | TAGTGCAAGCTAACAGTTGCTTTT-CACAGGCTCCAGGAAGGGTTTGGCCTCTGATTAG | 0.77% | -2 |
| CR00317-dgRNA | TAGTGCAAGCT-----------------------CCAGGAAGGGTTTGGCCTCTGATTAG | 0.67% | -23 |
| | ATCAGAGGCCAAACCCTTCC**acc** | | |
| CR001128-dgRNA | AGCTAACAGTTGCTTTTATCACAGGCTCCAGGA-GGGTTTGGCCTCTGATTAGGGTGGGG | 20.07% | -1 |
| CR001128-dgRNA | AGCTAACAGTTGCTTTTATCACAGGCTCCAGG-----TTTGGCCTCTGATTAGGGTGGGG | 5.00% | -5 |
| CR001128-dgRNA | AGCTAACAGTTGCTTTTATCACAGGCTCCAGGAAaGGGTTTGGCCTCTGATTAGGGTGGGG | 4.84% | 1 |
| CR001128-dgRNA | AGCTAACAGTTGCTTTTATCACAGGCTCCAGG----GTTTGGCCTCTGATTAGGGTGGGG | 4.01% | -4 |
| CR001128-dgRNA | AGCTAACAGTTGCTTTTA TCACAGGCTCCAGGA--GGTTTGGCCTCTGA TTAGGGTGGGG | 2.99% | -2 |
| CR001128-sgRNA | AGCTAACAGTTGCTTTTA TCACAGGCTCCAGGA-GGGTTTGGCCTCTGA TTAGGGTGGGG | 23.33% | -1 |
| CR001128-sgRNA | AGCTAACAGTTGCTTTTATCACAGGCTCCAGGAAGG-TTTGGCCTCTGATTAGGGTGGGG | 5.77% | -1 |
| CR001128-sgRNA | AGCTAACAGTTGCTTTTATCACAGGCTCCAGGAAaGGGTTTGGCCTCTGATTAGGGTGGGG | 5.77% | 1 |
| CR001128-sgRNA | AGCTAACAGTTGCTTTTATCACAGGCTCCAGGA--GGTTTGGCCTCTGATTAGGGTGGGG | 5.37% | -2 |
| CR001128-sgRNA | AGCTAACAGTTGCTTTTATCACAGGCTCCAGGAAG--TTTGGCCTCTGATTAGGGTGGGG | 3.70% | -2 |
| CR001128-sgRNA | AGCTAACAGTTGCTTTTATCACAGGCTCCAGG----------CCTCTGATTAGGGTGGGG | 3.62% | -10 |
| CR001128-sgRNA | AGCTAACAGTTGCTTTTATCACAGGC-----------------CTCTGATTAGGGTGGGG | 3.54% | -17 |
| CR001128-sgRNA | AGCTAACAGTTGCTTTTATCACAGGCTCCAGGA---GTTTGGCCTCTGATTAGGGTGGGG | 2.97% | -3 |
| | TTGCTTTTATCACAGGCTCC**agg** | | |
| CR00316-sgRNA | AGCTAACAGTTGCTTTTATCACAGG-------AAGGGTTTGGCCTCTGATTAGGGTGGGG | 16.03% | -7 |
| CR00316-sgRNA | AGCTAACAGTTGCTTTTATCACAGGCTtCCAGGAAGGGTTTGGCCTCTGATTAGGGTGGGG | 8.25% | 1 |
| CR00316-sgRNA | AGCTAACAGTTGCTTTTATC--------CAGGAAGGGTTTGGCCTCTGATTAGGGTGGGG | 8.04% | -8 |
| CR00316-sgRNA | AGCTAACAGTTGCTTTTATCACAGGCcTCCAGGAAGGGTTTGGCCTCTGATTAGGGTGGGG | 4.07% | 1 |
| CR00316-sgRNA | AGCTAACAGTTGCTTTTATCACAGGC-CCAGGAAGGGTTTGGCCTCTGATTAGGGTGGGG | 3.55% | -1 |
| CR00316-sgRNA | AGCTAACAGTTGCTTTTATCACAGGC--CAGGAAGGGTTTGGCCTCTGATTAGGGTGGGG | 2.37% | -2 |
| CR00316-dgRNA | AGCTAACAGTTGCTTTTATCACAGG-------AAGGGTTTGGCCTCTGATTAGGGTGGGG | 2.49% | -7 |
| CR00316-dgRNA | AGCTAACAGTTGCTTTTATCACAGGCTtCCAGGAAGGGTTTGGCCTCTGATTAGGGTGGGG | 1.78% | 1 |
| CR00316-dgRNA | AGCTAACAGTTGCTTTTATC--------CAGGAAGGGTTTGGCCTCTGATTAGGGTGGGG | 1.32% | -8 |
| CR00316-dgRNA | AGCTAACAGTTGCTTTTATCACAGGCcTCCAGGAAGGGTTTGGCCTCTGATTAGGGTGGGG | 1.26% | 1 |
| CR00316-d1gRNA | AGCTAACAGTTGCTTTTATCACAGG-------AAGGGTTTGGCCTCTGATTAGGGTGGGG | 6.94% | -7 |
| CR00316-d1gRNA | AGCTAACAGTTGCTTTTATC--------CAGGAAGGGTTTGGCCTCTGATTAGGGTGGGG | 4.99% | -8 |
| CR00316-d1gRNA | AGCTAACAGTTGCTTTTATCACAGGCTtCCAGGAAGGGTTTGGCCTCTGATTAGGGTGGGG | 4.69% | 1 |
| CR00316-d1gRNA | AGCTAACAGT------------------------------PATTAGGGTGGGG | 2.90% | -37 |
| CR00316-d1gRNA | AGCTAACAGTTGCTTTTATCACAGGC-CCAGGAAGGGTTTGGCCTCTGATTAGGGTGGGG | 1.54% | -1 |
| CR00316-d2gRNA | AGCTAACAGTTGCTTTTATCACAGG-------AAGGGTTTGGCCTCTGATTAGGGTGGGG | 11.64% | -7 |
| CR00316-d2gRNA | AGCTAACAGTTGCTTTTATC--------CAGGAAGGGTTTGGCCTCTGATTAGGGTGGGG | 6.04% | -8 |
| CR00316-d2gRNA | AGCTAACAGTTGCTTTTATCACAGGCTtCCAGGAAGGGTTTGGCCTCTGATTAGGGTGGGG | 5.40% | 1 |
| CR00316-d2gRNA | AGCTAACAGTTGCTTTTATCACAGGCcTCCAGGAAGGGTTTGGCCTCTGATTAGGGTGGGG | 1.80% | 1 |
| CR00316-d2gRNA | AGCTAACAGTTGCTTTTATCACAGGC-----GAAGGGTTTGGCCTCTGATTAGGGTGGGG | 1.43% | -5 |
| | TTTTATCACAGGCTCCAGGA**agg** | | |
| CR001125-dgRNA | AACAGTTGCTTTTATCACAGGCTCCAaGGAAGGGTTTGGCCTCTGATTAGGGTGGGGGCGT | 13.50% | 1 |
| CR001125-dgRNA | AACAGTTGCTTTTATCACAGGC CTCTGATTAGGGTGGGGGCGT | 1.82% | -17 |
| CR001125-dgRNA | AACAGTTGCTTTTATCACAGG------------GTTTGGCCTCTGATTAGGGTGGGGGCGT | 1.76% | -11 |
| CR001125-dgRNA | AACAGTTGCTTTTATCACAGG-------AAGGGTTTGGCCTCTGATTAGGGTGGGGGCGT | 1.59% | -7 |
| CR001125-dgRNA | AACAGTTGCTTTT------------------------GGTGGGGGCGT | 1.33% | -36 |
| CR001125-sgRNA | AACAGTTGCTTTTATCACAGGCTCCAaGGAAGGGTTTGGCCTCTGATTAGGGTGGGGGCGT | 26.27% | 1 |
| CR001125-sgRNA | AACAGTTGCTTTTATCACAGG-----------GTTTGGCCTCTGATTAGGGTGGGGGCGT | 4.64% | -11 |
| CR001125-sgRNA | AACAGTTGCTTTTATCACAGG-------AAGGGTTTGGCCTCTGATTAGGGTGGGGGCGT | 4.11% | -7 |
| CR001125-sgRNA | AACAGTTGCTTTTATCACAGGC------------CTCTGATTAGGGTGGGGGCGT | 2.66% | -17 |
| CR001125-sgRNA | AACAGTTGCTTTTATCACAGGCT-----------TGGCCTCTGATTAGGGTGGGGGCGT | 2.58% | -12 |
| CR001125-sgRNA | AACAGTTGCTTTTATCACAGGCTCC-GGAAGGGTTTGGCCTCTGATTAGGGTGGGGGCGT | 1.84% | -1 |
| | TTTATCACAGGCTCCAGGAA**ggg** | | |
| CR001126-dgRNA | ACAGTTGCTTTTATCACAGGCTCCAG-AAGGGTTTGGCCTCTGATTAGGGTGGGGGCGTG | 9.38% | -1 |
| CR001126-dgRNA | ACAGTTGCTTTTATCACAGG-------------GTTTGGCCTCTGATTAGGGTGGGGGCGTG | 3.28% | -11 |
| CR001126-dgRNA | ACAGTTGCTTTTATCACAGGCTCCAGGgAAGGGTTTGGCCTCTGATTAGGGTGGGGGCGTG | 2.91% | 1 |
| CR001126-dgRNA | ACAGTTGCTTTTATCACAGGC--------------------CTCTGATTAGGGTGGGGGCGTG | 2.82% | -17 |
| CR001126-dgRNA | ACAGTTGCTTTT-------------------------GGGGGCGTG | 1.61% | -39 |
| CR001126-dgRNA | ACAGTTGCTTT------------------------GGGGGCGTG | 1.12% | -40 |
| CR001126-sgRNA | ACAGTTGCTTTTATCACAGGCTCCAG-AAGGGTTTGGCCTCTGATTAGGGTGGGGGCGTG | 12.70% | -1 |
| CR001126-sgRNA | ACAGTTGCTTTTATCACAGG-----------GTTTGGCCTCTGATTAGGGTGGGGGCGTG | 4.37% | -11 |
| CR001126-sgRNA | ACAGTTGCTTTTATCACAGGCTCCAGGgAAGGGTTTGGCCTCTGATTAGGGTGGGGGCGTG | 3.22% | 1 |
| CR001126-sgRNA | ACAGTTGCTTTTATCACAGGCTCCAGaT------------------AAGGTGGGGGCGTG | 2.96% | -18 |
| CR001126-sgRNA | ACAGTTGCTTTTATCACAGGC-------------CTCTGATTAGGGTGGGGGCGTG | 2.68% | -17 |
| CR001126-sgRNA | ACAGTTGCTTTTATCACAGGCTCCAGG----GTTTGGCCTCTGATTAGGGTGGGGGCGTG | 2.59% | -4 |
| CR001126-sgRNA | ACAGTTGCTTTTATCACAGGCTCCAGcGAAGGGTTTGGCCTCTGATTAGGGTGGGGGCGTG | 1.80% | 1 |
| CR001126-sgRNA | ACAGTTGCTTTTATCACAGGCTCC-GGAAGGGTTTGGCCTCTGATTAGGGTGGGGGCGTG | 1.74% | -1 |
| | CACAGGCTCCAGGAAGGGTT**tgg** | | |
| CR001127-dgRNA | TGCTTTTATCACAGGCTCCAGGAAGG-TTTGGCCTCTGATTAGGGTGGGGGCGTGGGTGG | 4.00% | -1 |
| CR001127-dgRNA | TGCTTTTATCACAGGCcT----------------------------------GGGGCGTGGGTGG | 2.35% | -29 |
| CR001127-dgRNA | TGCTTTTATCACAGGC------------------------GTGGGTGG | 2.19% | -36 |
| CR001127-dgRNA | TGCTTTTATCACAGGC----------------------CTCTGATTAGGGTGGGGGCGTGGGTGG | 1.59% | -17 |
| CR001127-dgRNA | TGCTTTTA TCACAGGCTCCAGGAAGG------CCTCTGATTAGGGTGGGGGCGTGGGTGG | 1.48% | -6 |
| CR001127-sgRNA | TGCTTTTA TCACAGGCTCCAGGA-GGGTTTGGCCTCTGA TTAGGGTGGGGGCGTGGGTGG | 23.21 % | -1 |
| CR001127-sgRNA | TGCTTTTATCACAGGCTCCAGGAAGG-TTTGGCCTCTGATTAGGGTGGGGGCGTGGGTGG | 6.65% | -1 |
| CR001127-sgRNA | TGCTTTTATCACAGGC------------------------CTCTGATTAGGGTGGGGGCGTGGGTGG | 5.20% | -17 |
| CR001127-sgRNA | TGCTTTTATCACAGGCTCCAGGAAaGGGTTTGGCCTCTGATTAGGGTGGGGGCGTGGGTGG | 4.89% | 1 |
| CR001127-sgRNA | TGCTTTTATCACAGGCTCCAGC--------------------GCGTGGGTGG | 4.30% | -28 |
| CR001127-sgRNA | TGCTTTTATCACAGGCTCCAGG-----TTTGGCCTCTGATTAGGGTGGGGGCGTGGGTGG | 3.72% | -5 |
| CR001127-sgRNA | TGCTTTTATCACAGGC----------------------------GTGGGTGG | 3.71% | -36 |
| CR001127-sgRNA | TGCTTTTATCACAGGCTCCAGGAAG-TTTGGCCTCTGATTAGGGTGGGGGCGTGGGTGG | 3.53% | -2 |
| CR001127-sgRNA | TGCTTTTATCACAGGCTCCAGGAAGGtC---------------------CGTTTGCGTGGGTGG | 2.29% | -17 |
| | CACGCCCCCACCCTAATCAG**agg** | | |
| CR00309-sgRNA | TATCACAGGCTCCAGGAAGGGTTTGGC-------------------GTGGGTGGGGTAGA | 11.56% | -19 |
| CR00309-sgRNA | TATCACAGGCTCCAGGAAGGGTTTGGCCTCTGAaTTAGGGTGGGGGCGTGGGTGGGGTAGA | 7.29% | 1 |
| CR00309-sgRNA | TATCACAGGCTCCAGGAAGC-----------------CGTGGGTGGGGTAGA | 6.16% | -24 |
| CR00309-sgRNA | TATCACAGGCTCCAGGAAGGC------------------------GGCGTGGGTGGGGTAGA | 4.74% | -22 |
| CR00309-sgRNA | TATCACAGGCTCCAGGAAGGGTTTGG-----------------GCGTGGGTGGGGTAGA | 4.34% | -18 |
| CR00309-sgRNA | TATCACAGGCTCCAGGAAGGGTTTGC-------------------GTGGGGTAGA | 3.45% | -24 |
| CR00309-sgRNA | TATCACAGGCTCCAGC-----------------------GGCGTGGGTGGGGTAGA | 1.87% | -27 |
| | GAAGGGTTTGGCCTCTGATT**agg** | | |
| CR00315-dgRNA | AGGCTCCAGGAAGGGTTTGGC-----------------GTGGGTGGGGTAGAAGAGGA | 5.80% | -19 |
| CR00315-dgRNA | AGGCTCCAGGAAGGGTTTGG-------------------GCGTGGGTGGGGTAGAAGAGGA | 4.08% | -18 |
| CR00315-dgRNA | AGGCTCCAGC---------------------------GGCGTGGGTGGGGTAGAAGAGGA | 2.80% | -27 |
| CR00315-dgRNA | AGGCTCCAGGAAGGGT------------------------GGGGTAGAAGAGGA | 2.62% | -30 |
| CR00315-dgRNA | AGGCTCCAGGAAGGGTTTGGCCTCTG-----------GCGTGGGTGGGGTAGAAGAGGA | 2.49% | -12 |
| CR00315-dgRNA | AGGCTCCAGGAAGGC-------------------CGTGGGTGGGGTAGAAGAGGA | 2.44% | -24 |
| CR00315-dgRNA | AGGCTCCAGGAAGGGTTTGGCCTCTGcG---------------------TAGAAGAGGA | 1.55% | -22 |
| CR00315-dgRNA | AGGCTCCAGGAAGGGTT------------------------GGGTGGGGTAGAAGAGGA | 1.52% | -25 |
| CR00315-sgRNA | AGGCTCCAGGAAGGGTTTGGC--------------------GTGGGTGGGGTAGAAGAGGA | 8.40% | -19 |
| CR00315-sgRNA | AGGCTCCAGGAAGGGTTTGG---------------------GCGTGGGTGGGGTAGAAGAGGA | 4.66% | -18 |
| CR00315-sgRNA | AGGCTCCAGGAAGGGT---------------------------GGGGTAGAAGAGGA | 3.77% | -30 |
| CR00315-sgRNA | AGGCTCCAGGAAGGGTTTGC------------------------GGTAGAAGAGGA | 3.19% | -28 |
| CR00315-sgRNA | AGGCTCCAGGAAGGC------------------GGCGTGGGTGGGGTAGAAGAGGA | 2.78% | -22 |
| CR00315-sgRNA | AGGCTCCAGC------------------------------CGTGGGTGGGGTAGAAGAGGA | 2.71% | -29 |
| CR00315-sgRNA | AGGCTCCAGGAAGGC---------------------------CGTGGGTGGGGTAGAAGAGGA | 2.50% | -24 |
| CR00315-sgRNA | AGGCTCCAGC------------------------------------GTAGAAGAGGA | 2.28% | -39 |
| CR00315-sgRNA | AGGCTCCAGGAAGGGTTTGC-------------------------GTGGGGTAGAAGAGGA | 2.25% | -24 |
| | AAGGGTTTGGCCTCTGATTA**ggg** | | |
| CR00314-dgRNA | GGCTCCAGGAAGGGTTTGGCCTCTGATTccccATTGTGGGGGCGTGGGTGGGGTAGAAGAGGAC | 0.91% | 4 |
| CR00314-dgRNA | GGCTCCAGGAAGGGTTTGG---------------------TGGGGTAGAAGAGGAC | 0.86% | -25 |
| CR00314-sgRNA | GGCTCCAGGAAGGGTTTGGC--------------------GTGGGTGGGGTAGAAGAGGAC | 4.47% | -19 |
| CR00314-sgRNA | GGCTCCAGGAAGGG---------------------CGTGGGTGGGGTAGAAGAGGAC | 3.89% | -24 |
| CR00314-sgRNA | GGCTCCAGGAAGGGTTTGG---------------------------GTGGGGTAGAAGAGGAC | 3.75% | -24 |
| CR00314-sgRNA | GGCTCCAGGAAGGGTTTGGCCTCTGA---------GGGCGTGGGTGGGGTAGAAGAGGAC | 2.60% | -9 |
| CR00314-sgRNA | GGCTCCAGGAAGGGTTTGGCC-----------------GTGGGTGGGGTAGAAGAGGAC | 2.26% | -18 |
| CR00314-sgRNA | GGCTCCAGGAAGGGTTTGGCCTCTGAaTTAGGGTGGGGGCGTGGGTGGGGTAGAAGAGGAC | 2.11% | 1 |
| CR00314-sgRNA | GGCTCCAGGAAGGGTTTG----------------------CGTGGGTGGGGTAGAAGAGGAC | 2.05% | -20 |
| CR00314-sgRNA | GGCTCCAGGAAGGGTTTGG------------------GGTAGAAGAGGAC | 2.04% | -28 |
| CR00314-sgRNA | GGCTCCAGGAAGGGT----------------------GGGTGGGGTAGAAGAGGAC | 2.03% | -26 |
| | GGTTTGGCCTCTGA TTAGGG**tgg** | | |
| CR00313-dgRNA | TCCAGGAAGGGTTTGGC------------------GTGGGTGGGGTAGAAGAGGACTGG | 16.00% | -19 |
| CR00313-dgRNA | TCCAGGAAGGGT---------------------------GGGGTAGAAGAGGACTGG | 5.69% | -30 |
| CR00313-dgRNA | TCCAGGAAGGGTTTGGCCTCTGATTAGG-TGGGGGCGTGGGTGGGGTAGAAGAGGACTGG | 3.93% | -1 |
| CR00313-dgRNA | TCCAGGAAGGGT---------------------------------AGAGGACTGG | 3.57% | -35 |
| CR00313-dgRNA | TCCAGGAAGGC-----------------------GGCGTGGGTGGGGTAGAAGAGGACTGG | 2.75% | -22 |
| CR00313-dgRNA | TCCAGGAAGGC---------------------CGTGGGTGGGGTAGAAGAGGACTGG | 2.70% | -24 |
| CR00313-dgRNA | TCCAGGAAGGGTTTGG-----------------------------GTGGGGTAGAAGAGGACTGG | 2.66% | -24 |
| CR00313-dgRNA | TCCAGGAAGGGTTTGGCCTCTGATT-GGGTGGGGGCGTGGGTGGGGTAGAAGAGGACTGG | 2.17% | -1 |
| CR00313-sgRNA | TCCAGGAAGGGTTTGGC-----------------GTGGGTGGGGTAGAAGAGGACTGG | 11.86% | -19 |
| CR00313-sgRNA | TCCAGGAAGGC-----------------------GGCGTGGGTGGGGTAGAAGAGGACTGG | 3.86% | -22 |
| CR00313-sgRNA | TCCAGGAAGGGTTTGG---------------------------GTGGGGTAGAAGAGGACTGG | 3.36% | -24 |
| CR00313-sgRNA | TCCAGGAAGGGTTTGGCCTCTGATTAGG-TGGGGGCGTGGGTGGGGTAGAAGAGGACTGG | 3.31% | -1 |
| CR00313-sgRNA | TCCAGGAAGGGTT----------------------GGGGTAGAAGAGGACTGG | 2.83% | -29 |
| CR00313-sgRNA | TCCAGGAAGGGTTTGGCCT-----------------------GGGTGGGGTAGAAGAGGACTGG | 2.76% | -19 |
| CR00313-sgRNA | TCCAGGAAGGGTTTGG-----------------TAGAAGAGGACTGG | 2.31% | -30 |
| | GTTTGGCCTCTGATTAGGGT**ggg** | | |
| CR00312-dgRNA | CCAGGAAGGGTTTGGCCTCTGATTAGG-TGGGGGCGTGGGTGGGGTAGAAGAGGACTGGC | 16.87% | -1 |
| CR00312-dgRNA | CCAGGAAGGGTTTGGC--------------------GTGGGTGGGGTAGAAGAGGACTGGC | 9.86% | -19 |
| CR00312-dgRNA | CCAGGAAGGGTTTGGCCTCTGATT--GGTGGGGGCGTGGGTGGGGTAGAAGAGGACTGGC | 5.65% | -2 |
| CR00312-dgRNA | CCAGGAAGGG----------------------CGTGGGTGGGGTAGAAGAGGACTGGC | 3.83% | -24 |
| CR00312-dgRNA | CCAGGAA----------------GGCGTGGGTGGGGTAGAAGAGGACTGGC | 3.42% | -22 |
| CR00312-dgRNA | CCAGGAAGGGTTTGG---------------------GTGGGGTAGAAGAGGACTGGC | 2.10% | -24 |
| CR00312-dgRNA | CCAGGAAGGGTTTGGCCTCTGA----GGTGGGGGCGTGGGTGGGGTAGAAGAGGACTGGC | 1.85% | -4 |
| CR00312-sgRNA | CCAGGAAGGGTTTGGCCTCTGATTAGG-TGGGGGCGTGGGTGGGGTAGAAGAGGACTGGC | 13.35% | -1 |
| CR00312-sgRNA | CCAGGAAGGGTTTGGC------------------------GTGGGTGGGGTAGAAGAGGACTGGC | 7.85% | -19 |
| CR00312-sgRNA | CCAGGAAGGGTTTGG----------------GTGGGGTAGAAGAGGACTGGC | 4.19% | -24 |
| CR00312-sgRNA | CCAGGAAGGGTTTGGCCTCTGATT--GGTGGGGGCGTGGGTGGGGTAGAAGAGGACTGGC | 3.98% | -2 |
| CR00312-sgRNA | CCAGGAAGGGTTTGGCCTCTGATTAGGG-----GCGTGGGTGGGGTAGAAGAGGACTGGC | 2.95% | -5 |
| CR00312-sgRNA | CCAGGAAGGG------------------CGTGGGTGGGGTAGAAGAGGACTGGC | 2.29% | -24 |
| CR00312-sgRNA | CCAGGAAGGGTTTGGCCTCTGATTAGaGGTGGGGGCGTGGGTGGGGTAGAAGAGGACTGGC | 2.09% | 1 |
| CR00312-d1gRNA | CCAGGAAGGGTTTGGCCTCTGATTAGG-TGGGGGCGTGGGTGGGGTAGAAGAGGACTGGC | 19.22% | -1 |
| CR00312-d1gRNA | CCAGGAAGGGTTTGGCCTCTGATT--GGTGGGGGCGTGGGTGGGGTAGAAGAGGACTGGC | 6.28% | -2 |
| CR00312-d1gRNA | CCAGGAAGGGTTTGG---------------------GTGGGGTAGAAGAGGACTGGC | 5.31% | -24 |
| CR00312-d1gRNA | CCAGGAAGGGTTTGGC------------------------GTGGGTGGGGTAGAAGAGGACTGGC | 4.22% | -19 |
| CR00312-d1gRNA | CCAGGAAGGGTTTGG------------------------GGGCGTGGGTGGGGTAGAAGAGGACTGGC | 3.10% | -16 |
| CR00312-d1gRNA | CCAGGAAGGGTTTGGCCTCTGATTAG--TGGGGGCGTGGGTGGGGTAGAAGAGGACTGGC | 2.85% | -2 |
| CR00312-d1gRNA | CCAGGAAGGG------------------------CGTGGGTGGGGTAGAAGAGGACTGGC | 2.74% | -24 |
| CR00312-d2gRNA | CCAGGAAGGGTTTGGCCTCTGATTAGG-TGGGGGCGTGGGTGGGGTAGAAGAGGACTGGC | 23.15% | -1 |
| CR00312-d2gRNA | CCAGGAAGGGTTTGGC--------------------GTGGGTGGGGTAGAAGAGGACTGGC | 8.99% | -19 |
| CR00312-d2gRNA | CCAGGAAGGGTTTGGCCTCTGATT--GGTGGGGGCGTGGGTGGGGTAGAAGAGGACTGGC | 6.82% | -2 |
| CR00312-d2gRNA | CCAGGAAGGG--------------GCGTGGGTGGGGTAGAAGAGGACTGGC | 2.69% | -23 |
| CR00312-d2gRNA CR00312-d2gRNA | CCAGGAAGGG-------------CGTGGGTGGGGTAGAAGAGGACTGGC CCAGGAAGGGTTTGGCCTCTGATTAG--TGGGGGCGTGGGTGGGGTAGAAGAGGACTGGC | 2.29% 2.25% | -24 -2 |
| CR00312-d2gRNA | CCAGGAAGGGTTTGG----------------GGGCGTGGGTGGGGTAGAAGAGGACTGGC | 2.13% | -16 |
| | TTTGGCCTCTGATTAGGGTG**ggg** | | |
| CR00311-dgRNA | CAGGAAGGGTTTGGCCTCTGATTAGG-TGGGGGCGTGGGTGGGGTAGAAGAGGACTGGCA | 19.62% | -1 |
| CR00311-dgRNA | CAGGAAGGGTTTGGC----------------GTGGGTGGGGTAGAAGAGGACTGGCA | 3.01% | -19 |
| CR00311-dgRNA | CAGGAAGGGTTTGGCCTCTGATTAG--TGGGGGCGTGGGTGGGGTAGAAGAGGACTGGCA | 2.77% | -2 |
| CR00311-dgRNA | CAGGAAGGGTTTGGCCTCTGATTAGGG-----GCGTGGGTGGGGTAGAAGAGGACTGGCA | 2.23% | -5 |
| CR00311-dgRNA | CAGGAAGGGTTTGC---------------------GTGGGGTAGAAGAGGACTGGCA | 2.21% | -24 |
| CR00311-dgRNA | CAGGAAGGGTTTGG-----------------TGGGGGCGTGGGTGGGGTAGAAGAGGACTGGCA | 2.05% | -13 |
| CR00311-dgRNA | CAGGAAGGGTTTGGCCTCTG-------TGGGGGCGTGGGTGGGGTAGAAGAGGACTGGCA | 1.88% | -7 |
| CR00311-dgRNA | CAGGAAGGGT--------------------------GGGGTAGAAGAGGACTGGCA | 1.76% | -30 |
| CR00311-sgRNA | CAGGAAGGGTTTGGCCTCTGATTAGG-TGGGGGCGTGGGTGGGGTAGAAGAGGACTGGCA | 28.21% | -1 |
| CR00311-sgRNA | CAGGAAGGGTTTGGC------------------GTGGGTGGGGTAGAAGAGGACTGGCA | 4.88% | -19 |
| CR00311-sgRNA | CAGGAAGGGT----------------------GGGGTAGAAGAGGACTGGCA | 2.72% | -30 |
| CR00311-sgRNA | CAGGAAGGGTTTGGCCTCTGATTAG--TGGGGGCGTGGGTGGGGTAGAAGAGGACTGGCA | 2.62% | -2 |
| CR00311-sgRNA | CAGGAAGGGTTTGGCCTCTGATTAGGG----GGCGTGGGTGGGGTAGAAGAGGACTGGCA | 2.35% | -4 |
| CR00311-sgRNA | CAGGAAGGGTTTGGCCTCTGA TTAGGG-----GCGTGGGTGGGGTAGAAGAGGACTGGCA | 2.06% | -5 |
| CR00311-d1gRNA | CAGGAAGGGTTTGGCCTCTGATTAGG-TGGGGGCGTGGGTGGGGTAGAAGAGGACTGGCA | 31.34% | -1 |
| CR00311-d1gRNA | CAGGAAGGGTTTGGCCTCTGATTAG--TGGGGGCGTGGGTGGGGTAGAAGAGGACTGGCA | 4.00% | -2 |
| CR00311-d1gRNA | CAGGAAGGGTTTGGCCT----------------------AAGAGGACTGGCA | 2.84% | -30 |
| CR00311-d1gRNA | CAGGAAGGGTTTGC-------------------------GAGGACTGGCA | 2.41% | -35 |
| CR00311-d1gRNA | CAGGAAGGGTTT--------------------------AGAAGAGGACTGGCA | 2.35% | -33 |
| CR00311-d1gRNA | CAGGAAGGGTTTGGCCTC--------------------------CAGGACTGGCA | 2.11% | -31 |
| CR00311-d1gRNA | CAGGAAGGGTTTGGCCTCTGA TTAGGG----GGCGTGGGTGGGGTAGAAGAGGACTGGCA | 2.03% | -4 |
| CR00311-d1gRNA | CAGGAAGGGTTTGGCCTCTGATTAGGG---GGGCGTGGGTGGGGTAGAAGAGGACTGGCA | 1.71% | -3 |
| CR00311-d2gRNA | CAGGAAGGGTTTGGCCTCTGATTAGG-TGGGGGCGTGGGTGGGGTAGAAGAGGACTGGCA | 30.20% | -1 |
| CR00311-d2gRNA | CAGGAAGGGTTTGGC-------------------GTGGGTGGGGTAGAAGAGGACTGGCA | 7.57% | -19 |
| CR00311-d2gRNA | CAGGAAGGGTTTGGCCTCTGATTAG--TGGGGGCGTGGGTGGGGTAGAAGAGGACTGGCA | 3.57% | -2 |
| CR00311-d2gRNA | CAGGAAGGGTTTGG---------------------------GGTAGAAGAGGACTGGCA | 2.39% | -28 |
| CR00311-d2gRNA | CAGGAAGGGTTTGGCCTCTGA TTAGGG-----GCGTGGGTGGGGTAGAAGAGGACTGGCA | 2.10% | -5 |
| CR00311-d2gRNA | CAGGAAGGGTTTGGCCTCTGA TTAGGG----GGCGTGGGTGGGGTAGAAGAGGACTGGCA | 1.50% | -4 |
| CR00311-d2gRNA | CAGGAAGGGTTTGG------------------GGGCGTGGGTGGGGTAGAAGAGGACTGGCA | 1.49% | -16 |
| | TTGGCCTCTGATTAGGGTGG**ggg** | | |
| CR00310-sgRNA | AGGAAGGGTTTGGCCTCTGATTAGGG----GGCGTGGGTGGGGTAGAAGAGGACTGGCAG | 7.70% | -4 |
| CR00310-sgRNA | AGGAAGGGTTTGGCCTCTGATTAGGG------CGTGGGTGGGGTAGAAGAGGACTGGCAG | 7.13% | -6 |
| CR00310-sgRNA | AGGAAGGGTTTGGCCTCTGATTAGGG-----GCGTGGGTGGGGTAGAAGAGGACTGGCAG | 6.34% | -5 |
| CR00310-sgRNA | AGGAAGGGTTTGGCCTCTGATTAGG-TGGGGGCGTGGGTGGGGTAGAAGAGGACTGGCAG | 5.38% | -1 |
| CR00310-sgRNA | AGGAAGGGTTTGGCCTCTGATTAG--TGGGGGCGTGGGTGGGGTAGAAGAGGACTGGCAG | 3.73% | -2 |
| CR00310-sgRNA | AGGAAGGGTTTGGCCTCTGATT-----GGGGGCGTGGGTGGGGTAGAAGAGGACTGGCAG | 3.54% | -5 |
| CR00310-dgRNA | AGGAAGGGTTTGGCCTCTGATTAGGGTGGGGGCGTGGGTGGG-TAGAAGAGGACTGGCAG | 0.67% | -1 |
| | TCTGATTAGGGTGGGGGCGT**ggg** | | |
| CR00308-sgRNA | GGTTTGGCCTCTGATTAGGGTGGGG------------TAGAAGAGGACTGGCAGACCTCT | 16.28% | -12 |
| CR00308-sgRNA | GGTTTGGCCTCTGATTAGGGTGGGGG-----------TAGAAGAGGACTGGCAGACCTCT | 5.59% | -11 |
| CR00308-sgRNA | GGTTTGGCCTCTGATTAGGGTGGG--------TGGGGTAGAAGAGGACTGGCAGACCTCT | 4.51% | -8 |
| CR00308-sgRNA | GGTTTGGCCTCTGAT------------GGGGTAGAAGAGGACTGGCAGACCTCT | 3.54% | -18 |
| CR00308-sgRNA | GGTTTGGCCTCTGATTtA------------CTGGCAGACCTCT | 2.76% | -29 |
| CR00308-sgRNA | GGTTTGGCCTCTGATTgG------------CCGTGGGTGGGGTAGAAGAGGACTGGCAGACCTCT | 2.27% | -7 |
| CR00308-sgRNA | GGTTTGGCCTCTGATTAGGG------------GTAGAAGAGGACTGGCAGACCTCT | 2.26% | -16 |
| CR00308-sgRNA | GGTTTGGCCTCTGATcC--AAGAGCCCGTGGGTGGGGTAGAAGAGGACTGGCAGACCTCT | 1.99% | -2 |
| CR00308-dgRNA | GGTTTGGCCTCTGATTAGGGTGGGGGCGTGGGTGGG-TAGAAGAGGACTGGCAGACCTCT | 0.42% | -1 |
| | ATTAGGGTGGGGGCGTGGGT**ggg** | | |
| CR001131-dgRNA | TGGCCTCTGATTAGGGTGGGG------------TAGAAGAGGACTGGCAGACCTCTCCAT | 44.62% | -12 |
| CR001131-dgRNA | TGGCCTCTGATTAGGGTGGGGG------------TAGAAGAGGACTGGCAGACCTCTCCAT | 3.19% | -11 |
| CR001131-dgRNA | TGGCCTCTGATTAGGGTGGGGGCGTGG-TGGGGTAGAAGAGGACTGGCAGACCTCTCCAT | 1.72% | -1 |
| CR001131-dgRNA | TGGCCTCTGATTAGGGTGGGGGCG------------AAGAGGACTGGCAGACCTCTCCAT | 1.04% | -12 |
| | TTAGGGTGGGGGCGTGGGTG**ggg** | | |
| CR001124-dgRNA | GGCCTCTGATTAGGGTGGGG------------TAGAAGAGGACTGGCAGACCTCTCCATC | 8.52% | -12 |
| CR001124-dgRNA | GGCCTCTGATTAGGGTGGGGGCGTGG-TGGGGTAGAAGAGGACTGGCAGACCTCTCCATC | 2.12% | -1 |
| CR001124-dgRNA | GGCCTCTGATTAGGGTGGGGG------------TAGAAGAGGACTGGCAGACCTCTCCATC | 1.33% | -11 |
| | TGTAACTAATAAATACC**agg** | | |
| CR00260-dgRNA | CTGCTGAGGTGTAACTAATAAATACC-GGAGGCAGCATTTTAGTTCACAAGCTCGGAGCA | 26.54% | -1 |
| CR00260-dgRNA | CTGCTGAGGTGTAACTAATAAATAC-AGGAGGCAGCATTTTAGTTCACAAGCTCGGAGCA | 12.39% | -1 |
| CR00260-dgRNA | CTGCTGAGGTGTAACTAATAAATACCcAGGAGGCAGCATTTTAGTTCACAAGCTCGGAGCA | 12.32% | 1 |
| CR00260-dgRNA | CTGCTGAGGTGTAACTAA TAAA TACC--GAGGCAGCA TTTTAGTTCACAAGCTCGGAGCA | 2.37% | -2 |
| CR00260-dgRNA | CTGCTGAGGTGTAACTAA TAAA T--CAGGAGGCAGCA TTTTAGTTCACAAGCTCGGAGCA | 1.89% | -2 |
| CR00260-dgRNA | CTGCTGAGGTGTAACTAATAAATA---GGAGGCAGCATTTTAGTTCACAAGCTCGGAGCA | 1.28% | -3 |
| | CAGTGAGATGAGATATCAAA**ggg** | | |
| CR00246-dgRNA | GGGAGATGGAATGAACACTTTTCGTCCCCTTTGgATATCTCATCTCACTGAGCTCTGGGCC | 7.56% | 1 |
| CR00246-dgRNA | GGGAGATGGAATGAACACTTTTCGTCCCCTTTGAT--CTCATCTCACTGAGCTCTGGGCC | 4.46% | -2 |
| CR00246-dgRNA | GGGAGA TGGAA TGAACACTTTTCGTCCCCTTTG------CA TCTCACTGAGCTCTGGGCC | 3.50% | -6 |
| CR00246-dgRNA | GGGAGA TGGAA TGAACACTTTTCGTCCCCTTT-----CTCA TCTCACTGAGCTCTGGGCC | 2.98% | -5 |
| CR00246-dgRNA | GGGAGATGGAATGAACACTTTTCGTCCCCTTT-ATATCTCATCTCACTGAGCTCTGGGCC | 2.97% | -1 |
| CR00246-dgRNA | GGGAGATGGAATGAACACTTTTCGTCCCCTTTG---TCTCATCTCACTGAGCTCTGGGCC | 2.96% | -3 |
| CR00246-dgRNA | GGGAGATGGAATGAACACTTTTCGTCCCCTTT--TATCTCATCTCACTGAGCTCTGGGCC | 2.93% | -2 |
| CR00246-dgRNA | GGGAGATGGAATGAACACTTTTCGTCCCCTTTG-TATCTCATCTCACTGAGCTCTGGGCC | 2.70% | -1 |
| | TCAGTGAGATGAGATATCAA**agg** | | |
| CR00245-dgRNA | GGAGATGGAATGAACACTTTTCGTCCCCTTTGAaTATCTCATCTCACTGAGCTCTGGGCCG | 22.36% | 1 |
| CR00245-dgRNA | GGAGATGGAATGAACACTTTTCGTCCCCTTTGA-ATCTCATCTCACTGAGCTCTGGGCCG | 8.97% | -1 |
| CR00245-dgRNA | GGAGATGGAATGAACACTTTTCGTCCCCTTTGAT--CTCATCTCACTGAGCTCTGGGCCG | 4.03% | -2 |
| CR00245-dgRNA | GGAGATGGAATGAACACTTTTCGTCCCCTTTG-TATCTCATCTCACTGAGCTCTGGGCCG | 2.45% | -1 |
| CR00245-dgRNA | GGAGATGGAATGAACACTTTTCGTCCCCTT--ATATCTCATCTCACTGAGCTCTGGGCCG | 1.85% | -2 |
| CR00245-dgRNA | GGAGA TGGAA TGAACACTTTTCGTCCCCTTTGA-----CA TCTCACTGAGCTCTGGGCCG | 1.38% | -5 |
| | GTGCCAGATGAACTTCCCAT**tgg** | | |
| g7BCL11a-BC-1sgRNA | CTGTGGGCAGTGCCAGATGAACTTCC-ATTGGGGGACATTCTTATTTTTATCGAGCACAA | 15.63% | -1 |
| g7BCL11a-BC-1sgRNA | CTGTGGGCAGTGCCAGATGAACTTC--ATTGGGGGACATTCTTATTTTTATCGAGCACAA | 7.36% | -2 |
| g7BCL11a-BC-1sgRNA | CTGTGGGCAGTGCCAGATGAACTTCCCcATTGGGGGACATTCTTATTTTTATCGAGCACAA | 6.88% | 1 |
| g7BCL11a-BC-1sgRNA | CTGTGGGCAGTGCCAGATGAAC-----ATTGGGGGACATTCTTATTTTTATCGAGCACAA | 2.81% | -5 |
| g7BCL11a-BC-1sgRNA | CTGTGGGCAGTGCCAGATGAACTTCC-TTGGGGGACATTCTTATTTTTATCGAGCACAA | 2.59% | -2 |
| g7BCL11a-BC-2sgRNA | CTGTGGGCAGTGCCAGATGAACTTCC-ATTGGGGGACATTCTTATTTTTATCGAGCACAA | 18.89% | -1 |
| g7BCL11a-BC-2sgRNA | CTGTGGGCAGTGCCAGATGAACTTC--ATTGGGGGACATTCTTATTTTTATCGAGCACAA | 9.14% | -2 |
| g7BCL11a-BC-2sgRNA | CTGTGGGCAGTGCCAGATGAACTTCCCcATTGGGGGACATTCTTATTTTTATCGAGCACAA | 5.52% | 1 |
| g7BCL11a-BC-2sgRNA | CTGTGGGCAGTGCCAGATGAACTTCC--TTGGGGGACATTCTTATTTTTATCGAGCACAA | 4.39% | -2 |
| g7BCL11a-BC-2sgRNA | CTGTGGGCAGTGCCAGATGAACT---CATTGGGGGACATTCTTATTTTTATCGAGCACAA | 3.18% | -3 |

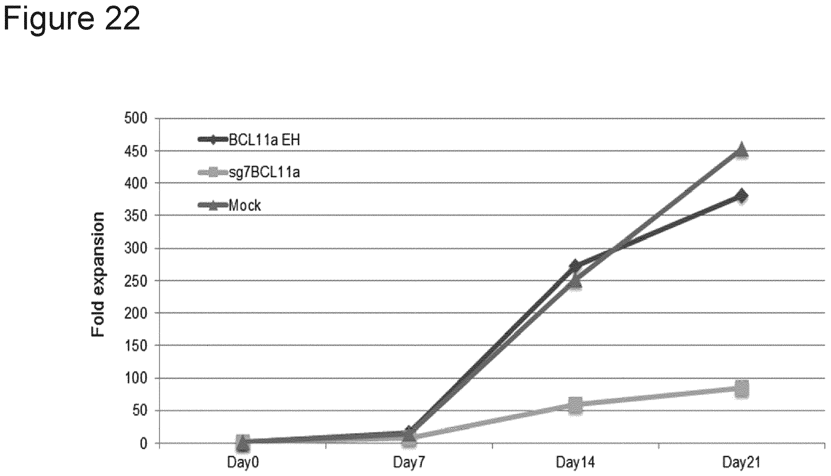

To determine whether CD34+ HSPC have retained their multilineage potential to differentiate into effector cells after ex vivo manipulations including genome editing, in vitro colony forming cell (CFC) unit assays were performed for select gRNAs. The genome edited cells were suspended in cytokine-supplemented methylcellulose and maintained for 14 days at 37°C in a humidified atmosphere of 5% CO2. Discrete colonies developed which were classified and counted based on the number and types of mature cells using morphological and phenotypic criteria (STEMvision) that they contain and scored for contribution to colony forming unit-erythroid (CFU-E), burst forming unit-erythroid (BFU-E), colony-forming unit - granulocyte/macrophage (CFU-GM) and colony-forming unit - granulocyte/erythrocyte/macrophage/megakaryocyte (CFU-GEMM; Figure 21). The colony forming potential of mock transfected cells was the highest followed by gRNAs targeting +58 erythroid enhancer of BCL11A. A similar number and types of colonies were observed for gRNAs targeting BCL11A erythroid enhancer. The guide RNA targeting exon 2 of BCL11A gave the least number of colonies (Figure 22).

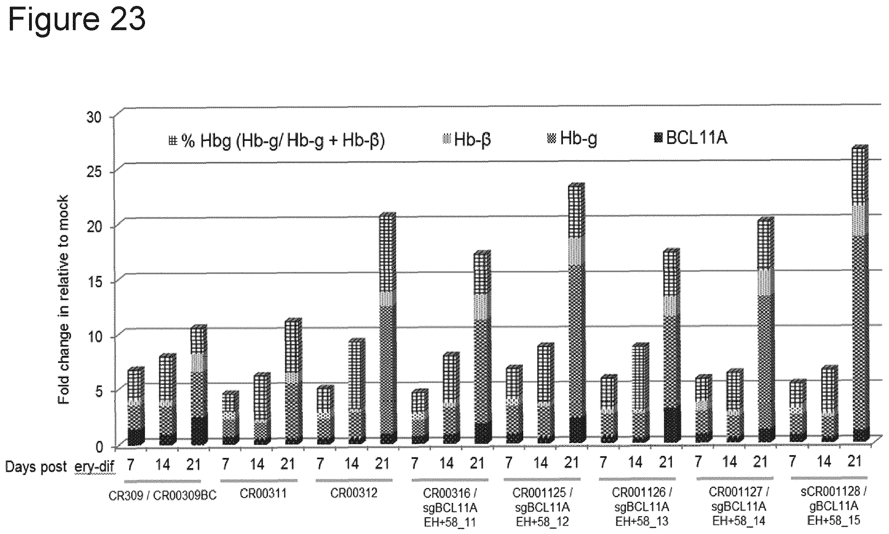

Relative mRNA expression levels of BCL11A, gamma- and beta-globin chains in unilineage erythroid cultures were quantified by Real-Time PCR. The transcript levels were normalized against human GAPDH transcript levels. The BCL11A mRNA levels were reduced in genome edited HSPC. In contrast, BCL11A mRNA levels remained the same in unedited or edited HSPC at the exon 2 of BCL11A on days 7 and 14 of erythroid differentiation (Figure 23). Gamma globin mRNA levels increased and correspondingly beta-globin mRNA decreased gradually in edited HSPC during erythroid differentiation (Figure 23). Increase in HbF and decrease in beta-globin (sickled globin) levels in erythrocytes derived from genome edited cells likely to have therapeutic implications by reduced sickling.

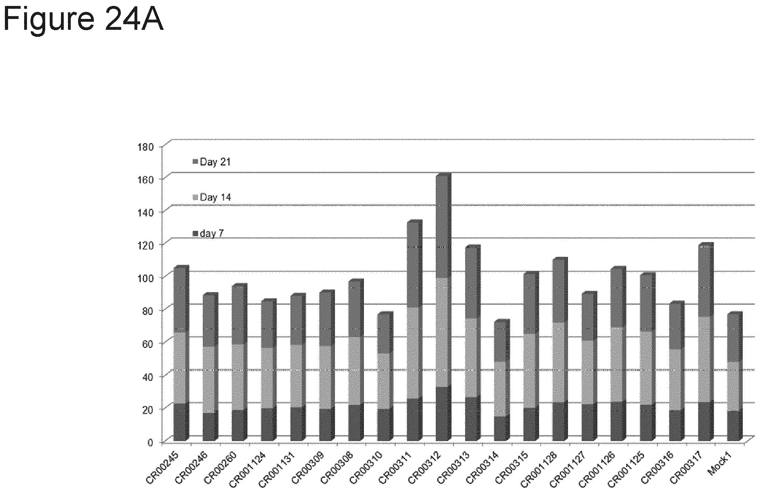

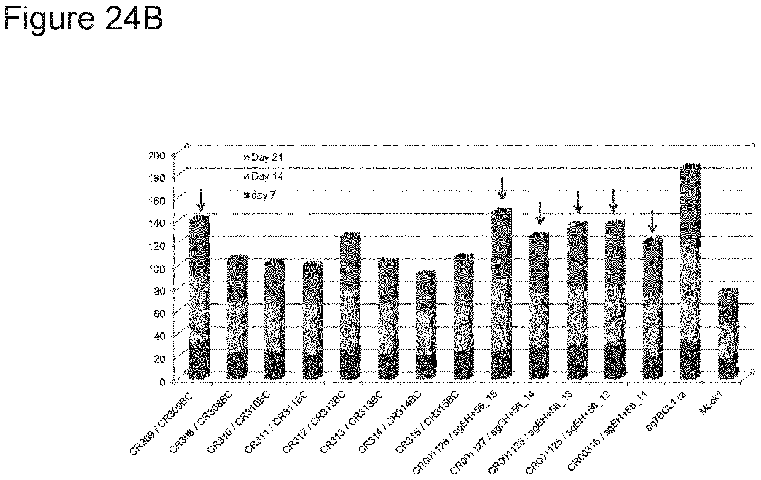

The genome edited and unedited HSPC at various stages (day 7, 14 and 21) of erythroid differentiation were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of 7AAD. Genome editing did not adversely affect erythroid differentiation as the cultured cells showed similar levels of CD71 and CD235A positivity consistent with late stage erythroblasts. Genome editing of +58 erythroid enhancer region resulted in a larger increase in HbF containing cells (up to 67%) than other gRNAs and the mock electroporated cells (Figure 24). Very high induction of HbF positive cells by g7 gRNA targeting exon 2 of BCL11A was observed. However, the cell proliferation and colony forming potential of exon 2 targeted cells was dramatically reduced (Figures 21 and 22).

Upon deep sequencing of the targeted region, we noticed that GATA1 and TALI binding motifs were intact in the genome edited target region (Figure 25). Recent literature demonstrated the importance of GATA1 binding site in regulation of BCL11A expression and derepression of fetal globin. Viestra et al., Nature Methods. 2015, 12:927. In contrast to the literature reports, which showed the importance of the two motifs for maintenance of BCL11A levels in adult erythroid cells, our findings demonstrate that sequences upstream of the two transcription factor binding sites are also important in regulation of BCL11a gene expression, and genome editing exclusively at these upstream sites leads to upregulation of HbF, even when the GATA1 and TAL1 binding sites remain intact.

### Example 3.1

### Methods:

Human CD34⁺ cell culture. Human CD34+ cells were isolated from G-CSF mobilized peripheral blood from adult donors (AllCells) using immunoselection (Miltenyi) according to the manufacturer's instructions and expanded for 3 days using StemSpan SFEM (StemCell Technologies; Cat no. 09650) supplemented with 50 ng/mL each of thrombopoietin (Tpo), Flt3 ligand (Flt-3L, Life Technologies, Cat. #PHC9413), human stem cell factor (SCF, Life Technologies, Cat. #PHC2113) and human interleukin-6 (IL-6, Life Technologies, Cat. #PHC0063), as well as 1x antibiotic/antimycotic (Gibco, Cat. #10378-016) and 500 nM compound 4.

Assembly of Cas9 and guide RNA ribonucleoprotein (RNP) complexes, preparation of HSPC, and electroporation of RNP into HSPC. Cas9-guide RNA ribonucleoprotein complexes (RNPs) were prepared immediately prior to electroporation. For formation of RNP using dual guide RNAs (dgRNAs), 3 µg of each of crRNA (in 2.24 µL) and tracr (in 1.25 µL) are first denatured at 95°C for 2 min in separate tubes and then cooled to room temperature. For preparation of Cas9 protein, 6 µg of CAS9 protein (in 1 µL) was mixed with 0.5 µL of 5 x CCE buffer (20 mM HEPES, 100 mM KCL, 5 mM MgCL2, 5% Glycerol and freshly added 1 mM DTT). Tracr was first mixed with the Cas9 preparation and incubated at 37°C for 5 min. The crRNA was then added to Tracr/CAS9 complexes and incubated for 5 min at 37°C. For the None/control condition, vehicle rather than crRNA was added to the Tracr/CAS9 complexes. The HSPC were collected by centrifugation and resuspended in P3 Primary Cell Solution (Lonza cat. No. PBP3-00675) at a cell density of 2.5 x 10⁶/mL. The RNP was mixed with 20 µL of cells by pipetting up and down and incubated at RT for 2 min. The RNP/cell mixture (20 µL) was electroporated using code CM-137 on the 4D-Nucleofector (Lonza). Duplicate 20 µL electroporations were performed. For the experiments in this Example 3.1, the tracr used was SEQ ID NO: 7808, and the crRNA had the following format and sequence, with 2'O-Methyl (m) and Phosphorothioate Bond (^{∗}) modifications indicated: mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the nucleotides of the indicated targeting domain (e.g., as indicated by the CRxxxxx identifier).

*In vitro* erythropoiesis and FACS analysis for HbF containing erythroid cells. After electroporation, the cells were immediately transferred into 250 µL pre-warmed erythroid differentiation medium (EDM) consisting of IMDM (Hyclone, Cat. #SH30228.01), 330 µg /mL human holo-transferrin (Invitria Cat# 777TRF029), 10 µg /mL recombinant human insulin (Gibco Cat#A1138211), 2 IU/mL heparin (Sigma, part #H3393), 5% human AB serum (Sigma, Cat# H4522), 125 ng/mL human erythropoietin (Peprotech #10779-058), and 1x antibiotic/antimycotic (Gibco, Cat. #10378-016). EDM was further supplemented with 1 µM hydrocortisone (Sigma H8672), 100 ng/mL human SCF (Life Technologies, Cat. #PHC2113), and 5 ng/mL human IL-3 (Peprotech #10779-598). After 2 days, the cell culture was diluted in fresh medium. Cultures were maintained for a total of 7 days, at which time the cells were analyzed by intracellular staining for HbF expression. Briefly, the cells were washed once with PBS, resuspended in LIVE/DEAD^{®} Fixable Violet Dead Cell Stain (ThermoFisher L34963; 1:1000 in PBS) and incubated for 30 min. Cell were then washed and stained with anti-CD71-BV711 (Fisher Scientific Company Llc. BDB563767) and anti-CD235a-APC (BD 551336) antibodies for 30 min. The cells were then washed, followed by fixation with fixation buffer (Biolegend, Cat# 420801) and permeabilized with 1x intracellular staining permeabilization wash buffer (Biolegend, Cat# 421002) according to the manufacturer's instructions. The cells were then incubated with 0.5 µL of anti-HbF-PE antibody (Life Technologies, part #MHFH04) in 50 µL of 1x intracellular staining perm wash buffer for 20 min at room temperature. The cells were washed twice with 2 mL of 1x intracellular staining Perm wash buffer and resuspend in staining buffer and analyzed on an LSRFortessa flow cytometer (BD Biosciences) for HbF expression. The results were analyzed using Flowjo and data were presented as % of HbF positive cells (F-cells) in the viable CD71 positive erythroid cell population.

Genomic DNA preparation and next generation sequencing (NGS). Genomic DNA was prepared from edited and unedited HSPC at 48 hours post-electroporation using Quick Extract DNA Extraction Solution (Epicentre Cat # QE09050). To determine editing efficiency and patterns of insertions and deletions (indels), PCR products were generated using primers flanking the target sites, which were then subjected to next generation sequencing (NGS). Percent editing of corresponding sequences in unedited samples was typically less than 1% and never exceeded 3%.

### Results:

Without being bound by theory, it is believed that targeted genetic disruption of the BCL11A erythroid enhancer region will relieve repression of γ-globin expression, allowing production of the red blood cells containing elevated HbF protein, (cells expressing fetal hemoglobin, e.g., expressing elevated levels of fetal hemoglobin, are sometimes referred to herein as "F-cells." In embodiments, a cell is considered an F-cell if it produces as least 6 picograms fetal hemoglobin per cell). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4, to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes.

The list and of gRNAs used in the study are shown in Table 17 (e.g., gRNAs comprising the targeting domain of the incidated CRxxxxxx identifier). The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 17. List of gRNAs targeting the BCL11A erythroid specific enhancer region used in the current study. All gRNA molecules were tested in duplicate in the dgRNA format as described in the materials and methods.**

| Guide RNA targeting domain ID | % HbF+ (F cells), average of replicates | %HbF+(F cells), standard deviation | % edited, average of replicates | % edited, standard deviation |
|---|---|---|---|---|
| CR000308 | 27.7 | 0.5 | 52.8 | 0.7 |
| CR000309 | 43.1 | 1.4 | 74.8 | 7.5 |
| CR000310 | 28.6 | 0.1 | 65.1 | 3.6 |
| CR000311 | 27.2 | 0.7 | 50.5 | 1.9 |
| CR000312 | 47.9 | 1.0 | 87.8 | 0.9 |
| CR000313 | 36.8 | 1.2 | 66.6 | 2.7 |
| CR000314 | 32.7 | 0.1 | 39.8 | 0.5 |
| CR000316 EH11 | 34.4 | 0.8 | 52.9 | 1.3 |
| CR000317 EH4 | 50.7 | 0.4 | 82.7 | 2.5 |
| CR001124 | 28.6 | 0.1 | 2.5 | 0.1 |
| CR001125 EH12 | 42.3 | 0.4 | 94.0 | 0.9 |
| CR001126 EH13 | 44.6 | 1.7 | 76.7 | 6.1 |
| CR001127 EH14 | 33.3 | 0.1 | 26.1 | 3.3 |
| CR001128 EH15 | 46.4 | 1.2 | 84.6 | 5.6 |
| CR001129 | 25.3 | 0.8 | 3.6 | 0.4 |
| CR001130 | 27.1 | 0.8 | 11.8 | 1.7 |
| CR001131 | 28.6 | 0.0 | 35.6 | 8.5 |
| None/control | 22.4 | 0.6 | n/a | n/a |
| g8 | 66.3 | 0.7 | 94.2 | 1.1 |

The genome edited and unedited HSPC were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of Live Dead Violet. BCL11A erythroid specific enhancer region targeting resulted in increased percentage of erythroid cells containing HbF (up to 50.7%) compared to mock electroporated cells (22.4%) (Table 17). Induction of HbF positive cells by dgRNA including the targeting domain of g8 targeting exon 2 of BCL11A was also observed in parallel. Genomic PCR products of the BCL11A erythroid enhancer region were also subjected to next generation sequencing (NGS) to determine the percentage of edited alleles in the cell population. The dgRNA treatments that resulted in greater than 40% HbF+ cells had a range of 74.8% to 94.0% editing (Table 17). For the same targeting domain, the dgRNA in this Example differed in tracr and crRNA sequence from that in Example 3; however, the editing and HbF induction were similar across dgRNA formats for most targeting domains. Of note, CR000317 _EH4 in the dgRNA format in the current study resulted in a high HbF induction to 50.7 % HbF+ cells. An increase in HbF-containing erythrocytes derived from genome edited cells is likely to have positive therapeutic implications by reduced sickling.

### Example 3.2. Optimization of gRNA editing and fetal hemoglobin upregulation using gRNAs targeting the +58 region of the BCL11a enhancer in HSPCs.

Based on the % editing results from the comprehensive screen of gRNAs targeting the +58 region of the BCL11a Enhancer, 8 gRNA targeting domains were selected for further study and optimization. To test the effect of modificaitons to the gRNA molecules on % editing, erythroid differentiation, and fetal hemoglobin expression, different versions of the eight gRNA molecules targeting sites within the +58 region of the BCL11a enhancer region were designed. The following versions of of gRNAs comprising the targeting domain of CR00309, CR00311, CR00312, CR00316, CR01125, CR01126, CR01127, and CR01128 were made:
dgRNA (all sequences other than those of the targeting domain as described in Example 1):
   1. Unmodified crRNA and unmodified tracr ("Unmodified" or "Unmod")
   2. Unmodified crRNA and unmodified tracr, but with only the 5' -18nt of the indicated targeting domain (not the full 20 nt) ("18nt")
   3. Three 3' nt and three 5' nt of crRNA modified with phosphorothioate linkages, and unmodified tracr ("PS")
   4. Additional inverted abasic residue at both 5'- and 3'- end of crRNA, and unmodified tracr ("Invd")
   5. Three 3' nt and three 5' nt of crRNA modified with phosphorothioate linkages and with 2'-Omethyl groups, and unmodified tracr ("OMePS")
   6. Three 3' nt and three 5' nt of crRNA modified with 2'-fluoro group, and unmodified tracr ("F")
   7. Three 3' nt and three 5' nt of crRNA modified with phosphorothioate linkages and with 2'-fluoro group, and unmodified tracr ("F-PS")
sgRNA (all sequences other than targeting domain are as described in Example 1):
   1. Unmodified sgRNA ("Unmodified" or "Unmod")
   2. Targeting domain consisting of only the 5'-18nt of the indicated targeting domain (not the full 20 nt) ("18nt")
   3. Three 3' nt and three 5' nt of sgRNA modified with phosphorothioate linkages ("PS")
   4. Additional inverted abasic residue at both 5' - and 3'- end of sgRNA ("Invd")
   5. Three 3' nt and three 5' nt of sgRNA modified with phosphorothioate linkages and with 2'-Omethyl groups ("OMePS")
   6. Three 3' nt and three 5' nt of sgRNA modified with 2'-fluoro group ("F")
   7. Three 3' nt and three 5' nt of sgRNA modified with phosphorothioate linkages and with 2'-fluoro group ("F-PS")

All other reagents and methods were as described in Example 3. All results were run in duplicate, and the results are reported in Figures 28-32. Figure 28A and Figure 28B show the % editing, as measured by NGS, in CD34+ cells 2 days after electroporation of RNP comprising the indicated dual guide RNAs (dgRNAs). Figure 29 shows the % editing, as measured by NGS, in CD34+ cells 2 days after electroporation of RNP comprising the indicated single guide RNAs (sgRNAs). Figure 30 shows the % HbF positive cells as measured by flow cytometry at day 14 following transfer of edited CD34+ cells to erythroid differentiation medium, after electroporation of RNP comprising the indicated dual guide RNAs (dgRNAs). Figure 31 shows the % HbF positive cells as measured by flow cytometry at day 14 following transfer of edited CD34+ cells to erythroid differentiation medium, after electroporation of RNP comprising the indicated single guide RNAs (sgRNAs). Figure 32 shows the fold-expansion of edited cells after 14 days in the erythroid differentiation medium. These results indicate that many of the selected gRNA molecules, in both dgRNA and sgRNA formats, are capable of achieving genome editing at the target site with greater than 90% efficiency, and in some cases, with greater than 98% efficiency, when delivered to CD34+ cells as RNP via electroporation. As well, many of the selected gRNAs were able to achieve a greater than 20% increase in % F cells relative to unmodified cells ("mock"). Finally, although genome editing of BCL11A erythroid enhancer in HSPC lowered the expansion capabilities of erythroid cells, with the effect more pronounced with some gRNAs (e.g., CR00309) than other gRNAs (e.g., CR00312), most edited cell populations differentiated into erythrocytes were non-the-less capable of achieving between 500-1500 population doublings in 14 days in erythroid differentiation medium ex vivo, indicating that the edited cells may be therapeutically useful in treating hemoglobinopathies such as sickle cell disease or beta thalassemia in patients.

### Example 4. HPFH region editing in Hematopoietic Stem and Progenitor Cells (HSPCs) using CRISPR-Cas9 for derepression of fetal globin expression in adult erythroid cells

### Methods:

Human CD34⁺ cell culture. Human CD34+ cells were isolated from G-CSF mobilized peripheral blood from adult donors (AllCells) using immunoselection (Miltenyi) according to the manufacturer's instructions and expanded for 4 to 6 days using StemSpan SFEM (StemCell Technologies; Cat no. 09650) supplemented with 50 ng/mL each of thrombopoietin (Tpo), Flt3 ligand (Flt-3L, Life Technologies, Cat. #PHC9413), human stem cell factor (SCF, Life Technologies, Cat. #PHC2113) and human interleukin-6 (IL-6, Life Technologies, Cat. #PHC0063), as well as 1x antibiotic/antimycotic (Gibco, Cat. #10378-016) and 500 nM compound 4. Throughout this example (including its subexamples), where the protocol indicates that cells were "expanded," this medium was used. This medium is also referred to as "stem cell expansion medium," or "expansion medium" throughout this example (including its subexamples).

Assembly of Cas9 and guide RNA ribonucleoprotein (RNP) complexes, preparation of HSPC, and electroporation of RNP into HSPC. Cas9-guide RNA ribonucleoprotein complexes (RNPs) were prepared immediately prior to electroporation. For formation of RNP using dual guide RNAs (dgRNAs), 3 µg of each of crRNA (in 2.24 µL) and tracr (in 1.25 µL) are first denatured at 95°C for 2 min in separate tubes and then cooled to room temperature. For preparation of Cas9 protein, 6 µg of CAS9 protein (in 0.8 to 1 µL) was mixed with 0.5 µL of 5 x CCE buffer (20 mM HEPES, 100 mM KCL, 5 mM MgCL2, 5% Glycerol and freshly added 1 mM DTT). Tracr was first mixed with the Cas9 preparation and incubated at 37°C for 5 min. The crRNA was then added to Tracr/CAS9 complexes and incubated for 5 min at 37°C. For the None/control condition, vehicle rather than crRNA was added to the Tracr/CAS9 complexes. The HSPC were collected by centrifugation and resuspended in T buffer that comes with Neon electroporation kit (Invitrogen; Cat#: MPK1096) at a cell density of 5 x 10⁶/mL. The RNP was mixed with 20 µL of cells by pipetting up and down and incubated at RT for 2 min. The RNP/cell mixture (10 µL) was transferred into Neon electroporation probe. The electroporation was performed with Neon transfection system (Invitrogen; MPK5000S) using 1700 volts/20 milliseconds and 1 pulse. Duplicate 10 µL electroporations were performed.

*In vitro* erythropoiesis and FACS analysis for HbF containing erythroid cells. After electroporation, the cells were immediately transferred into 250 µL pre-warmed erythroid differentiation medium (EDM) consisting of IMDM (Hyclone, Cat. #SH30228.01), 330 µg /mL human holo-transferrin (Invitria Cat# 777TRF029), 10 µg /mL recombinant human insulin (Gibco Cat#A1 138211), 2 IU/mL heparin (Sigma, part #H3393), 5% human AB serum (Sigma, Cat# H4522), 125 ng/mL human erythropoietin (Peprotech #10779-058), and 1x antibiotic/antimycotic (Gibco, Cat. #10378-016). EDM was further supplemented with 1 µM hydrocortisone (Sigma H8672), 100 ng/mL human SCF (Life Technologies, Cat. #PHC2113), and 5 ng/mL human IL-3 (Peprotech #10779-598). After 2 days, the cell culture was diluted in fresh medium. Cultures were maintained for a total of 7 days, at which time the cells were analyzed by intracellular staining for HbF expression. Briefly, the cells were washed once with PBS, resuspended in LIVE/DEAD^{®} Fixable Violet Dead Cell Stain (ThermoFisher L34963; 1:1000 in PBS) and incubated for 30 min. Cell were then washed and stained with anti-CD71-BV711 (Fisher Scientific Company Llc. BDB563767) and anti-CD235a-APC (BD 551336) antibodies for 30 min. The cells were then washed, followed by fixation with fixation buffer (Biolegend, Cat# 420801) and permeabilized with 1x intracellular staining permeabilization wash buffer (Biolegend, Cat# 421002) according to the manufacturers instructions. The cells were then incubated with 0.5 µL of anti-HbF-PE antibody (Life Technologies, part #MHFH04) in 50 µL of 1x intracellular staining perm wash buffer for 20 min at room temperature. The cells were washed twice with 2 mL of 1x intracellular staining Perm wash buffer and resuspend in staining buffer and analyzed on an LSRFortessa flow cytometer (BD Biosciences) for HbF expression. The results were analyzed using Flowjo and data were presented as % of HbF positive cells (F-cells) in the viable CD71 positive erythroid cell population.

Gene expression analysis. After 7 days of *in vitro* erythropoiesis as described, approximately 1 x 10⁵ cells were used to purify RNA using the Zymo Research ZR-96 quick RNA Kit (Zymo Cat# R1053). Up to 1µg RNA was used for 1st strand synthesis using Quantiscript Reverse Transcription Kit (Qiagen, Cat# 205313). Taq-man quantitative real-time PCR was performed using the 2x Taq-Man Fast Advance PR Mix (Life technologies, Cat# 4444963) and Taq-Man Gene Expression Assays for GAPDH (Life technologies, ID# Hs02758991_g1, VIC), HBB (Life technologies, ID# Hs00747223_g1, VIC) and HBG2/HBG1 (Life technologies, ID# Hs00361131_g1, FAM), according to the suppliers protocol. The relative expression of each gene was normalized to GAPDH expression and reported as fold of None/control RNP-delivered samples by the ΔΔCt method. Alternatively, after conversion to a GAPDH normalized relative quantity of transcript (2^-ΔCt), the HBG2/HBG1 expression level was calculated as a percentage of the sum of HBB and HBG2/HBG1 expression.

Genomic DNA preparation and next generation sequencing (NGS). Genomic DNA was prepared from edited and unedited HSPC at 48 hours post-electroporation using Quick Extract DNA Extraction Solution (Epicentre Cat # QE09050). To determine editing efficiency and patterns of insertions and deletions (indels), PCR products were generated using primers flanking the target sites, which were then subjected to next generation sequencing (NGS) as described in the literature. Percent editing of corresponding sequences in unedited samples (electroporated with RNPs consisting of Cas9 and Tracr only) was typically less than 1% and never exceeded 3%.

### Results:

Without being bound by theory, it is believed that targeted genetic disruption of the HPFH region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes. The list and sequences of gRNAs used in the study are shown in Table 16. The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 16. List of gRNAs targeting the HPFH region used in the current study. All gRNA molecules were tested in duplicate in the dgRNA format described above, except for g8 (crRNA consisting of mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the residues of the targeting domain, and modifications are indicated as follows: 2'O-Methyl (m) and Phosphorothioate Bond (^{∗}); with the tracr sequence SEQ ID NO: 7808) and None/control. The g8 and None/control were tested in 16 replicates each.**

| Guide RNA targeting domain ID | %HbF+(F cells), average of replicates | % HbF+ (F cells), standard deviation | % CD71+, average of replicates | % CD71+, standard deviation | % edited, average of replicates | % edited, standard deviation |
|---|---|---|---|---|---|---|
| CR001030 | 51.4 | 5.2 | 84.4 | 0.6 | 84.5 | 1.6 |
| CR001095 | 49.3 | 1.8 | 82.7 | 2.5 | 66.1 | 1.4 |
| CR001043 | 44.8 | 2.0 | 84.1 | 0.8 | 80.0 | 0.7 |
| CR001029 | 43.7 | 1.3 | 82.2 | 0.7 | 86.6 | 2.7 |
| CR001142 | 43.4 | 0.6 | 83.9 | 0.1 | 76.9 | 0.4 |
| CR001028 | 42.9 | 1.3 | 84.8 | 0.5 | 91.7 | 0.5 |
| CR001212 | 42.6 | 5.8 | 83.1 | 0.9 | 81.6 | 1.5 |
| CR001036 | 42.4 | 1.3 | 82.6 | 0.6 | 91.5 | 5.0 |
| CR001135 | 42.4 | 0.7 | 83.7 | 0.1 | 84.4 | 13.3 |
| CR001221 | 41.8 | 1.7 | 80.8 | 0.4 | 83.9 | 1.4 |
| CR001158 | 41.7 | 1.3 | 81.7 | 0.2 | 72.4 | 2.7 |
| CR001090 | 41.3 | 0.1 | 85.5 | 0.8 | 87.2 | 1.2 |
| CR001169 | 41.3 | 1.5 | 82.4 | 2.6 | 80.2 | 2.5 |
| CR001089 | 40.9 | 0.6 | 82.8 | 6.6 | 81.7 | 0.3 |
| CR001031 | 40.8 | 0.1 | 81.5 | 1.8 | 90.7 | 1.8 |
| CR001092 | 40.8 | 2.2 | 84.1 | 0.1 | 43.0 | 1.1 |
| CR001034 | 40.7 | 1.0 | 84.5 | 0.0 | 72.7 | 3.8 |
| CR001068 | 40.7 | 2.3 | 85.7 | 0.4 | 90.5 | 0.9 |
| CR001168 | 40.2 | 3.4 | 82.6 | 0.1 | 74.2 | 7.1 |
| CR001171 | 40.1 | 1.6 | 83.4 | 0.4 | 63.8 | 1.6 |
| CR001217 | 40.1 | 0.8 | 81.9 | 0.1 | 80.9 | 0.8 |
| CR001023 | 39.6 | 3.2 | 82.2 | 0.8 | 90.2 | 0.7 |
| CR001093 | 39.3 | 0.3 | 86 | 1.1 | 38.3 | 14.5 |
| CR001080 | 39.2 | 3.4 | 87.1 | 1.1 | 32.4 | 1.1 |
| CR001164 | 38.6 | 2.0 | 84.4 | 0.4 | 92.0 | 0.7 |
| CR001178 | 38.6 | 1.2 | 82.6 | 2.4 | 61.1 | 0.6 |
| CR001032 | 38.4 | 9.2 | 82.4 | 0.6 | 57.6 | 32.9 |
| CR001100 | 38.4 | 1.8 | 81.7 | 0.2 | 29.6 | 2.4 |
| CR001156 | 38.4 | 1.2 | 81.6 | 0.3 | 91.2 | 3.6 |
| CR001159 | 38.4 | 1.8 | 81.8 | 0.2 | 65.5 | 0.7 |
| CR001042 | 38.2 | 0.4 | 83.1 | 1.0 | 90.8 | 0.3 |
| CR001065 | 38.2 | 0.0 | 85.2 | 2.0 | 86.5 | 0.2 |
| CR001133 | 38.1 | 0.0 | 83.6 | 0.5 | 92.1 | 0.8 |
| CR001173 | 38 | 1.5 | 81.7 | 1.1 | 72.5 | 5.0 |
| CR001161 | 37.7 | 4.6 | 82.3 | 1.7 | 54.8 | 10.7 |
| CR001138 | 37.6 | 7.6 | 83.7 | 0.7 | 69.4 | 28.9 |
| CR001037 | 37.4 | 1.3 | 81.2 | 0.8 | 90.5 | 1.5 |
| CR001098 | 37.4 | 1.2 | 85.6 | 1.5 | 95.0 | 0.2 |
| CR001150 | 37.3 | 2.0 | 84.8 | 0.4 | 52.8 | 7.5 |
| CR001160 | 37.3 | 0.5 | 81.4 | 1.5 | 65.9 | 1.6 |
| CR001197 | 37.3 | 1.1 | 83.3 | 1.6 | 83.8 | 2.8 |
| CR001170 | 37.2 | 1.3 | 83.7 | 1.2 | 47.3 | 0.3 |
| CR001226 | 37 | 1.0 | 82.5 | 1.3 | 65.3 | 2.0 |
| CR001018 | 36.9 | 0.8 | 80.9 | 0.8 | 97.2 | 0.0 |
| CR001109 | 36.8 | 0.3 | 84.7 | 1.4 | 91.0 | 2.4 |
| CR001219 | 36.8 | 0.6 | 82.5 | 0.1 | 75.9 | 0.2 |
| CR001047 | 36.7 | 0.6 | 83.2 | 0.3 | 70.8 | 1.5 |
| CR001101 | 36.6 | 3.3 | 81.7 | 0.1 | 50.6 | 3.8 |
| CR001046 | 36.5 | 1.0 | 84 | 0.3 | 76.9 | 4.2 |
| CR001021 | 36.4 | 0.3 | 82.2 | 0.4 | 79.5 | 2.9 |
| CR001060 | 36.3 | 3.2 | 84.1 | 1.1 | 50.0 | 0.4 |
| CR001074 | 36.3 | 1.6 | 87.3 | 1.8 | 56.5 | 4.9 |
| CR001097 | 36.3 | 0.2 | 87.1 | 1.3 | 58.5 | 2.1 |
| CR001099 | 36.1 | 4.0 | 85.8 | 1.8 | 89.8 | 0.3 |
| CR001172 | 36.1 | 1.7 | 82.2 | 0.7 | 71.1 | 3.7 |
| CR001195 | 36.1 | 0.8 | 83.4 | 0.2 | 67.2 | 2.7 |
| CR001075 | 36 | 1.9 | 87.2 | 0.1 | 56.8 | 2.6 |
| CR001143 | 35.9 | 3.7 | 83.5 | 0.4 | 56.7 | 9.2 |
| CR001063 | 35.8 | 1.3 | 84.3 | 0.4 | 71.7 | 1.9 |
| CR001225 | 35.5 | 0.4 | 83.1 | 0.5 | 81.1 | 4.2 |
| CR001027 | 35.4 | 0.9 | 81.9 | 0.8 | 71.1 | 0.5 |
| CR001073 | 35.4 | 1.6 | 86.5 | 1.6 | 56.3 | 5.9 |
| CR001180 | 35.3 | 1.1 | 79.6 | 1.4 | 86.2 | 4.0 |
| CR001182 | 35.3 | 0.8 | 81.8 | 0.2 | 72.5 | 3.9 |
| CR001162 | 35.2 | 9.1 | 85 | 0.0 | 62.6 | 35.7 |
| CR001026 | 35.1 | 1.0 | 82.4 | 0.2 | 97.0 | 0.3 |
| CR001078 | 35 | 1.1 | 85 | 1.7 | 12.7 | 1.8 |
| CR001025 | 34.9 | 0.3 | 81.9 | 1.5 | 55.5 | 3.8 |
| CR001187 | 34.9 | 2.5 | 79.4 | 2.3 | 70.1 | 3.7 |
| CR001081 | 34.8 | 1.4 | 87.5 | 4.0 | 27.0 | 0.7 |
| CR001137 | 34.8 | 13.6 | 83.8 | 1.6 | 41.5 | 39.8 |
| CR001174 | 34.8 | 0.5 | 81.8 | 0.6 | 34.1 | 3.2 |
| CR001058 | 34.6 | 1.0 | 83.3 | 1.5 | 71.4 | 6.2 |
| CR001188 | 34.6 | 1.3 | 82.6 | 1.2 | 64.0 | 2.2 |
| CR001079 | 34.4 | 2.5 | 89.2 | 0.2 | 12.8 | 2.0 |
| CR001179 | 34.4 | 3.2 | 82 | 0.4 | 81.4 | 4.3 |
| CR001214 | 34 | 2.2 | 82.1 | 1.5 | 58.1 | 1.0 |
| CR001139 | 33.9 | 1.9 | 83 | 2.5 | 70.3 | 0.4 |
| CR001222 | 33.9 | 1.6 | 80.6 | 0.8 | 35.5 | 1.4 |
| CR001096 | 33.8 | 0.1 | 85.6 | 2.2 | 77.5 | 1.0 |
| CR001148 | 33.6 | 2.1 | 84.5 | 0.4 | 50.9 | 4.0 |
| CR001140 | 33.5 | 0.4 | 84 | 1.4 | 66.3 | 1.5 |
| CR001191 | 33.5 | 2.5 | 83.2 | 0.3 | 83.4 | 5.1 |
| CR001227 | 33.5 | 2.3 | 79 | 0.2 | 56.3 | 0.1 |
| CR001110 | 33.4 | 0.4 | 85.2 | 0.1 | 70.3 | 0.1 |
| CR001220 | 33.4 | 1.7 | 81.4 | 0.4 | 72.4 | 3.3 |
| CR001224 | 33.4 | 3.1 | 82.2 | 1.3 | 71.3 | 2.3 |
| CR001033 | 33.2 | 0.3 | 81.9 | 0.6 | 63.6 | 8.1 |
| CR001051 | 33.2 | 0.6 | 82.2 | 0.6 | 42.2 | 0.5 |
| CR001106 | 33.2 | 0.2 | 82.6 | 3.0 | 46.9 | 2.0 |
| CR001020 | 32.9 | 8.9 | 81.9 | 1.3 | 67.7 | 29.4 |
| CR001022 | 32.9 | 0.8 | 82.4 | 1.6 | 91.5 | 0.3 |
| CR001045 | 32.9 | 0.4 | 80.5 | 0.0 | 88.5 | 0.4 |
| CR001198 | 32.9 | 0.5 | 81.3 | 1.8 | 96.8 | 0.5 |
| CR001084 | 32.8 | 1.7 | 87.5 | 0.1 | 55.1 | 5.4 |
| CR001111 | 32.6 | 1.0 | 84.2 | 2.5 | 78.1 | 1.4 |
| CR001087 | 32.5 | 0.1 | 87.6 | 0.7 | 89.6 | 2.6 |
| CR001107 | 32.5 | 0.4 | 83.3 | 1.8 | 46.5 | 2.2 |
| CR001108 | 31.8 | 1.8 | 84.7 | 0.6 | 75.2 | 3.3 |
| CR001205 | 31.7 | 2.3 | 81.3 | 6.2 | 2.7 | 1.7 |
| CR001085 | 31.6 | 2.1 | 86 | 0.3 | 31.2 | 1.2 |
| CR001166 | 31.5 | 0.5 | 83.6 | 0.8 | 86.9 | 0.3 |
| CR001019 | 31.3 | 2.3 | 80.6 | 0.9 | 35.1 | 4.0 |
| CR001076 | 31.3 | 0.5 | 86.6 | 0.2 | 96.3 | 0.3 |
| CR001016 | 31.2 | 1.2 | 80.2 | 0.7 | 95.0 | 1.9 |
| CR001035 | 31.1 | 1.8 | 83.5 | 1.3 | 80.0 | 0.9 |
| CR001192 | 31 | 2.5 | 79.9 | 0.7 | 60.4 | 5.1 |
| CR001091 | 30.7 | 2.8 | 85.3 | 0.6 | 72.4 | 7.6 |
| CR001105 | 30.7 | 0.1 | 84.8 | 0.1 | 12.1 | 1.1 |
| CR001185 | 30.7 | 0.7 | 82.7 | 0.4 | 36.7 | 0.2 |
| CR001190 | 30.7 | 0.1 | 82.4 | 0.7 | 53.2 | 0.8 |
| CR001024 | 30.6 | 0.4 | 81.8 | 0.7 | 97.0 | 0.6 |
| CR001132 | 30.5 | 2.3 | 84.6 | 0.1 | 26.4 | 2.2 |
| CR001189 | 30.4 | 1.9 | 83.1 | 0.3 | 42.0 | 0.6 |
| CR001040 | 30.1 | 8.3 | 77.5 | 2.2 | 73.6 | 33.6 |
| CR001146 | 30.1 | 1.5 | 83.8 | 0.1 | 88.1 | 2.1 |
| CR001070 | 29.8 | 0.9 | 82.7 | 0.4 | 44.9 | 0.8 |
| CR001213 | 29.8 | 1.8 | 81.9 | 0.2 | 48.4 | 4.4 |
| CR001017 | 29.6 | 11.8 | 81.3 | 1.5 | 44.3 | 36.8 |
| CR001077 | 29.5 | 0.9 | 87.2 | 2.5 | 14.3 | 0.5 |
| CR001083 | 29.4 | 1.1 | 84.3 | 9.1 | 26.6 | 10.6 |
| CR001082 | 29 | 0.7 | 87.6 | 1.5 | 17.7 | 0.0 |
| CR001175 | 29 | 1.1 | 81.7 | 0.1 | 6.3 | 0.7 |
| CR001144 | 28.9 | 0.4 | 82.6 | 0.9 | 38.8 | 1.1 |
| CR001194 | 28.8 | 4.3 | 81.8 | 0.6 | 70.2 | 1.9 |
| CR001157 | 28.5 | 1.1 | 81 | 1.3 | 29.2 | 4.2 |
| CR001163 | 28.4 | 0.6 | 83.5 | 0.7 | 13.4 | 3.0 |
| CR001151 | 28.2 | 0.6 | 84.6 | 1.6 | 51.8 | 2.3 |
| CR001181 | 28.2 | 0.2 | 80.3 | 0.8 | 46.0 | 0.5 |
| CR001061 | 28 | 2.9 | 82.6 | 0.4 | 53.6 | 5.8 |
| CR001152 | 27.8 | 0.8 | 84.9 | 0.2 | 37.0 | 1.9 |
| CR001094 | 27.7 | 9.2 | 74.6 | 12.7 | 85.2 | 2.1 |
| CR001202 | 27.7 | 6.3 | 84 | 2.3 | 61.7 | 8.4 |
| CR001149 | 27.6 | 1.7 | 84.1 | 0.6 | 10.8 | 1.3 |
| CR001207 | 27.6 | 5.7 | 83.3 | 0.1 | 31.5 | 17.5 |
| CR001134 | 27.2 | 1.6 | 83.2 | 1.3 | 2.3 | 0.2 |
| CR001206 | 27.2 | 1.8 | 84.4 | 0.2 | 11.1 | 0.4 |
| CR001067 | 27 | 18.4 | 84.2 | 2.6 | 6.0 | 0.8 |
| CR001167 | 27 | 0.5 | 81.7 | 0.2 | 32.3 | 0.1 |
| CR001209 | 27 | 0.8 | 81.5 | 2.5 | 14.8 | 0.2 |
| CR001165 | 26.9 | 0.5 | 81.6 | 1.3 | 12.9 | 2.1 |
| CR001044 | 26.8 | 7.2 | 78.2 | 4.3 | 28.5 | 20.7 |
| CR001052 | 26.6 | 0.3 | 82.5 | 1.5 | 26.5 | 0.9 |
| CR001062 | 26.6 | 0.7 | 78.7 | 2.8 | 39.1 | 5.3 |
| CR001071 | 26.6 | 1.0 | 84.2 | 0.4 | 15.1 | 1.1 |
| CR001210 | 26.6 | 2.1 | 80.1 | 3.3 | 58.6 | 2.2 |
| CR001223 | 26.6 | 0.9 | 80.2 | 0.8 | 40.3 | 1.9 |
| CR001057 | 26.5 | 6.5 | 82.4 | 0.1 | 25.0 | 18.9 |
| CR001147 | 26.5 | 1.3 | 83.5 | 0.4 | 33.1 | 2.7 |
| CR001186 | 26.4 | 2.1 | 79.6 | 3.8 | 40.5 | 2.1 |
| CR001103 | 26.1 | 0.2 | 84.3 | 0.0 | 51.2 | 3.6 |
| CR001199 | 25.9 | 0.8 | 80.9 | 1.5 | 89.3 | 1.4 |
| CR001216 | 25.7 | 1.0 | 77.7 | 0.0 | 43.2 | 2.7 |
| CR001041 | 25.6 | 1.8 | 78.9 | 4.8 | 22.4 | 2.1 |
| CR001053 | 25.5 | 1.4 | 82.4 | 1.9 | 38.4 | 0.3 |
| CR001086 | 25.4 | 0.6 | 84.4 | 0.9 | 30.4 | 0.6 |
| CR001050 | 25.1 | 0.4 | 80.6 | 1.1 | 4.9 | 0.2 |
| CR001136 | 25.1 | 0.7 | 82.3 | 1.0 | 9.1 | 1.1 |
| CR001203 | 25.1 | 4.0 | 83.4 | 1.8 | 22.0 | 13.1 |
| CR001211 | 24.8 | 1.1 | 83.3 | 0.4 | 5.0 | 1.0 |
| CR001066 | 24.4 | 0.4 | 86.1 | 0.1 | 15.2 | 1.8 |
| CR001145 | 24.4 | 1.0 | 83.5 | 0.9 | 16.4 | 8.9 |
| CR001049 | 24.3 | 0.3 | 83.9 | 1.9 | 3.7 | 0.0 |
| CR001176 | 24.3 | 0.6 | 81.9 | 0.6 | 29.0 | 1.4 |
| CR001218 | 24.1 | 0.2 | 81.9 | 0.1 | 21.6 | 2.4 |
| CR001141 | 23.8 | 0.5 | 82.8 | 0.4 | 4.1 | 0.8 |
| CR001196 | 23.6 | 0.8 | 82.5 | 0.6 | 18.2 | 2.9 |
| CR001184 | 23.5 | 0.4 | 81.1 | 0.4 | 43.5 | 1.6 |
| CR001204 | 23.3 | 1.1 | 81.5 | 0.7 | 0.5 | 0.2 |
| CR001069 | 23.1 | 1.1 | 85 | 0.0 | 16.2 | 0.3 |
| CR001072 | 23 | 0.3 | 84.2 | 0.7 | 15.6 | 0.3 |
| CR001102 | 22.8 | 1.7 | 84.7 | 1.6 | 58.1 | 4.8 |
| CR001153 | 22.8 | 1.3 | 82.8 | 0.1 | 17.2 | 2.6 |
| CR001177 | 22.8 | 0.5 | 82.2 | 0.9 | 2.0 | 0.2 |
| CR001104 | 22.7 | 1.7 | 84.8 | 1.1 | 29.4 | 5.0 |
| CR001064 | 22.6 | 1.1 | 82.1 | 1.0 | 35.8 | 5.2 |
| CR001200 | 22.6 | 0.1 | 82 | 0.0 | 9.8 | 0.7 |
| CR001154 | 22.4 | 0.0 | 82.2 | 1.3 | 8.0 | 1.1 |
| CR001055 | 22.3 | 1.0 | 83.4 | 1.1 | 19.0 | 0.8 |
| CR001054 | 22 | 1.1 | 83.5 | 1.0 | 11.3 | 1.4 |
| CR001039 | 21.9 | 0.2 | 80.8 | 0.2 | 16.6 | 1.5 |
| CR001183 | 21.6 | 0.7 | 81.4 | 0.8 | 13.3 | 0.1 |
| CR001155 | 21.4 | 0.0 | 81.6 | 0.8 | 5.0 | 1.0 |
| CR001059 | 21.1 | 0.6 | 82.5 | 1.2 | 4.6 | 0.3 |
| CR001056 | 20.8 | 0.9 | 83.2 | 0.2 | 3.7 | 0.9 |
| CR001193 | 20.5 | 0.9 | 80.4 | 0.9 | 4.8 | 0.2 |
| CR001208 | 20.5 | 0.1 | 81.1 | 0.9 | 12.6 | 3.0 |
| CR001201 | 20.1 | 1.7 | 81.7 | 0.6 | 10.7 | 0.7 |
| CR001048 | 19.3 | 3.0 | 82.3 | 1.1 | 0.4 | 0.3 |
| CR001215 | 19.2 | 0.2 | 81.2 | 2.3 | 5.6 | 2.2 |
| CR001038 | 19.1 | 0.1 | 80.8 | 1.3 | 6.5 | 1.1 |
| CR001088 | 18.8 | 5.9 | 67.3 | 2.7 | 74.6 | 2.3 |
| None/control | 21.0 | 2.0 | 82.9 | 1.9 | n/a | n/a |
| g8 | 59.2 | 3.0 | 74.3 | 4.4 | 95.1 | 1.8 |

The genome edited and unedited HSPC were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of Live Dead Violet. Genome editing did not adversely affect erythroid differentiation as the cultured cells showed percentages of CD71+ cells consistent with erythroblasts similar to uneditied cells. HPFH region targeting resulted in increased percentage of erythroid cells containing HbF (up to 51.4%) compared to mock electroporated cells (21.0%) (Table 16). Induction of HbF positive cells by dgRNA including the targeting domain of g8 targeting exon 2 of BCL11A was also observed in parallel. Genomic PCR products of the HPFH region were also subjected to next generation sequencing (NGS) to determine the percentage of edited alleles in the cell population. High genome editing percentages at the HFPH locus was observed in many of the cell cultures electroporated with RNPs containing Cas9, crRNA of the given targeting domain and Tracr (Table 16), but not in control cells with no targeting domain delivered (RNPs containing only Cas9 and Tracr). In particular, the dgRNA treatments that resulted in greater than 40% HbF+ cells had a range of 43.0% to 91.7% edited alleles (Table 16).

**Table 18. Gene expression analysis of select cultures edited with gRNAs targeting the HPFH region in the current study. All gRNA molecules were tested in duplicate in the dgRNA format. Fetal gamma-globin (γ-globin, HBG2/HBG1 genes), adult beta-globin (β-globin, HBB gene) and Glyceraldehyde-3-Phosphate Dehydrogenase (GAPDH gene) expression were determined and reported as described in Materials and Methods.**

| Guide RNA targeting domain ID | Number of electroporation replicates | γ-globin expression, fold over control (average of replicates) | β-globin expression, fold over control (average of replicates) | % globin expression, γ/(γ+β) (average of replicates) |
|---|---|---|---|---|
| CR001030 | 2 | 3.8 | 0.91 | 26.6 |
| CR001142 | 2 | 1.6 | 0.76 | 20.0 |
| CR001212 | 2 | 2.2 | 0.58 | 18.6 |
| CR001036 | 2 | 2.6 | 0.91 | 19.7 |
| CR001221 | 2 | 2.3 | 0.79 | 15.2 |
| CR001217 | 2 | 1.9 | 0.72 | 13.8 |
| None/control | 5 | 1 | 1 | 7.7 |

Some cultures in this study were also analyzed for hemoglobin gene expression. Of these targeting domains, fetal gamma-globin gene expression was induced 1.6 to 3.8-fold over None/control, which was accompanied by a modest reduction in adult beta-globin expression (0.58 to 0.91-fold of None/control) (Table 18). These effects together resulted in gamma-globin expression levels ranging from 13.8% to 26.6% of the total beta-type globins (γ/[γ+β]) in the cell population (Table 17), with relative fetal globin induction across targeting domains similar to that observed for HbF protein induction (Table 16). Either an increase in HbF / gamma-globin or an increase in HbF / gamma-globin together with a decrease in beta-globin (sickled globin) levels in erythrocytes derived from genome edited cells is likely to have therapeutic implications by reduced sickling.

### Example 4.2

Methods: Methods are as in Example 4, with the following exceptions.

Assembly of Cas9 and guide RNA ribonucleoprotein (RNP) complexes, preparation of HSPC, and electroporation of RNP into HSPC. The HSPC collected by centrifugation were resuspended in P3 Primary Cell Solution (Lonza cat. No. PBP3-00675) at a cell density of 2.5 x 10⁶/mL. The RNP was mixed with 20 µL of cells by pipetting up and down and incubated at RT for 2 min. The RNP/cell mixture (20 µL) was electroporated using code CM-137 on the 4D-Nucleofector (Lonza).

### Results:

Without being bound by theory, it is believed that targeted genetic disruption of the HPFH region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes. The list of gRNAs used in the study are shown in Table 19. The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 19. List of gRNAs targeting the HPFH region used in the current study. All gRNA molecules were tested in the dgRNA format (except for g8 which was tested in the dgRNA format using : crRNA consisting of mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the residues of the targeting domain, and modifications are indicated as follows: 2'O-Methyl (m) and Phosphorothioate Bond (^{∗}); with the tracr sequence SEQ ID NO: 7808).**

| Guide RNA targeting domain ID | % HbF+ (F cells) | % CD71+ |
|---|---|---|
| CR003037 | 46.6 | 80.3 |
| CR003033 | 45.4 | 75.9 |
| CR003038 | 42.9 | 77.6 |
| CR003035 | 42.1 | 77.8 |
| CR003087 | 42.1 | 76.7 |
| CR003085 | 41.8 | 77.6 |
| CR003052 | 39.9 | 80.5 |
| CR003080 | 38.0 | 80.1 |
| CR003081 | 35.3 | 80.6 |
| CR003031 | 34.8 | 77.7 |
| CR003056 | 34.3 | 78.3 |
| CR003070 | 33.9 | 81.2 |
| CR003089 | 33.8 | 77.8 |
| CR003065 | 33.0 | 76.4 |
| CR003075 | 33.0 | 75.2 |
| CR003088 | 33.0 | 77.3 |
| CR003041 | 32.1 | 76.2 |
| CR003039 | 31.9 | 75.7 |
| CR003044 | 30.6 | 77.7 |
| CR003084 | 29.7 | 77.2 |
| CR003073 | 29.5 | 76.9 |
| CR003066 | 29.3 | 77.9 |
| CR003082 | 29.3 | 78.6 |
| CR003067 | 29.2 | 78.1 |
| CR003074 | 29.1 | 77.5 |
| CR003095 | 28.8 | 80.4 |
| CR003083 | 28.3 | 76.6 |
| CR003030 | 27.9 | 78.4 |
| CR003069 | 27.7 | 78.9 |
| CR003055 | 27.6 | 78.9 |
| CR003058 | 27.6 | 78.8 |
| CR003054 | 27.5 | 79.0 |
| CR003029 | 27.1 | 78.4 |
| CR003086 | 27.1 | 76.6 |
| CR003063 | 27.0 | 76.0 |
| CR003071 | 26.5 | 79.5 |
| CR003079 | 26.5 | 78.0 |
| CR003042 | 26.4 | 77.0 |
| CR003045 | 26.3 | 78.0 |
| CR003034 | 25.9 | 76.8 |
| CR003027 | 25.7 | 79.2 |
| CR003043 | 25.0 | 76.0 |
| CR003032 | 24.9 | 77.9 |
| CR003040 | 24.9 | 75.7 |
| CR003028 | 24.8 | 78.6 |
| CR003048 | 24.8 | 80.4 |
| CR003057 | 24.7 | 79.0 |
| CR003096 | 24.6 | 78.9 |
| CR003077 | 24.4 | 77.7 |
| CR003036 | 24.3 | 76.8 |
| CR003049 | 24.3 | 78.4 |
| CR003047 | 24.0 | 78.2 |
| CR003059 | 23.9 | 80.2 |
| CR003051 | 23.7 | 77.2 |
| CR003053 | 23.7 | 76.9 |
| CR003091 | 23.7 | 80.2 |
| CR003093 | 23.7 | 79.2 |
| CR003060 | 23.6 | 78.9 |
| CR003072 | 23.6 | 77.7 |
| CR003076 | 23.6 | 78.0 |
| CR003078 | 23.6 | 78.0 |
| CR003064 | 23.4 | 75.9 |
| CR003046 | 23.3 | 80.5 |
| CR003092 | 22.9 | 81.7 |
| CR003090 | 22.7 | 79.7 |
| CR003050 | 22.6 | 77.2 |
| CR003062 | 22.1 | 76.5 |
| CR003061 | 21.5 | 76.3 |
| CR003068 | 21.5 | 78.8 |
| CR003094 | 21.0 | 79.6 |
| None/control replicate 1 | 18.9 | 77.0 |
| None/control replicate 2 | 21.1 | 79.7 |
| g8 replicate 1 | 56.8 | 65.4 |
| g8 replicate 2 | 56.4 | 65.8 |

The genome edited and unedited HSPC were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of Live Dead Violet. Genome editing did not adversely affect erythroid differentiation as the cultured cells showed percentages of CD71+ cells consistent with erythroblasts similar to uneditied cells. HPFH region targeting resulted in increased percentage of erythroid cells containing HbF (up to 46.6%) compared to mock electroporated cells (18.9 and 21.1%) (Table 19). Induction of HbF positive cells by dgRNA including the targeting domain of g8 targeting exon 2 of BCL11A was also observed in parallel.

Finally, the indel pattern created at or near the target site for dgRNAs comprising the targeting domains of CR003031, CR003033, CR003035, CR003037, CR003038, CR003052, CR003085 were assessed by NGS. The top 5 most frequent indels at each location are shown in Table 27.

**Table 27.5 most frequent indels at each taget sequence after exposure to RNP comprising the indicated gRNA molecule. Each gRNA was tested in dgRNA. Capital letters are the naturally occurring nucleotides at and near the target sequence. Deletions relative to the unmodified target sequence are shown as "-"; insertions are shown as lowercase letters.**

| **dgRNA targeting domain** | **Variant Read (Genomic Sequence)** | **% of all reads** | **Indel Length** |
|---|---|---|---|
| CR003031 | 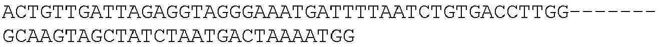 | 9.74% | -7 |
| CR003031 | 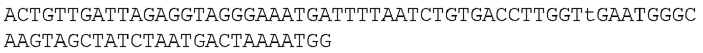 | 7.82% | 1 |
| CR003031 | 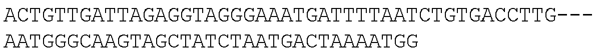 | 2.19% | -3 |
| CR003031 | 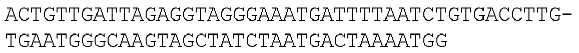 | 1.37% | -1 |
| CR003031 | ACTGTTGATTAGAGGTAGGGAAATGATTTTAATCTGTGA---- ATGGGCAAGTAGCTATCTAATGACTAAAATGG | 1.33% | -9 |
| CR003031 | | ... | ... |
| CR003033 | ATGGGCAAGTAGCTATCTAATGACTAAAATGGAA------ GAGAAACAGTTTTAGTATAACAAGTGAAATACCCAT | 25.21% | -10 |
| CR003033 | ATGGGCAAGTAGCTATCTAATGACTAAAATGGAAAAaCACTGGAAGAGAAACAGT TTTAGTATAACAAGTGAAATACCCAT | 7.77% | 1 |
| CR003033 | ATGGGCAAGTAGCTATCTAATGACTAAAATGGAA-- CACTGGAAGAGAAACAGTTTTAGTATAACAAGTGAAATACCCAT | 5.72% | -2 |
| CR003033 | ATGGGCAAGTAGCTATCTAATGACTAAAATGGAAAAC-- TGGAAGAGAAACAGTTTTAGTATAACAAGTGAAATACCCAT | 2.94% | -2 |
| CR003033 | ATGGGCAAGTAGCTATCTAATGACTAAAATGGAAAACcACTGGAAGAGAAACAGT TTTAGTATAACAAGTGAAATACCCAT | 1.68% | 1 |
| CR003033 | | ... | ... |
| CR003035 | CACTGGAAGAGAAACAGTTTTAGTATAACAAGTGAAATACCCATG--- AGTCTGAGGTGCCTATAGGACATCTATATAAA | 10.87% | -3 |
| CR003035 | CACTGGAAGAGAAACAGTTTTAGTATAACAAGTGAAATACCCAT- CTGAGTCTGAGGTGCCTATAGGACATCTATATAAA | 2.62% | -1 |
| CR003035 | CACTGGAAGAGAAACAGTTTTAGTATAACAAGTGAAATACCCA-- CTGAGTCTGAGGTGCCTATAGGACATCTATATAAA | 2.04% | -2 |
| CR003035 | CACTGGAAGAGAAACAGTTTTAGTATAACAAGTGAAATACCCATG-- GAGTCTGAGGTGCCTATAGGACATCTATATAAA | 1.79% | -2 |
| CR003035 | CACTGGAAGAGAAACAGTTTTAGTATAACAAGTGAAATACCC---- TGAGTCTGAGGTGCCTATAGGACATCTATATAAA | 1.77% | -4 |
| CR003035 | | ... | ... |
| CR003037 | TAACAAGTGAAATACCCATGCTGAGTCTGAGG---------- ACATCTATATAAATAAGCCCAGTACATTGTTTGATATA | 7.19% | -10 |
| CR003037 | TAACAAGTGAAATACCCATGCTGAGTCTGAGGTGCC- ATAGGACATCTATATAAATAAGCCCAGTACATTGTTTGATATA | 6.13% | -1 |
| CR003037 | TAACAAGTGAAATACCCATGCTGAGTCTGAGGTGCCcTATAGGACATCTATATAA ATAAGCCCAGTACATTGTTTGATATA | 4.17% | 1 |
| CR003037 | TAACAAGTGAAATACCCATGCTGAGTCTGAGGTGCCTtATAGGACATCTATATAA ATAAGCCCAGTACATTGTTTGATATA | 3.89% | 1 |
| CR003037 | TAACAAGTGAAATACCCATGCTGAGTCTGAGGTGC- TATAGGACATCTATATAAATAAGCCCAGTACATTGTTTGATATA | 3.72% | -1 |
| CR003037 | | ... | ... |
| CR003038 | TAGTATAACAAGTGAAATACCCATGCTGAGTCTGAGGTGCCTATA---- -TAAATAAGCCCAGTACATTGTTTG | 14.85% | -11 |
| CR003038 | TAGTATAACAAGTGAAATACCCATGCTGAGTCTGAGGTGCCTATAG- ACATCTATATAAATAAGCCCAGTACATTGTTTG | 6.25% | -1 |
| CR003038 | TAGTATAACAAGTGAAATACCCATGCTGAGTCTGAGGTGCCTAT- GGACATCTATATAAATAAGCCCAGTACATTGTTTG | 3.99% | -1 |
| CR003038 | TAGTATAACAAGTGAAATACCCATGCTGAGTCTGAGGTGCCTATAGGgACATCTA TATAAATAAGCCCAGTACATTGTTTG | 2.83% | 1 |
| CR003038 | TAGTATAACAAGTGAAATACCCATGCTGAGTCTGAGGTGCCTATAaGGACATCTA TATAAATAAGCCCAGTACATTGTTTG | 2.76% | 1 |
| CR003038 | | ... | ... |
| CR003052 | TCAGAGGTTAGAAATCAGAGTTGGGAATTGGATTA-- CAGGCTGTATTTAAGAGTTTAGATATAACTGTGAATCCAAGA | 14.04% | -2 |
| CR003052 | TCAGAGGTTAGAAATCAGAGTTGGGAATTGGGATTAaTACAGGCTGTATTTAAGA GTTTAGATATAACTGTGAATCCAAGA | 2.94% | 1 |
| CR003052 | TCAGAGGTTAGAAATCAGAGTTGGGAATTGGGATT- TACAGGCTGTATTTAAGAGTTTAGATATAACTGTGAATCCAAGA | 2.41% | -1 |
| CR003052 | TCAGAGGTTAGAAATCAGAGTTGGGAATTGGGATTATtACAGGCTGTATTTAAGA GTTTAGATATAACTGTGAATCCAAGA | 2.37% | 1 |
| CR003052 | TCAGAGGTTAGAAATCAGAGTTGGGAATTGGATTAaaTACAGGCTGTATTTAAG AGTTTAGATATAACTGTGAATCCAAGA | 2.28% | 2 |
| CR003052 | | ... | ... |
| CR003085 | TGTAAGGAGGATGAGCCACATGGTATGGGAGGTAT- CTAAGGACTCTAGGGTCAGAGAAATATGGGTTATATCCTTCTAC | 12.03% | -1 |
| CR003085 | TGTAAGGAGGATGAGCCACATGGTATGGGAGG----- ACTCTAGGGTCAGAGAAATATGGGTTATATCCTTCTAC | 8.70% | -10 |
| CR003085 | TGTAAGGAGGATGAGCCACATGGTATGGGA---------- CTCTAGGGTCAGAGAAATATGGGTTATATCCTTCTAC | 4.42% | -13 |
| CR003085 | TGTAAGGAGGATGAGCCACATGGTATGGGAGGTATAaCTAAGGACTCTAGGGTCA GAGAAATATGGGTTATATCCTTCTAC | 4.38% | 1 |
| CR003085 | TGTAAGGAGGATGAGCCACATGGTATGGGAGGTA----- AGGACTCTAGGGTCAGAGAAATATGGGTTATATCCTTCTAC | 3.41% | -5 |
| CR003085 | | ... | ... |
| CR003087 | GATGAGCCACATGGTATGGGAGGTATACTAAGGACTtCTAGGGTCAGAGAAATAT GGGTTATATCCTTCTACAAAATTCAC | 24.73% | 1 |
| CR003087 | GATGAGCCACATGGTATGGGAGGTATACTA----------- GGGTCAGAGAAATATGGGTTATATCCTTCTACAAAATTCAC | 15.83% | -9 |
| CR003087 | GATGAGCCACATGGTATGGGAGGTATACTAAGG------ GTCAGAGAAATATGGGTTATATCCTTCTACAAAATTCAC | 8.50% | -8 |
| CR003087 | GATGAGCCACATGGTATGGGAGGTATACTAAGGACT-- AGGGTCAGAGAAATATGGGTTATATCCTTCTACAAAATTCAC | 7.07% | -2 |
| CR003087 | GATGAGCCACATGGTATGGGAGGTATACTAAGG--------- TCAGAGAAATATGGGTTATATCCTTCTACAAAATTCAC | 1.75% | -9 |
| CR003087 | | ... | ... |

These results suppor the conclusion from earlier experiments described herein that small indels (e.g., in this case, indels from 1-20 nucleotides) in the HPFH region targeted by the indicated gRNA molecule can have a significant impact on expression and production of HbF. An increase in HbF-containing erythrocytes derived from genome edited cells is likely to have therapeutic implications by reduced sickling.

### Example 4.3

Methods: Methods are as in Example 4, with the following exceptions.

Human CD34⁺ cell culture. Human CD34+ cells were expanded for 3 days prior to RNP delivery in expansion medium.

Assembly of Cas9 and guide RNA ribonucleoprotein (RNP) complexes, preparation of HSPC, and electroporation of RNP into HSPC. The HSPC collected by centrifugation were resuspended in P3 Primary Cell Solution (Lonza cat. No. PBP3-00675) at a cell density of 6.4 x 10⁶/mL. The RNP was mixed with 20 µL of cells by pipetting up and down and incubated at RT for 2 min. The RNP/cell mixture (20 µL) was electroporated using code CM-137 on the 4D-Nucleofector (Lonza). Electroporations were performed in duplicate. The Tracr was SEQ ID NO: 7808 and the crRNA had the following format with 2'O-Methyl (m) and Phosphorothioate Bond (^{∗}) modifications indicated: mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the nucleotides of the targeting domain.

*In vitro* erythropoiesis and FACS analysis for HbF containing erythroid cells. After electroporation, the cells were immediately transferred into pre-warmed medium consisting of EDM and supplements. During days 0-7 of culture, EDM was further supplemented with 1 µM hydrocortisone (Sigma H8672), 100 ng/mL human SCF (Life Technologies, Cat. #PHC2113), and 5 ng/mL human IL-3 (Peprotech #10779-598). During days 7-11 of culture, EDM was supplemented with 100 ng/mL of human SCF only. During days 11-21 of culture, EDM had no additional supplements. Cell cultures were initiated at 2.6×10⁴ cells per ml on day 0, adjusted to 4.0×10⁴ cells per ml on day 7, and adjusted to 1.0×10⁶ cells per ml on day 11. On days 14 and 19, the media was replenished. Viable cells were counted at 1, 4, 7, 11, 14 and 21 days post-RNP delivery. All viable cell counts were determined by flow cytometry using AccuCheck Counting Beads (ThermoFisher cat. No. PCB100) with inviable cells discriminated by DAPI (4',6-Diamidino-2-Phenylindole) staining. On days 7, 16 and 21 of the culture, the cells (1 x 10⁵) were analyzed by intracellular staining for HbF expression. On days 16 and 21 intracellular staining for HbF expression did not include anti-CD71 and anti-CD235a antibodies, and % of HbF positive cells (F-cells) in the entire viable cell population was reported. On day 21 LIVE/DEAD^{®} Fixable Near-IR Dead Cell Stain (ThermoFisher L34975) was used as the viability dye.

Genomic DNA preparation and next generation sequencing (NGS). Genomic DNA was prepared from edited and unedited HSPC at 7 days post-electroporation using Quick Extract DNA Extraction Solution (Epicentre Cat # QE09050).

### Results:

Without being bound by theory, it is believed that targeted genetic disruption of the HPFH region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes. The list of gRNAs used in the study are shown in Table 20. The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 20. Relative viable cell proliferation in the current study. Individual electroporation replicates are shown. All gRNA molecules were tested in the dgRNA format using the tracr SEQ ID NO: 7808, and the crRNA had the following format with 2'O-Methyl (m) and Phosphorothioate Bond (^{∗}) modifications indicated: mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the nucleotides of the targeting domain. (n.d. not determined)**

| Guide RNA targeting domain ID and replicate | Day 0 | Day 1 | Day 4 | Day 7 | Day 11 | Day 14 | Day 21 |
|---|---|---|---|---|---|---|---|
| CR001030 replicate 1 | 1 | 0.66 | 4.3 | 43.1 | 627 | 680 | 772 |
| CR001030 replicate 2 | 1 | 0.65 | 4.0 | 38.4 | 651 | 694 | 880 |
| CR001028 replicate 1 | 1 | 0.61 | 4.0 | 33.2 | 588 | 509 | 742 |
| CR001028 replicate 2 | 1 | 0.77 | 6.1 | 44.9 | 724 | 759 | 838 |
| CR001095 replicate 1 | 1 | 0.66 | 4.0 | 40.7 | 665 | 503 | 835 |
| CR001095 replicate 2 | 1 | 0.96 | 5.2 | 42.2 | 651 | 777 | n.d. |
| CR001212 replicate 1 | 1 | 0.58 | 3.4 | 33.6 | 465 | 414 | 495 |
| CR001212 replicate 2 | 1 | 0.74 | 3.6 | 32.4 | 490 | 519 | 572 |
| g8 replicate 1 | 1 | 0.61 | 4.4 | 31.8 | 221 | 341 | 91 |
| g8 replicate 2 | 1 | 0.60 | 5.2 | 33.2 | 269 | 270 | 103 |
| None/control replicate 1 | 1 | 0.79 | 7.6 | 58.5 | 741 | 862 | 707 |
| None/control replicate 2 | 1 | 0.71 | 8.2 | 51.4 | 923 | 1204 | 1075 |

The genome edited and unedited HSPC were analyzed for proliferation in a three-stage erythroid differentiation cell culture protocol. Percent cell recovery at 1 day after electroporation ranged from 58% to 96% and was similar across conditions, including the unedited but electroporated control (Table 20). Proliferation was suppressed in the cells edited by dgRNA including the targeting domain of g8 targeting exon 2 of BCL11A (10-12% of unedited control at day 21, Table 20), but proliferation of cells edited with the included HPFH region targeting dgRNAs was similar to control (47-99% of unedited control at day 21, Table 20).

**Table 21. HbF induction in the current study. Individual electroporation replicates are shown. All gRNA molecules were tested in the dgRNA format. All gRNA molecules were tested in the dgRNA format using the tracr SEQ ID NO: 7808, and the crRNA had the following format with 2'O-Methyl (m) and Phosphorothioate Bond (^{∗}) modifications indicated: mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the nucleotides of the targeting domain. (n.d. not determined)**

| Guide RNA targeting domain ID and replicate | % edited, Day 7 | % CD71+, Day 7 | %HbF+ (F cells), Day 7 | % HbF+ (F cells), Day 16 | %HbF+ (F cells), Day 21 |
|---|---|---|---|---|---|
| CR001030 replicate 1 | 90.7 | 91.2 | 61.0 | 45.4 | 53.7 |
| CR001030 replicate 2 | 90.1 | 91.1 | 63.9 | 44.3 | 55.9 |
| CR001028 replicate 1 | 91.9 | 90.0 | 61.6 | 40.9 | 52.0 |
| CR001028 replicate 2 | 87.6 | 90.7 | 56.3 | 46.6 | 45.8 |
| CR001095 replicate 1 | 25.7 | 91.4 | 51.3 | 36.8 | 43.7 |
| CR001095 replicate 2 | 30.5 | 91.1 | 52.5 | 42.7 | n.d. |
| CR001212 replicate 1 | 86.2 | 90.8 | 57.5 | 38.7 | 45.2 |
| CR001212 replicate 2 | 80.9 | 90.4 | 54.7 | 36.5 | 42.7 |
| g8 replicate 1 | 95.3 | 84.6 | 70.7 | 71.9 | 63.4 |
| g8 replicate 2 | 94.3 | 82.6 | 70.0 | 72.6 | 54.8 |
| None/control replicate 1 | n/a | 94.0 | 27.1 | 24.4 | 22.8 |
| None/control replicate 2 | n/a | 93.8 | 26.2 | 25.7 | 21.1 |

The genome edited and unedited HSPC were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of Live/Dead Fixable Dead Cell Stain. Genome editing did not adversely affect erythroid differentiation as the cultured cells showed percentages of CD71+ cells consistent with erythroblasts similar to uneditied cells at day 7 of differentiation. Cultures treated with the included HPFH region targeting dgRNAs resulted in increased percentage of erythroid cells containing HbF compared to mock electroporated cells throughout the 21 day culture period (Table 21). Induction of HbF positive cells by dgRNA including the targeting domain of g8 targeting exon 2 of BCL11A was also observed in parallel. Genomic PCR products of the HPFH region were also subjected to next generation sequencing (NGS) to determine the percentage of edited alleles in the cell population. Genome editing at the HFPH locus was observed in cell cultures electroporated with RNPs containing Cas9, crRNA of the given targeting domain and Tracr (Table 21), but not in control cells with no targeting domain delivered (RNPs containing only Cas9 and Tracr). An increase in HbF-containing erythrocytes derived from genome edited cells is likely to have therapeutic implications by reduced sickling.

### Example 4.4

Methods: Methods are as in Example 4, with the following exceptions.

Human CD34⁺ cell culture. Human CD34+ cells were expanded for 3 to 6 days prior to RNP delivery in expansion medium, depending on study.

Assembly of Cas9 and guide RNA ribonucleoprotein (RNP) complexes, preparation of HSPC, and electroporation of RNP into HSPC. For some studies, the RNP complexes were delivered using the 4D-Nucleofector (Lonza). In those studies, the HSPC collected by centrifugation were resuspended in P3 Primary Cell Solution (Lonza cat. No. PBP3-00675) at a cell density of 2.2 x 10⁶/mL to 6.4 x 10⁶/mL, depending on study. The RNP was mixed with 20 µL of cells by pipetting up and down and incubated at RT for 2 min. The RNP/cell mixture (20 µL) was electroporated using code CM-137 on the 4D-Nucleofector (Lonza). Electroporations were performed in duplicate. The dgRNA format varied between studies and is indicated in Table 22 by the following notation. Form A is the dgRNA format described above in Example 1, with the targeting domain sequence indicated. Form B is a dgRNA format using a crRNA of rNrNrNrNrNrNrNrNrNrNrNrNrNrNrNrNrNrNrNrNrGrUrUrUrUrArGrArGrCrUrArUrGrCrU, where r indicates an RNA base and the N's correspond to the indicated targeting domain sequence, and a tracr consisting of SEQ ID NO: 7808. Form C is a dgRNA format using crRNA of the sequence mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where r indicates an RNA base, N's corresponds to the indicated targeting domain sequence, m indicates a base with 2'O-Methyl modification, and ^{∗} indicates a phosphorothioate bond, and the tracr consists of SEQ ID NO: 7808.

*In vitro* erythropoiesis and FACS analysis for HbF containing erythroid cells. In one study, cell cultures were initiated at 2.6×10⁴ cells per ml on day 0. For those initiated by seeding directly into 250 µl (as described in Example 4), the cell culture was diluted in fresh medium after 1 to 3 days, depending on the study.

Genomic DNA preparation and next generation sequencing (NGS). Genomic DNA was prepared from edited and unedited HSPC at 24 hours to 7 days post-electroporation, depending on the study, using Quick Extract DNA Extraction Solution (Epicentre Cat # QE09050).

### Results:

Without being bound by theory, it is believed that targeted genetic disruption of the HPFH region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes. The list of gRNAs used in the studies are shown in Table 22. The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 22. Summary data for multiple studies evaluating indicated gRNAs targeting the HPFH region and controls. All gRNA molecules were tested in dgRNA formats. Details of the dgRNA formats in the indicated studies are specified as described above. Note that evaluations 4 and 5 were performed in parallel and thus share control conditions.**

| Guide RNA targeting domain ID | | Evaluation number | | | | | | | |
|---|---|---|---|---|---|---|---|---|---|
| | | 1 | 2 | 3 | 4 | 5 | 6 | 7 | 8 |
| CR001028 | Format | A | A | B | B | C | | | C |
| | Replicates | 2 | 2 | 2 | 2 | 2 | | | 2 |
| | %HbF+ (F cells), average of replicates | 42.9 | 39.7 | 47.4 | 51.1 | 55.3 | | | 59.0 |
| | % edited, average of replicates | 91.7 | 78.0 | 87.5 | 90.9 | 84.6 | | | 89.8 |
| CR001030 | Format | A | A | B | B | C | C | C | C |
| | Replicates | 2 | 2 | 2 | 2 | 2 | 2 | 2 | 2 |
| | %HbF+ (F cells), | 51.4 | 48.7 | 50.9 | 51.8 | 58.0 | 39.7 | 50.6 | 62.5 |
| | average of replicates | | | | | | | | |
| | % edited, average of replicates | 84.5 | 61.8 | 74.3 | 75.0 | 85.6 | 94.1 | 85.4 | 90.4 |
| CR001036 | Format | A | A | B | | | | | |
| | Replicates | 2 | 2 | 2 | | | | | |
| | %HbF+ (F cells), average of replicates | 42.4 | 40.8 | 43.3 | | | | | |
| | % edited, average of replicates | 84.4 | 78.3 | 92.6 | | | | | |
| CR001043 | Format | A | A | B | | | | | |
| | Replicates | 2 | 2 | 2 | | | | | |
| | %HbF+ (F cells), average of replicates | 44.8 | 33.0 | 42.9 | | | | | |
| | % edited, average of replicates | 80.0 | 42.7 | 87.7 | | | | | |
| CR001080 | Format | A | A | | | C | | | |
| | Replicates | 2 | 2 | | | 2 | | | |
| | %HbF+ (F cells), average of replicates | 39.2 | 34.9 | | | 49.3 | | | |
| | % edited, average of replicates | 32.4 | 24.0 | | | 41.6 | | | |
| CR001095 | Format | A | A | B | B | C | C | C | C |
| | Replicates | 2 | 2 | 1 | 2 | 2 | 2 | 2 | 2 |
| | %HbF+ (F cells), average of replicates | 49.3 | 41.5 | 47 | 51.8 | 51.8 | 32.9 | 46.8 | 51.9 |
| | % edited, average of replicates | 66.1 | 49.8 | 65.1 | 64.6 | 64.2 | 66.8 | 56.1 | 28.1 |
| CR001137 | Format | A | A | | | | C | C | |
| | Replicates | 2 | 2 | | | | 2 | 2 | |
| | %HbF+ (F cells), average of replicates | 34.8 | 45.6 | | | | 31.0 | 40.8 | |
| | % edited, average of replicates | 41.5 | 46.9 | | | | 69.0 | 57.9 | |
| CR001142 | Format | A | A | | | C | | | |
| | Replicates | 2 | 2 | | | 2 | | | |
| | %HbF+ (F cells), average of replicates | 43.4 | 45.5 | | | 48.1 | | | |
| | % edited, average of replicates | 76.9 | 63.7 | | | 89.2 | | | |
| CR001158 | Format | A | A | | | C | | | |
| | Replicates | 2 | 2 | | | 2 | | | |
| | %HbF+ (F cells), average of replicates | 41.7 | 36.9 | | | 44.6 | | | |
| | % edited, average of replicates | 72.4 | 43.9 | | | 78.4 | | | |
| CR001212 | Format | A | A | B | B | C | C | C | C |
| | Replicates | 2 | 2 | 2 | 2 | 2 | 2 | 2 | 2 |
| | %HbF+ (F cells), average of replicates | 42.6 | 46.9 | 43.8 | 44.5 | 53.5 | 39.4 | 47.1 | 56.1 |
| | % edited, average of replicates | 81.6 | 58.1 | 48.6 | 47.5 | 69.2 | 82.0 | 67.0 | 83.6 |
| CR001217 | Format | A | A | | | | C | C | |
| | Replicates | 2 | 2 | | | | 2 | 2 | |
| | %HbF+ (F cells), average of replicates | 40.1 | 41.5 | | | | 29.9 | 45.9 | |
| | % edited, average of replicates | 80.9 | 50.3 | | | | 89.0 | 82.1 | |
| CR001221 | Format | A | A | B | B | C | | | |
| | Replicates | 2 | 2 | 1 | 2 | 2 | | | |
| | %HbF+ (F cells), average of replicates | 41.8 | 45.2 | 44.6 | 46.2 | 48.6 | | | |
| | % edited, average of replicates | 83.9 | 71.1 | 73.6 | 70.7 | 92.1 | | | |
| g8 | Format | C | C | c | c | c | | c | c |
| | Replicates | 16 | 2 | 2 | 2 | 2 | | 2 | 2 |
| | %HbF+ (F cells), average of replicates | 59.2 | 60.0 | 66.3 | 64.7 | 64.7 | | 55.8 | 70.4 |
| | % edited, average of replicates | 95.1 | 85.4 | 94.2 | 94.6 | 94.6 | | 91.7 | 94.8 |
| None/control | Format | n/a | n/a | n/a | n/a | n/a | n/a | n/a | n/a |
| | Replicates | 16 | 2 | 2 | 2 | 2 | 2 | 2 | 2 |
| | %HbF+ (F cells), average of replicates | 21.0 | 20.3 | 22.4 | 22.4 | 22.4 | 13.9 | 24.0 | 26.7 |
| | % edited, average of replicates | n/a | n/a | n/a | n/a | n/a | n/a | n/a | n/a |

The genome edited and unedited HSPC were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of Live Dead Violet. HPFH region targeting resulted in increased percentage of erythroid cells containing HbF compared to mock electroporated cells, a consistent result across multiple evaluations and dgRNA formats (Table 22). In some instances, an increase in %HbF+ cells was associated with a particular dgRNA form, for example Form C for CR001028 (Table 22). Induction of HbF positive cells by dgRNA including the targeting domain of g8 targeting exon 2 of BCL11A was also observed in parallel. Genomic PCR products of the HPFH region were also subjected to next generation sequencing (NGS) to determine the percentage of edited alleles in the cell population. Genome editing at the HFPH locus was observed in cell cultures electroporated with RNPs containing Cas9, crRNA of the given targeting domain and Tracr (Table 22), but not in control cells with no targeting domain delivered (RNPs containing only Cas9 and Tracr).

Finally, the indel pattern as well as frequency of each indel was assessed for each gRNA by NGS. The most frequently occurring indels at each target site are shown in Table 26.

**Table 26.5 most frequent indels at each taget sequence after exposure to RNP comprising the indicated gRNA molecule. Each gRNA was tested in dgRNA format in two separate experiments, and the results are shown seperately for each experiment. Capital letters are the naturally occurring nucleotides at and near the target sequence. Deletions relative to the unmodified target sequence are shown as "-"; insertions are shown as lowercase letters.**

| Experiment Number | dgRNA targeting domain | Variant Read (Genomic Sequence) | % of all reads | Indel Length |
|---|---|---|---|---|
| 1 | CR001028 | TTCTGTCATGTGTGTCTTGACTCAGAAACCCTGTTCTCCTCTA- ATATCTCCCCACCGCATCTCTTTCAGCAGTTGTTTC | 13.26% | -1 |
| 1 | CR001028 | TTCTGTCATGTGTGTCTTGACTCAGAAACCCTGTTCTCC----- CCACCGCATCTCTTTCAGCAGTTGTTTC | 12.48% | -13 |
| 1 | CR001028 | TTCTGTCATGTGTGTCTTGACTCAGAAACCCTGTTCTCCTCTA---- TCTCCCCACCGCATCTCTTTCAGCAGTTGTTTC | 6.04% | -4 |
| 1 | CR001028 | TTCTGTCATGTGTGTCTTGACTCAGAAACCCTGTTCTCCTCTA-- TATCTCCCCACCGCATCTCTTTCAGCAGTTGTTTC | 3.94% | -2 |
| 1 | CR001028 | TTCTGTCATGTGTGTCTTGACTCAGAAACCCTGTTCTCCTC----- ACCGCATCTCTTTCAGCAGTTGTTTC | 3.65% | -13 |
| 1 | CR001028 | | ... | ... |
| 2 | CR001028 | TTCTGTCATGTGTGTCTTGACTCAGAAACCCTGTTCTCC----- CCACCGCATCTCTTTCAGCAGTTGTTTC | 14.30% | -13 |
| 2 | CR001028 | TTCTGTCATGTGTGTCTTGACTCAGAAACCCTGTTCTCCTCTA- ATATCTCCCCACCGCATCTCTTTCAGCAGTTGTTTC | 11.51% | -1 |
| 2 | CR001028 | TTCTGTCATGTGTGTCTTGACTCAGAAACCCTGTTCTCCTCTA-- TATCTCCCCACCGCATCTCTTTCAGCAGTTGTTTC | 5.63% | -2 |
| 2 | CR001028 | TTCTGTCATGTGTGTCTTGACTCAGAAACCCTGTTCTCCTCTA---- TCTCCCCACCGCATCTCTTTCAGCAGTTGTTTC | 4.98% | -4 |
| 2 | CR001028 | TTCTGTCATGTGTGTCTTGACTCAGAAACCCTGTTCTCCTC----- ACCGCATCTCTTTCAGCAGTTGTTTC | 4.91% | -13 |
| 2 | CR001028 | | ... | ... |
| 1 | CR001030 | TCTTGACTCAGAAACCCTGTTCTCCTCTACATATCTC------ TTTCAGCAGTTGTTTCTAAAAATATCCTCC | 9.10% | -13 |
| 1 | CR001030 | TCTTGACTCAGAAACCCTGTTCTCCTCTACATATCTCCCCACC- CATCTCTTTCAGCAGTTGTTTCTAAAAATATCCTCC | 4.77% | -1 |
| 1 | CR001030 | TCTTGACTCAGAAACCCTGTTCTCCTCTACATATCTCCCCACC-- ATCTCTTTCAGCAGTTGTTTCTAAAAATATCCTCC | 3.54% | -2 |
| 1 | CR001030 | TCTTGACTCAGAAACCCTGTTCTCCTCTACATATCTCCCCACCGgCATCTCTTTCA GCAGTTGTTTCTAAAAATATCCTCC | 2.51% | 1 |
| 1 | CR001030 | TCTTGACTCAGAAACCCTGTTCTCCTCTACATATCTCCCCA----- TCTCTTTCAGCAGTTGTTTCTAAAAATATCCTCC | 2.39% | -5 |
| 1 | CR001030 | | ... | ... |
| 2 | CR001030 | TCTTGACTCAGAAACCCTGTTCTCCTCTACATATCTC------ TTTCAGCAGTTGTTTCTAAAAATATCCTCC | 9.67% | -13 |
| 2 | CR001030 | TCTTGACTCAGAAACCCTGTTCTCCTCTACATATCTCCCCACC- CATCTCTTTCAGCAGTTGTTTCTAAAAATATCCTCC | 4.87% | -1 |
| 2 | CR001030 | TCTTGACTCAGAAACCCTGTTCTCCTCTACATATCTCCCCACC-- ATCTCTTTCAGCAGTTGTTTCTAAAAATATCCTCC | 3.33% | -2 |
| 2 | CR001030 | TCTTGACTCAGAAACCCTGTTCTCCTCTACATATCTCCCCACCGgCATCTCTTTCA GCAGTTGTTTCTAAAAATATCCTCC | 2.93% | 1 |
| 2 | CR001030 | TCTTGACTCAGAAACCCTGTTCTCCTCTACATATCTCCCCAC- GCATCTCTTTCAGCAGTTGTTTCTAAAAATATCCTCC | 1.85% | -1 |
| 2 | CR001030 | | ... | ... |
| 1 | CR001036 | CCTAGTTTCATTTTTGCAGAAGTGTTTTAGGCTAAT- TAGTGGAATGTATCTTAGAGTTTAACTTATTTGTTTCTGTCAC | 19.08% | -1 |
| 1 | CR001036 | CCTAGTTTCATTTTTGCAGAAGTGTTTTAGGCTAATA-- GTGGAATGTATCTTAGAGTTTAACTTATTTGTTTCTGTCAC | 17.13% | -2 |
| 1 | CR001036 | CCTAGTTTCATTTTTGCAGAAGTGTTTTAG---------- TGGAATGTATCTTAGAGTTTAACTTATTTGTTTCTGTCAC | 3.55% | -10 |
| 1 | CR001036 | CCTAGTTTCATTTTTGCAGAAGTGTTTTAGGCTAATAaTAGTGGAATGTATCTTAG AGTTTAACTTATTTGTTTCTGTCAC | 2.42% | 1 |
| 1 | CR001036 | CCTAGTTTCATTTTTGCAGAAGTGTTTTAGGCTAATA- AGTGGAATGTATCTTAGAGTTTAACTTATTTGTTTCTGTCAC | 2.38% | -1 |
| 1 | CR001036 | | ... | ... |
| 2 | CR001036 | CCTAGTTTCATTTTTGCAGAAGTGTTTTAGGCTAATA-- GTGGAATGTATCTTAGAGTTTAACTTATTTGTTTCTGTCAC | 22.26% | -2 |
| 2 | CR001036 | CCTAGTTTCATTTTTGCAGAAGTGTTTTAGGCTAAT- TAGTGGAATGTATCTTAGAGTTTAACTTATTTGTTTCTGTCAC | 19.77% | -1 |
| 2 | CR001036 | CCTAGTTTCATTTTTGCAGAAGTGTTTTAG---------- TGGAATGTATCTTAGAGTTTAACTTATTTGTTTCTGTCAC | 6.25% | -10 |
| 2 | CR001036 | CCTAGTTTCATTTTTGCAGAAGTGTTTTAGGCTAATA- AGTGGAATGTATCTTAGAGTTTAACTTATTTGTTTCTGTCAC | 1.96% | -1 |
| 2 | CR001036 | CCTAGTTTCATTTTTGCAGAAGTGTTTTAGG------- AATGTATCTTAGAGTTTAACTTATTTGTTTCTGTCAC | 1.41% | -12 |
| 2 | CR001036 | | ... | ... |
| 1 | CR001043 | TAGAGATTTTTGTCTCCAAGGGAATTTTGAGAGGTTG- AATGGACAAATCTATTGCTGCAGTTTAAACTTGCTTGCTTCC | 5.28% | -1 |
| 1 | CR001043 | TAGAGATTTTTGTCTCCAAGGGAATTTTGAGAGGTTGGA----- CAAATCTATTGCTGCAGTTTAAACTTGCTTGCTTCC | 2.67% | -5 |
| 1 | CR001043 | TAGAGATTTTTGTCTCCAAGGGAATTTTGA--------- ATGGACAAATCTATTGCTGCAGTTTAAACTTGCTTGCTTCC | 2.62% | -9 |
| 1 | CR001043 | TAGAGATTTTTGTCTCCAAGGGAATTTTGAGAGG-------- ACAAATCTATTGCTGCAGTTTAAACTTGCTTGCTTCC | 2.22% | -9 |
| 1 | CR001043 | TAGAGATTTTTGTCTCCAAGGGAATTTTGAGAGGTTGGgAATGGACAAATCTATTG CTGCAGTTTAAACTTGCTTGCTTCC | 2.06% | 1 |
| 1 | CR001043 | | ... | ... |
| 2 | CR001043 | TAGAGATTTTTGTCTCCAAGGGAATTTTGAGAGGTTG- AATGGACAAATCTATTGCTGCAGTTTAAACTTGCTTGCTTCC | 5.87% | -1 |
| 2 | CR001043 | TAGAGATTTTTGTCTCCAAGGGAATTTTGAGAGG-------- ACAAATCTATTGCTGCAGTTTAAACTTGCTTGCTTCC | 3.11% | -9 |
| 2 | CR001043 | TAGAGATTTTTGTCTCCAAGGGAATTTTGAGAGGTTGGA----- CAAATCTATTGCTGCAGTTTAAACTTGCTTGCTTCC | 2.42% | -5 |
| 2 | CR001043 | TAGAGATTTTTGTCTCCAAGGGAATTTTGAGAGGTTGGgAATGGACAAATCTATTG CTGCAGTTTAAACTTGCTTGCTTCC | 1.90% | 1 |
| 2 | CR001043 | TAGAGATTTTTGTCTCCAAGGGAATTTTGA--------- ATGGACAAATCTATTGCTGCAGTTTAAACTTGCTTGCTTCC | 1.72% | -9 |
| 2 | CR001043 | | ... | ... |
| 1 | CR001080 | GAGAAAGGAAGGAAGGAAGAGGGAAGAGAGAGGATG- AAGGGATGGAGGAGAAGAAGGAAAAATAAATAATGGAGAGGA | 2.72% | -1 |
| 1 | CR001080 | GAGAAAGGAAGGAAGGAAGAGGGGAAGAGAGAGGATGGA----- GGAGAAGAAGGAAAAATAAATAATGGAGAGGA | 2.22% | -9 |
| 1 | CR001080 | GAGAAAGGAAGGAAGGAAGAGGGAAGAGAGAGG----- GATGGAGGAGAAGAAGGAAAAATAAATAATGGAGAGGA | 1.44% | -8 |
| 1 | CR001080 | GAGAAAGGAAGGAAGGAAGAGGGGAAGAGAGAGGATGGgAAGGGATGGAGGAGAAG AAGGAAAAATAAATAATGGAGAGGA | 1.41% | 1 |
| 1 | CR001080 | GAGAAAGGAAGGAAGGAAGAGGGGAAGAGAGAGGA---- AGGGATGGAGGAGAAGAAGGAAAAATAAATAATGGAGAGGA | 1.11% | -4 |
| 1 | CR001080 | | ... | ... |
| 2 | CR001080 | GAGAAAGGAAGGAAGGAAGAGGGGAAGAGAGAGGATGGA----- GGAGAAGAAGGAAAAATAAATAATGGAGAGGA | 3.43% | -9 |
| 2 | CR001080 | GAGAAAGGAAGGAAGGAAGAGGGAAGAGAGAGGATG- AAGGGATGGAGGAGAAGAAGGAAAAATAAATAATGGAGAGGA | 3.17% | -1 |
| 2 | CR001080 | GAGAAAGGAAGGAAGGAAGAGGGGAAGAGA---------- AGGGATGGAGGAGAAGAAGGAAAAATAAATAATGGAGAGGA | 1.61% | -9 |
| 2 | CR001080 | GAGAAAGGAAGGAAGGAAGAGGGGAAGAGAGAGGA---- AGGGATGGAGGAGAAGAAGGAAAAATAAATAATGGAGAGGA | 1.28% | -4 |
| 2 | CR001080 | GAGAAAGGAAGGAAGGAAGAGGGAAGAGAGAGG----- GATGGAGGAGAAGAAGGAAAAATAAATAATGGAGAGGA | 1.16% | -8 |
| 2 | CR001080 | | ... | ... |
| 1 | CR001095 | GAGAAAGAGAAAGGAAGGAAGAGAGGAAAGAAGA-- AGAGGAGAGAAAAGAAACGAAGAGAGGGGAAGGAAGGAAAA | 5.94% | -2 |
| 1 | CR001095 | GAGAAAGAGAAAGGAAGGAAGAGAGGAAAGAAGAG----- GAGAGAAAAGAAACGAAGAGAGGGAAGGAAGGAAAA | 5.54% | -5 |
| 1 | CR001095 | GAGAAAGAGAAAGGAAGGAAGAGAGGA-------------------- GAGAAAAGAAACGAAGAGAGGGGAAGGAAGGAAAA | 4.02% | -15 |
| 1 | CR001095 | GAGAAAGAGAAAGGGAAGGAAGAGAGGAAAGAA------ | 3.23% | -20 |
| | | ACGAAGAGAGGGGAAGGGAAGGAAAA | | |
| 1 | CR001095 | GAGAAAGAGAAAGGAAGGAAGAGAGGAAAGA-------- GGAGAGAAAAGAAACGAAGAGAGGGGAAGGAAGGAAAA | 2.49% | -8 |
| 1 | CR001095 | | ... | ... |
| 2 | CR001095 | GAGAAAGAGAAAGGAAGGAAGAGAGGAAAGAAGAG----- GAGAGAAAAGAAACGAAGAGAGGGAAGGAAGGAAAA | 7.89% | -5 |
| 2 | CR001095 | GAGAAAGAGAAAGGAAGGAAGAGAGGAAAGAAGA-- AGAGGAGAGAAAAGAAACGAAGAGAGGGAAGGAAGGAAAA | 6.18% | -2 |
| 2 | CR001095 | GAGAAAGAGAAAGGAAGGAAGAGAGGA----------- GAGAAAAGAAACGAAGAGAGGGGAAGGAAGGAAAA | 4.16% | -15 |
| 2 | CR001095 | GAGAAAGAGAAAGGAAGGAAGAGAGGAAAGAAGAGgAAGAGGAGAGAAAAGAAA CGAAGAGAGGGGAAGGAAGGAAAA | 2.77% | 1 |
| 2 | CR001095 | GAGAAAGAGAAAGGAAGGGA------------------ GGAGAGAAAAGAAACGAAGAGAGGGGAAGGAAGGAAAA | 1.98% | -20 |
| 2 | CR001095 | | ... | ... |
| 1 | CR001137 | AAATGCTTATGACAGCAATAATCATAAAAACCTCAAACC------ ACTTAAATGCTTACCAACAGTAGAATTGATAAAT | 6.73% | -7 |
| 1 | CR001137 | AAATGCTTATGACAGCAATAATCATAAAAACCTCAAACCGGTAGgCCACTTAAATG CTTACCAACAGTAGAATTGATAAAT | 4.02% | 1 |
| 1 | CR001137 | AAATGCTTATGACAGCAATAATCATAAAAACCTCAAACCGGTAaGCCACTTAAATG CTTACCAACAGTAGAATTGATAAAT | 2.87% | 1 |
| 1 | CR001137 | AAATGCTTATGACAGCAATAATCATAAAAACCTCA-------- GCCACTTAAATGCTTACCAACAGTAGAATTGATAAAT | 1.71% | -8 |
| 1 | CR001137 | AAATGCTTATGACAGCAATAATCATAAAAACC------- ACTTAAATGCTTACCAACAGTAGAATTGATAAAT | 0.95% | -14 |
| 1 | CR001137 | | ... | ... |
| 2 | CR001137 | AAATGCTTATGACAGCAATAATCATAAAAACCTCAAACC------ ACTTAAATGCTTACCAACAGTAGAATTGATAAAT | 7.25% | -7 |
| 2 | CR001137 | AAATGCTTATGACAGCAATAATCATAAAAACCTCAAACCGGTAGgCCACTTAAATG CTTACCAACAGTAGAATTGATAAAT | 3.44% | 1 |
| 2 | CR001137 | AAATGCTTATGACAGCAATAATCATAAAAACCTCAAACCGGTAaGCCACTTAAATG CTTACCAACAGTAGAATTGATAAAT | 2.50% | 1 |
| 2 | CR001137 | AAATGCTTATGACAGCAATAATCATAAAAACCTCAAACCGGTA----- AATGCTTACCAACAGTAGAATTGATAAAT | 1.37% | -8 |
| 2 | CR001137 | AAATGCTTATGACAGCAATAATCATAAAAACCTCAAACCGGT----- TAAATGCTTACCAACAGTAGAATTGATAAAT | 1.11% | -7 |
| 2 | CR001137 | | ... | ... |
| 1 | CR001142 | AAATGCATTTTACAGCATTTGGTTGATTAAAAGTAACCcAGAGGTGAGTTCAAACT ATATGACTTTATTTGTATATAGAAA | 8.00% | 1 |
| 1 | CR001142 | AAATGCATTTTACAGCATTTGGTTGATTAAAAG------- AGGTGAGTTCAAACTATATGACTTTATTGTATATAGAAA | 6.63% | -7 |
| 1 | CR001142 | AAATGCATTTTACAGCATTTGGTTGATTAAAAGTAAC- AGAGGTGAGTTCAAACTATATGACTTTATTTGTATATAGAAA | 2.86% | -1 |
| 1 | CR001142 | AAATGCATTTTACAGCATTTGGTTGATTAAAAG------ GTGAGTTCAAACTATATGACTTTATTTGTATATAGAAA | 2.03% | -9 |
| 1 | CR001142 | AAATGCATTTTACAGCATTTGGTTGA---------- GTTCAAACTATATGACTTTATTTGTATATAGAAA | 1.71% | -20 |
| 1 | CR001142 | | ... | ... |
| 2 | CR001142 | AAATGCATTTTACAGCATTTGGTTGATTAAAAGTAACCcAGAGGTGAGTTCAAACT ATATGACTTTATTTGTATATAGAAA | 9.94% | 1 |
| 2 | CR001142 | AAATGCATTTTACAGCATTTGGTTGATTAAAAG------- AGGTGAGTTCAAACTATATGACTTTATTTGTATATAGAAA | 5.81% | -7 |
| 2 | CR001142 | AAATGCATTTTACAGCATTTGGTTGATTAAAAGTAAC- AGAGGTGAGTTCAAACTATATGACTTTATTTGTATATAGAAA | 4.19% | -1 |
| 2 | CR001142 | AAATGCATTTTACAGCATTTGGTTGATTAAAAG--------- GTGAGTTCAAACTATATGACTTTATTTGTATATAGAAA | 2.59% | -9 |
| 2 | CR001142 | AAATGCATTTTACAGCATTTGGTTGA----------------- GTTCAAACTATATGACTTTATTTGTATATAGAAA | 2.26% | -20 |
| 2 | CR001142 | | ... | ... |
| 1 | CR001158 | CCTGGAAGCCAACGAAAGATATTGAATAATTCAAGAAAaGGTGGTGGCATGGTTTG ATTTGTGTCTTTAAAAGATTATTCT | 10.94% | 1 |
| 1 | CR001158 | CCTGGAAGCCAACGAAAGATATTGAATAATTCAAG---- GTGGTGGCATGGTTTGATTTGTGTCTTTAAAAGATTATTCT | 10.03% | -4 |
| 1 | CR001158 | CCTGGAAGCCAACGAAAGATATTGAATAATTCAAGAA- GGTGGTGGCATGGTTTGATTTGTGTCTTTAAAAGATTATTCT | 3.80% | -1 |
| 1 | CR001158 | CCTGGAAGCCAACGAAAGATATTGAATAATTCAAGA-- GGTGGTGGCATGGTTTGATTTGTGTCTTTAAAAGATTATTCT | 1.19% | -2 |
| 1 | CR001158 | CCTGGAAGCCAACGAAAGATATTGAATAATTCA----- GGTGGTGGCATGGTTTGATTTGTGTCTTTAAAAGATTATTCT | 0.78% | -5 |
| 1 | CR001158 | | ... | ... |
| 2 | CR001158 | CCTGGAAGCCAACGAAAGATATTGAATAATTCAAGAAAaGGTGGTGGCATGGTTTG ATTTGTGTCTTTAAAAGATTATTCT | 11.89% | 1 |
| 2 | CR001158 | CCTGGAAGCCAACGAAAGATATTGAATAATTCAAG---- GTGGTGGCATGGTTTGATTTGTGTCTTTAAAAGATTATTCT | 9.97% | -4 |
| 2 | CR001158 | CCTGGAAGCCAACGAAAGATATTGAATAATTCAAGAA- GGTGGTGGCATGGTTTGATTTGTGTCTTTAAAAGATTATTCT | 3.45% | -1 |
| 2 | CR001158 | CCTGGAAGCCAACGAAAGATATTGAATAATTCAAGA-- GGTGGTGGCATGGTTTGATTTGTGTCTTTAAAAGATTATTCT | 0.86% | -2 |
| 2 | CR001158 | CCTGGAAGCCAACGAAAGATATTGAATAATTCAAGAAcaAGGTGGTGGCATGGTTT GATTTGTGTCTTTAAAAGATTATTCT | 0.49% | 2 |
| 2 | CR001158 | | ... | ... |
| 1 | CR001212 | CCTTGGCCTCCCAAAGTGCTGGGTTACAAGCCTGAGCCA----- GGATAAGGTCTAAAAGTGGAAAGAATA | 11.38% | -13 |
| 1 | CR001212 | CCTTGGCCTCCCAAAGTGCTGGGTTTACAAGCCTGAGCCACC- CATCCAGCCAGGATAAGGTCTAAAAGTGGAAAGAATA | 3.98% | -1 |
| 1 | CR001212 | CCTTGGCCTCCCAAAGTGCTGGGTTACAAGCCTGAGCCA----- TCCAGCCAGGATAAGGTCTAAAAGTGGAAAGAATA | 3.05% | -5 |
| 1 | CR001212 | CCTTGGCCTCCCAAAGTGCTGGGTTACAAGCCTGAGCCACCG- ATCCAGCCAGGATAAGGTCTAAAAGTGGAAAGAATA | 2.94% | -1 |
| 1 | CR001212 | CCTTGGCCTCCCAAAGTGCTGGGTTTACAAGCCTGAGCCACC----- AGCCAGGATAAGGTCTAAAAGTGGAAAGAATA | 2.82% | -6 |
| 1 | CR001212 | | ... | ... |
| 2 | CR001212 | CCTTGGCCTCCCAAAGTGCTGGGTTACAAGCCTGAGCCA----- GGATAAGGTCTAAAAGTGGAAAGAATA | 11.42% | -13 |
| 2 | CR001212 | CCTTGGCCTCCCAAAGTGCTGGGTTTACAAGCCTGAGCCACC- CATCCAGCCAGGATAAGGTCTAAAAGTGGAAAGAATA | 6.41% | -1 |
| 2 | CR001212 | CCTTGGCCTCCCAAAGTGCTGGGTTTACAAGCCTGAGCCACCG- ATCCAGCCAGGATAAGGTCTAAAAGTGGAAAGAATA | 5.61% | -1 |
| 2 | CR001212 | CCTTGGCCTCCCAAAGTGCTGGGTTTACAAGCCTGAGCCACCG-- TCCAGCCAGGATAAGGTCTAAAAGTGGAAAGAATA | 2.85% | -2 |
| 2 | CR001212 | CCTTGGCCTCCCAAAGTGCTGGGTTTACAAGCCTGAGCCACC----- AGCCAGGATAAGGTCTAAAAGTGGAAAGAATA | 1.96% | -6 |
| 2 | CR001212 | | ... | ... |
| 1 | CR001217 | AAGTGCTGGGTTTACAAGCCTGAGCCACCGCATCCAGCCAGG----- TCTAAAAGTGGAAAGAATAGCATCTACTCTTG | 17.75% | -6 |
| 1 | CR001217 | AAGTGCTGGGTTTACAAGCCTGAGCCACCGCATCCAGCCAGGATtAAGGTCTAAAA GTGGAAAGAATAGCATCTACTCTTG | 4.06% | 1 |
| 1 | CR001217 | AAGTGCTGGGTTTACAAGCCTGAGCCACCGCATCCAGCCAGGAaTAAGGTCTAAAA GTGGAAAGAATAGCATCTACTCTTG | 2.38% | 1 |
| 1 | CR001217 | AAGTGCTGGGTTTACAAGCCTGAGCCACCGCATCCAGCCAGGA- AAGGTCTAAAAGTGGAAAGAATAGCATCTACTCTTG | 1.35% | -1 |
| 1 | CR001217 | AAGTGCTGGGTTTACAAGCCTGAGCCACCGCATCCAGCCAGGAT----- CTAAAAGTGGAAAGAATAGCATCTACTCTTG | 1.10% | -5 |
| 1 | CR001217 | | ... | ... |
| 2 | CR001217 | AAGTGCTGGGTTTACAAGCCTGAGCCACCGCATCCAGCCAGG----- TCTAAAAGTGGAAAGAATAGCATCTACTCTTG | 18.91% | -6 |
| 2 | CR001217 | AAGTGCTGGGTTTACAAGCCTGAGCCACCGCATCCAGCCAGGATtAAGGTCTAAAA GTGGAAAGAATAGCATCTACTCTTG | 5.59% | 1 |
| 2 | CR001217 | AAGTGCTGGGTTTACAAGCCTGAGCCACCGCATCCAGCCAGGAaTAAGGTCTAAAA GTGGAAAGAATAGCATCTACTCTTG | 1.42% | 1 |
| 2 | CR001217 | AAGTGCTGGGTTTACAAGCCTGAGCCACCGCATCCAGCCAGGA- AAGGTCTAAAAGTGGAAAGAATAGCATCTACTCTTG | 1.38% | -1 |
| 2 | CR001217 | AAGTGCTGGGTTTACAAGCCTGAGCCACCGCATCCAGCCAGGAT----- CTAAAAGTGGAAAGAATAGCATCTACTCTTG | 1.26% | -5 |
| 2 | CR001217 | | ... | ... |
| 1 | CR001221 | TAGCATCTACTCTTGTTCAGGAAACAATGAGGACCTG---- | 28.78% | -4 |
| | | GGCAGTAAGAGTGGTGATTAATAGATAGGGACAAATTGA | | |
| 1 | CR001221 | TAGCATCTACTCTTGTTCAGGAAACAATGAGGACCT- ACTGGGCAGTAAGAGTGGTGATTAATAGATAGGGACAAATTGA | 9.51% | -1 |
| 1 | CR001221 | TAGCATCTACTCTTGTTCAGGAAACAATGAGGAC----- TGGGCAGTAAGAGTGGTGATTAATAGATAGGGACAAATTGA | 4.35% | -5 |
| 1 | CR001221 | TAGCATCTACTCTTGTTCAGGAAACAATGAGG-------- CAGTAAGAGTGGTGATTAATAGATAGGGACAAATTGA | 1.87% | -11 |
| 1 | CR001221 | TAGCATCTACTCTTGTTCAGGAAACAATG----------- GGCAGTAAGAGTGGTGATTAATAGATAGGGACAAATTGA | 1.71% | -12 |
| 1 | CR001221 | | ... | ... |
| 2 | CR001221 | TAGCATCTACTCTTGTTCAGGAAACAATGAGGACCTG---- GGCAGTAAGAGTGGTGATTAATAGATAGGGACAAATTGA | 29.12% | -4 |
| 2 | CR001221 | TAGCATCTACTCTTGTTCAGGAAACAATGAGGACCT- ACTGGGCAGTAAGAGTGGTGATTAATAGATAGGGACAAATTGA | 10.32% | -1 |
| 2 | CR001221 | TAGCATCTACTCTTGTTCAGGAAACAATGAGGAC----- TGGGCAGTAAGAGTGGTGATTAATAGATAGGGACAAATTGA | 4.26% | -5 |
| 2 | CR001221 | TAGCATCTACTCTTGTTCAGGAAACAATGAGGAC-------- AGTAAGAGTGGTGATTAATAGATAGGGACAAATTGA | 1.89% | -10 |
| 2 | CR001221 | TAGCATCTACTCTTGTTCAGGAAACAATGAGG------- CAGTAAGAGTGGTGATTAATAGATAGGGACAAATTGA | 1.67% | -11 |
| 2 | CR001221 | | ... | ... |

Although in some cases, the relative abundance of the most frequent indels varied from experiment to experiment, the top indels were largely the same across both experiments for any given gRNA. This indicates that for these dgRNAs, the indel pattern created in HSC cells was consistent. As well, these results support the surprising finding from earlier expeirments that small indels (e.g., in this case, indels from 1-20 nucelotides) in the HPFH regions targeted by these gRNAs could result in significant upregulation of fetal hemoglobin. An increase in HbF-containing erythrocytes derived from genome edited cells is likely to have therapeutic implications by reduced sickling.

### Example 4.5

Methods: Methods are as in sub-example 4.1, with the following exceptions.

Human CD34⁺ cell culture. Human CD34+ cells were derived from bone marrow (Hemacare Corporation, Cat. No. BM34C-3). CD34+ cells were thawed and expanded in expansion medium for 1 day prior to RNP delivery. After RNP delivery, cells were immediatedly transferred back into 200 µl expansion medium and allowed to recover overnight. The following day, the cultures were counted and adjusted to 2.0×10⁵ viable cells per ml. Percent recovery was calculated as the viable cell count 1 day after RNP delivery as a percentage of the viable cells electroporated. After an additional 7 days in expansion medium (8 days following RNP delivery), the viable cell count was again determined. Fold proliferation was calculated as the viable cell count after 7 days in expansion medium divided by 2.0×10⁵ cells per ml. All viable cell counts were measured by flow cytometry using AccuCheck Counting Beads (ThermoFisher cat. No. PCB100) with inviable cells discriminated by DAPI (4',6-Diamidino-2-Phenylindole) staining. After an additional 1 day in expansion medium (9 days following RNP delivery), the cell cultures were phenotyped by flow cytometry as described under Cell phenotyping.

Assembly of Cas9 and guide RNA ribonucleoprotein (RNP) complexes, preparation of HSPC, and electroporation of RNP into HSPC. The HSPC collected by centrifugation were resuspended in P3 Primary Cell Solution (Lonza cat. No. PBP3-00675) at a cell density of 5.4 × 10⁶/mL. The RNP was mixed with 20 µL of cells by pipetting up and down and incubated at RT for 2 min. The RNP/cell mixture (20 µL) was electroporated using code CM-137 on the 4D-Nucleofector (Lonza). Electroporations were performed in duplicate. The crRNAs used were of the sequence mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where r indicates an RNA base, m indicates a base with 2'O-Methyl modification, and ^{∗} indicates a Phosphorothioate Bond. N's indicate the residues of the indicated targeting domain. The tracr sequence used was SEQ ID NO: 7808.

Colony forming unit cell assay. The day following RNP delivery, viable cells were counted as described in Human CD34+ cell culture. For the granulocyte/macrophage progenitor colony forming unit (CFU) assay, 227 cells per 1 mL Methocult H4035 methylcellulose medium (StemCell Technologies) was plated in duplicate. 1x antibiotic/antimycotic (Gibco, Cat. #10378-016) was added into the Methocult. The culture dishes were incubated in a humidified incubator at 37°C. Colonies containing at least 50 cells were counted at day 15 post-plating. Colony number per ml of Methocult was divided by 227 and multiplied by 1000 to obtain the CFU frequency per 1000 cells.

Cell phenotyping. Cells were analyzed for surface marker expression after staining with the following antibody panels. Panel 1: Antibodies specific for CD38 (FITC-conjugate, BD Biosciences #340926, clone HB7), CD133 epitope 1 (PE-conjugate, Miltenyi #130-080-801, clone AC133), CD34 (PerCP-conjugate, BD Biosciences #340666, clone 8G12), CD90 (APC-conjugate, BD Biosciences #598695, clone E10), CD45RA (Pe-Cy7-conjugate, eBioscience #25-0458-42, clone HI100). Panel 2: CD34 (PerCP-conjugate, BD Biosciences #340666, clone 8G12), CD33 (PE-Cy7-conjugate, BD Biosciences #333946, clone P67.6), CD14 (APC-H7-conjugate, BD Biosciences #560270, clone MϕP9), CD15 (PE-conjugate, Biolegend #301905, clone HI98). Panel 3: CD34 (PerCP-conjugate, BD Biosciences #340666, clone 8G12), CD41a (APC-H7-conjugate, BD Biosciences #561422, clone HIP8), CD71 (FITC-conjugate, BD Biosciences #555536, clone M-A712), CD19 (PE-conjugate, BD Biosciences #340720, clone SJ25C1), CD56 (APC-conjugate, Biolegend #318310, clone HCD56). Correspoding isotype control antibody panels were used to stain cultures in parallel, except that anti-CD45RA was included instead of its isotype control. Inviable cells were discriminated by DAPI (4',6-Diamidino-2-Phenylindole) staining. Stained samples were analyzed on an LSRFortessa flow cytometer (BD Biosciences) for cell surface protein expression. The results were analyzed using Flowjo, and data were presented as % of the DAPI negative viable cell population.

Genomic DNA preparation and next generation sequencing (NGS). Genomic DNA was prepared from edited and unedited HSPC at 1 and 8 days post-electroporation using Quick Extract DNA Extraction Solution (Epicentre Cat # QE09050).

### Results:

Without being bound by theory, it is believed that targeted genetic disruption of the HPFH or BCL11A erythroid enhancer region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes. The list of gRNAs used in the study are shown in Table 23. The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 23. Relative viable cell recovery, proliferation in CD34+ medium, and granulocyte/macrophage progenitor colony forming unit (CFU) frequency for edited and unedited cell cultures in the current study. Percent recovery, Fold proliferation and CFU frequency were calculated as described in Materials and Methods. Individual electroporation replicates are shown. All gRNA molecules were tested in the dgRNA format. The Tracr sequence was SEQ ID NO: 7808, and the crRNA had the following format with 2'O-Methyl (m) and Phosphorothioate Bond (^{∗}) modifications indicated: mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the residues of the indicated targeting domain.**

| Guide RNA targeting domain ID and replicate | % recovery | Fold proliferation (7 days) | CFU (per 1000 cells) |
|---|---|---|---|
| CR001030 replicate 1 | 77.7 | 7.1 | 117 |
| CR001030 replicate 2 | 87.9 | 4.8 | 95 |
| CR001028 replicate 1 | 85.3 | 10.6 | 176 |
| CR001028 replicate 2 | 82.1 | 10.9 | 141 |
| CR001095 replicate 1 | 72.5 | 7.3 | 121 |
| CR001095 replicate 2 | 73.3 | 7.3 | 126 |
| CR001212 replicate 1 | 75.6 | 7.7 | 123 |
| CR001212 replicate 2 | 75.5 | 8.2 | 97 |
| CR000312 replicate 1 | 77.7 | 12.2 | 145 |
| CR000312 replicate 2 | 81.0 | 9.0 | 143 |
| g8 replicate 1 | 80.0 | 16.6 | 145 |
| g8 replicate 2 | 84.6 | 17.3 | 145 |
| None/control replicate 1 | 89.8 | 19.3 | 185 |
| None/control replicate 2 | 81.2 | 20.5 | 154 |

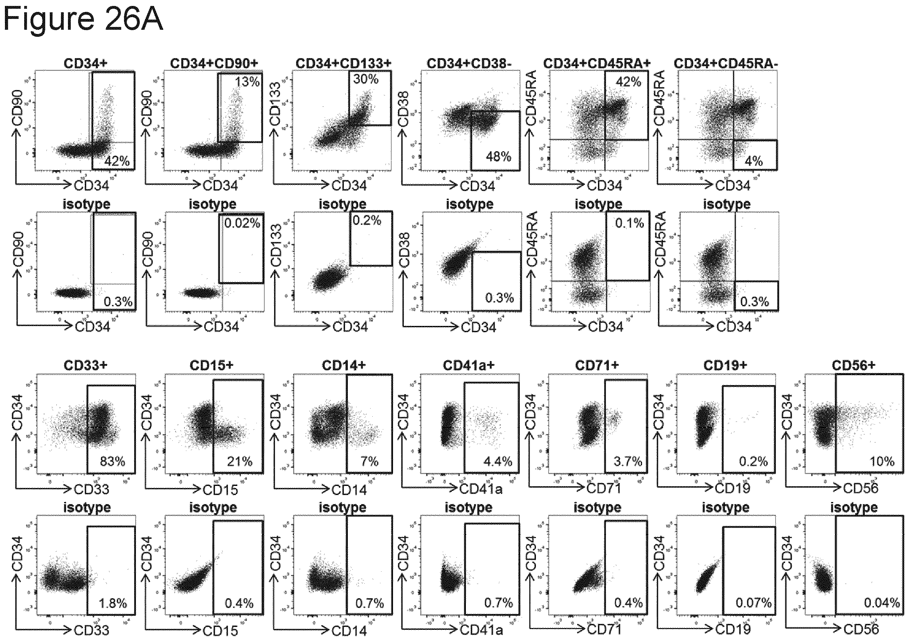

Percent cell recovery at 1 day after electroporation ranged from 72.5% to 89.8% and was similar across conditions, including the unedited but electroporated control (Table 23). The genome edited and unedited HSPC were analyzed for proliferation in CD34+ cell expansion medium. Proliferation ranged from 4.8-fold to 17.3-fold over 7 days for edited cell cultures, and ranged from 19.3 to 20.5-fold for unedited cells (Table 22). Editing by dgRNAs in this study targeting the HPFH or BCL11A erythroid enhancer regions did not alter the composition of cell types in the culture compared to unedited control, as determined by surface marker expression, indicating that targeting these sites does not alter HSPC fate under these conditions (Figure 26A-B). In contrast, editing with dgRNA targeting g8 in the BCL11A exon resulted in a modest reduction in proliferation but a marked shift in cell composition, specifically a decrease in the percentage of all CD34+ HSPC sub-types as well as of the CD71+ erythroid population and an increase in the percentage of CD14+ monocyte and CD15+ granulocyte poplulations (Table 23 and Figure 26A-B). The frequency of granulocyte/macrophage progenitor colony forming units (CFU) was similar across edited cultures, with only a modest CFU reduction in edited cultures (ranging from 95 to 145 CFU per 1000 cells) compared with unedited cultures (154 to 185 CFU per 1000 cells), suggesting that granulocyte/macrophage progenitor function is not grossly altered by targeting these sites (Table 23).

### Example 4.6

Methods: Methods are as in Example 4, with the following exceptions.

Assembly of Cas9 and guide RNA ribonucleoprotein (RNP) complexes, preparation of HSPC, and electroporation of RNP into HSPC. In some instances, crRNAs containing two different targeting domains were delivered into the same cell culture. In these instances, the total RNP delivered to cells contained 6 µg of Cas9, 3 µg of crRNA and 3 µg of Tracr, as in sub-example 4.1; however, the two crRNAs containing 1.5 µg each were independently complexed Tracr/CAS9 mixtures containing 1.5 µg Tracr and 3 µg Cas9 and only combined when added to the cells. The HSPC collected by centrifugation were resuspended in P3 Primary Cell Solution (Lonza cat. No. PBP3-00675) at a cell density of 2.5 × 10⁶/mL. The RNP was mixed with 20 µL of cells by pipetting up and down and incubated at RT for 2 min. The RNP/cell mixture (20 µL) was electroporated using code CM-137 on the 4D-Nucleofector (Lonza). Electroporations were performed in duplicate. The dgRNA format used a tracr having SEQ ID NO: 7808, and a crRNA of the sequence mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where r indicates an RNA base, m indicates a base with 2'O-Methyl modification, and ^{∗} indicates a Phosphorothioate Bond, and N's indicate the residues of the indicated targeting domain.

*In vitro* erythropoiesis and FACS analysis for HbF containing erythroid cells. The cell culture was diluted in fresh medium after 3 days.

Genomic DNA preparation and next generation sequencing (NGS). Genomic DNA was prepared from edited and unedited HSPC at 3 days post-electroporation using Quick Extract DNA Extraction Solution (Epicentre Cat # QE09050).

### Results:

Without being bound by theory, it is believed that targeted genetic disruption of the HPFH or BCL11A erythroid enhancer region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic dual gRNAs (dgRNA) that consist of crRNA and tracr to generate ribonucleoprotein (RNP) complexes. The list of gRNAs used in the study are shown in Table 24. The RNP complexes were electroporated into CD34+ HSPC via electroporation as described under materials and methods. The cells were expanded prior to the delivery of RNP complexes. Actively dividing cells may facilitate uptake of RNP complexes delivered by electroporation.

**Table 24. HbF induction by simultaneous targeting of both the BCL11A erythroid enhancer and HPFH regions. All gRNA molecules were tested in the dgRNA format in duplicate. The tracr used has the sequence of SEQ ID NO: 7808, and the crRNA had the following format with 2'O-Methyl (m) and Phosphorothioate Bond (^{∗}) modifications indicated: mN^{∗}mN^{∗}mN^{∗}rNrNrNrNrNrNrNrNrNrNrNrNrNrNrNrNrNrGrUrUrUrUrArGrArGrCrUrArU^{∗}mG^{∗}m C^{∗}mU, where N's are the residues of the indicated targeting domain.**

| Guide RNA targeting domain ID #1 | Guide RNA targeting domain ID #2 | %HbF+ (F cells), average of replicates | % edited at targeting site #1, average of replicates | % edited at targeting site #2, average of replicates |
|---|---|---|---|---|
| CR000312 | n/a | 41.3 | 91.3 | n/a |
| CR001128 EH15 | n/a | 43.7 | 77.5 | n/a |
| n/a | CR001030 | 58.0 | n/a | 85.6 |
| n/a | CR001221 | 48.6 | n/a | 92.1 |
| CR000312 | CR001030 | 66.8 | 58.3 | 81.2 |
| CR000312 | CR001221 | 60.6 | 64.2 | 85.0 |
| CR001128 EH15 | CR001030 | 69.1 | 49.8 | 82.9 |
| CR001128 EH15 | CR001221 | 62.7 | 58.5 | 84.8 |
| g8 | n/a | 64.7 | 94.6 | n/a |
| None/control | n/a | 23.8 | n/a | n/a |

The genome edited and unedited HSPC were analyzed by flow cytometry for expression levels of fetal globin and two erythroid cell surface markers, transferrin receptor (CD71) and glycophorin A (CD235a) using antibodies conjugated to fluorescent dyes. The live cells were identified and gated by exclusion of Live Dead Violet. HPFH region or BCL11A erythroid enhancer targeting resulted in increased percentage of erythroid cells containing HbF compared to mock electroporated cells (Table 24). Induction of HbF positive cells by dgRNA including the targeting domain of g8 targeting exon 2 of BCL11A was also observed in parallel. When the HPFH region and BCL11A erythroid enhancer were targeted simultaneously in the same cell population, the percentage of HbF containing cells was increased beyond that of targeting either region independently (Table 24). Thus, an increase in HbF-containing erythrocytes derived from edited cells can be achieved by targeting either the HPFH region or BCL11A erythroid enhancer, as well as to a greater extent by simultaneous targeting of both regions.

Genomic PCR products of both regions were independently subjected to next generation sequencing (NGS) to determine the percentage of edited alleles in the cell population. Genome editing was observed in cell cultures electroporated with RNPs containing Cas9, crRNA of the given targeting domain and Tracr (Table 24), but not in control cells with no targeting domain delivered (RNPs containing only Cas9 and Tracr). When RNPs targeting two sites were delivered into the same cell population, editing at both sites was observed (Table 24). In some instances, the percent edited cells at a given site was modestly reduced when two RNPs were delivered compared with when only one RNP was delivered (Table 24), possibly due to the lower RNP concentration targeting a given site in the former instance. Higher editing percentages and potentially higher percentages of HbF containing cells may be achieved by increasing the each individual RNP concentration when two RNPs are delivered simultaneously. An increase in HbF-containing erythrocytes derived from genome edited cells is likely to have therapeutic implications by reduced sickling.

### Example 4.7

Methods: Methods are as in Example 4, with the following exceptions.

Human CD34+ cell culture. Human CD34+ cells were derived from bone marrow (Lonza, Cat. No. 2M-101D). CD34+ cells were thawed and expanded for 2 days in expansion medium prior to RNP delivery.

Assembly of Cas9 and guide RNA ribonucleoprotein (RNP) complexes, preparation of HSPC, and electroporation of RNP into HSPC. For formation of RNP using single guide RNAs (sgRNAs), 6 µg of sgRNA was added to 6 µg of CAS9 protein (in 0.8 to 1 µL) and 0.5 µL of 5 × CCE buffer (20 mM HEPES, 100 mM KCL, 5 mM MgCL2, 5% Glycerol and freshly added 1 mM DTT) in a total volume of 5 µL and incubated for 5 min at 37oC. The HSPC were collected by centrifugation were resuspended in P3 Primary Cell Solution (Lonza cat. No. PBP3-00675) at a cell density of 6.4 × 106/mL. The RNP was mixed with 20 µL of cells by pipetting up and down and incubated at RT for 2 min. The RNP/cell mixture (20 µL) was electroporated using code CM-137 on the 4D-Nucleofector (Lonza). Electroporations were performed in duplicate. For dgRNA, dgRNA-PS and dgRNA-OMePS the Tracr was SEQ ID NO: 6660, with the exception of g8 which was used with Tracr SEQ ID NO: 7808. The formats for all gRNAs used in this experiment are shown in Table 36, with the exception of the dgRNA g8 OMePS, which included a crRNA having the sequence mC^{∗}mA^{∗}mC^{∗}AAACGGAAACAAUGCAAGUUUUAGAGCUAU^{∗}mG^{∗}mC^{∗}mU. and a tracr having the sequence of SEQ ID NO: 7808.

*In vitro* erythropoiesis and FACS analysis for HbF containing erythroid cells. After RNP delivery, cells were maintained as per Protocol 1 or Protocol 2. For Protocol 1, the cells were immediately transferred into pre-warmed medium consisting of EDM and supplements. During days 0-7 of culture, EDM was further supplemented with 1 µM hydrocortisone (Sigma H8672), 100 ng/mL human SCF (Life Technologies, Cat. #PHC2113), and 5 ng/mL human IL-3 (Peprotech #10779-598). During days 7-11 of culture, EDM was supplemented with 100 ng/mL of human SCF only. During days 11-21 of culture, EDM had no additional supplements. Cell cultures were adjusted to 4.0×10⁴ cells per ml on day 7. On days 11, 14 and 19, the media was replenished. For Protocol 2, cells were immediatedly transferred back into 200 µl expansion medium and allowed to expand for an additional 2 days. The cultures were counted at 2 days after RNP delivery. The cells were then pelleted and resuspended in pre-warmed medium consisting of EDM and supplements. During days 0-7 of EDM culture, EDM was further supplemented with 1 µM hydrocortisone (Sigma H8672), 100 ng/mL human SCF (Life Technologies, Cat. #PHC2113), and 5 ng/mL human IL-3 (Peprotech #10779-598). During days 7-11 of culture, EDM was supplemented with 100 ng/mL of human SCF only. During days 11-21 of culture, EDM had no additional supplements. Cell cultures were initiated at 4.0×10⁴ cells per ml on day 0, diluted 4-fold with fresh medium on day 4, and adjusted to 2.0×10⁵ cells per ml on day 7. On days 11, 14 and 19, the media was replenished. Viable cells were counted at 7, 18 and 21 days in EDM culture. All viable cell counts were determined by flow cytometry using AccuCheck Counting Beads (ThermoFisher cat. No. PCB100) with inviable cells discriminated by DAPI (4',6-Diamidino-2-Phenylindole) staining. For both protocols, on days 7, 14 and 21 of the erythroid differentiation culture, the cells were analyzed by intracellular staining for HbF expression. On days 14 and 21 intracellular staining for HbF expression did not include anti-CD71 and anti-CD235a antibodies, and % of HbF positive cells (F-cells) in the entire viable cell population was reported. LIVE/DEAD^{®} Fixable Near-IR Dead Cell Stain (ThermoFisher L34975) was used as the viability dye.

Genomic DNA preparation and next generation sequencing (NGS). Genomic DNA was prepared at 2 and 6 days post-electroporation from edited and unedited HSPC cultured by Protocol 2 using Quick Extract DNA Extraction Solution (Epicentre Cat # QE09050).

### Results:

Without being bound by theory, it is believed that targeted genetic disruption of the HPFH region will relieve repression of γ-globin expression, allowing production of the red blood cells containing elevated HbF protein, (cells expressing fetal hemoglobin are sometimes referred to herein as "F-cells"). The elevated HbF prevents sickling of the red blood cells under deoxygenated conditions and will be therapeutic/curative for the patients of both β-thalassemia and SCD. Autologous hematopoietic stem cell transplantation (HSCT) with *ex vivo* genome edited HSC from SCD patients was also combined with stem cell expansion enhancing technology, e.g., an aryl hydrocarbon receptor (AHR) inhibitor, e.g., as described in WO2010/059401 (the contents of which are incorporated by reference in their entirety), e.g., Compound 4 to improve ex vivo expansion and increase the dose of gene modified HSC delivered.

For efficient genome editing via programmable nuclease, Cas9, the successful delivery of guide RNA (gRNA) and Cas9 protein into target cells and tissues is essential. Recent reports demonstrated that Cas9 ribonucleoprotein (RNP) complexes when delivered into target cells by electroporation can accomplish efficient and specific genome editing in several different cell types. Cas9-RNP complexes cleave chromosomal DNA almost immediately after delivery and are degraded rapidly in cells, reducing off-target effects. In contrast, use of plasmid and viral vector systems used to deliver Cas9 results in prolonged expression of the enzyme aggravating off-target effects associated with the system. Additionally, delivery of RNPs into the target cells requires no additional tools which would greatly facilitate translation of genome editing for therapeutic purposes in the clinic. Purified recombinant Cas9 protein and gRNA complexes (RNPs) were delivered into cultured HSPC and an overview experiments used are shown in Figure 17.

Recombinant Cas9 protein was purified from Escherichia coli and complexed with synthetic single gRNAs (sgRNA) or dual gRNAs (dgRNA) that consist of crRNA and tracr to generate ribonucleoprotein (RNP) complexes. The formats of gRNAs used in the study are shown in Table 36. The RNP complexes were electroporated into CD34+ HSPC via electroporation as described under materials and methods. The cells were expanded prior to the delivery of RNP complexes and, in some cases (Protocol 2) also after electroporation. Actively dividing cells may facilitate uptake of RNP complexes delivered by electroporation.

Genomic PCR products of the HPFH region were subjected to next generation sequencing (NGS) to determine the percentage of edited alleles in the cell population. Genome editing at the HFPH locus was observed in cell cultures electroporated with RNPs containing Cas9, crRNA of the given targeting domain and Tracr (Figure 43), but not in control cells with no targeting domain delivered (RNPs containing only Cas9 and Tracr). In some instances, editing was greater at a given target site usind modified sgRNAs relative to the other gRNA formats (Figure 43). Finally, the indel pattern as well as frequency of each indel was assessed for each gRNA by NGS. Indel pattern across multiple replicates using the same gRNA/Cas9 were similar. Representative most frequently occurring indels at each target site are shown in Table 37.

**Table 36. gRNA sequences used in the experiments described in this sub-example. N indicates the residues of the indicated targeting domain; mN indicates a 2'-OMe-modified nucleic acid; ^{∗} indicates a phosphorothioate modification.**

| **Label** | **Format** | **Sequence** |
|---|---|---|
| CRxxxxxx sg | sgRNA | NNNNNNNNNNNNNNNNNNNNGUUUUAGAGCUAGAAAUAGCAAGU UAAAAUAAGGCUAGUCCGUUAUCAACUUGAAAAAGUGGCACCGA GUCGGUGCUUUU |
| CRxxxxxx OMePS sg or CRxxxxxx sg OMePS | sgRNA | mN*mN*mN*NNNNNNNNNNNNNNNNNGUUUUAGAGCUAGAAAUA GCAAGUUAAAAUAAGGCUAGUCCGUUAUCAACUUGAAAAAGUGG CACCGAGUCGGUGCmU*mU*mU*U |
| CRxxxxxx PS sg or CRxxxxxx sg PS | sgRNA | N*N*N*NNNNNNNNNNNNNNNNNGUUUUAGAGCUAGAAAUAGCAA GUUAAAAUAAGGCUAGUCCGUUAUCAACUUGAAAAAGUGGCACC GAGUCGGUGCU*U*U*U |
| CRxxxxxx | dgRNA | NNNNNNNNNNNNNNNNNNNNGUUUUAGAGCUAUGCUGUUUUG |
| | | and |
| | | AACAGCAUAGCAAGUUAAAAUAAGGCUAGUCCGUUAUCAACUUG AAAAAGUGGCACCGAGUCGGUGCUUUUUUU (SEQ ID NO: 6660) |
| CRxxxxxx OMePS | dgRNA | mN*mN*mN*NNNNNNNNNNNNNNNNNGUUUUAGAGCUAUGCUGU U*mU*mU*mG |
| | | and |
| | | AACAGCAUAGCAAGUUAAAAUAAGGCUAGUCCGUUAUCAACUUG AAAAAGUGGCACCGAGUCGGUGCUUUUUUU (SEQ ID NO: 6660) |
| CRxxxxxx PS | dgRNA | N*N*N*NNNNNNNNNNNNNNNNNGUUUUAGAGCUAUGCUGUU*U* U*G |
| | | and |
| | | AACAGCAUAGCAAGUUAAAAUAAGGCUAGUCCGUUAUCAACUUG AAAAAGUGGCACCGAGUCGGUGCUUUUUUU (SEQ ID NO: 6660) |

**Table 37. Top indels observed for each gRNA (targeting domain and format) from NGS sequencing at Day 6 post-RNP introduction. gRNA sequences are as indicated in Table 36. Capital letters are the naturally occurring nucleotides at and near the target sequence. Deletions relative to the unmodified target sequence are shown as "-"; insertions are shown as lowercase letters.**

| **gRNA targeting domain ID and format** | **Variant Read (Genomic Sequence)** | **% of all reads** | **Indel length** |
|---|---|---|---|
| g8 dgRNA OMePS | ACATTCTTATTTTTATCGAGCACAAACGGAAACAATG----- GCAGCCTCTGCTTAGAAAAAGCTGTGGATAAGCCACCT | 47.57% | -5 |
| g8 dgRNA OMePS | ACATTCTTATTTTTATCGAGCACAAACGGAAACAATG- AATGGCAGCCTCTGCTTAGAAAAAGCTGTGGATAAGCCA CCT | 13.92% | -1 |
| g8 dgRNA OMePS | ACATTCTTATTTTTATCGAGCACAAACGGAAACAATG-- ATGGCAGCCTCTGCTTAGAAAAAGCTGTGGATAAGCCAC CT | 2.89% | -2 |
| g8 dgRNA | ACATTCTTATTTTATCGAGCACAAACGGAAACAATGCA-- | 2.01% | -6 |
| OMePS | 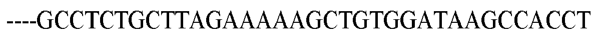 | | |
| g8 dgRNA OMePS | 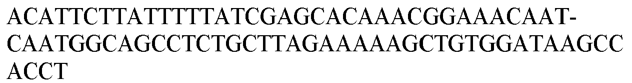 | 1.65% | -1 |
| g8 dgRNA OMePS | 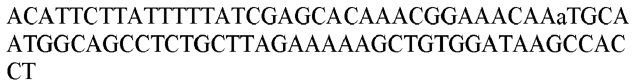 | 1.53% | 1 |
| | | | |
| CR001028 | 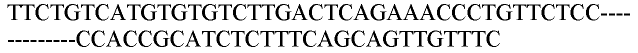 | 14.45% | -13 |
| CR001028 | TTCTGTCATGTGTGTCTTGACTCAGAAACCCTGTTCTCCTC TgCATATCTCCCCACCGCATCTCTTTCAGCAGTTGTTTC | 7.55% | 0 |
| CR001028 | TTCTGTCATGTGTGTCTTGACTCAGAAACCCTGTTCTCCTC TA-ATATCTCCCCACCGCATCTCTTTCAGCAGTTGTTTC | 7.49% | -1 |
| CR001028 | TTCTGTCATGTGTGTCTTGACTCAGAAACCCTGTTCTCCTC -------------ACCGCATCTCTTTCAGCAGTTGTTTC | 4.94% | -13 |
| CR001028 | TTCTGTCATGTGTGTCTTGACTCAGAAACCCTGTTCTCCTC TA----TCTCCCACCGCATCTCTTTCAGCAGTTGTTTC | 4.33% | -4 |
| CR001028 | TTCTGTCATGTGTGTCTTGACTCAGAAACCCTGTTCTCCTC TA--TATCTCCCCACCGCATCTCTTTCAGCAGTTGTTTC | 2.83% | -2 |
| | | | |
| CR001028 OMePS sg | TTCTGTCATGTGTGTCTTGACTCAGAAACCCTGTTCTCC---- ----------CCACCGCATCTCTTTCAGCAGTTGTTTC | 19.29% | -13 |
| CR001028 OMePS sg | TTCTGTCATGTGTGTCTTGACTCAGAAACCCTGTTCTCCTC TgCATATCTCCCCACCGCATCTCTTTCAGCAGTTGTTTC | 7.11% | 0 |
| CR001028 OMePS sg | TTCTGTCATGTGTGTCTTGACTCAGAAACCCTGTTCTCCTC -------------ACCGCATCTCTTTCAGCAGTTGTTTC | 6.26% | -13 |
| CR001028 OMePS sg | TTCTGTCATGTGTGTCTTGACTCAGAAACCCTGTTCTCCTC TA-ATATCTCCCCACCGCATCTCTTTCAGCAGTTGTTTC | 6.19% | -1 |
| CR001028 OMePS sg | TTCTGTCATGTGTGTCTTGACTCAGAAACCCTGTTCTCCTC TA----TCTCCCCACCGCATCTCTTTCAGCAGTTGTTTC | 5.44% | -4 |
| CR001028 OMePS sg | TTCTGTCATGTGTGTCTTGACTCAGAAACCCTGTTCTCCTC TA--TATCTCCCCACCGCATCTCTTTCAGCAGTTGTTTC | 2.89% | -2 |
| | | | |
| CR001028 OMePS | TTCTGTCATGTGTGTCTTGACTCAGAAACCCTGTTCTCC---- ----------CCACCGCATCTCTTTCAGCAGTTGTTTC | 15.91% | -13 |
| CR001028 OMePS | TTCTGTCATGTGTGTCTTGACTCAGAAACCCTGTTCTCCTC TgCATATCTCCCCACCGCATCTCTTTCAGCAGTTGTTTC | 8.91% | 0 |
| CR001028 OMePS | TTCTGTCATGTGTGTCTTGACTCAGAAACCCTGTTCTCCTC TA----TCTCCCCACCGCATCTCTTTCAGCAGTTGTTTC | 4.80% | -4 |
| CR001028 OMePS | TTCTGTCATGTGTGTCTTGACTCAGAAACCCTGTTCTCCTC -------------ACCGCATCTCTTTCAGCAGTTGTTTC | 4.59% | -13 |
| CR001028 OMePS | 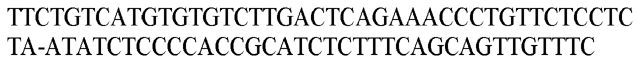 | 2.92% | -1 |
| CR001028 OMePS | TTCTGTCATGTGTGTCTTGACTCAGAAACCtTGTTCTCCTC TACATATCTCCCCACCGCATCTCTTTCAGCAGTTGTTTC | 1.75% | 0 |
| | | | |
| CR001028 PS | TTCTGTCATGTGTGTCTTGACTCAGAAACCCTGTTCTCC---- ----------CCACCGCATCTCTTTCAGCAGTTGTTTC | 13.02% | -13 |
| CR001028 PS | TTCTGTCATGTGTGTCTTGACTCAGAAACCCTGTTCTCCTC TgCATATCTCCCCACCGCATCTCTTTCAGCAGTTGTTTC | 8.11% | 0 |
| CR001028 PS | TTCTGTCATGTGTGTCTTGACTCAGAAACCCTGTTCTCCTC TA-ATATCTCCCCACCGCATCTCTTTCAGCAGTTGTTTC | 6.08% | -1 |
| CR001028 PS | TTCTGTCATGTGTGTCTTGACTCAGAAACCCTGTTCTCCTC -------------ACCGCATCTCTTTCAGCAGTTGTTTC | 4.43% | -13 |
| CR001028 PS | 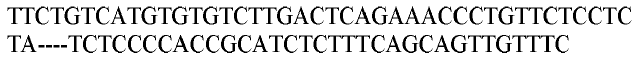 | 3.86% | -4 |
| CR001028 PS | TTCTGTCATGTGTGTCTTGACTCAGAAACCCTGTTCTCCTC TA--TATCTCCCCACCGCATCTCTTTCAGCAGTTGTTTC | 2.96% | -2 |
| | | | |
| CR001028 PS sg | TTCTGTCATGTGTGTCTTGACTCAGAAACCCTGTTCTCC---- ----------CCACCGCATCTCTTTCAGCAGTTGTTTC | 17.48% | -13 |
| CR001028 PS | TTCTGTCATGTGTGTCTTGACTCAGAAACCCTGTTCTCCTC -------------ACCGCATCTCTTTCAGCAGTTGTTTC | 7.01% | -13 |
| CR001028 PS sg | 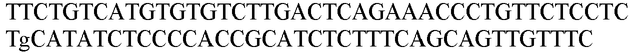 | 6.84% | 0 |
| CR001028 PS sg | TTCTGTCATGTGTGTCTTGACTCAGAAACCCTGTTCTCCTC TA----TCTCCCCACCGCATCTCTTTCAGCAGTTGTTTC | 6.05% | -4 |
| CR001028 PS sg | TTCTGTCATGTGTGTCTTGACTCAGAAACCCTGTTCTCCTC TA--TATCTCCCCACCGCATCTCTTTCAGCAGTTGTTTC | 4.59% | -2 |
| CR001028 PS sg | TTCTGTCATGTGTGTCTTGACTCAGAAACCCTGTTCTCCTC TA-ATATCTCCCCACCGCATCTCTTTCAGCAGTTGTTTC | 3.53% | -1 |
| | | | |
| CR001028 sg | TTCTGTCATGTGTGTCTTGACTCAGAAACCCTGTTCTCC---- ----------CCACCGCATCTCTTTCAGCAGTTGTTTC | 10.58% | -13 |
| CR001028 sg | TTCTGTCATGTGTGTCTTGACTCAGAAACCCTGTTCTCCTC TgCATATCTCCCCACCGCATCTCTTTCAGCAGTTGTTTC | 9.11% | 0 |
| CR001028 sg | TTCTGTCATGTGTGTCTTGACTCAGAAACCCTGTTCTCCTC TA-ATATCTCCCCACCGCATCTCTTTCAGCAGTTGTTTC | 6.47% | -1 |
| CR001028 sg | TTCTGTCATGTGTGTCTTGACTCAGAAACCCTGTTCTCCTC TA--TATCTCCCCACCGCATCTCTTTCAGCAGTTGTTTC | 4.78% | -2 |
| CR001028 sg | TTCTGTCATGTGTGTCTTGACTCAGAAACCCTGTTCTCCTC -------------ACCGCATCTCTTTCAGCAGTTGTTTC | 4.06% | -13 |
| CR001028 sg | 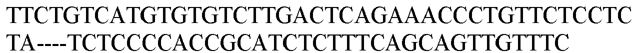 | 3.41% | -4 |
| | | | |
| CR001030 | 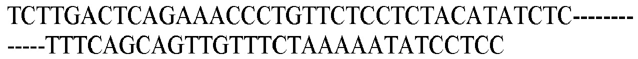 | 8.30% | -13 |
| CR001030 | TCTTGACTCAGAAACCCTGTTCTCCTCTgCATATCTCCCCA CCGCATCTCTTTCAGCAGTTGTTTCTAAAAATATCCTCC | 4.39% | 0 |
| CR001030 | TCTTGACTCAGAAACCCTGTTCTCCTCTACATATCTCCCC A-----TCTCTTTCAGCAGTTGTTTCTAAAAATATCCTCC | 2.61% | -5 |
| CR001030 | TCTTGACTCAGAAACCCTGTTCTCCTCTACATATCTCCCC ACC-CATCTCTTTCAGCAGTTGTTTCTAAAAATATCCTCC | 2.18% | -1 |
| CR001030 | TCTTGACTCAGAAACCtTGTTCTCCTCTACATATCTCCCCA CCGCATCTCTTTCAGCAGTTGTTTCTAAAAATATCCTCC | 2.12% | 0 |
| CR001030 | TCTTGACTCAGAAACCCTGTTCTCCTCTACATATCTCCCC ACC--ATCTCTTTCAGCAGTTGTTTCTAAAAATATCCTCC | 2.10% | -2 |
| | | | |
| CR001030 IDT | TCTTGACTCAGAAACCCTGTTCTCCTCTACATATCTC-------- -----TTTCAGCAGTTGTTTCTAAAAATATCCTCC | 10.40% | -13 |
| CR001030 IDT | TCTTGACTCAGAAACCCTGTTCTCCTCTACATATCTCCCC ACCGgCATCTCTTTCAGCAGTTGTTTCTAAAAATATCCTCC | 4.56% | 1 |
| CR001030 IDT | TCTTGACTCAGAAACCCTGTTCTCCTCTACATATCTCCCC AC-GCATCTCTTTCAGCAGTTGTTTCTAAAAATATCCTCC | 3.83% | -1 |
| CR001030 IDT | TCTTGACTCAGAAACCCTGTTCTCCTCTACATATCTCCCC ACC--ATCTCTTTCAGCAGTTGTTTCTAAAAATATCCTCC | 3.68% | -2 |
| CR001030 IDT | TCTTGACTCAGAAACCCTGTTCTCCTCTACATATCTCCCC A-----TCTCTTTCAGCAGTTGTTTCTAAAAATATCCTCC | 2.87% | -5 |
| CR001030 IDT | TCTTGACTCAGAAACCCTGTTCTCCTCTACATATCT---------- -----TTCAGCAGTTGTTTCTAAAAATATCCTCC | 2.54% | -15 |
| | | | |
| CR001030 | TCTTGACTCAGAAACCCTGTTCTCCTCTACATATCTC-------- -----TTTCAGCAGTTGTTTCTAAAAATATCCTCC | 9.90% | -13 |
| CR001030 | TCTTGACTCAGAAACCCTGTTCTCCTCTACATATCTCCCC ACCGgCATCTCTTTCAGCAGTTGTTTCTAAAAATATCCTCC | 4.63% | 1 |
| CR001030 | TCTTGACTCAGAAACCCTGTTCTCCTCTACATATCTCCCC ACC-CATCTCTTTCAGCAGTTGTTTCTAAAAATATCCTCC | 3.71% | -1 |
| CR001030 | TCTTGACTCAGAAACCCTGTTCTCCTCTACATATCTCCCC ACC--ATCTCTTTCAGCAGTTGTTTCTAAAAATATCCTCC | 2.39% | -2 |
| CR001030 | TCTTGACTCAGAAACCCTGTTCTCCTCTgCATATCTCCCCA CCGCATCTCTTTCAGCAGTTGTTTCTAAAAATATCCTCC | 2.03% | 0 |
| CR001030 | TCTTGACTCAGAAACCCTGTTCTCCTCTACATATCTCCCC A-----TCTCTTTCAGCAGTTGTTTCTAAAAATATCCTCC | 1.95% | -5 |
| | | | |
| CR001030 OMePS sg | TCTTGACTCAGAAACCCTGTTCTCCTCTACATATCTC-------- -----TTTCAGCAGTTGTTTCTAAAAATATCCTCC | 19.91% | -13 |
| CR001030 OMePS sg | TCTTGACTCAGAAACCCTGTTCTCCTCTACATATCTCCCC ACC-CATCTCTTTCAGCAGTTGTTTCTAAAAATATCCTCC | 5.76% | -1 |
| CR001030 OMePS sg | TCTTGACTCAGAAACCCTGTTCTCCTCTACATATCTCCCC ACCGgCATCTCTTTCAGCAGTTGTTTCTAAAAATATCCTCC | 3.09% | 1 |
| CR001030 OMePS sg | TCTTGACTCAGAAACCCTGTTCTCCTCTACATATCTCCCC ACCG----TCTTTCAGCAGTTGTTTCTAAAAATATCCTCC | 3.03% | -4 |
| CR001030 OMePS sg | TCTTGACTCAGAAACCCTGTTCTCCTCTACATATCTCCCC AC------TCTTTCAGCAGTTGTTTCTAAAAATATCCTCC | 2.98% | -6 |
| CR001030 OMePS sg | TCTTGACTCAGAAACCCTGTTCTCCTCTgCATATCT---------- -----TTCAGCAGTTGTTTCTAAAAATATCCTCC | 2.81% | -15 |
| | | | |
| CR001030 OMePS | TCTTGACTCAGAAACCCTGTTCTCCTCTACATATCTC-------- -----TTTCAGCAGTTGTTTCTAAAAATATCCTCC | 8.52% | -13 |
| CR001030 OMePS | TCTTGACTCAGAAACCCTGTTCTCCTCTgCATATCTCCCCA CCGCATCTCTTTCAGCAGTTGTTTCTAAAAATATCCTCC | 5.21% | 0 |
| CR001030 OMePS | TCTTGACTCAGAAACCCTGTTCTCCTCTACATATCTCCCC ACCGgCATCTCTTTCAGCAGTTGTTTCTAAAAATATCCTCC | 2.63% | 1 |
| CR001030 OMePS | TCTTGACTCAGAAACCCTGTTCTCCTCTACATATCTCCCC AC-GCATCTCTTTCAGCAGTTGTTTCTAAAAATATCCTCC | 2.25% | -1 |
| CR001030 OMePS | TCTTGACTCAGAAACCCTGTTCTCCTCTACATATCTCCCC A-----TCTCTTTCAGCAGTTGTTTCTAAAAATATCCTCC | 1.81% | -5 |
| CR001030 OMePS | TCTTGACTCAGAAACCCTGTTCTCCTCTACATATCTCCCC ACC-CATCTCTTTCAGCAGTTGTTTCTAAAAATATCCTCC | 1.77% | -1 |
| | | | |
| CR001030 PS | TCTTGACTCAGAAACCCTGTTCTCCTCTACATATCTC-------- -----TTTCAGCAGTTGTTTCTAAAAATATCCTCC | 5.99% | -13 |
| CR001030 PS | TCTTGACTCAGAAACCCTGTTCTCCTCTgCATATCTCCCCA CCGCATCTCTTTCAGCAGTTGTTTCTAAAAATATCCTCC | 4.09% | 0 |
| CR001030 PS | TCTTGACTCAGAAACCCTGTTCTCCTCTACATATCTCCCC ACCGgCATCTCTTTCAGCAGTTGTTTCTAAAAATATCCTCC | 3.90% | 1 |
| CR001030 PS | TCTTGACTCAGAAACCCTGTTCTCCTCTACATATCTCCCC A-----TCTCTTTCAGCAGTTGTTTCTAAAAATATCCTCC | 3.59% | -5 |
| CR001030 PS | TCTTGACTCAGAAACCCTGTTCTCCTCTACATATCTCCCC ACC-CATCTCTTTCAGCAGTTGTTTCTAAAAATATCCTCC | 2.86% | -1 |
| CR001030 PS | TCTTGACTCAGAAACCCTGTTCTCCTCTACATATCTCCCC ACC--ATCTCTTTCAGCAGTTGTTTCTAAAAATATCCTCC | 2.39% | -2 |
| | | | |
| CR001030 PS sg | TCTTGACTCAGAAACCCTGTTCTCCTCTACATATCTC-------- -----TTTCAGCAGTTGTTTCTAAAAATATCCTCC | 11.86% | -13 |
| CR001030 PS sg | TCTTGACTCAGAAACCCTGTTCTCCTCTACATATCTCCCC ACC-CATCTCTTTCAGCAGTTGTTTCTAAAAATATCCTCC | 5.20% | -1 |
| CR001030 PS sg | TCTTGACTCAGAAACCCTGTTCTCCTCTACATATCTCCCC ACCGgCATCTCTTTCAGCAGTTGTTTCTAAAAATATCCTCC | 3.65% | 1 |
| CR001030 PS sg | TCTTGACTCAGAAACCCTGTTCTCCTCTACATATCTCCCC ACC--ATCTCTTTCAGCAGTTGTTTCTAAAAATATCCTCC | 3.33% | -2 |
| CR001030 PS sg | TCTTGACTCAGAAACCCTGTTCTCCTCTACATATCTCCCC A-----TCTCTTTCAGCAGTTGTTTCTAAAAATATCCTCC | 2.92% | -5 |
| CR001030 PS sg | TCTTGACTCAGAAACCCTGTTCTCCTCTACAT--------------- CTCTTTCAGCAGTTGTTTCTAAAAATATCCTCC | 2.14% | -15 |
| CR001030 PS sg | TCTTGACTCAGAAACCCTGTTCTC--------------------------------------------------- TTTCAGCAGTTGTTTCTAAAAATATCCTCC | 2.08% | -26 |
| | | | |
| CR001030 sg | TCTTGACTCAGAAACCCTGTTCTCCTCTACATATCTC-------- -----TTTCAGCAGTTGTTTCTAAAAATATCCTCC | 11.22% | -13 |
| CR001030 sg | TCTTGACTCAGAAACCCTGTTCTCCTCTACATATCTCCCC ACC-CATCTCTTTCAGCAGTTGTTTCTAAAAATATCCTCC | 4.64% | -1 |
| CR001030 sg | TCTTGACTCAGAAACCCTGTTCTCCTCTACATATCTCCCC ACCGgCATCTCTTTCAGCAGTTGTTTCTAAAAATATCCTCC | 2.78% | 1 |
| CR001030 sg | TCTTGACTCAGAAACCCTGTTCTCCTCTACATATCTCCCC A-----TCTCTTTCAGCAGTTGTTTCTAAAAATATCCTCC | 2.73% | -5 |
| CR001030 sg | TCTTGACTCAGAAACCCTGTTCTCCTCTACATATCTCCCC AC-GCATCTCTTTCAGCAGTTGTTTCTAAAAATATCCTCC | 2.34% | -1 |
| CR001030 sg | TCTTGACTCAGAAACCCTGTTCTCCTCTACATATCTCCCC AC------TCTTTCAGCAGTTGTTTCTAAAAATATCCTCC | 2.29% | -6 |
| | | | |
| CR001137 | AAATGCTTATGACAGCAATAATCATAAAAACCTCAAACC GGTAGgCCACTTAAATGCTTACCAACAGTAGAATTGATAA AT | 4.26% | 1 |
| CR001137 | AAATGCTTATGACAGCAATAATCATAAAAACCTCAAACC- -------ACTTAAATGCTTACCAACAGTAGAATTGATAAAT | 3.80% | -7 |
| CR001137 | AAATGCTTATGACAGCAATAATCATAAAAACCTCAAACC GGTAaGCCACTTAAATGCTTACCAACAGTAGAATTGATAA AT | 1.32% | 1 |
| CR001137 | AAATGCTTATGACAGCAATAATCATAAAAACCTCAAACC GGTA--------AATGCTTACCAACAGTAGAATTGATAAAT | 0.98% | -8 |
| CR001137 | AAATGCTTATGACAGCAATAATCATAAAAACCTCAAACC GGT-------TAAATGCTTACCAACAGTAGAATTGATAAAT | 0.97% | -7 |
| CR001137 | AAATGCTTATGACAGCAATAATCATAAAAACCTCAAA----- -GCCACTTAAATGCTTACCAACAGTAGAATTGATAAAT | 0.85% | -6 |
| CR001137 | AAATGCTTATGACAGCAATAATCATAAAAACCTCAAACC GGT- GCCACTTAAATGCTTACCAACAGTAGAATTGATAAAT | 0.73% | -1 |
| | | | |
| CR001137 OMePS sg | AAATGCTTATGACAGCAATAATCATAAAAACCTCAAACC GGTAGgCCACTTAAATGCTTACCAACAGTAGAATTGATAA AT | 19.40% | 1 |
| CR001137 OMePS sg | AAATGCTTATGACAGCAATAATCATAAAAACCTCAAACC- -------ACTTAAATGCTTACCAACAGTAGAATTGATAAAT | 7.73% | -7 |
| CR001137 OMePS sg | AAATGCTTATGACAGCAATAATCATAAAAACCTCAA------- GCCACTTAAATGCTTACCAACAGTAGAATTGATAAAT | 2.97% | -7 |
| CR001137 OMePS sg | AAATGCTTATGACAGCAATAATCATAAAAACCTCAAACC GGTA--------AATGCTTACCAACAGTAGAATTGATAAAT | 2.78% | -8 |
| CR001137 OMePS sg | AAATGCTTATGACAGCAATAATCATAAAAACCTCAAACC GGTAaGCCACTTAAATGCTTACCAACAGTAGAATTGATAA AT | 2.27% | 1 |
| CR001137 OMePS sg | AAATGCTTATGACAGCAATAAT----------------- TACCAACAGTAGAATTGATAAAT | 2.06% | -35 |
| CR001137 OMePS sg | AAATGCTTATGACAGCAATAATCATAAAAACCTCAAA----- -----------TGCTTACCAACAGTAGAATTGATAAAT | 2.04% | -16 |
| | | | |
| CR001137 OMePS | AAATGCTTATGACAGCAATAATCATAAAAACCTCAAACC GGTAGgCCACTTAAATGCTTACCAACAGTAGAATTGATAA AT | 5.27% | 1 |
| CR001137 OMePS | AAATGCTTATGACAGCAATAATCATAAAAACCTCAAACC- -------ACTTAAATGCTTACCAACAGTAGAATTGATAAAT | 4.66% | -7 |
| CR001137 OMePS | AAATGCTTATGACAGCAATAATCATAAAAACCTCAAACC GGTA--------AATGCTTACCAACAGTAGAATTGATAAAT | 2.38% | -8 |
| CR001137 OMePS | AAATGCTTATGACAGCAATAATCATAAAAACCTCAAACC GGT-------TAAATGCTTACCAACAGTAGAATTGATAAAT | 1.91% | -7 |
| CR001137 OMePS | AAATGCTTATGACAGCAATAATCATAAAAACCTCAAACC GGTAaGCCACTTAAATGCTTACCAACAGTAGAATTGATAA AT | 1.72% | 1 |
| CR001137 OMePS | AAATGCTTATGACAGCAATAATCATAAAAACCTCAA------- GCCACTTAAATGCTTACCAACAGTAGAATTGATAAAT | 1.03% | -7 |
| CR001137 OMePS | AAATGCTTATGACAGCAATAATCATAAAAACCTCA-------- GCCACTTAAATGCTTACCAACAGTAGAATTGATAAAT | 0.82% | -8 |
| | | | |
| CR001137 PS | AAATGCTTATGACAGCAATAATCATAAAAACCTCAAACC- ------ACTTAAATGCTTACCAACAGTAGAATTGATAAAT | 7.49% | -7 |
| CR001137 PS | AAATGCTTATGACAGCAATAATCATAAAAACCTCAAACC GGTAGgCCACTTAAATGCTTACCAACAGTAGAATTGATAA AT | 6.85% | 1 |
| CR001137 PS | AAATGCTTATGACAGCAATAATCATAAAAACCTCAAACC GGTA--------AATGCTTACCAACAGTAGAATTGATAAAT | 2.64% | -8 |
| CR001137 PS | AAATGCTTATGACAGCAATAATCATAAAAACCTCAAACC GGTAaGCCACTTAAATGCTTACCAACAGTAGAATTGATAA AT | 2.11% | 1 |
| CR001137 PS | AAATGCTTATGACAGCAATAATCATAAAAACCTCAAACC GG---CCACTTAAATGCTTACCAACAGTAGAATTGATAAAT | 1.91% | -3 |
| CR001137 PS | AAATGCTTATGACAGCAATAATCATAAAAACCTCAAAC--- -------TTAAATGCTTACCAACAGTAGAATTGATAAAT | 1.81% | -10 |
| CR001137 | AAATGCTTATGACAGCAATAATCATAAAAACCTCAAACC | 1.28% | -7 |
| PS | GGT-------TAAATGCTTACCAACAGTAGAATTGATAAAT | | |
| | | | |
| CR001137 PS sg | AAATGCTTATGACAGCAATAATCATAAAAACCTCAAACC GGTAGgCCACTTAAATGCTTACCAACAGTAGAATTGATAA AT | 11.90% | 1 |
| CR001137 PS sg | AAATGCTTATGACAGCAATAATCATAAAAACCTCAAACC- -------ACTTAAATGCTTACCAACAGTAGAATTGATAAAT | 7.45% | -7 |
| CR001137 PS sg | AAATGCTTATGACAGCAATAATCATAAAAACCTCAAACC GGTAG--------------------------AATTGATAAAT | 4.87% | -25 |
| CR001137 PS sg | 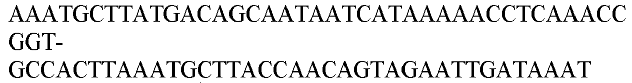 | 3.82% | -1 |
| CR001137 PS sg | AAATGCTTATGACAGCAATAATCATAAAAACCTCAAACC GGTAaGCCACTTAAATGCTTACCAACAGTAGAATTGATAA AT | 3.57% | 1 |
| CR001137 PS sg | AAATGCTTATGACAGCAATAATCATAAAAACCTCAAACC GGTA- CCACTTAAATGCTTACCAACAGTAGAATTGATAAAT | 2.81% | -1 |
| CR001137 PS sg | AAATGCTTATGACAGCAATAATCATAAAAACCTCAAACC GGTA---ACTTAAATGCTTACCAACAGTAGAATTGATAAAT | 2.35% | -3 |
| | | | |
| CR001137 sg | AAATGCTTATGACAGCAATAATCATAAAAACCTCAAACC- ------ACTTAAATGCTTACCAACAGTAGAATTGATAAAT | 6.84% | -7 |
| CR001137 sg | AAATGCTTATGACAGCAATAATCATAAAAACCTCAAACC GGTAGgCCACTTAAATGCTTACCAACAGTAGAATTGATAA AT | 4.54% | 1 |
| CR001137 sg | AAATGCTTATGACAGCAATAATCATAAAAACCTCAAACC GGTAaGCCACTTAAATGCTTACCAACAGTAGAATTGATAA AT | 2.46% | 1 |
| CR001137 sg | AAATGCTTATGACAGCAATAATCATAAAAACCTCAAACC GGT-------TAAATGCTTACCAACAGTAGAATTGATAAAT | 1.83% | -7 |
| CR001137 sg | AAATGCTTATGACAGCAATAATCATAAAAACCTCAAACC GGTA--------AATGCTTACCAACAGTAGAATTGATAAAT | 1.11% | -8 |
| CR001137 sg | AAATGCTTATGACAGCAATAATCATAAAAACCTCAAACC GGTA----CTTAAATGCTTACCAACAGTAGAATTGATAAAT | 0.78% | -4 |
| CR001137 sg | AAATGCTTATGACAGCAATAATCATAAAAACCTCAAACC GGTAtGCCACTTAAATGCTTACCAACAGTAGAATTGATAA AT | 0.72% | 1 |
| | | | |
| CR001221 | 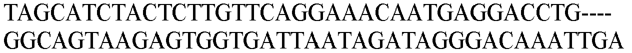 | 29.13% | -4 |
| CR001221 | TAGCATCTACTCTTGTTCAGGAAACAATGAGGACCT- ACTGGGCAGTAAGAGTGGTGATTAATAGATAGGGACAAA TTGA | 4.65% | -1 |
| CR001221 | TAGCATCTACTCTTGTTCAGGAAACAATGAGGAC----- TGGGCAGTAAGAGTGGTGATTAATAGATAGGGACAAATT GA | 4.27% | -5 |
| CR001221 | TAGCATCTACTCTTGTTCAGGAAACAATGAGG---------- GCAGTAAGAGTGGTGATTAATAGATAGGGACAAATTGA | 1.47% | -10 |
| CR001221 | TAGCATCTACTCTTGTTCAGGAAACAATGAGGAC---------- AGTAAGAGTGGTGATTAATAGATAGGGACAAATTGA | 1.14% | -10 |
| CR001221 | TAGCATCTACTCTTGTTCAGG------------- | 1.04% | -25 |
| | TAAGAGTGGTGATTAATAGATAGGGACAAATTGA | | |
| CR001221 | TAGCATCTACTCTTGTTCAGGAAACAATGAGGACCTtACT GGGCAGTAAGAGTGGTGATTAATAGATAGGGACAAATTG A | 0.86% | 0 |
| | | | |
| CR001221 OMePS sg | TAGCATCTACTCTTGTTCAGGAAACAATGAGGACCTG---- GGCAGTAAGAGTGGTGATTAATAGATAGGGACAAATTGA | 26.33% | -4 |
| CR001221 OMePS sg | TAGCATCTACTCTTGTTCAGGAAACAATGAGGACCT- ACTGGGCAGTAAGAGTGGTGATTAATAGATAGGGACAAA TTGA | 15.83% | -1 |
| CR001221 OMePS sg | TAGCATCTACTCTTGTTCAGGAAACAATGAGGAC----- TGGGCAGTAAGAGTGGTGATTAATAGATAGGGACAAATT GA | 6.26% | -5 |
| CR001221 OMePS sg | TAGCATCTACTCTTGTTCAGGAAACAATGAGGACCTG-- TGGGCAGTAAGAGTGGTGATTAATAGATAGGGACAAATT GA | 3.75% | -2 |
| CR001221 OMePS sg | TAGCATCTACTCTTGTTCAGGAAACAATGAGGACCTGtAC TGGGCAGTAAGAGTGGTGATTAATAGATAGGGACAAATT GA | 2.61% | 1 |
| CR001221 OMePS sg | TAGCATCTACTCTTGTTCAGGAAACAATGAGGAC---------- AGTAAGAGTGGTGATTAATAGATAGGGACAAATTGA | 2.15% | -10 |
| CR001221 OMePS sg | TAGCATCTACTCTTGTTCAGGAAACAATGAGGACCTGAaC TGGGCAGTAAGAGTGGTGATTAATAGATAGGGACAAATT GA | 1.82% | 1 |
| | | | |
| CR001221 OMePS | TAGCATCTACTCTTGTTCAGGAAACAATGAGGACCTG---- GGCAGTAAGAGTGGTGATTAATAGATAGGGACAAATTGA | 32.73% | -4 |
| CR001221 OMePS | TAGCATCTACTCTTGTTCAGGAAACAATGAGGACCT- ACTGGGCAGTAAGAGTGGTGATTAATAGATAGGGACAAA TTGA | 6.46% | -1 |
| CR001221 OMePS | TAGCATCTACTCTTGTTCAGGAAACAATGAGGAC----- TGGGCAGTAAGAGTGGTGATTAATAGATAGGGACAAATT GA | 5.49% | -5 |
| CR001221 OMePS | TAGCATCTACTCTTGTTCAGGAAACAATGAGG----------- CAGTAAGAGTGGTGATTAATAGATAGGGACAAATTGA | 3.16% | -11 |
| CR001221 OMePS | TAGCATCTACTCTTGTTCAGGAAACAATGAGG---------- GCAGTAAGAGTGGTGATTAATAGATAGGGACAAATTGA | 2.03% | -10 |
| CR001221 OMePS | TAGCATCTACTCTTGTTCAGGAAACAATGAGGACCTGtAC TGGGCAGTAAGAGTGGTGATTAATAGATAGGGACAAATT GA | 1.53% | 1 |
| CR001221 OMePS | TAGCATCTACTCTTGTTCAGGAAACAATG------------ GGCAGTAAGAGTGGTGATTAATAGATAGGGACAAATTGA | 1.05% | -12 |
| | | | |
| CR001221 PS | TAGCATCTACTCTTGTTCAGGAAACAATGAGGACCTG---- GGCAGTAAGAGTGGTGATTAATAGATAGGGACAAATTGA | 33.94% | -4 |
| CR001221 PS | TAGCATCTACTCTTGTTCAGGAAACAATGAGGACCT- ACTGGGCAGTAAGAGTGGTGATTAATAGATAGGGACAAA TTGA | 7.39% | -1 |
| CR001221 PS | TAGCATCTACTCTTGTTCAGGAAACAATGAGGAC----- TGGGCAGTAAGAGTGGTGATTAATAGATAGGGACAAATT GA | 5.05% | -5 |
| CR001221 PS | TAGCATCTACTCTTGTTCAGGAAACAATGAGGAC---------- AGTAAGAGTGGTGATTAATAGATAGGGACAAATTGA | 4.03% | -10 |
| CR001221 PS | TAGCATCTACTCTTGTTCAGGAAACAATGAGGACCTGtAC TGGGCAGTAAGAGTGGTGATTAATAGATAGGGACAAATT GA | 1.52% | 1 |
| CR001221 PS | TAGCATCTACTCTTGTTCAGGAAACAATG------------ GGCAGTAAGAGTGGTGATTAATAGATAGGGACAAATTGA | 1.48% | -12 |
| CR001221 PS | TAGCATCTACTCTTGTTCAGGAAACAATGAGG----------- CAGTAAGAGTGGTGATTAATAGATAGGGACAAATTGA | 1.43% | -11 |
| | | | |
| CR001221 PS sg | TAGCATCTACTCTTGTTCAGGAAACAATGAGGACCTG---- GGCAGTAAGAGTGGTGATTAATAGATAGGGACAAATTGA | 27.00% | -4 |
| CR001221 PS sg | TAGCATCTACTCTTGTTCAGGAAACAATGAGGACCT- ACTGGGCAGTAAGAGTGGTGATTAATAGATAGGGACAAA TTGA | 13.43% | -1 |
| CR001221 PS sg | TAGCATCTACTCTTGTTCAGGAAACAATGAGGAC----- TGGGCAGTAAGAGTGGTGATTAATAGATAGGGACAAATT GA | 6.57% | -5 |
| CR001221 PS sg | TAGCATCTACTCTTGTTCAGGAAACAATGAGGACCTGtAC TGGGCAGTAAGAGTGGTGATTAATAGATAGGGACAAATT GA | 4.28% | 1 |
| CR001221 PS sg | TAGCATCTACTCTTGTTCAGGAAACAATGAGGACC-- ACTGGGCAGTAAGAGTGGTGATTAATAGATAGGGACAAA TTGA | 3.13% | -2 |
| CR001221 PS sg | TAGCATCTACTCTTGTTCAGGAAACAATGAGGAC---------- AGTAAGAGTGGTGATTAATAGATAGGGACAAATTGA | 2.40% | -10 |
| CR001221 PS sg | TAGCATCTACTCTTGTTCAGGAAACAATGAGGACCTG------ --GTAAGAGTGGTGATTAATAGATAGGGACAAATTGA | 2.13% | -8 |
| | | | |
| CR001221 sg | TAGCATCTACTCTTGTTCAGGAAACAATGAGGACCTG---- GGCAGTAAGAGTGGTGATTAATAGATAGGGACAAATTGA | 26.35% | -4 |
| CR001221 sg | TAGCATCTACTCTTGTTCAGGAAACAATGAGGACCT- ACTGGGCAGTAAGAGTGGTGATTAATAGATAGGGACAAA TTGA | 8.55% | -1 |
| CR001221 sg | TAGCATCTACTCTTGTTCAGGAAACAATGAGGAC----- TGGGCAGTAAGAGTGGTGATTAATAGATAGGGACAAATT GA | 7.25% | -5 |
| CR001221 sg | TAGCATCTACTCTTGTTCAGGAAACAATGAGGAC---------- AGTAAGAGTGGTGATTAATAGATAGGGACAAATTGA | 2.41% | -10 |
| CR001221 sg | TAGCATCTACTCTTGTTCAGGAAACAATGAGG----------- CAGTAAGAGTGGTGATTAATAGATAGGGACAAATTGA | 1.90% | -11 |
| CR001221 sg | TAGCATCTACTCTTGTTCAGGAAACAATGAGGACCTG------ ---TAAGAGTGGTGATTAATAGATAGGGACAAATTGA | 1.82% | -9 |
| CR001221 sg | TAGCATCTACTCTTGTTCAGGAAACAATG------------ GGCAGTAAGAGTGGTGATTAATAGATAGGGACAAATTGA | 1.81% | -12 |
| | | | |
| CR003035 | 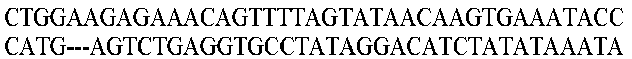 | 12.23% | -3 |
| CR003035 | 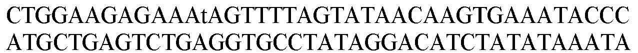 | 8.25% | 0 |
| CR003035 | 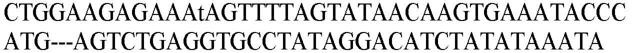 | 4.47% | -3 |
| CR003035 | 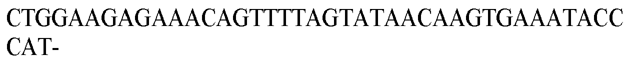 | 3.60% | -1 |
| | CTGAGTCTGAGGTGCCTATAGGACATCTATATAAATA | | |
| CR003035 | CTGGAAGAGAAACAGTTTTAGTATAACAAGTGA------------- -GTCTGAGGTGCCTATAGGACATCTATATAAATA | 2.79% | -14 |
| CR003035 | CTGGAAGAGAAACAGTTTTAGTATAACAAGTGAAATACC CATG-- GAGTCTGAGGTGCCTATAGGACATCTATATAAATA | 2.55% | -2 |
| CR003035 | CTGGAAGAGAAACAGTTTTAGTATAACAAGTGAAATAC--- ---TGAGTCTGAGGTGCCTATAGGACATCTATATAAATA | 1.68% | -6 |
| | | | |
| CR003035 OMePS sg | CTGGAAGAGAAACAGTTTTAGTATAACAAGTGAAATACC CATG---AGTCTGAGGTGCCTATAGGACATCTATATAAATA | 13.59% | -3 |
| CR003035 OMePS sg | CTGGAAGAGAAACAGTTTTAGTATAACAAGTGAAATACC CA--CTGAGTCTGAGGTGCCTATAGGACATCTATATAAATA | 5.56% | -2 |
| CR003035 OMePS sg | CTGGAAGAGAAACAGTTTTAGTATAACAAGTGAAATACC CAT- CTGAGTCTGAGGTGCCTATAGGACATCTATATAAATA | 5.34% | -1 |
| CR003035 OMePS sg | CTGGAAGAGAAACAGTTTTAGTATAACAAGTGAAATACC CATG-- GAGTCTGAGGTGCCTATAGGACATCTATATAAATA | 5.30% | -2 |
| CR003035 OMePS sg | CTGGAAGAGAAACAGTTTTAGTATAACAAGTGA------------- -GTCTGAGGTGCCTATAGGACATCTATATAAATA | 4.70% | -14 |
| CR003035 OMePS sg | CTGGAAGAGAAAtAGTTTTAGTATAACAAGTGAAATACCC ATG---AGTCTGAGGTGCCTATAGGACATCTATATAAATA | 4.44% | -3 |
| CR003035 OMePS sg | CTGGAAGAGAAACAGTTTTAGTATAACAAGTGAAATACC CATG- TGAGTCTGAGGTGCCTATAGGACATCTATATAAATA | 2.05% | -1 |
| | | | |
| CR003035 OMePS | CTGGAAGAGAAACAGTTTTAGTATAACAAGTGAAATACC CATG---AGTCTGAGGTGCCTATAGGACATCTATATAAATA | 9.22% | -3 |
| CR003035 OMePS | CTGGAAGAGAAAtAGTTTTAGTATAACAAGTGAAATACCC ATGCTGAGTCTGAGGTGCCTATAGGACATCTATATAAATA | 7.81% | 0 |
| CR003035 OMePS | CTGGAAGAGAAAtAGTTTTAGTATAACAAGTGAAATACCC ATG---AGTCTGAGGTGCCTATAGGACATCTATATAAATA | 3.79% | -3 |
| CR003035 OMePS | CTGGAAGAGAAACAGTTTTAGTATAACAAGTGAAATACC CAT- CTGAGTCTGAGGTGCCTATAGGACATCTATATAAATA | 2.63% | -1 |
| CR003035 OMePS | CTGGAAGAGAAACAGTTTTAGTATAACAAGTGAAATACC CA--CTGAGTCTGAGGTGCCTATAGGACATCTATATAAATA | 2.00% | -2 |
| CR003035 OMePS | CTGGAAGAGAAACAGTTTTAGTATAACAAGTGA------------- -GTCTGAGGTGCCTATAGGACATCTATATAAATA | 1.92% | -14 |
| CR003035 OMePS | CTGGAAGAGAAACAGTTTTAGTATAACAAGTGAAATACC- ----TGAGTCTGAGGTGCCTATAGGACATCTATATAAATA | 1.69% | -5 |
| | | | |
| CR003035 PS | CTGGAAGAGAAACAGTTTTAGTATAACAAGTGAAATACC CATG---AGTCTGAGGTGCCTATAGGACATCTATATAAATA | 13.91% | -3 |
| CR003035 PS | CTGGAAGAGAAAtAGTTTTAGTATAACAAGTGAAATACCC ATG---AGTCTGAGGTGCCTATAGGACATCTATATAAATA | 6.11% | -3 |
| CR003035 PS | CTGGAAGAGAAAtAGTTTTAGTATAACAAGTGAAATACCC ATGCTGAGTCTGAGGTGCCTATAGGACATCTATATAAATA | 3.77% | 0 |
| CR003035 PS | CTGGAAGAGAAACAGTTTTAGTATAACAAGTGAAATACC CAT- CTGAGTCTGAGGTGCCTATAGGACATCTATATAAATA | 3.64% | -1 |
| CR003035 PS | CTGGAAGAGAAACAGTTTTAGTATAACAAGTGAAATACC CATG-- GAGTCTGAGGTGCCTATAGGACATCTATATAAATA | 2.39% | -2 |
| CR003035 PS | CTGGAAGAGAAACAGTTTTAGTATAACAAGTGAAATACC CA--CTGAGTCTGAGGTGCCTATAGGACATCTATATAAATA | 2.16% | -2 |
| CR003035 PS | CTGGAAGAGAAAtAGTTTTAGTATAACAAGTGAAATACCC AT-CTGAGTCTGAGGTGCCTATAGGACATCTATATAAATA | 1.87% | -1 |
| | | | |
| CR003035 PS sg | CTGGAAGAGAAACAGTTTTAGTATAACAAGTGAAATACC CAT- CTGAGTCTGAGGTGCCTATAGGACATCTATATAAATA | 8.32% | -1 |
| CR003035 PS sg | CTGGAAGAGAAACAGTTTTAGTATAACAAGTGAAATACC CATG---AGTCTGAGGTGCCTATAGGACATCTATATAAATA | 6.41% | -3 |
| CR003035 PS sg | CTGGAAGAGAAACAGTTTTAGTATAACAAGTGAAATACC CATG-- GAGTCTGAGGTGCCTATAGGACATCTATATAAATA | 4.19% | -2 |
| CR003035 PS sg | CTGGAAGAGAAACAGTTTTAGTATAACAAGTGAAATACC CA--CTGAGTCTGAGGTGCCTATAGGACATCTATATAAATA | 4.08% | -2 |
| CR003035 PS sg | CTGGAAGAGAAAtAGTTTTAGTATAACAAGTGAAATACCC AT-CTGAGTCTGAGGTGCCTATAGGACATCTATATAAATA | 3.31% | -1 |
| CR003035 PS sg | CTGGAAGAGAAACAGTTTTAGTATAACAAGTGAAATACC C----TGAGTCTGAGGTGCCTATAGGACATCTATATAAATA | 2.99% | -4 |
| CR003035 PS sg | CTGGAAGAGAAACAGTTTTAGTATAACAAGTGAAATACC- ----TGAGTCTGAGGTGCCTATAGGACATCTATATAAATA | 2.23% | -5 |
| | | | |
| CR003035 sg | CTGGAAGAGAAACAGTTTTAGTATAACAAGTGAAATACC CATG---AGTCTGAGGTGCCTATAGGACATCTATATAAATA | 10.92% | -3 |
| CR003035 sg | CTGGAAGAGAAAtAGTTTTAGTATAACAAGTGAAATACCC ATG---AGTCTGAGGTGCCTATAGGACATCTATATAAATA | 6.05% | -3 |
| CR003035 sg | CTGGAAGAGAAACAGTTTTAGTATAACAAGTGAAATACC CAT- CTGAGTCTGAGGTGCCTATAGGACATCTATATAAATA | 5.42% | -1 |
| CR003035 sg | CTGGAAGAGAAAtAGTTTTAGTATAACAAGTGAAATACCC ATGCTGAGTCTGAGGTGCCTATAGGACATCTATATAAATA | 4.55% | 0 |
| CR003035 sg | CTGGAAGAGAAACAGTTTTAGTATAACAAGTGAAATACC CA--CTGAGTCTGAGGTGCCTATAGGACATCTATATAAATA | 4.53% | -2 |
| CR003035 sg | CTGGAAGAGAAACAGTTTTAGTATAACAAGTGAAATACC CATG-- GAGTCTGAGGTGCCTATAGGACATCTATATAAATA | 3.59% | -2 |
| CR003035 sg | 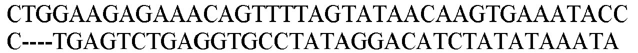 | 2.30% | -4 |
| | | | |
| CR001128 | 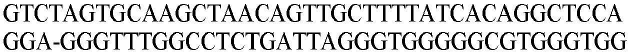 | 10.38% | -1 |
| CR001128 | 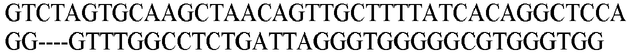 | 5.87% | -4 |
| CR001128 | GTCTAGTGCAAGCTAACAGTTGCTTTTATCACAGGCTCCA GGAAaGGGTTTGGCCTCTGATTAGGGTGGGGGCGTGGGT GG | 5.50% | 1 |
| CR001128 | GTCTAGTGCAAGCTAACAGTTGCTTTTATCACAGGCTCCA GG-----TTTGGCCTCTGATTAGGGTGGGGGCGTGGGTGG | 4.87% | -5 |
| CR001128 | GTCTAGTGCAAGCTAACAGTTGCTTTATCACAGGC----------------- -------------------------------GTGGGTGG | 4.61% | -36 |
| CR001128 | GTCTAGTGCAAGCTAACAaC-------------------------------------- ATCACAGGCTCCAGGATAGGGGGCGTGGGTGG | 2.38% | -28 |
| CR001128 | GTCTAGTGCAAGCTAACAGTTGCTTTTATCACAGGCTCCA GGA--GGTTTGGCCTCTGATTAGGGTGGGGGCGTGGGTGG | 2.00% | -2 |
| | | | |
| CR001128 OMePS | GTCTAGTGCAAGCTAACAGTTGCTTTTATCACAGGCTCCA GG------------------------TGGGGGCGTGGGTGG | 7.56% | -23 |
| CR001128 OMePS | GTCTAGTGCAAGCTAACAGTTGCTTTATCACAGGCTCCA GG----GTTTGGCCTCTGATTAGGGTGGGGGCGTGGGTGG | 6.53% | -4 |
| CR001128 OMePS | GTCTAGTGCAAGCTAACAGTTGCTTTTATCACAGGCTCCA GG-----TTTGGCCTCTGATTAGGGTGGGGGCGTGGGTGG | 6.18% | -5 |
| CR001128 OMePS | GTCTAGTGCAAGCTAACAGTTGCTTTTATCACAGGCTCCA GGA-GGGTTTGGCCTCTGATTAGGGTGGGGGCGTGGGTGG | 6.06% | -1 |
| CR001128 OMePS | GTCTAGTGCAAGCTAACAGTTGCTTTTATCACAGGCTCCA GGAAaGGGTTTGGCCTCTGATTAGGGTGGGGGCGTGGGT GG | 3.92% | 1 |
| CR001128 OMePS | GTCTAGTGCAAGCTAACAGTTGCTTTTATCACAGGCTCCA GG----------CCTCTGATTAGGGTGGGGGCGTGGGTGG | 2.55% | -10 |
| | | | |
| CR001128 sg | GTCTAGTGCAAGCTAACAGTTGCTTTATCACAGGCTCCA GGA-GGGTTTGGCCTCTGATTAGGGTGGGGGCGTGGGTGG | 10.12% | -1 |
| CR001128 sg | GTCTAGTGCAAGCTAACAGTTGCTTTTATCACAGGCTCCA GG-----TTTGGCCTCTGATTAGGGTGGGGGCGTGGGTGG | 7.05% | -5 |
| CR001128 sg | GTCTAGTGCAAGCTAACAGTTGCTTTATCACAGGCTCCA GG----GTTTGGCCTCTGATTAGGGTGGGGGCGTGGGTGG | 4.59% | -4 |
| CR001128 sg | GTCTAGTGCAAGCTAACAGTTGCTTTTATCACAGGCTCCA GGAAaGGGTTTGGCCTCTGATTAGGGTGGGGGCGTGGGT GG | 4.24% | 1 |
| CR001128 sg | GTCTAGTGCAAGCTAACAGTTGCTTTTATCACAGGCTCCA GG------------------------------GGCGTGGGTGG | 2.21% | -27 |
| CR001128 sg | GTCTAGTGCAAGCTAACAGTTGCTTTTATCACAGGCTCCA G--------------------------------CGTGGGTGG | 1.66% | -30 |
| CR001128 sg | GTCTAGTGCAAGCTAACAGTTGCTTTATCACAGGC-------- ---------CTCTGATTAGGGTGGGGGCGTGGGTGG | 1.48% | -17 |
| | | | |
| CR001128 sg OMePS | GTCTAGTGCAAGCTAACAGTTGCTTTTATCACAGGCTCCA GG-----TTTGGCCTCTGATTAGGGTGGGGGCGTGGGTGG | 10.59% | -5 |
| CR001128 sg OMePS | GTCTAGTGCAAGCTAACAGTTGCTTTATCACAGGCTCCA GG----GTTTGGCCTCTGATTAGGGTGGGGGCGTGGGTGG | 7.59% | -4 |
| CR001128 sg OMePS | GTCTAGTGCAAGCTAACAGTTGCTTTTATCACAGGCTCCA GGA-GGGTTTGGCCTCTGATTAGGGTGGGGGCGTGGGTGG | 5.92% | -1 |
| CR001128 sg OMePS | GTCTAGTGCAAGCTAACAGTTGCTTTATCACAGGCTCCA GGAAaGGGTTTGGCCTCTGATTAGGGTGGGGGCGTGGGT GG | 3.38% | 1 |
| CR001128 sg OMePS | GTCTAGTGCAAGCTAACAGTTGCTTTTATCACAGGCTCCA GGAAGagGGTTTGGCCTCTGATTAGGGTGGGGGCGTGGGT | 2.49% | 2 |
| | 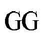 | | |
| CR001128 sg OMePS | 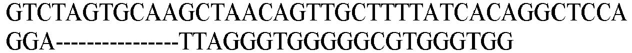 | 1.95% | -16 |
| CR001128 sg OMePS | 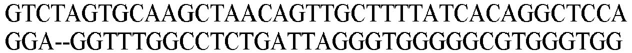 | 1.85% | -2 |
| | | | |
| CR000312 | 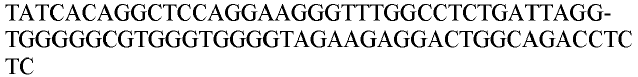 | 11.83% | -1 |
| CR000312 | TATCACAGGCTCCAGGAAGGGTTTGG---------------- GGGCGTGGGTGGGGTAGAAGAGGACTGGCAGACCTCTC | 5.11% | -16 |
| CR000312 | TATCACAGGCTCCAGGAAGGGTTTGGC------------------- GTGGGTGGGGTAGAAGAGGACTGGCAGACCTCTC | 4.33% | -19 |
| CR000312 | TATCACAGGCTCCAGGAAGGGTTTGGCCTCTGATT-- GGTGGGGGCGTGGGTGGGGTAGAAGAGGACTGGCAGAC CTCTC | 3.63% | -2 |
| CR000312 | TATCACAGGCTCCAGGAAGGG----------------------- GCGTGGGTGGGGTAGAAGAGGACTGGCAGACCTCTC | 2.50% | -23 |
| CR000312 | TATCACAGGCTCCAGGAAGGGTTTGGCCTCTG------ GTGGGGGCGTGGGTGGGGTAGAAGAGGACTGGCAGACCT CTC | 2.47% | -6 |
| CR000312 | TATCACAGGCTCCAGGAAGGGTTTGGCCTCTG--------- GGGGCGTGGGTGGGGTAGAAGAGGACTGGCAGACCTCTC | 2.35% | -9 |
| | | | |
| CR000312 OMePS | TATCACAGGCTCCAGGAAGGGTTTGGCCTCTGATTAGG- TGGGGGCGTGGGTGGGGTAGAAGAGGACTGGCAGACCTC TC | 12.06% | -1 |
| CR000312 OMePS | TATCACAGGCTCCAGGAAGGGTTTGG------------- TGGGGGCGTGGGTGGGGTAGAAGAGGACTGGCAGACCTC TC | 4.75% | -13 |
| CR000312 OMePS | TATCACAGGCTCCAGGAAGGGTTTGGCCTCTGATT-- GGTGGGGGCGTGGGTGGGGTAGAAGAGGACTGGCAGAC CTCTC | 4.32% | -2 |
| CR000312 OMePS | TATCACAGGCTCCAGGAAGGGTTTGGC------------------- GTGGGTGGGGTAGAAGAGGACTGGCAGACCTCTC | 3.97% | -19 |
| CR000312 OMePS | TATCACAGGCTCCAGGAAGGGTTTGG---------------- GGGCGTGGGTGGGGTAGAAGAGGACTGGCAGACCTCTC | 3.28% | -16 |
| CR000312 OMePS | TATCACAGGCTCCAGGAAGGGTTTGGCCTCTGATTAGaGG TGGGGGCGTGGGTGGGGTAGAAGAGGACTGGCAGACCTC TC | 3.25% | 1 |
| CR000312 OMePS | TATCACAGGCTCCAGGAAGGGTTTGGCCTCTGATTAG-- TGGGGGCGTGGGTGGGGTAGAAGAGGACTGGCAGACCTC TC | 1.91% | -2 |
| | | | |
| CR000312 sg | TATCACAGGCTCCAGGAAGGGTTTGGCCTCTGATTAG-- TGGGGGCGTGGGTGGGGTAGAAGAGGACTGGCAGACCTC TC | 6.69% | -2 |
| CR000312 sg | TATCACAGGCTCCAGGAAGGGTTTGGCCTCTGATTAGG- TGGGGGCGTGGGTGGGGTAGAAGAGGACTGGCAGACCTC TC | 6.28% | -1 |
| CR000312 sg | TATCACAGGCTCCAGGAAGGGTTTGGCCTCTGA-------------- ----------------AGGACTGGCAGACCTCTC | 4.58% | -29 |
| CR000312 sg | TATCACAGGCTCCAGGAAGGGTTTGGCCTCTGATTAGaGG TGGGGGCGTGGGTGGGGTAGAAGAGGACTGGCAGACCTC TC | 3.83% | 1 |
| CR000312 sg | TATCACAGGCTCCAGGAAGGGTTTGG---------------- GGGCGTGGGTGGGGTAGAAGAGGACTGGCAGACCTCTC | 3.74% | -16 |
| CR000312 sg | TATCACAGGCTCCAGGAAGGGTTTGGCCTCTG--------- GGGGCGTGGGTGGGGTAGAAGAGGACTGGCAGACCTCTC | 3.66% | -9 |
| CR000312 sg | TATCACAGGCTCCAGGAAGGGTTTGGCCTCTGATTAGGG- ------CGTGGGTGGGGTAGAAGAGGACTGGCAGACCTCTC | 3.19% | -6 |
| CR000312 sg | TATCACAGGCTCCAGGAAGGGTTTGG------------- TGGGGGCGTGGGTGGGGTAGAAGAGGACTGGCAGACCTC TC | 3.18% | -13 |
| | | | |
| CR000312 sg OMePS | TATCACAGGCTCCAGGAAGGGTTTGGCCTCTGATTAGG- TGGGGGCGTGGGTGGGGTAGAAGAGGACTGGCAGACCTC TC | 12.59% | -1 |
| CR000312 sg OMePS | TATCACAGGCTCCAGGAAGGGTTTGGCCTCTGATT-- GGTGGGGGCGTGGGTGGGGTAGAAGAGGACTGGCAGAC CTCTC | 7.73% | -2 |
| CR000312 sg OMePS | TATCACAGGCTCCAGGAAGGGTTTGGCCTCTG--------- GGGGCGTGGGTGGGGTAGAAGAGGACTGGCAGACCTCTC | 6.49% | -9 |
| CR000312 sg OMePS | TATCACAGGCTCCAGGAAGGGTTTGG---------------- GGGCGTGGGTGGGGTAGAAGAGGACTGGCAGACCTCTC | 5.32% | -16 |
| CR000312 sg OMePS | TATCACAGGCTCCAGGAAGGGTTTGGC------------------- GTGGGTGGGGTAGAAGAGGACTGGCAGACCTCTC | 3.89% | -19 |
| CR000312 sg OMePS | TATCACAGGCTCCAGGAAGGG---------------------- GGCGTGGGTGGGGTAGAAGAGGACTGGCAGACCTCTC | 2.50% | -22 |
| CR000312 sg OMePS | TATCACAGGCTCCAGGAAGGGTTTGG------------- TGGGGGCGTGGGTGGGGTAGAAGAGGACTGGCAGACCTC TC | 2.50% | -13 |
| CR000312 sg OMePS | TATCACAGGCTCCAGGAAGGGTTTGGCCTCTGATTAG-- TGGGGGCGTGGGTGGGGTAGAAGAGGACTGGCAGACCTC TC | 2.25% | -2 |
| | | | |
| CR003085 | 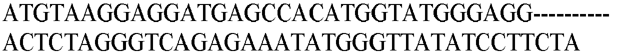 | 12.51% | -10 |
| CR003085 | ATGTAAGGAGGATGAGCCACATGGTATGGGAGGTAT- CTAAGGACTCTAGGGTCAGAGAAATATGGGTTATATCCTT CTA | 8.89% | -1 |
| CR003085 | ATGTAAGGAGGATGAGCCACATGGTATGGGAGGTATAaC TAAGGACTCTAGGGTCAGAGAAATATGGGTTATATCCTTC TA | 7.30% | 1 |
| CR003085 | ATGTAAGGAGGATGAGCCACATGGTATGGGA------------ CTCTAGGGTCAGAGAAATATGGGTTATATCCTTCTA | 6.20% | -13 |
| CR003085 | ATGTAAGGAGGATGAGCCACATGGTA-------------- AGGACTCTAGGGTCAGAGAAATATGGGTTATATCCTTCTA | 3.50% | -14 |
| CR003085 | ATGTAAGGAGGATGAGCCACATGGTATGGGAGGTA----- AGGACTCTAGGGTCAGAGAAATATGGGTTATATCCTTCTA | 3.09% | -5 |
| CR003085 | ATGTAAGGAGGATGAGCCACATGGTATGGGAGGTATA--- AGGACTCTAGGGTCAGAGAAATATGGGTTATATCCTTCTA | 2.29% | -3 |
| CR003085 | ATGTAAGGAGGATGAGCCACATGGTATGGGAGGTATA- TAAGGACTCTAGGGTCAGAGAAATATGGGTTATATCCTTC TA | 1.64% | -1 |
| CR003085 | ATGTAAGGAGGATGAGCCACATGGTATGG--------- TAAGGACTCTAGGGTCAGAGAAATATGGGTTATATCCTTC TA | 1.43% | -9 |
| CR003085 | ATGTAAGGAGGATGAGCCACATGGTATGGGAGGTATA-- AAGGACTCTAGGGTCAGAGAAATATGGGTTATATCCTTCT A | 1.39% | -2 |
| | | | |
| CR003085 OMePS sg | ATGTAAGGAGGATGAGCCACATGGTATGGGAGGTATAaC TAAGGACTCTAGGGTCAGAGAAATATGGGTTATATCCTTC TA | 13.65% | 1 |
| CR003085 OMePS sg | ATGTAAGGAGGATGAGCCACATGGTATGGGAGG---------- ACTCTAGGGTCAGAGAAATATGGGTTATATCCTTCTA | 10.96% | -10 |
| CR003085 OMePS sg | ATGTAAGGAGGATGAGCCACATGGTATGGGA------------ CTCTAGGGTCAGAGAAATATGGGTTATATCCTTCTA | 8.21% | -13 |
| CR003085 OMePS sg | ATGTAAGGAGGATGAGCCACATGGTATGGGAGGTAT- CTAAGGACTCTAGGGTCAGAGAAATATGGGTTATATCCTT CTA | 8.15% | -1 |
| CR003085 OMePS sg | ATGTAAGGAGGATGAGCCACATGGTA-------------- AGGACTCTAGGGTCAGAGAAATATGGGTTATATCCTTCTA | 6.29% | -14 |
| CR003085 OMePS sg | ATGTAAGGAGGATGAGCCACATGGTATGGGAGGTA----- AGGACTCTAGGGTCAGAGAAATATGGGTTATATCCTTCTA | 4.53% | -5 |
| CR003085 OMePS sg | ATGTAAGGAGGATGAGCCACATGGTATGGGAGGTATA--- AGGACTCTAGGGTCAGAGAAATATGGGTTATATCCTTCTA | 3.60% | -3 |
| CR003085 OMePS sg | ATGTAAGGAGGATGAGCCACATGGTATGGGAGGTA-- CTAAGGACTCTAGGGTCAGAGAAATATGGGTTATATCCTT CTA | 3.47% | -2 |
| CR003085 OMePS sg | ATGTAAGGAGGATGAGCCACATGGTATGGGAGGTATA- TAAGGACTCTAGGGTCAGAGAAATATGGGTTATATCCTTC TA | 1.30% | -1 |
| CR003085 OMePS sg | ATGTAAGGAGGATGAGCCACATGGTATGGG-------------- ------------------TTATATCCTTCTA | 1.00% | -37 |
| | | | |
| CR003085 OMePS | ATGTAAGGAGGATGAGCCACATGGTATGGGAGGTATAaC TAAGGACTCTAGGGTCAGAGAAATATGGGTTATATCCTTC TA | 10.09% | 1 |
| CR003085 OMePS | ATGTAAGGAGGATGAGCCACATGGTATGGGAGG---------- ACTCTAGGGTCAGAGAAATATGGGTTATATCCTTCTA | 9.05% | -10 |
| CR003085 OMePS | ATGTAAGGAGGATGAGCCACATGGTATGGGAGGTAT- CTAAGGACTCTAGGGTCAGAGAAATATGGGTTATATCCTT CTA | 6.52% | -1 |
| CR003085 OMePS | ATGTAAGGAGGATGAGCCACATGGTATGGGA------------- CTCTAGGGTCAGAGAAATATGGGTTATATCCTTCTA | 4.94% | -13 |
| CR003085 OMePS | ATGTAAGGAGGATGAGCCACATGGTA-------------- AGGACTCTAGGGTCAGAGAAATATGGGTTATATCCTTCTA | 4.55% | -14 |
| CR003085 OMePS | ATGTAAGGAGGATGAGCCACATGGTATGGGAGGTA----- AGGACTCTAGGGTCAGAGAAATATGGGTTATATCCTTCTA | 2.40% | -5 |
| CR003085 OMePS | ATGTAAGGAGGATGAGCCACATGGTATGGGAGGTA-- CTAAGGACTCTAGGGTCAGAGAAATATGGGTTATATCCTT CTA | 2.00% | -2 |
| CR003085 OMePS | ATGTAAGGAGGATGAGCCACATGGTATGGGAGGTATA--- AGGACTCTAGGGTCAGAGAAATATGGGTTATATCCTTCTA | 1.82% | -3 |
| CR003085 OMePS | ATGTAAGGAGGATGAGCCACATGGTATGGGAGGTATA- TAAGGACTCTAGGGTCAGAGAAATATGGGTTATATCCTTC | 1.57% | -1 |
| | TA | | |
| CR003085 OMePS | ATGTAAGGAGGATGAGCCACATGGTATGGGAGGT---------------- ---------------------------TATATCCTTCTA | 1.16% | -34 |
| | | | |
| CR003085 PS | ATGTAAGGAGGATGAGCCACATGGTATGGGAGG---------- ACTCTAGGGTCAGAGAAATATGGGTTATATCCTTCTA | 15.06% | -10 |
| CR003085 PS | ATGTAAGGAGGATGAGCCACATGGTATGGGAGGTAT- CTAAGGACTCTAGGGTCAGAGAAATATGGGTTATATCCTT CTA | 11.15% | -1 |
| CR003085 PS | ATGTAAGGAGGATGAGCCACATGGTATGGGAGGTATAaC TAAGGACTCTAGGGTCAGAGAAATATGGGTTATATCCTTC TA | 8.37% | 1 |
| CR003085 PS | ATGTAAGGAGGATGAGCCACATGGTATGGGA------------- CTCTAGGGTCAGAGAAATATGGGTTATATCCTTCTA | 7.44% | -13 |
| CR003085 PS | ATGTAAGGAGGATGAGCCACATGGTA-------------- AGGACTCTAGGGTCAGAGAAATATGGGTTATATCCTTCTA | 5.33% | -14 |
| CR003085 PS | ATGTAAGGAGGATGAGCCACATGGTATGGGAGGTA----- AGGACTCTAGGGTCAGAGAAATATGGGTTATATCCTTCTA | 3.58% | -5 |
| CR003085 PS | ATGTAAGGAGGATGAGCCACATGGTATGGGAGGTATA--- AGGACTCTAGGGTCAGAGAAATATGGGTTATATCCTTCTA | 3.50% | -3 |
| CR003085 PS | ATGTAAGGAGGATGAGCCACATGGTATGGGAGGTA-- CTAAGGACTCTAGGGTCAGAGAAATATGGGTTATATCCTT CTA | 2.05% | -2 |
| CR003085 PS | ATGTAAGGAGGATGAGCCACATGGTATGG-------------- ACTCTAGGGTCAGAGAAATATGGGTTATATCCTTCTA | 1.39% | -14 |
| CR003085 PS | ATGTAAGGAGGATGAGCCACATGGTATGGGAGGTATA- TAAGGACTCTAGGGTCAGAGAAATATGGGTTATATCCTTC TA | 0.86% | -1 |
| | | | |
| CR003085 PS sg | ATGTAAGGAGGATGAGCCACATGGTATGGGAGGTAT- CTAAGGACTCTAGGGTCAGAGAAATATGGGTTATATCCTT CTA | 12.62% | -1 |
| CR003085 PS sg | ATGTAAGGAGGATGAGCCACATGGTATGGGAGGTATAaC TAAGGACTCTAGGGTCAGAGAAATATGGGTTATATCCTTC TA | 11.73% | 1 |
| CR003085 PS sg | ATGTAAGGAGGATGAGCCACATGGTATGGGAGG---------- ACTCTAGGGTCAGAGAAATATGGGTTATATCCTTCTA | 8.86% | -10 |
| CR003085 PS sg | ATGTAAGGAGGATGAGCCACATGGTATGGGA------------- CTCTAGGGTCAGAGAAATATGGGTTATATCCTTCTA | 6.59% | -13 |
| CR003085 PS sg | ATGTAAGGAGGATGAGCCACATGGTA-------------- AGGACTCTAGGGTCAGAGAAATATGGGTTATATCCTTCTA | 4.88% | -14 |
| CR003085 PS sg | ATGTAAGGAGGATGAGCCACATGGTATGGGAGGTATA--- AGGACTCTAGGGTCAGAGAAATATGGGTTATATCCTTCTA | 3.68% | -3 |
| CR003085 PS sg | ATGTAAGGAGGATGAGCCACATGGTATGGGAGGTA-- CTAAGGACTCTAGGGTCAGAGAAATATGGGTTATATCCTT CTA | 3.62% | -2 |
| CR003085 PS sg | ATGTAAGGAGGATGAGCCACATGGTATGGGAGGTATA- TAAGGACTCTAGGGTCAGAGAAATATGGGTTATATCCTTC TA | 3.48% | -1 |
| CR003085 PS sg | ATGTAAGGAGGATGAGCCACATGGTATGGGAGGTA----- AGGACTCTAGGGTCAGAGAAATATGGGTTATATCCTTCTA | 2.90% | -5 |
| CR003085 | ATGTAAGGAGGATGAGCCACATGGTAT----------- | 1.10% | -12 |
| PS sg | AAGGACTCTAGGGTCAGAGAAATATGGGTTATATCCTTCT A | | |
| | | | |
| CR003085 sg | ATGTAAGGAGGATGAGCCACATGGTATGGGAGG---------- ACTCTAGGGTCAGAGAAATATGGGTTATATCCTTCTA | 11.79% | -10 |
| CR003085 sg | ATGTAAGGAGGATGAGCCACATGGTATGGGAGGTAT- CTAAGGACTCTAGGGTCAGAGAAATATGGGTTATATCCTT CTA | 9.21% | -1 |
| CR003085 sg | ATGTAAGGAGGATGAGCCACATGGTATGGGAGGTATAaC TAAGGACTCTAGGGTCAGAGAAATATGGGTTATATCCTTC TA | 6.84% | 1 |
| CR003085 sg | ATGTAAGGAGGATGAGCCACATGGTATGGGA------------- CTCTAGGGTCAGAGAAATATGGGTTATATCCTTCTA | 6.44% | -13 |
| CR003085 sg | ATGTAAGGAGGATGAGCCACATGGTA-------------- AGGACTCTAGGGTCAGAGAAATATGGGTTATATCCTTCTA | 4.12% | -14 |
| CR003085 sg | ATGTAAGGAGGATGAGCCACATGGTATGGGAGGTA----- AGGACTCTAGGGTCAGAGAAATATGGGTTATATCCTTCTA | 3.46% | -5 |
| CR003085 sg | ATGTAAGGAGGATGAGCCACATGGTATGGGAGGTATA--- AGGACTCTAGGGTCAGAGAAATATGGGTTATATCCTTCTA | 3.22% | -3 |
| CR003085 sg | ATGTAAGGAGGATGAGCCACATGGTATGGGAGGTA-- CTAAGGACTCTAGGGTCAGAGAAATATGGGTTATATCCTT CTA | 1.65% | -2 |
| CR003085 sg | ATGTAAGGAGGATGAGCCACATGGTATGGGAGGTATA- TAAGGACTCTAGGGTCAGAGAAATATGGGTTATATCCTTC TA | 1.07% | -1 |
| CR003085 sg | ATGTAAGGAGGATGAGCCACATGGTATGG-------------- ACTCTAGGGTCAGAGAAATATGGGTTATATCCTTCTA | 0.95% | -14 |

The genome edited and unedited HSPC were analyzed for proliferation and HbF upregulation in a three-stage erythroid differentiation cell culture protocol. The genome edited and unedited HSPC were analyzed by flow cytometry for expression levels of fetal globin and at day 7 for two erythroid cell surface markers, transferrin receptor (CD71) and glycophorin A (CD235a) using antibodies conjugated to fluorescent dyes. The live cells were identified and gated by exclusion of Live/Dead Fixable Dead Cell Stain. Genome editing with the included HPFH region targeting and BCL11A erythroid enhancer targeting gRNAs did not adversely affect erythroid differentiation as the cultured cells showed percentages of CD71+ cells consistent with erythroblasts similar to unedited cells at day 7 of differentiation, although Protocol 1 and Protocol 2 differed in percentages of CD71+ across conditions (Figure 44). Cultures treated with the included HPFH region targeting and BCL11A erythroid enhancer targeting gRNAs resulted in similar patterns of relative changes in the percentage of erythroid cells containing HbF compared to mock electroporated cells throughout the 21 day culture period and between the two protocols (Figures 45, Figure 46 and Figure 47). HbF was induced in cells exposed ot RNPs and maintined in either Protocol 1 or Protocol 2, however, the percentage of cells that were HbF positive tended to be lower across conditions and timepoints with Protocol 2 (Figures 45, Figure 46 and Figure 47). Induction of HbF was most pronounced with gRNAs consisting of target domains CR001030, CR00312 and CR001128, particularly at the later timepoints (Figures 45, Figure 46 and Figure 47). Across targeting domains, sgRNAs trended to higher induction of HbF than dgRNAs, particularly OMePS modified sgRNAs (Figures 45, Figure 46 and Figure 47). Relatively low induction of HbF with targeting domain CR001137 may be explained by the lower level of editing at this target site (Figures 43, Figure 45-Figure 47). Induction of HbF positive cells by dgRNA including the targeting domain of g8 targeting exon 2 of BCL11A was also observed in parallel (Figure 45-Figure 47). An increase in HbF-containing erythrocytes derived from genome edited cells is likely to have therapeutic implications by reduced sickling. Edited cells were capable of proliferating in erythroid differentiation conditions (9 to 53-fold at day 7 and 36 to 143-fold at day 21, Figure 48), however, in some cases, proliferation was suppressed relative to unedited control cells (14-83% of unedited control at day 7 and 18-95% of unedited control at day 21) (Figure 48). Lowest proliferation was observed with the cells edited by dgRNA including the targeting domain of g8 targeting exon 2 of BCL11A (30% of unedited control at day 7 and 18% of unedited control at day 21) and with many of the OMePS modified sgRNAs (Figure 48).

### Example 5 Excision using two gRNAs to the HPFH region

Primary human CD34+ HSPCs were electroporated with RNP containing two different gRNA moleucles to different sites within the HPFH region. For each RNP, dgRNA molecules as described above, were used. Briefly, RNP comprising a dgRNA targeting a first site within the French HPFH region and RNP comprising a dgRNA targeting a second site within the French HPFH region were combined, and electroporated into CD34+ HSPCs in the following ratios: 0.5X first dgRNA RNP + 0.5X second dgRNA RNP; 1.0X g2 dgRNA ("g2" = positive control comprising a targeting domain of CR000088, which targets a coding sequence of the BCL11a gene); no RNPs ("control"). Cells were cultured as described above, and differentiated into erythrocytes using the protocol described above (see Figure 17). Fetal hemoglobin expression was assessed. The results are showin in Figure 27 and Table 25. Without being bound by theory, it is believed that simultaneous introduction of RNPs comprising gRNA molecules to two target sequences will cause the deletion (i.e., excision) of the DNA between the two cut sites (e.g., all or most of the DNA between the two cut sites), including any regulatory or coding sequences contained within that region. These results show that RNPs targeting two separate regions within the HPFH region could be delivered simultaneously to CD34+ HSPCs by electroporation and induce fetal hemoglobin expression in edited cells differentiated into erythrocytes.

**Table 25.**

| Guide RNA targeting domain ID 1/Guide RNA targeting domain ID 2 | % HbF+ (F cells), average of replicates |
|---|---|
| CR001016/CR001021 | 50.9 |
| CR001021/CR001034 | 53.4 |
| CR001034/CR001037 | 56.1 |
| CR001037/CR001045 | 50.6 |
| CR00 1045/CR001058 | 45.2 |
| CR001058/CR001065 | 42.6 |
| CR001065/CR001075 | 43.3 |
| CR001075/CR001086 | 40.4 |
| CR001086/CR001096 | 39.3 |
| CR001096/CR001107 | 41.6 |
| CR001107/CR001135 | 45.6 |
| CR001143/CR001151 | 40.1 |
| CR001151/CR001159 | 40.9 |
| CR001159/CR001166 | 42.0 |
| CR001166/CR001173 | 44.8 |
| CR001173/CR001179 | 43.1 |
| CR001179/CR001191 | 41.9 |
| CR001191/CR001199 | 42.7 |
| CR001199/CR001212 | 48.3 |
| CR001212/CR001227 | 45.6 |
| CR001016/CR001034 | 53.4 |
| CR001034/CR001045 | 50.0 |
| CR001045/CR001065 | 44.2 |
| CR001065/CR001086 | 39.1 |
| CR001086/CR001107 | 43.4 |
| CR001107/CR001143 | 44.8 |
| CR001143/CR001159 | 45.0 |
| CR001159/CR001173 | 46.6 |
| CR001173/CR001191 | 44.8 |
| CR001191/CR001212 | 51.7 |
| CR001021/CR001037 | 56.9 |
| CR001037/CR001058 | 48.0 |
| CR001058/CR001075 | 41.7 |
| CR001075/CR001096 | 41.5 |
| CR001096/CR001135 | 44.3 |
| CR001135/CR001151 | 42.9 |
| CR001151/CR001166 | 41.9 |
| CR001166/CR001179 | 40.4 |
| CR001179/CR001199 | 38.5 |
| CR001199/CR001227 | 40.0 |
| g2 | 54.0 |
| ctrl | 34.5 |

### Example 6. Potential Off-Target Editing Assessment of gRNAs Targeting the BCL11a Enhancer

An oligo insertion based assay (See, e.g., Tsai et al., Nature Biotechnology. 33, 187-197; 2015) was used to determine potential off-target genomic sites cleaved by Cas9 targeting the BCL11a enhancer.

A total of 28 guides (dual guide RNAs comprising the indicated targeting domain) targeting the BCL11a enhancer and 10 gRNAs targeting the HPFH region were screened in the Cas9-expressing HEK293 cells described above, and the results are plotted in Figure 33 (BCL11a enhancer) and Figure 49 (HPFH). Withr repsect to the gRNAs targeting the BCL11a enhancer, the assay identified potential off-target sites for some guides, however targeted deep sequencing was used to determine whether the potential sites were bona fide off-target sites cleaved by Cas9. Using primers that flanked the potential off-target sites, isolated genomic DNA was amplified and the amplicons were sequenced. If the potential off-target site was cleaved by Cas9, indels should be detected at the site. From these follow-on interrogations of the potential off-target sites, no indels were detected at the potential off-target sites for at least three of the guide RNAs: CR000311, CR000312, CR001128. With respect to the gRNAs targeting the BCL11a enhancer, the assay did not identify any potential off-target sites for at least gRNAs CR001137, CR001212, and CR001221. Targeted deep sequencing will be performed to determine whetehr the potential off target sites identified for the other gRNA molecules are bona-fide off target sites cleaved by Cas9.

### Example 7: Evaluation of Cas9 Variants

### Evaluation in CD34+ hematopoietic stem cells

We evaluated 14 purified *Streptococcus pyogenes* Cas9 (SPyCas9) proteins by measuring their efficiency of knocking out the beta-2-microglobulin (B2M) gene in primary human hematopoietic stem cells (HSCs). These proteins were divided into 3 groups: the first group consisted of SPyCas9 variants with improved selectivity (Slaymaker et al. 2015, Science 351: 84 (e 1.0, e 1.1 and K855A); Kleinstiver et al. 2016, Nature 529: 490 (HF)). The second group consisted of wild type SPyCas9 with different numbers and/or positions of the SV40 nuclear localization signal (NLS) and the 6xHistidine (His6) or 8xHistidine (His8) tag with or without a cleavable TEV site, and a SPyCas9 protein with two cysteine substitutions (C80L, C574E), which have been reported to stabilize Cas9 for structural studies (Nishimasu et al. 2014, Cell 156:935). The third group consisted of the same recombinant SPyCas9 produced by different processes (Fig 60). B2M knockout was determined by FACS and next generation sequencing (NGS).

### Methods

### Materials

1. Neon electroporation instrument (Invitrogen, MPK5000)
2. Neon electroporation kit (Invitrogen, MPK1025)
3. crRNA (targeting domain sequence of GGCCACGGAGCGAGACAUCU, complementary to a sequence in the B2M gene, fused to SEQ ID NO: 6607)
4. tracrRNA (SEQ ID NO: 6660)
5. Cas9 storage buffer: 20 mM Tris-Cl, pH 8.0, 200mM KCl, 10mM MgCl₂
6. Bone marrow derived CD34+ HSCs (Lonza, 2M-101C)
7. Cell culture media (Stemcell Technologies, StemSpam SFEM II with StemSpam CC-100)
8. FACS wash buffer: 2% FCS in PBS
9. FACS block buffer: per mL PBS, add 0.5 ug mouse IgG, 150 ug Fc block, 20 uL FCS
10. Chelex suspension: 10% Chelex 100 (bioRad, Cat# 142-1253) in H₂O
11. Anti-B2M antibody: Biolegend, cat# 316304

### Process

Thaw and grow the cells following Lonza's recommendations, add media every 2-3 days. On day 5, pellet the cells at 200 x g for 15 min, wash once with PBS, resuspend the cells with T-buffer from NEON kit at 2×10⁴/uL, put on ice. Dilute Cas 9 protein with Cas9 storage buffer to 5 mg/ml. Reconstitute crRNA and tracrRNA to 100 uM with H₂O. The ribonucleoprotein (RNP) complex is made by mixing 0.8 uL each of CAS 9 protein, crRNA and tracrRNA with 0.6 uL of Cas9 storage buffer, incubate at room temperature for 10 min. Mix 7 uL of HSCs with RNP complex for two minutes and transfer the entire 10 uL into a Neon pipette tip, electroporate at 1700v, 20 ms and 1 pulse. After electroporation, immediately transfer cells into a well of 24-well plate containing 1 ml media pre-calibrated at 37°C, 5% CO₂. Harvest cells 72 hrs post-electroproation for FACS and NGS analysis.

FACS: take 250 uL of the cells from each well of 24-well plate, to wells of 96-well U-bottom plate and pellet the cells. Wash once with 2% FCS (fetal calf serum)-PBS. Add 50 uL FACS block buffer to the cells and incubate on ice for 10 minutes, add 1 uL FITC labeled B2M antibody and incubate for 30 minutes. Wash with 150 uL FACS wash buffer once followed by once more with 200 uL FACS wash buffer once. Cells were resuspended in 200 uL FACS buffer FACS analysis.

NGS sample prep: transfer 250 uL of cell suspension from each well of the 24-well plate to a 1.5 ml Eppendorf tube, add 1 mL PBS and pellet the cells. Add 100 uL of Chelex suspension, incubate at 99°C for 8 minutes and vortex 10 seconds followed by incubating at 99°C for 8 minutes, vortex 10 seconds. Pellet down the resin by centrifuging at 10,000 x g for 3 minutes and the supernatant lysate is used for PCR. Take 4 uL lysate and do PCR reaction with the b2m primers (b2mg67F: CAGACAGCAAACTCACCCAGT, b2mg67R: CTGACGCTTATCGACGCCCT) using Titanium kit (Clonetech, cat# 639208) and follow the manufacturer's instruction. The following PCR conditions are used: 5 minutes at 98°C for 1 cycle; 15 seconds at 95°C, 15 seconds at 62°C, and 1 minute at 72°C for 30 cycles; and finally 3 minutes at 72°C for 1 cycle. The PCR product was used for NGS.

Statistics: The percentage of B2M KO cells by FACS and the percentage of indels by NGS are used to evaluate the CAS 9 cleavage efficiency. The experiment was designed with Cas9 as fixed effect. Each experiment is nested within donors, as nested random effects. Therefore, the mixed linear model was applied for the analysis of FACS and NGS data.

### Results

In order to normalize the experimental and donor variations, we graphed the relative activity of each protein to iProt105026, the original design with two SV40 NLS flanking the wild type SPyCas9 and the His6 tag at the C-terminal of the protein (Fig 34). The statistical analysis shows that compared with the reference Cas9 protein iProt105026, iProt106331, iProt106518, iProtl06520 and iProt106521 are not significantly different in knocking out B2M in HSCs, while the other variants tested (PID426303, iProt106519, iProt106522, iProt106545, iProt106658, iProt106745, iProt106746, iProt106747, iProt106884) are highly significantly different from the reference iProtl05026 in knocking out B2M in HSCs. We found that moving the His6 tag from the C-terminal to N-terminal (iProtl06520) did not affect the activity of the protein (Fig. 34). One NLS was sufficient to maintain activity only when it was placed at the C-terminal of the protein (iProt106521 vs. iProt106522, Fig. 34). Proteins purified from process 1 had consistent higher knockout efficiency than those from processes 2 and 3 (iProt106331 vs. iProtl06545 & PID426303, Fig. 34). In general, the SPyCas9 variants with a reported improved selectivity were not as active as the wild type SPyCas9 (iProtl06745, iProtl06746 and iProt106747, Fig. 34). Interestingly iProtl06884 did not cut the targeting site. This is consistent with the report by Kleinstiver et al that this variant failed to cut up to 20% of the legitimate targeting sites in mammalian cells (Kleinstiver et al. 2016, Nature 529: 490). Finally, the Cas9 variant with two cysteine substitutions (iProt106518) maintained high levels of enzymatic activity (Fig. 34).

Next, editing efficiency of RNPs comprising different Cas9 variants were tested with either modified or unmodified gRNAs targeting the +58 enhancer region. sgRNA molecules comprising the targeting domains of cr00312 and cr1128 were tested in either unmodified (CR00312 = SEQ ID NO: 342; CR001128 = SEQ ID NO: 347) or modified (OMePS CR00312 = SEQ ID NO: 1762; OMePS CR001128 = SEQ ID NO: 1763), and were precomplexed with Cas9 variants shown in Table 35.

**Table 35. Cas9 variants tested for biological function with +58 enhancer region-targeting gRNA molecules.**

| **iProt Code** | **Construct** |
|---|---|
| iProt106518 | NLS-Cas9(C80L/C574E)-NLS-His6 |
| iProtl06331 | NLS-Cas9-NLS-His6 |
| iProt106745 | NLS-Cas9(K855A)-NLS-His6 |
| iProtl06884 | NLS-Cas9(HF)-NLS-His6 |

RNP formed as described above were delivered to HSPC cells as described above, and the cells assessed for % editing (by NGS) and % HbF induction upon erythroid differentiation, as described in these Examples. The results are shown in Figure 42A (% editing) and Figure 42B (HbF induction). The results show that varying the Cas9 component between those variants tested did not alter the gene editing efficiency of both unmodified and modified versions of sgRNA CR00312 and CR001128, nor did the Cas9 variants impact the ability of the erythroid-differentiated gene edited cells to produce HbF.

### Example 8: Ex Vivo Expansion of Gene Edited HSPCs, and Editing Analysis in HSPC subsets

### Expansion of cells prior to gene-editing

Human bone marrow CD34⁺ cells were purchased from either AllCells (Cat#: ABM017F) or Lonza (Cat#: 2M-101C) and expanded for 2 to 3 days using StemSpan SFEM (StemCell Technologies; Cat no. 09650) supplemented with 50 ng/mL of thrombopoietin (Tpo, Peprotech; Cat# 300-18 ), 50 ng/mL of human Flt3 ligand ( Flt-3L, Peprotech; Cat# 300-19), 50ng/mL of human stem cell factor ( SCF, Peprotech; Cat# 300-07), human interleukin-6 (IL-6, Peprotech; Cat# 200-06), 1% L-glutamine; 2% penicillin/streptomycin, and Compound 4 (0.75 µM).

### Electroporation of Cas9 and guide RNA ribonucleoprotein (RNP) complexes into cells

Cas9-guide RNA ribonucleoprotein complexes (RNPs) were prepared immediately prior to electroporation. For formation of RNP using dual guide RNAs, 3 µg of each of crRNA (in 2.24 µL) and tracrRNA (in 1.25 µL) were first denatured at 95°C for 2 min in separate tubes and then cooled to room temperature. For preparation of Cas9 protein, 7.3 µg of CAS9 protein (in 1.21 µL) was mixed with 0.52 µL of 5 × CCE buffer (20 mM HEPES, 100 mM KCL, 5 mM MgCL2, 5% Glycerol and freshly added 1 mM DTT). TracrRNA was first mixed with the Cas9 preparation and incubated at 37°C for 5 min. The CrRNA was then added to TracrRNA/CAS9 complexes and incubated for 5 min at 37°C. The HSPC were collected by centrifugation at 200 × g for 15 min and resuspended in T buffer affiliated with the Neon electroporation kit (Invitrogen; Cat#: MPK1096) at a cell density of 2 × 10⁷/mL. RNP was mixed with 12 µL of cells by pipetting up and down 3 times gently. To prepare single guide RNA (sgRNA) and Cas9 complexes, 2.25 µg sgRNA (in 1.5 µL) was mixed with 2.25 µg Cas9 protein (in 3 µL) and incubated at RT for 5 min. The sgRNA/Cas9 complex was then mixed with 10.5 µL of 2 × 10⁷ /mL cells by pipetting up and down several times gently and incubated at RT for 2 min. The RNP/cell mixture (10 µL) was transferred into a Neon electroporation probe. Electroporation was performed with Neon transfection system (Invitrogen; MPK5000S) using 1700 volts/20 milliseconds and 1 pulse.

### Expansion of cells after gene-editing

After electroporation, the cells were transferred into 0.5 mL pre-warmed StemSpan SFEM medium supplemented with growth factors, cytokines and Compound 4 as described above and cultured at 37°C for 3 days. After a total of 10 days of cell expansion, 3 days before electroporation and 7 days after electroporation, the total cell number increased 2-7 fold relative to the starting cell number (Figure 35).

### Genomic DNA preparation

Genomic DNA was prepared from edited and unedited HSPC at 48 hours post-electroporation. Cells were lysed in 10 mM Tris-HCL, pH 8.0; 0.05% SDS and freshly added Protease K of 25 µg/mL and incubated at 37°C for 1 hour followed by additional incubation at 85°C for 15 min to inactivate protease K.

### T7E1 assay

To determine editing efficiency of HSPCs, T7E1 assay was performed. PCR was performed using Phusion Hot Start II High-Fidelity kit (Thermo Scientific; Cat#: F-549L) with the following cycling condition: 98°C for 30"; 35 cycles of 98°C for 5", 68°C for 20', 72°C for 30"; 72°C for 5 min. The following primers were used: forward primer: 5'-AGCTCCAAACTCTCAAACCACAGGG-3' and reverse primer: 5'-TACAATTTTGGGAGTCCACACGGCA-3'. PCR products were denatured and re-annealed using the following condition: 95°C for 5 min, 95-85°C at -2°C/s, 85-25°C at -0.1°C/s, 4°C hold. After annealing, 1 µL of mismatch-sensitive T7 El nuclease (NEB, Cat# M0302L) was added into 10 µL of PCR product above and further incubated at 37°C for 15 min for digestion of hetero duplexes, and the resulting DNA fragments were analyzed by agarose gel (2%) electrophoresis. Editing efficiency was quantified using ImageJ software (http://rsb.info.nih.gov/ij/).

### PCR Preparation for next generation sequencing (NGS)

To determine editing efficiency more precisely and patterns of insertions and deletions (indels), the PCR products were subjected to next generation sequencing (NGS). The PCR were performed in duplicate using Titanium Taq PCR kit (Clontech Laboratories; Cat#: 639210) with the following cycling condition: 98°C for 5 min; 30 cycles of 95°C for 15 seconds, 68°C for 15 seconds, 72°C 1 min; 72°C for 7 min. The following primers were used: forward primer: 5'-AGCTCCAAACTCTCAAACCACAGGG-3' and reverse primer: 5'-TACAATTTTGGGAGTCCACACGGCA-3'. The PCR products were analyzed by 2% agarose gel electrophoresis and submitted for deep sequencing.

**Flow cytometry analysis of editing efficiency in hematopoietic stem and progenitor subpopulations.**

Cells were subjected to flow cytometry to characterize editing efficiency in stem and progenitor cell (HSPC) subpopulations. The frequencies of HSPC subsets, including CD34+ cells, CD34+CD90+ cells, CD34+CD90+CD45RA- cells, and CD34+CD90+CD45RA-CD49f+ cells, prior to (48 hours in culture) and after genome editing (10 days in culture; 3 days after genome editing) was determined from sampling aliquots of cell cultures (Figure 36, Figure 37, Figure 38). Cells were incubated with anti-CD34 (BD Biosciences, Cat# 555824), anti-CD38 (BD Biosciences, Cat# 560677), anti-CD90 (BD Biosciences, Cat# 555596), anti-CD45RA (BD Biosciences, Cat# 563963), anti-CD49f (BD Biosciences, Cat# 562582) in modified FACS buffer, containing PBS supplemented with 0.5% BSA, 2mM EDTA pH7.4, at 4°C, in dark for 30 min. Cell viability was determined by 7AAD. Cells were washed with modified FACS buffer and multicolor FACS analysis was performed on a Fortessa (BD Biosciences). Flow cytometry results were analyzed using Flowjo software. Genome edited cells grown in the presence of Compound 4 (alone) exhibited detectible levels of all HSPC subsets assessed, including CD34+, CD34+CD90+, CD34+CD90+CD45RA-, and CD34+CD90+CD45RA-CD49f+ subsets, indicating that long-term HSC populations persist in cell culture in the presence of Compound 4 (alone) after genome editing (Figure 38).

Four hours after gene editing, aliquots of cell cultures were sampled and cells were sorted into HSPC subsets (CD34+, CD34+CD38+, CD34+CD38-, CD34+CD38-CD45RA-, CD34+CD38-CD45RA-CD90+CD49f-, CD34+CD38-CD45RA-CD90-CD49f-, and CD34+CD38-CD45RA-CD90-CD49f+ cells) and subjected to NGS to measure the editing efficiency (Table 28). It is important to note that all hematopoietic subsets, including the subsets enriched in long-term HSCs, displayed similarly high gene editing efficiencies (>95%). This high gene editing efficiency in long-term HSCs has significant therapeutic impact as these cells are capable of engrafting patients in the long-term and sustain lifelong hematopoietic multi-lineage regeneration.

**Table 28. Editing efficiency in each HSPC subset measured by NGS.**

| **gRNA** | **HSPC subsets** | **Avg(% Indels)** |
|---|---|---|
| Dg CR00312 | Total (singlets) | 97.98% |
| | CD34+CD38-CD45RA- | 98.66% |
| | CD34+CD38-CD45RA-CD90+CD49f- | 98.19% |
| | CD34+CD38-CD45RA-CD90-CD49f- | 98.70% |
| | CD34+CD38-CD45RA-CD90-CD49f+ | 98.81% |
| Dg CR001128 | Total (singlets) | 97.62% |
| | CD34+CD38-CD45RA- | 95.93% |
| | CD34+CD38-CD45RA-CD90-CD49f- | 98.00% |
| | CD34+CD38-CD45RA-CD90-CD49f+ | 99.01% |

### Example 9 Evaluation of Potential Off-Target Editing

### HSPC culture and CRISPR/Cas9 genome editing

Methods for RNP generation and cell manipulation are as in Example 4, with the following exceptions. Human CD34⁺ cell culture. Human CD34+ cells were either isolated from G-CSF mobilized peripheral blood from adult donors (AllCells, Cat. No. mPB025-Reg.E) using immunoselection (Miltenyi) according to the manufacturer's instructions or derived from bone marrow (Hemacare Corporation, Cat. No. BM34C-3). CD34+ cells were thawed and expanded for 2 days or 5 days prior to RNP delivery. After RNP delivery, cells were either cultured for an additional 13 days in expansion medium or cultured in erythroid differentiation medium for 7 or 11 days as follows. During days 0-7 of culture, EDM was further supplemented with 1 µM hydrocortisone (Sigma H8672), 100 ng/mL human SCF (Life Technologies, Cat. #PHC2113), and 5 ng/mL human IL-3 (Peprotech #10779-598). During days 7-11 of culture, EDM was supplemented with 100 ng/mL of human SCF only. At the conclusion of the culture period, cells were harvested for genomic DNA preparation and analysis.

Assembly of Cas9 and guide RNA ribonucleoprotein (RNP) complexes, preparation of HSPC, and electroporation of RNP into HSPC. The HSPC collected by centrifugation were resuspended in P3 Primary Cell Solution (Lonza cat. No. PBP3-00675). The RNP was mixed with 20 µL of cells by pipetting up and down and incubated at RT for 2 min. The RNP/cell mixture (20 µL) was electroporated using code CM-137 on the 4D-Nucleofector (Lonza). dgRNAs comprising a crRNA having the sequence NNNNNNNNNNNNNNNNNNNNGUUUUAGAGCUAUGCUGUUUUG (where N indicates a residue of the indicated targeting domain) and a tracr having the sequence of SEQ ID NO: 6660 were used.

### Genomic DNA extraction

Genomic DNA was isolated from RNP treated and untreated HSPC using the DNeasy Blood & Tissue Kit (Qiagen, Cat # 69506) following the manufacturer's recommendations.

### In silico identification of potential gRNA off-target loci

Potential off-target loci for the BCL11A +58 region gRNAs CR00309, CR00311, CR00312, CR00316, CR001125, CR001126, CR001127, and CR001128, and the HPFH region gRNAs CR001028, CR001030, CR001137, CR001221, CR003035, and CR003085 were identified as follows. For each gRNA, the 20 nucleotide gRNA protospacer sequence was aligned to the human genome reference sequence (build GRCh38) using the BFAST sequence aligner (version 0.6.4f, Homer et al, PLoS One, 2009, 4(11), e7767, PMID: 19907642) using standard parameters allowing up to 5 nucleotide mismatches. Loci identified were filtered to only contain sites that are 5' adjacent to the Cas9 canonical 5'-NGG-3' PAM sequence (i.e. 5'-off-target locus-PAM-3'). Using the BEDTools script (version 2.11.2, Quinlan and Hall, Bioinformatics, 2010 26(6):841-2, PMID: 20110278) sites with 5 nucleotide mismatches were further filtered against RefSeq gene annotations (Pruitt et al, Nucleic Acids Res., 2014 42(Database issue):D756-63, PMID: 24259432) to only contain loci annotated as exons. Counts of the potential off-target loci identified for the BCL11A +58 region and the HPFH region gRNAs are shown in Tables 1 and 2 respectively.

**Table 29. Counts of in silico off-target loci identified for the BCL11A +58 region gRNAs CR00309, CR00311, CR00312, CR00316, CR001125, CR001126, CR001127, and CR001128 with 0, 1, 2, 3 and 4 nucleotide mismatches and 5 nucleotide mismatches within RefSeq exons are shown.**

| **gRNA targeting domain ID** | **Number of off-targets with N mismatches** | | | | | | |
|---|---|---|---|---|---|---|---|
| | **0** | **1** | **2** | **3** | **4** | **5 RefSeq exons** | **Total sites** |
| CR00309 | 0 | 0 | 2 | 9 | 77 | 52 | 140 |
| CR00311 | 0 | 0 | 3 | 13 | 213 | 38 | 267 |
| CR00312 | 0 | 0 | 0 | 6 | 126 | 33 | 165 |
| CR00316 | 0 | 0 | 0 | 22 | 234 | 90 | 346 |
| CR001125 | 0 | 0 | 1 | 16 | 221 | 83 | 321 |
| CR001126 | 0 | 0 | 2 | 26 | 235 | 86 | 349 |
| CR001127 | 0 | 0 | 1 | 35 | 331 | 157 | 524 |
| CR001128 | 0 | 0 | 0 | 9 | 156 | 42 | 207 |

**Table 30. Counts of in silico off-target loci identified for the HPFH region gRNAs CR001028, CR001030, CR001137, CR001221, CR003035, and CR003085 with 0, 1, 2, 3 and 4 nucleotide mismatches and 5 nucleotide mismatches within RefSeq exons are shown.**

| **gRNA targeting domain ID** | **Number of off-targets with N mismatches** | | | | | | |
|---|---|---|---|---|---|---|---|
| | **0** | **1** | **2** | **3** | **4** | **5 RefSeq exons** | **Total sites** |
| CR001028 | 0 | 0 | 1 | 11 | 154 | 67 | 233 |
| CR001030 | 0 | 0 | 0 | 14 | 125 | 85 | 224 |
| CR001137 | 0 | 0 | 1 | 6 | 85 | 21 | 113 |
| CR001221 | 0 | 0 | 2 | 17 | 166 | 44 | 229 |
| CR003035 | 0 | 0 | 4 | 10 | 158 | 77 | 249 |
| CR003085 | 0 | 0 | 0 | 5 | 75 | 16 | 96 |

### PCR primer design for targeted amplification of potential off-target sites

PCR amplicons targeting potential off-target loci (and the on-target locus) identified for the BCL11A +58 region gRNAs (CR00309, CR00311, CR00312, CR00316, CR001125, CR001126, CR001127, and CR001128) and the HPFH region gRNAs (CR001028, CR001030, CR001137, CR001221, CR003035, and CR003085) were design using Primer3 (version 2.3.6, Untergasser et al, Nucleic Acids Res., 2012 40(15):e115, PMID: 22730293) using default parameters aiming for an amplicon size range of approximately 160-300 base pairs in length with the gRNA protospacer sequence located in the center of the amplicon. For gRNA CR001127 PCR primers were only designed for sites with 0-4 nucleotide mismatches, no primers were designed for sites with 5 nucleotide mismatches within RefSeq exons. Resulting PCR primer pairs and amplicon sequences were checked for uniqueness by BLAST searching (version 2.2.19, Altschul et al, J Mol Biol., 1990, 215(3):403-10, PMID: 2231712) sequences against the human genome reference sequence (build GRCh38). Primer pairs resulting in more than one amplicon sequence were discarded and redesigned. Tables 3 and 5 show counts of successful PCR primer pairs designed for the BCL11A +58 region and the HPFH region gRNAs respectively.

### Illumina sequencing library preparation, quantification and sequencing

Genomic DNA from RNP treated (2 replicates per gRNA) and untreated (1 replicate per gRNA) HSPC samples was quantified using the Quant-iT PicoGreen dsDNA kit (Thermo Fisher, Cat # P7581) using manufacture's recommendations. Illumina sequencing libraries targeting individual off-target loci (and the on-target locus) were generated for each sample using two sequential PCR reactions. The first PCR amplified the target locus using target specific PCR primers (designed above) that were tailed with universal Illumina sequencing compatible sequences. The second PCR added additional Illumina sequencing compatible sequences to the first PCR amplicon, including sample barcodes to enable multiplexing during sequencing. PCR 1 was performed in a final volume of 10 µL with each reaction containing 3-6 ng of gDNA (equivalent to approximately 500-1000 cells), PCR 1 primer pairs (Integrated DNA Technologies) at a final concentration of 0.25 µM and 1x final concentration of Q5 Hot Start Master Mix (New England BioLabs, Cat # 102500-140). PCR 1 left primers were 5' tailed (i.e. 5'-tail-target specific left primer-3') with sequence 5'-TCGTCGGCAGCGTCAGATGTGTATAAGAGACAG-3' and right primers were 5' tailed (i.e. 5'-tail- target specific right primer-3') with sequence 5'-GTCTCGTGGGCTCGGAGATGTGTATAAGAGACAG-3'. PCR 1 was performed on a thermocycler using the following cycling conditions: 1 cycle of 98°C for 1 min; 25 cycles of 98°C for 10 sec, 63°C for 20 sec, and 72°C for 30 sec; 1 cycle at 72°C for 2 min. PCR 1 was then diluted 1 in 100 using nuclease free water (Ambion, Cat # AM9932) and used as input into PCR 2. PCR 2 was performed in a final volume of 10 µL with each reaction containing 2 µL of diluted PCR 1 product, PCR 2 primer pairs (Integrated DNA Technologies) at a final concentration of 0.5 µM and 1x final concentration of Q5 Hot Start Master Mix (New England BioLabs, Cat # 102500-140). PCR 2 left primer sequence used was 5'-AATGATACGGCGACCACCGAGATCTACACNNNNNNNNTCGTCGGCAGCGTC-3' and PCR 2 right primer sequence used was 5'-CAAGCAGAAGACGGCATACGAGATNNNNNNNNGTCTCGTGGGCTCGG-3' where the NNNNNNNN denote an 8 nucleotide barcode sequence used for sample multiplexing as part of the standard Illumina sequencing process. PCR 2 was performed on a thermocycler using the following cycling conditions: 1 cycle of 72°C for 3 min; 1 cycle of 98°C for 2 min; 15 cycles of 98°C for 10 sec, 63°C for 30 sec, and 72°C for 2 min. PCR 2 amplicons, now viable Illumina sequencing libraries, were cleaned up using Agencourt AMPure XP beads (Beckman Coulter, Cat # A63882) following the manufacture's recommendations. The cleaned Illumina sequencing libraries were then quantified using standard qPCR quantification methods using Power SYBR Green PCR master mix (Life Technologies, Cat # 4367660) and primers specific to the Illumina sequencing library ends (forward primer sequence 5'- CAAGCAGAAGACGGCATACGA-3' and reverse primer sequence 5'-AATGATACGGCGACCACCGAGA-3'). Illumina sequencing libraries were then pooled equimolar and subjected to Illumina sequencing on a MiSeq instrument (Illumina, Cat # SY-410-1003) with 300 base paired-end reads using a MiSeq Reagent Kit v3 (Illumina, Cat # MS-102-3003) following the manufacture's recommendations. A minimum of 1000-fold sequence coverage was generated for each locus for at least one treated replicate. PCR, cleanup, pooling and sequencing of treated and untreated samples were performed separately to avoid any possibility of cross contamination between treated and untreated samples or PCR amplicons generated therefrom.

### Illumina sequencing data QC and variant analysis

Using default parameters, the Illumina MiSeq analysis software (MiSeq reporter, version 2.6.2, Illumina) was used to generate amplicon specific FASTQ sequencing data files (Cock et al, Nucleic Acids Res. 2010, 38(6):1767-71, PMID: 20015970). FASTQ files were then processed through an internally developed variant analysis pipeline consisting of a series of public domain software packages joined together using a standard Perl script wrapper. The workflow used was divided into five stages.

**Stage1, PCR primer and on- and off-target sequence QC:** For both on- and off- target sites the 20 nucleotide gRNA protospacer sequence plus PAM sequence and target specific PCR primer sequences (left and right without the additional Illumina sequences) were aligned to the human genome reference sequence (build GRCh38) using a BLAST search (version 2.2.29+, Altschul et al, J Mol Biol., 1990, 215(3):403-10, PMID: 2231712). On- and off-target sites with multiple genomic locations were flagged.

**Stage 2, sequencer file decompression:** Illumina sequencer generated FASTQ.GZ files were decompressed to FASTQ files using the gzip script (version 1.3.12) and number of reads per file was calculated. Files with no reads were excluded from further analysis.

**Stage 3, sequence read alignment and quality trimming:** Sequencing reads in FASTQ files were aligned to the human genome reference sequence (build GRCh38) using the BWA-MEM aligner (version 0.7.4-r385, Li and Durbin, Bioinformatics, 2009, 25(14):1754-60, PMID: 19451168) using 'hard-clipping' to trim 3' ends of reads of Illumina sequences and low quality bases. Resulting aligned reads, in the BAM file format (Li et al, Bioinformatics, 2009 25(16):2078-9, PMID: 19505943), were converted to FASTQ files using the SAMtools script (version 0.1.19-44428cd, Li et al, Bioinformatics, 2009 25(16):2078-9, PMID: 19505943). FASTQ files were then aligned again to the human genome reference sequence (build GRCh38) using the BWA-MEM aligner, this time without 'hard-clipping'.

**Stage 4, variant (SNP and INDEL) analysis:** BAM files of aligned reads were processed using the VarDict variant caller (version 1.0 'Cas9 aware' modified by developer ZhangWu Lia, Lai et al, Nucleic Acids Res., 2016, 44(11):e108, PMID: 27060149) with allele frequency detection limit set at >=0.0001 to identify variants (SNPs and INDELs). The Cas9 aware VarDict caller is based on a public domain package but able to move ambiguous variant calls, generated due to repetitive sequences in the alignment region of the variant events, toward the potential Cas9 nuclease cut site in the gRNA protospacer sequence located 3 bases 5' of the PAM sequence. The SAMtools script was used to calculate read coverage per sample amplicon to determine whether the on- and off-target sites were covered at >1000-fold sequence coverage. Sites with <1000-fold sequence coverage were flagged.

**Stage 5, dbSNP filtering and treated/untreated differential analysis:** Variants identified were filtered for known variants (SNPs and INDELs) found in dbSNP (build 142, Shery et al, Nucleic Acids Res. 2001, 29(1):308-11, PMID: 11125122). Variants in the treated samples were further filtered to exclude: 1) variants identified in the untreated control samples; 2) variants with a VarDict strand bias of 2:1 (where forward and reverse read counts supporting the reference sequence are balanced but imbalanced for the non-reference variant call); 3) variants located outside a 100bp window around the potential Cas9 cut site; 4) single nucleotide variants within a 100bp window around the potential Cas9 cut site. Finally only sites with a combined INDEL frequency of >2% (editing in more than approximately 10-20 cell) in a 100bp window of the potential Cas9 cut site were considered. Potential active editing sites were further examined at the read alignment level using the Integrative Genome Viewer (IGV version 2.3, Robinson et al, Nat Biotechnol. 2011, 9(1):24-6, PMID: 21221095) that allows for visual inspection of read alignments to the genome reference sequence.

### On- and off-target analysis results

On-target sites for the BCL11A +58 region and the HPFH region gRNAs showed robust editing at the intended Cas9 cut site in treated samples with an INDEL frequency greater than 88% for all gRNAs. Tables 3 and 5 show number of off-target sites characterized in at least one biological replicate. Uncharacterized sites failed PCR primer design or PCR amplification and remain under investigation.

### BCL11A +58 region in silico targeted analysis results

**gRNA CR00309:** The on-target site for gRNA CR00309 showed robust editing at the intended Cas9 cut site with an average INDEL frequency of approximately 92%. Table 31 shows the number of off-target sites characterized. Uncharacterized sites failed in PCR primer design or PCR amplification and remain under investigation. Characterization of potential off-target sites identified four off-target sites in the 100bp window around the proposed Cas9 cut site in both treated replicates. Site 1 has an average INDEL frequency approximately 18%, has 3 mismatches relative to the on-target gRNA protospacer sequence (5'-CtCaCCtCCACCCTAATCAG-PAM-3', PAM = AGG) and is located in intron 1 of the Anoctamin 5 gene (ANO5), on chromosome 11 at base pair position 22,195,800-22,195,819, approximately 2.3kb away from exon 1. Site 2 has an average INDEL frequency of approximately 18%, has 2 mismatches relative to the on-target gRNA protospacer sequence (5'-CACGCCCaCACCtTAATCAG -PAM-3', PAM = GGG) and is located in an intergenic region of chromosome 12 at base pair position 3,311,901-3,311,926. Site 3 has an average INDEL frequency approximately 80%, has 2 mismatches relative to the on-target gRNA protospacer sequence (5'-CACGaCCCaACCCTAATCAG-PAM-3', PAM = AGG) and is located in a GenBank (Clark et al., Nucleic Acids Res. 2016 Jan 4; 44(Database issue): D67-D72, PMID: 26590407) transcript (BC012501 not annotated in RefSeq) on chromosome 1 at base pair position 236,065,415-236,065,434. Site 4 has an average INDEL frequency of approximately 2%, has 4 mismatches relative to the on-target gRNA protospacer sequence (5'-CtaGCCCCtACCCTAATaAG-3', PAM = TGG) and is located in intron 1 of a GenBank annotated transcript called polyhomeotic 2 protein (not annotated in RefSeq) on chromosome 1 at base pair position 33,412,659-33,412,678.

**gRNA CR00311:** The on-target site for gRNA CR00311 showed robust editing at the intended Cas9 cut site with an average INDEL frequency of approximately 95%. Table 31 shows the number of off-target sites successfully characterized. Uncharacterized sites failed in PCR primer design or PCR amplification and remain under investigation. To date no significant off-target activity was observed at sites examined.

**gRNA CR000312:** The on-target site for gRNA CR000312 showed robust editing at the intended Cas9 cut site with an average INDEL frequency of approximately 98%. Table 31 shows the number of off-target sites successfully characterized. Uncharacterized sites failed in PCR primer design or PCR amplification and remain under investigation. To date no significant off-target activity was observed at sites examined.

**gRNA CR000316:** The on-target site for gRNA CR000316 showed robust editing at the intended Cas9 cut site in with an average INDEL frequency of approximately 91%, however the majority of off-target sites remain to be characterized.

**gRNA CR001125:** The on-target site for gRNA CR0001125 showed robust editing at the intended Cas9 cut site with an average INDEL frequency of approximately 91%. Table 31 shows the number of off-target sites successfully characterized. Uncharacterized sites failed in PCR primer design or PCR amplification and remain under investigation. To date no significant off-target activity was observed at sites examined.

**gRNA CR001126:** The on-target site for gRNA CR0001126 showed robust editing at the intended Cas9 cut site with an average INDEL frequency of approximately 92%. Table 31 shows the number of off-target sites successfully characterized. Uncharacterized sites failed in PCR primer design or PCR amplification and remain under investigation. Characterization of potential off-target sites identified two off-target sites to date in the 100bp window around the proposed Cas9 cut site in both treated replicates. The first site has an average INDEL frequency approximately 2%, has 2 mismatches relative to the on-target gRNA protospacer sequence (5'-TTTATaACAGGCTCCAGaAA -PAM-3', PAM = TGG) and is located in an intergenic region on chromosome 4 at base pair position 35,881,815-35,881,834, approximately 9.5kb away from the nearest GenBank transcript (not annotated in RefSeq). The second site has an average INDEL frequency of approximately 1.7%, has 4 mismatches relative to the on-target gRNA protospacer sequence (5' caTATCACAGGCcCCAGGAg -PAM-3', PAM = GGG) and is located in intron 6 of the Ataxin 1 gene (ATXN1), on chromosome 6 at base pair position 16,519,907-16,519,926, approximately 2.7kb away from exon 6.

**gRNA CR001127:** The on-target site for gRNA CR0001127 showed robust editing at the intended Cas9 cut site with an average INDEL frequency of approximately 97%. Table 31 shows the number of off-target sites successfully characterized. Uncharacterized sites failed in PCR primer design or PCR amplification and remain under investigation. To date no significant off-target activity was observed at sites examined.

**gRNA CR001128:** The on-target site for gRNA CR0001128 showed robust editing at the intended Cas9 cut site with an average INDEL frequency of approximately 94%. Table 31 shows the number of off-target sites successfully characterized. Uncharacterized sites failed in PCR primer design or PCR amplification and remain under investigation. To date no significant off-target activity was observed at sites examined.

Manual inspection of all actively edited BCL11 +58 region off-target sites identified showed typical INDEL patterns surrounding the proposed Cas9 cut site typical of Cas9 mediated double stranded break non homologous end joining DNA repair. It is unclear whether editing at the sites identified has any detrimental effect on gene expression or cell viability, further analysis is required.

**Table 31. Counts of in silico off-target sites identified, sites successfully characterized in at least one treated replicate and sites that show editing for the BCL11A +58 region gRNAs CR00309, CR00311, CR00312, CR00316, CR001125, CR001126, CR001127, and CR001128 are shown. ^{∗}only sites with 0-4 nucleotide mismatches were examined.**

| **gRNA name** | **Total number of *in silico* off-target sites** | **Number of *in silico* off-target sites characterized** | **Number of active *in silico* off-target sites identified** |
|---|---|---|---|
| CR00309 | 141 | 140 | 4 |
| CR00311 | 267 | 245 | 0 |
| CR00312 | 165 | 163 | 0 |
| CR00316 | 346 | TBD | TBD |
| CR001125 | 321 | 319 | 0 |
| CR001126 | 349 | 348 | 2 |
| CR001127^{∗} | 367 | 365 | 0 |
| CR001128 | 207 | 194 | 0 |

### BCL11A +58 region GUIDE-Seq hit validation results

Potential off-target hits identified by GUIDE-seq (Tsai et al, Nat Biotechnol. 2015, 33(2):187-97, PMID: 25513782) were characterized in RNP treated and untreated HSPC using targeted sequencing methods described above for *in silico* defined sites. GUIDE-seq potential hits were identified for gRNAs CR00312, CR00316, CR001125, CR001126, CR001127 and CR001128. No potential hits were identified for gRNA CR00311 and CR001128. When the potential hits were interrogated in HSPCs using targeted sequencing, none of the potential hits validated in treated HSPC for gRNA CR00312, CR001125 and CR001127. One potential hit validated for gRNA CR001126, the same site that was identified by the *in silico* directed method in intron 6 of ATXN1 (chromosome 6:16,519,907-16,519,926 base pairs).Validation of potential hits for gRNAs CR00309 and CR00316 is still in progress.

### HPFH region in silico targeted analysis results

**gRNA CR001028**: The on-target site for gRNA CR0001028 showed robust editing at the intended Cas9 cut site with an average INDEL frequency of approximately 91%. Table 32 shows the number of off-target sites successfully characterized. Uncharacterized sites failed in PCR primer design or PCR amplification and remain under investigation. Characterization of potential off-target sites identified two off-target sites to date in the 100bp window around the proposed Cas9 cut site in both treated replicates. Site 1 has an average INDEL frequency approximately 2.8%, has 2 mismatches relative to the on-target gRNA protospacer sequence (5'- TGgGGTGGGGAGATATGaAG -PAM-3', PAM = AGG) and is located in intron 2 of a non-coding RNA (LF330410, not annotated in RefSeq) located on chromosome 4 at base pair position 58,169,308-58,169,327. Site 2 has an average INDEL frequency approximately 3.5%, has 3 mismatches relative to the on-target gRNA protospacer sequence (5'- gGaGGTGGGGAGAgATGTAG-PAM-3', PAM = AGG) and is located in intron 1 of the Butyrophilin subfamily 3 member A2 gene (BTN3A2) located on chromosome 6 at base pair position 26,366,733-26,366,752, approximately 1.3kb from exon 2.

**gRNA CR001030**: The on-target site for gRNA CR0001030 showed robust editing at the intended Cas9 cut site with an average INDEL frequency of approximately 89%. Table 32 shows the number of off-target sites successfully characterized. Uncharacterized sites failed in PCR primer design or PCR amplification and remain under investigation. Characterization of potential off-target sites identified one off-target sites to date with average INDEL frequency of 57% in the 100bp window around the proposed Cas9 cut site in both treated replicates. The site has 3 mismatches relative to the on-target gRNA protospacer sequence (5'-caTGCgGAAAGAGATGCGGT-PAM-3', PAM = TGG) and is located in exon 4 of the Pyruvate Dehydrogenase Kinase 3 gene (PDK3) located on the X chromosome at base pair position 24,503,476-24,503,495.

**gRNA CR001137**: The on-target site for gRNA CR0001037 showed robust editing at the intended Cas9 cut site with an average INDEL frequency of approximately 84%. Table 32 shows the number of off-target sites successfully characterized. Uncharacterized sites failed in PCR primer design or PCR amplification and remain under investigation. To date no significant off-target activity was observed at sites examined.

**gRNA CR001221:** The on-target site for gRNA CR0001221 showed robust editing at the intended Cas9 cut site with an average INDEL frequency of approximately 95%. Table 32 shows the number of off-target sites successfully characterized. Uncharacterized sites failed in PCR primer design or PCR amplification and remain under investigation. To date no significant off-target activity was observed at sites examined.

**gRNA CR003035:** The on-target site for gRNA CR0003035 showed robust editing at the intended Cas9 cut site with an average INDEL frequency of approximately 89%. Table 32 shows the number of off-target sites successfully characterized. Uncharacterized sites failed in PCR primer design or PCR amplification and remain under investigation. Characterization of potential off-target sites identified two off-target sites to date in the 100bp window around the proposed Cas9 cut site in both treated replicates. Site 1 has an average INDEL frequency of approximately 20%, has 2 mismatches relative to the on-target gRNA protospacer sequence (5'- AGGtACCTCAaACTCAGCAT-PAM-3', PAM = AGG) and is located in an intergenic region on chromosome 2 at base pair position 66,281,781-66,281,800 and is approximately 7.1kb from the nearest transcript (JD432564, not annotated in RefSeq). Site 2 has an average INDEL frequency approximately 2.5%, has 3 mismatches relative to the on-target gRNA protospacer sequence (5'-AGagACCcCAGACTCAGCAT-PAM-3', PAM = AGG) and is located in an intergenic region on chromosome 10 at base pair position 42,997,289-42,997,308, and is approximately 0.3kb from MicroRNA 5100 (MIR5100).

**gRNA CR003085:** The on-target site for gRNA CR0003038 showed robust editing at the intended Cas9 cut site with an average INDEL frequency of approximately 94%. Table 32 shows the number of off-target sites successfully characterized. Uncharacterized sites failed in PCR primer design or PCR amplification and remain under investigation. Characterization of potential off-target sites identified one off-target sites to date with average INDEL frequency of approximately 2% in the 100bp window around the proposed Cas9 cut site in both treated replicates. The site has 3 mismatches relative to the on-target gRNA protospacer sequence (5'-ATGGTATGaGAaaTATACTA-PAM-3', PAM = TGG) and is located in intron 2 of the Solute Carrier Family 25 Member 12 gene (SLC25A12) located on chromosome 2 at base pair position 171,872,630-171,872,649, and is approximately 3.8kb from exon 3.

Manual inspection of all actively edited HPFH region off-target sites identified showed typical INDEL patterns surrounding the proposed Cas9 cut site typical of Cas9 mediated double stranded break non homologous end joining DNA repair. It is unclear whether editing at the sites identified has any detrimental effect on gene expression or cell viability, further analysis is required.

**Table 32. Counts of potential in silico off-target sites identified, sites successfully characterized in at least one treated replicate and sites that show editing for the HPFH region gRNAs CR001028, CR001030, CR001137, CR001221, CR003035 and CR003085 are shown.**

| **gRNA name** | **Total number of *in silico* off-target sites** | **Number of *in silico* off-target sites characterized** | **Number of active *in silico* off-target sites identified** |
|---|---|---|---|
| CR001028 | 233 | 217 | 2 |
| CR001030 | 224 | 219 | 1 |
| CR001137 | 113 | 109 | 0 |
| CR001221 | 229 | 224 | 0 |
| CR003035 | 249 | 243 | 2 |
| CR003085 | 96 | 95 | 1 |

### HPFH region GUIDE-Seq hit validation results

Potential off-target hits identified using GUIDE-seq (Tsai et al, Nat Biotechnol. 2015, 33(2):187-97, PMID: 25513782) were interrogated in RNP treated and untreated HSPC using the same methods described for *in silico* sites. GUIDE-seq potential off-target hits were identified for gRNA CR001028, CR001030, CR003035 and CR003085 (to date). No hits were identified for gRNAs CR001137 and CR001221 to date. One hit for gRNA CR001028 validated in HSPC, the same site that was identified by the *in silico* directed method on chromosome 4 at base pair position 58,169,308-58,169,327. One hit validated for gRNA CR001030 in HSPC, the same site that was identified by the *in silico* directed method in exon 4 of the PDK3 gene. One hit validated for gRNA CR003035 in HSPC, the same site that was identified by the *in silico* directed method in the intergenic region of chromosome 2 at base pair position 66,281,781-66,281,800. One hit validated for gRNA CR003085 in HSPC, the same site as was identified by the *in silico* directed method on chromosome 2 at base pair position 171,872,630-171,872,649.

### On-target analysis for gRNA editing efficiency screening

PCR amplicons targeting the gRNA on-target site were cleaned up using Agencourt AMPure XP beads (Beckman Coulter, Cat # A63882) following the manufacture's recommendations. Samples were quantified using the Quant-iT PicoGreen dsDNA kit (Thermo Fisher, Cat # P7581) using manufacture's recommendations. Amplicons were transformed into Illumina sequencing libraries using the Nextera DNA Library Preparation Kit (Illumina, Cat# FC-121-1031) following the manufacture's recommendations. Resulting sequencing libraries were AMPure bead cleaned up, qPCR quantified, pooled and sequenced with an Illumina MiSeq instrument using 150 base (Illumina, Cat# MS-102-2002) or 250 base (Illumina, Cat# MS-102-2003) paired-end reads as described in the off-target analysis section. For each library a minimum of a 1000-fold sequencing coverage was generated and variants were called using a series of in house scripts. In short, sequencing reads spanning an 80-100 base pair window centered on the gRNA sequence were identified using a string based sequence search, compared to the amplicon sequence, binned by sequence differences and variant frequencies were calculated. Additionally, sequencing reads were aligned to the amplicon sequence using the Illumina MiSeq reporter software (Illumina version 2.6.1) and resulting read alignments were examined using the Integrative Genome Viewer (IGV version 2.3, Robinson et al, Nat Biotechnol. 2011, 9(1):24-6, PMID: 21221095).

### Example 10: Evaluation of RNP Concentration On Gene Editing and HbF Induction

### RNP dilution assay to determine optimal RNP concentration for gene editing

Gene editing was performed using a serial dilution of Cas9 (iProt: 106331) and guide ribonucleotide complexes (RNP) concentrations (Table 33). Various gRNA used, their formats, and modification applied in this assay are listed in Table 34.

**Table 33: RNP dilution used to investigate RNP concentration effect on gene editing and HbF induction.**

| **gRNA Format** | **RNP Components** | **RNP Conc RNP-1** | **RNP Conc RNP-2** | **RNP Conc RNP-3** | **RNP Conc RNP-4** | **RNP Conc RNP-5** |
|---|---|---|---|---|---|---|
| **Single gRNA** | **crRNA(ug)** | 3 | 1.5 | 0.75 | 0.37 | 0.18 |
| | **Cas9(ug)** | 3 | 1.5 | 0.75 | 0.37 | 0.18 |
| | **RNP(uM)** | 1.94 | 0.97 | 0.48 | 0.24 | 0.12 |
| **Dual gRNA** | **crRNA(ug)** | 3.86 | 1.93 | 0.97 | 0.48 | 0.24 |
| | **tracrRNA(ug)** | 3.86 | 1.93 | 0.97 | 0.48 | 0.24 |
| | **Cas9(ug)** | 3.86 | 1.93 | 0.97 | 0.48 | 0.24 |
| | **RNP(uM)** | 5.89 | 2.94 | 1.47 | 0.74 | 0.37 |

**Table 34: List of gRNAs, in single or dual guide format, with or without chemical modification used in the RNP dilution assay. "OMePS" modification, with respect to dgRNA format, refers to modified crRNA having the following structure: mN^{∗}mN^{∗}mN^{∗}NNNNNNNNNNNNNNNNNGUUUUAGAGCUAUGCUGUU^{∗}mU^{∗}mU^{∗}mG paired with unmodified tracr (i.e., tracrRNA) of SEQ ID NO: 6660; and with respect to sgRNA, refers to a gRNA having the following structure: mN^{∗}mN^{∗}mN^{∗}NNNNNNNNNNNNNNNNNGUUUUAGAGCUAGAAAUAGCAAGUUAAAAUA AGGCUAGUCCGUUAUCAACUUGAAAAAGUGGCACCGAGUCGGUGCmU^{∗}mU^{∗}mU^{∗}U, where N = a nucleotide of the targeting domain, mN refers to a 2'-OMe-modified nucelotide, and ^{∗} refers to a phosphorothioate bond. "Unmodified" refers to a gRNA having no RNA modifications: dgRNA consist of NNNNNNNNNNNNNNNNNNNNGUUUUAGAGCUAUGCUGUUUUG paired with SEQ ID NO: 6660; sgRNA refer to NNNNNNNNNNNNNNNNNNNNGUUUUAGAGCUAGAAAUAGCAAGUUAAAAUAAGGCUA GUCCGUUAUCAACUUGAAAAAGUGGCACCGAGUCGGUGCUUUU, where N = the nucleotides of the indicated targeting domain.**

| **gRNA Format** | **Sample ID** | **gRNA Targeting Domain ID** | **gRNA Modification** |
|---|---|---|---|
| **Dual gRNA** | 1 | CR00312 | Unmodified |
| | 2 | CR001128 | Unmodified |
| | 3 | CR00312 | OMePS |
| | 4 | CR001128 | OMePS |
| **Single gRNA** | 5 | CR00312 | Unmodified |
| | 6 | CR001128 | Unmodified |
| | 7 | CR00312 | OMePS |
| | 8 | CR001128 | OMePS |
| | 9 | Mock electroporation control | N/A |

CD34+ cells were electroporated as described previously, and subjected to measurement of cell viability, gene editing efficiency, and ability to produce HbF. When assessing cell viability, the results showed that varying RNP concentration had no impact on cell viability upon electroporation, either when single or dual guide format is used (Figure 39A and Figure 39B). Upon gene editing of CD34+ cells, NGS data showed that RNP concentrations down to 1.47 uM achieved maximum % gene editing efficiency for umodified CR00312 and CR001128, and modified CR00312, while maximum % gene editing efficiency for modified CR001228 was achieved at RNP concentrations as low as 2.94 uM (Figure 40A). For sgRNA formats, all gRNAs tested achieved maximum % editigin at RNP concnetrations down to 0.48 uM (Figure 40B). In measuring the ability of gene edited cells to differentiate into erythroid lineage and produce HbF, our data showed that the RNP concentration down to 2.94 uM for dgRNA-containing RNP (Figure 41A) and down to 0.97 uM for sgRNA-containing RNP (Figure 41B) resulted in maximum levels of HbF induction.

This application is being filed with a sequence listing. To the extent there are any discrepancies between the sequence listing and any sequence recited in the specification, the sequence recited in the specification should be considered the correct sequence. Unless otherwise indicated, all genomic locations are according to hg38.

### INCORPORATION BY REFERENCE

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

### EQUIVALENTS

Those skilled in the art will recognize, or be able to ascertain using no more than routine experimentation, many equivalents to the specific embodiments of the invention described herein. While this invention has been disclosed with reference to specific aspects, it is apparent that other aspects and variations of this invention may be devised by others skilled in the art without departing from the true spirit and scope of the invention. The appended claims are intended to be construed to include all such aspects and equivalent variations.

## Claims

1. A guide RNA (gRNA) molecule comprising, from 5' to 3', [targeting domain]-SEQ ID NO: 7811, wherein the targeting domain comprises SEQ ID NO: 253, wherein the targeting domain and said SEQ ID NO: 7811 are disposed on a single nucleic acid molecule, and wherein said SEQ ID NO: 7811 is disposed immediately 3' to said targeting domain.

2. The gRNA molecule of claim 1, wherein the nucleic acid molecule comprising the gRNA molecule comprises:
(a) one or more, e.g., three, phosphorothioate modifications at the 3' end of said nucleic acid molecule;
(b) one or more, e.g., three, phosphorothioate modifications at the 5' end of said nucleic acid molecule;
(c) one or more, e.g., three, 2'-O-methyl modifications at the 3' end of said nucleic acid molecule;
(d) one or more, e.g., three, 2'-O-methyl modifications at the 5' end of said nucleic acid molecule;
(e) a 2' O-methyl modification at each of the 4^{th}-to-terminal, 3^{rd}-to-terminal, and 2^{nd}-to-terminal 3' residues of said nucleic acid molecule;
(f) a 2' O-methyl modification at each of the 4^{th}-to-terminal, 3^{rd}-to-terminal, and 2^{nd}-to-terminal 5' residues of said nucleic acid molecule; or
(g) any combination thereof.

3. A composition comprising:
(a) the gRNA molecule of claim 1 or 2 and a Cas9 molecule;
(b) the gRNA molecule of claim 1 or 2 and a nucleic acid sequence encoding a Cas9 molecule;
(c) a nucleic acid sequence encoding the gRNA molecule of claim 1 or 2 and a Cas9 molecule; or
(d) a nucleic acid sequence encoding the gRNA molecule of claim 1 or 2 and a nucleic acid sequence encoding a Cas9 molecule.

4. The composition of claim 3, wherein the Cas9 molecule is an active or inactive *s*. *pyogenes* Cas9.

5. The composition of claim 3 or 4, wherein the Cas9 molecule comprises SEQ ID NO: 6611, SEQ ID NO: 7821, SEQ ID NO: 7822, SEQ ID NO: 7823, SEQ ID NO: 7824, SEQ ID NO: 7825, SEQ ID NO: 7826, SEQ ID NO: 7827, SEQ ID NO: 7828, SEQ ID NO: 7829, SEQ ID NO: 7830, or SEQ ID NO: 7831.

6. A nucleic acid sequence that encodes the gRNA molecule of claim 1 or 2.

7. A vector comprising the nucleic acid sequence of claim 6, wherein the vector is selected from the group consisting of a lentiviral vector, an adenoviral vector, an adeno-associated viral (AAV) vector, a herpes simplex virus (HSV) vector, a plasmid, a minicircle, a nanoplasmid, and an RNA vector.

8. A method of altering a cell at or near a target sequence within said cell, comprising contacting said cell with:
(a) the gRNA molecule of claim 1 or 2 and a Cas9 molecule;
(b) the gRNA molecule of claim 1 or 2 and a nucleic acid sequence encoding a Cas9 molecule;
(c) a nucleic acid sequence encoding the gRNA molecule of claim 1 or 2 and a Cas9 molecule;
(d) a nucleic acid sequence encoding the gRNA molecule of claim 1 or 2 and a nucleic acid sequence encoding a Cas9 molecule;
(e) the composition of any one of claims 3-5; or
(f) the vector of claim 7.

9. The method of claim 8, wherein:
(a) the method results in a population of cells wherein at least about 50% of the cells of the population have been altered;
(b) the altering results in a cell that is capable of differentiating into a differentiated cell of an erythroid lineage, wherein said differentiated cell exhibits an increased level of fetal hemoglobin relative to an unaltered cell;
(c) the altering results in a population of cells that is capable of differentiating into a population of differentiated cells, wherein said population of differentiated cells has an increased fraction of F cells relative to a population of unaltered cells; and/or
(d) the altering results in a cell that is capable of differentiating into a differentiated cell, wherein said differentiated cell produces at least about 6 picograms fetal hemoglobin per cell.

10. A cell altered by the method of claim 8 or 9.

11. A cell comprising the gRNA molecule of claim 1 or 2, the composition of any one of claims 3-5, the nucleic acid sequence of claim 6, or the vector of claim 7.

12. The cell of claim 10 or 11, wherein the cell is a CD34+ cell.

13. A population of cells comprising the cell of any one of claims 10-12, wherein at least about 50% of the cells of the population are a cell according to any one of claims 10-12.

14. A composition comprising the cell of any one of claims 10-12, or the population of cells of claim 13, and a pharmaceutically acceptable medium.

15. The gRNA molecule of claim 1 or 2, the composition of any one of claims 3-5 or 14, the nucleic acid sequence of claim 6, the vector of claim 7, the cell of any one of claims 10-12, or the population of cells of claim 13, for use as a medicament.

16. The gRNA molecule of claim 1 or 2, the composition of any one of claims 3-5 or 14, the nucleic acid sequence of claim 6, the vector of claim 7, the cell of any one of claims 10-12, or the population of cells of claim 13, for use in the treatment of hemoglobinopathy.

17. The gRNA molecule, the composition, the nucleic acid sequence, the vector, the cell, or the population of cells for use according to claim 16, wherein the hemoglobinopathy is sickle cell disease or beta-thalassemia.
